# EUROPEAN PATENT APPLICATION

(11) **EP 4 807 001 A1**
(43) Date of publication of application: **16.09.2026**
(21) Application number: 24876754.3
(22) Date of filing: 31.12.2024
(51) Int. Cl.: C12N 15/113, A61K 31/713, A61K 38/46, A61P 9/12, A61P 9/00

(54) **MODIFICATION TEMPLATE FOR IMPROVING INHIBITORY EFFECT OF DOUBLE-STRANDED OLIGONUCLEOTIDE ON TARGET GENE, COMBINATION THEREOF AND USE THEREOF**

(30) Priority: 06.11.2023 CN 202311463375; 08.07.2024 CN 202410909405; 26.09.2024 CN 202411357089
(71) Applicant: Hangzhou Tianlong Pharmaceutical Co., Ltd., Hangzhou, Zhejiang 310020 (CN)
(72) Inventor: YANG, Shuo, Hangzhou, Zhejiang 310009 (CN); HUANG, Zeao, Hangzhou, Zhejiang 310009 (CN); SONG, Gengshen, Hangzhou, Zhejiang 310009 (CN); GUO, Lei, Hangzhou, Zhejiang 310009 (CN); CUI, Yiwen, Hangzhou, Zhejiang 310009 (CN)
(74) Representative: Dehns
(86) International application number: PCT/CN2024/144646
(87) International publication number: WO 2025/077949

(57) **Abstract**

A modification template for improving the inhibitory effect of a double-stranded oligonucleotide on a target gene, a combination thereof and the use thereof. The double-stranded oligonucleotide is an siRNA and contains a sense strand and an antisense strand, and each nucleotide of the sense strand and antisense strand is a modified nucleotide. The double-stranded oligonucleotide is modified with a special modification template, and has a significantly improved inhibition rate on a target gene compared with a basic sequence.

## Description

The present application claims priority to Chinese Patent Application No. 2023114633757 filed on November 6, 2023, Chinese Patent Application No. 2024113570897 filed on September 26, 2024, and Chinese Patent Application No. 2024109094050 filed on July 8, 2024. The contents of the above Chinese Patent Applications are incorporated herein by reference in their entireties.

### TECHNICAL FIELD

The present disclosure belongs to the field of nucleic acid modification technology, and specifically relates to a modification template for enhancing the inhibitory effect of a double-stranded oligonucleotide on a target gene, and a combination thereof and the use thereof.

### BACKGROUND

Oligonucleotide, particularly oligonucleotide drugs, have been widely used due to their high specificity and strong target-regulation capability by acting upstream of protein expression based on the central dogma and binding to mRNA associated with the expression of pathogenic proteins according to the Watson-Crick base pairing principle. Oligonucleotide drugs typically include antisense oligonucleotides (ASOs), small interfering RNAs (siRNAs), microRNAs (miRNAs), and aptamers.

Oligonucleotides are short DNA or RNA molecules or oligomers that can readily bind to their complementary oligonucleotides, DNA, or RNA in a sequence-specific manner to form duplexes, or less commonly, hybrids of a higher order. The fundamental property enables their broad applications in genetic testing, research, and medicine. In nature, oligonucleotides are typically small RNA molecules involved in the regulation of gene expression or intermediates derived from the degradation of larger nucleic acid molecules.

RNA interference (RNAi) is a natural defense mechanism against exogenous genes. siRNA can knock out target genes by recognizing specific sequences and degrading target mRNA.

The effective molecule of classical RNAi consists of a hallmark polymer structure of 19 + 2 nucleotides (a double helix consisting of a 21-nucleotide RNA molecule and a 19-nucleotide RNA molecule with complementary bases, containing a 3' overhang of two nucleotides). One strand of the siRNA (the guide strand or antisense strand) is complementary to the mRNA transcript of the target gene, while the other strand is designated as the passenger strand (sense strand). The siRNA (antisense strand) guides Argonaute protein (AGO2) to bind complementarily to the target transcript and becomes part of the RNA-induced silencing complex (RISC). Perfect complementarity between the siRNA (antisense strand) and the target leads to cleavage of the target transcript at the position opposite nucleotides 10 to 11 of the guide strand (antisense strand), catalyzed by AGO2.

siRNA has inherent advantages over small molecules and antibodies due to the fact that siRNA functions through Watson-Crick base pairing with mRNA, whereas small molecules and monoclonal antibodies must recognize complex three-dimensional structures of specific proteins. As a result, many diseases cannot be treated by small molecules or monoclonal antibodies due to the high activity of their target molecules and the inability to recognize molecular structures with sufficient affinity and binding specificity. The mechanism of action of siRNA drugs allows regulation of the expression of target proteins at the genetic level, offering targeting specificity compared to small molecules or antibodies. The mechanism based on the base pairing principle also enables siRNA to have a broader therapeutic scope, simpler design, and shorter development cycles.

Oligonucleotides can bind sequence-specifically to complementary RNA strands, and upon hybridization, can induce RNase H-mediated cleavage of the target RNA. In natural oligonucleotides, nucleotides are linked by phosphodiester bonds, making them particularly susceptible to nuclease-mediated degradation under physiological conditions. Therefore, natural, unstructured, and unmodified oligonucleotide drugs are rapidly degraded by nucleases *in vivo*, exhibiting low activity and poor druggability. Chemical modification of oligonucleotide structures is an effective approach to enhance their activity, which can improve their stability to nucleases and affinity for RNA, and better facilitate endocytosis and tissue targeting, thereby effectively regulating the expression of target genes.

Based on the fundamental structure of oligonucleotides, *i.e.*, bases, sugar rings, and phosphate backbones, chemical modifications can be divided into three parts:
1) Base modifications: These mainly include purine modifications, pyrimidine modifications, and base substitutions. Purine modifications include N6-methyladenosine, N1-methyladenosine, and 7-methylguanosine modifications. Pyrimidine modifications include 3-methyluridine, 5-methyluridine, 5-methylcytidine, N4-acetylcytidine, pseudouridine, thiouridine, propynyluridine, and dihydrouridine modifications.
2) Sugar modifications: These mainly include modifications and substitutions of the sugar ring. Sugar modifications include 2'-modifications, 4'-modifications, 5'-modifications, isomeric modifications, and combinations thereof. The most common modifications in oligonucleotides include 2'-fluoro, 2'-alkoxy (such as 2'-O-methoxyethyl (*i.e.*, 2'-MOE) and 2'-O-methyl), 2,4'-LNA (2,4'-locked nucleic acid), and 2,4'-cEt (2,4'-constrained ethyl) modifications. Compared to natural oligonucleotides, modified oligonucleotides exhibit higher Tm values, improved serum stability, and enhanced biological activity.
3) Phosphate backbone modifications: These mainly include phosphorothioate modifications; methylphosphonate, selenophosphate, boranophosphate, and dithiophosphate modifications; substitution of the bridging oxygen atom in the phosphodiester linkage with a sulfur atom; and complete substitution of the phosphodiester linkage between nucleosides with a non-phosphorus-containing group, such as substitution of the phosphorus atom with a carbon atom, a sulfur atom, and/or a nitrogen atom, thereby forming linkages such as guanidinium or S-methylthiourea.
4) Terminal modifications: These include covalent conjugation of special groups at the 5' or/and 3' end of the sense strand and phosphorylation at the 5' end of the antisense strand.

All marketed oligonucleotide drugs are chemically modified. Since the approval of the first oligonucleotide drug, Fomivirsen (Vitravene), in 1998, the chemical modification technology for oligonucleotide drugs has continuously evolved. To date, a total of 22 oligonucleotide drugs have been approved worldwide.

**Table 1: Approved oligonucleotide drugs worldwide**

| **No.** | **Generic name (Brand name)** | **Company** | **Category** | **Indication** | **Approval date** |
|---|---|---|---|---|---|
| 1 | Fomivirsen (Vitravene) | Ionis/Novartis | ASO | Cytomegalovirus (CMV) retinitis | August 1, 1998 |
| 2 | Pegaptanib (Macugen) | NeXstar/Eyetech | Nucleic acid aptamer | Neovascular age-related macular degeneration | December 1, 2004 |
| 3 | Mipomersen (Kynamro) | Ionis/Genzyme/Kastle | ASO | Homozygous familial hypercholesterolemia | January 1, 2013 |
| 4 | Defibrotide (Defitelio) | Jazz | Oligonucleotide mixture | Hepatic veno-occlusive disease | March 1, 2016 |
| 5 | Eteplirsen (Exondys 51) | Sarepta | ASO | Duchenne muscular dystrophy | September 1, 2016 |
| 6 | Nusinersen (Spinraza) | Ionis/Biogen | ASO | Spinal muscular atrophy | December 1, 2016 |
| 7 | Patisiran (Onpattro) | Alnylam | siRNA | Hereditary transthyretin amyloidosis with polyneuropathy | August 1, 2018 |
| 8 | Inotersen (Tegsedi) | Ionis/Akcea | ASO | Hereditary transthyretin amyloidosis with polyneuropathy | October 1, 2018 |
| 9 | Volanesorsen (Waylivra) | Ionis | ASO | Familial chylomicronemia syndrome (FCS) | May 3, 2019 |
| 10 | Givosiran (Givlaari) | Alnylam | siRNA | Hepatic porphyria | November 1, 2019 |
| 11 | Golodirsen (Vyondys 53) | Sareta | ASO | Duchenne muscular dystrophy | December 1, 2019 |
| 12 | Viltolarsen (Viltepso) | Nippon Shinyaku | ASO | Duchenne muscular dystrophy | March 25, 2020 |
| 13 | Lumasiran (Oxlumo) | Alnylam Pharmaceuticals, Dicerna Pharmaceuticals (Novo Nordisk) | siRNA | Hyperoxaluria | November 24, 2020 |
| 14 | Casimersen (Amondys 45) | Sarepta Therapeutics, University of Western Australia | ASO | Duchenne muscular dystrophy | February 25, 2021 |
| 15 | Inclisiran (Leqvio) | Alnylam Pharmaceuticals, The Medicines Company (Novartis) | siRNA | Atherosclerosis, hypercholesterolemia, mixed dyslipidemia | December 22, 2021 |
| 16 | Vutrisiran (Amvuttra) | Alnylam | siRNA | Amyloidosis with polyneuropathy | June 13, 2022 |
| 17 | Tofersen (Qalsody) | Biogen | ASO | Amyotrophic lateral sclerosis (ALS) | April 25, 2023 |
| 18 | Avacincaptad Pegol (Izervay) | Iveric Bio | Nucleic acid aptamer | Geographic atrophy (GA) secondary to age-related macular degeneration (AMD) | August 4, 2023 |
| 19 | Nedosiran (Rivfloza) | Dicerna Pharmaceuticals, Inc. | siRNA | Primary hyperoxaluria | September 29, 2023 |
| 20 | Eplontersen (Wainua) | Ionis Pharmaceuticals/ AstraZeneca | ASO | Hereditary transthyretin (TTR)-mediated amyloidosis with polyneuropathy (ATTRv-PN) in adults | December 21, 2023 |
| 21 | Nusinersen | Generium | ASO | Spinal muscular atrophy | May 14, 2024 |
| 22 | Olezarsen | Akcea Therapeutics (Ionis Pharmaceuticals); Theratechnologies | ASO | Familial chylomicronemia syndrome | December 19, 2024 |

There remains a need in the art to further improve the chemical modification technology for oligonucleotide drugs in order to enhance their therapeutic efficacy.

### SUMMARY

The present disclosure is based on basic sequences of small nucleic acid molecules, including single-stranded oligonucleotides and siRNA duplexes. These basic sequences are modified using different modification patterns to obtain corresponding derivatives. Based on the above, the present disclosure provides a modification template for enhancing the inhibitory effect of a double-stranded oligonucleotide on a target gene, and a combination thereof and the use thereof.

The present disclosure addresses the above technical problems through the following technical solutions.

In one aspect, the present disclosure provides a modification template for enhancing the inhibitory effect of a double-stranded oligonucleotide on a target gene, wherein the double-stranded oligonucleotide is an siRNA comprising a sense strand and an antisense strand; each nucleotide of the sense strand and the antisense strand is a modified nucleotide; wherein the sense strand comprises nucleotide sequence 1, and the antisense strand comprises nucleotide sequence 2; the nucleotide sequence 1 and the nucleotide sequence 2 are at least partially reverse complementary to form a duplex region; the reverse complementary duplex region is 17 to 21 bp in length;

from the 5' end to the 3' end, in the nucleotide sequence 2, at least two nucleotides among positions 2 to 6 are 2'-fluoro-modified nucleotides; the nucleotides at position 14 and/or 16 are 2'-fluoro-modified nucleotides; the nucleotides at the remaining positions are 2'-O-methyl-modified nucleotides; and positions 1 and 2 as well as positions of the second and third from last comprise phosphorothioate backbones.

In some embodiments of the present disclosure, at least two nucleotides among positions 2 to 6 of the nucleotide sequence 2 are selected from the following combinations:
(1) nucleotides at positions 2 and 6;
(2) nucleotides at positions 2, 3, 4, and 6;
(3) nucleotides at positions 2, 4, 5, and 6;
(4) nucleotides at positions 2, 5, and 6;
(5) nucleotides at positions 2, 3, 5, and 6;
(6) nucleotides at positions 2, 3, and 6.

In some embodiments of the present disclosure, from the 5' end to the 3' end, the nucleotide sequence 1 is modified as follows:
(1) the nucleotides at positions 6 and 16 are 2'-fluoro-modified nucleotides; at least two nucleotides among positions 8 to 10, preferably positions 8 to 10 or positions 8 and 10, are 2'-fluoro-modified nucleotides; the nucleotides at the remaining positions are 2'-O-methyl-modified nucleotides; and positions 1 and 2 comprise phosphorothioate backbones; or
(2) the nucleotides at positions 7 and 17 are 2'-fluoro-modified nucleotides; at least two nucleotides among positions 9 to 11, preferably positions 9 to 11 or positions 9 and 11, are 2'-fluoro-modified nucleotides; the nucleotides at the remaining positions are 2'-O-methyl-modified nucleotides; and positions 1 and 2 comprise phosphorothioate backbones.

In some embodiments of the present disclosure, the nucleotide sequence 1 is 21 or 20 nucleotides in length, and the nucleotide sequence 2 is 23 or 22 nucleotides in length.

In some embodiments of the present disclosure, the nucleotide sequence 1 is 20 nucleotides in length, and the nucleotide sequence 2 is 22 nucleotides in length; or, the nucleotide sequence 1 is 21 nucleotides in length, and the nucleotide sequence 2 is 23 nucleotides in length.

In some embodiments of the present disclosure, from the 5' end to the 3' end, the nucleotides in the antisense strand are modified according to any one of the following patterns A, B, C, D, E, F, and G:
A: indicates that, from the 5' end of the antisense strand, the nucleotides at positions 1, 3, 4, 5, 7, 8, 9, 10, 11, 12, 13, 15, 17, 18, 19, 20, 21, 22, and 23 are 2'-O-methyl-modified nucleotides; the nucleotides at positions 2, 6, 14, and 16 are 2'-fluoro-modified nucleotides; and positions 1, 2, 21, and 22 comprise phosphorothioate backbones;
B: indicates that, from the 5' end of the antisense strand, the nucleotides at positions 1, 5, 7, 8, 9, 10, 11, 12, 13, 15, 17, 18, 19, 20, 21, 22, and 23 are 2'-O-methyl-modified nucleotides; the nucleotides at positions 2, 3, 4, 6, 14, and 16 are 2'-fluoro-modified nucleotides; and positions 1, 2, 21, and 22 comprise phosphorothioate backbones;
C: indicates that, from the 5' end of the antisense strand, the nucleotides at positions 1, 3, 7, 8, 9, 10, 11, 12, 13, 15, 17, 18, 19, 20, 21, 22, and 23 are 2'-O-methyl-modified nucleotides; the nucleotides at positions 2, 4, 5, 6, 14, and 16 are 2'-fluoro-modified nucleotides; and positions 1, 2, 21, and 22 comprise phosphorothioate backbones;
D: indicates that, from the 5' end of the antisense strand, the nucleotides at positions 1, 3, 4, 7, 8, 9, 10, 11, 12, 13, 15, 17, 18, 19, 20, 21, 22, and 23 are 2'-O-methyl-modified nucleotides; the nucleotides at positions 2, 5, 6, 14, and 16 are 2'-fluoro-modified nucleotides; and positions 1, 2, 21, and 22 comprise phosphorothioate backbones;
E: indicates that, from the 5' end of the antisense strand, the nucleotides at positions 1, 4, 7, 8, 9, 10, 11, 12, 13, 15, 17, 18, 19, 20, 21, 22, and 23 are 2'-O-methyl-modified nucleotides; the nucleotides at positions 2, 3, 5, 6, 14, and 16 are 2'-fluoro-modified nucleotides; and positions 1, 2, 21, and 22 comprise phosphorothioate backbones;
F: indicates that, from the 5' end of the antisense strand, the nucleotides at positions 1, 4, 5, 7, 8, 9, 10, 11, 12, 13, 15, 17, 18, 19, 20, 21, 22, and 23 are 2'-O-methyl-modified nucleotides; the nucleotides at positions 2, 3, 6, 14, and 16 are 2'-fluoro-modified nucleotides; and positions 1, 2, 21, and 22 comprise phosphorothioate backbones; G: indicates that, from the 5' end of the antisense strand, the nucleotides at positions 1, 3, 4, 5, 7, 8, 9, 10, 11, 12, 13, 15, 17, 18, 19, 20, 21, 22, and 23 are 2'-O-methyl-modified nucleotides; the nucleotides at positions 2, 6, 14, and 16 are 2'-fluoro-modified nucleotides; and positions 1, 2, 21, and 22 comprise phosphorothioate backbones;
H: indicates that, from the 5' end of the antisense strand, the nucleotides at positions 1, 3, 4, 5, 7, 8, 9, 10, 11, 12, 13, 15, 16, 17, 18, 19, 20, 21, 22, and 23 are 2'-O-methyl-modified nucleotides; the nucleotides at positions 2, 6, and 14 are 2'-fluoro-modified nucleotides; and positions 1, 2, 21, and 22 comprise phosphorothioate backbones;
and/or, from the 5' end to the 3' end, the nucleotides in the sense strand are modified according to any one of the following patterns a to d:
a: indicates that, from the 5' end of the sense strand, the nucleotides at positions 1, 2, 3, 4, 5, 6, 8, 12, 13, 14, 15, 16, 18, 19, 20, and 21 are 2'-O-methyl-modified nucleotides; the nucleotides at positions 7, 9, 10, 11, and 17 are 2'-fluoro-modified nucleotides; and positions 1 and 2 comprise phosphorothioate backbones;
b: indicates that, from the 5' end of the sense strand, the nucleotides at positions 1, 2, 3, 4, 5, 6, 8, 10, 12, 13, 14, 15, 16, 18, 19, 20, and 21 are 2'-O-methyl-modified nucleotides; the nucleotides at positions 7, 9, 11, and 17 are 2'-fluoro-modified nucleotides; and positions 1 and 2 comprise phosphorothioate backbones;
c: indicates that, from the 5' end of the sense strand, the nucleotides at positions 1, 2, 3, 4, 5, 6, 8, 12, 13, 14, 15, 16, 17, 18, 19, 20, and 21 are 2'-O-methyl-modified nucleotides; the nucleotides at positions 7, 9, 10, and 11 are 2'-fluoro-modified nucleotides; and positions 1 and 2 comprise phosphorothioate backbones;
d: indicates that, from the 5' end of the sense strand, the nucleotides at positions 1, 2, 3, 4, 5, 6, 8, 11, 12, 13, 14, 15, 16, 17, 18, 19, 20, and 21 are 2'-O-methyl-modified nucleotides; the nucleotides at positions 7, 9, and 10 are 2'-fluoro-modified nucleotides; and positions 1 and 2 comprise phosphorothioate backbones.

In some embodiments of the present disclosure, from the 5' end to the 3' end, the nucleotides in the antisense strand are modified according to any one of the following patterns A', B', C', D', E', F', and G':
A': indicates that, from the 5' end of the antisense strand, the nucleotides at positions 1, 3, 4, 5, 7, 8, 9, 10, 11, 12, 13, 15, 17, 18, 19, 20, 21, and 22 are 2'-O-methyl-modified nucleotides; the nucleotides at positions 2, 6, 14, and 16 are 2'-fluoro-modified nucleotides; and positions 1, 2, 20, and 21 comprise phosphorothioate backbones;
B': indicates that, from the 5' end of the antisense strand, the nucleotides at positions 1, 5, 7, 8, 9, 10, 11, 12, 13, 15, 17, 18, 19, 20, 21, and 22 are 2'-O-methyl-modified nucleotides; the nucleotides at positions 2, 3, 4, 6, 14, and 16 are 2'-fluoro-modified nucleotides; and positions 1, 2, 20, and 21 comprise phosphorothioate backbones;
C': indicates that, from the 5' end of the antisense strand, the nucleotides at positions 1, 3, 7, 8, 9, 10, 11, 12, 13, 15, 17, 18, 19, 20, 21, and 22 are 2'-O-methyl-modified nucleotides; the nucleotides at positions 2, 4, 5, 6, 14, and 16 are 2'-fluoro-modified nucleotides; and positions 1, 2, 20, and 21 comprise phosphorothioate backbones;
D': indicates that, from the 5' end of the antisense strand, the nucleotides at positions 1, 3, 4, 7, 8, 9, 10, 11, 12, 13, 15, 17, 18, 19, 20, 21, and 22 are 2'-O-methyl-modified nucleotides; the nucleotides at positions 2, 5, 6, 14, and 16 are 2'-fluoro-modified nucleotides; and positions 1, 2, 20, and 21 comprise phosphorothioate backbones;
E': indicates that, from the 5' end of the antisense strand, the nucleotides at positions 1, 4, 7, 8, 9, 10, 11, 12, 13, 15, 17, 18, 19, 20, 21, and 22 are 2'-O-methyl-modified nucleotides; the nucleotides at positions 2, 3, 5, 6, 14, and 16 are 2'-fluoro-modified nucleotides; and positions 1, 2, 20, and 21 comprise phosphorothioate backbones;
F': indicates that, from the 5' end of the antisense strand, the nucleotides at positions 1, 4, 5, 7, 8, 9, 10, 11, 12, 13, 15, 17, 18, 19, 20, 21, and 22 are 2'-O-methyl-modified nucleotides; the nucleotides at positions 2, 3, 6, 14, and 16 are 2'-fluoro-modified nucleotides; and positions 1, 2, 20, and 21 comprise phosphorothioate backbones;
G': indicates that, from the 5' end of the antisense strand, the nucleotides at positions 1, 3, 4, 5, 7, 8, 9, 10, 11, 12, 13, 15, 16, 17, 18, 19, 20, 21, and 22 are 2'-O-methyl-modified nucleotides; the nucleotides at positions 2, 6, and 14 are 2'-fluoro-modified nucleotides; and positions 1, 2, 20, and 21 comprise phosphorothioate backbones;
and/or, from the 5' end to the 3' end, the nucleotides in the sense strand are modified according to any one of the following patterns a', b', c', and d':
a': indicates that, from the 5' end of the sense strand, the nucleotides at positions 1, 2, 3, 4, 5, 7, 11, 12, 13, 14, 15, 17, 18, 19, and 20 are 2'-O-methyl-modified nucleotides; the nucleotides at positions 6, 8, 9, 10, and 16 are 2'-fluoro-modified nucleotides; and positions 1 and 2 comprise phosphorothioate backbones;
b': indicates that, from the 5' end of the sense strand, the nucleotides at positions 1, 2, 3, 4, 5, 7, 9, 11, 12, 13, 14, 15, 17, 18, 19, and 20 are 2'-O-methyl-modified nucleotides; the nucleotides at positions 6, 8, 10, and 16 are 2'-fluoro-modified nucleotides; and positions 1 and 2 comprise phosphorothioate backbones;
c': indicates that, from the 5' end of the sense strand, the nucleotides at positions 1, 2, 3, 4, 5, 7, 11, 12, 13, 14, 15, 16, 17, 18, 19, and 20 are 2'-O-methyl-modified nucleotides; the nucleotides at positions 6, 8, 9, and 10 are 2'-fluoro-modified nucleotides; and positions 1 and 2 comprise phosphorothioate backbones;
d': indicates that, from the 5' end of the sense strand, the nucleotides at positions 1, 2, 3, 4, 5, 7, 10, 11, 12, 13, 14, 15, 16, 17, 18, 19, and 20 are 2'-O-methyl-modified nucleotides; the nucleotides at positions 6, 8, and 9 are 2'-fluoro-modified nucleotides; and positions 1 and 2 comprise phosphorothioate backbones.

In some embodiments of the present disclosure, from the 5' end to the 3' end, the double-stranded oligonucleotide is modified according to any one of the following combinations (i) to (x):
(i) the antisense strand is modified according to pattern A, and the sense strand is modified according to pattern a;
(ii) the antisense strand is modified according to pattern B, and the sense strand is modified according to pattern a;
(iii) the antisense strand is modified according to pattern C, and the sense strand is modified according to pattern a;
(iv) the antisense strand is modified according to pattern B, and the sense strand is modified according to pattern b;
(v) the antisense strand is modified according to pattern C, and the sense strand is modified according to pattern b;
(vi) the antisense strand is modified according to pattern G, and the sense strand is modified according to pattern c;
(vii) the antisense strand is modified according to pattern A, and the sense strand is modified according to pattern d;
(viii) the antisense strand is modified according to pattern D, and the sense strand is modified according to pattern b;
(ix) the antisense strand is modified according to pattern E, and the sense strand is modified according to pattern b;
(x) the antisense strand is modified according to pattern F, and the sense strand is modified according to pattern b.

In some embodiments of the present disclosure, from the 5' end to the 3' end, the double-stranded oligonucleotide is modified according to any one of the following combinations (1) to (8):
(1) the antisense strand is modified according to pattern A', and the sense strand is modified according to pattern a';
(2) the antisense strand is modified according to pattern B', and the sense strand is modified according to pattern a';
(3) the antisense strand is modified according to pattern C', and the sense strand is modified according to pattern a';
(4) the antisense strand is modified according to pattern B', and the sense strand is modified according to pattern b';
(5) the antisense strand is modified according to pattern C', and the sense strand is modified according to pattern b';
(6) the antisense strand is modified according to pattern D', and the sense strand is modified according to pattern b';
(7) the antisense strand is modified according to pattern E', and the sense strand is modified according to pattern b';
(8) the antisense strand is modified according to pattern F', and the sense strand is modified according to pattern b'.

In some embodiments of the present disclosure, the nucleotide at position 1 from the 5' end of the antisense strand is a 5'-phosphorylated nucleotide or a nucleotide modified with a 5'-phosphate analog, including, but not limited to, the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are as follows: 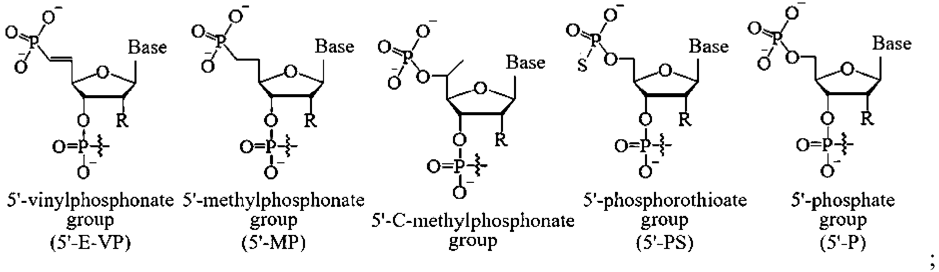
wherein R is hydrogen, hydroxyl, amino, C₁₋₄ alkyl, aryl, C₁-₄ alkoxy, C₁-₄ alkylcarbonylamino, or halogen;
the base is selected from adenine, guanine, cytosine, thymine, and uracil.

In some embodiments of the present disclosure, the nucleotide at position 1 from the 5' end of the antisense strand is a 5'-vinyl-(E)-phosphonate-2'-O-methyl-modified nucleotide.

In some embodiments of the present disclosure, one or more nucleotides at positions 2 to 8 from the 5' end of the antisense strand, preferably at position 6 and/or 7, comprises a modification group, wherein the modification group is selected from UNA, GNA, and DNA, wherein the structures of UNA and GNA are as follows: 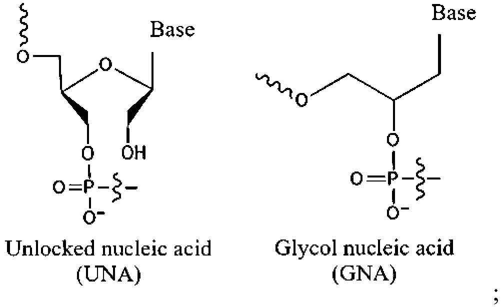 wherein the base is selected from one or more adenine, guanine, cytosine, thymine, and uracil.

In another aspect, the present disclosure provides a double-stranded oligonucleotide modified using the modification template described herein.

In some embodiments of the present disclosure, the double-stranded oligonucleotide targets the mRNA of one of the following genes: PCSK9 gene, HBV gene, and AGT gene.

In another aspect, the present disclosure provides a conjugate for reducing gene expression, wherein the conjugate comprises the double-stranded oligonucleotide described herein and a ligand conjugated thereto.

In some embodiments of the present disclosure, the ligand is conjugated to the 3' end or 5' end of the sense strand of the double-stranded oligonucleotide.

In some embodiments of the present disclosure, the ligand is one or more GalNAc derivatives attached via a bivalent or trivalent branched linker.

In some embodiments of the present disclosure, the ligand is as follows: 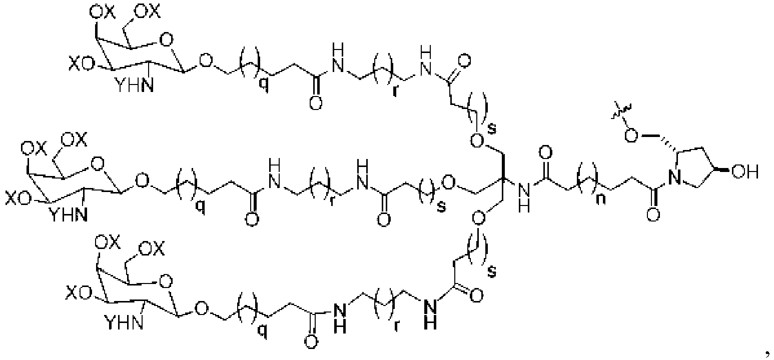 wherein X is hydrogen, a hydroxyl protecting group, or H, the hydroxyl protecting group including acetyl, benzoyl, or isobutyryl; Y is an amino protecting group or H, the amino protecting group being formyl, acetyl, propionyl, *n*-butyryl, or isobutyryl; n is an integer from 0 to 20; q, r, and s are independently integers from 1 to 7.

In some embodiments of the present disclosure, the ligand is as follows:

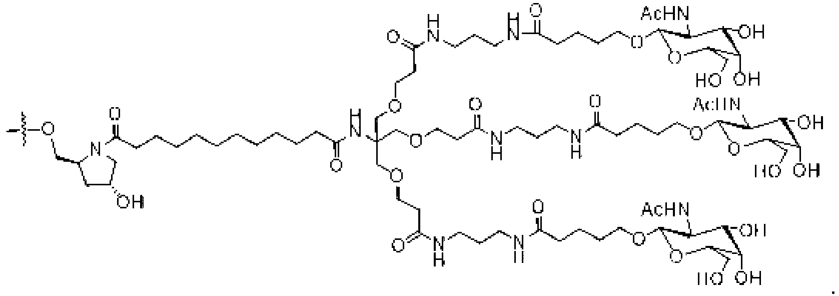

In some embodiments of the present disclosure, the ligand is as follows: 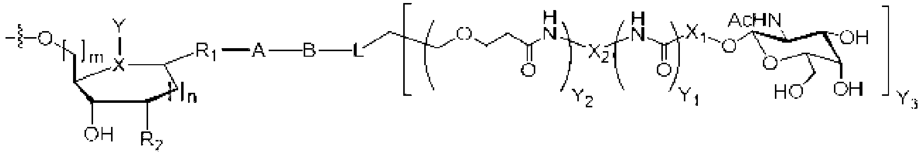
wherein X is oxygen, nitrogen, or sulfur;
Y is alkyl or aryl;
R₁ is oxygen or sulfur;
R₂ is hydrogen, amino, C₁₋₄ alkyl, aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
Y₁ is 0 or 1;
Y₂ is 0, 1, or 2;
Y₃ is 1, 2, or 3;
m is an integer from 0 to 4;
n is an integer from 0 to 4.

In some embodiments of the present disclosure, the ligand is G4, G5, G6, G7, G101, G102, G103, G105, or G106:

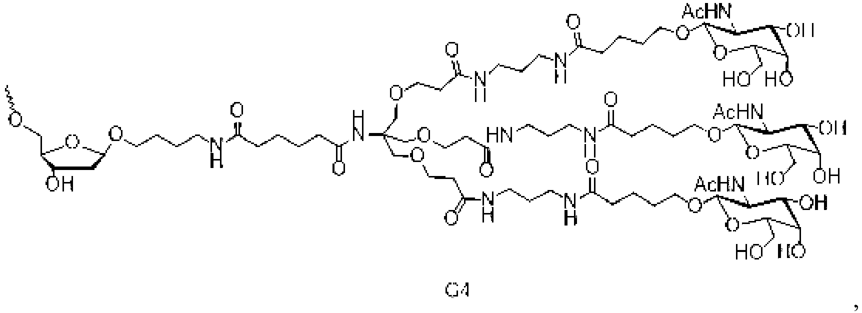

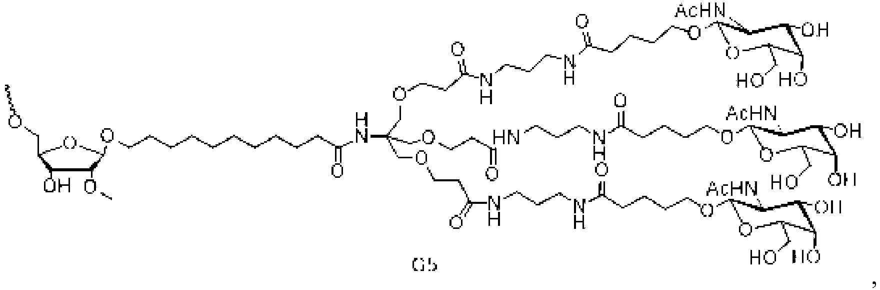

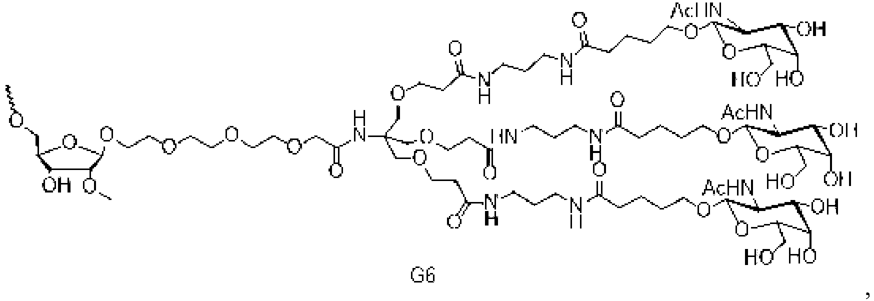

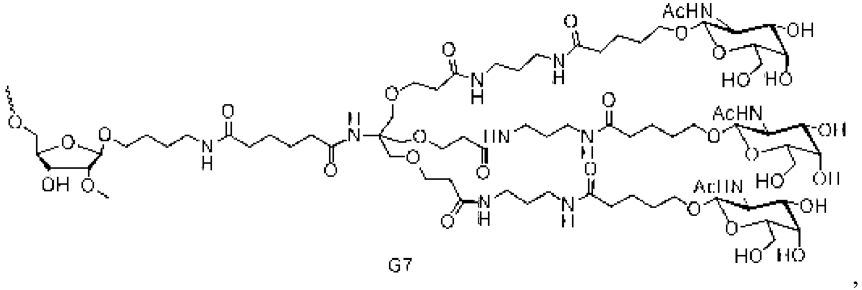

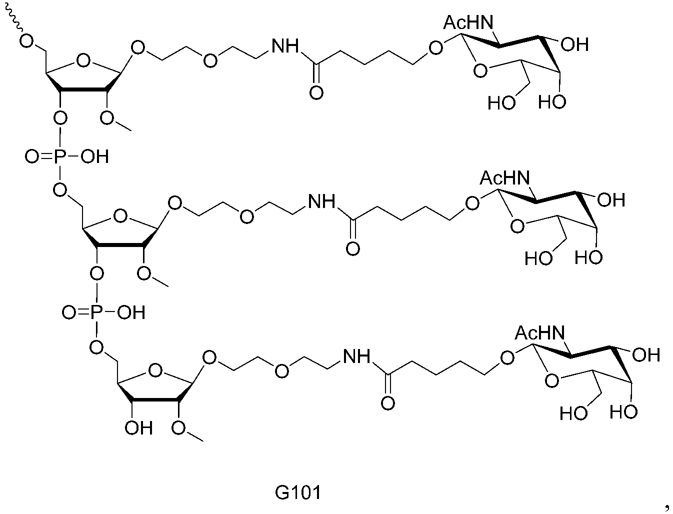

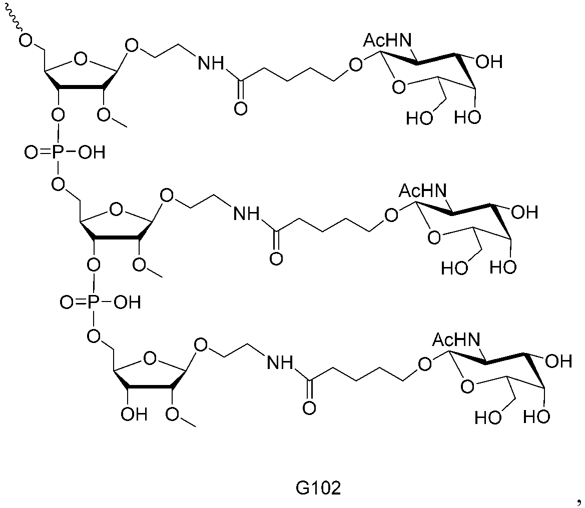

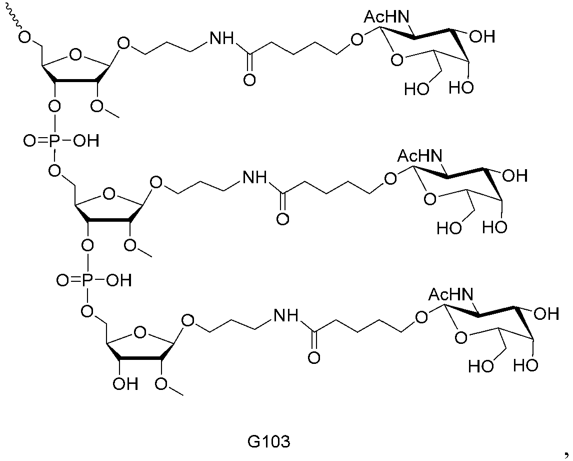

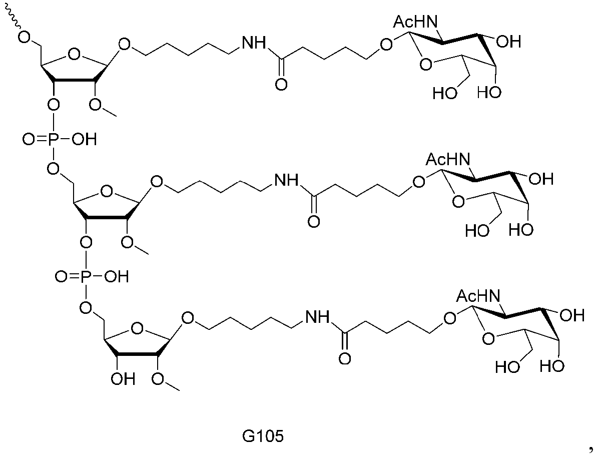

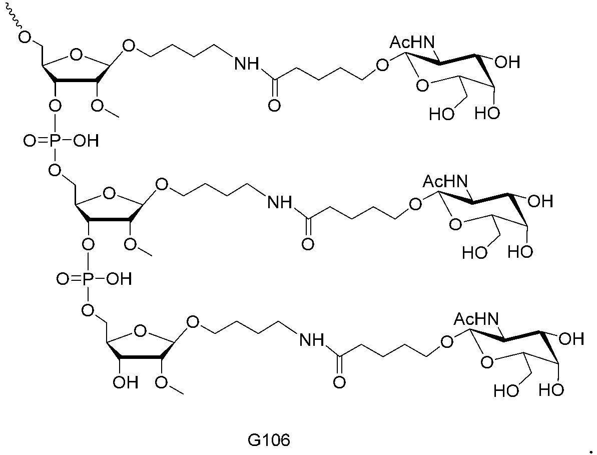

In some embodiments of the present disclosure, the conjugate has a structure as shown below:

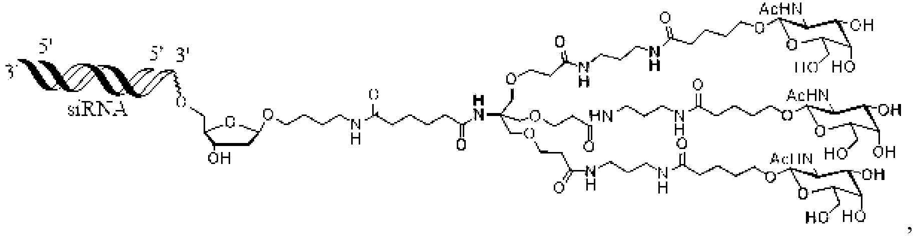

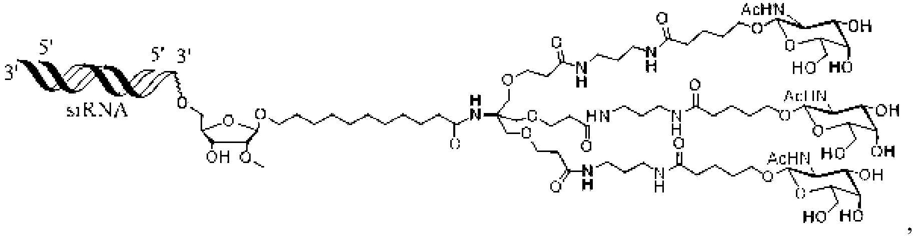

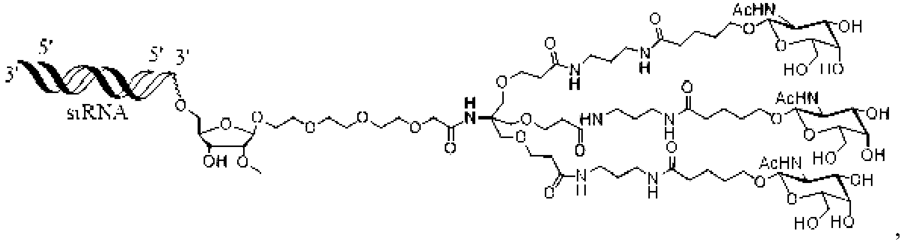

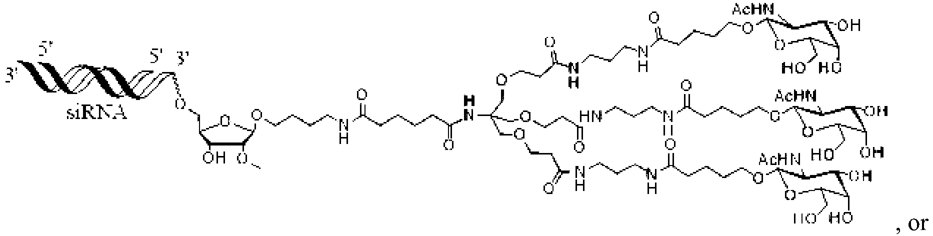

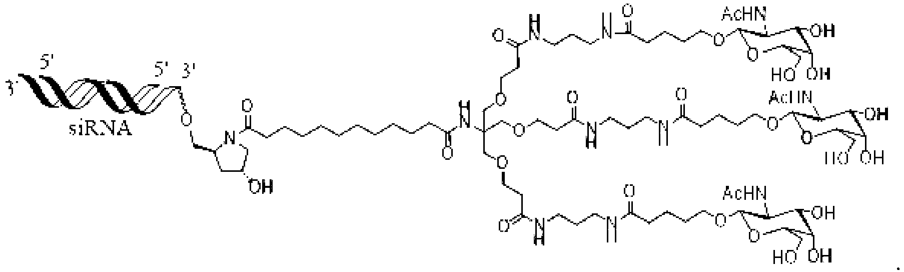

In some embodiments of the present disclosure, the double-stranded oligonucleotide is conjugated to ligand G4, G5, G6, or G7.

In another aspect, the present disclosure also provides a pharmaceutical composition comprising the double-stranded oligonucleotide described herein or the conjugate described herein, and a pharmaceutically acceptable carrier.

In some embodiments of the present disclosure, the carrier is water, saline, or a buffered solution.

In some embodiments of the present disclosure, the buffer in the buffered solution comprises acetate, citrate, prolamin, carbonate or phosphate, Tris-HCl, or any combination thereof; for example, phosphate.

In another aspect, the present disclosure also provides the use of the double-stranded oligonucleotide described herein, the conjugate described herein, or the pharmaceutical composition described herein in the manufacture of a medicament for modulating or treating a disease or disorder; wherein the medicament achieves the effect of modulating or treating the disease or disorder by downregulating gene expression via the double-stranded oligonucleotide, the conjugate, or the pharmaceutical composition.

In some embodiments of the present disclosure, the gene is selected from the following genes: PCSK9 gene, HBV gene, and AGT gene.

In another aspect, the present disclosure also provides the double-stranded oligonucleotide described herein, the conjugate described herein, or the pharmaceutical composition described herein for use in modulating or treating a disease or disorder; wherein the double-stranded oligonucleotide, the conjugate, or the pharmaceutical composition achieves the effect of modulating or treating the disease or disorder by downregulating gene expression.

The present disclosure also provides a method of treating or preventing a disease or disorder that can be modulated or treated by downregulating gene expression, comprising administering to a subject in need thereof an effective amount of the double-stranded oligonucleotide described herein, the conjugate described herein, or the pharmaceutical composition described herein.

In some embodiments of the present disclosure, the gene is selected from the following genes: PCSK9 gene, HBV gene, and AGT gene.

In some embodiments of the present disclosure, the disease is selected from one or more chronic hepatitis B, liver fibrosis, cirrhosis, liver cancer, acute hepatitis B, and diseases associated with co-infection of HBV and hepatitis D virus.

In some embodiments of the present disclosure, the disease is selected from hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction.

In some embodiments of the present disclosure, the disease is selected from hypercholesterolemia, atherosclerosis, dyslipidemia, and cardiovascular and cerebrovascular diseases.

Based on common knowledge in the art, the aforementioned preferred conditions may be combined in any manner to obtain preferred examples of the present disclosure.

The reagents and raw materials used in the present disclosure are all commercially available.

The positive and progressive effects of the present disclosure are as follows:
(1) The multiply modified sequences can achieve inhibition rates of up to greater than 90%. Furthermore, conjugation of the modified sequences with GalNAc compounds enables efficient delivery to the liver in animals, significantly inhibiting target gene expression and significantly reducing blood pressure levels.
(2) The sequences modified using the modification templates of the present disclosure exhibit significantly enhanced target gene inhibitory activity compared to the unmodified sequences with minor differences disclosed in the prior art, with an increase of up to 91.0%.

### BRIEF DESCRIPTION OF THE DRAWINGS

To more clearly illustrate the technical solutions of the embodiments of the present disclosure, the accompanying drawings used in the embodiments will be briefly described below. It is apparent that the drawings described below relate only to certain embodiments of the present disclosure and are not intended to limit the present disclosure.
FIG. 1 shows alternately modified sequences with significant inhibitory effects on the PCSK9 gene, all of which exhibit inhibition rates greater than 50%.
FIG. 2 shows alternately modified sequences with inhibition rates of 30% to 50% against the PCSK9 gene.

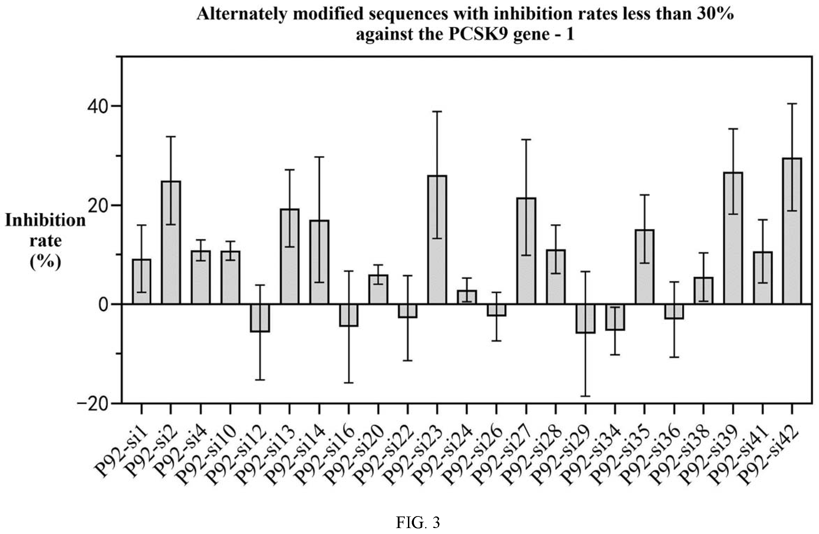

FIG. 3 shows alternately modified sequences with inhibition rates less than 30% against the PCSK9 gene.
FIG. 4 shows alternately modified sequences with inhibition rates less than 30% against the PCSK9 gene.
FIG. 5 shows unmodified sequences with significant inhibitory effects on the PCSK9 gene, all of which exhibit inhibition rates greater than 50%.
FIG. 6 shows unmodified sequences with inhibition rates less than 40% against the PCSK9 gene.
FIG. 7 shows the inhibition rates of the PCSK9 gene by basic sequence 5 modified using different templates.
FIG. 8 shows the inhibition rates of the PCSK9 gene by basic sequences 5, 51, 81, and 84 using template modifications, anti-off-target modifications, or/and 5'-E-VP modifications.
FIG. 9 shows the inhibition rates of PCSK9 protein in animal serum by basic sequences 5, 51, 81, 82, and 84 using different modification patterns and conjugated with GalNAc compounds.
FIG. 10 shows reduction in low-density lipoprotein cholesterol (LDL-C) levels in animal serum by basic sequences 5, 51, 81, 82, and 84 using different modification patterns and conjugated with GalNAc compounds.
FIG. 11 shows reduction in total cholesterol (TC) levels in animal serum by basic sequences 5, 51, 81, 82, and 84 using different modification patterns and conjugated with GalNAc compounds.
FIG. 12A shows serum HBsAg levels in AAV-HBV model mice treated with candidate siRNA sequences at a dose of 3 mg/kg.
FIG. 12B shows serum HBV DNA levels in AAV-HBV model mice treated with candidate siRNA sequences at a dose of 3 mg/kg.
FIG. 12C shows serum HBeAg levels in AAV-HBV model mice treated with candidate siRNA sequences at a dose of 3 mg/kg.
FIG. 12D shows serum HBsAb levels in AAV-HBV model mice treated with candidate siRNA sequences at a dose of 3 mg/kg.
FIG. 12E shows serum ALT levels in AAV-HBV model mice treated with candidate siRNA sequences at a dose of 3 mg/kg.
FIG. 12F shows body weight changes in AAV-HBV model mice treated with candidate siRNA sequences at a dose of 3 mg/kg.
FIG. 13A shows serum HBsAg levels in mice after secondary challenge.
FIG. 13B shows serum HBV DNA levels in mice after secondary challenge.
FIG. 13C shows serum HBeAg levels in mice after secondary challenge.
FIG. 13D shows serum HBbAg levels in mice after secondary challenge.
FIG. 13E shows serum ALT levels in mice after secondary challenge.
FIG. 13F shows body weight changes in mice after secondary challenge.
FIG. 14A shows HBsAg levels in AAV-HBV model mice treated with candidate siRNA sequences at a dose of 0.5 mg/kg.
FIG. 14B shows HBV DNA levels in AAV-HBV model mice treated with candidate siRNA sequences at a dose of 0.5 mg/kg.
FIG. 14C shows HBeAg levels in AAV-HBV model mice treated with candidate siRNA sequences at a dose of 0.5 mg/kg.
FIG. 14D shows HBsAb levels in AAV-HBV model mice treated with candidate siRNA sequences at a dose of 0.5 mg/kg.
FIG. 14E shows ALT levels in AAV-HBV model mice treated with candidate siRNA sequences at a dose of 0.5 mg/kg.
FIG. 14F shows body weight changes in AAV-HBV model mice treated with candidate siRNA sequences at a dose of 0.5 mg/kg.
FIG. 15A shows HBsAg levels in AAV-HBV model mice treated with candidate siRNA sequences conjugated with different GalNAc moieties.
FIG. 15B shows HBV DNA levels in AAV-HBV model mice treated with candidate siRNA sequences conjugated with different GalNAc moieties.
FIG. 15C shows HBeAg levels in AAV-HBV model mice treated with candidate siRNA sequences conjugated with different GalNAc moieties.
FIG. 15D shows ALT levels in AAV-HBV model mice treated with candidate siRNA sequences conjugated with different GalNAc moieties.
FIG. 15E shows body weight changes in AAV-HBV model mice treated with candidate siRNA sequences conjugated with different GalNAc moieties.
FIG. 16 shows alternately modified sequences with significant inhibitory effects on the AGT gene, all of which exhibit inhibition rates greater than 40%.
FIG. 17 shows alternately modified sequences with inhibition rates of 25% to 40% against the AGT gene.
FIG. 18 shows alternately modified sequences with inhibition rates less than 25% against the AGT gene.
FIG. 19 shows unmodified sequences with significant inhibitory effects on the AGT gene, all of which exhibit inhibition rates greater than 45%.
FIG. 20 shows unmodified sequences with inhibition rates less than 45% against the AGT gene.
FIG. 21 shows the inhibition rates of the AGT gene by unmodified sequence 1579 modified using different templates, as well as the inhibition rate of the AGT gene by sequence 579P disclosed in the prior art.
FIG. 22 shows the inhibition rates of the AGT gene by unmodified sequences 1578s and 1835 using template modifications and anti-off-target modifications.
FIG. 23 shows the inhibition rates of AGT protein in animal serum by unmodified sequences 1579, 1789, 1812, 1576s, 1578s, and 1585s using different modification patterns and conjugated with GalNAc compounds.
FIG. 24 shows the inhibition rates of AGT protein in animal serum at different time points by unmodified sequences 1579, 1789, 1812, 1576s, 1578s, and 1585s using different modification patterns and conjugated with GalNAc compounds.

### DETAILED DESCRIPTION OF THE PREFERRED EMBODIMENT

To facilitate understanding of the present disclosure, certain terms are first defined. In addition, it should be noted that whenever a value or a range of values for a parameter is recited, it is intended that intermediate values and ranges between the recited values are also intended to be encompassed within the scope of the present disclosure.

As used herein, the articles "a" and "an" refer to one or more than one (*i.e.*, at least one) of the grammatical object of the article. By way of example, "an element" refers to one element or more than one element, such as multiple elements.

The term "including" as used herein means, and is used interchangeably with, the phrase "including, but not limited to".

The term "or" as used herein means, and is used interchangeably with, the term "and/or", unless the context clearly indicates otherwise.

As used herein, the term "about" or "approximately", as applied to one or more target values, refers to a value that is similar to the stated reference value. In certain embodiments, unless otherwise specified or clearly evident from the context, the term "approximately" or "about" refers to a value within 20%, 19%, 18%, 17%, 16%, 15%, 14%, 13%, 12%, 11%, 10%, 9%, 8%, 7%, 6%, 5%, 4%, 3%, 2%, 1%, or less of the stated reference value in either direction (greater than or less than) (unless such a range would exceed 100% of the possible value).

As used herein, "PCSK9" refers to the proprotein convertase subtilisin/kexin type 9 gene or protein.

As used herein, "HBV" refers to hepatitis B virus, including genotypes A, B, C, D, E, F, G, H, I, J, and their subtypes, but is not limited to any specific genotype.

As used herein, "AGT" refers to the angiotensinogen gene or protein.

"G", "C", "A", and "U" each generally represent nucleotides containing guanine, cytosine, adenine, and uracil as their bases, respectively. "T" and "dT" are used interchangeably herein and refer to a deoxyribonucleotide in which the nucleobase is thymine, such as deoxyribothymine, 2'-deoxythymidine, or thymidine. However, it should be understood that the term "ribonucleotide", "nucleotide", or "deoxyribonucleotide" may also refer to a modified nucleotide (as described in further detail below) or a surrogate replacement moiety. Those skilled in the art will readily appreciate that guanine, cytosine, adenine, and uracil may be replaced by other moieties without substantially altering the base pairing properties of an oligonucleotide (including a nucleotide comprising such a replacement moiety). For example, without limitation, a nucleotide containing inosine as its base may pair with a nucleotide containing adenine, cytosine, or uracil. Thus, nucleotides containing uracil, guanine, or adenine may be replaced in the nucleotide sequences of the present disclosure by a nucleotide containing, for example, inosine. Sequences containing such replacement moieties are applicable to the double-stranded oligonucleotides, conjugates, pharmaceutical compositions, and methods of the present disclosure.

The terms "siRNA duplex", "siRNA", "double-stranded oligonucleotide", and "RNAi agent" are used interchangeably herein and refer to a class of double-stranded RNA molecules, also known in the art as short interfering RNA or silencing RNA. siRNAs typically comprise a sense strand (also known as a passenger strand) and an antisense strand (also known as a guide strand), wherein each strand is 17 to 30 nucleotides in length, typically 19 to 25 nucleotides in length, wherein the antisense strand is complementary (*e.g.*, at least 95% complementary, such as fully complementary) to a target nucleic acid (suitably a mature mRNA sequence), and the sense strand is complementary to the antisense strand, such that the sense strand and the antisense strand form a duplex or duplex region. The siRNA strands may form a blunt-ended duplex, or preferably, the 3' ends of the sense and antisense strands may form a 3' overhang, for example, 1, 2, or 3 nucleotides, to resemble the product produced by Dicer, which may form a RISC substrate *in vivo*. Effective extended forms of Dicer substrates have been described in US 8,349,809 and US 8,513,207, which are incorporated herein by reference. In some examples, the sense strand and/or the antisense strand have a 3' overhang of two nucleotides in length. Thus, the duplex region may be, for example, 17 to 25 nucleotides in length, such as 21 to 23 nucleotides in length. In some examples, each nucleotide of the siRNA contains a chemical modification.

The term "antisense strand" refers to the strand of RNAi (*e.g.*, siRNA) which includes a region that is substantially complementary to a target sequence. As used herein, the term "region of complementarity" refers to a region on the antisense strand that is substantially complementary to a sequence defined herein (*e.g.*, a target sequence). When the region of complementarity is not fully complementary to the target sequence, mismatches may occur in the internal or terminal regions of the molecule. Typically, mismatches are most tolerated in the terminal regions, for example, within 5, 4, 3, or 2 nucleotides from the 5' and/or 3' ends.

As used herein, the term "sense strand" refers to the strand of RNAi that includes a region substantially complementary to the antisense strand (as defined herein).

As used herein, the term "basic sequence" refers to a single-stranded oligonucleotide, siRNA duplex, or the like that does not contain modified nucleotides. In some cases, the term also refers to the corresponding nucleotide sequence of an antisense oligonucleotide or siRNA. In the present disclosure, "antisense oligonucleotide basic sequence", "unmodified ASO", and "ASO basic sequence" are used interchangeably; "siRNA basic sequence", "unmodified siRNA", and "siRNA duplex basic sequence" are used interchangeably.

In the present disclosure, "other agents that reduce PCSK9, HBV, or AGT gene expression" refer to agents that do not contain the siRNAs and/or siRNA derivatives of the present disclosure.

As used herein, the term "reducing" is used interchangeably with "decreasing", "silencing", "downregulating", "suppressing", "inhibiting", and other similar terms, and includes any level of reduction.

As used herein, the phrase "inhibiting the expression of PCSK9" includes inhibition of expression of any PCSK9 gene (*e.g.*, a mouse PCSK9 gene, a rat PCSK9 gene, a monkey PCSK9 gene, or a human PCSK9 gene) as well as variants (*e.g.*, naturally occurring variants) or mutants of the PCSK9 gene. Thus, the PCSK9 gene may be a wild-type PCSK9 gene, a mutant PCSK9 gene, or a transgenic PCSK9 gene in the context of genetically manipulated cells, cell populations, or organisms.

"Inhibiting PCSK9 gene expression" includes any level of inhibition of the PCSK9 gene, *e.g.*, at least partial inhibition of PCSK9 gene expression, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

PCSK9 gene expression may be assessed based on the level of any variable associated with PCSK9 gene expression, such as PCSK9 mRNA levels, PCSK9 protein levels, or serum lipid levels. Inhibition may be assessed by a reduction in the absolute or relative level of one or more of these variables compared to a control level. The control level may be any type of control level utilized in the art, *e.g.*, a pre-dose baseline level or a level determined from a similar subject, cell, or sample that is untreated or treated with a control (*e.g.*, a buffer-only control or an inert control).

As used herein, an "PCSK9-associated disease" is intended to include any disease associated with the PCSK9 gene or protein. The disease may be caused, for example, by overproduction of the PCSK9 protein, by mutations in the PCSK9 gene, by abnormal cleavage of the PCSK9 protein, or by abnormal interaction between PCSK9 and other proteins or other endogenous or exogenous substances. Exemplary PCSK9-associated diseases include lipid disorders, such as hyperlipidemia, and other forms of lipid imbalance, such as hypercholesterolemia and hypertriglyceridemia, as well as pathological conditions associated with these disorders, such as cardiovascular and circulatory diseases.

As used herein, the phrase "inhibiting HBV gene expression" includes inhibiting the expression of HBV DNA, HBV mRNA, HBsAg, HBeAg, hepatitis B core antigen (HBcAg), and the like.

"Inhibiting the expression of HBV antigens" includes inhibiting the expression of HBsAg, HBeAg, and HBcAg proteins.

"Inhibiting HBV gene expression" includes any level of inhibition of HBV DNA, HBV mRNA, HBsAg, HBeAg, or HBcAg, *e.g.*, at least partial inhibition of the expression of HBV DNA, HBV mRNA, HBsAg, HBeAg, or HBcAg, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

HBV gene expression may be assessed based on the level of any variable associated with HBV gene expression, such as HBV DNA levels, HBV mRNA levels, HBV antigen levels, or HBV particle levels. Inhibition may be assessed by a reduction in the absolute or relative level of one or more of these variables compared to a control level. The control level may be any type of control level utilized in the art, *e.g.*, a pre-dose baseline level or a level determined from a similar subject, cell, or sample that is untreated or treated with a control (*e.g.*, a buffer-only control or an inert control).

As used herein, a "disease associated with HBV gene expression" is intended to include any disease associated with the HBV gene or protein. The disease may be caused, for example, by overproduction of the HBV antigen, by mutations in the HBV gene, by abnormal cleavage of the HBV antigen, or by abnormal interaction between the HBV antigen and other proteins or other endogenous or exogenous substances. Exemplary HBV-associated diseases include hepatitis associated with HBV infection, such as chronic hepatitis B, acute hepatitis B, and diseases associated with co-infection of HBV and hepatitis D virus (HDV).

The phrase "inhibiting the expression of AGT" includes inhibition of expression of any AGT gene (*e.g.*, a mouse AGT gene, a rat AGT gene, a monkey AGT gene, or a human AGT gene) as well as variants (*e.g.*, naturally occurring variants) or mutants of the AGT gene. Thus, the AGT gene may be a wild-type AGT gene, a mutant AGT gene, or a transgenic AGT gene in the context of genetically manipulated cells, cell populations, or organisms.

"Inhibiting AGT gene expression" includes any level of inhibition of the AGT gene, *e.g.*, at least partial inhibition of AGT gene expression, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

AGT gene expression may be assessed based on the level of any variable associated with AGT gene expression, such as AGT mRNA levels or AGT protein levels. Inhibition may be assessed by a reduction in the absolute or relative level of one or more of these variables compared to a control level. The control level may be any type of control level utilized in the art, *e.g.*, a pre-dose baseline level or a level determined from a similar subject, cell, or sample that is untreated or treated with a control (*e.g.*, a buffer-only control or an inert control).

As used herein, an "AGT-associated disease" is intended to include any disease associated with the AGT gene or protein. The disease may be caused, for example, by overproduction of the AGT protein, by mutations in the AGT gene, by abnormal cleavage of the AGT protein, or by abnormal interaction between AGT and other proteins or other endogenous or exogenous substances. Exemplary AGT-associated diseases include hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction.

As used herein, a "patient" or "subject" is intended to include humans or non-human animals, preferably mammals, such as mice. Most preferably, the subject or patient is a human.

As used herein, a "therapeutically effective amount" is intended to include an amount of a double-stranded oligonucleotide that, when administered to a patient for treating a PCSK9/HBV/AGT-associated disease, is sufficient to treat the disease (*e.g.*, by attenuating, ameliorating, or maintaining the existing disease or one or more symptoms of the disease). The "therapeutically effective amount" may vary depending on the double-stranded oligonucleotide, how the agent is administered, the disease and its severity, medical history, age, weight, family history, genetic makeup, stage of pathological processes mediated by PCSK9/HBV/AGT gene expression, type of prior or concomitant therapy (if any), and other individual characteristics of the patient to be treated.

As used herein, a "prophylactically effective amount" is intended to include an amount of a double-stranded oligonucleotide that, when administered to a subject who has not yet experienced or exhibited symptoms of a PCSK9/HBV/AGT-associated disease, but who may be susceptible to the disease, is sufficient to prevent or ameliorate the disease or one or more symptoms of the disease. Amelioration of the disease includes slowing the progression of the disease or reducing the severity of later-onset disease. The "prophylactically effective amount" may vary depending on the double-stranded oligonucleotide, how the agent is administered, degree of risk of the disease, medical history, age, weight, family history, genetic makeup, type of prior or concomitant therapy (if any), and other individual characteristics of the patient to be treated.

The "therapeutically effective amount" or "prophylactically effective amount" also includes an amount of a double-stranded oligonucleotide that produces a desired local or systemic effect at a reasonable benefit/risk ratio applicable to any treatment. The double-stranded oligonucleotide used in the methods of the present disclosure may be administered in an amount sufficient to achieve a reasonable benefit/risk ratio applicable to such treatment.

As used herein, the term "sample" includes a collection of similar fluids, cells, or tissues isolated from a subject, as well as fluids, cells, or tissues present in the subject. Examples of biological fluids include blood, serum and serous fluid, plasma, cerebrospinal fluid, ocular fluid, lymph, urine, saliva, *etc*. Tissue samples may include samples from tissues, organs, or localized regions. For example, samples may be derived from specific organs, parts of organs, or fluids or cells within these organs. In certain examples, samples may be derived from the liver (*e.g.*, the entire liver or a portion thereof, or certain types of cells in the liver, such as hepatocytes). In a preferred example, a "sample derived from a subject" refers to blood or plasma drawn from the subject. In other examples, a "sample derived from a subject" refers to liver tissue (or subcomponents thereof) derived from the subject.

In one aspect, the present disclosure provides a modification template for enhancing the inhibitory effect of a double-stranded oligonucleotide on a target gene, wherein the double-stranded oligonucleotide is an siRNA comprising a sense strand and an antisense strand; each nucleotide of the sense strand and the antisense strand is a modified nucleotide; wherein the sense strand comprises nucleotide sequence 1, and the antisense strand comprises nucleotide sequence 2; the nucleotide sequence 1 and the nucleotide sequence 2 are at least partially reverse complementary to form a duplex region; the reverse complementary duplex region is 17 to 21 bp in length.

In some embodiments, the modified nucleotide is any one selected from the group consisting of a deoxy-nucleotide, a 3'-deoxy-thymidine nucleotide, a 2'-O-methyl-modified nucleotide, a 2'-fluoro-modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-O-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising a phosphorothioate group, a nucleotide comprising a methylphosphonate group, a nucleotide comprising a 5'-phosphate group, and a nucleotide comprising a 5'-phosphate mimic, or a combination of at least two thereof.

In some embodiments, the siRNA is 10, 11, 12, 13, 14, 15, 16, 17, 18, 19, 20, 21, 22, 23, 24, 25, 26, 27, 28, 29, or 30 nucleotides in length.

In some embodiments, the siRNA is 10 to 30 nucleotides in length.

In some embodiments, the siRNA is 13 to 25 nucleotides in length.

In some embodiments, the siRNA is 13 to 20 nucleotides in length.

In some embodiments, the modification contained in the nucleotide is a chemical modification at the 2' position of the ribose of the nucleotide.

In some embodiments, the chemical modification at the 2' position of the ribose of the nucleotide is any one selected from the group consisting of 2'-O-methyl modification, 2'-O-methoxyethyl modification, 2'-fluoro modification, 2'-benzyloxy modification, 2'-methylcarbonylamino modification, 2'-pyridylmethoxy modification, 2,4'-locked nucleic acid modification, and 2,4'-constrained ethyl nucleotide modification, or a combination of at least two thereof.

In some embodiments, the chemical modification at the 2' position of the ribose of the nucleotide is a 2,4'-locked nucleic acid modification.

In some embodiments, the chemical modification at the 2' position of the ribose of certain nucleotides of the siRNA is a 2,4'-constrained ethyl nucleotide modification.

In some embodiments, the nucleotides are linked via a 3',5'-phosphodiester bond.

In some embodiments, the 3',5'-phosphodiester bond comprises a phosphorothioate modification.

In some embodiments, the 3',5'-phosphodiester bond comprises a phosphorothioate modification, forming a stereochemically pure 3',5'-phosphorothioate bond.

In some embodiments, the siRNA comprises a duplex region, a 3' overhang, and/or a 5' overhang.

In some embodiments, the duplex region consists of 6, 7, 8, 9, 10, 11, 12, 13, 14, 15, or 16 consecutive nucleotides.

In some embodiments, from the 5' end to the 3' end, in the nucleotide sequence 2, at least two nucleotides among positions 2 to 6 are 2'-fluoro-modified nucleotides; the nucleotides at position 14 and/or 16 are 2'-fluoro-modified nucleotides; the nucleotides at the remaining positions are 2'-O-methyl-modified nucleotides; and positions 1 and 2 as well as positions of the second and third from last comprise phosphorothioate backbones.

In some embodiments of the present disclosure, at least two nucleotides among positions 2 to 6 of the nucleotide sequence 2 are selected from the following combinations:
(1) nucleotides at positions 2 and 6;
(2) nucleotides at positions 2, 3, 4, and 6;
(3) nucleotides at positions 2, 4, 5, and 6;
(4) nucleotides at positions 2, 5, and 6;
(5) nucleotides at positions 2, 3, 5, and 6;
(6) nucleotides at positions 2, 3, and 6.

In some embodiments of the present disclosure, from the 5' end to the 3' end, the nucleotide sequence 1 is modified as follows:
(1) the nucleotides at positions 6 and 16 are 2'-fluoro-modified nucleotides; at least two nucleotides among positions 8 to 10, preferably positions 8 to 10 or positions 8 and 10, are 2'-fluoro-modified nucleotides; the nucleotides at the remaining positions are 2'-O-methyl-modified nucleotides; and positions 1 and 2 comprise phosphorothioate backbones; or
(2) the nucleotides at positions 7 and 17 are 2'-fluoro-modified nucleotides; at least two nucleotides among positions 9 to 11, preferably positions 9 to 11 or positions 9 and 11, are 2'-fluoro-modified nucleotides; the nucleotides at the remaining positions are 2'-O-methyl-modified nucleotides; and positions 1 and 2 comprise phosphorothioate backbones.

In some embodiments of the present disclosure, the nucleotide sequence 1 is 21 or 20 nucleotides in length, and the nucleotide sequence 2 is 23 or 22 nucleotides in length.

In some embodiments of the present disclosure, the nucleotide sequence 1 is 20 nucleotides in length, and the nucleotide sequence 2 is 22 nucleotides in length; or, the nucleotide sequence 1 is 21 nucleotides in length, and the nucleotide sequence 2 is 23 nucleotides in length.

In some embodiments of the present disclosure, from the 5' end to the 3' end, the nucleotides in the antisense strand are modified according to any one of the following patterns A, B, C, D, E, F, and G:
A: indicates that, from the 5' end of the antisense strand, the nucleotides at positions 1, 3, 4, 5, 7, 8, 9, 10, 11, 12, 13, 15, 17, 18, 19, 20, 21, 22, and 23 are 2'-O-methyl-modified nucleotides; the nucleotides at positions 2, 6, 14, and 16 are 2'-fluoro-modified nucleotides; and positions 1, 2, 21, and 22 comprise phosphorothioate backbones;
B: indicates that, from the 5' end of the antisense strand, the nucleotides at positions 1, 5, 7, 8, 9, 10, 11, 12, 13, 15, 17, 18, 19, 20, 21, 22, and 23 are 2'-O-methyl-modified nucleotides; the nucleotides at positions 2, 3, 4, 6, 14, and 16 are 2'-fluoro-modified nucleotides; and positions 1, 2, 21, and 22 comprise phosphorothioate backbones;
C: indicates that, from the 5' end of the antisense strand, the nucleotides at positions 1, 3, 7, 8, 9, 10, 11, 12, 13, 15, 17, 18, 19, 20, 21, 22, and 23 are 2'-O-methyl-modified nucleotides; the nucleotides at positions 2, 4, 5, 6, 14, and 16 are 2'-fluoro-modified nucleotides; and positions 1, 2, 21, and 22 comprise phosphorothioate backbones;
D: indicates that, from the 5' end of the antisense strand, the nucleotides at positions 1, 3, 4, 7, 8, 9, 10, 11, 12, 13, 15, 17, 18, 19, 20, 21, 22, and 23 are 2'-O-methyl-modified nucleotides; the nucleotides at positions 2, 5, 6, 14, and 16 are 2'-fluoro-modified nucleotides; and positions 1, 2, 21, and 22 comprise phosphorothioate backbones;
E: indicates that, from the 5' end of the antisense strand, the nucleotides at positions 1, 4, 7, 8, 9, 10, 11, 12, 13, 15, 17, 18, 19, 20, 21, 22, and 23 are 2'-O-methyl-modified nucleotides; the nucleotides at positions 2, 3, 5, 6, 14, and 16 are 2'-fluoro-modified nucleotides; and positions 1, 2, 21, and 22 comprise phosphorothioate backbones;
F: indicates that, from the 5' end of the antisense strand, the nucleotides at positions 1, 4, 5, 7, 8, 9, 10, 11, 12, 13, 15, 17, 18, 19, 20, 21, 22, and 23 are 2'-O-methyl-modified nucleotides; the nucleotides at positions 2, 3, 6, 14, and 16 are 2'-fluoro-modified nucleotides; and positions 1, 2, 21, and 22 comprise phosphorothioate backbones;
G: indicates that, from the 5' end of the antisense strand, the nucleotides at positions 1, 3, 4, 5, 7, 8, 9, 10, 11, 12, 13, 15, 17, 18, 19, 20, 21, 22, and 23 are 2'-O-methyl-modified nucleotides; the nucleotides at positions 2, 6, 14, and 16 are 2'-fluoro-modified nucleotides; and positions 1, 2, 21, and 22 comprise phosphorothioate backbones;
H: indicates that, from the 5' end of the antisense strand, the nucleotides at positions 1, 3, 4, 5, 7, 8, 9, 10, 11, 12, 13, 15, 16, 17, 18, 19, 20, 21, 22, and 23 are 2'-O-methyl-modified nucleotides; the nucleotides at positions 2, 6, and 14 are 2'-fluoro-modified nucleotides; and positions 1, 2, 21, and 22 comprise phosphorothioate backbones;
and/or, from the 5' end to the 3' end, the nucleotides in the sense strand are modified according to any one of the following patterns a to d:
a: indicates that, from the 5' end of the sense strand, the nucleotides at positions 1, 2, 3, 4, 5, 6, 8, 12, 13, 14, 15, 16, 18, 19, 20, and 21 are 2'-O-methyl-modified nucleotides; the nucleotides at positions 7, 9, 10, 11, and 17 are 2'-fluoro-modified nucleotides; and positions 1 and 2 comprise phosphorothioate backbones;
b: indicates that, from the 5' end of the sense strand, the nucleotides at positions 1, 2, 3, 4, 5, 6, 8, 10, 12, 13, 14, 15, 16, 18, 19, 20, and 21 are 2'-O-methyl-modified nucleotides; the nucleotides at positions 7, 9, 11, and 17 are 2'-fluoro-modified nucleotides; and positions 1 and 2 comprise phosphorothioate backbones;
c: indicates that, from the 5' end of the sense strand, the nucleotides at positions 1, 2, 3, 4, 5, 6, 8, 12, 13, 14, 15, 16, 17, 18, 19, 20, and 21 are 2'-O-methyl-modified nucleotides; the nucleotides at positions 7, 9, 10, and 11 are 2'-fluoro-modified nucleotides; and positions 1 and 2 comprise phosphorothioate backbones;
d: indicates that, from the 5' end of the sense strand, the nucleotides at positions 1, 2, 3, 4, 5, 6, 8, 11, 12, 13, 14, 15, 16, 17, 18, 19, 20, and 21 are 2'-O-methyl-modified nucleotides; the nucleotides at positions 7, 9, and 10 are 2'-fluoro-modified nucleotides; and positions 1 and 2 comprise phosphorothioate backbones.

In some embodiments of the present disclosure, from the 5' end to the 3' end, the nucleotides in the antisense strand are modified according to any one of the following patterns A', B', C', D', E', F', and G':
A': indicates that, from the 5' end of the antisense strand, the nucleotides at positions 1, 3, 4, 5, 7, 8, 9, 10, 11, 12, 13, 15, 17, 18, 19, 20, 21, and 22 are 2'-O-methyl-modified nucleotides; the nucleotides at positions 2, 6, 14, and 16 are 2'-fluoro-modified nucleotides; and positions 1, 2, 20, and 21 comprise phosphorothioate backbones;
B': indicates that, from the 5' end of the antisense strand, the nucleotides at positions 1, 5, 7, 8, 9, 10, 11, 12, 13, 15, 17, 18, 19, 20, 21, and 22 are 2'-O-methyl-modified nucleotides; the nucleotides at positions 2, 3, 4, 6, 14, and 16 are 2'-fluoro-modified nucleotides; and positions 1, 2, 20, and 21 comprise phosphorothioate backbones;
C': indicates that, from the 5' end of the antisense strand, the nucleotides at positions 1, 3, 7, 8, 9, 10, 11, 12, 13, 15, 17, 18, 19, 20, 21, and 22 are 2'-O-methyl-modified nucleotides; the nucleotides at positions 2, 4, 5, 6, 14, and 16 are 2'-fluoro-modified nucleotides; and positions 1, 2, 20, and 21 comprise phosphorothioate backbones;
D': indicates that, from the 5' end of the antisense strand, the nucleotides at positions 1, 3, 4, 7, 8, 9, 10, 11, 12, 13, 15, 17, 18, 19, 20, 21, and 22 are 2'-O-methyl-modified nucleotides; the nucleotides at positions 2, 5, 6, 14, and 16 are 2'-fluoro-modified nucleotides; and positions 1, 2, 20, and 21 comprise phosphorothioate backbones;
E': indicates that, from the 5' end of the antisense strand, the nucleotides at positions 1, 4, 7, 8, 9, 10, 11, 12, 13, 15, 17, 18, 19, 20, 21, and 22 are 2'-O-methyl-modified nucleotides; the nucleotides at positions 2, 3, 5, 6, 14, and 16 are 2'-fluoro-modified nucleotides; and positions 1, 2, 20, and 21 comprise phosphorothioate backbones;
F': indicates that, from the 5' end of the antisense strand, the nucleotides at positions 1, 4, 5, 7, 8, 9, 10, 11, 12, 13, 15, 17, 18, 19, 20, 21, and 22 are 2'-O-methyl-modified nucleotides; the nucleotides at positions 2, 3, 6, 14, and 16 are 2'-fluoro-modified nucleotides; and positions 1, 2, 20, and 21 comprise phosphorothioate backbones;
G': indicates that, from the 5' end of the antisense strand, the nucleotides at positions 1, 3, 4, 5, 7, 8, 9, 10, 11, 12, 13, 15, 16, 17, 18, 19, 20, 21, and 22 are 2'-O-methyl-modified nucleotides; the nucleotides at positions 2, 6, and 14 are 2'-fluoro-modified nucleotides; and positions 1, 2, 20, and 21 comprise phosphorothioate backbones;
and/or, from the 5' end to the 3' end, the nucleotides in the sense strand are modified according to any one of the following patterns a', b', c', and d':
   a': indicates that, from the 5' end of the sense strand, the nucleotides at positions 1, 2, 3, 4, 5, 7, 11, 12, 13, 14, 15, 17, 18, 19, and 20 are 2'-O-methyl-modified nucleotides; the nucleotides at positions 6, 8, 9, 10, and 16 are 2'-fluoro-modified nucleotides; and positions 1 and 2 comprise phosphorothioate backbones;
   b': indicates that, from the 5' end of the sense strand, the nucleotides at positions 1, 2, 3, 4, 5, 7, 9, 11, 12, 13, 14, 15, 17, 18, 19, and 20 are 2'-O-methyl-modified nucleotides; the nucleotides at positions 6, 8, 10, and 16 are 2'-fluoro-modified nucleotides; and positions 1 and 2 comprise phosphorothioate backbones;
   c': indicates that, from the 5' end of the sense strand, the nucleotides at positions 1, 2, 3, 4, 5, 7, 11, 12, 13, 14, 15, 16, 17, 18, 19, and 20 are 2'-O-methyl-modified nucleotides; the nucleotides at positions 6, 8, 9, and 10 are 2'-fluoro-modified nucleotides; and positions 1 and 2 comprise phosphorothioate backbones;
   d': indicates that, from the 5' end of the sense strand, the nucleotides at positions 1, 2, 3, 4, 5, 7, 10, 11, 12, 13, 14, 15, 16, 17, 18, 19, and 20 are 2'-O-methyl-modified nucleotides; the nucleotides at positions 6, 8, and 9 are 2'-fluoro-modified nucleotides; and positions 1 and 2 comprise phosphorothioate backbones.

In some embodiments of the present disclosure, from the 5' end to the 3' end, the double-stranded oligonucleotide is modified according to any one of the following combinations (i) to (x):
(i) the antisense strand is modified according to pattern A, and the sense strand is modified according to pattern a;
(ii) the antisense strand is modified according to pattern B, and the sense strand is modified according to pattern a;
(iii) the antisense strand is modified according to pattern C, and the sense strand is modified according to pattern a;
(iv) the antisense strand is modified according to pattern B, and the sense strand is modified according to pattern b;
(v) the antisense strand is modified according to pattern C, and the sense strand is modified according to pattern b;
(vi) the antisense strand is modified according to pattern G, and the sense strand is modified according to pattern c;
(vii) the antisense strand is modified according to pattern A, and the sense strand is modified according to pattern d;
(viii) the antisense strand is modified according to pattern D, and the sense strand is modified according to pattern b;
(ix) the antisense strand is modified according to pattern E, and the sense strand is modified according to pattern b;
(x) the antisense strand is modified according to pattern F, and the sense strand is modified according to pattern b.

In some embodiments of the present disclosure, from the 5' end to the 3' end, the double-stranded oligonucleotide is modified according to any one of the following combinations (1) to (8):
(1) the antisense strand is modified according to pattern A', and the sense strand is modified according to pattern a';
(2) the antisense strand is modified according to pattern B', and the sense strand is modified according to pattern a';
(3) the antisense strand is modified according to pattern C', and the sense strand is modified according to pattern a';
(4) the antisense strand is modified according to pattern B', and the sense strand is modified according to pattern b';
(5) the antisense strand is modified according to pattern C', and the sense strand is modified according to pattern b';
(6) the antisense strand is modified according to pattern D', and the sense strand is modified according to pattern b';
(7) the antisense strand is modified according to pattern E', and the sense strand is modified according to pattern b';
(8) the antisense strand is modified according to pattern F', and the sense strand is modified according to pattern b'.

In some embodiments of the present disclosure, the nucleotide at position 1 from the 5' end of the antisense strand is a 5'-phosphorylated nucleotide or a nucleotide modified with a 5'-phosphate analog, including, but not limited to, the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are as follows: 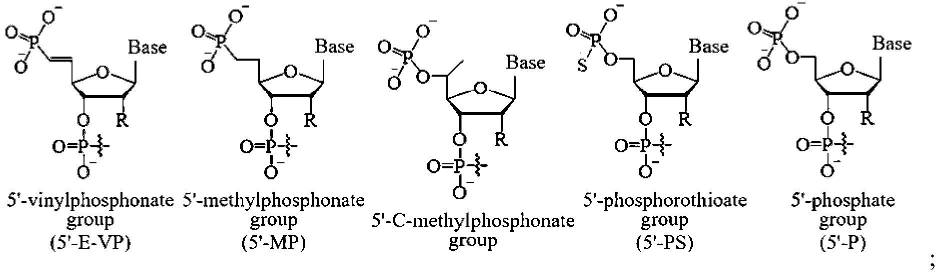
wherein R is hydrogen, hydroxyl, amino, C₁-₄ alkyl, aryl, C₁-₄ alkoxy, C₁-₄ alkylcarbonylamino, or halogen;
the base is selected from adenine, guanine, cytosine, thymine, and uracil.

In some embodiments of the present disclosure, the nucleotide at position 1 from the 5' end of the antisense strand is a 5'-vinyl-(E)-phosphonate-2'-O-methyl-modified nucleotide.

In some embodiments of the present disclosure, the antisense strand adopts one of the following modification patterns:

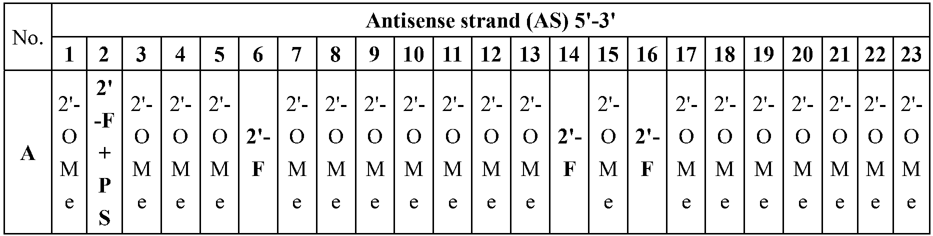

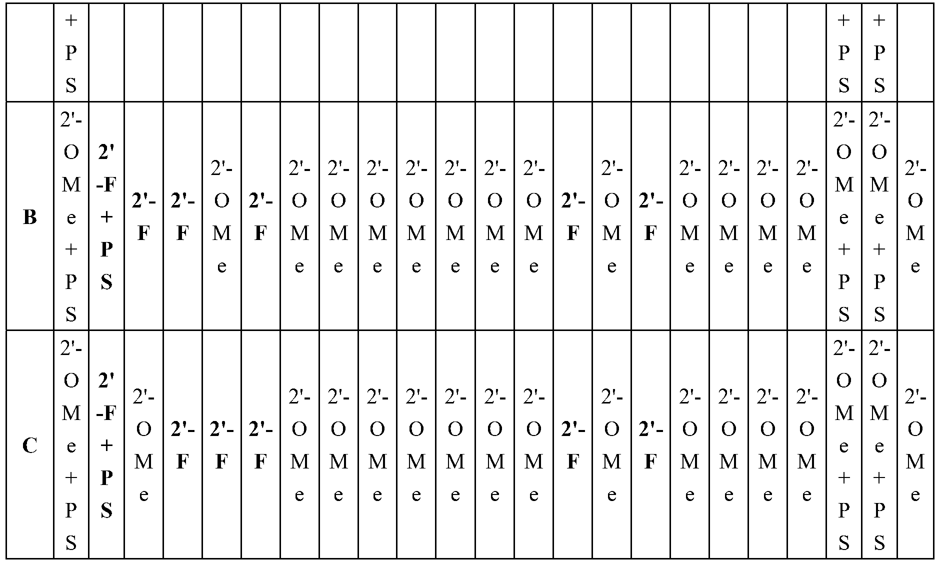

and the sense strand adopts one of the following modification patterns:

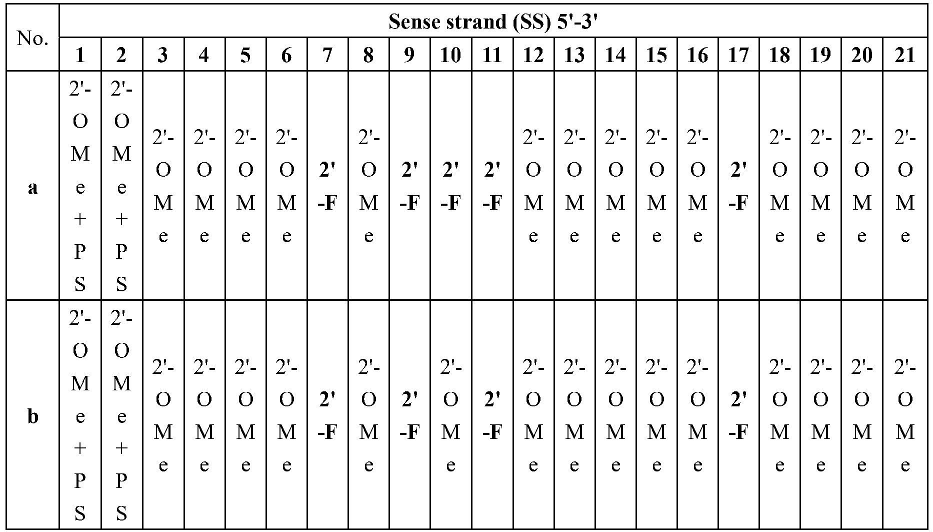

In the above tables, PS represents a phosphorothioate backbone.

The siRNA modification template, in which the antisense strand is modified according to modification pattern A and the sense strand is modified according to modification pattern a, is designated as DV25.

The siRNA modification template, in which the antisense strand is modified according to modification pattern B and the sense strand is modified according to modification pattern a, is designated as DV26.

The siRNA modification template, in which the antisense strand is modified according to modification pattern C and the sense strand is modified according to modification pattern a, is designated as DV27.

The siRNA modification template, in which the antisense strand is modified according to modification pattern B and the sense strand is modified according to modification pattern b, is designated as DV28.

The siRNA modification template, in which the antisense strand is modified according to modification pattern C and the sense strand is modified according to modification pattern b, is designated as DV29.

In some embodiments of the present disclosure, the antisense strand adopts the following modification template:

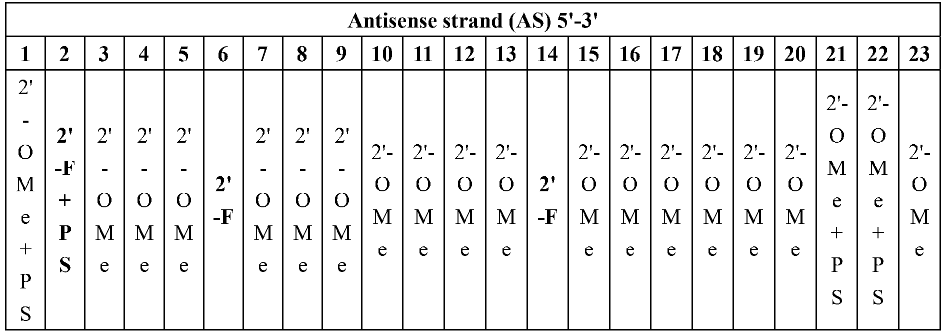

and the sense strand adopts the following modification template:

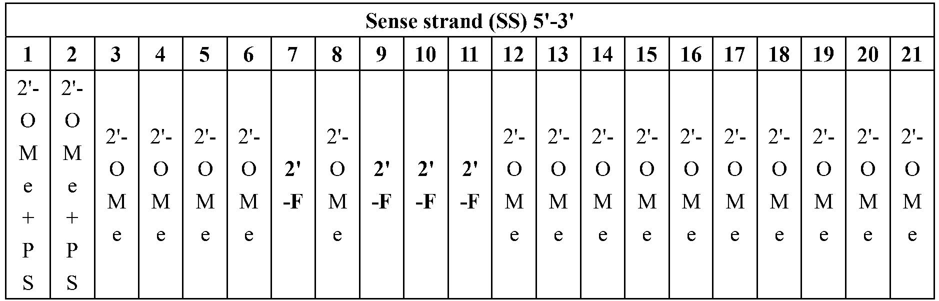

In the above tables, PS represents a phosphorothioate backbone.

In some embodiments of the present disclosure, the antisense strand adopts the following modification template:

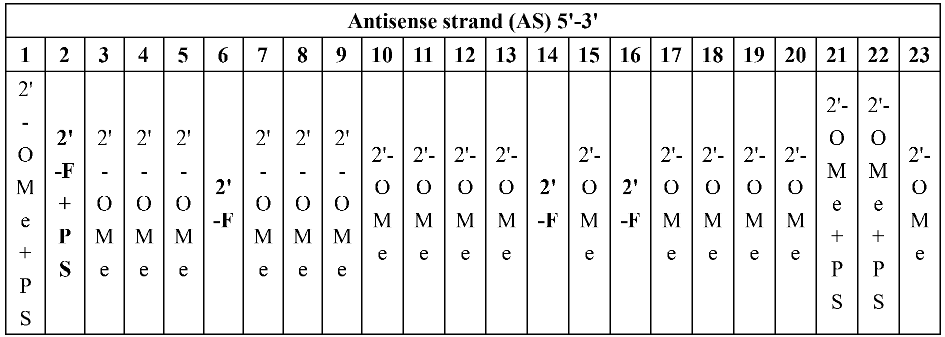

and the sense strand adopts the following modification template:

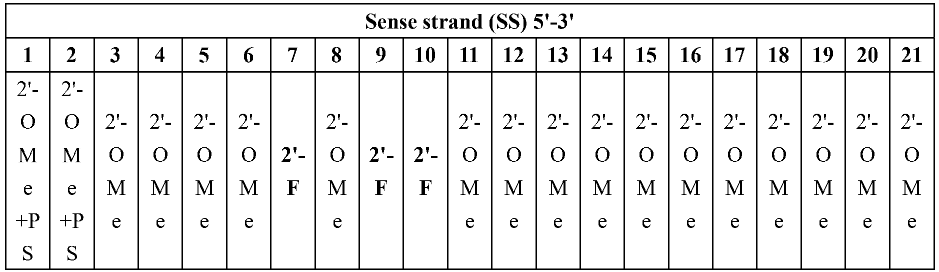

In the above tables, PS represents a phosphorothioate backbone.

In some embodiments of the present disclosure, the nucleotide at position 1 from the 5' end of the antisense strand is a 5'-phosphorylated nucleotide or a nucleotide modified with a 5'-phosphate analog, including, but not limited to, the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are as follows: 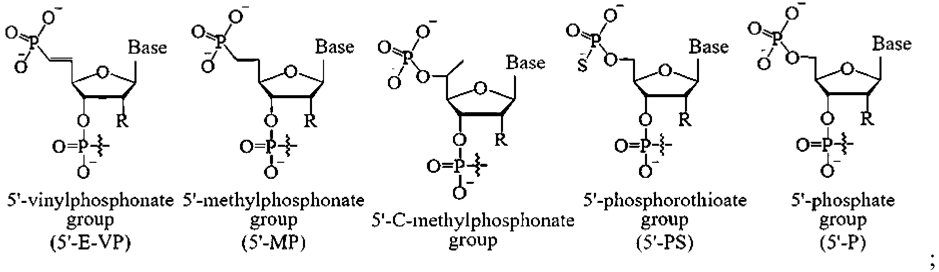
wherein R is hydrogen, hydroxyl, amino, C₁-₄ alkyl, aryl, C₁-₄ alkoxy, C₁-₄ alkylcarbonylamino, or halogen;
the base is selected from adenine, guanine, cytosine, thymine, and uracil.

In some embodiments of the present disclosure, the nucleotide at position 1 from the 5' end of the antisense strand is a 5'-vinyl-(E)-phosphonate-2'-O-methyl-modified nucleotide.

In some embodiments of the present disclosure, the antisense strand adopts one of the following modification patterns:

| No. | **Antisense strand (AS) 5'-3'** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **G** | **1** | **2** | **3** | **4** | **5** | **6** | **7** | **8** | **9** | **10** | **11** | **12** |
| | 2'-OMe +PS | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |

and the sense strand adopts one of the following modification patterns:

| No. | Sense strand (SS) 5'-3' | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | 2'-F | 2'-OMe | 2'-F | 2'-F | 2'-F |
| | **12** | **13** | **14** | **15** | **16** | **17** | **18** | **19** | **20** | **21** | |
| | **2'-OMe** | **2'-OMe** | **2'-OMe** | **2'-OMe** | **2'-OMe** | **2'-F** | **2'-OMe** | **2'-OMe** | **2'-OMe** | **2'-OMe** | |
| **b** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **c** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-OMe | 2'-OMe | 2'-OMe | 2'-OMe | 2'-OMe | |

In the above tables, PS represents a phosphorothioate backbone.

The siRNA modification template, in which the antisense strand is modified according to modification pattern A and the sense strand is modified according to modification pattern a, is designated as DV25P.

The siRNA modification template, in which the antisense strand is modified according to modification pattern B and the sense strand is modified according to modification pattern a, is designated as DV26P.

The siRNA modification template, in which the antisense strand is modified according to modification pattern C and the sense strand is modified according to modification pattern a, is designated as DV27P.

The siRNA modification template, in which the antisense strand is modified according to modification pattern B and the sense strand is modified according to modification pattern b, is designated as DV28P.

The siRNA modification template, in which the antisense strand is modified according to modification pattern C and the sense strand is modified according to modification pattern b, is designated as DV29P.

The siRNA modification template, in which the antisense strand is modified according to modification pattern D and the sense strand is modified according to modification pattern b, is designated as DV32P.

The siRNA modification template, in which the antisense strand is modified according to modification pattern E and the sense strand is modified according to modification pattern b, is designated as DV33P.

The siRNA modification template, in which the antisense strand is modified according to modification pattern F and the sense strand is modified according to modification pattern b, is designated as DV34P.

In some embodiments of the present disclosure, the antisense strand adopts one of the following modification patterns:

| No. | **Antisense strand (AS) 5'-3'** | | | | | | | | | | |
|---|---|---|---|---|---|---|---|---|---|---|---|
| **A**' | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **B**' | **1** | **2** | **3** | **4** | **5** | **6** | **7** | **8** | **9** | **10** | **11** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C**' | **1** | **2** | **3** | **4** | **5** | **6** | **7** | **8** | **9** | **10** | **11** |
| | 2'-OMe | **2'-F** | 2'-OMe | **2'-F** | **2'-F** | **2'-F** | 2'-OMe | 2'-OMe | 2'-OMe | 2'-OMe | 2'-OMe |
| | +PS +EVP | **+PS** | | | | | | | | | |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **E'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

and the sense strand adopts one of the following modification patterns:

| No. | **Sense strand (SS) 5'-3'** | | | | | | | | | |
|---|---|---|---|---|---|---|---|---|---|---|
| **a'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **c'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-OMe | 2'-OMe | 2'-OMe | 2'-OMe | 2'-OMe |

In the above tables, 2'-OMe represents a 2'-O-methyl modification; 2'-F represents a 2'-fluoro modification; PS represents a phosphorothioate backbone.

The siRNA modification template, in which the antisense strand is modified according to modification pattern A' and the sense strand is modified according to modification pattern a', is designated as DV25SP.

The siRNA modification template, in which the antisense strand is modified according to modification pattern B' and the sense strand is modified according to modification pattern a', is designated as DV26SP.

The siRNA modification template, in which the antisense strand is modified according to modification pattern C' and the sense strand is modified according to modification pattern a', is designated as DV27SP.

The siRNA modification template, in which the antisense strand is modified according to modification pattern B' and the sense strand is modified according to modification pattern b', is designated as DV28SP.

The siRNA modification template, in which the antisense strand is modified according to modification pattern C' and the sense strand is modified according to modification pattern b', is designated as DV29SP.

The siRNA modification template, in which the antisense strand is modified according to modification pattern D' and the sense strand is modified according to modification pattern b', is designated as DV32SP.

The siRNA modification template, in which the antisense strand is modified according to modification pattern E' and the sense strand is modified according to modification pattern b', is designated as DV33SP.

The siRNA modification template, in which the antisense strand is modified according to modification pattern F' and the sense strand is modified according to modification pattern b', is designated as DV34SP.

In some embodiments of the present disclosure, one or more nucleotides at positions 2 to 8 from the 5' end of the antisense strand, preferably at position 6 and/or 7, comprises a modification group, wherein the modification group is selected from UNA, GNA, and DNA, wherein the structures of UNA and GNA are as follows: 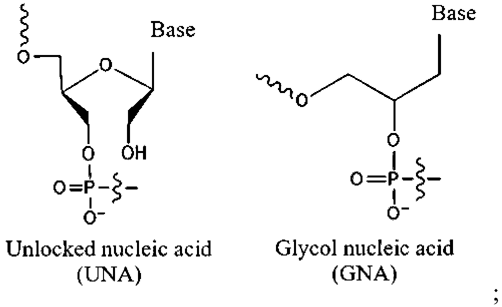 wherein the base is selected from adenine, guanine, cytosine, thymine, and uracil.

In some embodiments of the present disclosure, position 6 and/or 7 from the 5' end of the antisense strand comprises a modification group, wherein the modification group is DNA.

In another aspect, the present disclosure provides a double-stranded oligonucleotide modified using the modification template described herein.

In some embodiments of the present disclosure, the double-stranded oligonucleotide targets the mRNA of one of the following genes: PCSK9 gene, HBV gene, and AGT gene.

In some specific embodiments of the present disclosure, the nucleotide sequences of the double-stranded oligonucleotide are shown in Tables 13, 47, 49, 63-5, 79, and 85.

In another aspect, the present disclosure provides a conjugate comprising the double-stranded oligonucleotide described above, and a ligand conjugated to the double-stranded oligonucleotide.

In some embodiments, the ligand is conjugated to the 3' end or 5' end of the sense strand of the oligonucleotide.

In some embodiments, the ligand is one or more GalNAc derivatives attached via a bivalent or trivalent branched linker.

In some embodiments, the ligand is as follows: 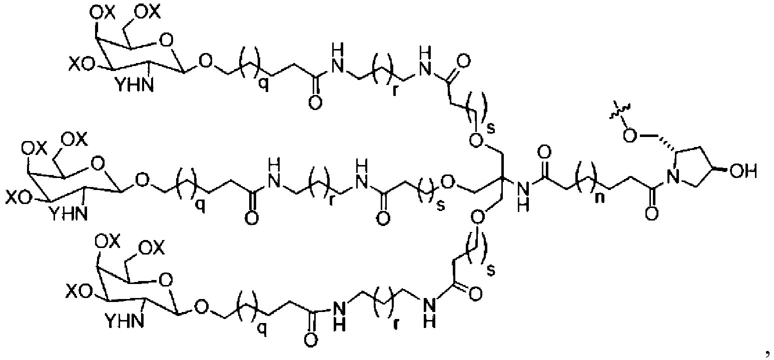 wherein X is hydrogen, a hydroxyl protecting group, or H, the hydroxyl protecting group including acetyl, benzoyl, or isobutyryl; Y is an amino protecting group or H, the amino protecting group being formyl, acetyl, propionyl, *n*-butyryl, or isobutyryl; n is an integer from 0 to 20; q, r, and s are independently integers from 1 to 7.

In some embodiments, the ligand is as follows:

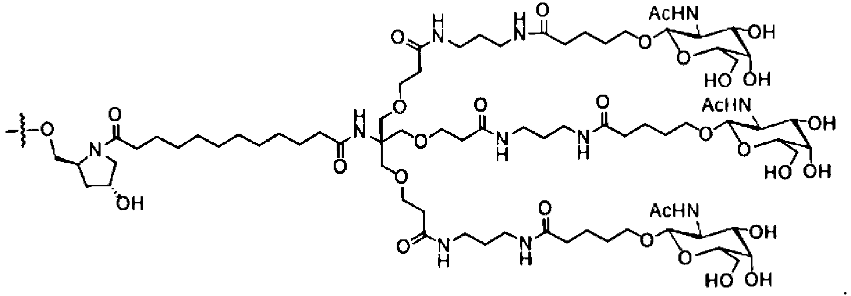

In some embodiments, the ligand is as follows: 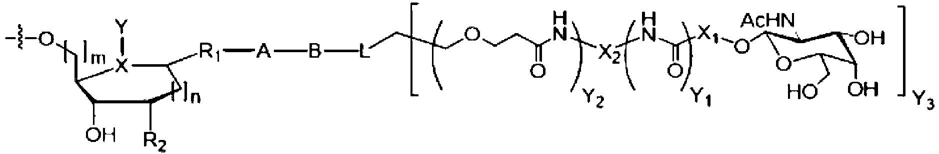
wherein X is oxygen, nitrogen, or sulfur;
Y is alkyl or aryl;
R₁ is oxygen or sulfur;
R₂ is hydrogen, amino, C₁₋₄ alkyl, aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
Y₁ is 0 or 1;
Y₂ is 0, 1, or 2;
Y₃ is 1, 2, or 3;
m is an integer from 0 to 4;
n is an integer from 0 to 4.

In some embodiments, the ligand is G4, G5, G6, G7, G101, G102, G103, G105, or G106:

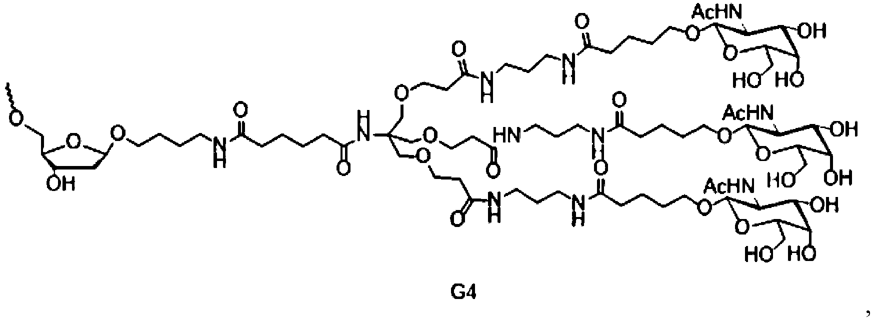

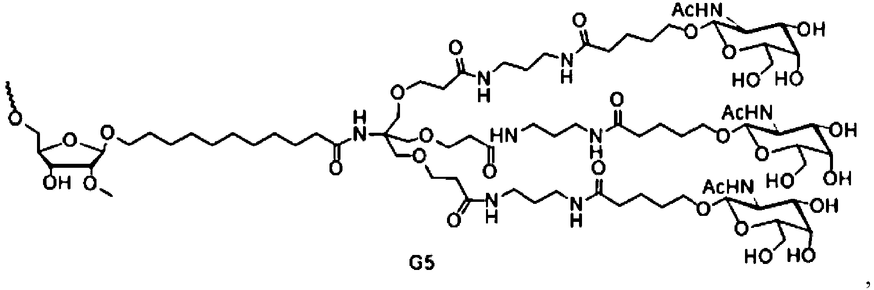

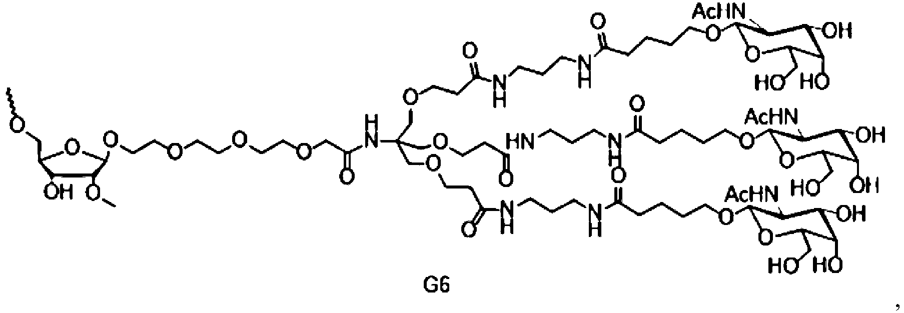

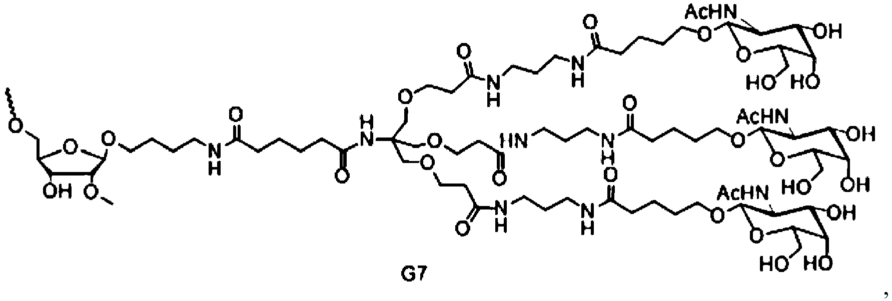

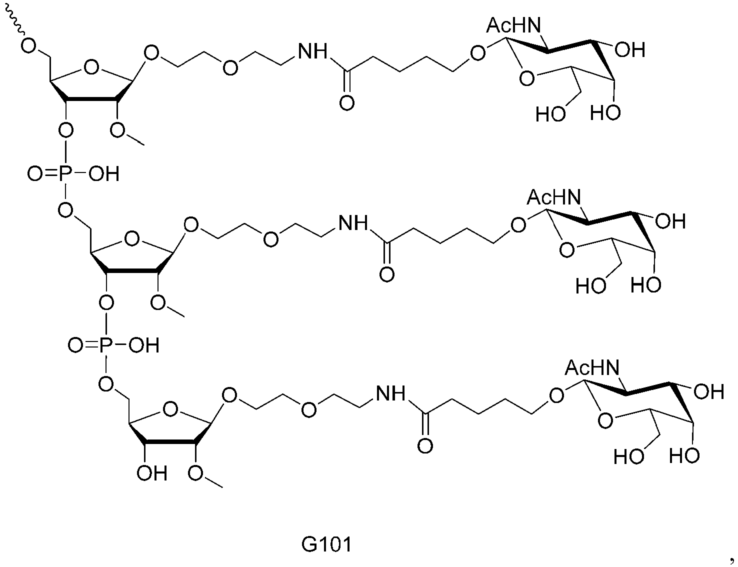

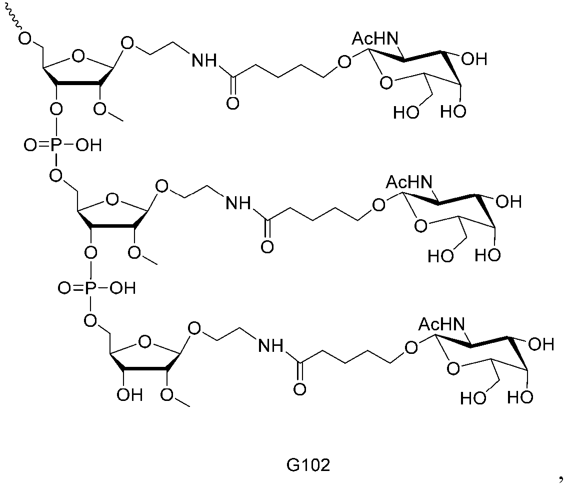

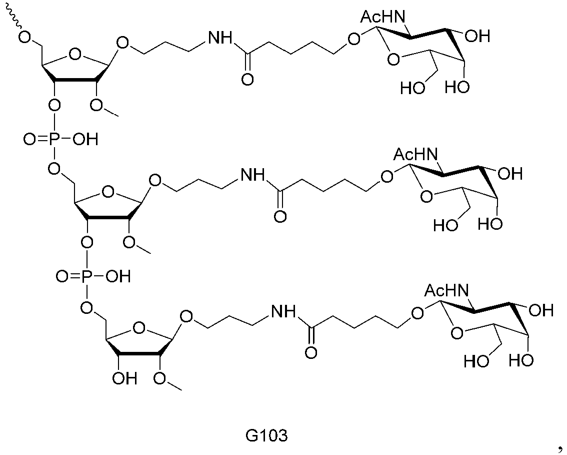

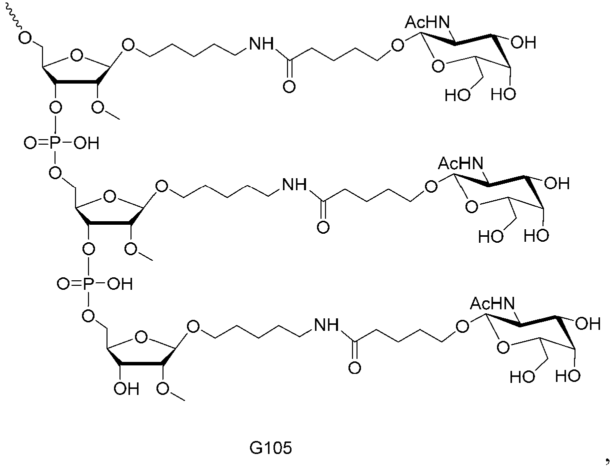

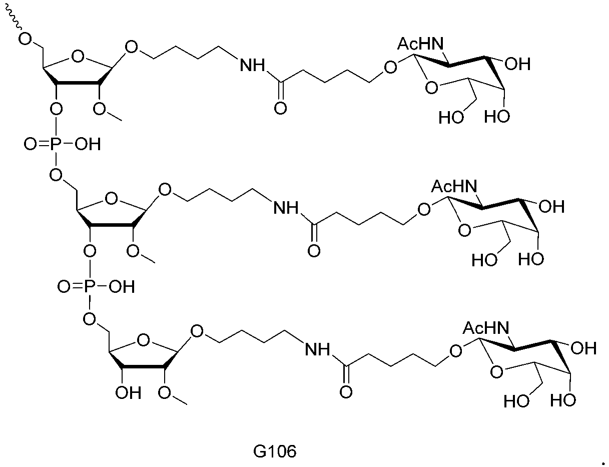

In some embodiments, the conjugate has a structure as shown below: 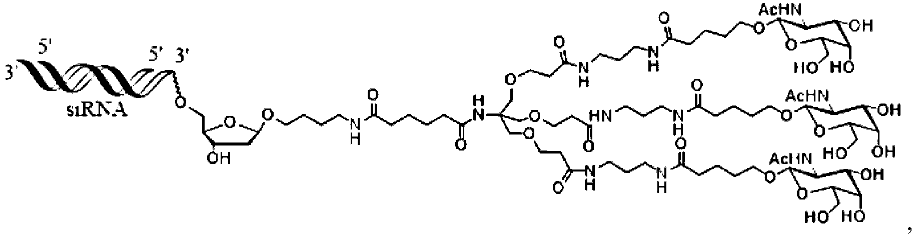 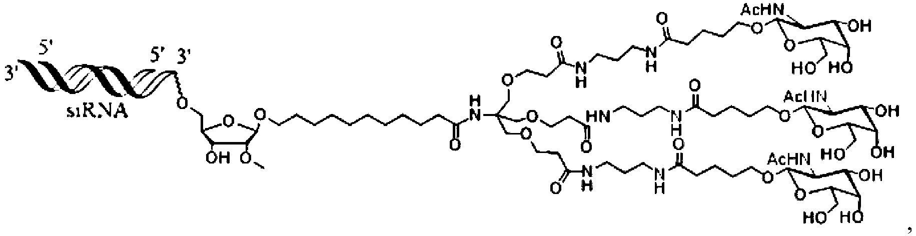 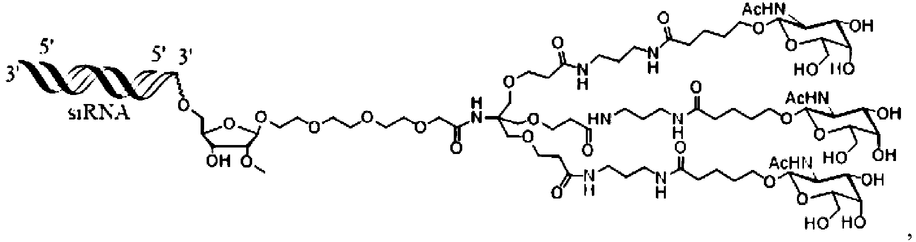 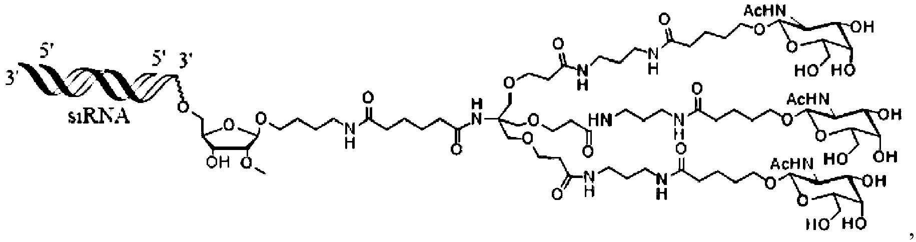 or

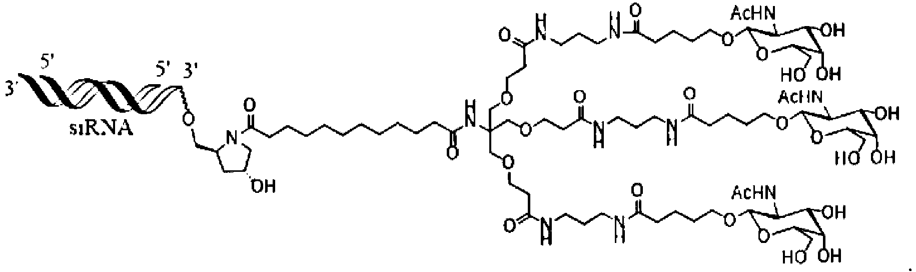

In other embodiments, the conjugate has a structure as shown below:

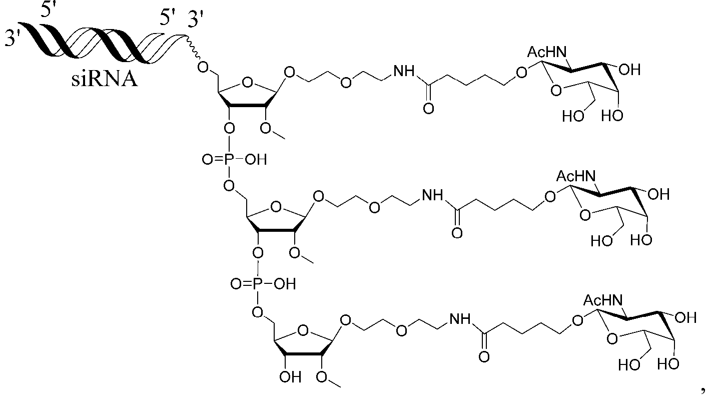

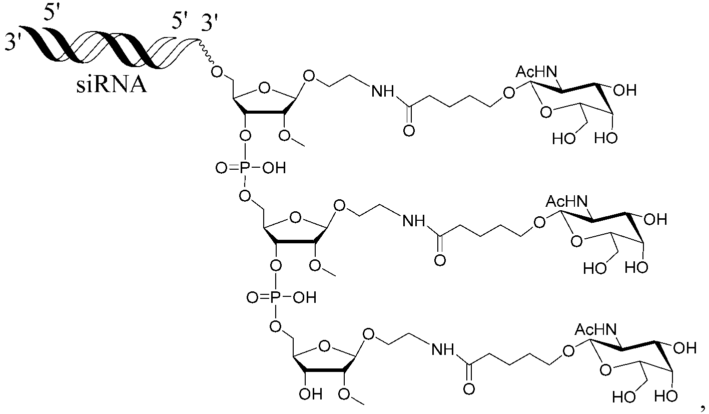

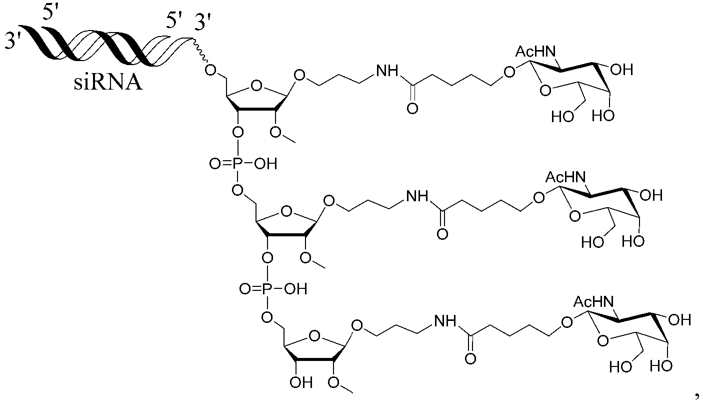

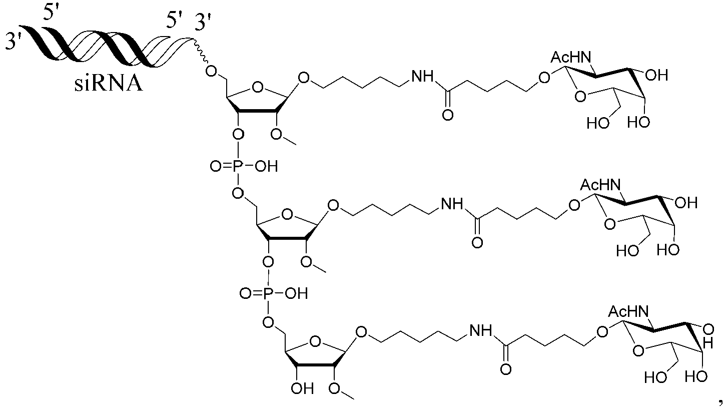

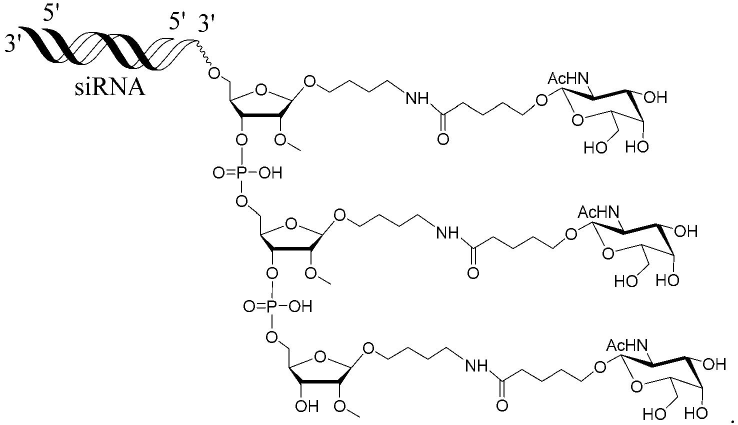

In some embodiments, the conjugate is used for inhibiting PCSK9, HBV, or AGT gene expression.

In another aspect, the present disclosure provides a pharmaceutical composition comprising the double-stranded oligonucleotide described above or the conjugate described above, and a pharmaceutically acceptable carrier.

In one example, provided herein is a pharmaceutical composition comprising the double-stranded oligonucleotide described herein and a pharmaceutically acceptable carrier. The pharmaceutical composition comprising the double-stranded oligonucleotide may be used for treating a disease or disorder associated with the expression or activity of PCSK9, HBV, or AGT genes, such as lipid disorders, hypercholesterolemia, chronic hepatitis B, or hypertension. Such pharmaceutical compositions are formulated based on the mode of delivery. One example is a composition formulated for systemic administration via parenteral delivery, *e.g.*, via intravenous (i.v.) or subcutaneous (s.c.) injection. Another example is a composition formulated for direct delivery into the brain parenchyma, *e.g.*, via infusion into the brain, such as by continuous pump infusion.

The pharmaceutical composition comprising the double-stranded oligonucleotide disclosed herein may be, for example, a solution with or without a buffer, or a composition comprising a pharmaceutically acceptable carrier. Such compositions include, for example, aqueous or crystalline compositions, liposomal formulations, micellar formulations, emulsions, and gene therapy vectors.

In the methods of the present disclosure, the double-stranded oligonucleotide may be administered in a solution. A free double-stranded oligonucleotide may be administered in a non-buffered solution, *e.g*.*,* in saline or in water. Alternatively, the free double-stranded oligonucleotide may also be administered in a suitable buffered solution. The buffered solution may comprise acetate, citrate, prolamin, carbonate, or phosphate, or any combination thereof. In a preferred example, the buffered solution is phosphate-buffered saline (PBS). The pH and osmolarity of the buffered solution comprising the double-stranded oligonucleotide may be adjusted such that it is suitable for administration to a subject.

In some examples, the buffered solution further comprises an agent for controlling the osmolarity of the solution, such that the osmolarity is maintained at a desired value, *e.g.*, at the physiological value of human plasma. Solutes that may be added to the buffered solution to control the osmolarity include, but are not limited to, proteins, peptides, amino acids, non-metabolized polymers, vitamins, ions, sugars, metabolites, organic acids, lipids, or salts. In some examples, the agent for controlling the osmolarity of the solution is a salt. In certain examples, the agent for controlling the osmolarity of the solution is sodium chloride or potassium chloride.

The pharmaceutical composition of the present disclosure may be administered at a dose sufficient to inhibit PCSK9, HBV, or AGT gene expression. Generally, suitable doses of the double-stranded oligonucleotide disclosed herein range from about 0.001 to about 200.0 mg per kilogram body weight per day, typically ranging from about 0.1 to 50 mg per kilogram body weight per day. For example, the double-stranded oligonucleotide (*e.g.*, siRNA) may be administered at a single dose of about 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 31, 32, 33, 34, 34, 35, 36, 37, 38, 39, 40, 41, 42, 43, 44, 45, 46, 47, 48, 49, or about 50 mg/kg.

The pharmaceutical composition may be administered once daily, or the double-stranded oligonucleotide may be administered as two, three, or more sub-doses at appropriate intervals within a day, or even using continuous infusion or delivery via a controlled-release formulation. In such cases, the amount of double-stranded oligonucleotide in each sub-dose must be correspondingly reduced in order to achieve the total daily dose. Dosage units may also be compounded for delivery over several days, *e.g.*, using a conventional sustained-release formulation that provides sustained release of the double-stranded oligonucleotide over a period of several days. Sustained-release formulations are well known in the art and are particularly useful for delivering agents to specific sites, and thus may be used with the agents of the present disclosure. In such examples, the dosage unit contains a corresponding plurality of daily doses.

In other examples, a single dose of the pharmaceutical composition may be long-acting, such that subsequent doses are administered at intervals of no more than 3, 4, or 5 days, or no more than 1, 2, 3, or 4 weeks. In some examples of the present disclosure, a single dose of the pharmaceutical composition of the present disclosure is administered once weekly. In other examples of the present disclosure, a single dose of the pharmaceutical composition of the present disclosure is administered once monthly.

Those skilled in the art will appreciate that certain factors may influence the dosage and dosing schedule required for effective treatment of a subject, including, but not limited to, the severity of the disease or disorder, prior treatments, the general health and/or age of the subject, and other existing diseases. Furthermore, treating a subject with a therapeutically effective amount of the composition may involve a single treatment or a series of treatments. As described elsewhere herein, the effective dose and *in vivo* half-life for each double-stranded oligonucleotide encompassed by the present disclosure may be estimated using conventional methods or on the basis of *in vivo* testing using suitable animal models.

The pharmaceutical composition of the present disclosure may be administered in various ways depending upon whether local or systemic treatment is desired and upon the area to be treated. Administration may be topical (*e.g.*, via a transdermal patch); pulmonary; *e.g.*, via inhalation or insufflation of a powder or aerosol, including via a nebulizer; intratracheal; intranasal; epidermal and transdermal; oral; or parenteral. Parenteral administration includes intravenous, intra-arterial, subcutaneous, intraperitoneal, or intramuscular injection or infusion; subdermal administration, *e.g.*, via an implantable device; or intracranial administration, *e.g.*, intraparenchymal, intrathecal, or intraventricular administration.

The double-stranded oligonucleotide for use in the compositions and methods of the present disclosure may be formulated for delivery in membranous molecular assemblies, such as liposomes or micelles. As used herein, the term "liposome" refers to a vesicle composed of amphipathic lipids arranged in at least one bilayer (*e.g.*, a single bilayer or multiple bilayers). Liposomes include unilamellar or multilamellar vesicles having a membrane formed from a lipophilic material and an internal aqueous compartment. The aqueous portion contains the double-stranded oligonucleotide composition. The lipophilic material separates the internal aqueous compartment from the external aqueous environment, which typically does not contain the double-stranded oligonucleotide composition (although in some instances it may). Liposomes are useful for transferring and delivering active ingredients to sites of action. Because liposomal membranes are structurally similar to biological membranes, when liposomes are applied to a tissue, the liposomal bilayer fuses with the bilayer of the cell membrane. Upon fusion of the liposomes with cells, the internal aqueous contents, including the double-stranded oligonucleotide that can specifically bind to a target RNA and mediate RNAi, are delivered into the cells. In some cases, these liposomes are also specifically targeted, for example, to direct the double-stranded oligonucleotide to particular cell types.

Liposomes containing the double-stranded oligonucleotide may be prepared by various methods. In one example, a lipid component of the liposome is dissolved in a detergent such that micelles are formed with the lipid component. For example, the lipid component may be an amphipathic cationic lipid or a lipid conjugate. The detergent may have a high critical micelle concentration and may be nonionic. Exemplary detergents include cholate, CHAPS, octyl glucoside, deoxycholate, and lauroyl sarcosinate. The double-stranded oligonucleotide formulation is then added to micelles containing the lipid component. The cationic groups on the lipid interact with the double-stranded oligonucleotide and condense around the double-stranded oligonucleotide to form liposomes. After condensation, the detergent is removed, *e.g.*, by dialysis, to obtain a liposomal formulation of the double-stranded oligonucleotide.

The double-stranded oligonucleotide, such as the siRNA disclosed herein, may be fully encapsulated in a lipid formulation (*e.g.*, a lipid nanoparticle (LNP) or another nucleic acid-lipid particle).

As used herein, the term "lipid nanoparticle (LNP)" refers to a stable nucleic acid-lipid particle. The LNP comprises a cationic lipid, a non-cationic lipid, and a lipid that prevents aggregation of the particles (*e.g.*, a PEG-lipid conjugate). LNPs are extremely useful for synthetic applications, as they exhibit a prolonged circulation lifetime following intravenous (i.v.) injection and accumulate at distal sites (*e.g.*, sites physically separated from the site of administration).

In one example, the lipid-to-drug ratio (mass-to-mass ratio) (*e.g.*, lipid-to-siRNA ratio) is in the range of about 1:1 to about 50:1, about 1:1 to about 25:1, about 3:1 to about 15:1, about 4:1 to about 10:1, about 5:1 to about 9:1, or about 6:1 to about 9:1.

In some embodiments, the double-stranded oligonucleotide or the conjugate is administered in a non-buffered solution.

In some embodiments, the non-buffered solution is saline or water.

In some embodiments, the double-stranded oligonucleotide or the conjugate is administered with a buffered solution.

In some embodiments, the buffered solution comprises acetate, citrate, prolamin, carbonate, or phosphate, or any combination thereof.

In some embodiments, the buffered solution is phosphate-buffered saline.

In some embodiments, the double-stranded oligonucleotide or the conjugate is formulated as a lipid formulation for delivery in membranous molecular assemblies.

In some embodiments, the lipid formulation is a nucleic acid-lipid particle, such as a lipid nanoparticle.

In some embodiments, the mass-to-mass ratio of lipid to the double-stranded oligonucleotide or the conjugate is 1:1 to 50:1, 1:1 to 25:1, 3:1 to 15:1, 4:1 to 10:1, 5:1 to 9:1, or 6:1 to 9:1.

In some embodiments, the lipid nanoparticle comprises a cationic lipid, a neutral lipid, a structured lipid, and a polymer-conjugated lipid.

In some embodiments, the cationic lipid is a compound having the structure of formula (I), or an N-oxide, a solvate, a pharmaceutically acceptable salt, or a stereoisomer thereof,

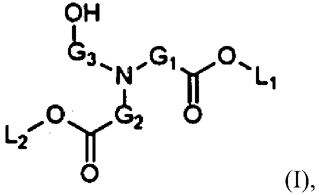

G₁ is C₁₋₆ alkylene; G₂ is C₂₋₈ alkylene; G₃ is C₁₋₃ alkylene; L₁ is C₆₋₁₅ linear alkyl; L₂ is C₁₂₋₂₅ branched alkyl.

In some embodiments, the cationic lipid is YK-009 having the structure of formula (I-I) (see patent CN114044741B):

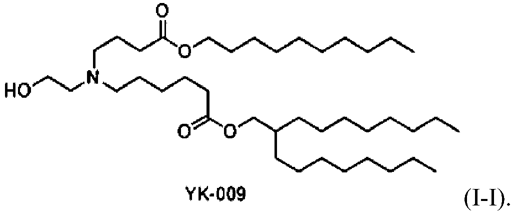

In some embodiments, the cationic lipid is a compound having the structure of formula (II), or an N-oxide, a solvate, a pharmaceutically acceptable salt, or a stereoisomer thereof,

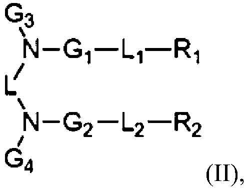

G₁ is C₂₋₈ alkylene; G₂ is C₂₋₈ alkylene; L₁ is -C(O)O- or -OC(O)-; L₂ is -C(O)O- or - OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; L is (CH₂)₂-, -(CH₂)₃-, or -(CH₂)₄-.

In some embodiments, the cationic lipid is YK-401 having the structure of formula (II-I) or YK-402 having the structure of formula (II-II) (see patent CN115784921B):

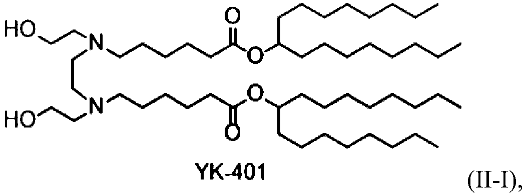

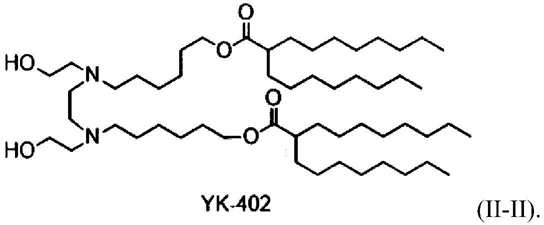

In some embodiments, the cationic lipid is a compound having the structure of formula (III), or an N-oxide, a solvate, a pharmaceutically acceptable salt, or a stereoisomer thereof,

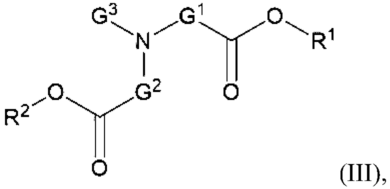

G₁ is C₁₋₆ alkylene; G₂ is C₂₋₈ alkylene; R₁ is C₆₋₂₀ linear or branched alkyl; R₂ is C₁₂₋₂₅ branched alkyl; G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂-, or CH₃CH₂NH(CH₂)₂-.

In some embodiments, the cationic lipid is YK-201 having the structure of formula (III-I) or YK-202 having the structure of formula (III-II) (see patent CN115677518B):

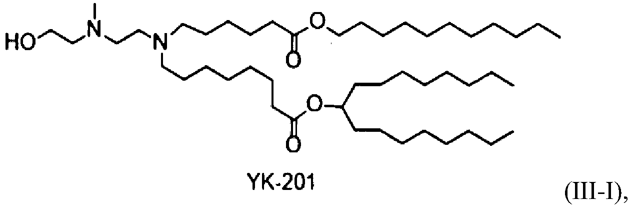

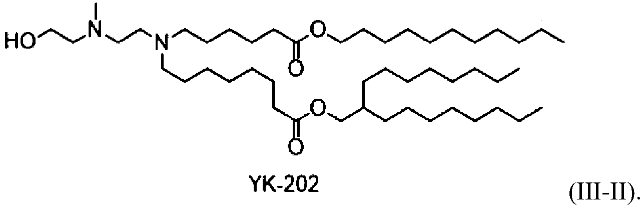

In some embodiments, the cationic lipid is a compound having the structure of formula (IV), or an N-oxide, a solvate, a pharmaceutically acceptable salt, or a stereoisomer thereof,

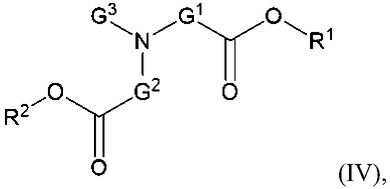

G₁ is C₁₋₈ alkylene; G₂ is C₂₋₈ alkylene; R₁ is C₆₋₂₅ linear or branched alkyl; R₂ is C₁₂₋₂₅ linear or branched alkyl; G₃ is HO(CH₂)₂N(R₃)CH₂CH(OH)CH₂-, wherein R₃ is -CH₃, - CH₂CH₃, or -CH₂CH₂OH.

In some embodiments, the cationic lipid is YK-305 having the structure of formula (IV-I) or YK-310 having the structure of formula (IV-II) (see patent CN115745820B):

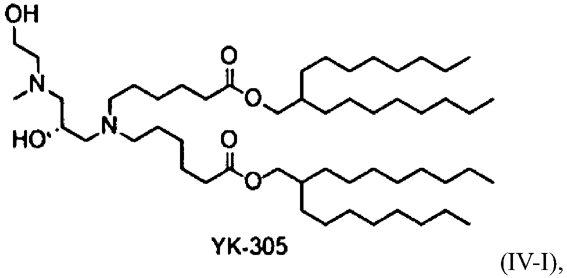

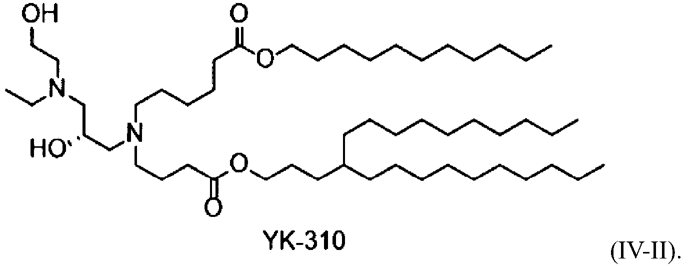

In some embodiments, the cationic lipid is a compound having the structure of formula (V), or an N-oxide, a solvate, a pharmaceutically acceptable salt, or a stereoisomer thereof,

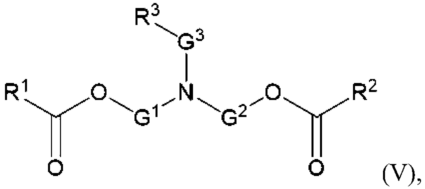

G¹ and G² are each independently unsubstituted C₆-C₁₀ alkylene; G³ is unsubstituted C₁-C₁₂ alkylene; R¹ and R² are each independently C₆-C₂₄ alkyl or C₆-C₂₄ alkenyl; R³ is OR⁵, N, -C(=O)OR⁴, -OC(=O)R⁴, or -NR⁵C(=O)R⁴; R⁴ is C₁-C₁₂ hydrocarbyl; and R⁵ is H or C₁-C₆ hydrocarbyl.

In some embodiments, the cationic lipid is ALC0315 having the structure of formula (V-I) (see patent CN108368028B):

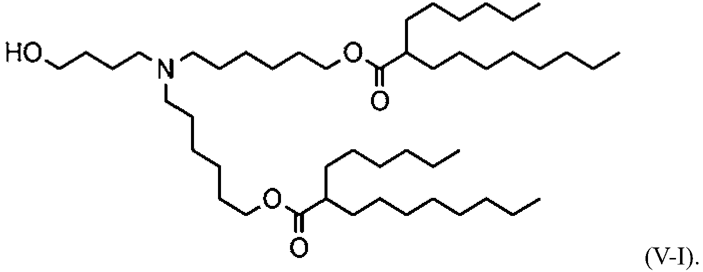

In some embodiments, the cationic lipid is a compound having the structure of formula (VI), or an N-oxide, a solvate, a pharmaceutically acceptable salt, or a stereoisomer thereof,

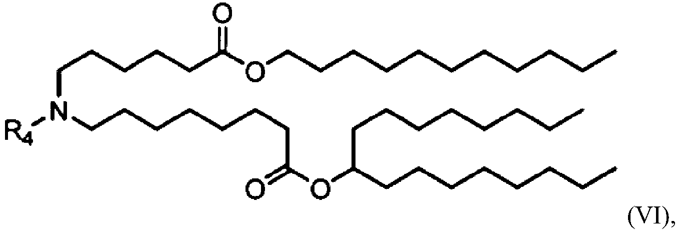

R₄ is selected from -(CH₂)ₙQ and -(CH₂)ₙCHQR; Q is selected from the group consisting of -OR, -OH, -O(CH₂)ₙN(R)₂, -OC(O)R, -CX₃, -CN, -N(R)C(O)R, -N(H)C(O)R, - N(R)S(O)₂R, -N(H)S(O)₂R, -N(R)C(O)N(R)₂, -N(H)C(O)N(R)₂, -N(H)C(O)N(H)(R), - N(R)C(S)N(R)₂, -N(H)C(S)N(R)₂, -N(H)C(S)N(H)(R), -N(R)S(O)₂R₈, and heterocycle; n is 1, 2, or 3.

In some embodiments, the cationic lipid is SM102 having the structure of formula (VI-I) (see patent CN110520409A):

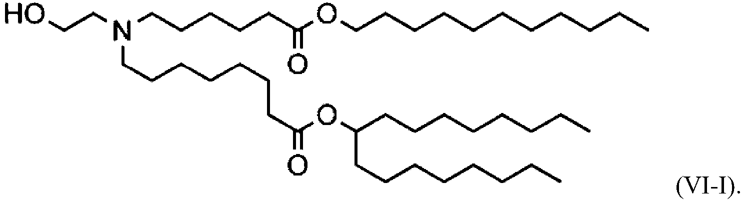

In some embodiments, the cationic lipid is compound DLIN-MC3-DMA having the structure of formula (VII), or an N-oxide, a solvate, a pharmaceutically acceptable salt, or a stereoisomer thereof,

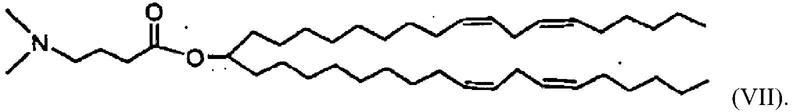

In some embodiments, the cationic lipid includes selected from one or more YK-009, YK-401, YK-305, ALC0315, SM102, and DLIN-MC3-DMA.

In some embodiments, the molar ratio of the cationic lipid to the neutral lipid is 1:1 to 10:1.

In some embodiments, the molar ratio of the cationic lipid to the structured lipid is 1:1 to 5:1.

In some embodiments, the molar ratio of the cationic lipid, the neutral lipid, the structured lipid, and the polymer-conjugated lipid is (25 to 65):(5 to 25):(25 to 70):(0.5 to 5).

In some embodiments, the molar ratio of the cationic lipid, the neutral lipid, the structured lipid, and the polymer-conjugated lipid is (25 to 65):(5 to 25):(25 to 45):(0.5 to 5).

In some embodiments, the molar ratio of the cationic lipid, the neutral lipid, the structured lipid, and the polymer-conjugated lipid is 50:10:38.5:1.5 or 49:10:39.5:1.5.

In some embodiments, the neutral lipid includes selected from one or more phosphatidylcholine, phosphatidylethanolamine, sphingomyelin, ceramide, sterols, and derivatives thereof.

In some embodiments, the neutral lipid is selected from one or more 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and mixtures thereof.

In some embodiments, the neutral lipid is 1,2-dioleoyl-sn-glycero-3-phosphoethanolamine and/or 1,2-distearoyl-sn-glycero-3-phosphocholine.

In some embodiments, the structured lipid is selected from one or more cholesterol, nonsterols, sitosterol, ergosterol, campesterol, stigmasterol, brassinosterol, tomatidine, ursolic acid, α-tocopherol, and corticosteroids.

In some embodiments, the structured lipid is cholesterol.

In some embodiments, the polymer-conjugated lipid is selected from one or more PEG-modified phosphatidylethanolamine, PEG-modified phosphatidic acid, PEG-modified ceramide, PEG-modified dialkylamine, PEG-modified diacylglycerol, and PEG-modified dialkylglycerol.

In some embodiments, the polymer-conjugated lipid is selected from one or more distearoyl phosphatidylethanolamine polyethylene glycol 2000 (DSPE-PEG2000), dimyristoylglycero-3-methoxypolyethylene glycol 2000 (DMG-PEG2000), and methoxypolyethylene glycol ditetradecylacetamide (ALC-0159).

The pharmaceutical composition of the present disclosure includes, but is not limited to, solutions, emulsions, and liposome-containing formulations. These compositions may be derived from a variety of components, including, but not limited to, preformed liquids, self-emulsifying solids, and self-emulsifying semisolids. Particularly preferred are formulations targeting the liver when treating hepatic disorders (*e.g.*, hepatocellular carcinoma).

The pharmaceutical composition of the present disclosure (which may conveniently be presented in unit dosage forms) may be prepared using conventional techniques well known in the pharmaceutical industry. Such techniques include the step of bringing into association the active ingredients with the pharmaceutical carrier(s) or excipient(s). Generally, these pharmaceutical compositions are prepared by uniformly and intimately bringing into association the active ingredients with a liquid carrier or a finely divided solid carrier or both, and then, if necessary, shaping the product.

The pharmaceutical composition of the present disclosure may be formulated into any of a variety of possible dosage forms such as, but not limited to, tablets, capsules, gel capsules, liquid syrups, softgels, suppositories, and enemas. The pharmaceutical composition of the present disclosure may also be formulated as suspensions in aqueous, non-aqueous, or mixed media. Aqueous suspensions may further comprise substances that increase the viscosity of the suspension, including, for example, sodium carboxymethylcellulose, sorbitol, and/or dextran. The suspension may also comprise a stabilizer.

Certain pharmaceutical compositions of the present disclosure further incorporate a carrier compound into the formulation. As used herein, the term "carrier compound" or "carrier" may refer to a nucleic acid or analog thereof that is inert (*i.e.*, lacks biological activity per se) but is recognized as a nucleic acid during *in vivo* processes that reduce the bioavailability of a biologically active nucleic acid, for example by degrading the biologically active nucleic acid or facilitating its removal from circulation. Co-administration of a nucleic acid and a carrier compound (typically the latter in excess) may result in a substantial reduction in the amount of nucleic acid recovered in the liver, kidney, or other extracirculatory reservoirs, presumably due to competition between the carrier compound and the nucleic acid for common receptors. For example, when co-administered with polyinosinic acid, dextran sulfate, polycytidylic acid, or 4-acetamido-4'-isothiocyano-stilbene-2,2'-disulfonic acid, the recovery of partially phosphorothioated siRNA in hepatic tissue may be reduced (Miyao et al., DsRNA Res. Dev., 1995, 5:115-121; Takakura et al., DsRNA & Nucl. Acid Drug Dev., 1996, 6:177-183).

In contrast to carrier compounds, a "pharmaceutical carrier" or "excipient" refers to a pharmaceutically acceptable solvent, suspending agent, or any other pharmaceutically inert vehicle for delivering one or more nucleic acids to an animal. The excipient may be liquid or solid and is selected with reference to the intended mode of administration in order to provide the desired bulk, consistency, and other properties, when combined with the nucleic acid and other components of a particular pharmaceutical composition Typical pharmaceutical carriers include, but are not limited to, binders (*e.g.*, pregelatinized corn starch, polyvinylpyrrolidone, or hydroxypropyl methylcellulose); fillers (*e.g.*, lactose and other sugars, microcrystalline cellulose, pectin, gelatin, calcium sulfate, ethyl cellulose, polyacrylates, or calcium hydrogen phosphate); lubricants (*e.g.*, magnesium stearate, talc, silica, colloidal silicon dioxide, stearic acid, metallic stearate, hydrogenated vegetable oil, corn starch, polyethylene glycol, sodium benzoate, or sodium acetate); disintegrants (*e.g.*, starch or sodium starch glycolate); and wetting agents (*e.g.*, sodium lauryl sulfate).

Pharmaceutically acceptable organic or inorganic excipients suitable for non-parenteral administration that do not deleteriously react with nucleic acids may also be used to formulate the pharmaceutical compositions of the present disclosure. Suitable pharmaceutically acceptable carriers include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

Formulations suitable for topical administration of nucleic acids may include sterile or non-sterile aqueous solutions or non-aqueous solutions in common solvents such as alcohols, or nucleic acid solutions in liquid or solid oil bases. These solutions may further include buffers, diluents, and other suitable additives. Pharmaceutically acceptable organic or inorganic excipients suitable for non-parenteral administration that do not deleteriously react with nucleic acids may be used.

Suitable pharmaceutically acceptable excipients include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

The dosage forms, carrier compounds, pharmaceutical carriers, excipients, and the like of the pharmaceutical compositions are described in US10125369B2, which is incorporated herein by reference.

In another aspect, the present disclosure provides a kit comprising kit A, wherein the kit A comprises one or more of the double-stranded oligonucleotide, conjugate, or pharmaceutical composition described above.

In some embodiments, the kit further comprises kit B, wherein the kit B comprises one or both of the following:
(1) other agents that reduce PCSK9, HBV, or AGT gene expression or compositions comprising the agents that reduce PCSK9, HBV, or AGT gene expression;
(2) selected from one or more the group consisting of hormone preparations, targeted small-molecule agents, proteasome inhibitors, imaging agents, diagnostic agents, chemotherapeutic agents, oncolytic agents, cytotoxic agents, cytokines, activators of co-stimulatory molecules, inhibitors of inhibitory molecules, and vaccines.

As used herein, "other agents that reduce PCSK9, HBV, or AGT gene expression or compositions comprising the agents that reduce PCSK9, HBV, or AGT gene expression" refer to agents that do not contain the double-stranded oligonucleotide, conjugate, or pharmaceutical composition provided by the present disclosure.

In another aspect, the present disclosure provides the use of the double-stranded oligonucleotide, conjugate, or pharmaceutical composition described above in the manufacture of a medicament for preventing and/or treating a disease associated with PCSK9, HBV, or AGT gene expression.

In some embodiments, the disease associated with PCSK9 gene expression is hypercholesterolemia, atherosclerosis, dyslipidemia, or cardiovascular and cerebrovascular diseases.

In some embodiments, the disease associated with HBV gene expression is selected from chronic hepatitis B, liver fibrosis, cirrhosis, liver cancer, acute hepatitis B, and diseases associated with co-infection of HBV and hepatitis D virus.

In some embodiments, the disease associated with AGT gene expression is selected from hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction.

In another aspect, the present disclosure provides a method of reducing PCSK9, HBV, or AGT gene expression or inhibiting PCSK9, HBV, or AGT replication, comprising administering one or more of the double-stranded oligonucleotide, conjugate, pharmaceutical composition, and kit described above to a sample.

In some embodiments, the method is for non-prophylactic and/or non-therapeutic purposes.

As used herein, "non-prophylactic and/or non-therapeutic purposes" refers to reducing PCSK9, HBV, or AGT gene expression or inhibiting PCSK9, HBV, or AGT replication for research purposes, for example, in a laboratory setting.

In another aspect, the present disclosure provides a method of preventing and/or treating a subject suffering from a disease associated with PCSK9, HBV, or AGT gene expression, comprising administering to the subject a prophylactically effective amount or a therapeutically effective amount of the double-stranded oligonucleotide, conjugate, or pharmaceutical composition described above.

The method is used for treating and/or preventing diseases and disorders that can be modulated by downregulating PCSK9, HBV, or AGT gene expression. For example, the double-stranded oligonucleotide described herein may be used for treating diseases associated with PCSK9, HBV, or AGT gene expression. These methods comprise administering to the subject a therapeutically effective amount or a prophylactically effective amount of the double-stranded oligonucleotide, conjugate, or pharmaceutical composition disclosed herein.

In some embodiments, the double-stranded oligonucleotide described herein may be used for treating lipid disorders, such as hyperlipidemia, and other forms of lipid imbalance, such as hypercholesterolemia and hypertriglyceridemia, as well as pathological conditions associated with these disorders, such as cardiovascular and circulatory diseases. Other diseases and disorders that can be modulated by downregulating PCSK9 gene expression include lysosomal storage diseases, including, but not limited to, Niemann-Pick disease, Tay-Sachs disease, lysosomal acid lipase deficiency, and Gaucher disease. The RNAi agents described herein may be used for treating cardiovascular diseases, such as coronary heart disease (CHD), cerebrovascular disease (CVD), aortic stenosis, peripheral vascular disease, atherosclerosis, arteriosclerosis, myocardial infarction (heart attack), cerebrovascular disease (stroke), transient ischemic attack (TIA), angina (stable and unstable), atrial fibrillation, arrhythmia, valvular disease, and/or congestive heart failure, or any other condition. These methods comprise administering to the subject a therapeutically effective amount or a prophylactically effective amount of the double-stranded oligonucleotide, conjugate, or composition disclosed herein. In some examples, the method comprises administering a therapeutic amount of a PCSK9 siRNA to a patient with a heterozygous LDLR genotype.

The double-stranded oligonucleotide disclosed herein may be administered to a subject using any route of administration known in the art, including, but not limited to, subcutaneous, intravenous, intramuscular, intraocular, intrabronchial, intrapleural, intraperitoneal, intra-arterial, lymphatic, or cerebrospinal administration, and any combination thereof. In a preferred example, the agents are administered subcutaneously.

In another example, the siRNA is administered in combination with an additional therapeutic agent. The siRNA and the additional therapeutic agent may be co-administered in the same composition, *e.g.*, parenterally, or the additional therapeutic agent may be administered as part of a separate composition or by another method described herein.

Examples of additional therapeutic agents include those known for treating hepatitis B. For example, additional agents for treating chronic hepatitis B are selected from nucleos(t)ide analogs (*e.g.*, entecavir, tenofovir disoproxil fumarate, tenofovir alafenamide, and emtricitabine) and α-interferons (*e.g.*, pegylated interferon α).

Examples of additional therapeutic agents include those known for treating hypertension or those known for treating cardiovascular and cerebrovascular diseases. For example, additional antihypertensive agents are selected from angiotensin-converting enzyme inhibitors (*e.g.*, captopril, enalapril, benazepril, perindopril), angiotensin II receptor antagonists (*e.g.*, losartan, losartan/hydrochlorothiazide, valsartan, valsartan/hydrochlorothiazide, telmisartan, telmisartan/hydrochlorothiazide, olmesartan medoxomil), and β-blockers (*e.g.*, propranolol, bisoprolol, metoprolol tartrate, metoprolol succinate).

Examples of additional therapeutic agents include those known for treating lipid disorders such as hypercholesterolemia, atherosclerosis, dyslipidemia, or cardiovascular and cerebrovascular diseases. For example, additional agents for treating hyperlipidemia are selected from fibrates, statins, bile acid sequestrants, and niacin. Additional agents for treating cardiovascular and cerebrovascular diseases are selected from angiotensin-converting enzyme inhibitors (*e.g.*, captopril, enalapril, benazepril, perindopril), angiotensin II receptor antagonists (*e.g.*, losartan, losartan/hydrochlorothiazide, valsartan, valsartan/hydrochlorothiazide, telmisartan, telmisartan/hydrochlorothiazide, olmesartan medoxomil), and β-blockers (*e.g.*, propranolol, bisoprolol, metoprolol tartrate, metoprolol succinate).

In one example, the double-stranded oligonucleotide is administered to a patient, followed by administration of the additional therapeutic agent (or vice versa). In another example, the double-stranded oligonucleotide and the additional therapeutic agent are administered simultaneously.

In some embodiments, the subject is a primate or a rodent.

In some embodiments, the subject is a human.

In some embodiments, the double-stranded oligonucleotide is administered at a dose of 0.01 mg/kg to 10 mg/kg or 0.5 mg/kg to 50 mg/kg.

In some embodiments, the double-stranded oligonucleotide is administered at a dose of about 1 to 5 mg/kg.

In some embodiments, the double-stranded oligonucleotide is administered subcutaneously.

In some embodiments, the double-stranded oligonucleotide is administered intravenously.

In some embodiments, the double-stranded oligonucleotide is administered in two or more doses.

In some embodiments, the double-stranded oligonucleotide is administered according to a dosing regimen comprising a loading phase followed by a maintenance phase, wherein
the loading phase comprises administration of a dose of 2 mg/kg, 1 mg/kg, or 0.5 mg/kg, five times weekly; and
the maintenance phase comprises administration of a dose of 2 mg/kg, 1 mg/kg, or 0.5 mg/kg, once weekly, twice weekly, three times weekly, once every two weeks, once every three weeks, once monthly, once every two months, once every three months, once every four months, once every five months, or once every six months.

In some embodiments, the double-stranded oligonucleotide is administered at an interval selected from the group consisting of once every 7 days, once every 14 days, once every 35 days, once every 42 days, and once every 49 days.

In some embodiments, the disease associated with HBV gene expression is selected from chronic hepatitis B, liver fibrosis, cirrhosis, liver cancer, acute hepatitis B, and diseases associated with co-infection of HBV and hepatitis D virus.

The abbreviations for nucleotides used herein are as follows:
A = adenosine-3'-phosphate
Am = 2'-O-methyladenosine-3'-phosphate
Ams = 2'-O-methyladenosine-3'-phosphorothioate
Af = 2'-fluoroadenosine-3'-phosphate
Afs = 2'-fluoroadenosine-3'-phosphorothioate
G = guanosine-3'-phosphate
Gm = 2'-O-methylguanosine-3'-phosphate
Gms = 2'-O-methylguanosine-3'-phosphorothioate
Gf = 2'-fluoroguanosine-3'-phosphate
Gfs = 2'-fluoroguanosine-3'-phosphorothioate
C = cytidine-3'-phosphate
Cm = 2'-O-methylcytidine-3'-phosphate
Cms = 2'-O-methylcytidine-3'-phosphorothioate
Cf = 2'-fluorocytidine-3'-phosphate
Cfs = 2'-fluorocytidine-3'-phosphorothioate
U = uridine-3'-phosphate
Um = 2'-O-methyluridine-3'-phosphate
Ums = 2'-O-methyluridine-3'-phosphorothioate
Uf = 2'-fluorouridine-3'-phosphate
Ufs = 2'-fluorouridine-3'-phosphorothioate
AmsEVP = 5'-vinyl-(E)-phosphonate-2'-O-methyladenosine-3'-phosphorothioate
UmsEVP = 5'-vinyl-(E)-phosphonate-2'-O-methyluridine-3'-phosphorothioate
A(gna) = adenosine-glycol nucleic acid
C(gna) = cytidine-glycol nucleic acid
G(gna) = guanosine-glycol nucleic acid
T(gna) = thymidine-glycol nucleic acid
U(gna) = uridine-glycol nucleic acid
dA = deoxyadenosine-3'-phosphate
dG = deoxyguanosine-3'-phosphate
dC = deoxycytidine-3'-phosphate
dT = deoxythymidine-3'-phosphate

The following examples are provided to illustrate the present disclosure but are not intended to limit the scope thereof. Unless otherwise specified, the technical methods used in the examples are conventional methods well known to those skilled in the art, and the raw materials used are commercially available products.

### Examples

### Example 1: Synthesis of alternately modified small interfering oligonucleotides

A total of 84 siRNA basic sequences were designed based on the PCSK9 mRNA sequence. To enhance the inhibitory efficiency and stability of the sequences, the basic sequences were alternately modified with 2'-O-methyl (2'-OMe) and 2'-fluoro (2'-F), with terminal phosphorothioate modifications. In the sense strand, all odd-numbered positions are modified with 2'-F, and all even-numbered positions are modified with 2'-OMe. In the antisense strand, all odd-numbered positions are modified with 2'-OMe, and all even-numbered positions are modified with 2'-F. In addition, there are two phosphorothioate modifications at the 5' end of the sense strand, while there are two phosphorothioate modifications at both the 5' and 3' ends of the antisense strand. The alternately modified siRNA sequences are shown in Table 2.

### 1. Synthesis of alternately modified sequence P92-si5

The siRNA basic sequence designated as P92-si5 in Table 2 is as follows:
Sense strand: 5'-AAGAUCCUGCAUGUCUUCCAU-3' (SEQ ID NO: 1)
Antisense strand: 5'-AUGGAAGACAUGCAGGAUCUUGG-3' (SEQ ID NO: 13)

In the sense strand, all odd-numbered positions are modified with 2'-F, and all even-numbered positions are modified with 2'-OMe. In the antisense strand, all odd-numbered positions are modified with 2'-OMe, and all even-numbered positions are modified with 2'-F. In addition, there are two phosphorothioate modifications at the 5' end of the sense strand, while there are two phosphorothioate modifications at both the 5' and 3' ends of the antisense strand.

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-OMe phosphoramidite monomer (Formula 1), DMT-C-OMe phosphoramidite monomer (Formula 2), DMT-G-OMe phosphoramidite monomer (Formula 3), DMT-U-OMe phosphoramidite monomer (Formula 4), DMT-A-F phosphoramidite monomer (Formula 5), DMT-C-F phosphoramidite monomer (Formula 6), DMT-G-F phosphoramidite monomer (Formula 7), and DMT-U-F phosphoramidite monomer (Formula 8).

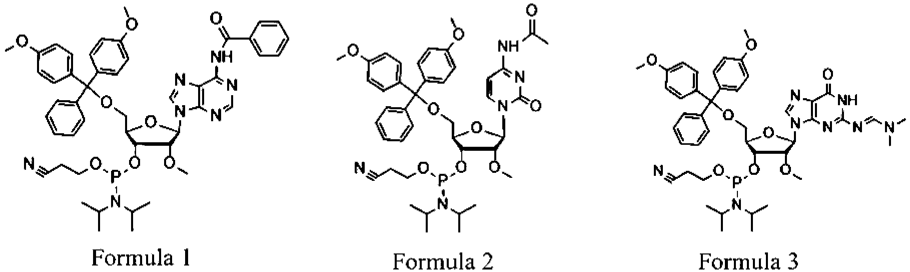

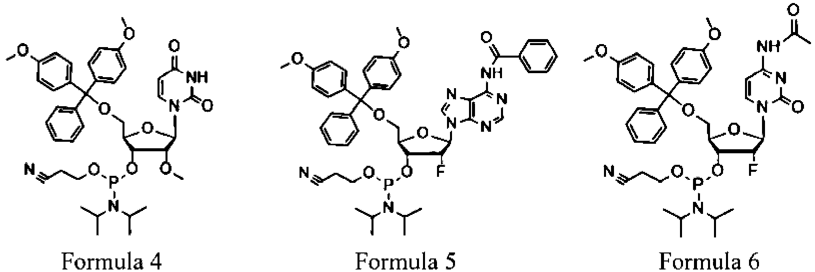

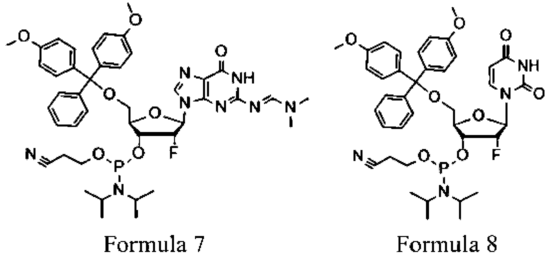

The preparation was carried out according to the following steps:

### (1) Deprotection

3% dichloroacetic acid in toluene was used as a deprotection reagent to remove the DMT protecting group, followed by washing with acetonitrile.

### (2) Coupling

Each nucleotide monomer in acetonitrile was coupled using 0.25 M 5-ethylthio-1*H-*tetrazole as an activator, followed by washing with acetonitrile.

### (3) Oxidation or sulfurization

Oxidation: 0.05 M iodine in pyridine/water (90/10) was used as an oxidant for oxidation, followed by washing with acetonitrile.

Sulfurization: 3% xanthane hydride in pyridine was used as a sulfurizing agent for sulfurization, followed by washing with acetonitrile.

### (4) Hydroxyl protection

10% acetic anhydride in tetrahydrofuran (CAP A) and tetrahydrofuran/pyridine/*N-*methylimidazole (74/10/16, v/v/v) (CAP B) were used as hydroxyl protecting reagents for hydroxyl protection, followed by washing with acetonitrile.

The above steps were repeated according to the set sequence to obtain a fully protected product.

(5) 3% dichloroacetic acid in toluene was used as a deprotection reagent to remove the DMT protecting group from the last nucleotide, followed by washing with acetonitrile.

### (6) Ammonolysis and purification

The solid-phase support was transferred to a reactor and treated with concentrated aqueous ammonia (25 to 28%) at 60°C for 12 hours. The system was then cooled to room temperature, and the mixture was transferred to a pressure filter, followed by washing with a mixture of purified water and ethanol. The filtrates were combined, subjected to column chromatography, concentrated, and lyophilized to obtain a product.

### (7) Annealing

The purified sense and antisense strands were mixed at a molar ratio of 1:1, heated to 95°C and maintained for 3 minutes, and slowly cooled to room temperature to form a duplex.

P92-si5 exhibited a purity of 97.4% and a measured molecular weight of 14493.52.

### 2. Synthesis of other sequences

All other sequences listed in Table 2 were synthesized using the method described above.

**Table 2: Alternately modified siRNA sequences**

| Sequence ID | Sense sequence 5'-3' | SEQ ID NO:/corr. basic SEQ ID NO: | Antisense sequence 5'-3' | SEQ ID NO:/corr. basic SEQ ID NO: |
|---|---|---|---|---|
| P92-si5 | Afs-Ams-Gf-Am-Uf-Cm-Cf- Um-Gf-Cm-Af-Um-Gf-Um- Cf-Um-Uf-Cm-Cf-Am-Uf | 169/1 | Ams-Ufs-Gm-Gf-Am-Af-Gm-Af- Cm-Af-Um-Gf-Cm-Af-Gm-Gf- Am-Uf-Cm-Uf-Ums-Gfs-Gm | 181/13 |
| P92-si51 | Ufs-Gms-Gf-Am-Gf-Gm-Cf- Um-Uf-Am-Gf-Cm-Uf-Um- Uf-Cm-Uf-Gm-Gf-Am-Uf | 170/2 | Ams-Ufs-Cm-Cf-Am-Gf-Am-Af- Am-Gf-Cm-Uf-Am-Af-Gm-Cf- Cm-Uf-Cm-Cf-Ams-Ufs-Um | 182/14 |
| P92-si81 | Cfs-Ums-Uf-Um-Uf-Gm-Uf- Am-Af-Cm-Uf-Um-Gf-Am- Af-Gm-Af-Um-Af-Um-Uf | 171/3 | Ams-Afs-Um-Af-Um-Cf-Um-Uf- Cm-Af-Am-Gf-Um-Uf-Am-Cf- Am-Af-Am-Af-Gms-Cfs-Am | 183/15 |
| P92-si82 | Gfs-Ams-Af-Gm-Af-Um-Af- Um-Uf-Um-Af-Um-Uf-Cm- Uf-Gm-Gf-Gm-Uf-Um-Uf | 172/4 | Ams-Afs-Am-Cf-Cm-Cf-Am-Gf- Am-Af-Um-Af-Am-Af-Um-Af- Um-Cf-Um-Uf-Cms-Afs-Am | 184/16 |
| P92-si84 | Gfs-Ums-Uf-Um-Uf-Gm-Uf- Am-Gf-Cm-Af-Um-Uf-Um- Uf-Um-Af-Um-Uf-Am-Af | 173/5 | Ums-Ufs-Am-Af-Um-Af-Am-Af- Am-Af-Um-Gf-Cm-Uf-Am-Cf- Am-Af-Am-Af-Cms-Cfs-Cm | 185/17 |
| P92-si3 | Cfs-Ams-Cf-Cm-Af-Am-Gf- Am-Uf-Cm-Cf-Um-Gf-Cm- Af-Um-Gf-Um-Cf-Um-Uf | 174/6 | Ams-Afs-Gm-Af-Cm-Af-Um-Gf- Cm-Af-Gm-Gf-Am-Uf-Cm-Uf- Um-Gf-Gm-Uf-Gms-Afs-Gm | 186/18 |
| P92-si8 | Cfs-Ums-Cf-Am-Uf-Am-Gf- Gm-Cf-Cm-Uf-Gm-Gf-Am- Gf-Um-Uf-Um-Af-Um-Uf | 175/7 | Ams-Afs-Um-Af-Am-Af-Cm-Uf- Cm-Cf-Am-Gf-Gm-Cf-Cm-Uf- Am-Uf-Gm-Af-Gms-Gfs-Gm | 187/19 |
| P92-si9 | Gfs-Gms-Cf-Cm-Uf-Gm-Gf- Am-Gf-Um-Uf-Um-Af-Um- Uf-Cm-Gf-Gm-Af-Am-Af | 176/8 | Ums-Ufs-Um-Cf-Cm-Gf-Am-Af- Um-Af-Am-Af-Cm-Uf-Cm-Cf- Am-Gf-Gm-Cf-Cms-Ufs-Am | 188/20 |
| P92-si21 | Cfs-Ams-Gf-Cm-Af-Cm-Cf- Um-Gf-Cm-Uf-Um-Uf-Gm- Uf-Gm-Uf-Cm-Af-Cm-Af | 177/9 | Ums-Gfs-Um-Gf-Am-Cf-Am-Cf- Am-Af-Am-Gf-Cm-Af-Gm-Gf- Um-Gf-Cm-Uf-Gms-Cfs-Am | 189/21 |
| P92-si31 | Gfs-Gms-Uf-Cm-Uf-Gm-Gf- Am-Af-Um-Gf-Cm-Af-Am- Af-Gm-Uf-Cm-Af-Am-Gf | 178/10 | Cms-Ufs-Um-Gf-Am-Cf-Um-Uf- Um-Gf-Cm-Af-Um-Uf-Cm-Cf- Am-Gf-Am-Cf-Cms-Ufs-Gm | 190/22 |
| P92-si73 | Gfs-Cms-Gf-Gm-Af-Gm-Af- Um-Gf-Cm-Uf-Um-Cf-Um- Af-Am-Gf-Gm-Cf-Am-Uf | 179/11 | Ams-Ufs-Gm-Cf-Cm-Uf-Um-Af- Gm-Af-Am-Gf-Cm-Af-Um-Cf- Um-Cf-Cm-Gf-Cms-Cfs-Am | 191/23 |
| P92-si83 | Gfs-Gms-Gf-Um-Uf-Um-Uf- Gm-Uf-Am-Gf-Cm-Af-Um- Uf-Um-Uf-Um-Af-Um-Uf | 180/12 | Ams-Afs-Um-Af-Am-Af-Am-Af- Um-Gf-Cm-Uf-Am-Cf-Am-Af- Am-Af-Cm-Cf-Cms-Afs-Gm | 192/24 |
| P92-si1 | Gfs-Gms-Gf-Am-Uf-Am-Cf- Cm-Uf-Cm-Af-Cm-Cf-Am- Af-Gm-Af-Um-Cf-Cm-Uf | 193/25 | Ams-Gfs-Gm-Af-Um-Cf-Um-Uf- Gm-Gf-Um-Gf-Am-Gf-Gm-Uf- Am-Uf-Cm-Cf-Cms-Cfs-Gm | 265/97 |
| P92-si2 | Afs-Ums-Af-Cm-Cf-Um-Cf- Am-Cf-Cm-Af-Am-Gf-Am- Uf-Cm-Cf-Um-Gf-Cm-Af | 194/26 | Ums-Gfs-Cm-Af-Gm-Gf-Am-Uf- Cm-Uf-Um-Gf-Gm-Uf-Gm-Af- Gm-Gf-Um-Af-Ums-Cfs-Cm | 266/98 |
| P92-si4 | Cfs-Ams-Af-Gm-Af-Um-Cf- Cm-Uf-Gm-Cf-Am-Uf-Gm- Uf-Cm-Uf-Um-Cf-Cm-Af | 195/27 | Ums-Gfs-Gm-Af-Am-Gf-Am-Cf- Am-Uf-Gm-Cf-Am-Gf-Gm-Af- Um-Cf-Um-Uf-Gms-Gfs-Um | 267/99 |
| P92-si6 | Gfs-Gms-Cf-Um-Uf-Cm-Cf- Um-Gf-Gm-Uf-Gm-Af-Am- Gf-Am-Uf-Gm-Af-Gm-Uf | 196/28 | Ams-Cfs-Um-Cf-Am-Uf-Cm-Uf- Um-Cf-Am-Cf-Cm-Af-Gm-Gf- Am-Af-Gm-Cf-Cms-Afs-Gm | 268/100 |
| P92-si7 | Gfs-Cms-Uf-Gm-Gf-Am-Gf- Cm-Uf-Gm-Gf-Cm-Cf-Um- Uf-Gm-Af-Am-Gf-Um-Uf | 197/29 | Ams-Afs-Cm-Uf-Um-Cf-Am-Af- Gm-Gf-Cm-Cf-Am-Gf-Cm-Uf- Cm-Cf-Am-Gf-Cms-Afs-Gm | 269/101 |
| P92-si10 | Cfs-Cms-Uf-Gm-Gf-Am-Gf- Um-Uf-Um-Af-Um-Uf-Cm- Gf-Gm-Af-Am-Af-Am-Gf | 198/30 | Cms-Ufs-Um-Uf-Um-Cf-Cm-Gf- Am-Af-Um-Af-Am-Af-Cm-Uf- Cm-Cf-Am-Gf-Gms-Cfs-Cm | 270/102 |
| P92-si11 | Gfs-Cms-Cf-Am-Gf-Cm-Uf- Gm-Gf-Um-Cf-Cm-Af-Gm- Cf-Cm-Uf-Gm-Uf-Gm-Gf | 199/31 | Cms-Cfs-Am-Cf-Am-Gf-Gm-Cf- Um-Gf-Gm-Af-Cm-Cf-Am-Gf- Cm-Uf-Gm-Gf-Cms-Ufs-Um | 271/103 |
| P92-si12 | Afs-Cms-Uf-Gm-Gf-Um-Gf- Gm-Uf-Gm-Cf-Um-Gf-Cm- Uf-Gm-Cf-Cm-Cf-Cm-Uf | 200/32 | Ams-Gfs-Gm-Gf-Gm-Cf-Am-Gf- Cm-Af-Gm-Cf-Am-Cf-Cm-Af- Cm-Cf-Am-Gf-Ums-Gfs-Gm | 272/104 |
| P92-si13 | Ufs-Gms-Cf-Cm-Cf-Cm-Uf- Gm-Gf-Cm-Gf-Gm-Gf-Um- Gf-Gm-Gf-Um-Af-Cm-Af | 201/33 | Ums-Gfs-Um-Af-Cm-Cf-Cm-Af- Cm-Cf-Cm-Gf-Cm-Cf-Am-Gf- Gm-Gf-Gm-Cf-Ams-Gfs-Cm | 273/105 |
| P92-si14 | Ufs-Gms-Gf-Gm-Uf-Am-Cf- Am-Gf-Cm-Cf-Gm-Cf-Gm- Uf-Cm-Cf-Um-Cf-Am-Af | 202/34 | Ums-Ufs-Gm-Af-Gm-Gf-Am-Cf- Gm-Cf-Gm-Gf-Cm-Uf-Gm-Uf- Am-Cf-Cm-Cf-Ams-Cfs-Cm | 274/106 |
| P92-si15 | Cfs-Gms-Uf-Cm-Cf-Um-Cf- Am-Af-Cm-Gf-Cm-Cf-Gm- Cf-Cm-Uf-Gm-Cf-Cm-Af | 203/35 | Ums-Gfs-Gm-Cf-Am-Gf-Gm-Cf- Gm-Gf-Cm-Gf-Um-Uf-Gm-Af- Gm-Gf-Am-Cf-Gms-Cfs-Gm | 275/107 |
| P92-si16 | Gfs-Gms-Uf-Cm-Gf-Um-Gf- Cm-Uf-Gm-Gf-Um-Cf-Am- Cf-Cm-Gf-Cm-Uf-Gm-Cf | 204/36 | Gms-Cfs-Am-Gf-Cm-Gf-Gm-Uf- Gm-Af-Cm-Cf-Am-Gf-Cm-Af- Cm-Gf-Am-Cf-Cms-Cfs-Cm | 276/108 |
| P92-si17 | Afs-Gms-Gf-Am-Cf-Am-Uf- Cm-Af-Um-Uf-Gm-Gf-Um- Gf-Cm-Cf-Um-Cf-Cm-Af | 205/37 | Ums-Gfs-Gm-Af-Gm-Gf-Cm-Af- Cm-Cf-Am-Af-Um-Gf-Am-Uf- Gm-Uf-Cm-Cf-Ums-Cfs-Cm | 277/109 |
| P92-si18 | Ufs-Cms-Af-Um-Uf-Gm-Gf- Um-Gf-Cm-Cf-Um-Cf-Cm- Af-Gm-Cf-Gm-Af-Cm-Uf | 206/38 | Ams-Gfs-Um-Cf-Gm-Cf-Um-Gf- Gm-Af-Gm-Gf-Cm-Af-Cm-Cf- Am-Af-Um-Gf-Ams-Ufs-Gm | 278/110 |
| P92-si19 | Gfs-Ums-Gf-Cm-Cf-Um-Cf- Cm-Af-Gm-Cf-Gm-Af-Cm- Uf-Gm-Cf-Am-Gf-Cm-Af | 207/39 | Ums-Gfs-Cm-Uf-Gm-Cf-Am-Gf- Um-Cf-Gm-Cf-Um-Gf-Gm-Af- Gm-Gf-Cm-Af-Cms-Cfs-Am | 279/111 |
| P92-si20 | Afs-Gms-Cf-Gm-Af-Cm-Uf- Gm-Cf-Am-Gf-Cm-Af-Cm- Cf-Um-Gf-Cm-Uf-Um-Uf | 208/40 | Ams-Afs-Am-Gf-Cm-Af-Gm-Gf- Um-Gf-Cm-Uf-Gm-Cf-Am-Gf- Um-Cf-Gm-Cf-Ums-Gfs-Gm | 280/112 |
| P92-si22 | Gfs-Cms-Af-Cm-Cf-Um-Gf- Cm-Uf-Um-Uf-Gm-Uf-Gm- Uf-Cm-Af-Cm-Af-Gm-Af | 209/41 | Ums-Cfs-Um-Gf-Um-Gf-Am-Cf- Am-Cf-Am-Af-Am-Gf-Cm-Af- Gm-Gf-Um-Gf-Cms-Ufs-Gm | 281/113 |
| P92-si23 | Cfs-Ums-Uf-Um-Gf-Um-Gf- Um-Cf-Am-Cf-Am-Gf-Am- Gf-Um-Gf-Gm-Gf-Am-Cf | 210/42 | Gms-Ufs-Cm-Cf-Cm-Af-Cm-Uf- Cm-Uf-Gm-Uf-Gm-Af-Cm-Af- Cm-Af-Am-Af-Gms-Cfs-Am | 282/114 |
| P92-si24 | Afs-Gms-Uf-Gm-Gf-Gm-Af- Cm-Af-Um-Cf-Am-Cf-Am- Gf-Gm-Cf-Um-Gf-Cm-Uf | 211/43 | Ams-Gfs-Cm-Af-Gm-Cf-Cm-Uf- Gm-Uf-Gm-Af-Um-Gf-Um-Cf- Cm-Cf-Am-Cf-Ums-Cfs-Um | 283/115 |
| P92-si25 | Afs-Ums-Cf-Am-Cf-Am-Gf- Gm-Cf-Um-Gf-Cm-Uf-Gm- Cf-Cm-Cf-Am-Cf-Gm-Uf | 212/44 | Ams-Cfs-Gm-Uf-Gm-Gf-Gm-Cf- Am-Gf-Cm-Af-Gm-Cf-Cm-Uf- Gm-Uf-Gm-Af-Ums-Gfs-Um | 284/116 |
| P92-si26 | Ufs-Gms-Uf-Cm-Uf-Gm-Cf- Cm-Gf-Am-Gf-Cm-Cf-Gm- Gf-Am-Gf-Cm-Uf-Cm-Af | 213/45 | Ums-Gfs-Am-Gf-Cm-Uf-Cm-Cf- Gm-Gf-Cm-Uf-Cm-Gf-Gm-Cf- Am-Gf-Am-Cf-Ams-Gfs-Cm | 285/117 |
| P92-si27 | Gfs-Gms-Cf-Cm-Gf-Am-Gf- Um-Uf-Gm-Af-Gm-Gf-Cm- Af-Gm-Af-Gm-Af-Cm-Uf | 214/46 | Ams-Gfs-Um-Cf-Um-Cf-Um-Gf- Cm-Cf-Um-Cf-Am-Af-Cm-Uf- Cm-Gf-Gm-Cf-Cms-Afs-Gm | 286/118 |
| P92-si28 | Cfs-Ams-Uf-Cm-Cf-Am-Cf- Gm-Cf-Um-Uf-Cm-Cf-Um- Gf-Cm-Uf-Gm-Cf-Cm-Af | 215/47 | Ums-Gfs-Gm-Cf-Am-Gf-Cm-Af- Gm-Gf-Am-Af-Gm-Cf-Gm-Uf- Gm-Gf-Am-Uf-Gms-Cfs-Um | 287/119 |
| P92-si29 | Cfs-Cms-Cf-Am-Gf-Gm-Uf- Cm-Uf-Gm-Gf-Am-Af-Um- Gf-Cm-Af-Am-Af-Gm-Uf | 216/48 | Ams-Cfs-Um-Uf-Um-Gf-Cm-Af- Um-Uf-Cm-Cf-Am-Gf-Am-Cf- Cm-Uf-Gm-Gf-Gms-Gfs-Cm | 288/120 |
| P92-si30 | Afs-Gms-Gf-Um-Cf-Um-Gf- Gm-Af-Am-Uf-Gm-Cf-Am- Af-Am-Gf-Um-Cf-Am-Af | 217/49 | Ums-Ufs-Gm-Af-Cm-Uf-Um-Uf- Gm-Cf-Am-Uf-Um-Cf-Cm-Af- Gm-Af-Cm-Cf-Ums-Gfs-Gm | 289/121 |
| P92-si32 | Gfs-Ams-Af-Um-Gf-Cm-Af- Am-Af-Gm-Uf-Cm-Af-Am- Gf-Gm-Af-Gm-Cf-Am-Uf | 218/50 | Ams-Ufs-Gm-Cf-Um-Cf-Cm-Uf- Um-Gf-Am-Cf-Um-Uf-Um-Gf- Cm-Af-Um-Uf-Cms-Cfs-Am | 290/122 |
| P92-si33 | Cfs-Ams-Af-Am-Gf-Um-Cf- Am-Af-Gm-Gf-Am-Gf-Cm- Af-Um-Gf-Gm-Af-Am-Uf | 219/51 | Ams-Ufs-Um-Cf-Cm-Af-Um-Gf- Cm-Uf-Cm-Cf-Um-Uf-Gm-Af- Cm-Uf-Um-Uf-Gms-Cfs-Am | 291/123 |
| P92-si34 | Cfs-Cms-Cf-Um-Cf-Am-Gf- Gm-Af-Gm-Cf-Am-Gf-Gm- Uf-Gm-Af-Cm-Cf-Gm-Uf | 220/52 | Ams-Cfs-Gm-Gf-Um-Cf-Am-Cf- Cm-Uf-Gm-Cf-Um-Cf-Cm-Uf- Gm-Af-Gm-Gf-Gms-Gfs-Cm | 292/124 |
| P92-si35 | Gfs-Ams-Gf-Gm-Af-Gm-Gf- Gm-Cf-Um-Gf-Gm-Af-Cm- Cf-Cm-Uf-Gm-Af-Cm-Uf | 221/53 | Ams-Gfs-Um-Cf-Am-Gf-Gm-Gf- Um-Cf-Cm-Af-Gm-Cf-Cm-Cf- Um-Cf-Cm-Uf-Cms-Gfs-Cm | 293/125 |
| P92-si36 | Gfs-Cms-Uf-Gm-Cf-Am-Gf- Um-Gf-Cm-Cf-Cm-Uf-Cm- Cf-Cm-Uf-Gm-Gf-Gm-Af | 222/54 | Ums-Cfs-Cm-Cf-Am-Gf-Gm-Gf- Am-Gf-Gm-Gf-Cm-Af-Cm-Uf- Gm-Cf-Am-Gf-Cms-Cfs-Am | 294/126 |
| P92-si37 | Cfs-Cms-Uf-Cm-Cf-Cm-Uf- Gm-Gf-Gm-Af-Cm-Cf-Um- Cf-Cm-Cf-Am-Cf-Gm-Uf | 223/55 | Ams-Cfs-Gm-Uf-Gm-Gf-Gm-Af- Gm-Gf-Um-Cf-Cm-Cf-Am-Gf- Gm-Gf-Am-Gf-Gms-Gfs-Cm | 295/127 |
| P92-si38 | Cfs-Cms-Cf-Um-Gf-Gm-Gf- Am-Cf-Cm-Uf-Cm-Cf-Cm- Af-Cm-Gf-Um-Cf-Cm-Uf | 224/56 | Ams-Gfs-Gm-Af-Cm-Gf-Um-Gf- Gm-Gf-Am-Gf-Gm-Uf-Cm-Cf- Cm-Af-Gm-Gf-Gms-Afs-Gm | 296/128 |
| P92-si39 | Gfs-Gms-Gf-Cm-Cf-Um-Af- Cm-Gf-Cm-Cf-Gm-Uf-Am- Gf-Am-Cf-Am-Af-Cm-Af | 225/57 | Ums-Gfs-Um-Uf-Gm-Uf-Cm-Uf- Am-Cf-Gm-Gf-Cm-Gf-Um-Af- Gm-Gf-Cm-Cf-Cms-Cfs-Cm | 297/129 |
| P92-si40 | Gfs-Cms-Cf-Gm-Uf-Am-Gf- Am-Cf-Am-Af-Cm-Af-Cm- Gf-Um-Gf-Um-Gf-Um-Af | 226/58 | Ums-Afs-Cm-Af-Cm-Af-Cm-Gf- Um-Gf-Um-Uf-Gm-Uf-Cm-Uf- Am-Cf-Gm-Gf-Cms-Gfs-Um | 298/130 |
| P92-si41 | Cfs-Cms-Gf-Um-Af-Gm-Af- Cm-Af-Am-Cf-Am-Cf-Gm- Uf-Gm-Uf-Gm-Uf-Am-Gf | 227/59 | Cms-Ufs-Am-Cf-Am-Cf-Am-Cf- Gm-Uf-Gm-Uf-Um-Gf-Um-Cf- Um-Af-Cm-Gf-Gms-Cfs-Gm | 299/131 |
| P92-si42 | Ufs-Ams-Gf-Am-Cf-Am-Af- Cm-Af-Cm-Gf-Um-Gf-Um- Gf-Um-Af-Gm-Uf-Cm-Af | 228/60 | Ums-Gfs-Am-Cf-Um-Af-Cm-Af- Cm-Af-Cm-Gf-Um-Gf-Um-Uf- Gm-Uf-Cm-Uf-Ams-Cfs-Gm | 300/132 |
| P92-si43 | Gfs-Ums-Gf-Um-Gf-Um-Af- Gm-Uf-Cm-Af-Gm-Gf-Am- Gf-Cm-Cf-Gm-Gf-Gm-Af | 229/61 | Ums-Cfs-Cm-Cf-Gm-Gf-Cm-Uf- Cm-Cf-Um-Gf-Am-Cf-Um-Af- Cm-Af-Cm-Af-Cms-Gfs-Um | 301/133 |
| P92-si44 | Gfs-Ams-Gf-Cm-Cf-Gm-Gf- Gm-Af-Cm-Gf-Um-Cf-Am- Gf-Cm-Af-Cm-Uf-Am-Cf | 230/62 | Gms-Ufs-Am-Gf-Um-Gf-Cm-Uf- Gm-Af-Cm-Gf-Um-Cf-Cm-Cf- Gm-Gf-Cm-Uf-Cms-Cfs-Um | 302/134 |
| P92-si45 | Ufs-Cms-Af-Gm-Cf-Am-Cf- Um-Af-Cm-Af-Gm-Gf-Cm- Af-Gm-Cf-Am-Cf-Cm-Af | 231/63 | Ums-Gfs-Gm-Uf-Gm-Cf-Um-Gf- Cm-Cf-Um-Gf-Um-Af-Gm-Uf- Gm-Cf-Um-Gf-Ams-Cfs-Gm | 303/135 |
| P92-si46 | Ufs-Cms-Uf-Cm-Cf-Am-Gf- Cm-Uf-Am-Af-Cm-Uf-Gm- Uf-Gm-Gf-Am-Gf-Am-Af | 232/64 | Ums-Ufs-Cm-Uf-Cm-Cf-Am-Cf- Am-Gf-Um-Uf-Am-Gf-Cm-Uf- Gm-Gf-Am-Gf-Ams-Ufs-Gm | 304/136 |
| P92-si47 | Gfs-Cms-Uf-Cm-Cf-Cm-Uf- Gm-Af-Um-Uf-Am-Af-Um- Gf-Gm-Af-Gm-Gf-Cm-Uf | 233/65 | Ams-Gfs-Cm-Cf-Um-Cf-Cm-Af- Um-Uf-Am-Af-Um-Cf-Am-Gf- Gm-Gf-Am-Gf-Cms-Cfs-Cm | 305/137 |
| P92-si48 | Ufs-Cms-Cf-Cm-Uf-Gm-Af- Um-Uf-Am-Af-Um-Gf-Gm- Af-Gm-Gf-Cm-Uf-Um-Af | 234/66 | Ums-Afs-Am-Gf-Cm-Cf-Um-Cf- Cm-Af-Um-Uf-Am-Af-Um-Cf- Am-Gf-Gm-Gf-Ams-Gfs-Cm | 306/138 |
| P92-si49 | Cfs-Cms-Uf-Gm-Af-Um-Uf- Am-Af-Um-Gf-Gm-Af-Gm- Gf-Cm-Uf-Um-Af-Gm-Cf | 235/67 | Gms-Cfs-Um-Af-Am-Gf-Cm-Cf- Um-Cf-Cm-Af-Um-Uf-Am-Af- Um-Cf-Am-Gf-Gms-Gfs-Am | 307/139 |
| P92-si50 | Gfs-Ams-Uf-Um-Af-Am-Uf- Gm-Gf-Am-Gf-Gm-Cf-Um- Uf-Am-Gf-Cm-Uf-Um-Uf | 236/68 | Ams-Afs-Am-Gf-Cm-Uf-Am-Af- Gm-Cf-Cm-Uf-Cm-Cf-Am-Uf- Um-Af-Am-Uf-Cms-Afs-Gm | 308/140 |
| P92-si52 | Gfs-Gms-Uf-Gm-Gf-Um-Cf- Am-Cf-Am-Gf-Gm-Cf-Um- Gf-Um-Gf-Cm-Cf-Um-Uf | 237/69 | Ams-Afs-Gm-Gf-Cm-Af-Cm-Af- Gm-Cf-Cm-Uf-Gm-Uf-Gm-Af- Cm-Cf-Am-Cf-Cms-Afs-Gm | 309/141 |
| P92-si53 | Ufs-Cms-Af-Cm-Af-Gm-Gf- Cm-Uf-Gm-Uf-Gm-Cf-Cm- Uf-Um-Gf-Gm-Uf-Um-Uf | 238/70 | Ams-Afs-Am-Cf-Cm-Af-Am-Gf- Gm-Cf-Am-Cf-Am-Gf-Cm-Cf- Um-Gf-Um-Gf-Ams-Cfs-Cm | 310/142 |
| P92-si54 | Gfs-Ums-Gf-Cm-Cf-Um-Uf- Gm-Gf-Um-Uf-Um-Cf-Cm- Uf-Gm-Af-Gm-Cf-Cm-Af | 239/71 | Ums-Gfs-Gm-Cf-Um-Cf-Am-Gf- Gm-Af-Am-Af-Cm-Cf-Am-Af- Gm-Gf-Cm-Af-Cms-Afs-Gm | 311/143 |
| P92-si55 | Afs-Gms-Cf-Cm-Af-Cm-Cf- Um-Uf-Um-Af-Cm-Uf-Cm- Uf-Gm-Cf-Um-Cf-Um-Af | 240/72 | Ums-Afs-Gm-Af-Gm-Cf-Am-Gf- Am-Gf-Um-Af-Am-Af-Gm-Gf- Um-Gf-Gm-Cf-Ums-Cfs-Am | 312/144 |
| P92-si56 | Cfs-Cms-Af-Cm-Cf-Um-Uf- Um-Af-Cm-Uf-Cm-Uf-Gm- Cf-Um-Cf-Um-Af-Um-Gf | 241/73 | Cms-Afs-Um-Af-Gm-Af-Gm-Cf- Am-Gf-Am-Gf-Um-Af-Am-Af- Gm-Gf-Um-Gf-Gms-Cfs-Um | 313/145 |
| P92-si57 | Cfs-Ums-Uf-Um-Af-Cm-Uf- Cm-Uf-Gm-Cf-Um-Cf-Um- Af-Um-Gf-Cm-Cf-Am-Gf | 242/74 | Cms-Ufs-Gm-Gf-Cm-Af-Um-Af- Gm-Af-Gm-Cf-Am-Gf-Am-Gf- Um-Af-Am-Af-Gms-Gfs-Um | 314/146 |
| P92-si58 | Gfs-Cms-Uf-Gm-Uf-Gm-Cf- Um-Af-Gm-Cf-Am-Af-Cm- Af-Cm-Cf-Cm-Af-Am-Af | 243/75 | Ums-Ufs-Um-Gf-Gm-Gf-Um-Gf- Um-Uf-Gm-Cf-Um-Af-Gm-Cf- Am-Cf-Am-Gf-Cms-Cfs-Um | 315/147 |
| P92-si59 | Ufs-Cms-Af-Cm-Cf-Um-Af- Gm-Gf-Am-Cf-Um-Gf-Am- Cf-Um-Cf-Gm-Gf-Cm-Af | 244/76 | Ums-Gfs-Cm-Cf-Gm-Af-Gm-Uf- Cm-Af-Gm-Uf-Cm-Cf-Um-Af- Gm-Gf-Um-Gf-Ams-Ufs-Gm | 316/148 |
| P92-si60 | Afs-Cms-Uf-Gm-Af-Cm-Uf- Cm-Gf-Gm-Cf-Am-Gf-Um- Gf-Um-Gf-Cm-Af-Gm-Uf | 245/77 | Ams-Cfs-Um-Gf-Cm-Af-Cm-Af- Cm-Uf-Gm-Cf-Cm-Gf-Am-Gf- Um-Cf-Am-Gf-Ums-Cfs-Cm | 317/149 |
| P92-si61 | Ufs-Cms-Gf-Gm-Cf-Am-Gf- Um-Gf-Um-Gf-Cm-Af-Gm- Uf-Gm-Gf-Um-Gf-Cm-Af | 246/78 | Ums-Gfs-Cm-Af-Cm-Cf-Am-Cf- Um-Gf-Cm-Af-Cm-Af-Cm-Uf- Gm-Cf-Cm-Gf-Ams-Gfs-Um | 318/150 |
| P92-si62 | Gfs-Gms-Cf-Am-Gf-Um-Gf- Um-Gf-Cm-Af-Gm-Uf-Gm- Gf-Um-Gf-Cm-Af-Um-Gf | 247/79 | Cms-Afs-Um-Gf-Cm-Af-Cm-Cf- Am-Cf-Um-Gf-Cm-Af-Cm-Af- Cm-Uf-Gm-Cf-Cms-Gfs-Am | 319/151 |
| P92-si63 | Gfs-Cms-Af-Gm-Uf-Gm-Gf- Um-Gf-Cm-Af-Um-Gf-Cm- Af-Cm-Uf-Gm-Uf-Cm-Uf | 248/80 | Ams-Gfs-Am-Cf-Am-Gf-Um-Gf- Cm-Af-Um-Gf-Cm-Af-Cm-Cf- Am-Cf-Um-Gf-Cms-Afs-Cm | 320/152 |
| P92-si64 | Gfs-Ums-Gf-Cm-Af-Um-Gf- Cm-Af-Cm-Uf-Gm-Uf-Cm- Uf-Cm-Af-Gm-Cf-Cm-Af | 249/81 | Ums-Gfs-Gm-Cf-Um-Gf-Am-Gf- Am-Cf-Am-Gf-Um-Gf-Cm-Af- Um-Gf-Cm-Af-Cms-Cfs-Am | 321/153 |
| P92-si65 | Afs-Ams-Cf-Cm-Cf-Gm-Cf- Um-Cf-Cm-Af-Cm-Uf-Am- Cf-Cm-Cf-Gm-Gf-Cm-Af | 250/82 | Ums-Gfs-Cm-Cf-Gm-Gf-Gm-Uf- Am-Gf-Um-Gf-Gm-Af-Gm-Cf- Gm-Gf-Gm-Uf-Ums-Gfs-Gm | 322/154 |
| P92-si66 | Afs-Cms-Uf-Am-Cf-Cm-Cf- Gm-Gf-Cm-Af-Gm-Gf-Gm- Uf-Am-Cf-Am-Cf-Am-Uf | 251/83 | Ams-Ufs-Gm-Uf-Gm-Uf-Am-Cf- Cm-Cf-Um-Gf-Cm-Cf-Gm-Gf- Gm-Uf-Am-Gf-Ums-Gfs-Gm | 323/155 |
| P92-si67 | Cfs-Cms-Uf-Am-Cf-Um-Uf- Cm-Af-Cm-Af-Gm-Af-Gm- Gf-Am-Af-Gm-Af-Am-Af | 252/84 | Ums-Ufs-Um-Cf-Um-Uf-Cm-Cf- Um-Cf-Um-Gf-Um-Gf-Am-Af- Gm-Uf-Am-Gf-Gms-Gfs-Gm | 324/156 |
| P92-si68 | Cfs-Cms-Af-Gm-Gf-Am-Af- Gm-Cf-Um-Cf-Gm-Gf-Um- Gf-Am-Gf-Um-Gf-Am-Uf | 253/85 | Ams-Ufs-Cm-Af-Cm-Uf-Cm-Af- Cm-Cf-Gm-Af-Gm-Cf-Um-Uf- Cm-Cf-Um-Gf-Gms-Ufs-Cm | 325/157 |
| P92-si69 | Afs-Gms-Af-Am-Cf-Gm-Af- Um-Gf-Cm-Cf-Um-Gf-Cm- Af-Gm-Gf-Cm-Af-Um-Gf | 254/86 | Cms-Afs-Um-Gf-Cm-Cf-Um-Gf- Cm-Af-Gm-Gf-Cm-Af-Um-Cf- Gm-Uf-Um-Cf-Ums-Gfs-Cm | 326/158 |
| P92-si70 | Ufs-Gms-Cf-Cm-Uf-Gm-Cf- Am-Gf-Gm-Cf-Am-Uf-Gm- Gf-Am-Af-Cm-Uf-Um-Uf | 255/87 | Ams-Afs-Am-Gf-Um-Uf-Cm-Cf- Am-Uf-Gm-Cf-Cm-Uf-Gm-Cf- Am-Gf-Gm-Cf-Ams-Ufs-Cm | 327/159 |
| P92-si71 | Cfs-Gms-Uf-Um-Af-Um-Cf- Am-Cf-Cm-Cf-Am-Gf-Gm- Cf-Cm-Uf-Gm-Af-Um-Uf | 256/88 | Ams-Afs-Um-Cf-Am-Gf-Gm-Cf- Cm-Uf-Gm-Gf-Gm-Uf-Gm-Af- Um-Af-Am-Cf-Gms-Gfs-Am | 328/160 |
| P92-si72 | Cfs-Ams-Cf-Cm-Cf-Am-Gf- Gm-Cf-Cm-Uf-Gm-Af-Um- Uf-Cm-Af-Cm-Uf-Gm-Gf | 257/89 | Cms-Cfs-Am-Gf-Um-Gf-Am-Af- Um-Cf-Am-Gf-Gm-Cf-Cm-Uf- Gm-Gf-Gm-Uf-Gms-Afs-Um | 329/161 |
| P92-si74 | Cfs-Gms-Gf-Am-Gf-Am-Uf- Gm-Cf-Um-Uf-Cm-Uf-Am- Af-Gm-Gf-Cm-Af-Um-Gf | 258/90 | Cms-Afs-Um-Gf-Cm-Cf-Um-Uf- Am-Gf-Am-Af-Gm-Cf-Am-Uf- Cm-Uf-Cm-Cf-Gms-Cfs-Cm | 330/162 |
| P92-si75 | Afs-Gms-Af-Um-Gf-Cm-Uf- Um-Cf-Um-Af-Am-Gf-Gm- Cf-Am-Uf-Gm-Gf-Um-Cf | 259/91 | Gms-Afs-Cm-Cf-Am-Uf-Gm-Cf- Cm-Uf-Um-Af-Gm-Af-Am-Gf- Cm-Af-Um-Cf-Ums-Cfs-Cm | 331/163 |
| P92-si76 | Afs-Cms-Af-Am-Cf-Um-Gf- Um-Cf-Cm-Cf-Um-Cf-Cm- Uf-Um-Gf-Am-Gf-Cm-Af | 260/92 | Ums-Gfs-Cm-Uf-Cm-Af-Am-Gf- Gm-Af-Gm-Gf-Gm-Af-Cm-Af- Gm-Uf-Um-Gf-Ums-Ufs-Gm | 332/164 |
| P92-si77 | Cfs-Ams-Cf-Cm-Cf-Am-Af- Gm-Cf-Am-Af-Gm-Cf-Am- Gf-Am-Cf-Am-Uf-Um-Uf | 261/93 | Ams-Afs-Am-Uf-Gm-Uf-Cm-Uf- Gm-Cf-Um-Uf-Gm-Cf-Um-Uf- Gm-Gf-Gm-Uf-Gms-Gfs-Gm | 333/165 |
| P92-si78 | Cfs-Ams-Af-Gm-Cf-Am-Af- Gm-Cf-Am-Gf-Am-Cf-Am- Uf-Um-Uf-Am-Uf-Cm-Uf | 262/94 | Ams-Gfs-Am-Uf-Am-Af-Am-Uf- Gm-Uf-Cm-Uf-Gm-Cf-Um-Uf- Gm-Cf-Um-Uf-Gms-Gfs-Gm | 334/166 |
| P92-si79 | Ufs-Ums-Uf-Gm-Gf-Gm-Uf- Cm-Uf-Gm-Uf-Cm-Cf-Um- Cf-Um-Cf-Um-Gf-Um-Uf | 263/95 | Ams-Afs-Cm-Af-Gm-Af-Gm-Af- Gm-Gf-Am-Cf-Am-Gf-Am-Cf- Cm-Cf-Am-Af-Ams-Afs-Gm | 335/167 |
| P92-si80 | Gfs-Ums-Cf-Um-Gf-Um-Cf- Cm-Uf-Cm-Uf-Cm-Uf-Gm- Uf-Um-Gf-Cm-Cf-Um-Uf | 264/96 | Ams-Afs-Gm-Gf-Cm-Af-Am-Cf- Am-Gf-Am-Gf-Am-Gf-Gm-Af- Cm-Af-Gm-Af-Cms-Cfs-Cm | 336/168 |

### Example 2: Inhibitory effect of alternately modified sequences on PCSK9 gene expression

The siRNA sequences alternately modified with 2'-OMe and 2'-F synthesized in Example 1 were transfected into HELA cells via lipid nanoparticles (LNPs). The inhibitory effects of these sequences on PCSK9 gene expression were then evaluated using qPCR.

### 1. Experimental materials

Test sample: the alternately modified siRNA sequence P92-si1-84 listed in Table 2 (synthesized in Example 1).

Cell line: HELA cells.

Solvents: sterile nuclease-free water, Gibco Opti-MEM.

### 2. Experimental methods

The inhibitory effects of the test samples on PCSK9 mRNA expression in HELA cells were evaluated by qRT-PCR.

### 2.1 Cell culture

Subcultured HELA cells in the logarithmic growth phase were cultured in RPMI 1640 culture medium containing 10% fetal bovine serum (supplemented with 100× penicillin and streptomycin at 10 µL/mL), and incubated in a cell incubator at 37°C with 5% CO₂. The medium was exchanged once daily. The cells were digested with 0.25% trypsin, followed by centrifugation at 1000 rpm for 5 minutes. The supernatant was discarded, and fresh medium was added for continued culture.

### 2.2 Cell transfection

Preparation of transfection mixture: Lipofectamine RNAiMAX was mixed with Opti-MEM at a volume ratio of 1.5:98.5 and vortexed thoroughly.

Preparation of transfection reagent: 60 µL of siRNA solution (diluted in Opti-MEM) was mixed with 60 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 60 µL of transfection mixture was added to 60 µL of Opti-MEM, vortexed thoroughly, followed by incubation at room temperature for 15 minutes.

The prepared transfection reagent was added to a 24-well cell culture plate (100 µL per well), resulting in a final siRNA concentration of 1 nM per well. Subsequently, 500 µL of cell suspension (6 × 10⁴ cells per mL) was added to each well. After cross-mixing, the plate was incubated in a cell incubator at 37°C with 5% CO₂ for 40 hours.

### 2.3 PCSK9 mRNA analysis

1) RNA extraction
   a. The culture medium was removed from a 12-well plate. 0.5 mL of 1× PBS was then added to each well to wash the cells, and the PBS was discarded. 0.5 mL of TRIzol reagent was added to each well, followed by pipetting to ensure complete lysis of the cells. The lysate was transferred to a 1.5 mL RNase-free EP tube, followed by incubation at room temperature for 5 minutes.
   b. 0.1 mL of chloroform was added to each tube, shaken vigorously for 15 seconds, followed by incubation at room temperature for 5 minutes. After centrifugation at 12000 × g for 15 minutes at 4°C, 200 µL of the supernatant was transferred to a new EP tube.
   c. An equal volume of isopropanol was added to the tube, which was gently mixed by inversion, followed by incubation at -20°C for 10 minutes. After centrifugation at 12000 × g for 15 minutes at 4°C, the supernatant was discarded.
   d. 0.5 mL of 75% ethanol was added to gently wash the RNA pellet, followed by centrifugation at 12000 × g for 5 minutes at 4°C, and the supernatant was discarded. The washing step was repeated once, followed by centrifugation at 12000 × g for 1 minute at 4°C, and residual ethanol was thoroughly removed using a micropipette tip.
   e. The residual ethanol was air-dried at room temperature for 2 to 3 minutes, followed by addition of 40 µL of RNase-free ddH₂O for dissolution.
2) Measurement of RNA concentration
   RNA concentration was measured using a NanoDrop spectrophotometer, using 2 µL of RNase-free ddH₂O as a blank control and 2 µL of RNA sample for each measurement. The sample concentration was recorded.
3) Quantification of PCSK9 mRNA

In a 15 mL centrifuge tube, all components except primers and templates were added in 7.5 × 84 = 630 aliquots (labeled as A).

A total of 84 1.5 mL EP tubes were labeled. 77 µL of A and 420 ng of total RNA were added in 7 aliquots per tube, and mixed well for later use (labeled as B).

The forward and reverse primers for both the internal reference gene GAPDH and the target gene PCSK9 were mixed well for later use (labeled as C).

11 µL of B and 1 µL of C were added to each well of a PCR plate, which was sealed, then centrifuged at 3000 rpm for 1 minute, and subjected to instrumental analysis.

*The step was performed on ice under low-temperature conditions.

The plate was placed into the qPCR instrument and run according to the following program:
Reverse transcription: 55°C for 15 minutes;
Pre-denaturation: 95°C for 30 seconds;
Cycling: 95°C for 10 seconds; 60°C for 35 seconds; 40 cycles;
Melting curve: 95°C for 15 seconds; 60°C for 60 seconds; 95°C for 15 seconds.

Total run time: approximately 2 hours. After analysis of the experimental results, 2^{-ΔΔCt} was calculated.

### 2.4 Data processing

PCSK9 mRNA expression rate (%) was calculated using the following formula: 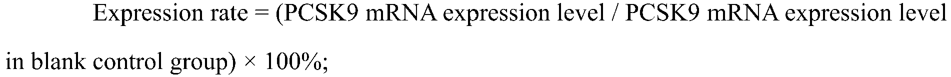 Expression rate = (PCSK9 mRNA expression level / PCSK9 mRNA expression level in blank control group) × 100%;$Inhibition rate of PCSK 9 gene expression = 100 % - expression rate \left(%\right) .$

### 3. Experimental results

In this example, the inhibition rate represents the average of four independent experiments. The inhibition rates of PCSK9 mRNA expression in HELA cells by these sequences are shown in Tables 4 to 6.

The experimental results demonstrated that certain sequences with alternating 2'-OMe and 2'-F modifications, including P92-si5, P92-si51, P92-si81, P92-si82, P92-si84, P92-si3, P92-si8, P92-si9, P92-si21, P92-si31, P92-si73, and P92-si83 (see Table 3 for specific sequences), exhibited significant inhibitory effects on PCSK9 mRNA expression in HELA cells, with inhibition rates exceeding 50%. Specifically, P92-si5, P92-si81, P92-si82, P92-si3, P92-si8, and P92-si31 exhibited inhibition rates exceeding 70%.

The other sequences exhibited relatively weak inhibitory effects on PCSK9 mRNA expression, with inhibition rates below 50%.

**Table 3: siRNA sequences with significant inhibitory effects on PCSK9 gene expression**

| **Sequence ID** | **Sense sequence 5'-3'** | **SEQ ID NO:/corr. basic SEQ ID NO:** | **Antisense sequence 5'-3'** | **SEQ ID NO:/corr. basic SEQ ID NO:** |
|---|---|---|---|---|
| P92-si5 | Afs-Ams-Gf-Am-Uf-Cm-Cf- Um-Gf-Cm-Af-Um-Gf-Um- Cf-Um-Uf-Cm-Cf-Am-Uf | 169/1 | Ams-Ufs-Gm-Gf-Am-Af-Gm-Af- Cm-Af-Um-Gf-Cm-Af-Gm-Gf-Am- Uf-Cm-Uf-Ums-Gfs-Gm | 181/13 |
| P92-si51 | Ufs-Gms-Gf-Am-Gf-Gm-Cf- Um-Uf-Am-Gf-Cm-Uf-Um- Uf-Cm-Uf-Gm-Gf-Am-Uf | 170/2 | Ams-Ufs-Cm-Cf-Am-Gf-Am-Af- Am-Gf-Cm-Uf-Am-Af-Gm-Cf-Cm- Uf-Cm-Cf-Ams-Ufs-Um | 182/14 |
| P92-si81 | Cfs-Ums-Uf-Um-Uf-Gm-Uf- Am-Af-Cm-Uf-Um-Gf-Am- Af-Gm-Af-Um-Af-Um-Uf | 171/3 | Ams-Afs-Um-Af-Um-Cf-Um-Uf- Cm-Af-Am-Gf-Um-Uf-Am-Cf-Am- Af-Am-Af-Gms-Cfs-Am | 183/15 |
| P92-si82 | Gfs-Ams-Af-Gm-Af-Um-Af- Um-Uf-Um-Af-Um-Uf-Cm- Uf-Gm-Gf-Gm-Uf-Um-Uf | 172/4 | Ams-Afs-Am-Cf-Cm-Cf-Am-Gf- Am-Af-Um-Af-Am-Af-Um-Af-Um- Cf-Um-Uf-Cms-Afs-Am | 184/16 |
| P92-si84 | Gfs-Ums-Uf-Um-Uf-Gm-Uf- Am-Gf-Cm-Af-Um-Uf-Um- Uf-Um-Af-Um-Uf-Am-Af | 173/5 | Ums-Ufs-Am-Af-Um-Af-Am-Af- Am-Af-Um-Gf-Cm-Uf-Am-Cf-Am- Af-Am-Af-Cms-Cfs-Cm | 185/17 |
| P92-si3 | Cfs-Ams-Cf-Cm-Af-Am-Gf- Am-Uf-Cm-Cf-Um-Gf-Cm- Af-Um-Gf-Um-Cf-Um-Uf | 174/6 | Ams-Afs-Gm-Af-Cm-Af-Um-Gf- Cm-Af-Gm-Gf-Am-Uf-Cm-Uf-Um- Gf-Gm-Uf-Gms-Afs-Gm | 186/18 |
| P92-si8 | Cfs-Ums-Cf-Am-Uf-Am-Gf- Gm-Cf-Cm-Uf-Gm-Gf-Am- Gf-Um-Uf-Um-Af-Um-Uf | 175/7 | Ams-Afs-Um-Af-Am-Af-Cm-Uf- Cm-Cf-Am-Gf-Gm-Cf-Cm-Uf-Am- Uf-Gm-Af-Gms-Gfs-Gm | 187/19 |
| P92-si9 | Gfs-Gms-Cf-Cm-Uf-Gm-Gf- Am-Gf-Um-Uf-Um-Af-Um- Uf-Cm-Gf-Gm-Af-Am-Af | 176/8 | Ums-Ufs-Um-Cf-Cm-Gf-Am-Af- Um-Af-Am-Af-Cm-Uf-Cm-Cf-Am- Gf-Gm-Cf-Cms-Ufs-Am | 188/20 |
| P92-si21 | Cfs-Ams-Gf-Cm-Af-Cm-Cf- Um-Gf-Cm-Uf-Um-Uf-Gm- Uf-Gm-Uf-Cm-Af-Cm-Af | 177/9 | Ums-Gfs-Um-Gf-Am-Cf-Am-Cf- Am-Af-Am-Gf-Cm-Af-Gm-Gf-Um- Gf-Cm-Uf-Gms-Cfs-Am | 189/21 |
| P92-si31 | Gfs-Gms-Uf-Cm-Uf-Gm-Gf- Am-Af-Um-Gf-Cm-Af-Am- Af-Gm-Uf-Cm-Af-Am-Gf | 178/10 | Cms-Ufs-Um-Gf-Am-Cf-Um-Uf- Um-Gf-Cm-Af-Um-Uf-Cm-Cf-Am- Gf-Am-Cf-Cms-Ufs-Gm | 190/22 |
| P92-si73 | Gfs-Cms-Gf-Gm-Af-Gm-Af- Um-Gf-Cm-Uf-Um-Cf-Um- Af-Am-Gf-Gm-Cf-Am-Uf | 179/11 | Ams-Ufs-Gm-Cf-Cm-Uf-Um-Af- Gm-Af-Am-Gf-Cm-Af-Um-Cf-Um- Cf-Cm-Gf-Cms-Cfs-Am | 191/23 |
| P92-si83 | Gfs-Gms-Gf-Um-Uf-Um-Uf- Gm-Uf-Am-Gf-Cm-Af-Um- Uf-Um-Uf-Um-Af-Um-Uf | 180/12 | Ams-Afs-Um-Af-Am-Af-Am-Af- Um-Gf-Cm-Uf-Am-Cf-Am-Af-Am- Af-Cm-Cf-Cms-Afs-Gm | 192/24 |

The inhibition rates of PCSK9 mRNA expression in HELA cells by these sequences are shown in Tables 4 to 6.
**(1) Among the 84 designed sequences, 12 sequences exhibited significant inhibitory effects on PCSK9 gene expression, with inhibition rates exceeding 50%, including P92-si5, P92-si51, P92-si81, P92-si82, P92-si84, P92-si3, P92-si8, P92-si9, P92-si21, P92-si31, P92-si73, and P92-si83.Specifically, P92-si5, P92-si81, P92-si82, P92-si3, P92-si8, and P92-si31 exhibited inhibition rates exceeding 70%.**

**Table 4: Alternately modified sequences with significant inhibitory effects on PCSK9 gene expression (inhibition rate > 50%)**

| **Basic SEQ ID NO:** | **Sequence ID** | **Inhibition rate (%)** | **Basic SEQ ID NO:** | **Sequence ID** | **Inhibition rate (%)** |
|---|---|---|---|---|---|
| 5 | P92-si5 | 72.7 | 8 | P92-si8 | 72.0 |
| 51 | P92-si51 | 61.4 | 9 | P92-si9 | 63.9 |
| 81 | P92-si81 | 71.8 | 21 | P92-si21 | 62.8 |
| 82 | P92-si82 | 75.3 | 31 | P92-si31 | 70.4 |
| 84 | P92-si84 | 66.2 | 73 | P92-si73 | 60.6 |
| 3 | P92-si3 | 70.9 | 83 | P92-si83 | 58.8 |

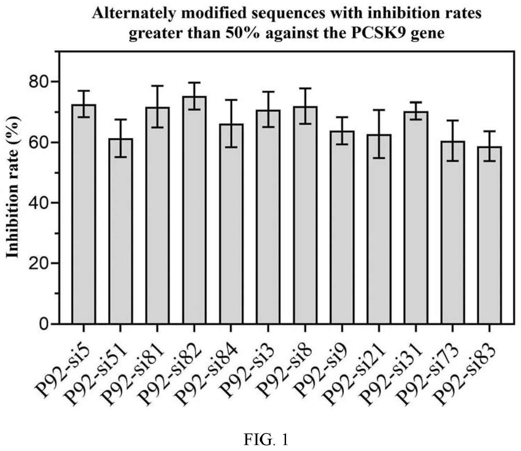

As shown in Table 4, a total of 12 alternately modified sequences, including P92-si5, P92-si51, P92-si81, P92-si82, P92-si84, P92-si3, P92-si8, P92-si9, P92-si21, P92-si31, P92-si73, and P92-si83 (see Table 3 for specific sequences), exhibited significant inhibitory effects on PCSK9 mRNA expression, with inhibition rates exceeding 50%. Specifically, P92-si5, P92-si81, P92-si82, P92-si3, P92-si8, and P92-si31 exhibited inhibition rates exceeding 70% (FIG. 1). These 12 sequences can serve as candidate sequences.

**(2) A total of 26 sequences exhibited inhibition rates of 30% to 50% on PCSK9 gene expression. For example, the inhibition rates of P92-si6 and P92-si7 were 38.3% and 35.7%, respectively.**

**Table 5: Alternately modified sequences with inhibition rates of 30% to 50% on PCSK9 gene expression**

| **Basic SEQ ID NO:** | **Sequence ID** | **Inhibition rate (%)** | **Basic SEQ ID NO:** | **Sequence ID** | **Inhibition rate (%)** |
|---|---|---|---|---|---|
| 6 | P92-si6 | 38.3 | 47 | P92-si47 | 31.7 |
| 7 | P92-si7 | 35.7 | 48 | P92-si48 | 40.9 |
| 11 | P92-si11 | 33.2 | 56 | P92-si56 | 30.3 |
| 15 | P92-si15 | 31.6 | 59 | P92-si59 | 39.8 |
| 17 | P92-si17 | 32.2 | 61 | P92-si61 | 37.4 |
| 18 | P92-si18 | 48.2 | 63 | P92-si63 | 33.6 |
| 19 | P92-si19 | 35.1 | 65 | P92-si65 | 38.2 |
| 25 | P92-si25 | 48.1 | 67 | P92-si67 | 35.2 |
| 30 | P92-si30 | 31.0 | 70 | P92-si70 | 46.4 |
| 32 | P92-si32 | 30.1 | 71 | P92-si71 | 32.9 |
| 33 | P92-si33 | 47.4 | 75 | P92-si75 | 40.7 |
| 37 | P92-si37 | 37.0 | 78 | P92-si78 | 34.1 |
| 40 | P92-si40 | 46.0 | 80 | P92-si80 | 42.1 |

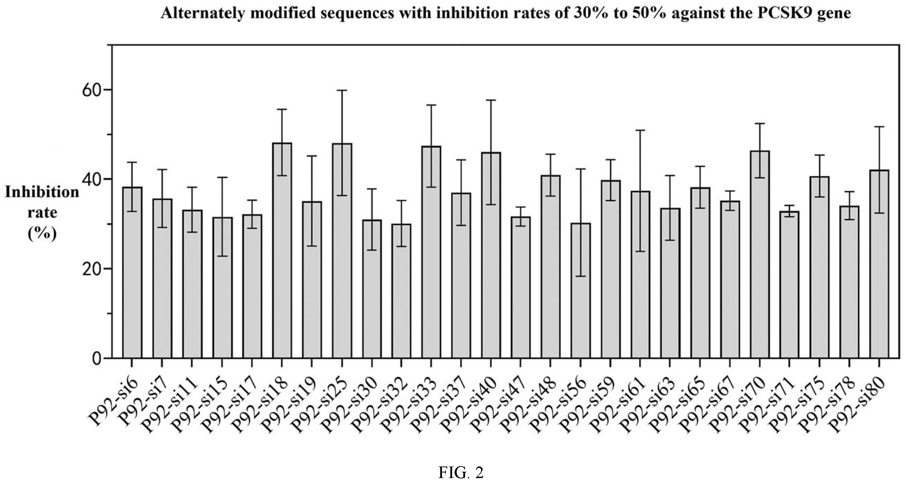

The 26 sequences listed in Table 5 exhibited relatively weak inhibitory effects on PCSK9 gene expression, with inhibition rates below 50%, ranging from 30% to 50%. For example, the inhibition rates of P92-si6 and P92-si7 were 38.3% and 35.7%, respectively (FIG. 2).

**(3) A total of 46 sequences exhibited inhibition rates below 30% on PCSK9 gene expression. For example, the inhibition rates of P92-si1 and P92-si20 were only 9.2% and 6.0%, respectively.**

**Table 6: Alternately modified sequences with inhibition rates below 30% on PCSK9 gene expression**

| **Basic SEQ ID NO:** | **Sequence ID** | **Inhibition rate (%)** | **Basic SEQ ID NO:** | **Sequence ID** | **Inhibition rate (%)** |
|---|---|---|---|---|---|
| 1 | P92-si1 | 9.2 | 43 | P92-si43 | 20.4 |
| 2 | P92-si2 | 25.0 | 44 | P92-si44 | 15.7 |
| 4 | P92-si4 | 10.9 | 45 | P92-si45 | 29.7 |
| 10 | P92-si10 | 10.8 | 46 | P92-si46 | -6.4 |
| 12 | P92-si12 | -5.7 | 49 | P92-si49 | 29.0 |
| 13 | P92-si13 | 19.4 | 50 | P92-si50 | 14.6 |
| 14 | P92-si14 | 17.1 | 52 | P92-si52 | -1.2 |
| 16 | P92-si16 | -4.6 | 53 | P92-si53 | 28.0 |
| 20 | P92-si20 | 6.0 | 54 | P92-si54 | 7.3 |
| 22 | P92-si22 | -2.8 | 55 | P92-si55 | 14.1 |
| 23 | P92-si23 | 26.1 | 57 | P92-si57 | 18.2 |
| 24 | P92-si24 | 2.9 | 58 | P92-si58 | 25.4 |
| 26 | P92-si26 | -2.5 | 60 | P92-si60 | -4.6 |
| 27 | P92-si27 | 21.6 | 62 | P92-si62 | 4.7 |
| 28 | P92-si28 | 11.1 | 64 | P92-si64 | -7.9 |
| 29 | P92-si29 | -6.0 | 66 | P92-si66 | 22.9 |
| 34 | P92-si34 | -5.4 | 68 | P92-si68 | 11.8 |
| 35 | P92-si35 | 15.2 | 69 | P92-si69 | -6.0 |
| 36 | P92-si36 | -3.1 | 72 | P92-si72 | -3.9 |
| 38 | P92-si38 | 5.5 | 74 | P92-si74 | -1.6 |
| 39 | P92-si39 | 26.8 | 76 | P92-si76 | 5.2 |
| 41 | P92-si41 | 10.7 | 77 | P92-si77 | 5.7 |
| 42 | P92-si42 | 29.7 | 79 | P92-si79 | 25.4 |

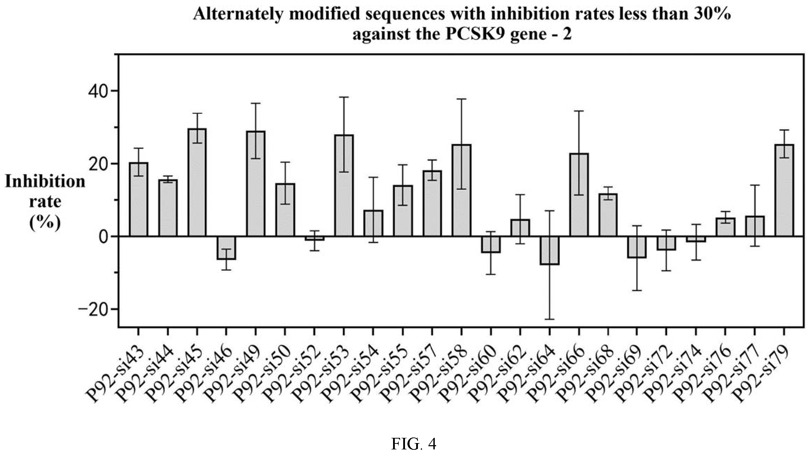

The 46 sequences listed in Table 6 exhibited very weak inhibitory effects on PCSK9 mRNA expression, with inhibition rates below 30%. For example, the inhibition rates of P92-si1 and P92-si20 were only 9.2% and 6.0%, respectively (FIG. 4).

**(4) siRNAs with similar sequences exhibited significant differences in activity. For example, the inhibition rate of P92-si5 was 61.8% higher than that of P92-si4, indicating a significant improvement.**

A comparison of the inhibitory effects of certain siRNAs with similar sequences on PCSK9 mRNA expression is shown in Table 7.

**Table 7: Comparison of inhibition rates of PCSK9 gene expression by siRNAs with similar sequences**

| **Group of similar sequences** | **Basic SEQ ID NO:** | **Sequence ID** | **Sense strand of corr. basic sequence** | **Corr. basic SEQ ID NO:** | **Antisense strand of corr. basic sequence** | **Corr. basic SEQ ID NO:** | **Inhibition rate (%)** |
|---|---|---|---|---|---|---|---|
| A | 4 | P92-si4 | CAAGAUCCUGC AUGUCUUCCA | 27 | UGGAAGACAUGCA GGAUCUUGGU | 99 | 10.9 |
| | 5 | P92-si5 | AAGAUCCUGCA UGUCUUCCAU | 1 | AUGGAAGACAUGC AGGAUCUUGG | 13 | 72.7 |
| B | 50 | P92-si50 | GAUUAAUGGAG GCUUAGCUUU | 68 | AAAGCUAAGCCUC CAUUAAUCAG | 140 | 14.6 |
| | 51 | P92-si51 | UGGAGGCUUAG CUUUCUGGAU | 2 | AUCCAGAAAGCUA AGCCUCCAUU | 14 | 61.4 |
| C | 2 | P92-si2 | AUACCUCACCAA GAUCCUGCA | 26 | UGCAGGAUCUUGG UGAGGUAUCC | 98 | 25.0 |
| | 3 | P92-si3 | CACCAAGAUCC UGCAUGUCUU | 6 | AAGACAUGCAGGA UCUUGGUGAG | 18 | 70.9 |
| D | 9 | P92-si9 | GGCCUGGAGUU UAUUCGGAAA | 8 | UUUCCGAAUAAAC UCCAGGCCUA | 20 | 63.9 |
| | 10 | P92-si10 | CCUGGAGUUUA UUCGGAAAAG | 30 | CUUUUCCGAAUAA ACUCCAGGCC | 102 | 10.8 |
| E | 21 | P92-si21 | CAGCACCUGCU UUGUGUCACA | 9 | UGUGACACAAAGC AGGUGCUGCA | 21 | 62.8 |
| | 22 | P92-si22 | GCACCUGCUUU GUGUCACAGA | 41 | UCUGUGACACAAA GCAGGUGCUG | 113 | -2.8 |
| F | 30 | P92-si30 | AGGUCUGGAAU GCAAAGUCAA | 49 | UUGACUUUGCAUU CCAGACCUGG | 121 | 31.0 |
| | 31 | P92-si31 | GGUCUGGAAUG CAAAGUCAAG | 10 | CUUGACUUUGCAU UCCAGACCUG | 22 | 70.4 |
| G | 73 | P92-si73 | GCGGAGAUGCU UCUAAGGCAU | 11 | AUGCCUUAGAAGC AUCUCCGCCA | 23 | 60.6 |
| | 74 | P92-si74 | CGGAGAUGCUU CUAAGGCAUG | 90 | CAUGCCUUAGAAG CAUCUCCGCC | 162 | -1.6 |

As shown in Table 7, certain siRNAs with similar sequences exhibited vastly different inhibitory effects on PCSK9 mRNA expression.

Compared with basic sequence 5, basic sequence 4 differs only in one base at the terminal position. For the sense strand, basic sequence 4 contains an additional C at the 5' end, while basic sequence 5 contains an additional U at the 3' end, with all other bases being identical. For the antisense strand, basic sequence 4 contains an additional U at the 3' end, while basic sequence 5 contains an additional A at the 5' end, with all other bases being identical. However, the inhibition rate of the alternately modified sequence P92-si5 was 61.8% higher than that of P92-si4, indicating a significant improvement.

Compared with basic sequence 51, basic sequence 50 differs only in six bases at the terminal positions. For the sense strand, the 5' end of basic sequence 50 is GAUUAA, while the 3' end of basic sequence 51 is CUGGAU, with all other bases being identical. For the antisense strand, the 3' end of basic sequence 50 is AAUCAG, while the 5' end of basic sequence 51 is AUCCAG, with all other bases being identical. However, the inhibition rate of the alternately modified sequence P92-si51 was 46.8% higher than that of P92-si50, indicating a significant improvement.

Compared with basic sequence 3, basic sequence 2 differs only in six bases at the terminal positions. For the sense strand, the 5' end of basic sequence 2 is AUACCU, while the 3' end of basic sequence 3 is UGUCUU, with all other bases being identical. For the antisense strand, the 3' end of basic sequence 2 is GUAUCC, while the 5' end of basic sequence 3 is AAGACA, with all other bases being identical. However, the inhibition rate of the alternately modified sequence P92-si3 was 45.9% higher than that of P92-si2, indicating a significant improvement.

Compared with basic sequence 10, basic sequence 9 differs only in two bases at the terminal positions. For the sense strand, the 5' end of basic sequence 9 is GG, while the 3' end of basic sequence 10 is AG, with all other bases being identical. For the antisense strand, the 3' end of basic sequence 9 is UA, while the 5' end of basic sequence 10 is CU, with all other bases being identical. However, the inhibition rate of the alternately modified sequence P92-si9 was 53.1% higher than that of P92-si10, indicating a significant improvement.

Compared with basic sequence 22, basic sequence 21 differs only in two bases at the terminal positions. For the sense strand, the 5' end of basic sequence 21 is CA, while the 3' end of basic sequence 22 is GA, with all other bases being identical. For the antisense strand, the 3' end of basic sequence 21 is CA, while the 5' end of basic sequence 22 is UC, with all other bases being identical. However, the inhibition rate of the alternately modified sequence P92-si21 was 65.6% higher than that of P92-si22, indicating a significant improvement.

Compared with basic sequence 31, basic sequence 30 differs only in one base at the terminal position. For the sense strand, the 5' end of basic sequence 30 is A, while the 3' end of basic sequence 31 is G, with all other bases being identical. For the antisense strand, the 3' end of basic sequence 30 is G, while the 5' end of basic sequence 31 is C, with all other bases being identical. However, the inhibition rate of the alternately modified sequence P92-si31 was 39.4% higher than that of P92-si30, indicating a significant improvement.

Compared with basic sequence 74, basic sequence 73 differs only in one base at the terminal position. For the sense strand, the 5' end of basic sequence 73 is G, while the 3' end of basic sequence 74 is G, with all other bases being identical. For the antisense strand, the 3' end of basic sequence 73 is A, while the 5' end of basic sequence 74 is C, with all other bases being identical. However, the inhibition rate of the alternately modified sequence P92-si73 was 62.2% higher than that of P92-si74, indicating a significant improvement.

Therefore, identifying oligonucleotide sequences with significant inhibitory activity from a vast number of candidate sequences designed against the PCSK9 mRNA sequence is not straightforward and requires substantial creative effort.

### Summary:

(1) Among the 84 designed sequences, 12 sequences exhibited significant inhibitory effects on PCSK9 gene expression, with inhibition rates exceeding 50%, including P92-si5, P92-si51, P92-si81, P92-si82, P92-si84, P92-si3, P92-si8, P92-si9, P92-si21, P92-si31, P92-si73, and P92-si83. Specifically, P92-si5, P92-si81, P92-si82, P92-si3, P92-si8, and P92-si31 exhibited inhibition rates exceeding 70%.
(2) A total of 26 sequences exhibited inhibition rates of 30% to 50% on PCSK9 gene expression. For example, the inhibition rates of P92-si6 and P92-si7 were 38.3% and 35.7%, respectively.
(3) A total of 46 sequences exhibited inhibition rates below 30% on PCSK9 gene expression. For example, the inhibition rates of P92-si1 and P92-si20 were only 9.2% and 6.0%, respectively.
(4) siRNAs with similar sequences exhibited significant differences in activity. For example, the inhibition rate of P92-si5 was 61.8% higher than that of P92-si4, indicating a significant improvement. Therefore, identifying oligonucleotide sequences with significant inhibitory activity from a vast number of candidate sequences designed against the PCSK9 mRNA sequence is not straightforward and requires substantial creative effort.

### Example 3: Inhibitory effect of unmodified sequences on PCSK9 gene expression

In this example, certain unmodified sequences (*i.e.*, basic sequences) corresponding to the modified sequences in Example 2 were synthesized and transfected into HELA cells via lipid nanoparticles (LNPs). The inhibitory effects of these unmodified sequences on PCSK9 gene expression were evaluated using qPCR to identify unmodified siRNA sequences with favorable inhibitory activity.

### 1. Experimental materials

### Test samples:

The unmodified siRNA sequences listed in Table 8. All sequences were synthesized using the method described in Example 1.

**Table 8: Unmodified siRNA sequences**

| **Sequence ID** | **Sense sequence (basic sequence) 5'-3'** | **SEQ ID NO:** | **Antisense sequence (basic sequence) 5'-3'** | **SEQ ID NO:** |
|---|---|---|---|---|
| si5 | AAGAUCCUGCAUGUCUUCCAU | 1 | AUGGAAGACAUGCAGGAUCUUGG | 13 |
| si51 | UGGAGGCUUAGCUUUCUGGAU | 2 | AUCCAGAAAGCUAAGCCUCCAUU | 14 |
| si81 | CUUUUGUAACUUGAAGAUAUU | 3 | AAUAUCUUCAAGUUACAAAAGCA | 15 |
| si82 | GAAGAUAUUUAUUCUGGGUUU | 4 | AAACCCAGAAUAAAUAUCUUCAA | 16 |
| si84 | GUUUUGUAGCAUUUUUAUUAA | 5 | UUAAUAAAAAUGCUACAAAACCC | 17 |
| si3 | CACCAAGAUCCUGCAUGUCUU | 6 | AAGACAUGCAGGAUCUUGGUGAG | 18 |
| si8 | CUCAUAGGCCUGGAGUUUAUU | 7 | AAUAAACUCCAGGCCUAUGAGGG | 19 |
| si9 | GGCCUGGAGUUUAUUCGGAAA | 8 | UUUCCGAAUAAACUCCAGGCCUA | 20 |
| si21 | CAGCACCUGCUUUGUGUCACA | 9 | UGUGACACAAAGCAGGUGCUGCA | 21 |
| si31 | GGUCUGGAAUGCAAAGUCAAG | 10 | CUUGACUUUGCAUUCCAGACCUG | 22 |
| si73 | GCGGAGAUGCUUCUAAGGCAU | 11 | AUGCCUUAGAAGCAUCUCCGCCA | 23 |
| si83 | GGGUUUUGUAGCAUUUUUAUU | 12 | AAUAAAAAUGCUACAAAACCCAG | 24 |
| si1 | GGGAUACCUCACCAAGAUCCU | 25 | AGGAUCUUGGUGAGGUAUCCCCG | 97 |
| si2 | AUACCUCACCAAGAUCCUGCA | 26 | UGCAGGAUCUUGGUGAGGUAUCC | 98 |
| si4 | CAAGAUCCUGCAUGUCUUCCA | 27 | UGGAAGACAUGCAGGAUCUUGGU | 99 |
| si6 | GGCUUCCUGGUGAAGAUGAGU | 28 | ACUCAUCUUCACCAGGAAGCCAG | 100 |
| si7 | GCUGGAGCUGGCCUUGAAGUU | 29 | AACUUCAAGGCCAGCUCCAGCAG | 101 |
| si10 | CCUGGAGUUUAUUCGGAAAAG | 30 | CUUUUCCGAAUAAACUCCAGGCC | 102 |
| si11 | GCCAGCUGGUCCAGCCUGUGG | 31 | CCACAGGCUGGACCAGCUGGCUU | 103 |
| si12 | ACUGGUGGUGCUGCUGCCCCU | 32 | AGGGGCAGCAGCACCACCAGUGG | 104 |
| si13 | UGCCCCUGGCGGGUGGGUACA | 33 | UGUACCCACCCGCCAGGGGCAGC | 105 |
| si14 | UGGGUACAGCCGCGUCCUCAA | 34 | UUGAGGACGCGGCUGUACCCACC | 106 |
| si15 | CGUCCUCAACGCCGCCUGCCA | 35 | UGGCAGGCGGCGUUGAGGACGCG | 107 |
| si16 | GGUCGUGCUGGUCACCGCUGC | 36 | GCAGCGGUGACCAGCACGACCCC | 108 |
| si17 | AGGACAUCAUUGGUGCCUCCA | 37 | UGGAGGCACCAAUGAUGUCCUCC | 109 |
| si18 | UCAUUGGUGCCUCCAGCGACU | 38 | AGUCGCUGGAGGCACCAAUGAUG | 110 |
| si19 | GUGCCUCCAGCGACUGCAGCA | 39 | UGCUGCAGUCGCUGGAGGCACCA | 111 |
| si20 | AGCGACUGCAGCACCUGCUUU | 40 | AAAGCAGGUGCUGCAGUCGCUGG | 112 |
| si22 | GCACCUGCUUUGUGUCACAGA | 41 | UCUGUGACACAAAGCAGGUGCUG | 113 |
| si23 | CUUUGUGUCACAGAGUGGGAC | 42 | GUCCCACUCUGUGACACAAAGCA | 114 |
| si24 | AGUGGGACAUCACAGGCUGCU | 43 | AGCAGCCUGUGAUGUCCCACUCU | 115 |
| si25 | AUCACAGGCUGCUGCCCACGU | 44 | ACGUGGGCAGCAGCCUGUGAUGU | 116 |
| si26 | UGUCUGCCGAGCCGGAGCUCA | 45 | UGAGCUCCGGCUCGGCAGACAGC | 117 |
| si27 | GGCCGAGUUGAGGCAGAGACU | 46 | AGUCUCUGCCUCAACUCGGCCAG | 118 |
| si28 | CAUCCACGCUUCCUGCUGCCA | 47 | UGGCAGCAGGAAGCGUGGAUGCU | 119 |
| si29 | CCCAGGUCUGGAAUGCAAAGU | 48 | ACUUUGCAUUCCAGACCUGGGGC | 120 |
| si30 | AGGUCUGGAAUGCAAAGUCAA | 49 | UUGACUUUGCAUUCCAGACCUGG | 121 |
| si32 | GAAUGCAAAGUCAAGGAGCAU | 50 | AUGCUCCUUGACUUUGCAUUCCA | 122 |
| si33 | CAAAGUCAAGGAGCAUGGAAU | 51 | AUUCCAUGCUCCUUGACUUUGCA | 123 |
| si34 | CCCUCAGGAGCAGGUGACCGU | 52 | ACGGUCACCUGCUCCUGAGGGGC | 124 |
| si35 | GAGGAGGGCUGGACCCUGACU | 53 | AGUCAGGGUCCAGCCCUCCUCGC | 125 |
| si36 | GCUGCAGUGCCCUCCCUGGGA | 54 | UCCCAGGGAGGGCACUGCAGCCA | 126 |
| si37 | CCUCCCUGGGACCUCCCACGU | 55 | ACGUGGGAGGUCCCAGGGAGGGC | 127 |
| si38 | CCCUGGGACCUCCCACGUCCU | 56 | AGGACGUGGGAGGUCCCAGGGAG | 128 |
| si39 | GGGCCUACGCCGUAGACAACA | 57 | UGUUGUCUACGGCGUAGGCCCCC | 129 |
| si40 | GCCGUAGACAACACGUGUGUA | 58 | UACACACGUGUUGUCUACGGCGU | 130 |
| si41 | CCGUAGACAACACGUGUGUAG | 59 | CUACACACGUGUUGUCUACGGCG | 131 |
| si42 | UAGACAACACGUGUGUAGUCA | 60 | UGACUACACACGUGUUGUCUACG | 132 |
| si43 | GUGUGUAGUCAGGAGCCGGGA | 61 | UCCCGGCUCCUGACUACACACGU | 133 |
| si44 | GAGCCGGGACGUCAGCACUAC | 62 | GUAGUGCUGACGUCCCGGCUCCU | 134 |
| si45 | UCAGCACUACAGGCAGCACCA | 63 | UGGUGCUGCCUGUAGUGCUGACG | 135 |
| si46 | UCUCCAGCUAACUGUGGAGAA | 64 | UUCUCCACAGUUAGCUGGAGAUG | 136 |
| si47 | GCUCCCUGAUUAAUGGAGGCU | 65 | AGCCUCCAUUAAUCAGGGAGCCC | 137 |
| si48 | UCCCUGAUUAAUGGAGGCUUA | 66 | UAAGCCUCCAUUAAUCAGGGAGC | 138 |
| si49 | CCUGAUUAAUGGAGGCUUAGC | 67 | GCUAAGCCUCCAUUAAUCAGGGA | 139 |
| si50 | GAUUAAUGGAGGCUUAGCUUU | 68 | AAAGCUAAGCCUCCAUUAAUCAG | 140 |
| si52 | GGUGGUCACAGGCUGUGCCUU | 69 | AAGGCACAGCCUGUGACCACCAG | 141 |
| si53 | UCACAGGCUGUGCCUUGGUUU | 70 | AAACCAAGGCACAGCCUGUGACC | 142 |
| si54 | GUGCCUUGGUUUCCUGAGCCA | 71 | UGGCUCAGGAAACCAAGGCACAG | 143 |
| si55 | AGCCACCUUUACUCUGCUCUA | 72 | UAGAGCAGAGUAAAGGUGGCUCA | 144 |
| si56 | CCACCUUUACUCUGCUCUAUG | 73 | CAUAGAGCAGAGUAAAGGUGGCU | 145 |
| si57 | CUUUACUCUGCUCUAUGCCAG | 74 | CUGGCAUAGAGCAGAGUAAAGGU | 146 |
| si58 | GCUGUGCUAGCAACACCCAAA | 75 | UUUGGGUGUUGCUAGCACAGCCU | 147 |
| si59 | UCACCUAGGACUGACUCGGCA | 76 | UGCCGAGUCAGUCCUAGGUGAUG | 148 |
| si60 | ACUGACUCGGCAGUGUGCAGU | 77 | ACUGCACACUGCCGAGUCAGUCC | 149 |
| si61 | UCGGCAGUGUGCAGUGGUGCA | 78 | UGCACCACUGCACACUGCCGAGU | 150 |
| si62 | GGCAGUGUGCAGUGGUGCAUG | 79 | CAUGCACCACUGCACACUGCCGA | 151 |
| si63 | GCAGUGGUGCAUGCACUGUCU | 80 | AGACAGUGCAUGCACCACUGCAC | 152 |
| si64 | GUGCAUGCACUGUCUCAGCCA | 81 | UGGCUGAGACAGUGCAUGCACCA | 153 |
| si65 | AACCCGCUCCACUACCCGGCA | 82 | UGCCGGGUAGUGGAGCGGGUUGG | 154 |
| si66 | ACUACCCGGCAGGGUACACAU | 83 | AUGUGUACCCUGCCGGGUAGUGG | 155 |
| si67 | CCUACUUCACAGAGGAAGAAA | 84 | UUUCUUCCUCUGUGAAGUAGGGG | 156 |
| si68 | CCAGGAAGCUCGGUGAGUGAU | 85 | AUCACUCACCGAGCUUCCUGGUC | 157 |
| si69 | AGAACGAUGCCUGCAGGCAUG | 86 | CAUGCCUGCAGGCAUCGUUCUGC | 158 |
| si70 | UGCCUGCAGGCAUGGAACUUU | 87 | AAAGUUCCAUGCCUGCAGGCAUC | 159 |
| si71 | CGUUAUCACCCAGGCCUGAUU | 88 | AAUCAGGCCUGGGUGAUAACGGA | 160 |
| si72 | CACCCAGGCCUGAUUCACUGG | 89 | CCAGUGAAUCAGGCCUGGGUGAU | 161 |
| si74 | CGGAGAUGCUUCUAAGGCAUG | 90 | CAUGCCUUAGAAGCAUCUCCGCC | 162 |
| si75 | AGAUGCUUCUAAGGCAUGGUC | 91 | GACCAUGCCUUAGAAGCAUCUCC | 163 |
| si76 | ACAACUGUCCCUCCUUGAGCA | 92 | UGCUCAAGGAGGGACAGUUGUUG | 164 |
| si77 | CACCCAAGCAAGCAGACAUUU | 93 | AAAUGUCUGCUUGCUUGGGUGGG | 165 |
| si78 | CAAGCAAGCAGACAUUUAUCU | 94 | AGAUAAAUGUCUGCUUGCUUGGG | 166 |
| si79 | UUUGGGUCUGUCCUCUCUGUU | 95 | AACAGAGAGGACAGACCCAAAAG | 167 |
| si80 | GUCUGUCCUCUCUGUUGCCUU | 96 | AAGGCAACAGAGAGGACAGACCC | 168 |

Cell line: HELA cells.

Solvents: sterile nuclease-free water, Gibco Opti-MEM.

### 2. Experimental methods

Refer to Example 2.

### 3. Experimental results

The inhibition rates of PCSK9 mRNA expression by these unmodified sequences are shown in Tables 9 and 10.

The unmodified sequences si5, si51, si81, si82, si84, si3, si8, si9, si21, si31, si73, and si83 exhibited significant inhibitory effects on PCSK9 mRNA expression in HELA cells, with inhibition rates exceeding 50%. Specifically, si5, si81, si82, si3, and si8 exhibited inhibition rates exceeding 60%.
**(1) A total of 12 unmodified sequences exhibited significant inhibitory effects on PCSK9 gene expression, with inhibition rates exceeding 50%, including si5, si51, si81, si82, si84, si3, si8, si9, si21, si31, si73, and si83. Specifically, si5, si81, si82, si3, and si8 exhibited inhibition rates exceeding 60%.**

**Table 9: Unmodified sequences with significant inhibitory effects on PCSK9 gene expression (inhibition rate > 50%)**

| **Basic SEQ ID NO:** | **Sequence ID** | **Inhibition rate (%)** | **Basic SEQ ID NO:** | **Sequence ID** | **Inhibition rate (%)** |
|---|---|---|---|---|---|
| 5 | si5 | 60.3 | 8 | si8 | 60.6 |
| 51 | si51 | 53.4 | 9 | si9 | 52.6 |
| 81 | si81 | 62.0 | 21 | si21 | 51.2 |
| 82 | si82 | 63.1 | 31 | si31 | 59.2 |
| 84 | si84 | 55.7 | 73 | si73 | 52.4 |
| 3 | si3 | 62.3 | 83 | si83 | 50.1 |

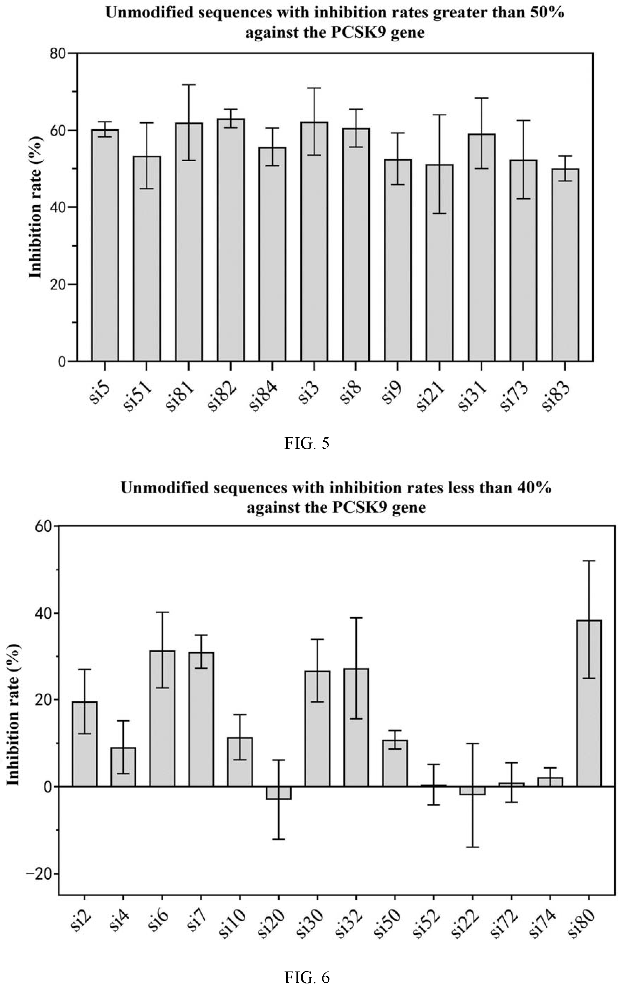

The unmodified sequences listed in Table 9, including si5, si51, si81, si82, si84, si3, si8, si9, si21, si31, si73, and si83, exhibited significant inhibitory effects on PCSK9 mRNA expression, with inhibition rates exceeding 50%. Specifically, si5, si81, si82, si3, and si8 exhibited inhibition rates exceeding 60% (FIG. 5). These 12 sequences can serve as candidate sequences.

**(2) The other unmodified sequences exhibited inhibition rates below 40% on PCSK9 gene expression. For example, the inhibition rates of si52 and si74 were only 0.5% and 2.2%, respectively.**

**Table 10: Unmodified sequences with inhibition rates below 40% on PCSK9 gene expression**

| **Basic SEQ ID NO:** | **Sequence ID** | **Inhibition rate (%)** | **Basic SEQ ID NO:** | **Sequence ID** | **Inhibition rate (%)** |
|---|---|---|---|---|---|
| 2 | si2 | 19.6 | 32 | si32 | 27.3 |
| 4 | si4 | 9.1 | 50 | si50 | 10.8 |
| 6 | si6 | 31.5 | 52 | si52 | 0.5 |
| 7 | si7 | 31.1 | 22 | si22 | -2.0 |
| 10 | si10 | 11.4 | 72 | si72 | 1.0 |
| 20 | si20 | -3.0 | 74 | si74 | 2.2 |
| 30 | si30 | 26.7 | 80 | si80 | 38.5 |

The sequences listed in Table 10 exhibited very weak inhibitory effects on PCSK9 mRNA expression, with inhibition rates below 40%. For example, the inhibition rates of si52 and si74 were only 0.5% and 2.2%, respectively (FIG. 6).

**(3) siRNAs with similar sequences exhibited significant differences in activity. For example, the inhibition rate of basic sequence 5 was 51.2% higher than that of basic sequence 4, indicating a significant improvement.**

A comparison of the inhibitory effects of certain siRNAs with similar sequences on PCSK9 mRNA expression is shown in Table 11.

**Table 11: Comparison of inhibition rates of PCSK9 gene expression by unmodified sequences with similar sequences**

| **Group of similar sequences** | **Basic SEQ ID NO:** | **Sequence ID** | **Sense strand** | **Basic SEQ ID NO:** | **Antisense strand** | **Basic SEQ ID NO:** | **Inhibition rate (%)** |
|---|---|---|---|---|---|---|---|
| A | 4 | si4 | CAAGAUCCUGC AUGUCUUCCA | 27 | UGGAAGACAUGC AGGAUCUUGGU | 99 | 9.1 |
| | 5 | si5 | AAGAUCCUGCA UGUCUUCCAU | 1 | AUGGAAGACAUG CAGGAUCUUGG | 13 | 60.3 |
| B | 50 | si50 | GAUUAAUGGAG GCUUAGCUUU | 68 | AAAGCUAAGCCU CCAUUAAUCAG | 140 | 10.8 |
| | 51 | si51 | UGGAGGCUUAG CUUUCUGGAU | 2 | AUCCAGAAAGCUA AGCCUCCAUU | 14 | 53.4 |
| C | 2 | si2 | AUACCUCACCAA GAUCCUGCA | 26 | UGCAGGAUCUUG GUGAGGUAUCC | 98 | 19.6 |
| | 3 | si3 | CACCAAGAUCC UGCAUGUCUU | 6 | AAGACAUGCAGG AUCUUGGUGAG | 18 | 62.3 |
| D | 9 | si9 | GGCCUGGAGUU UAUUCGGAAA | 8 | UUUCCGAAUAAA CUCCAGGCCUA | 20 | 52.6 |
| | 10 | si10 | CCUGGAGUUUA UUCGGAAAAG | 30 | CUUUUCCGAAUAA ACUCCAGGCC | 102 | 11.4 |
| E | 21 | si21 | CAGCACCUGCU UUGUGUCACA | 9 | UGUGACACAAAG CAGGUGCUGCA | 21 | 51.2 |
| | 22 | si22 | GCACCUGCUUU GUGUCACAGA | 41 | UCUGUGACACAA AGCAGGUGCUG | 113 | -2.0 |
| F | 30 | si30 | AGGUCUGGAAU GCAAAGUCAA | 49 | UUGACUUUGCAU UCCAGACCUGG | 121 | 26.7 |
| | 31 | si31 | GGUCUGGAAUG CAAAGUCAAG | 10 | CUUGACUUUGCA UUCCAGACCUG | 22 | 59.2 |
| G | 73 | si73 | GCGGAGAUGCU UCUAAGGCAU | 11 | AUGCCUUAGAAG CAUCUCCGCCA | 23 | 52.4 |
| | 74 | si74 | CGGAGAUGCUU CUAAGGCAUG | 90 | CAUGCCUUAGAA GCAUCUCCGCC | 162 | 2.2 |

As shown in Table 11, certain siRNAs with similar sequences exhibited vastly different inhibitory effects on PCSK9 mRNA expression.

Compared with basic sequence 5, basic sequence 4 differs only in one base at the terminal position. For the sense strand, basic sequence 4 contains an additional C at the 5' end, while basic sequence 5 contains an additional U at the 3' end, with all other bases being identical. For the antisense strand, basic sequence 4 contains an additional U at the 3' end, while basic sequence 5 contains an additional A at the 5' end, with all other bases being identical. However, the inhibition rate of basic sequence 5 was 51.2% higher than that of basic sequence 4, indicating a significant improvement.

Compared with basic sequence 51, basic sequence 50 differs only in six bases at the terminal positions. For the sense strand, the 5' end of basic sequence 50 is GAUUAA, while the 3' end of basic sequence 51 is CUGGAU, with all other bases being identical. For the antisense strand, the 3' end of basic sequence 50 is AAUCAG, while the 5' end of basic sequence 51 is AUCCAG, with all other bases being identical. However, the inhibition rate of basic sequence 51 was 42.6% higher than that of basic sequence 50, indicating a significant improvement.

Compared with basic sequence 3, basic sequence 2 differs only in six bases at the terminal positions. For the sense strand, the 5' end of basic sequence 2 is AUACCU, while the 3' end of basic sequence 3 is UGUCUU, with all other bases being identical. For the antisense strand, the 3' end of basic sequence 2 is GUAUCC, while the 5' end of basic sequence 3 is AAGACA, with all other bases being identical. However, the inhibition rate of basic sequence 3 was 42.7% higher than that of basic sequence 2, indicating a significant improvement.

Compared with basic sequence 10, basic sequence 9 differs only in two bases at the terminal positions. For the sense strand, the 5' end of basic sequence 9 is GG, while the 3' end of basic sequence 10 is AG, with all other bases being identical. For the antisense strand, the 3' end of basic sequence 9 is UA, while the 5' end of basic sequence 10 is CU, with all other bases being identical. However, the inhibition rate of basic sequence 9 was 41.2% higher than that of basic sequence 10, indicating a significant improvement.

Compared with basic sequence 22, basic sequence 21 differs only in two bases at the terminal positions. For the sense strand, the 5' end of basic sequence 21 is CA, while the 3' end of basic sequence 22 is GA, with all other bases being identical. For the antisense strand, the 3' end of basic sequence 21 is CA, while the 5' end of basic sequence 22 is UC, with all other bases being identical. However, the inhibition rate of basic sequence 21 was 53.2% higher than that of basic sequence 22, indicating a significant improvement.

Compared with basic sequence 31, basic sequence 30 differs only in one base at the terminal position. For the sense strand, the 5' end of basic sequence 30 is A, while the 3' end of basic sequence 31 is G, with all other bases being identical. For the antisense strand, the 3' end of basic sequence 30 is G, while the 5' end of basic sequence 31 is C, with all other bases being identical. However, the inhibition rate of basic sequence 31 was 32.5% higher than that of basic sequence 30, indicating a significant improvement.

Compared with basic sequence 74, basic sequence 73 differs only in one base at the terminal position. For the sense strand, the 5' end of basic sequence 73 is G, while the 3' end of basic sequence 74 is G, with all other bases being identical. For the antisense strand, the 3' end of basic sequence 73 is A, while the 5' end of basic sequence 74 is C, with all other bases being identical. However, the inhibition rate of basic sequence 73 was 50.2% higher than that of basic sequence 74, indicating a significant improvement.

Therefore, identifying oligonucleotide sequences with significant inhibitory activity from a vast number of candidate sequences designed against the PCSK9 mRNA sequence is not straightforward and requires substantial creative effort.

**(4) The effects of alternating modifications on activity varied across different sequences. For some sequences, the inhibition rate was significantly improved. For example, for basic sequence 5, the alternately modified sequence exhibited a 12.4% increase in inhibition rate compared to the unmodified sequence.** F**or others, the improvement was minimal. For example, basic sequences 4, 22, and 52 exhibited little to no difference in inhibition rate between alternately modified and unmodified sequences.**

**Table 12: Comparison of inhibition rates of PCSK9 gene expression between alternately modified and unmodified sequences**

| **Basic SEQ ID NO:** | **Sequence ID** | **Unmodified inhibition rate (%)** | **Alternately modified inhibition rate (%)** | **Increase in inhibition rate (%)** |
|---|---|---|---|---|
| 5 | si5 | 60.3 | 72.7 | 12.4 |
| 51 | si51 | 53.4 | 61.4 | 8.0 |
| 81 | si81 | 62.0 | 71.8 | 9.8 |
| 82 | si82 | 63.1 | 75.3 | 12.2 |
| 84 | si84 | 55.7 | 66.2 | 10.5 |
| 3 | si3 | 62.3 | 70.9 | 8.6 |
| 8 | si8 | 60.6 | 72.0 | 11.4 |
| 9 | si9 | 52.6 | 63.9 | 11.3 |
| 21 | si21 | 51.2 | 62.8 | 11.6 |
| 31 | si31 | 59.2 | 70.4 | 11.2 |
| 73 | si73 | 52.4 | 60.6 | 8.2 |
| 83 | si83 | 50.1 | 58.8 | 8.7 |
| 2 | si2 | 19.6 | 25.0 | 5.4 |
| 4 | si4 | 9.1 | 10.9 | 1.8 |
| 6 | si6 | 31.5 | 38.3 | 6.8 |
| 7 | si7 | 31.1 | 35.7 | 4.6 |
| 10 | si10 | 11.4 | 10.8 | -0.6 |
| 20 | si20 | -3.0 | 6.0 | 9.0 |
| 22 | si22 | -2.0 | -2.8 | -0.8 |
| 30 | si30 | 26.7 | 31.0 | 4.3 |
| 32 | si32 | 27.3 | 30.1 | 2.8 |
| 50 | si50 | 10.8 | 14.6 | 3.8 |
| 52 | si52 | 0.5 | -1.2 | -1.7 |
| 72 | si72 | 1.0 | -3.9 | -4.9 |
| 74 | si74 | 2.2 | -1.6 | -3.8 |
| 80 | si80 | 38.5 | 42.1 | 3.6 |

As shown in Table 12, the effects of alternating modifications on the inhibition rate of PCSK9 mRNA expression varied across different sequences. For some sequences, the inhibition rate was significantly improved. For example, for basic sequence 5, the alternately modified sequence exhibited a 12.4% increase in inhibition rate compared to the unmodified sequence; for basic sequence 82, the alternately modified sequence exhibited a 12.2% increase in inhibition rate compared to the unmodified sequence. For others, the improvement was minimal. For example, basic sequences 4, 22, and 52 exhibited little to no difference in inhibition rate between alternately modified and unmodified sequences.

Therefore, not all unmodified sequences exhibited improved activity after alternating modifications, and the effects of alternating modifications on activity varied across different sequences.

### Summary:

(1) A total of 12 unmodified sequences exhibited significant inhibitory effects on PCSK9 gene expression, with inhibition rates exceeding 50%, including si5, si51, si81, si82, si84, si3, si8, si9, si21, si31, si73, and si83. Specifically, si5, si81, si82, si3, and si8 exhibited inhibition rates exceeding 60%.
(2) The other unmodified sequences exhibited inhibition rates below 40% on PCSK9 gene expression. For example, the inhibition rates of si52 and si74 were only 0.5% and 2.2%, respectively.
(3) siRNAs with similar sequences exhibited significant differences in activity. For example, the inhibition rate of basic sequence 5 was 51.2% higher than that of basic sequence 4, indicating a significant improvement.
(4) The effects of alternating modifications on activity varied across different sequences. For some sequences, the inhibition rate was significantly improved. For example, for basic sequence 5, the alternately modified sequence exhibited a 12.4% increase in inhibition rate compared to the unmodified sequence. For others, the improvement was minimal. For example, basic sequences 4, 22, and 52 exhibited little to no difference in inhibition rate between alternately modified and unmodified sequences.

### Example 4: Inhibitory effect of template-modified sequences on PCSK9 gene expression

In this example, a total of 12 sequences identified in Example 3, namely the unmodified sequences si5, si51, si81, si82, si84, si3, si8, si9, si21, si31, si73, and si83, were modified using different modification templates. Specifically, DV25, DV26, DV27, DV28, DV29, DV30, and DV31 are novel modification templates designed in the present disclosure, while DV21 and DV22 are previously disclosed Advanced ESC modification templates.

### 1. Experimental materials

### Test samples:

The sequences si5, si51, si81, si82, si84, si3, si8, si9, si21, si31, si73, and si83 from Example 3, modified using different templates (DV25, DV26, DV27, DV28, DV29, DV30, DV31, DV21, and DV22) as listed in Table 13, as well as their corresponding alternately modified sequences P92-si5, P92-si51, P92-si81, P92-si82, P92-si84, P92-si3, P92-si8, P92-si9, P92-si21, P92-si31, P92-si73, and P92-si83 (synthesized in Example 1).

The modification principles for the templates disclosed herein are as follows: **DV25 to DV29:**
The antisense strand adopts one of the following modification patterns:

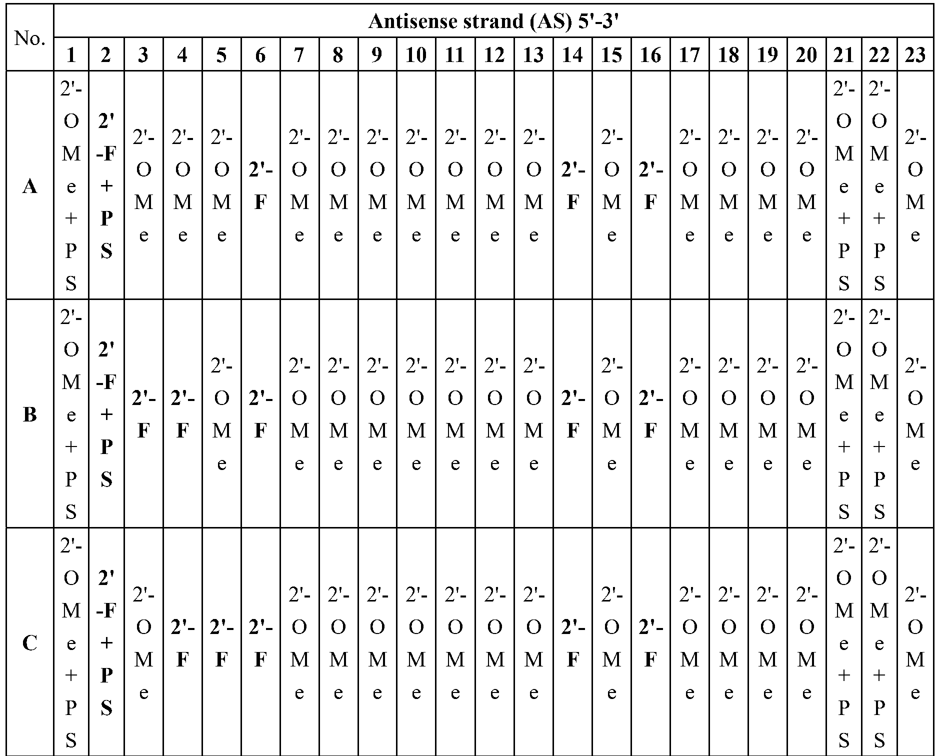

The sense strand adopts one of the following modification patterns:

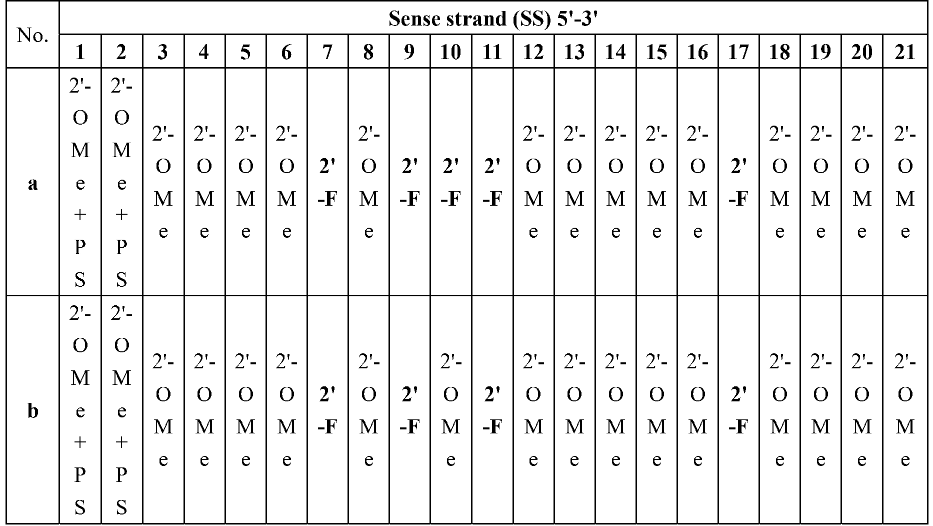

In the above tables, PS represents a phosphorothioate backbone.

The siRNA modification template, in which the antisense strand is modified according to modification pattern A and the sense strand is modified according to modification pattern a, is designated as DV25.

The siRNA modification template, in which the antisense strand is modified according to modification pattern B and the sense strand is modified according to modification pattern a, is designated as DV26.

The siRNA modification template, in which the antisense strand is modified according to modification pattern C and the sense strand is modified according to modification pattern a, is designated as DV27.

The siRNA modification template, in which the antisense strand is modified according to modification pattern B and the sense strand is modified according to modification pattern b, is designated as DV28.

The siRNA modification template, in which the antisense strand is modified according to modification pattern C and the sense strand is modified according to modification pattern b, is designated as DV29.

### DV30:

### Antisense strand:

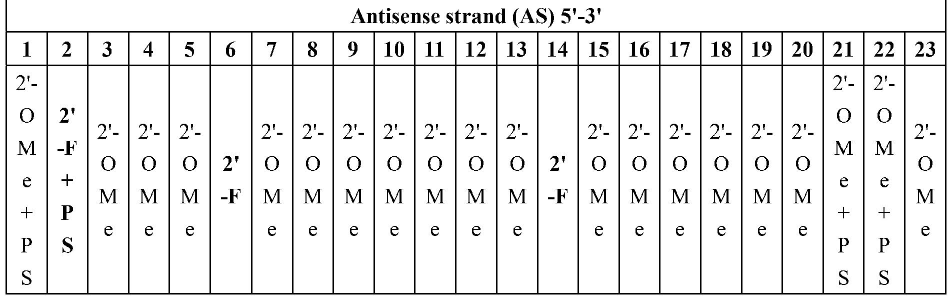

### Sense strand:

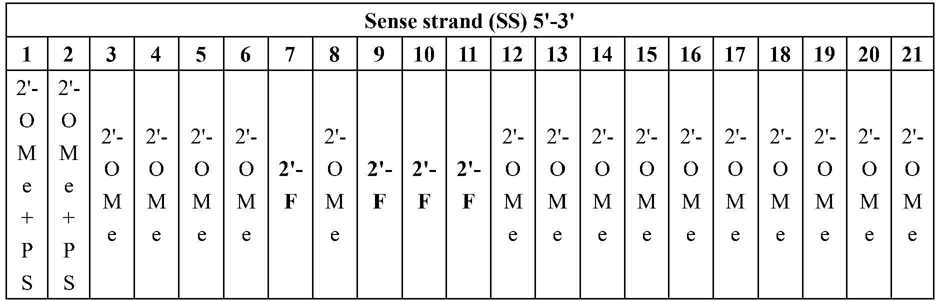

In the above tables, PS represents a phosphorothioate backbone.

### DV31:

### Antisense strand:

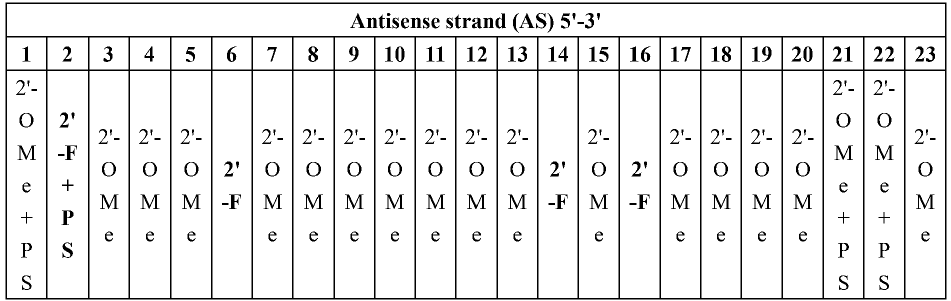

### Sense strand:

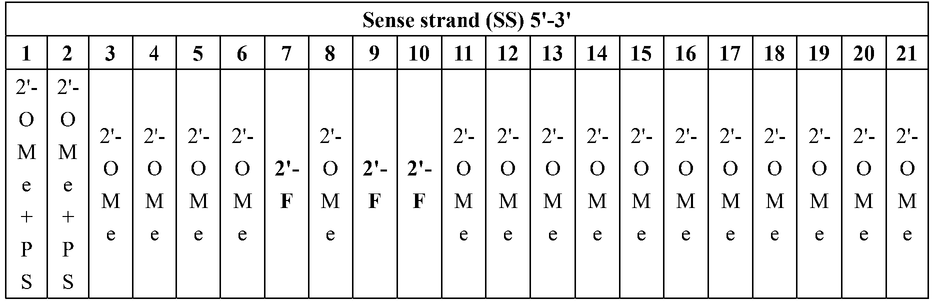

In the above tables, PS represents a phosphorothioate backbone.

### DV21:

### Antisense strand:

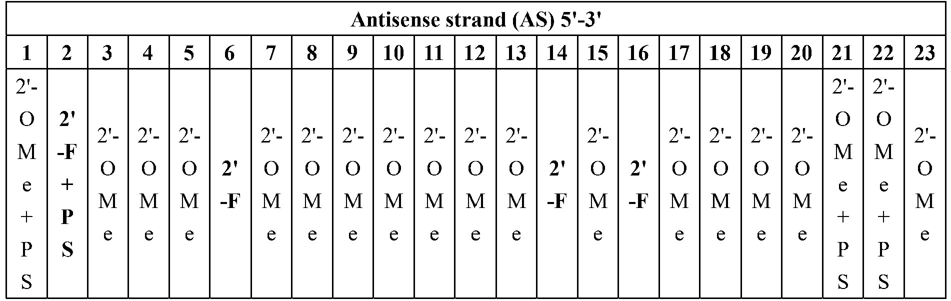

### Sense strand:

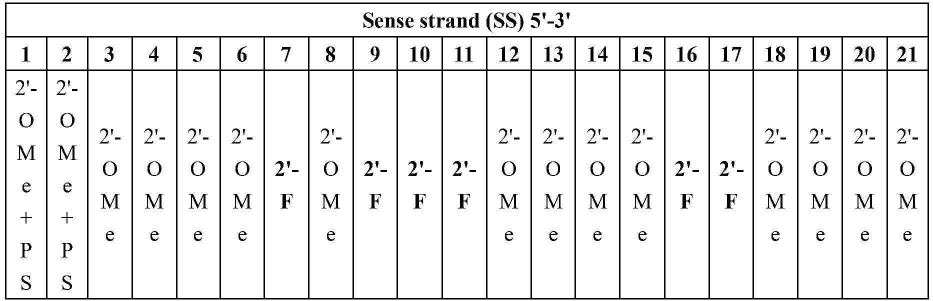

In the above tables, PS represents a phosphorothioate backbone.

### DV22:

### Antisense strand:

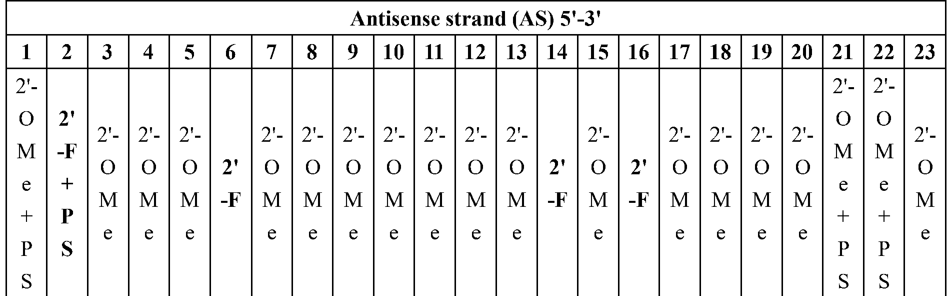

### Sense strand:

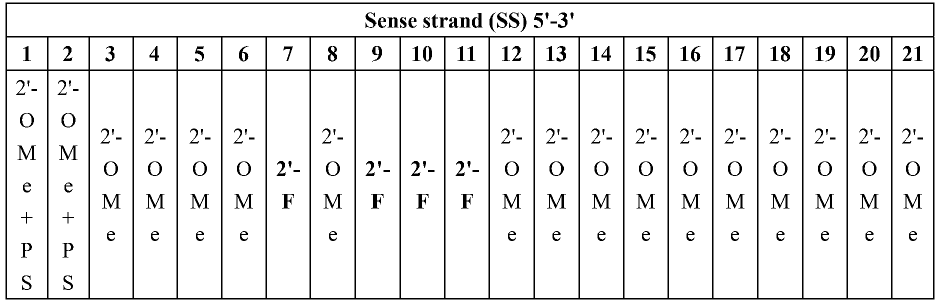

In the above tables, PS represents a phosphorothioate backbone.

The template-modified sequences are detailed in Table 13. These sequences were synthesized using the same method as described in Example 1.

**Table 13: Sequences modified using different templates**

| **Basic SEQ ID NO:** | **Template -modified sequence ID** | **Sense sequence 5'-3'** | **Modified SEQ ID NO:/corr. basic SEQ ID NO:** | **Antisense sequence 5'-3'** | **Modified SEQ ID NO:/corr. basic SEQ ID NO:** |
|---|---|---|---|---|---|
| | P92-si5-DV25 | Ams-Ams-Gm-Am-Um-Cm- Cf-Um-Gf-Cf-Af-Um-Gm- Um-Cm-Um-Uf-Cm-Cm- Am-Um | 337/1 | Ams-Ufs-Gm-Gm-Am-Af-Gm- Am-Cm-Am-Um-Gm-Cm-Af- Gm-Gf-Am-Um-Cm-Um-Ums- Gms-Gm | 376/13 |
| | P92-si5-DV26 | Ams-Ams-Gm-Am-Um-Cm- Cf-Um-Gf-Cf-Af-Um-Gm- Um-Cm-Um-Uf-Cm-Cm- Am-Um | 337/1 | Ams-Ufs-Gf-Gf-Am-Af-Gm- Am-Cm-Am-Um-Gm-Cm-Af- Gm-Gf-Am-Um-Cm-Um-Ums- Gms-Gm | 377/13 |
| | P92-si5-DV27 | Ams-Ams-Gm-Am-Um-Cm- Cf-Um-Gf-Cf-Af-Um-Gm- Um-Cm-Um-Uf-Cm-Cm- Am-Um | 337/1 | Ams-Ufs-Gm-Gf-Af-Af-Gm- Am-Cm-Am-Um-Gm-Cm-Af- Gm-Gf-Am-Um-Cm-Um-Ums- Gms-Gm | 378/13 |
| | P92-si5-DV28 | Ams-Ams-Gm-Am-Um-Cm- Cf-Um-Gf-Cm-Af-Um-Gm- Um-Cm-Um-Uf-Cm-Cm- Am-Um | 338/1 | Ams-Ufs-Gf-Gf-Am-Af-Gm- Am-Cm-Am-Um-Gm-Cm-Af- Gm-Gf-Am-Um-Cm-Um-Ums- Gms-Gm | 377/13 |
| 5 | P92-si5-DV29 | Ams-Ams-Gm-Am-Um-Cm- Cf-Um-Gf-Cm-Af-Um-Gm- Um-Cm-Um-Uf-Cm-Cm- Am-Um | 338/1 | Ams-Ufs-Gm-Gf-Af-Af-Gm- Am-Cm-Am-Um-Gm-Cm-Af- Gm-Gf-Am-Um-Cm-Um-Ums- Gms-Gm | 378/13 |
| | P92-si5-DV30 | Ams-Ams-Gm-Am-Um-Cm- Cf-Um-Gf-Cf-Af-Um-Gm- Um-Cm-Um-Um-Cm-Cm- Am-Um | 339/1 | Ams-Ufs-Gm-Gm-Am-Af-Gm- Am-Cm-Am-Um-Gm-Cm-Af- Gm-Gm-Am-Um-Cm-Um-Ums- Gms-Gm | 379/13 |
| | P92-si5-DV31 | Ams-Ams-Gm-Am-Um-Cm- Cf-Um-Gf-Cf-Am-Um-Gm- Um-Cm-Um-Um-Cm-Cm- Am-Um | 340/1 | Ams-Ufs-Gm-Gm-Am-Af-Gm- Am-Cm-Am-Um-Gm-Cm-Af- Gm-Gf-Am-Um-Cm-Um-Ums- Gms-Gm | 376/13 |
| | P92-si5-DV21 | Ams-Ams-Gm-Am-Um-Cm- Cf-Um-Gf-Cf-Af-Um-Gm- Um-Cm-Uf-Uf-Cm-Cm-Am- Um | 341/1 | Ams-Ufs-Gm-Gm-Am-Af-Gm- Am-Cm-Am-Um-Gm-Cm-Af- Gm-Gf-Am-Um-Cm-Um-Ums- Gms-Gm | 376/13 |
| | P92-si5-DV22 | Ams-Ams-Gm-Am-Um-Cm- Cf-Um-Gf-Cf-Af-Um-Gm- Um-Cm-Um-Um-Cm-Cm- Am-Um | 339/1 | Ams-Ufs-Gm-Gm-Am-Af-Gm- Am-Cm-Am-Um-Gm-Cm-Af- Gm-Gf-Am-Um-Cm-Um-Ums- Gms-Gm | 376/13 |
| 51 | P92-si51-DV25 | Ums-Gms-Gm-Am-Gm-Gm- Cf-Um-Uf-Af-Gf-Cm-Um- Um-Um-Cm-Uf-Gm-Gm- Am-Um | 342/2 | Ams-Ufs-Cm-Cm-Am-Gf-Am- Am-Am-Gm-Cm-Um-Am-Af- Gm-Cf-Cm-Um-Cm-Cm-Ams- Ums-Um | 380/14 |
| | P92-si51-DV26 | Ums-Gms-Gm-Am-Gm-Gm- Cf-Um-Uf-Af-Gf-Cm-Um- Um-Um-Cm-Uf-Gm-Gm- Am-Um | 342/2 | Ams-Ufs-Cf-Cf-Am-Gf-Am- Am-Am-Gm-Cm-Um-Am-Af- Gm-Cf-Cm-Um-Cm-Cm-Ams- Ums-Um | 381/14 |
| | P92-si51-DV27 | Ums-Gms-Gm-Am-Gm-Gm- Cf-Um-Uf-Af-Gf-Cm-Um- Um-Um-Cm-Uf-Gm-Gm- Am-Um | 342/2 | Ams-Ufs-Cm-Cf-Af-Gf-Am- Am-Am-Gm-Cm-Um-Am-Af- Gm-Cf-Cm-Um-Cm-Cm-Ams- Ums-Um | 382/14 |
| | P92-si51-DV28 | Ums-Gms-Gm-Am-Gm-Gm- Cf-Um-Uf-Am-Gf-Cm-Um- Um-Um-Cm-Uf-Gm-Gm- Am-Um | 343/2 | Ams-Ufs-Cf-Cf-Am-Gf-Am- Am-Am-Gm-Cm-Um-Am-Af- Gm-Cf-Cm-Um-Cm-Cm-Ams- Ums-Um | 381/14 |
| | P92-si51-DV29 | Ums-Gms-Gm-Am-Gm-Gm- Cf-Um-Uf-Am-Gf-Cm-Um- Um-Um-Cm-Uf-Gm-Gm- Am-Um | 343/2 | Ams-Ufs-Cm-Cf-Af-Gf-Am- Am-Am-Gm-Cm-Um-Am-Af- Gm-Cf-Cm-Um-Cm-Cm-Ams- Ums-Um | 382/14 |
| | P92-si51-DV30 | Ums-Gms-Gm-Am-Gm-Gm- Cf-Um-Uf-Af-Gf-Cm-Um- Um-Um-Cm-Um-Gm-Gm- Am-Um | 344/2 | Ams-Ufs-Cm-Cm-Am-Gf-Am- Am-Am-Gm-Cm-Um-Am-Af- Gm-Cm-Cm-Um-Cm-Cm-Ams- Ums-Um | 383/14 |
| | P92-si51-DV31 | Ums-Gms-Gm-Am-Gm-Gm- Cf-Um-Uf-Af-Gm-Cm-Um- Um-Um-Cm-Um-Gm-Gm- Am-Um | 345/2 | Ams-Ufs-Cm-Cm-Am-Gf-Am- Am-Am-Gm-Cm-Um-Am-Af- Gm-Cf-Cm-Um-Cm-Cm-Ams- Ums-Um | 380/14 |
| | P92-si51-DV21 | Ums-Gms-Gm-Am-Gm-Gm- Cf-Um-Uf-Af-Gf-Cm-Um- Um-Um-Cf-Uf-Gm-Gm-Am- Um | 346/2 | Ams-Ufs-Cm-Cm-Am-Gf-Am- Am-Am-Gm-Cm-Um-Am-Af- Gm-Cf-Cm-Um-Cm-Cm-Ams- Ums-Um | 380/14 |
| | P92-si51-DV22 | Ums-Gms-Gm-Am-Gm-Gm- Cf-Um-Uf-Af-Gf-Cm-Um- Um-Um-Cm-Um-Gm-Gm- Am-Um | 344/2 | Ams-Ufs-Cm-Cm-Am-Gf-Am- Am-Am-Gm-Cm-Um-Am-Af- Gm-Cf-Cm-Um-Cm-Cm-Ams- Ums-Um | 380/14 |
| 81 | P92-si81-DV25 | Cms-Ums-Um-Um-Um-Gm- Uf-Am-Af-Cf-Uf-Um-Gm- Am-Am-Gm-Af-Um-Am- Um-Um | 347/3 | Ams-Afs-Um-Am-Um-Cf-Um- Um-Cm-Am-Am-Gm-Um-Uf- Am-Cf-Am-Am-Am-Am-Gms- Cms-Am | 384/15 |
| | P92-si81-DV26 | Cms-Ums-Um-Um-Um-Gm- Uf-Am-Af-Cf-Uf-Um-Gm- Am-Am-Gm-Af-Um-Am- Um-Um | 347/3 | Ams-Afs-Uf-Af-Um-Cf-Um- Um-Cm-Am-Am-Gm-Um-Uf- Am-Cf-Am-Am-Am-Am-Gms- Cms-Am | 385/15 |
| | P92-si81-DV27 | Cms-Ums-Um-Um-Um-Gm- Uf-Am-Af-Cf-Uf-Um-Gm- Am-Am-Gm-Af-Um-Am- Um-Um | 347/3 | Ams-Afs-Um-Af-Uf-Cf-Um- Um-Cm-Am-Am-Gm-Um-Uf- Am-Cf-Am-Am-Am-Am-Gms- Cms-Am | 386/15 |
| | P92-si81-DV28 | Cms-Ums-Um-Um-Um-Gm- Uf-Am-Af-Cm-Uf-Um-Gm- Am-Am-Gm-Af-Um-Am- Um-Um | 348/3 | Ams-Afs-Uf-Af-Um-Cf-Um- Um-Cm-Am-Am-Gm-Um-Uf- Am-Cf-Am-Am-Am-Am-Gms- Cms-Am | 385/15 |
| | P92-si81-DV29 | Cms-Ums-Um-Um-Um-Gm- Uf-Am-Af-Cm-Uf-Um-Gm- Am-Am-Gm-Af-Um-Am- Um-Um | 348/3 | Ams-Afs-Um-Af-Uf-Cf-Um- Um-Cm-Am-Am-Gm-Um-Uf- Am-Cf-Am-Am-Am-Am-Gms- Cms-Am | 386/15 |
| | P92-si81-DV30 | Cms-Ums-Um-Um-Um-Gm- Uf-Am-Af-Cf-Uf-Um-Gm- Am-Am-Gm-Am-Um-Am- Um-Um | 349/3 | Ams-Afs-Um-Am-Um-Cf-Um- Um-Cm-Am-Am-Gm-Um-Uf- Am-Cm-Am-Am-Am-Am-Gms- Cms-Am | 387/15 |
| | P92-si81-DV31 | Cms-Ums-Um-Um-Um-Gm- Uf-Am-Af-Cf-Um-Um-Gm- Am-Am-Gm-Am-Um-Am- Um-Um | 350/3 | Ams-Afs-Um-Am-Um-Cf-Um- Um-Cm-Am-Am-Gm-Um-Uf- Am-Cf-Am-Am-Am-Am-Gms- Cms-Am | 384/15 |
| | P92-si81-DV21 | Cms-Ums-Um-Um-Um-Gm- Uf-Am-Af-Cf-Uf-Um-Gm- Am-Am-Gf-Af-Um-Am-Um- Um | 351/3 | Ams-Afs-Um-Am-Um-Cf-Um- Um-Cm-Am-Am-Gm-Um-Uf- Am-Cf-Am-Am-Am-Am-Gms- Cms-Am | 384/15 |
| | P92-si81-DV22 | Cms-Ums-Um-Um-Um-Gm- Uf-Am-Af-Cf-Uf-Um-Gm- Am-Am-Gm-Am-Um-Am- Um-Um | 349/3 | Ams-Afs-Um-Am-Um-Cf-Um- Um-Cm-Am-Am-Gm-Um-Uf- Am-Cf-Am-Am-Am-Am-Gms- Cms-Am | 384/15 |
| 82 | P92-si82-DV25 | Gms-Ams-Am-Gm-Am-Um- Af-Um-Uf-Uf-Af-Um-Um- Cm-Um-Gm-Gf-Gm-Um- Um-Um | 352/4 | Ams-Afs-Am-Cm-Cm-Cf-Am- Gm-Am-Am-Um-Am-Am-Af- Um-Af-Um-Cm-Um-Um-Cms- Ams-Am | 388/16 |
| | P92-si82-DV26 | Gms-Ams-Am-Gm-Am-Um- Af-Um-Uf-Uf-Af-Um-Um- Cm-Um-Gm-Gf-Gm-Um- Um-Um | 352/4 | Ams-Afs-Af-Cf-Cm-Cf-Am- Gm-Am-Am-Um-Am-Am-Af- Um-Af-Um-Cm-Um-Um-Cms- Ams-Am | 389/16 |
| | P92-si82-DV27 | Gms-Ams-Am-Gm-Am-Um- Af-Um-Uf-Uf-Af-Um-Um- Cm-Um-Gm-Gf-Gm-Um- Um-Um | 352/4 | Ams-Afs-Am-Cf-Cf-Cf-Am- Gm-Am-Am-Um-Am-Am-Af- Um-Af-Um-Cm-Um-Um-Cms- Ams-Am | 390/16 |
| | P92-si82-DV28 | Gms-Ams-Am-Gm-Am-Um- Af-Um-Uf-Um-Af-Um-Um- Cm-Um-Gm-Gf-Gm-Um- Um-Um | 353/4 | Ams-Afs-Af-Cf-Cm-Cf-Am- Gm-Am-Am-Um-Am-Am-Af- Um-Af-Um-Cm-Um-Um-Cms- Ams-Am | 389/16 |
| | P92-si82-DV29 | Gms-Ams-Am-Gm-Am-Um- Af-Um-Uf-Um-Af-Um-Um- Cm-Um-Gm-Gf-Gm-Um- Um-Um | 353/4 | Ams-Afs-Am-Cf-Cf-Cf-Am- Gm-Am-Am-Um-Am-Am-Af- Um-Af-Um-Cm-Um-Um-Cms- Ams-Am | 390/16 |
| | P92-si82-DV30 | Gms-Ams-Am-Gm-Am-Um- Af-Um-Uf-Uf-Af-Um-Um- Cm-Um-Gm-Gm-Gm-Um- Um-Um | 354/4 | Ams-Afs-Am-Cm-Cm-Cf-Am- Gm-Am-Am-Um-Am-Am-Af- Um-Am-Um-Cm-Um-Um-Cms- Ams-Am | 391/16 |
| | P92-si82-DV31 | Gms-Ams-Am-Gm-Am-Um- Af-Um-Uf-Uf-Am-Um-Um- Cm-Um-Gm-Gm-Gm-Um- Um-Um | 355/4 | Ams-Afs-Am-Cm-Cm-Cf-Am- Gm-Am-Am-Um-Am-Am-Af- Um-Af-Um-Cm-Um-Um-Cms- Ams-Am | 388/16 |
| | P92-si82-DV21 | Gms-Ams-Am-Gm-Am-Um- Af-Um-Uf-Uf-Af-Um-Um- Cm-Um-Gf-Gf-Gm-Um-Um- Um | 356/4 | Ams-Afs-Am-Cm-Cm-Cf-Am- Gm-Am-Am-Um-Am-Am-Af- Um-Af-Um-Cm-Um-Um-Cms- Ams-Am | 388/16 |
| | P92-si82-DV22 | Gms-Ams-Am-Gm-Am-Um- Af-Um-Uf-Uf-Af-Um-Um- Cm-Um-Gm-Gm-Gm-Um- Um-Um | 354/4 | Ams-Afs-Am-Cm-Cm-Cf-Am- Gm-Am-Am-Um-Am-Am-Af- Um-Af-Um-Cm-Um-Um-Cms- Ams-Am | 388/16 |
| 84 | P92-si84-DV25 | Gms-Ums-Um-Um-Um-Gm- Uf-Am-Gf-Cf-Af-Um-Um- Um-Um-Um-Af-Um-Um- Am-Am | 357/5 | Ums-Ufs-Am-Am-Um-Af-Am- Am-Am-Am-Um-Gm-Cm-Uf- Am-Cf-Am-Am-Am-Am-Cms- Cms-Cm | 392/17 |
| | P92-si84-DV26 | Gms-Ums-Um-Um-Um-Gm- Uf-Am-Gf-Cf-Af-Um-Um- Um-Um-Um-Af-Um-Um- Am-Am | 357/5 | Ums-Ufs-Af-Af-Um-Af-Am- Am-Am-Am-Um-Gm-Cm-Uf- Am-Cf-Am-Am-Am-Am-Cms- Cms-Cm | 393/17 |
| | P92-si84-DV27 | Gms-Ums-Um-Um-Um-Gm- Uf-Am-Gf-Cf-Af-Um-Um- Um-Um-Um-Af-Um-Um- Am-Am | 357/5 | Ums-Ufs-Am-Af-Uf-Af-Am- Am-Am-Am-Um-Gm-Cm-Uf- Am-Cf-Am-Am-Am-Am-Cms- Cms-Cm | 394/17 |
| | P92-si84-DV28 | Gms-Ums-Um-Um-Um-Gm- Uf-Am-Gf-Cm-Af-Um-Um- Um-Um-Um-Af-Um-Um- Am-Am | 358/5 | Ums-Ufs-Af-Af-Um-Af-Am- Am-Am-Am-Um-Gm-Cm-Uf- Am-Cf-Am-Am-Am-Am-Cms- Cms-Cm | 393/17 |
| | P92-si84-DV29 | Gms-Ums-Um-Um-Um-Gm- Uf-Am-Gf-Cm-Af-Um-Um- Um-Um-Um-Af-Um-Um- Am-Am | 358/5 | Ums-Ufs-Am-Af-Uf-Af-Am- Am-Am-Am-Um-Gm-Cm-Uf- Am-Cf-Am-Am-Am-Am-Cms- Cms-Cm | 394/17 |
| | P92-si84-DV30 | Gms-Ums-Um-Um-Um-Gm- Uf-Am-Gf-Cf-Af-Um-Um- Um-Um-Um-Am-Um-Um- Am-Am | 359/5 | Ums-Ufs-Am-Am-Um-Af-Am- Am-Am-Am-Um-Gm-Cm-Uf- Am-Cm-Am-Am-Am-Am-Cms- Cms-Cm | 395/17 |
| | P92-si84-DV31 | Gms-Ums-Um-Um-Um-Gm- Uf-Am-Gf-Cf-Am-Um-Um- Um-Um-Um-Am-Um-Um- Am-Am | 360/5 | Ums-Ufs-Am-Am-Um-Af-Am- Am-Am-Am-Um-Gm-Cm-Uf- Am-Cf-Am-Am-Am-Am-Cms- Cms-Cm | 392/17 |
| | P92-si84-DV21 | Gms-Ums-Um-Um-Um-Gm- Uf-Am-Gf-Cf-Af-Um-Um- Um-Um-Uf-Af-Um-Um-Am- Am | 361/5 | Ums-Ufs-Am-Am-Um-Af-Am- Am-Am-Am-Um-Gm-Cm-Uf- Am-Cf-Am-Am-Am-Am-Cms- Cms-Cm | 392/17 |
| | P92-si84-DV22 | Gms-Ums-Um-Um-Um-Gm- Uf-Am-Gf-Cf-Af-Um-Um- Um-Um-Um-Am-Um-Um- Am-Am | 359/5 | Ums-Ufs-Am-Am-Um-Af-Am- Am-Am-Am-Um-Gm-Cm-Uf- Am-Cf-Am-Am-Am-Am-Cms- Cms-Cm | 392/17 |
| 3 | P92-si3-DV25 | Cms-Ams-Cm-Cm-Am-Am- Gf-Am-Uf-Cf-Cf-Um-Gm- Cm-Am-Um-Gf-Um-Cm- Um-Um | 362/6 | Ams-Afs-Gm-Am-Cm-Af-Um- Gm-Cm-Am-Gm-Gm-Am-Uf- Cm-Uf-Um-Gm-Gm-Um-Gms- Ams-Gm | 396/18 |
| | P92-si3-DV26 | Cms-Ams-Cm-Cm-Am-Am- Gf-Am-Uf-Cf-Cf-Um-Gm- Cm-Am-Um-Gf-Um-Cm- Um-Um | 362/6 | Ams-Afs-Gf-Af-Cm-Af-Um- Gm-Cm-Am-Gm-Gm-Am-Uf- Cm-Uf-Um-Gm-Gm-Um-Gms- Ams-Gm | 397/18 |
| | P92-si3-DV27 | Cms-Ams-Cm-Cm-Am-Am- Gf-Am-Uf-Cf-Cf-Um-Gm- Cm-Am-Um-Gf-Um-Cm- Um-Um | 362/6 | Ams-Afs-Gm-Af-Cf-Af-Um- Gm-Cm-Am-Gm-Gm-Am-Uf- Cm-Uf-Um-Gm-Gm-Um-Gms- Ams-Gm | 398/18 |
| | P92-si3-DV28 | Cms-Ams-Cm-Cm-Am-Am- Gf-Am-Uf-Cm-Cf-Um-Gm- Cm-Am-Um-Gf-Um-Cm- Um-Um | 363/6 | Ams-Afs-Gf-Af-Cm-Af-Um- Gm-Cm-Am-Gm-Gm-Am-Uf- Cm-Uf-Um-Gm-Gm-Um-Gms- Ams-Gm | 397/18 |
| | P92-si3-DV29 | Cms-Ams-Cm-Cm-Am-Am- Gf-Am-Uf-Cm-Cf-Um-Gm- Cm-Am-Um-Gf-Um-Cm- Um-Um | 363/6 | Ams-Afs-Gm-Af-Cf-Af-Um- Gm-Cm-Am-Gm-Gm-Am-Uf- Cm-Uf-Um-Gm-Gm-Um-Gms- Ams-Gm | 398/18 |
| 8 | P92-si8-DV25 | Cms-Ums-Cm-Am-Um-Am- Gf-Gm-Cf-Cf-Uf-Gm-Gm- Am-Gm-Um-Uf-Um-Am- Um-Um | 364/7 | Ams-Afs-Um-Am-Am-Af-Cm- Um-Cm-Cm-Am-Gm-Gm-Cf- Cm-Uf-Am-Um-Gm-Am-Gms- Gms-Gm | 399/19 |
| | P92-si8-DV26 | Cms-Ums-Cm-Am-Um-Am- Gf-Gm-Cf-Cf-Uf-Gm-Gm- Am-Gm-Um-Uf-Um-Am- Um-Um | 364/7 | Ams-Afs-Uf-Af-Am-Af-Cm- Um-Cm-Cm-Am-Gm-Gm-Cf- Cm-Uf-Am-Um-Gm-Am-Gms- Gms-Gm | 400/19 |
| | P92-si8-DV27 | Cms-Ums-Cm-Am-Um-Am- Gf-Gm-Cf-Cf-Uf-Gm-Gm- Am-Gm-Um-Uf-Um-Am- Um-Um | 364/7 | Ams-Afs-Um-Af-Af-Af-Cm- Um-Cm-Cm-Am-Gm-Gm-Cf- Cm-Uf-Am-Um-Gm-Am-Gms- Gms-Gm | 401/19 |
| | P92-si8-DV28 | Cms-Ums-Cm-Am-Um-Am- Gf-Gm-Cf-Cm-Uf-Gm-Gm- Am-Gm-Um-Uf-Um-Am- Um-Um | 365/7 | Ams-Afs-Uf-Af-Am-Af-Cm- Um-Cm-Cm-Am-Gm-Gm-Cf- Cm-Uf-Am-Um-Gm-Am-Gms- Gms-Gm | 400/19 |
| | P92-si8-DV29 | Cms-Ums-Cm-Am-Um-Am- Gf-Gm-Cf-Cm-Uf-Gm-Gm- Am-Gm-Um-Uf-Um-Am- Um-Um | 365/7 | Ams-Afs-Um-Af-Af-Af-Cm- Um-Cm-Cm-Am-Gm-Gm-Cf- Cm-Uf-Am-Um-Gm-Am-Gms- Gms-Gm | 401/19 |
| 9 | P92-si9-DV25 | Gms-Gms-Cm-Cm-Um-Gm- Gf-Am-Gf-Uf-Uf-Um-Am- Um-Um-Cm-Gf-Gm-Am- Am-Am | 366/8 | Ums-Ufs-Um-Cm-Cm-Gf-Am- Am-Um-Am-Am-Am-Cm-Uf- Cm-Cf-Am-Gm-Gm-Cm-Cms- Ums-Am | 402/20 |
| | P92-si9-DV26 | Gms-Gms-Cm-Cm-Um-Gm- Gf-Am-Gf-Uf-Uf-Um-Am- Um-Um-Cm-Gf-Gm-Am- Am-Am | 366/8 | Ums-Ufs-Uf-Cf-Cm-Gf-Am- Am-Um-Am-Am-Am-Cm-Uf- Cm-Cf-Am-Gm-Gm-Cm-Cms- Ums-Am | 403/20 |
| | P92-si9-DV27 | Gms-Gms-Cm-Cm-Um-Gm- Gf-Am-Gf-Uf-Uf-Um-Am- Um-Um-Cm-Gf-Gm-Am- Am-Am | 366/8 | Ums-Ufs-Um-Cf-Cf-Gf-Am- Am-Um-Am-Am-Am-Cm-Uf- Cm-Cf-Am-Gm-Gm-Cm-Cms- Ums-Am | 404/20 |
| | P92-si9-DV28 | Gms-Gms-Cm-Cm-Um-Gm- Gf-Am-Gf-Um-Uf-Um-Am- Um-Um-Cm-Gf-Gm-Am- Am-Am | 367/8 | Ums-Ufs-Uf-Cf-Cm-Gf-Am- Am-Um-Am-Am-Am-Cm-Uf- Cm-Cf-Am-Gm-Gm-Cm-Cms- Ums-Am | 403/20 |
| | P92-si9-DV29 | Gms-Gms-Cm-Cm-Um-Gm- Gf-Am-Gf-Um-Uf-Um-Am- Um-Um-Cm-Gf-Gm-Am- Am-Am | 367/8 | Ums-Ufs-Um-Cf-Cf-Gf-Am- Am-Um-Am-Am-Am-Cm-Uf- Cm-Cf-Am-Gm-Gm-Cm-Cms- Ums-Am | 404/20 |
| 21 | P92-si21-DV25 | Cms-Ams-Gm-Cm-Am-Cm- Cf-Um-Gf-Cf-Uf-Um-Um- Gm-Um-Gm-Uf-Cm-Am- Cm-Am | 368/9 | Ums-Gfs-Um-Gm-Am-Cf-Am- Cm-Am-Am-Am-Gm-Cm-Af- Gm-Gf-Um-Gm-Cm-Um-Gms- Cms-Am | 405/21 |
| | P92-si21-DV26 | Cms-Ams-Gm-Cm-Am-Cm- Cf-Um-Gf-Cf-Uf-Um-Um- Gm-Um-Gm-Uf-Cm-Am- Cm-Am | 368/9 | Ums-Gfs-Uf-Gf-Am-Cf-Am- Cm-Am-Am-Am-Gm-Cm-Af- Gm-Gf-Um-Gm-Cm-Um-Gms- Cms-Am | 406/21 |
| | P92-si21-DV27 | Cms-Ams-Gm-Cm-Am-Cm- Cf-Um-Gf-Cf-Uf-Um-Um- Gm-Um-Gm-Uf-Cm-Am- Cm-Am | 368/9 | Ums-Gfs-Um-Gf-Af-Cf-Am- Cm-Am-Am-Am-Gm-Cm-Af- Gm-Gf-Um-Gm-Cm-Um-Gms- Cms-Am | 407/21 |
| | P92-si21-DV28 | Cms-Ams-Gm-Cm-Am-Cm- Cf-Um-Gf-Cm-Uf-Um-Um- Gm-Um-Gm-Uf-Cm-Am- Cm-Am | 369/9 | Ums-Gfs-Uf-Gf-Am-Cf-Am- Cm-Am-Am-Am-Gm-Cm-Af- Gm-Gf-Um-Gm-Cm-Um-Gms- Cms-Am | 406/21 |
| | P92-si21-DV29 | Cms-Ams-Gm-Cm-Am-Cm- Cf-Um-Gf-Cm-Uf-Um-Um- Gm-Um-Gm-Uf-Cm-Am- Cm-Am | 369/9 | Ums-Gfs-Um-Gf-Af-Cf-Am- Cm-Am-Am-Am-Gm-Cm-Af- Gm-Gf-Um-Gm-Cm-Um-Gms- Cms-Am | 407/21 |
| 31 | P92-si31-DV25 | Gms-Gms-Um-Cm-Um-Gm- Gf-Am-Af-Uf-Gf-Cm-Am- Am-Am-Gm-Uf-Cm-Am- Am-Gm | 370/10 | Cms-Ufs-Um-Gm-Am-Cf-Um- Um-Um-Gm-Cm-Am-Um-Uf- Cm-Cf-Am-Gm-Am-Cm-Cms- Ums-Gm | 408/22 |
| | P92-si31-DV26 | Gms-Gms-Um-Cm-Um-Gm- Gf-Am-Af-Uf-Gf-Cm-Am- Am-Am-Gm-Uf-Cm-Am- Am-Gm | 370/10 | Cms-Ufs-Uf-Gf-Am-Cf-Um- Um-Um-Gm-Cm-Am-Um-Uf- Cm-Cf-Am-Gm-Am-Cm-Cms- Ums-Gm | 409/22 |
| | P92-si31-DV27 | Gms-Gms-Um-Cm-Um-Gm- Gf-Am-Af-Uf-Gf-Cm-Am- Am-Am-Gm-Uf-Cm-Am- Am-Gm | 370/10 | Cms-Ufs-Um-Gf-Af-Cf-Um- Um-Um-Gm-Cm-Am-Um-Uf- Cm-Cf-Am-Gm-Am-Cm-Cms- Ums-Gm | 410/22 |
| | P92-si31-DV28 | Gms-Gms-Um-Cm-Um-Gm- Gf-Am-Af-Um-Gf-Cm-Am- Am-Am-Gm-Uf-Cm-Am- Am-Gm | 371/10 | Cms-Ufs-Uf-Gf-Am-Cf-Um- Um-Um-Gm-Cm-Am-Um-Uf- Cm-Cf-Am-Gm-Am-Cm-Cms- Ums-Gm | 409/22 |
| | P92-si31-DV29 | Gms-Gms-Um-Cm-Um-Gm- Gf-Am-Af-Um-Gf-Cm-Am- Am-Am-Gm-Uf-Cm-Am- Am-Gm | 371/10 | Cms-Ufs-Um-Gf-Af-Cf-Um- Um-Um-Gm-Cm-Am-Um-Uf- Cm-Cf-Am-Gm-Am-Cm-Cms- Ums-Gm | 410/22 |
| 73 | P92-si73-DV25 | Gms-Cms-Gm-Gm-Am-Gm- Af-Um-Gf-Cf-Uf-Um-Cm- Um-Am-Am-Gf-Gm-Cm- Am-Um | 372/11 | Ams-Ufs-Gm-Cm-Cm-Uf-Um- Am-Gm-Am-Am-Gm-Cm-Af- Um-Cf-Um-Cm-Cm-Gm-Cms- Cms-Am | 411/23 |
| | P92-si73-DV26 | Gms-Cms-Gm-Gm-Am-Gm- Af-Um-Gf-Cf-Uf-Um-Cm- Um-Am-Am-Gf-Gm-Cm- Am-Um | 372/11 | Ams-Ufs-Gf-Cf-Cm-Uf-Um- Am-Gm-Am-Am-Gm-Cm-Af- Um-Cf-Um-Cm-Cm-Gm-Cms- Cms-Am | 412/23 |
| | P92-si73-DV27 | Gms-Cms-Gm-Gm-Am-Gm- Af-Um-Gf-Cf-Uf-Um-Cm- Um-Am-Am-Gf-Gm-Cm- Am-Um | 372/11 | Ams-Ufs-Gm-Cf-Cf-Uf-Um- Am-Gm-Am-Am-Gm-Cm-Af- Um-Cf-Um-Cm-Cm-Gm-Cms- Cms-Am | 413/23 |
| | P92-si73-DV28 | Gms-Cms-Gm-Gm-Am-Gm- Af-Um-Gf-Cm-Uf-Um-Cm- Um-Am-Am-Gf-Gm-Cm- Am-Um | 373/11 | Ams-Ufs-Gf-Cf-Cm-Uf-Um- Am-Gm-Am-Am-Gm-Cm-Af- Um-Cf-Um-Cm-Cm-Gm-Cms- Cms-Am | 412/23 |
| | P92-si73-DV29 | Gms-Cms-Gm-Gm-Am-Gm- Af-Um-Gf-Cm-Uf-Um-Cm- Um-Am-Am-Gf-Gm-Cm- Am-Um | 373/11 | Ams-Ufs-Gm-Cf-Cf-Uf-Um- Am-Gm-Am-Am-Gm-Cm-Af- Um-Cf-Um-Cm-Cm-Gm-Cms- Cms-Am | 413/23 |
| | P92-si83-DV25 | Gms-Gms-Gm-Um-Um-Um- Uf-Gm-Uf-Af-Gf-Cm-Am- Um-Um-Um-Uf-Um-Am- Um-Um | 374/12 | Ams-Afs-Um-Am-Am-Af-Am- Am-Um-Gm-Cm-Um-Am-Cf- Am-Af-Am-Am-Cm-Cm-Cms- Ams-Gm | 414/24 |
| | P92-si83-DV26 | Gms-Gms-Gm-Um-Um-Um- Uf-Gm-Uf-Af-Gf-Cm-Am- Um-Um-Um-Uf-Um-Am- Um-Um | 374/12 | Ams-Afs-Uf-Af-Am-Af-Am- Am-Um-Gm-Cm-Um-Am-Cf- Am-Af-Am-Am-Cm-Cm-Cms- Ams-Gm | 415/24 |
| 83 | P92-si83-DV27 | Gms-Gms-Gm-Um-Um-Um- Uf-Gm-Uf-Af-Gf-Cm-Am- Um-Um-Um-Uf-Um-Am- Um-Um | 374/12 | Ams-Afs-Um-Af-Af-Af-Am- Am-Um-Gm-Cm-Um-Am-Cf- Am-Af-Am-Am-Cm-Cm-Cms- Ams-Gm | 416/24 |
| | P92-si83-DV28 | Gms-Gms-Gm-Um-Um-Um- Uf-Gm-Uf-Am-Gf-Cm-Am- Um-Um-Um-Uf-Um-Am- Um-Um | 375/12 | Ams-Afs-Uf-Af-Am-Af-Am- Am-Um-Gm-Cm-Um-Am-Cf- Am-Af-Am-Am-Cm-Cm-Cms- Ams-Gm | 415/24 |
| | P92-si83-DV29 | Gms-Gms-Gm-Um-Um-Um- Uf-Gm-Uf-Am-Gf-Cm-Am- Um-Um-Um-Uf-Um-Am- Um-Um | 375/12 | Ams-Afs-Um-Af-Af-Af-Am- Am-Um-Gm-Cm-Um-Am-Cf- Am-Af-Am-Am-Cm-Cm-Cms- Ams-Gm | 416/24 |

Cell line: HEP3B cells.

Solvents: sterile nuclease-free water, Gibco DMEM (purchased from Thermo Fisher Scientific; Cat. No.: 10569010).

### 2. Experimental methods

The inhibitory effects of the test samples on PCSK9 mRNA expression in HEP3B cells were evaluated by qRT-PCR. Hep3B cells were provided by WuXi AppTec Co., Ltd. (ATCC-tings-1618164).

### 2.1 Cell culture

Subcultured HEP3B cells in the logarithmic growth phase were cultured in RPMI 1640 culture medium containing 10% fetal bovine serum (supplemented with penicillin and streptomycin at 100 µL/mL), and incubated in a cell incubator at 37°C with 5% CO₂. The medium was exchanged once daily. The cells were digested with 0.25% trypsin, followed by centrifugation at 1000 rpm for 5 minutes. The supernatant was discarded, and fresh medium was added for continued culture.

### 2.2 Cell transfection

Preparation of transfection mixture: Lipofectamine RNAiMAX was mixed with Opti-MEM at a volume ratio of 1.5:98.5 and vortexed thoroughly.

Preparation of transfection reagent: 60 µL of siRNA solution (diluted in Opti-MEM) was mixed with 60 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 60 µL of transfection mixture was added to 60 µL of Opti-MEM, vortexed thoroughly, followed by incubation at room temperature for 15 minutes.

The prepared transfection reagent was added to a 24-well cell culture plate (100 µL per well), resulting in a final siRNA concentration of 0.05 nM per well. Subsequently, 500 µL of cell suspension (6 × 10⁴ cells per mL) was added to each well. After cross-mixing, the plate was incubated in a cell incubator at 37°C with 5% CO₂ for 40 hours.

### 2.3 PCSK9 mRNA analysis

The procedure was the same as described in "2.3 PCSK9 mRNA analysis" of Example 2.

### 2.4 Data processing

PCSK9 mRNA expression rate (%) was calculated using the following formula: 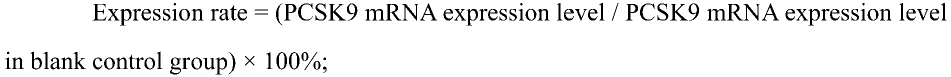 Expression rate = (PCSK9 mRNA expression level / PCSK9 mRNA expression level in blank control group) × 100%;
$Inhibition rate of PCSK 9 gene expression = 100 % - expression rate \left(%\right) .$

### 2.5 EC₅₀ assay

The siRNAs used for EC₅₀ determination were subjected to serial dilution starting from 0.2 nM with 4-fold dilutions, totaling 8 concentration points (0.2 nM, 0.05 nM, 0.0125 nM, 3.13 pM, 0.78 pM, 0.2 pM, 0.05 pM, and 0.01 pM). The inhibition rates for these sequences at each concentration were measured, and the resulting data were plotted to calculate EC₅₀ values.

### 3. Experimental results

Based on three independent experiments, the inhibition rates of PCSK9 gene expression in Hep3B cells by these modified sequences are shown in Tables 14 to 25.

The activity assay results demonstrated that all 12 candidate sequences (unmodified sequences si5, si51, si81, si82, si84, si3, si8, si9, si21, si31, si73, and si83), when modified using templates DV25 to 29 designed in the present disclosure, exhibited significant inhibitory effects on PCSK9 gene expression, with inhibition rates exceeding 70%. Specifically, P92-si81-DV26, P92-si82-DV27, and P92-si82-DV29 exhibited inhibition rates of 89.3%, 89.3%, and 89.1%, respectively.

The sequences modified using templates DV25 to DV29 disclosed herein showed significantly enhanced inhibition of PCSK9 gene expression as compared to the alternately modified sequences. For example, for basic sequence 51, the inhibition rate of P92-si51-DV27 (the sequence modified using template DV27) was 29.1% higher than that of the alternately modified sequence; for basic sequence 81, the inhibition rate of P92-si81-DV26 (the sequence modified using template DV26) was 26.9% higher than that of the alternately modified sequence.

Moreover, the same siRNA sequence exhibited significant differences in activity when modified using different modification templates. For example, for basic sequence 51, the sequence modified using template DV27 disclosed herein exhibited a 22.8% increase in inhibition rate compared to the sequence modified using template DV31 disclosed herein, and a 21.3% increase in inhibition rate compared to the sequence modified using the previously disclosed Advanced ESC template DV21.

EC₅₀ assays demonstrated that the sequences modified using the templates designed in the present disclosure exhibited EC₅₀ values ranging from 0.0001 to 0.005 nM. For example, the EC₅₀ values for P92-si81-DV27, P92-si84-DV26, and P92-si82-DV27 were 0.0003 nM, 0.0004 nM, and 0.0006 nM, respectively. These sequences effectively inhibited PCSK9 gene expression at low concentrations.

### (i) Inhibition of PCSK9 gene expression by basic sequences modified using different templates

### (1) Basic sequence 5

**Table 14: Inhibition rates of PCSK9 gene expression by basic sequence 5 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si5-DV25 | 81.8 | 65.4 | 16.4 |
| | P92-si5-DV26 | 84.9 | 65.4 | 19.5 |
| | P92-si5-DV27 | 82.0 | 65.4 | 16.6 |
| | P92-si5-DV28 | 80.1 | 65.4 | 14.7 |
| 5 | P92-si5-DV29 | 80.7 | 65.4 | 15.3 |
| | P92-si5-DV30 | 65.3 | 65.4 | -0.1 |
| | P92-si5-DV31 | 62.4 | 65.4 | -3.0 |
| | P92-si5-DV21 | 68.2 | 65.4 | 2.8 |
| | P92-si5-DV22 | 69.1 | 65.4 | 3.7 |

### I. Templates DV25 to DV29 disclosed herein

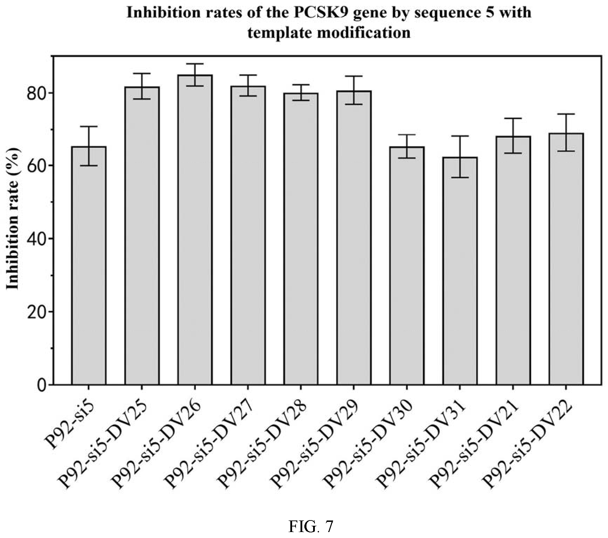

Basic sequence 5 modified using templates DV25 to DV29P designed in the present disclosure exhibited inhibition rates exceeding 80% on PCSK9 gene expression, indicating significantly enhanced inhibition of PCSK9 gene expression as compared to alternating 2'-O-methyl and 2'-fluoro modifications. For example, after modification using templates DV26 and DV27, the inhibition rates of P92-si5-DV26 and P92-si5-DV27 were increased by 19.5% and 16.6%, respectively (FIG. 7).

### II. Other modification templates disclosed herein

For basic sequence 5, after modification using templates DV30 and DV31, the resulting sequences P92-si5-DV30 and P92-si5-DV31 exhibited inhibition rates of 65.3% and 62.4% on PCSK9 gene expression, respectively, which were 0.1% and 3.0% lower than those of the corresponding alternately modified sequences, respectively.

After modification using templates DV26 and DV27 disclosed herein, the inhibition rates of P92-si5-DV26 and P92-si5-DV27 were increased by 19.6% and 16.7%, respectively, compared to P92-si5-DV30, and increased by 22.5% and 19.6%, respectively, compared to P92-si5-DV31, indicating a significant improvement.

### III. Advanced ESC templates DV21 (fluorinated sites: positions 2, 6, 14, and 16 of the antisense strand; positions 7, 9, 10, 11, 16, and 17 of the sense strand) and DV22 (fluorinated sites: positions 2, 6, 14, and 16 of the antisense strand; positions 7, 9, 10, and 11 of the sense strand) disclosed in the prior art

For basic sequence 5, after modification using templates DV21 and DV22, the resulting sequences P92-si5-DV21 and P92-si5-DV22 exhibited inhibition rates of 68.2% and 69.1% on PCSK9 gene expression, respectively, which were 2.8% and 3.7% higher than those of the alternately modified sequences, respectively.

After modification using templates DV26 and DV27 disclosed herein, the inhibition rates of P92-si5-DV26 and P92-si5-DV27 were increased by 16.7% and 13.8%, respectively, compared to P92-si5-DV21, and increased by 15.8% and 12.9%, respectively, compared to P92-si5-DV22, indicating a significant improvement.

It can be concluded that:
1) Basic sequence 5 modified using templates DV25 to DV29 disclosed herein showed significantly enhanced inhibition of PCSK9 gene expression as compared to the alternately modified sequences. For example, when basic sequence 5 was modified using DV26, the inhibition rate was increased by 19.5% compared to the alternately modified sequence.
2) The same siRNA sequence exhibited significant differences in activity when modified using different modification templates. For example, when basic sequence 5 was modified using templates DV26 and DV27 disclosed herein, the inhibition rate was increased by up to 22.5% compared to modification using templates DV30 and DV31 disclosed herein, indicating a significant improvement; the inhibition rate was increased by up to 16.7% compared to modification using the previously disclosed Advanced ESC templates DV21 and DV22, indicating a significant improvement.
3) The siRNA sequences modified using different templates varied greatly in activity, with differences of up to 20%. Thus, it is uncertain which modification template would confer high activity to a given siRNA sequence.

### (2) Basic sequence 51

**Table 15: Inhibition rates of PCSK9 gene expression by basic sequence 51 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si51-DV25 | 83.1 | 56.5 | 26.6 |
| | P92-si51-DV26 | 84.2 | 56.5 | 27.7 |
| | P92-si51-DV27 | 85.6 | 56.5 | 29.1 |
| | P92-si51-DV28 | 76.8 | 56.5 | 20.3 |
| 51 | P92-si51-DV29 | 78.9 | 56.5 | 22.4 |
| | P92-si51-DV30 | 63.5 | 56.5 | 7.0 |
| | P92-si51-DV31 | 62.8 | 56.5 | 6.3 |
| | P92-si51-DV21 | 64.3 | 56.5 | 7.8 |
| | P92-si51-DV22 | 65.1 | 56.5 | 8.6 |

### I. Templates DV25 to 29 disclosed herein

Basic sequence 51 modified using templates DV25 to DV29P designed in the present disclosure exhibited inhibition rates exceeding 75% on PCSK9 gene expression, indicating significantly enhanced inhibition of PCSK9 gene expression as compared to alternating 2'-O-methyl and 2'-fluoro modifications. For example, after modification using templates DV26 and DV27, the inhibition rates of P92-si51-DV26 and P92-si51-DV27 were increased by 27.7% and 29.1%, respectively.

### II. Other modification templates disclosed herein

For basic sequence 51, after modification using templates DV30 and DV31, the resulting sequences P92-si51-DV30 and P92-si51-DV31 exhibited inhibition rates of 63.5% and 62.8% on PCSK9 gene expression, respectively, which were only 7.0% and 6.3% higher than those of the corresponding alternately modified sequences, respectively.

After modification using templates DV26 and DV27 disclosed herein, the inhibition rates of P92-si51-DV26 and P92-si51-DV27 were increased by 20.7% and 22.1%, respectively, compared to P92-si51-DV30, and increased by 21.4% and 22.8%, respectively, compared to P92-si51-DV31, indicating a significant improvement.

### III. Advanced ESC templates DV21 (fluorinated sites: positions 2, 6, 14, and 16 of the antisense strand; positions 7, 9, 10, 11, 16, and 17 of the sense strand) and DV22 (fluorinated sites: positions 2, 6, 14, and 16 of the antisense strand; positions 7, 9, 10, and 11 of the sense strand) disclosed in the prior art

For basic sequence 51, after modification using the previously disclosed Advanced ESC templates DV21 and DV22, the resulting sequences P92-si51-DV21 and P92-si51-DV22 exhibited inhibition rates of 64.3% and 65.1% on PCSK9 gene expression, respectively, which were 7.8% and 8.6% higher than those of the alternately modified sequences, respectively.

After modification using templates DV26 and DV27 disclosed herein, the inhibition rates of P92-si51-DV26 and P92-si51-DV27 were increased by 19.9% and 21.3%, respectively, compared to P92-si51-DV21, and increased by 19.1% and 20.5%, respectively, compared to P92-si5-DV22, indicating a significant improvement.

It can be concluded that:
1) Basic sequence 51 modified using templates DV25 to DV29 disclosed herein showed significantly enhanced inhibition of PCSK9 gene expression as compared to the alternately modified sequences. For example, when basic sequence 51 was modified using DV27, the inhibition rate was increased by 29.1% compared to the alternately modified sequence.
2) The same siRNA sequence exhibited significant differences in activity when modified using different modification templates. For example, when basic sequence 51 was modified using templates DV26 and DV27 disclosed herein, the inhibition rate was increased by up to 22.8% compared to modification using templates DV30 and DV31 disclosed herein, indicating a significant improvement; the inhibition rate was increased by up to 21.3% compared to modification using the previously disclosed Advanced ESC templates DV21 and DV22, indicating a significant improvement.
3) The siRNA sequences modified using different templates varied greatly in activity, with differences of up to 20%. Thus, it is uncertain which modification template would confer high activity to a given siRNA sequence.

### (3) Basic sequence 81

**Table 16: Inhibition rates of PCSK9 gene expression by basic sequence 81 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si81-DV25 | 85.9 | 62.4 | 23.5 |
| | P92-si81-DV26 | 89.3 | 62.4 | 26.9 |
| | P92-si81-DV27 | 87.1 | 62.4 | 24.7 |
| | P92-si81-DV28 | 84.8 | 62.4 | 22.4 |
| 81 | P92-si81-DV29 | 85.6 | 62.4 | 23.2 |
| | P92-si81-DV30 | 71.6 | 62.4 | 9.2 |
| | P92-si81-DV31 | 73.5 | 62.4 | 11.1 |
| | P92-si81-DV21 | 78.3 | 62.4 | 15.9 |
| | P92-si81-DV22 | 75.1 | 62.4 | 12.7 |

### I. Templates DV25 to DV29 disclosed herein

Basic sequence 81 modified using templates DV25 to DV29P designed in the present disclosure exhibited inhibition rates exceeding 80% on PCSK9 gene expression, indicating significantly enhanced inhibition of PCSK9 gene expression as compared to alternating 2'-O-methyl and 2'-fluoro modifications. For example, after modification using templates DV26 and DV27, the inhibition rates of P92-si81-DV26 and P92-si81-DV27 were increased by 26.9% and 24.7%, respectively.

### II. Other modification templates disclosed herein

For basic sequence 81, after modification using templates DV30 and DV31, the resulting sequences P92-si81-DV30 and P92-si81-DV31 exhibited inhibition rates of 71.6% and 73.5% on PCSK9 gene expression, respectively, which were 9.2% and 11.1% higher than those of the corresponding alternately modified sequences, respectively.

After modification using templates DV26 and DV27 disclosed herein, the inhibition rates of P92-si81-DV26 and P92-si81-DV27 were increased by 17.7% and 15.5%, respectively, compared to P92-si81-DV30, and increased by 15.8% and 13.6%, respectively, compared to P92-si81-DV31, indicating a significant improvement.

### III. Advanced ESC templates DV21 (fluorinated sites: positions 2, 6, 14, and 16 of the antisense strand; positions 7, 9, 10, 11, 16, and 17 of the sense strand) and DV22 (fluorinated sites: positions 2, 6, 14, and 16 of the antisense strand; positions 7, 9, 10, and 11 of the sense strand) disclosed in the prior art

For basic sequence 81, after modification using templates DV21 and DV22, the resulting sequences P92-si81-DV21 and P92-si81-DV22 exhibited inhibition rates of 78.3% and 75.1% on PCSK9 gene expression, respectively, which were 15.9% and 12.7% higher than those of the alternately modified sequences, respectively.

After modification using templates DV26 and DV27 disclosed herein, the inhibition rates of P92-si81-DV26 and P92-si81-DV27 were increased by 11.0% and 8.8%, respectively, compared to P92-si81-DV21, and increased by 14.2% and 12.0%, respectively, compared to P92-si81-DV22, indicating a significant improvement.

It can be concluded that:
1) Basic sequence 81 modified using templates DV25 to 29 disclosed herein showed significantly enhanced inhibition of PCSK9 gene expression as compared to the alternately modified sequences. For example, when basic sequence 81 was modified using DV26, the inhibition rate was increased by 26.9% compared to the alternately modified sequence.
2) The same siRNA sequence exhibited significant differences in activity when modified using different modification templates. For example, when basic sequence 81 was modified using templates DV26 and DV27 disclosed herein, the inhibition rate was increased by up to 17.7% compared to modification using templates DV30 and DV31 disclosed herein, indicating a significant improvement; the inhibition rate was increased by up to 14.2% compared to modification using the previously disclosed Advanced ESC templates DV21 and DV22, indicating a significant improvement.
3) The siRNA sequences modified using different templates varied greatly in activity, with differences of up to approximately 20%. Thus, it is uncertain which modification template would confer high activity to a given siRNA sequence.

### (4) Basic sequence 82

**Table 17: Inhibition rates of PCSK9 gene expression by basic sequence 82 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si82-DV25 | 83.8 | 69.7 | 14.1 |
| | P92-si82-DV26 | 84.4 | 69.7 | 14.7 |
| | P92-si82-DV27 | 89.3 | 69.7 | 19.6 |
| | P92-si82-DV28 | 86.7 | 69.7 | 17.0 |
| 82 | P92-si82-DV29 | 89.1 | 69.7 | 19.4 |
| | P92-si82-DV30 | 70.2 | 69.7 | 0.5 |
| | P92-si82-DV31 | 69.7 | 69.7 | 0.0 |
| | P92-si82-DV21 | 76.5 | 69.7 | 6.8 |
| | P92-si82-DV22 | 75.4 | 69.7 | 5.7 |

### I. Templates DV25 to DV29 disclosed herein

Basic sequence 82 modified using templates DV25 to 29 designed in the present disclosure exhibited inhibition rates exceeding 80% on PCSK9 gene expression, indicating significantly enhanced inhibition of PCSK9 gene expression as compared to alternating 2'-O-methyl and 2'-fluoro modifications. For example, after modification using templates DV27 and DV29, the inhibition rates of P92-si82-DV27 and P92-si82-DV29 were increased by 19.6% and 19.4%, respectively.

### II. Other modification templates disclosed herein

For basic sequence 82, after modification using templates DV30 and DV31, the resulting sequences P92-si82-DV30 and P92-si82-DV31 exhibited inhibition rates of 70.2% and 69.7% on PCSK9 gene expression, respectively, which were comparable to those of the corresponding alternately modified sequences.

After modification using templates DV27 and DV29 disclosed herein, the inhibition rates of P92-si82-DV27 and P92-si82-DV29 were increased by 19.1% and 18.9%, respectively, compared to P92-si82-DV30, and increased by 19.6% and 19.4%, respectively, compared to P92-si82-DV31, indicating a significant improvement.

### III. Advanced ESC templates DV21 (fluorinated sites: positions 2, 6, 14, and 16 of the antisense strand; positions 7, 9, 10, 11, 16, and 17 of the sense strand) and DV22 (fluorinated sites: positions 2, 6, 14, and 16 of the antisense strand; positions 7, 9, 10, and 11 of the sense strand) disclosed in the prior art

For basic sequence 82, after modification using templates DV21 and DV22, the resulting sequences P92-si82-DV21 and P92-si82-DV22 exhibited inhibition rates of 76.5% and 75.4% on PCSK9 gene expression, respectively, which were 6.8% and 5.7% higher than those of the alternately modified sequences, respectively.

After modification using templates DV27 and DV29 disclosed herein, the inhibition rates of P92-si82-DV27 and P92-si82-DV29 were increased by 12.8% and 12.6%, respectively, compared to P92-si82-DV21, and increased by 13.9% and 13.7%, respectively, compared to P92-si82-DV22, indicating a significant improvement.

It can be concluded that:
1) Basic sequence 82 modified using templates DV25 to DV29 disclosed herein showed significantly enhanced inhibition of PCSK9 gene expression as compared to the alternately modified sequences. For example, when basic sequence 82 was modified using DV27, the inhibition rate was increased by 19.6% compared to the alternately modified sequence.
2) The same siRNA sequence exhibited significant differences in activity when modified using different modification templates. For example, when basic sequence 82 was modified using templates DV27 and DV29 disclosed herein, the inhibition rate was increased by up to 19.6% compared to modification using templates DV30 and DV31 disclosed herein, indicating a significant improvement; the inhibition rate was increased by up to 13.9% compared to modification using the previously disclosed Advanced ESC templates DV21 and DV22, indicating a significant improvement.
3) The siRNA sequences modified using different templates varied greatly in activity, with differences of up to approximately 20%. Thus, it is uncertain which modification template would confer high activity to a given siRNA sequence.

### (5) Basic sequence 84

**Table 18: Inhibition rates of PCSK9 gene expression by basic sequence 84 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si84-DV25 | 86.0 | 61.6 | 24.4 |
| | P92-si84-DV26 | 85.7 | 61.6 | 24.1 |
| | P92-si84-DV27 | 86.8 | 61.6 | 25.2 |
| | P92-si84-DV28 | 88.4 | 61.6 | 26.8 |
| 84 | P92-si84-DV29 | 87.5 | 61.6 | 25.9 |
| | P92-si84-DV30 | 70.3 | 61.6 | 8.7 |
| | P92-si84-DV31 | 72.4 | 61.6 | 10.8 |
| | P92-si84-DV21 | 77.7 | 61.6 | 16.1 |
| | P92-si84-DV22 | 75.1 | 61.6 | 13.5 |

### I. Templates DV25 to 29 disclosed herein

Basic sequence 84 modified using templates DV25 to DV29P designed in the present disclosure exhibited inhibition rates exceeding 85% on PCSK9 gene expression, indicating significantly enhanced inhibition of PCSK9 gene expression as compared to alternating 2'-O-methyl and 2'-fluoro modifications. For example, after modification using templates DV28 and DV29, the inhibition rates of P92-si84-DV28 and P92-si84-DV29 were increased by 26.8% and 25.9%, respectively.

### II. Other modification templates disclosed herein

For basic sequence 84, after modification using templates DV30 and DV31, the resulting sequences P92-si84-DV30 and P92-si84-DV31 exhibited inhibition rates of 70.3% and 72.4% on PCSK9 gene expression, respectively, which were 8.7% and 10.8% higher than those of the corresponding alternately modified sequences, respectively.

After modification using templates DV28 and DV29 disclosed herein, the inhibition rates of P92-si84-DV28 and P92-si84-DV29 were increased by 18.1% and 17.2%, respectively, compared to P92-si84-DV30, and increased by 16.0% and 15.1%, respectively, compared to P92-si84-DV31, indicating a significant improvement.

### III. Advanced ESC templates DV21 (fluorinated sites: positions 2, 6, 14, and 16 of the antisense strand; positions 7, 9, 10, 11, 16, and 17 of the sense strand) and DV22 (fluorinated sites: positions 2, 6, 14, and 16 of the antisense strand; positions 7, 9, 10, and 11 of the sense strand) disclosed in the prior art

For basic sequence 84, after modification using templates DV21 and DV22, the resulting sequences P92-si84-DV21 and P92-si84-DV22 exhibited inhibition rates of 77.7% and 75.1% on PCSK9 gene expression, respectively, which were 16.1% and 13.5% higher than those of the alternately modified sequences, respectively.

After modification using templates DV28 and DV29 disclosed herein, the inhibition rates of P92-si84-DV28 and P92-si84-DV29 were increased by 10.7% and 9.8%, respectively, compared to P92-si84-DV21, and increased by 13.3% and 12.4%, respectively, compared to P92-si84-DV22, indicating a significant improvement.

It can be concluded that:
1) Basic sequence 84 modified using templates DV25 to DV29 disclosed herein showed significantly enhanced inhibition of PCSK9 gene expression as compared to the alternately modified sequences. For example, when basic sequence 84 was modified using DV28, the inhibition rate was increased by 26.8% compared to the alternately modified sequence.
2) The same siRNA sequence exhibited significant differences in activity when modified using different modification templates. For example, when basic sequence 84 was modified using templates DV28 and DV29 disclosed herein, the inhibition rate was increased by up to 18.1% compared to modification using templates DV30 and DV31 disclosed herein, indicating a significant improvement; the inhibition rate was increased by up to 13.3% compared to modification using the previously disclosed Advanced ESC templates DV21 and DV22, indicating a significant improvement.
3) The siRNA sequences modified using different templates varied greatly in activity, with differences of up to approximately 20%. Thus, it is uncertain which modification template would confer high activity to a given siRNA sequence.

### (6) Basic sequence 3

**Table 19: Inhibition rates of PCSK9 gene expression by basic sequence 3 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si3-DV25 | 79.7 | 60.3 | 19.4 |
| | P92-si3-DV26 | 80.9 | 60.3 | 20.6 |
| 3 | P92-si3-DV27 | 83.0 | 60.3 | 22.7 |
| | P92-si3-DV28 | 84.3 | 60.3 | 24.0 |
| | P92-si3-DV29 | 85.0 | 60.3 | 24.7 |

1) Basic sequence 3 modified using templates DV25 to 29 designed in the present disclosure exhibited inhibition rates exceeding 75% on PCSK9 gene expression, indicating significantly enhanced inhibition of PCSK9 gene expression as compared to alternating 2'-O-methyl and 2'-fluoro modifications. For example, after modification using templates DV28 and DV29, the inhibition rates of P92-si3-DV28 and P92-si3-DV29 were increased by 24.0% and 24.7%, respectively.
2) It can be concluded that basic sequence 3 modified using templates DV25 to 29 disclosed herein showed significantly enhanced inhibition of PCSK9 gene expression as compared to alternating modifications.

### (7) Basic sequence 8

**Table 20: Inhibition rates of PCSK9 gene expression by basic sequence 8 modified using different templates**

| **Basic sequence** | **Template-modified sequence ID** | **Inhibition rate (%) of template-modified sequence** | **Inhibition rate (%) of corr. alternately modified sequence** | **Increase (%)** |
|---|---|---|---|---|
| | P92-si8-DV25 | 79.8 | 61.9 | 17.9 |
| | P92-si8-DV26 | 80.6 | 61.9 | 18.7 |
| 8 | P92-si8-DV27 | 81.6 | 61.9 | 19.7 |
| | P92-si8-DV28 | 82.1 | 61.9 | 20.2 |
| | P92-si8-DV29 | 79.5 | 61.9 | 17.6 |

1) Basic sequence 8 modified using templates DV25 to 29 designed in the present disclosure exhibited inhibition rates exceeding 75% on PCSK9 gene expression, indicating significantly enhanced inhibition of PCSK9 gene expression as compared to alternating 2'-O-methyl and 2'-fluoro modifications. For example, after modification using templates DV27 and DV28, the inhibition rates of P92-si8-DV27 and P92-si8-DV28 were increased by 19.7% and 20.2%, respectively.
2) It can be concluded that basic sequence 8 modified using templates DV25 to 29 disclosed herein showed significantly enhanced inhibition of PCSK9 gene expression as compared to alternating modifications.

### (8) Basic sequence 9

**Table 21: Inhibition rates of PCSK9 gene expression by basic sequence 9 modified using different templates**

| **Basic sequence** | **Template-modified sequence ID** | **Inhibition rate (%) of template-modified sequence** | **Inhibition rate (%) of corr. alternately modified sequence** | **Increase (%)** |
|---|---|---|---|---|
| | P92-si9-DV25 | 71.9 | 52.4 | 19.5 |
| | P92-si9-DV26 | 73.7 | 52.4 | 21.3 |
| 9 | P92-si9-DV27 | 79.6 | 52.4 | 27.2 |
| | P92-si9-DV28 | 77.8 | 52.4 | 25.4 |
| | P92-si9-DV29 | 76.8 | 52.4 | 24.4 |

1) Basic sequence 9 modified using templates DV25 to 29 designed in the present disclosure exhibited inhibition rates exceeding 70% on PCSK9 gene expression, indicating significantly enhanced inhibition of PCSK9 gene expression as compared to alternating 2'-O-methyl and 2'-fluoro modifications. For example, after modification using templates DV27 and DV28, the inhibition rates of P92-si9-DV27 and P92-si9-DV28 were increased by 27.2% and 25.4%, respectively.
2) It can be concluded that basic sequence 9 modified using templates DV25 to 29 disclosed herein showed significantly enhanced inhibition of PCSK9 gene expression as compared to alternating modifications.

### (9) Basic sequence 21

**Table 22: Inhibition rates of PCSK9 gene expression by basic sequence 21 modified using different templates**

| **Basic sequence** | **Template-modified sequence ID** | **Inhibition rate (%) of template-modified sequence** | **Inhibition rate (%) of corr. alternately modified sequence** | **Increase (%)** |
|---|---|---|---|---|
| | P92-si21-DV25 | 74.0 | 49.6 | 24.4 |
| | P92-si21-DV26 | 76.1 | 49.6 | 26.5 |
| 21 | P92-si21-DV27 | 71.1 | 49.6 | 21.5 |
| | P92-si21-DV28 | 71.5 | 49.6 | 21.9 |
| | P92-si21-DV29 | 72.0 | 49.6 | 22.4 |

1) Basic sequence 21 modified using templates DV25 to 29 designed in the present disclosure exhibited inhibition rates exceeding 70% on PCSK9 gene expression, indicating significantly enhanced inhibition of PCSK9 gene expression as compared to alternating 2'-O-methyl and 2'-fluoro modifications. For example, after modification using templates DV25 and DV26, the inhibition rates of P92-si21-DV25 and P92-si21-DV26 were increased by 24.4% and 26.5%, respectively.
2) It can be concluded that basic sequence 21 modified using templates DV25 to 29 disclosed herein showed significantly enhanced inhibition of PCSK9 gene expression as compared to alternating modifications.

### (10) Basic sequence 31

**Table 23: Inhibition rates of PCSK9 gene expression by basic sequence 31 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si31-DV25 | 79.2 | 56.3 | 22.9 |
| | P92-si31-DV26 | 78.9 | 56.3 | 22.6 |
| 31 | P92-si31-DV27 | 71.2 | 56.3 | 14.9 |
| | P92-si31-DV28 | 70.6 | 56.3 | 14.3 |
| | P92-si31-DV29 | 70.0 | 56.3 | 13.7 |

1) Basic sequence 31 modified using templates DV25 to 29 designed in the present disclosure exhibited inhibition rates exceeding 70% on PCSK9 gene expression, indicating significantly enhanced inhibition of PCSK9 gene expression as compared to alternating 2'-O-methyl and 2'-fluoro modifications. For example, after modification using templates DV25 and DV26, the inhibition rates of P92-si31-DV25 and P92-si31-DV26 were increased by 22.9% and 22.6%, respectively.
2) It can be concluded that basic sequence 31 modified using templates DV25 to 29 disclosed herein showed significantly enhanced inhibition of PCSK9 gene expression as compared to alternating modifications.

### (11) Basic sequence 73

**Table 24: Inhibition rates of PCSK9 gene expression by basic sequence 73 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si73-DV25 | 70.1 | 49.4 | 20.7 |
| | P92-si73-DV26 | 71.8 | 49.4 | 22.4 |
| 73 | P92-si73-DV27 | 77.6 | 49.4 | 28.2 |
| | P92-si73-DV28 | 80.0 | 49.4 | 30.6 |
| | P92-si73-DV29 | 82.1 | 49.4 | 32.7 |

1) Basic sequence 73 modified using templates DV25 to 29 designed in the present disclosure exhibited inhibition rates exceeding 70% on PCSK9 gene expression, indicating significantly enhanced inhibition of PCSK9 gene expression as compared to alternating 2'-O-methyl and 2'-fluoro modifications. For example, after modification using templates DV28 and DV29, the inhibition rates of P92-si73-DV28 and P92-si73-DV29 were increased by 30.6% and 32.7%, respectively.
2) It can be concluded that basic sequence 73 modified using templates DV25 to 29 disclosed herein showed significantly enhanced inhibition of PCSK9 gene expression as compared to alternating modifications.

### (12) Basic sequence 83

**Table 25: Inhibition rates of PCSK9 gene expression by basic sequence 83 modified using different templates**

| **Basic SEQ ID NO:** | **Template-modified sequence ID** | **Inhibition rate (%) of template-modified sequence** | **Inhibition rate (%) of corr. alternately modified sequence** | **Increase (%)** |
|---|---|---|---|---|
| | P92-si83-DV25 | 77.9 | 51.7 | 26.2 |
| | P92-si83-DV26 | 75.0 | 51.7 | 23.3 |
| 83 | P92-si83-DV27 | 82.6 | 51.7 | 30.9 |
| | P92-si83-DV28 | 78.6 | 51.7 | 26.9 |
| | P92-si83-DV29 | 81.4 | 51.7 | 29.7 |

1) Basic sequence 83 modified using templates DV25 to 29 designed in the present disclosure exhibited inhibition rates exceeding 75% on PCSK9 gene expression, indicating significantly enhanced inhibition of PCSK9 gene expression as compared to alternating 2'-O-methyl and 2'-fluoro modifications. For example, after modification using templates DV27 and DV29, the inhibition rates of P92-si83-DV27 and P92-si83-DV29 were increased by 30.9% and 29.7%, respectively.
2) It can be concluded that basic sequence 83 modified using templates DV25 to 29 disclosed herein showed significantly enhanced inhibition of PCSK9 gene expression as compared to alternating modifications.

### Summary:

(1) The identified basic sequences 5, 51, 81, 82, 84, 3, 8, 9, 21, 31, 73, and 83, when modified using templates DV25 to 29 designed in the present disclosure, exhibited significant inhibitory effects on PCSK9 gene expression, with inhibition rates exceeding 70%. Specifically, P92-si81-DV26, P92-si82-DV27, and P92-si82-DV29 exhibited inhibition rates of 89.3%, 89.3%, and 89.1%, respectively.
(2) The 12 sequences modified using templates DV25 to 29 disclosed herein showed significantly enhanced inhibition of PCSK9 gene expression as compared to the alternately modified sequences. For example, for basic sequence 51, the inhibition rate of P92-si51-DV27 (the sequence modified using template DV27) was 29.1% higher than that of the alternately modified sequence; for basic sequence 81, the inhibition rate of P92-si81-DV26 (the sequence modified using template DV26) was 26.9% higher than that of the alternately modified sequence.
(3) The same sequence modified using templates DV25 to 29 disclosed herein showed significantly enhanced inhibition of PCSK9 gene expression as compared to modification using the templates disclosed in the prior art. For example, for basic sequence 51, the sequence modified using template DV27 disclosed herein exhibited a 21.3% increase in inhibition rate compared to the sequence modified using the previously disclosed Advanced ESC template DV21.
(4) The same sequence modified using templates DV25 to 29 disclosed herein showed significantly enhanced inhibition of PCSK9 gene expression as compared to modification using templates DV30 and DV31 disclosed herein. For example, for basic sequence 51, the sequence modified using template DV27 disclosed herein exhibited a 22.8% increase in inhibition rate compared to the sequence modified using template DV31 disclosed herein.
(5) The sequences modified using different templates varied greatly in activity. For example, when basic sequence 5 was modified using DV26, the inhibition rate was increased by 22.5% compared to modification using DV31; when basic sequence 51 was modified using DV27, the inhibition rate was increased by 20.5% compared to modification using DV22. Thus, it is uncertain which modification template would confer high activity to a given siRNA sequence.

### (ii) EC₅₀ assay

A total of 12 template-modified sequences, including P92-si5-DV26, P92-si51-DV25, P92-si81-DV27, P92-si82-DV27, P92-si84-DV26, P92-si3-DV28, P92-si8-DV29, P92-si9-DV28, P92-si21-DV25, P92-si31-DV27, P92-si73-DV28, and P92-si83-DV26, were selected for EC₅₀ determination. The EC₅₀ values of these sequences were determined. The experimental results are shown in Table 26.

**Table 26: EC₅₀ values of modified sequences**

| Sequence ID | Sequence EC₅₀ (nM) | Sequence ID | Sequence EC₅₀ (nM) |
|---|---|---|---|
| P92-si5-DV26 | 0.0010 | P92-si8-DV29 | 0.0039 |
| P92-si51-DV25 | 0.0027 | P92-si9-DV28 | 0.0047 |
| P92-si81-DV27 | 0.0003 | P92-si21-DV25 | 0.0048 |
| P92-si82-DV27 | 0.0006 | P92-si31-DV27 | 0.0035 |
| P92-si84-DV26 | 0.0004 | P92-si73-DV28 | 0.0044 |
| P92-si3-DV28 | 0.0022 | P92-si83-DV26 | 0.0025 |

As shown in Table 26, these 12 sequences exhibited EC₅₀ values ranging from 0.0001 nM to 0.005 nM. Specifically, the EC₅₀ values for P92-si81-DV27, P92-si84-DV26, and P92-si82-DV27 were 0.0003 nM, 0.0004 nM, and 0.0006 nM, respectively. The results demonstrated that these sequences effectively inhibited PCSK9 gene expression at low concentrations.

### Example 5: Comparison of inhibitory effects of sequences disclosed in the prior art on PCSK9 gene expression

In this example, unmodified sequences disclosed in the prior art that are identical or similar to basic sequences 3, 5, 8, 9, 31, 81, 82, 83, and 84 disclosed herein were compared with the alternately modified sequences and the sequences modified using templates DV25 to 29 disclosed herein, in terms of their inhibitory effects on PCSK9 gene expression.

### 1. Experimental materials

### Test samples:

(1) Sequences disclosed in the prior art

**Table 27: Sequences disclosed in the prior art**

| Sequence ID | Position | Sense strand | **SEQ ID NO:** | Antisense strand | **SEQ ID NO:** | Source |
|---|---|---|---|---|---|---|
| si81 | 3546 | CUUUUGUAACUU GAAGAUAUU | 3 | AAUAUCUUCAAGUUA CAAAAGCA | 15 | CN115992138A |
| si84 | 3574 | GUUUUGUAGCAU UUUUAUUAA | 5 | UUAAUAAAAAUGCUA CAAAACCC | 17 | CN115992138A |
| si3 | 614 | CACCAAGAUCCUG CAUGUCUU | 6 | AAGACAUGCAGGAUC UUGGUGAG | 18 | WO2014089313A1, page 104 |
| si8 | 1083 | CUCAUAGGCCUGG AGUUUAUU | 7 | AAUAAACUCCAGGCC UAUGAGGG | 19 | CN108220295B, page 91 |
| si9 | 1089 | GGCCUGGAGUUU AUUCGGAAA | 8 | UUUCCGAAUAAACUC CAGGCCUA | 20 | WO2014089313A1, page 106 |
| si83 | 3572 | GGGUUUGUAGC AUUUUUAUU | 12 | AAUAAAAAUGCUACA AAACCCAG | 24 | CN115992138A, page 16 |
| 5P | 618 | AAGAUCCUGCAU GUCUUCCAU | 1 | AUGGAAGACAUGCAG GAUCUU | 417 | WO2023/017004Al |
| 82P | 3557 | GAAGAUAUUUAU UCUGGGUUU | 4 | AAACCCAGAAUAAAU AUCUUC | 418 | WO2023/017004Al |
| 31P | 2103 | GGUCUGGAAUGC AAAGUCAAG | 10 | CUUGACUUUGCAUUC CAGACC | 419 | CN115066498A, page 25 |

| | | | | | | |
|---|---|---|---|---|---|---|
| Note: The sequences disclosed in the above patent applications are all unmodified RNA sequences. | | | | | | |

(2) siRNA sequences with alternating 2'-OMe and 2'-F modifications and terminal phosphorothioate modifications in Example 2, including P92-si5, P92-si81, P92-si82, P92-si84, P92-si3, P92-si8, P92-si9, P92-si31, and P92-si83.
(3) siRNA sequences modified using templates DV25 to 29 (see Table 28 for specific sequences).

**Table 28: Sequences modified using templates DV25 to 29**

| **Basic SEQ ID NO:** | **Template-modified sequence ID** | **Sense sequence 5'-3'** | **Modified SEQ ID NO:/corr. basic SEQ ID NO:** | **Antisense sequence 5'-3'** | **Modified SEQ ID NO:/corr. basic SEQ ID NO:** |
|---|---|---|---|---|---|
| 5 | P92-si5-DV25 | Ams-Ams-Gm-Am-Um- Cm-Cf-Um-Gf-Cf-Af-Um- Gm-Um-Cm-Um-Uf-Cm- Cm-Am-Um | 337/1 | Ams-Ufs-Gm-Gm-Am-Af- Gm-Am-Cm-Am-Um-Gm- Cm-Af-Gm-Gf-Am-Um-Cm- Um-Ums-Gms-Gm | 376/13 |
| | P92-si5-DV26 | Ams-Ams-Gm-Am-Um- Cm-Cf-Um-Gf-Cf-Af-Um- Gm-Um-Cm-Um-Uf-Cm- Cm-Am-Um | 337/1 | Ams-Ufs-Gf-Gf-Am-Af-Gm- Am-Cm-Am-Um-Gm-Cm-Af- Gm-Gf-Am-Um-Cm-Um- Ums-Gms-Gm | 377/13 |
| | P92-si5-DV27 | Ams-Ams-Gm-Am-Um- Cm-Cf-Um-Gf-Cf-Af-Um- Gm-Um-Cm-Um-Uf-Cm- Cm-Am-Um | 337/1 | Ams-Ufs-Gm-Gf-Af-Af-Gm- Am-Cm-Am-Um-Gm-Cm-Af- Gm-Gf-Am-Um-Cm-Um- Ums-Gms-Gm | 378/13 |
| | P92-si5-DV28 | Ams-Ams-Gm-Am-Um- Cm-Cf-Um-Gf-Cm-Af- Um-Gm-Um-Cm-Um-Uf- Cm-Cm-Am-Um | 338/1 | Ams-Ufs-Gf-Gf-Am-Af-Gm- Am-Cm-Am-Um-Gm-Cm-Af- Gm-Gf-Am-Um-Cm-Um- Ums-Gms-Gm | 377/13 |
| | P92-si5-DV29 | Ams-Ams-Gm-Am-Um- Cm-Cf-Um-Gf-Cm-Af- Um-Gm-Um-Cm-Um-Uf- Cm-Cm-Am-Um | 338/1 | Ams-Ufs-Gm-Gf-Af-Af-Gm- Am-Cm-Am-Um-Gm-Cm-Af- Gm-Gf-Am-Um-Cm-Um- Ums-Gms-Gm | 378/13 |
| 81 | P92-si81-DV25 | Cms-Ums-Um-Um-Um- Gm-Uf-Am-Af-Cf-Uf-Um- Gm-Am-Am-Gm-Af-Um- Am-Um-Um | 347/3 | Ams-Afs-Um-Am-Um-Cf- Um-Um-Cm-Am-Am-Gm- Um-Uf-Am-Cf-Am-Am-Am- Am-Gms-Cms-Am | 384/15 |
| | P92-si81-DV26 | Cms-Ums-Um-Um-Um- Gm-Uf-Am-Af-Cf-Uf-Um- Gm-Am-Am-Gm-Af-Um- Am-Um-Um | 347/3 | Ams-Afs-Uf-Af-Um-Cf-Um- Um-Cm-Am-Am-Gm-Um-Uf- Am-Cf-Am-Am-Am-Am- Gms-Cms-Am | 385/15 |
| | P92-si81-DV27 | Cms-Ums-Um-Um-Um- Gm-Uf-Am-Af-Cf-Uf-Um- Gm-Am-Am-Gm-Af-Um- Am-Um-Um | 347/3 | Ams-Afs-Um-Af-Uf-Cf-Um- Um-Cm-Am-Am-Gm-Um-Uf- Am-Cf-Am-Am-Am-Am- Gms-Cms-Am | 386/15 |
| | P92-si81-DV28 | Cms-Ums-Um-Um-Um- Gm-Uf-Am-Af-Cm-Uf- Um-Gm-Am-Am-Gm-Af- Um-Am-Um-Um | 348/3 | Ams-Afs-Uf-Af-Um-Cf-Um- Um-Cm-Am-Am-Gm-Um-Uf- Am-Cf-Am-Am-Am-Am- Gms-Cms-Am | 385/15 |
| | P92-si81-DV29 | Cms-Ums-Um-Um-Um- Gm-Uf-Am-Af-Cm-Uf- Um-Gm-Am-Am-Gm-Af- Um-Am-Um-Um | 348/3 | Ams-Afs-Um-Af-Uf-Cf-Um- Um-Cm-Am-Am-Gm-Um-Uf- Am-Cf-Am-Am-Am-Am- Gms-Cms-Am | 386/15 |
| 82 | P92-si82-DV25 | Gms-Ams-Am-Gm-Am- Um-Af-Um-Uf-Uf-Af- Um-Um-Cm-Um-Gm-Gf- Gm-Um-Um-Um | 352/4 | Ams-Afs-Am-Cm-Cm-Cf-Am- Gm-Am-Am-Um-Am-Am-Af- Um-Af-Um-Cm-Um-Um- Cms-Ams-Am | 388/16 |
| | P92-si82-DV26 | Gms-Ams-Am-Gm-Am- Um-Af-Um-Uf-Uf-Af- Um-Um-Cm-Um-Gm-Gf- Gm-Um-Um-Um | 352/4 | Ams-Afs-Af-Cf-Cm-Cf-Am- Gm-Am-Am-Um-Am-Am-Af- Um-Af-Um-Cm-Um-Um- Cms-Ams-Am | 389/16 |
| | P92-si82-DV27 | Gms-Ams-Am-Gm-Am- Um-Af-Um-Uf-Uf-Af- Um-Um-Cm-Um-Gm-Gf- Gm-Um-Um-Um | 352/4 | Ams-Afs-Am-Cf-Cf-Cf-Am- Gm-Am-Am-Um-Am-Am-Af- Um-Af-Um-Cm-Um-Um- Cms-Ams-Am | 390/16 |
| | P92-si82-DV28 | Gms-Ams-Am-Gm-Am- Um-Af-Um-Uf-Um-Af- Um-Um-Cm-Um-Gm-Gf- Gm-Um-Um-Um | 353/4 | Ams-Afs-Af-Cf-Cm-Cf-Am- Gm-Am-Am-Um-Am-Am-Af- Um-Af-Um-Cm-Um-Um- Cms-Ams-Am | 389/16 |
| | P92-si82-DV29 | Gms-Ams-Am-Gm-Am- Um-Af-Um-Uf-Um-Af- Um-Um-Cm-Um-Gm-Gf- Gm-Um-Um-Um | 353/4 | Ams-Afs-Am-Cf-Cf-Cf-Am- Gm-Am-Am-Um-Am-Am-Af- Um-Af-Um-Cm-Um-Um- Cms-Ams-Am | 390/16 |
| 84 | P92-si84-DV25 | Gms-Ums-Um-Um-Um- Gm-Uf-Am-Gf-Cf-Af-Um- Um-Um-Um-Um-Af-Um- Um-Am-Am | 357/5 | Ums-Ufs-Am-Am-Um-Af- Am-Am-Am-Am-Um-Gm- Cm-Uf-Am-Cf-Am-Am-Am- Am-Cms-Cms-Cm | 392/17 |
| | P92-si84-DV26 | Gms-Ums-Um-Um-Um- Gm-Uf-Am-Gf-Cf-Af-Um- Um-Um-Um-Um-Af-Um- Um-Am-Am | 357/5 | Ums-Ufs-Af-Af-Um-Af-Am- Am-Am-Am-Um-Gm-Cm-Uf- Am-Cf-Am-Am-Am-Am- Cms-Cms-Cm | 393/17 |
| | P92-si84-DV27 | Gms-Ums-Um-Um-Um- Gm-Uf-Am-Gf-Cf-Af-Um- Um-Um-Um-Um-Af-Um- Um-Am-Am | 357/5 | Ums-Ufs-Am-Af-Uf-Af-Am- Am-Am-Am-Um-Gm-Cm-Uf- Am-Cf-Am-Am-Am-Am- Cms-Cms-Cm | 394/17 |
| | P92-si84-DV28 | Gms-Ums-Um-Um-Um- Gm-Uf-Am-Gf-Cm-Af- Um-Um-Um-Um-Um-Af- Um-Um-Am-Am | 358/5 | Ums-Ufs-Af-Af-Um-Af-Am- Am-Am-Am-Um-Gm-Cm-Uf- Am-Cf-Am-Am-Am-Am- Cms-Cms-Cm | 393/17 |
| | P92-si84-DV29 | Gms-Ums-Um-Um-Um- Gm-Uf-Am-Gf-Cm-Af- Um-Um-Um-Um-Um-Af- Um-Um-Am-Am | 358/5 | Ums-Ufs-Am-Af-Uf-Af-Am- Am-Am-Am-Um-Gm-Cm-Uf- Am-Cf-Am-Am-Am-Am- Cms-Cms-Cm | 394/17 |
| 3 | P92-si3-DV25 | Cms-Ams-Cm-Cm-Am- Am-Gf-Am-Uf-Cf-Cf-Um- Gm-Cm-Am-Um-Gf-Um- Cm-Um-Um | 362/6 | Ams-Afs-Gm-Am-Cm-Af- Um-Gm-Cm-Am-Gm-Gm- Am-Uf-Cm-Uf-Um-Gm-Gm- Um-Gms-Ams-Gm | 396/18 |
| | P92-si3-DV26 | Cms-Ams-Cm-Cm-Am- Am-Gf-Am-Uf-Cf-Cf-Um- Gm-Cm-Am-Um-Gf-Um- Cm-Um-Um | 362/6 | Ams-Afs-Gf-Af-Cm-Af-Um- Gm-Cm-Am-Gm-Gm-Am-Uf- Cm-Uf-Um-Gm-Gm-Um- Gms-Ams-Gm | 397/18 |
| | P92-si3-DV27 | Cms-Ams-Cm-Cm-Am- Am-Gf-Am-Uf-Cf-Cf-Um- Gm-Cm-Am-Um-Gf-Um- Cm-Um-Um | 362/6 | Ams-Afs-Gm-Af-Cf-Af-Um- Gm-Cm-Am-Gm-Gm-Am-Uf- Cm-Uf-Um-Gm-Gm-Um- Gms-Ams-Gm | 398/18 |
| | P92-si3-DV28 | Cms-Ams-Cm-Cm-Am- Am-Gf-Am-Uf-Cm-Cf- Um-Gm-Cm-Am-Um-Gf- Um-Cm-Um-Um | 363/6 | Ams-Afs-Gf-Af-Cm-Af-Um- Gm-Cm-Am-Gm-Gm-Am-Uf- Cm-Uf-Um-Gm-Gm-Um- Gms-Ams-Gm | 397/18 |
| | P92-si3-DV29 | Cms-Ams-Cm-Cm-Am- Am-Gf-Am-Uf-Cm-Cf- Um-Gm-Cm-Am-Um-Gf- Um-Cm-Um-Um | 363/6 | Ams-Afs-Gm-Af-Cf-Af-Um- Gm-Cm-Am-Gm-Gm-Am-Uf- Cm-Uf-Um-Gm-Gm-Um- Gms-Ams-Gm | 398/18 |
| 8 | P92-si8-DV25 | Cms-Ums-Cm-Am-Um- Am-Gf-Gm-Cf-Cf-Uf-Gm- Gm-Am-Gm-Um-Uf-Um- Am-Um-Um | 364/7 | Ams-Afs-Um-Am-Am-Af- Cm-Um-Cm-Cm-Am-Gm- Gm-Cf-Cm-Uf-Am-Um-Gm- Am-Gms-Gms-Gm | 399/19 |
| | P92-si8-DV26 | Cms-Ums-Cm-Am-Um- Am-Gf-Gm-Cf-Cf-Uf-Gm- Gm-Am-Gm-Um-Uf-Um- Am-Um-Um | 364/7 | Ams-Afs-Uf-Af-Am-Af-Cm- Um-Cm-Cm-Am-Gm-Gm-Cf- Cm-Uf-Am-Um-Gm-Am- Gms-Gms-Gm | 400/19 |
| | P92-si8-DV27 | Cms-Ums-Cm-Am-Um- Am-Gf-Gm-Cf-Cf-Uf-Gm- Gm-Am-Gm-Um-Uf-Um- Am-Um-Um | 364/7 | Ams-Afs-Um-Af-Af-Af-Cm- Um-Cm-Cm-Am-Gm-Gm-Cf- Cm-Uf-Am-Um-Gm-Am- Gms-Gms-Gm | 401/19 |
| | P92-si8-DV28 | Cms-Ums-Cm-Am-Um- Am-Gf-Gm-Cf-Cm-Uf- Gm-Gm-Am-Gm-Um-Uf- Um-Am-Um-Um | 365/7 | Ams-Afs-Uf-Af-Am-Af-Cm- Um-Cm-Cm-Am-Gm-Gm-Cf- Cm-Uf-Am-Um-Gm-Am- Gms-Gms-Gm | 400/19 |
| | P92-si8-DV29 | Cms-Ums-Cm-Am-Um- Am-Gf-Gm-Cf-Cm-Uf- Gm-Gm-Am-Gm-Um-Uf- Um-Am-Um-Um | 365/7 | Ams-Afs-Um-Af-Af-Af-Cm- Um-Cm-Cm-Am-Gm-Gm-Cf- Cm-Uf-Am-Um-Gm-Am- Gms-Gms-Gm | 401/19 |
| 9 | P92-si9-DV25 | Gms-Gms-Cm-Cm-Um- Gm-Gf-Am-Gf-Uf-Uf- Um-Am-Um-Um-Cm-Gf- Gm-Am-Am-Am | 366/8 | Ums-Ufs-Um-Cm-Cm-Gf-Am- Am-Um-Am-Am-Am-Cm-Uf- Cm-Cf-Am-Gm-Gm-Cm-Cms- Ums-Am | 402/20 |
| | P92-si9-DV26 | Gms-Gms-Cm-Cm-Um- Gm-Gf-Am-Gf-Uf-Uf- Um-Am-Um-Um-Cm-Gf- Gm-Am-Am-Am | 366/8 | Ums-Ufs-Uf-Cf-Cm-Gf-Am- Am-Um-Am-Am-Am-Cm-Uf- Cm-Cf-Am-Gm-Gm-Cm-Cms- Ums-Am | 403/20 |
| | P92-si9-DV27 | Gms-Gms-Cm-Cm-Um- Gm-Gf-Am-Gf-Uf-Uf- Um-Am-Um-Um-Cm-Gf- Gm-Am-Am-Am | 366/8 | Ums-Ufs-Um-Cf-Cf-Gf-Am- Am-Um-Am-Am-Am-Cm-Uf- Cm-Cf-Am-Gm-Gm-Cm-Cms- Ums-Am | 404/20 |
| | P92-si9-DV28 | Gms-Gms-Cm-Cm-Um- Gm-Gf-Am-Gf-Um-Uf- Um-Am-Um-Um-Cm-Gf- Gm-Am-Am-Am | 367/8 | Ums-Ufs-Uf-Cf-Cm-Gf-Am- Am-Um-Am-Am-Am-Cm-Uf- Cm-Cf-Am-Gm-Gm-Cm-Cms- Ums-Am | 403/20 |
| | P92-si9-DV29 | Gms-Gms-Cm-Cm-Um- Gm-Gf-Am-Gf-Um-Uf- Um-Am-Um-Um-Cm-Gf- Gm-Am-Am-Am | 367/8 | Ums-Ufs-Um-Cf-Cf-Gf-Am- Am-Um-Am-Am-Am-Cm-Uf- Cm-Cf-Am-Gm-Gm-Cm-Cms- Ums-Am | 404/20 |
| 31 | P92-si31-DV25 | Gms-Gms-Um-Cm-Um- Gm-Gf-Am-Af-Uf-Gf-Cm- Am-Am-Am-Gm-Uf-Cm- Am-Am-Gm | 370/10 | Cms-Ufs-Um-Gm-Am-Cf-Um- Um-Um-Gm-Cm-Am-Um-Uf- Cm-Cf-Am-Gm-Am-Cm-Cms- Ums-Gm | 408/22 |
| | P92-si31-DV26 | Gms-Gms-Um-Cm-Um- Gm-Gf-Am-Af-Uf-Gf-Cm- Am-Am-Am-Gm-Uf-Cm- Am-Am-Gm | 370/10 | Cms-Ufs-Uf-Gf-Am-Cf-Um- Um-Um-Gm-Cm-Am-Um-Uf- Cm-Cf-Am-Gm-Am-Cm-Cms- Ums-Gm | 409/22 |
| | P92-si31-DV27 | Gms-Gms-Um-Cm-Um- Gm-Gf-Am-Af-Uf-Gf-Cm- Am-Am-Am-Gm-Uf-Cm- Am-Am-Gm | 370/10 | Cms-Ufs-Um-Gf-Af-Cf-Um- Um-Um-Gm-Cm-Am-Um-Uf- Cm-Cf-Am-Gm-Am-Cm-Cms- Ums-Gm | 410/22 |
| | P92-si31-DV28 | Gms-Gms-Um-Cm-Um- Gm-Gf-Am-Af-Um-Gf- Cm-Am-Am-Am-Gm-Uf- Cm-Am-Am-Gm | 371/10 | Cms-Ufs-Uf-Gf-Am-Cf-Um- Um-Um-Gm-Cm-Am-Um-Uf- Cm-Cf-Am-Gm-Am-Cm-Cms- Ums-Gm | 409/22 |
| | P92-si31-DV29 | Gms-Gms-Um-Cm-Um- Gm-Gf-Am-Af-Um-Gf- Cm-Am-Am-Am-Gm-Uf- Cm-Am-Am-Gm | 371/10 | Cms-Ufs-Um-Gf-Af-Cf-Um- Um-Um-Gm-Cm-Am-Um-Uf- Cm-Cf-Am-Gm-Am-Cm-Cms- Ums-Gm | 410/22 |
| 83 | P92-si83-DV25 | Gms-Gms-Gm-Um-Um- Um-Uf-Gm-Uf-Af-Gf-Cm- Am-Um-Um-Um-Uf-Um- Am-Um-Um | 374/12 | Ams-Afs-Um-Am-Am-Af- Am-Am-Um-Gm-Cm-Um- Am-Cf-Am-Af-Am-Am-Cm- Cm-Cms-Ams-Gm | 414/24 |
| | P92-si83-DV26 | Gms-Gms-Gm-Um-Um- Um-Uf-Gm-Uf-Af-Gf-Cm- Am-Um-Um-Um-Uf-Um- Am-Um-Um | 374/12 | Ams-Afs-Uf-Af-Am-Af-Am- Am-Um-Gm-Cm-Um-Am-Cf- Am-Af-Am-Am-Cm-Cm-Cms- Ams-Gm | 415/24 |
| | P92-si83-DV27 | Gms-Gms-Gm-Um-Um- Um-Uf-Gm-Uf-Af-Gf-Cm- Am-Um-Um-Um-Uf-Um- Am-Um-Um | 374/12 | Ams-Afs-Um-Af-Af-Af-Am- Am-Um-Gm-Cm-Um-Am-Cf- Am-Af-Am-Am-Cm-Cm-Cms- Ams-Gm | 416/24 |
| | P92-si83-DV28 | Gms-Gms-Gm-Um-Um- Um-Uf-Gm-Uf-Am-Gf- Cm-Am-Um-Um-Um-Uf- Um-Am-Um-Um | 375/12 | Ams-Afs-Uf-Af-Am-Af-Am- Am-Um-Gm-Cm-Um-Am-Cf- Am-Af-Am-Am-Cm-Cm-Cms- Ams-Gm | 415/24 |
| | P92-si83-DV29 | Gms-Gms-Gm-Um-Um- Um-Uf-Gm-Uf-Am-Gf- Cm-Am-Um-Um-Um-Uf- Um-Am-Um-Um | 375/12 | Ams-Afs-Um-Af-Af-Af-Am- Am-Um-Gm-Cm-Um-Am-Cf- Am-Af-Am-Am-Cm-Cm-Cms- Ams-Gm | 416/24 |

Cell line: Hep3B cells, provided by WuXi AppTec Co., Ltd. (ATCC-tings-1618164).

Hep3B cells were cultured in EMEM (ATCC-30-2003) supplemented with 10% fetal bovine serum (FBS, ExCell Bio-FSP500), 1% penicillin-streptomycin (HyClone-SV30010), 1% non-essential amino acids (Gibco-11140-050), and 1% GlutaMAX (Gibco-35050-061).

Solvents: sterile nuclease-free water, Gibco DMEM.

### 2. Experimental methods

The inhibitory effects of the test samples on PCSK9 mRNA expression in HEP3B cells were evaluated by qRT-PCR.

### 2.1 Cell culture

Subcultured Hep3B cells in the logarithmic growth phase were cultured in RPMI 1640 culture medium containing 10% fetal bovine serum (supplemented with penicillin and streptomycin at 100 µL/mL), and incubated in a cell incubator at 37°C with 5% CO₂. The medium was exchanged once daily. The cells were digested with 0.25% trypsin, followed by centrifugation at 1000 rpm for 5 minutes. The supernatant was discarded, and fresh medium was added for continued culture.

### 2.2 Cell transfection

According to a ratio of 1:0.06 (wt/wt), 2 µL of siRNA (1 µg/µL) was added to 18 µL of sterile nuclease-free water, mixed well, followed by addition of 22 µL of the prepared LNP formulation. The mixture was mixed well and incubated at room temperature for 15 minutes. A 12.5 µL aliquot was taken for encapsulation efficiency analysis. The final transfection amount per well was calculated as: (1 µg theoretical loading amount / siRNA purity) / drug loading concentration, resulting in a final siRNA concentration of 0.05 nM per well.

Negative control group: 22 µL of the prepared LNP formulation was added to 20 µL of sterile nuclease-free water, mixed well, followed by incubation at room temperature for 15 minutes.

After cross-mixing, the plate was incubated in a cell incubator at 37°C with 5% CO₂ for 40 hours.

### 2.3 PCSK9 mRNA analysis

### 1) RNA extraction

The procedure was the same as "1) RNA extraction" described in "2.3 PCSK9 mRNA analysis" of Example 2.

### 2) Measurement of RNA concentration

The procedure was the same as "2) Measurement of RNA concentration" described in "2.3 PCSK9 mRNA analysis" of Example 2.

### 3) Quantification of PCSK9 mRNA

In a 15 mL centrifuge tube, all components except primers and templates were added in 7.5 × 84 = 630 aliquots (labeled as A).

1.5 mL EP tubes were labeled. 77 µL of A and 420 ng of total RNA were added in 7 aliquots per tube, and mixed well for later use.

The forward and reverse primers for both the internal reference gene GAPDH and the target gene PCSK9 were mixed well for later use.

11 µL of B and 1 µL of C were added to each well of a PCR plate, which was sealed, then centrifuged at 3000 rpm for 1 minute, and subjected to instrumental analysis.

*The step was performed on ice under low-temperature conditions.

The plate was placed into the qPCR instrument and run according to the following program:
Reverse transcription: 55°C for 15 minutes;
Pre-denaturation: 95°C for 30 seconds;
Cycling: 95°C for 10 seconds; 60°C for 35 seconds; 40 cycles;
Melting curve: 95°C for 15 seconds; 60°C for 60 seconds; 95°C for 15 seconds.

The total run time was approximately 2 hours. After analysis of the experimental results, 2^{-ΔΔCt} was calculated.

### 2.4 Data processing

PCSK9 mRNA expression rate (%) was calculated using the following formula: 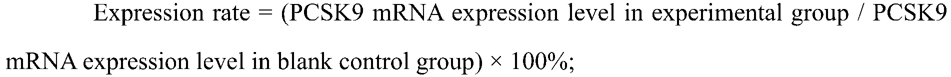 Expression rate = (PCSK9 mRNA expression level in experimental group / PCSK9 mRNA expression level in blank control group) × 100%;
$Inhibition rate of PCSK 9 gene expression = 100 % - expression rate \left(%\right) .$

### 3. Experimental results

The detailed experimental results are shown in Tables 29 and 30.

The experimental results demonstrated that both the alternately modified sequences and the sequences modified using specific templates disclosed herein exhibited significantly enhanced PCSK9 inhibitory activity compared to identical or similar unmodified sequences disclosed in the prior art. For example, the identical unmodified sequence si84 exhibited an inhibition rate of 38.6%. After alternating modification, the inhibition rate was increased to 61.6%, which was 23.0% higher than that of the unmodified sequence si84. After modification using template DV26 disclosed herein, the inhibition rate was increased to 86.8%, which was 48.2% higher than that of the unmodified sequence si84.

The sequence 31P disclosed in the prior art differs from the sequence si31 disclosed herein only by lacking two bases (UG) at the 3' end of the antisense strand, with all other positions being identical. However, the unmodified sequence si31 disclosed herein exhibited a 10.4% higher inhibition rate than 31P. The alternately modified sequence exhibited an inhibition rate of 59.5%, which was 21.2% higher than that of 31P. After modification using template DV26 disclosed herein, the inhibition rate was increased to 80.2%, which was 41.9% higher than that of 31P.
**(1) Compared to the identical unmodified sequences disclosed in the prior art, the alternately modified sequences and the sequences modified using specific templates disclosed herein showed significantly enhanced inhibition of PCSK9 gene expression, with an increase of up to 40%.**

Compared to the identical sequences disclosed in the prior art, the alternately modified sequences and the sequences modified using specific templates disclosed herein showed significantly enhanced inhibition of PCSK9 gene expression. For example, the unmodified sequence si84 exhibited an inhibition rate of 38.6%. After alternating modification, the inhibition rate was increased to 61.6%, which was 23.0% higher than that of the unmodified sequence si84. After modification using template DV26 disclosed herein, the inhibition rate was increased to 86.8%, which was 48.2% higher than that of the unmodified sequence si84.

**Table 29: Inhibition rates of PCSK9 gene expression by identical sequences disclosed in the prior art**

| **Sequences disclosed in the prior** a**rt** | | **Alternately modified sequence** | | | **Template-modified sequence** | | |
|---|---|---|---|---|---|---|---|
| **Name** | **Inh (%)** | **Sequence ID** | **Inh (%)** | **Increase vs sequences disclosed in the prior art (%)** | **Sequence ID** | **Inh (%)** | **Increase vs sequences disclosed in the prior art (%)** |
| si81 | 50.0 | P92-si81 | 60.4 | 10.4 | P92-si81-DV25 | 85.5 | 35.5 |
| | | | | | P92-si81-DV26 | 87.7 | 37.7 |
| | | | | | P92-si81-DV27 | 86.7 | 36.7 |
| | | | | | P92-si81-DV28 | 84.6 | 34.6 |
| | | | | | P92-si81-DV29 | 84.0 | 34.0 |
| si84 | 38.6 | P92-si84 | 61.6 | 23.0 | P92-si84-DV25 | 84.9 | 46.3 |
| | | | | | P92-si84-DV26 | 86.8 | 48.2 |
| | | | | | P92-si84-DV27 | 84.5 | 45.9 |
| | | | | | P92-si84-DV28 | 86.2 | 47.6 |
| | | | | | P92-si84-DV29 | 86.4 | 47.8 |
| si3 | 51.7 | P92-si3 | 61.7 | 10.0 | P92-si3-DV25 | 77.4 | 25.7 |
| | | | | | P92-si3-DV26 | 78.5 | 26.8 |
| | | | | | P92-si3-DV27 | 84.2 | 32.5 |
| | | | | | P92-si3-DV28 | 82.5 | 30.8 |
| | | | | | P92-si3-DV29 | 85.2 | 33.5 |
| si8 | 55.2 | P92-si8 | 62.4 | 7.2 | P92-si8-DV25 | 77.2 | 22.0 |
| | | | | | P92-si8-DV26 | 78.3 | 23.1 |
| | | | | | P92-si8-DV27 | 72.0 | 16.8 |
| | | | | | P92-si8-DV28 | 73.1 | 17.9 |
| | | | | | P92-si8-DV29 | 74.8 | 19.6 |
| si9 | 41.5 | P92-si9 | 54.7 | 13.2 | P92-si9-DV25 | 71.2 | 29.7 |
| | | | | | P92-si9-DV26 | 70.8 | 29.3 |
| | | | | | P92-si9-DV27 | 80.1 | 38.6 |
| | | | | | P92-si9-DV28 | 76.5 | 35.0 |
| | | | | | P92-si9-DV29 | 75.0 | 33.5 |
| si83 | 35.2 | P92-si83 | 51.5 | 16.3 | P92-si83-DV25 | 78.6 | 43.4 |
| | | | | | P92-si83-DV26 | 77.3 | 42.1 |
| | | | | | P92-si83-DV27 | 81.4 | 46.2 |
| | | | | | P92-si83-DV28 | 79.2 | 44.0 |
| | | | | | P92-si83-DV29 | 83.4 | 48.2 |

As shown in Table 29, the unmodified sequences si81, si84, si3, si8, si9, and si83 disclosed in the prior art, which are identical to the sequences disclosed herein, showed significantly enhanced inhibition of PCSK9 gene expression after alternating modification and modification using the templates disclosed herein. For example, the unmodified sequence si84 exhibited an inhibition rate of 38.6%. After alternating modification, P92-si84 exhibited an inhibition rate of 61.6%, which was 23.0% higher than that of the unmodified sequence si84. After modification using templates DV25 to 29 disclosed herein, P92-si84-DV25, P92-si84-DV26, P92-si84-DV27, P92-si84-DV28, and P92-si84-DV29 exhibited inhibition rates of 84.9%, 86.8%, 84.5%, 86.2%, and 86.4%, respectively, which were more than 40% higher than that of the unmodified sequence si84.

**(2) Compared to the unmodified sequences with minor differences disclosed in the prior art, the unmodified sequences, alternately modified sequences, and template-modified sequences disclosed herein showed significantly enhanced inhibition of PCSK9 gene expression, with an increase of up to 40%.**

Compared to the sequences with minor differences disclosed in the prior art, the unmodified sequences, alternately modified sequences, and template-modified sequences disclosed herein showed significantly enhanced inhibition of PCSK9 gene expression. For example, the unmodified sequence 31P exhibited an inhibition rate of 38.3%. The similar unmodified sequence si31 disclosed herein exhibited an inhibition rate of 48.7%, which was 10.4% higher than that of 31P. The alternately modified sequence exhibited an inhibition rate of 59.5%, which was 21.2% higher than that of 31P. After modification using template DV26 disclosed herein, the inhibition rate was increased to 80.2%, which was 41.9% higher than that of 31P.

**Table 30: Inhibition rates of PCSK9 gene expression by similar sequences disclosed in the prior art and disclosed herein**

| **Sequences disclosed in the prior art** | | **Unmodified sequences disclosed herein** | | | **Alternately modified sequences disclosed herein** | | | **Template-modified sequences disclosed herein** | | |
|---|---|---|---|---|---|---|---|---|---|---|
| **Name** | **Inh (%)** | **Seq ID** | **Inh (%)** | **Increase vs sequences disclosed in the prior art (%)** | **Seq ID** | **Inh (%)** | **Increase vs sequences disclosed in the prior art (%)** | **Seq ID** | **Inh (%)** | **Increase vs sequences disclosed in the prior art (%)** |
| 5P | 41.8 | si5 | 50.1 | 8.3 | P92-si5 | 63.8 | 22.0 | P92-si5-DV25 | 81.2 | 39.4 |
| | | | | | | | | P92-si5-DV26 | 81.5 | 39.7 |
| | | | | | | | | P92-si5-DV27 | 82.6 | 40.8 |
| | | | | | | | | P92-si5-DV28 | 77.0 | 35.2 |
| | | | | | | | | P92-si5-DV29 | 76.1 | 34.3 |
| 82P | 46.7 | si82 | 53.8 | 7.1 | P92-si82 | 68.3 | 21.6 | P92-si82-DV25 | 82.1 | 35.4 |
| | | | | | | | | P92-si82-DV26 | 84.2 | 37.5 |
| | | | | | | | | P92-si82-DV27 | 88.0 | 41.3 |
| | | | | | | | | P92-si82-DV28 | 86.7 | 40.0 |
| | | | | | | | | P92-si82-DV29 | 89.5 | 42.8 |
| 31P | 38.3 | si31 | 48.7 | 10.4 | P92-si31 | 59.5 | 21.2 | P92-si31-DV25 | 79.6 | 41.3 |
| | | | | | | | | P92-si31-DV26 | 80.2 | 41.9 |
| | | | | | | | | P92-si31-DV27 | 70.0 | 31.7 |
| | | | | | | | | P92-si31-DV28 | 73.9 | 35.6 |
| | | | | | | | | P92-si31-DV29 | 70.8 | 32.5 |

### I. Sequence comparison

As shown in Table 31, the sequences 5P, 31P, and 82P disclosed in the prior art are highly similar to the sequences si5, si31, and si82 disclosed herein, with only minor differences. See the table below for a detailed comparison.

**Table 31: Comparison between sequences disclosed in the prior art and sequences disclosed herein**

| **Name** | **Sense strand** | **Basic SEQ ID NO:** | **Antisense strand** | **Basic SEQ ID NO:** | **Source** |
|---|---|---|---|---|---|
| 5P | AAGAUCCUGCAUGU CUUCCAU | 1 | AUGGAAGACAUGCAGG AUCUU | 417 | WO2023/017004Al |
| si5 | AAGAUCCUGCAUGU CUUCCAU | 1 | AUGGAAGACAUGCAGG AUCUUGG | 13 | Sequences disclosed herein |
| 31P | GGUCUGGAAUGCAA AGUCAAG | 10 | CUUGACUUUGCAUUCCA GACC | 419 | CN115066498A, page 25 |
| si31 | GGUCUGGAAUGCAA AGUCAAG | 10 | CUUGACUUUGCAUUCCA GACCUG | 22 | Sequences disclosed herein |
| 82P | GAAGAUAUUUAUUC UGGGUUU | 4 | AAACCCAGAAUAAAUA UCUUC | 418 | WO2023/017004Al |
| si82 | GAAGAUAUUUAUUC UGGGUUU | 4 | AAACCCAGAAUAAAUA UCUUCAA | 16 | Sequences disclosed herein |

As shown in Table 31, the sequence 5P disclosed in the prior art differs from the sequence si5 disclosed herein only by lacking two bases (GG) at the 3' end of the antisense strand; the sequence 31P differs from the sequence si31 disclosed herein only by lacking two bases (UG) at the 3' end of the antisense strand; the sequence 82P differs from the sequence si82 disclosed herein only by lacking two bases (AA) at the 3' end of the antisense strand, with all other positions being identical.

### II. Activity comparison

**1) The unmodified sequences disclosed herein exhibited significantly enhanced activity compared to the similar unmodified sequences disclosed in the prior art. For example, the unmodified sequence si31 disclosed herein exhibited a 10.4% increase in inhibition rate compared to the structurally similar 31P.**

The unmodified sequence si5 disclosed herein exhibited an inhibition rate of 50.1% on PCSK9 gene expression, which was 8.3% higher than that of the similar sequence 5P disclosed in the prior art (inhibition rate of 41.8%), indicating a significant improvement.

The unmodified sequence si82 disclosed herein exhibited an inhibition rate of 53.8% on PCSK9 gene expression, which was 7.1% higher than that of the similar sequence 82P disclosed in the prior art (inhibition rate of 46.7%), indicating a significant improvement.

The unmodified sequence si31 disclosed herein exhibited an inhibition rate of 48.7% on PCSK9 gene expression, which was 10.4% higher than that of the similar sequence 31P disclosed in the prior art (inhibition rate of 38.3%), indicating a significant improvement.

**2) The alternately modified sequences disclosed herein exhibited significantly enhanced activity compared to the similar unmodified sequences disclosed in the prior art. For example, the alternately modified sequence P92-si5 disclosed herein exhibited a 22.0% increase in inhibition rate compared to the sequence 5P.**

For basic sequence 5 disclosed herein, after alternating modification, the resulting sequence P92-si5 exhibited an inhibition rate of 63.8% on PCSK9 gene expression, which was 22.0% higher than that of the unmodified sequence 5P disclosed in the prior art, indicating a significant improvement.

For basic sequence 82 disclosed herein, after alternating modification, the resulting sequence P92-si82 exhibited an inhibition rate of 68.3% on PCSK9 gene expression, which was 21.6% higher than that of the unmodified sequence 82P disclosed in the prior art, indicating a significant improvement.

For basic sequence 31 disclosed herein, after alternating modification, the resulting sequence P92-si31 exhibited an inhibition rate of 59.5% on PCSK9 gene expression, which was 21.2% higher than that of the unmodified sequence 31P disclosed in the prior art, indicating a significant improvement.

**3) The sequences modified using the templates disclosed herein exhibited significantly enhanced activity compared to the similar unmodified sequences disclosed in the prior art.** F**or example, the alternately modified sequence P92-si82-DV29 disclosed herein exhibited a 42.8% increase in inhibition rate compared to the sequence 82P.**

For basic sequence 5 disclosed herein, after modification using templates 25 to 29, the inhibition rate was increased by more than 30% compared to the unmodified sequence 5P disclosed in the prior art. For example, P92-si5-DV27 exhibited a 40.8% increase, indicating a significant improvement.

For basic sequence 82 disclosed herein, after modification using templates 25 to 29, the inhibition rate was increased by more than 30% compared to the unmodified sequence 82P disclosed in the prior art. For example, P92-si82-DV29 exhibited a 42.8% increase, indicating a significant improvement.

For basic sequence 31 disclosed herein, after modification using templates 25 to 29, the inhibition rate was increased by more than 30% compared to the unmodified sequence 31P disclosed in the prior art. For example, P92-si31-DV26 exhibited a 41.9% increase, indicating a significant improvement.

It can be concluded that, compared to the similar unmodified sequences disclosed in the prior art, the unmodified sequences, alternately modified sequences, and template-modified sequences disclosed herein showed significantly enhanced inhibition of PCSK9 gene expression, with an increase of up to 40%.

### Summary:

(1) Compared to the identical sequences disclosed in the prior art, the alternately modified sequences and the sequences modified using specific templates disclosed herein showed significantly enhanced inhibition of PCSK9 gene expression. For example, the unmodified sequence si84 exhibited an inhibition rate of 38.6%. After alternating modification, the inhibition rate was increased to 61.6%, which was 23.0% higher than that of the unmodified sequence si84. After modification using template DV26 disclosed herein, the inhibition rate was increased to 86.8%, which was 48.2% higher than that of the unmodified sequence si84.
(2) Compared to the similar sequences disclosed in the prior art, the unmodified sequences, alternately modified sequences, and template-modified sequences disclosed herein showed significantly enhanced inhibition of PCSK9 gene expression. For example, the unmodified sequence si31 disclosed herein exhibited a 10.4% increase in inhibition rate compared to the similar sequence 31P disclosed in the prior art. For the sequence si5 disclosed herein, the alternately modified sequence P92-si5 exhibited a 22.0% increase in inhibition rate compared to the unmodified sequence 5P disclosed in the prior art, which was similar to si5. For the sequence si82 disclosed herein, after modification using template DV29 disclosed herein, the resulting sequence P92-si82-DV29 exhibited a 42.8% increase in inhibition rate compared to the unmodified sequence 82P disclosed in the prior art, which was similar to si82.

### Example 6: Inhibitory effect of sequences with specific anti-off-target designs on PCSK9 and off-target genes

In practical applications of siRNA, there are numerous instances where the expression of non-target mRNAs, which are only partially complementary to the guide strand (antisense strand), is inhibited. Research by Alnylam Pharmaceuticals has demonstrated that the hepatotoxicity of N-acetylgalactosamine (GalNAc)-conjugated siRNAs is primarily attributed to off-target effects, which arise from a microRNA (miRNA)-like recognition mechanism leading to unintended gene silencing.

To address the issue, Alnylam Pharmaceuticals introduced a glycol nucleic acid (GNA) modification at position 7 of the antisense strand of siRNA in their latest fifth-generation template design to disrupt the seed region of the antisense strand, thereby significantly reducing off-target effects and mitigating hepatotoxicity. Such modifications can interfere with the binding of siRNA to unintended targets via seed-mediated recognition, thereby inhibiting off-target effects.

Natural 5'-phosphorylation or directly introduced 5'-phosphorylation may undergo dephosphorylation in cells. Directly 5'-phosphorylated oligonucleotide strands may undergo 90% dephosphorylation after 2 hours of circulation in blood and be completely dephosphorylated after 24 hours. The 5'-phosphorylation design (5'-E-VP), in which E-vinylphosphonate replaces the bridging oxygen, provides optimal phosphorylation efficiency and enhanced stability.

Based on the above, to reduce off-target effects of the sequences and to evaluate the impact of 5'-phosphorylation on off-target effects, in this example, basic sequences 5, 51, 81, and 84 modified using DV25 to 29 were employed, which incorporate 5'-E-VP modification at the 5' end of the antisense strand and GNA anti-off-target modification at position 7.

Furthermore, the sequences P92-si3, P92-si8, P92-si21, P92-si31, P92-si73, and P92-si83 with alternating 2'-O-methyl and 2'-fluoro modifications were compared with their counterparts (P92-si3+, P92-si8+, P92-si21+, P92-si31+, P92-si73+, and P92-si83+) incorporating GNA anti-off-target modification at position 7 of the antisense strand to evaluate off-target effects.

The experimental results demonstrated that, at a concentration of 10 nM, the sequences incorporating anti-off-target design exhibited significant inhibitory effects on the target gene PCSK9, while their inhibitory effects on off-target genes were significantly reduced. The findings indicate that anti-off-target design does not compromise the inhibitory effects of the alternately modified and template-modified sequences disclosed herein on PCSK9 gene expression, but can significantly inhibit off-target effects.

### 1. Experimental materials

### 1) Test samples:

Alternately modified sequences: P92-si3, P92-si8, P92-si9, P92-si21, P92-si31, P92-si73, and P92-si83.

Alternately modified sequences with anti-off-target design: P92-si3+, P92-si8+, P92-si9+, P92-si21+, P92-si31+, P92-si73+, and P92-si83+ (Table 32).

Basic sequences 5, 84, 81, and 51: incorporating template modification; template modification and 5'-E-VP modification; template modification and anti-off-target design; template modification, 5'-E-VP modification, and anti-off-target design (Table 33).

### 2) Sequence synthesis

### I. Alternately modified and template-modified sequences

The sequences were synthesized using the method described in Example 1.

### II. siRNA sequences containing 5'-E-VP

The siRNA sequences were synthesized using the method described in Example 1, except that the base at the 5' end of the antisense strand (the last base) was synthesized using a monomer containing a phosphonate group at the 5' end, such as vinyl-(E)-phosphonate-A-OMe phosphoramidite monomer (Formula 9) or vinyl-(E)-phosphonate-U-OMe phosphoramidite monomer (Formula 10), the structures of which are as follows:

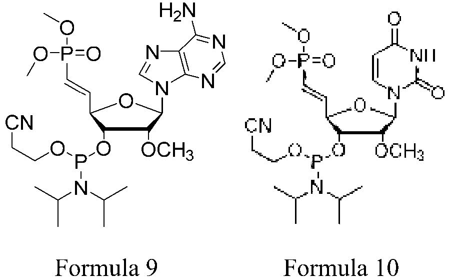

### III. siRNA sequences with anti-off-target design

The siRNA sequences were synthesized using the method described in Example 1, except that the base at position 7 from the 5' end of the antisense strand was synthesized using a GNA monomer, whose structure is as follows:

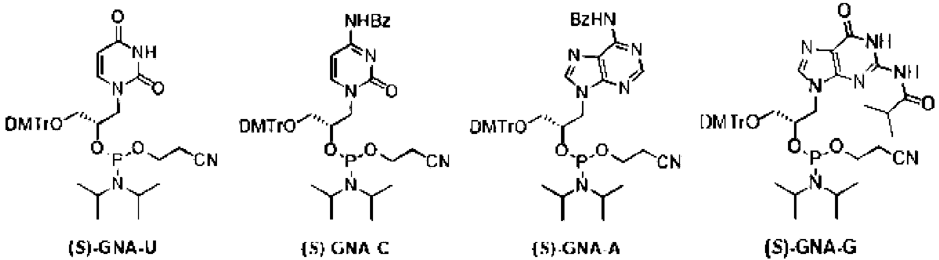

**Table 32: Alternately modified sequences with anti-off-target design**

| **Sequence ID** | **Sense strand 5'-3'** | **Modified SEQ ID NO:/corr. basic SEQ ID NO:** | **Antisense strand 5'-3'** | **Modified SEQ ID NO:/corr. basic SEQ ID NO:** |
|---|---|---|---|---|
| P92-si3+ | Cfs-Ams-Cf-Cm-Af-Am-Gf- Am-Uf-Cm-Cf-Um-Gf-Cm-Af- Um-Gf-Um-Cf-Um-Uf | 174/6 | Ams-Afs-Gm-Af-Cm-Af-Ugna- Gf-Cm-Af-Gm-Gf-Am-Uf-Cm- Uf-Um-Gf-Gm-Uf-Gms-Afs-Gm | 420/18 |
| P92-si8+ | Cfs-Ums-Cf-Am-Uf-Am-Gf- Gm-Cf-Cm-Uf-Gm-Gf-Am-Gf- Um-Uf-Um-Af-Um-Uf | 175/7 | Ams-Afs-Um-Af-Am-Af-Cgna- Uf-Cm-Cf-Am-Gf-Gm-Cf-Cm- Uf-Am-Uf-Gm-Af-Gms-Gfs-Gm | 421/19 |
| P92-si9+ | Gfs-Gms-Cf-Cm-Uf-Gm-Gf- Am-Gf-Um-Uf-Um-Af-Um-Uf- Cm-Gf-Gm-Af-Am-Af | 176/8 | Ums-Ufs-Um-Cf-Cm-Gf-Agna- Af-Um-Af-Am-Af-Cm-Uf-Cm- Cf-Am-Gf-Gm-Cf-Cms-Ufs-Am | 422/20 |
| P92-si21+ | Cfs-Ams-Gf-Cm-Af-Cm-Cf- Um-Gf-Cm-Uf-Um-Uf-Gm-Uf- Gm-Uf-Cm-Af-Cm-Af | 177/9 | Ums-Gfs-Um-Gf-Am-Cf-Agna- Cf-Am-Af-Am-Gf-Cm-Af-Gm- Gf-Um-Gf-Cm-Uf-Gms-Cfs-Am | 423/21 |
| P92-si31+ | Gfs-Gms-Uf-Cm-Uf-Gm-Gf- Am-Af-Um-Gf-Cm-Af-Am-Af- Gm-Uf-Cm-Af-Am-Gf | 178/10 | Cms-Ufs-Um-Gf-Am-Cf-Ugna- Uf-Um-Gf-Cm-Af-Um-Uf-Cm- Cf-Am-Gf-Am-Cf-Cms-Ufs-Gm | 424/22 |
| P92-si73+ | Gfs-Cms-Gf-Gm-Af-Gm-Af- Um-Gf-Cm-Uf-Um-Cf-Um-Af- Am-Gf-Gm-Cf-Am-Uf | 179/11 | Ams-Ufs-Gm-Cf-Cm-Uf-Ugna- Af-Gm-Af-Am-Gf-Cm-Af-Um- Cf-Um-Cf-Cm-Gf-Cms-Cfs-Am | 425/23 |
| P92-si83+ | Gfs-Gms-Gf-Um-Uf-Um-Uf- Gm-Uf-Am-Gf-Cm-Af-Um-Uf- Um-Uf-Um-Af-Um-Uf | 180/12 | Ams-Afs-Um-Af-Am-Af-Agna- Af-Um-Gf-Cm-Uf-Am-Cf-Am- Af-Am-Af-Cm-Cf-Cms-Afs-Gm | 426/24 |

All of the sequences listed above adopt an alternating 2'-O-methyl (2'-OMe) and 2'-fluoro (2'-F) modification pattern. Specifically:
(1) In the sense strand, all odd-numbered positions are modified with 2'-F, and all even-numbered positions are modified with 2'-OMe.
(2) In the antisense strand, all odd-numbered positions are modified with 2'-OMe, and all even-numbered positions are modified with 2'-F.
(3) There are two phosphorothioate modifications at the 5' end of the sense strand and at both the 5' and 3' ends of the antisense strand.
(4) The base at position 7 (5'-3') of the antisense strand is modified with GNA.

**Table 33: Sequences incorporating template modification, 5'-E-VP modification, and anti-off-target design**

| **Sequence ID** | **Sense strand 5'-3'** | **Modified SEQ ID NO:/corr. basic SEQ ID NO:** | **Antisense strand 5'-3'** | **Modified SEQ ID NO:/corr. basic SEQ ID NO:** |
|---|---|---|---|---|
| D5-DV25 | Ams-Ams-Gm-Am-Um-Cm-Cf- Um-Gf-Cf-Af-Um-Gm-Um- Cm-Um-Uf-Cm-Cm-Am-Um | 337/1 | Ams-Ufs-Gm-Gm-Am-Af-Gm-Am- Cm-Am-Um-Gm-Cm-Af-Gm-Gf- Am-Um-Cm-Um-Ums-Gms-Gm | 376/13 |
| D5-DV25+ | Ams-Ams-Gm-Am-Um-Cm-Cf- Um-Gf-Cf-Af-Um-Gm-Um- Cm-Um-Uf-Cm-Cm-Am-Um | 337/1 | Ams-Ufs-Gm-Gm-Am-Af-Ggna-Am- Cm-Am-Um-Gm-Cm-Af-Gm-Gf- Am-Um-Cm-Um-Ums-Gms-Gm | 427/13 |
| D5-DV25P | Ams-Ams-Gm-Am-Um-Cm-Cf- Um-Gf-Cf-Af-Um-Gm-Um- Cm-Um-Uf-Cm-Cm-Am-Um | 337/1 | AmsEVP-Ufs-Gm-Gm-Am-Af-Gm- Am-Cm-Am-Um-Gm-Cm-Af-Gm- Gf-Am-Um-Cm-Um-Ums-Gms-Gm | 428/13 |
| D5-DV25+P | Ams-Ams-Gm-Am-Um-Cm-Cf- Um-Gf-Cf-Af-Um-Gm-Um- Cm-Um-Uf-Cm-Cm-Am-Um | 337/1 | AmsEVP-Ufs-Gm-Gm-Am-Af-Ggna- Am-Cm-Am-Um-Gm-Cm-Af-Gm- Gf-Am-Um-Cm-Um-Ums-Gms-Gm | 429/13 |
| D51-DV26 | Ums-Gms-Gm-Am-Gm-Gm-Cf- Um-Uf-Af-Gf-Cm-Um-Um- Um-Cm-Uf-Gm-Gm-Am-Um | 342/2 | Ams-Ufs-Cf-Cf-Am-Gf-Am-Am- Am-Gm-Cm-Um-Am-Af-Gm-Cf- Cm-Um-Cm-Cm-Ams-Ums-Um | 381/14 |
| D51-DV26+ | Ums-Gms-Gm-Am-Gm-Gm-Cf- Um-Uf-Af-Gf-Cm-Um-Um- Um-Cm-Uf-Gm-Gm-Am-Um | 342/2 | Ams-Ufs-Cf-Cf-Am-Gf-Agna-Am- Am-Gm-Cm-Um-Am-Af-Gm-Cf- Cm-Um-Cm-Cm-Ams-Ums-Um | 430/14 |
| D51-DV26P | Ums-Gms-Gm-Am-Gm-Gm-Cf- Um-Uf-Af-Gf-Cm-Um-Um- Um-Cm-Uf-Gm-Gm-Am-Um | 342/2 | AmsEVP-Ufs-Cf-Cf-Am-Gf-Am- Am-Am-Gm-Cm-Um-Am-Af-Gm- Cf-Cm-Um-Cm-Cm-Ams-Ums-Um | 431/14 |
| D51-DV26+P | Ums-Gms-Gm-Am-Gm-Gm-Cf- Um-Uf-Af-Gf-Cm-Um-Um- Um-Cm-Uf-Gm-Gm-Am-Um | 342/2 | AmsEVP-Ufs-Cf-Cf-Am-Gf-Agna- Am-Am-Gm-Cm-Um-Am-Af-Gm- Cf-Cm-Um-Cm-Cm-Ams-Ums-Um | 432/14 |
| D81-DV25 | Cms-Ums-Um-Um-Um-Gm-Uf- Am-Af-Cf-Uf-Um-Gm-Am- Am-Gm-Af-Um-Am-Um-Um | 347/3 | Ams-Afs-Um-Am-Um-Cf-Um-Um- Cm-Am-Am-Gm-Um-Uf-Am-Cf- Am-Am-Am-Am-Gms-Cms-Am | 384/15 |
| D81-DV25+ | Cms-Ums-Um-Um-Um-Gm-Uf- Am-Af-Cf-Uf-Um-Gm-Am- Am-Gm-Af-Um-Am-Um-Um | 347/3 | Ams-Afs-Um-Am-Um-Cf-Ugna-Um- Cm-Am-Am-Gm-Um-Uf-Am-Cf- Am-Am-Am-Am-Gms-Cms-Am | 433/15 |
| D81-DV25P | Cms-Ums-Um-Um-Um-Gm-Uf- Am-Af-Cf-Uf-Um-Gm-Am- Am-Gm-Af-Um-Am-Um-Um | 347/3 | AmsEVP-Afs-Um-Am-Um-Cf-Um- Um-Cm-Am-Am-Gm-Um-Uf-Am- Cf-Am-Am-Am-Gms-Cms-Am | 434/15 |
| D81-DV25+P | Cms-Ums-Um-Um-Um-Gm-Uf- Am-Af-Cf-Uf-Um-Gm-Am- Am-Gm-Af-Um-Am-Um-Um | 347/3 | AmsEVP-Afs-Um-Am-Um-Cf-Ugna- Um-Cm-Am-Am-Gm-Um-Uf-Am- Cf-Am-Am-Am-Gms-Cms-Am | 435/15 |
| D81-DV27 | Cms-Ums-Um-Um-Um-Gm-Uf- Am-Af-Cf-Uf-Um-Gm-Am- Am-Gm-Af-Um-Am-Um-Um | 347/3 | Ams-Afs-Um-Af-Uf-Cf-Um-Um- Cm-Am-Am-Gm-Um-Uf-Am-Cf- Am-Am-Am-Am-Gms-Cms-Am | 386/15 |
| D81-DV27+ | Cms-Ums-Um-Um-Um-Gm-Uf- Am-Af-Cf-Uf-Um-Gm-Am- Am-Gm-Af-Um-Am-Um-Um | 347/3 | Ams-Afs-Um-Af-Uf-Cf-Ugna-Um- Cm-Am-Am-Gm-Um-Uf-Am-Cf- Am-Am-Am-Am-Gms-Cms-Am | 436/15 |
| D81-DV27P | Cms-Ums-Um-Um-Um-Gm-Uf- Am-Af-Cf-Uf-Um-Gm-Am- Am-Gm-Af-Um-Am-Um-Um | 347/3 | AmsEVP-Afs-Um-Af-Uf-Cf-Um- Um-Cm-Am-Am-Gm-Um-Uf-Am- Cf-Am-Am-Am-Am-Gms-Cms-Am | 437/15 |
| D81-DV27+P | Cms-Ums-Um-Um-Um-Gm-Uf- Am-Af-Cf-Uf-Um-Gm-Am- Am-Gm-Af-Um-Am-Um-Um | 347/3 | AmsEVP-Afs-Um-Af-Uf-Cf-Ugna- Um-Cm-Am-Am-Gm-Um-Uf-Am- Cf-Am-Am-Am-Am-Gms-Cms-Am | 438/15 |
| D81-DV28 | Cms-Ums-Um-Um-Um-Gm-Uf- Am-Af-Cm-Uf-Um-Gm-Am- Am-Gm-Af-Um-Am-Um-Um | 348/3 | Ams-Afs-Uf-Af-Um-Cf-Um-Um- Cm-Am-Am-Gm-Um-Uf-Am-Cf- Am-Am-Am-Am-Gms-Cms-Am | 385/15 |
| D81-DV28+ | Cms-Ums-Um-Um-Um-Gm-Uf- Am-Af-Cm-Uf-Um-Gm-Am- Am-Gm-Af-Um-Am-Um-Um | 348/3 | Ams-Afs-Uf-Af-Um-Cf-Ugna-Um- Cm-Am-Am-Gm-Um-Uf-Am-Cf- Am-Am-Am-Am-Gms-Cms-Am | 439/15 |
| D81-DV28P | Cms-Ums-Um-Um-Um-Gm-Uf- Am-Af-Cm-Uf-Um-Gm-Am- Am-Gm-Af-Um-Am-Um-Um | 348/3 | AmsEVP-Afs-Uf-Af-Um-Cf-Um- Um-Cm-Am-Am-Gm-Um-Uf-Am- Cf-Am-Am-Am-Am-Gms-Cms-Am | 440/15 |
| D81-DV28+P | Cms-Ums-Um-Um-Um-Gm-Uf- Am-Af-Cm-Uf-Um-Gm-Am- Am-Gm-Af-Um-Am-Um-Um | 348/3 | AmsEVP-Afs-Uf-Af-Um-Cf-Ugna- Um-Cm-Am-Am-Gm-Um-Uf-Am- Cf-Am-Am-Am-Am-Gms-Cms-Am | 441/15 |
| D84-DV26 | Gms-Ums-Um-Um-Um-Gm-Uf- Am-Gf-Cf-Af-Um-Um-Um- Um-Um-Af-Um-Um-Am-Am | 357/5 | Ums-Ufs-Af-Af-Um-Af-Am-Am- Am-Am-Um-Gm-Cm-Uf-Am-Cf- Am-Am-Am-Am-Cms-Cms-Cm | 393/17 |
| D84-DV26+ | Gms-Ums-Um-Um-Um-Gm-Uf- Am-Gf-Cf-Af-Um-Um-Um- Um-Um-Af-Um-Um-Am-Am | 357/5 | Ums-Ufs-Af-Af-Um-Af-Agna-Am- Am-Am-Um-Gm-Cm-Uf-Am-Cf- Am-Am-Am-Am-Cms-Cms-Cm | 442/17 |
| D84-DV26P | Gms-Ums-Um-Um-Um-Gm-Uf- Am-Gf-Cf-Af-Um-Um-Um- Um-Um-Af-Um-Um-Am-Am | 357/5 | UmsEVP-Ufs-Af-Af-Um-Af-Am- Am-Am-Am-Um-Gm-Cm-Uf-Am- Cf-Am-Am-Am-Cms-Cms-Cm | 443/17 |
| D84-DV26+P | Gms-Ums-Um-Um-Um-Gm-Uf- Am-Gf-Cf-Af-Um-Um-Um- Um-Um-Af-Um-Um-Am-Am | 357/5 | UmsEVP-Ufs-Af-Af-Um-Af-Agna- Am-Am-Am-Um-Gm-Cm-Uf-Am- Cf-Am-Am-Am-Cms-Cms-Cm | 444/17 |
| D84-DV27 | Gms-Ums-Um-Um-Um-Gm-Uf- Am-Gf-Cf-Af-Um-Um-Um- Um-Um-Af-Um-Um-Am-Am | 357/5 | Ums-Ufs-Am-Af-Uf-Af-Am-Am- Am-Am-Um-Gm-Cm-Uf-Am-Cf- Am-Am-Am-Am-Cms-Cms-Cm | 394/17 |
| D84-DV27+ | Gms-Ums-Um-Um-Um-Gm-Uf- Am-Gf-Cf-Af-Um-Um-Um- Um-Um-Af-Um-Um-Am-Am | 357/5 | Ums-Ufs-Am-Af-Uf-Af-Agna-Am- Am-Am-Um-Gm-Cm-Uf-Am-Cf- Am-Am-Am-Am-Cms-Cms-Cm | 445/17 |
| D84-DV27P | Gms-Ums-Um-Um-Um-Gm-Uf- Am-Gf-Cf-Af-Um-Um-Um- Um-Um-Af-Um-Um-Am-Am | 357/5 | UmsEVP-Ufs-Am-Af-Uf-Af-Am- Am-Am-Am-Um-Gm-Cm-Uf-Am- Cf-Am-Am-Am-Cms-Cms-Cm | 446/17 |
| D84-DV27+P | Gms-Ums-Um-Um-Um-Gm-Uf- Am-Gf-Cf-Af-Um-Um-Um- Um-Um-Af-Um-Um-Am-Am | 357/5 | UmsEVP-Ufs-Am-Af-Uf-Af-Agna- Am-Am-Am-Um-Gm-Cm-Uf-Am- Cf-Am-Am-Am-Am-Cms-Cms-Cm | 447/17 |

Cell line: Hep3B cells, provided by WuXi AppTec Co., Ltd. (ATCC-tings-1618164).

Hep3B cells were cultured in EMEM (ATCC-30-2003) supplemented with 10% fetal bovine serum (FBS, ExCell Bio-FSP500), 1% penicillin-streptomycin (HyClone-SV30010), 1% non-essential amino acids (Gibco-11140-050), and 1% GlutaMAX (Gibco-35050-061).

Solvents: sterile nuclease-free water, Gibco DMEM.

### 2. Experimental methods

### 1) Compound dilution

For the off-target assay, the test samples were diluted to a concentration of 10 nM and tested in triplicate.

### Digestion and counting of Hep3B cells:

Hep3B cells at 80% confluence were rinsed with Dulbecco's Phosphate-Buffered Saline (DPBS), and digested with 0.05% trypsin for 3 to 10 minutes. The cells were collected, counted using Countstar Rigel S2, and adjusted to a density of 2 × 10⁵ cells/mL.

### 2) Preparation of transfection reagent

Preparation of RNAiMAX transfection reagent: RNAiMAX was mixed with Opti-MEM at a ratio of 3:97 in a 15 mL centrifuge tube according to the required volume, vortexed thoroughly for 15 seconds, followed by incubation at room temperature for 15 minutes. 40 µL of RNAiMAX Opti-MEM and 40 µL of appropriately diluted compound were added to each corresponding well of a dilution plate, mixed well, followed by incubation for 15 minutes.

### 3) Cell seeding

Hep3B cells (2 × 10⁴ cells/well) were seeded into a 96-well cell culture plate, during which a mixture of siRNA compound and RNAiMAX Opti-MEM transfection reagent was added to each well. A control group containing RNAiMAX Opti-MEM without siRNA compound was also included.

Anti-off-target assay: The mixture of siRNA compound and RNAiMAX Opti-MEM was transferred from the dilution plate to a 96-well cell culture plate at 20 µL/well, followed by addition of 100 µL of cells per well, resulting in a final volume of 120 µL per well. After seeding, the plate was incubated in an incubator at 37°C with 5% CO₂ for 24 hours.

### 4) RNA extraction and reverse transcription

At 24 hours post-transfection, the culture medium was removed and the cells were collected for RNA extraction. Total RNA was extracted using the RNeasy^{®} 96 Kit (QIAGEN, 74182) according to the instructions provided by the manufacturer. Subsequently, cDNA was synthesized using the FastKing RT Kit (with gDNase) (TIANGEN, KR116-02) according to the instructions provided by the manufacturer.

### 5) RT-qPCR

Target cDNA was tested by qPCR, with GAPDH cDNA used as an internal control in parallel. 8 µL of prepared qPCR reaction mixture and 2 µL of sample cDNA were added to a 384-well plate. TaqMan qPCR was performed according to the following program: 95°C for 10 minutes, followed by 40 cycles of 95°C for 15 seconds and 60°C for 1 minute. SYBR qPCR was performed according to the following program: 50°C for 2 minutes, 95°C for 10 minutes, followed by 40 cycles of 95°C for 15 seconds and 60°C for 1 minute; finally, a melting curve analysis was performed at 95°C for 15 seconds, 60°C for 1 minute, and 95°C for 15 seconds.

### 6) Data analysis

The RNA expression levels of the target gene in each sample were calculated based on Ct values using the ΔΔCt relative quantification method. Relative expression of the target gene was expressed as 2^{-ΔΔCt}.

The calculation formula is as follows:
$ΔCt = mean Ct value of the target gene - mean Ct value of the reference gene ;$
$ΔΔCt = ΔCt \left(treatment group\right) - ΔCt \left(RNAiMAX control group\right) ;$

Relative mRNA expression of the target gene = 2^{-ΔΔCt}; 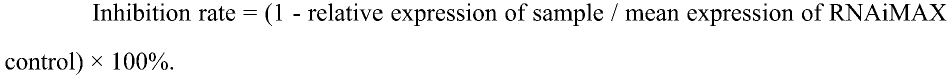 Inhibition rate = (1 - relative expression of sample / mean expression of RNAiMAX control) × 100%.

### 3. Experimental results

**(1) All modified sequences exhibited significant inhibitory effects on PCSK9 gene expression.** For example, the alternately modified sequence P92-si3 exhibited an inhibition rate of 75.6%, while the template-modified and 5'-E-VP-modified sequence D84-DV27P exhibited an inhibition rate as high as 94.4%.

The inhibition rates of PCSK9 gene expression by all test samples are shown in Tables 34 to 36.

All alternately modified sequences exhibited significant inhibitory effects on PCSK9 gene expression at a concentration of 10 nM. For example, the alternately modified sequence P92-si3 exhibited an inhibition rate of 75.6%.

**I. The alternately modified sequences exhibited significant inhibitory effects on PCSK9 gene expression, with inhibition rates exceeding 60%, among which P92-si3 exhibited an inhibition rate of 75.6%.**

**Table 34: Inhibition rates of PCSK9 gene expression by alternately modified sequences**

| **Basic SEQ ID NO:** | **Alternately modified sequence ID** | **Inhibition rate (%)** |
|---|---|---|
| 3 | P92-si3 | 75.6 |
| 8 | P92-si8 | 74.0 |
| 9 | P92-si9 | 69.7 |
| 21 | P92-si21 | 67.3 |
| 31 | P92-si31 | 72.3 |
| 51 | P92-si51 | 70.1 |
| 73 | P92-si73 | 69.9 |
| 83 | P92-si83 | 66.5 |

As shown in Table 34, at a concentration of 10 nM, the alternately modified sequences P92-si3, P92-si8, P92-si9, P92-si21, P92-si31, P92-si51, P92-si73, and P92-si83 all exhibited significant inhibitory effects on PCSK9 gene expression, with inhibition rates exceeding 60%, among which P92-si3 achieved the highest inhibition rate of 75.6%.

**II. The template-modified sequences incorporating anti-off-target design or/and 5'-E-VP modification exhibited significant inhibitory effects on PCSK9 gene expression. For example, the template-modified and 5'-E-VP-modified sequence D84-DV27P exhibited an inhibition rate as high as 94.4%.**

**Table 35: Inhibition rates of PCSK9 gene expression by template-modified sequences incorporating anti-off-target or/and 5'-E-VP modifications**

| **Basic SEQ ID NO:** | **Sequence ID** | **Modification pattern** | **Inhibition rate (%)** |
|---|---|---|---|
| 5 | D5-DV25 | Template | 87.2 |
| | D5-DV25+ | Template + anti-off-target | 82.2 |
| | D5-DV25P | Template + 5'-E-VP | 87.9 |
| | D5-DV25+P | Template + anti-off-target + 5'-E-VP | 82.7 |
| 51 | D51-DV26 | Template | 90.7 |
| | D51-DV26+ | Template + anti-off-target | 87.2 |
| | D51-DV26P | Template + 5'-E-VP | 91.8 |
| | D51-DV26+P | Template + anti-off-target + 5'-E-VP | 84.0 |
| 81 | D81-DV25 | Template | 92.6 |
| | D81-DV25+ | Template + anti-off-target | 88.1 |
| | D81-DV25P | Template + 5'-E-VP | 93.3 |
| | D81-DV25+P | Template + anti-off-target + 5'-E-VP | 87.5 |
| 81 | D81-DV27 | Template | 93.0 |
| | D81-DV27+ | Template + anti-off-target | 86.5 |
| | D81-DV27P | Template + 5'-E-VP | 93.7 |
| | D81-DV27+P | Template + anti-off-target + 5'-E-VP | 87.8 |
| 81 | D81-DV28 | Template | 90.7 |
| | D81-DV28+ | Template + anti-off-target | 89.2 |
| | D81-DV28P | Template + 5'-E-VP | 91.6 |
| | D81-DV28+P | Template + anti-off-target + 5'-E-VP | 87.8 |
| 84 | D84-DV26 | Template | 92.0 |
| | D84-DV26+ | Template + anti-off-target | 90.1 |
| | D84-DV26P | Template + 5'-E-VP | 93.1 |
| | D84-DV26+P | Template + anti-off-target + 5'-E-VP | 89.8 |
| 84 | D84-DV27 | Template | 91.5 |
| | D84-DV27+ | Template + anti-off-target | 89.2 |
| | D84-DV27P | Template + 5'-E-VP | 94.4 |
| | D84-DV27+P | Template + anti-off-target + 5'-E-VP | 90.4 |

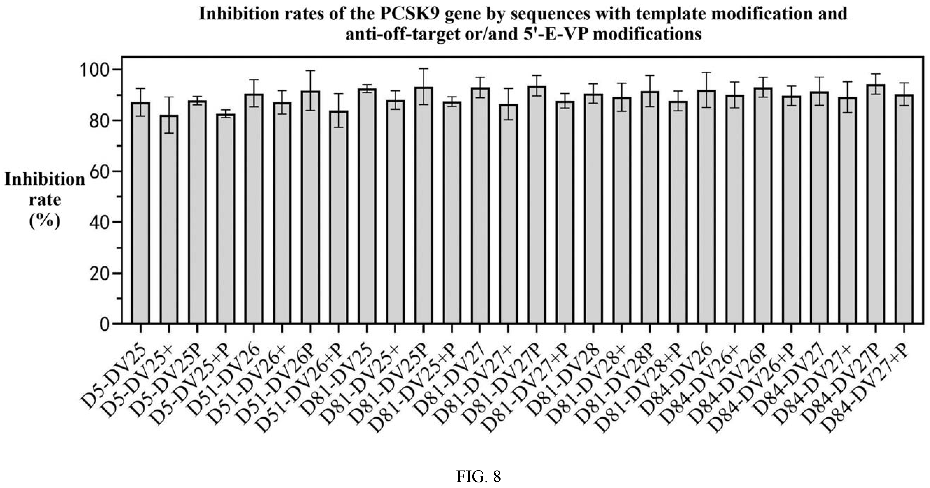

1) For basic sequences 5, 51, 81, and 84, after modification using the templates disclosed herein, the resulting sequences exhibited significant inhibitory effects on PCSK9 gene expression, with inhibition rates exceeding 90%. Moreover, the inhibitory effects were further enhanced compared to the alternately modified sequences. For example, for basic sequence 51, after alternating modification, the resulting sequence P92-si51 exhibited an inhibition rate of 70.1% (Table 34); after modification using template DV26, the resulting sequence D51-DV26 exhibited an inhibition rate of 90.7%, which was 20.6% higher than that of the alternately modified sequence, indicating a significant improvement (FIG. 8).
2) After template modification followed by anti-off-target design, the resulting sequence exhibited significant inhibitory activity on PCSK9 gene expression. For example, for basic sequence 84, after modification using template DV26 followed by anti-off-target design, the resulting sequence D84-DV26+ exhibited an inhibition rate as high as 90.1%.
3) After template modification followed by 5'-E-VP modification, the resulting sequence showed enhanced inhibition of PCSK9 gene expression. For example, for basic sequence 84, after modification using template DV27, the resulting sequence D84-DV27 exhibited an inhibition rate of 91.5%; after further 5'-E-VP modification, the resulting sequence D84-DV27P exhibited an inhibition rate 94.4%, indicating a 2.9% increase.
4) After template modification followed by anti-off-target design and 5'-E-VP modification, the resulting sequence exhibited an inhibition rate exceeding 90% on PCSK9 gene expression. For example, for basic sequence 84, after modification using template DV27 followed by anti-off-target design and 5'-E-VP modification, the resulting sequence D84-DV27+P exhibited an inhibition rate of 90.4%.

It can be concluded that the sequences modified using templates DV25 to 29 disclosed herein showed further enhanced inhibitory effects compared to the alternately modified sequences. Moreover, the sequences modified using templates DV25 to 29 disclosed herein, when further subjected to anti-off-target design or/and 5'-E-VP modification, exhibited significant inhibitory effects on PCSK9 gene expression, with inhibition rates exceeding 90%.

**(2) After adopting any one or more of the modification patterns disclosed herein followed by anti-off-target design, the resulting sequences exhibited significant anti-off-target effects on off-target genes, thereby reducing inhibition of off-target genes.**

The experimental results demonstrated that:
1) The alternately modified or template-modified sequences disclosed herein, when incorporating anti-off-target design, can reduce inhibition of off-target genes, thereby achieving significant anti-off-target effects.
2) The template-modified sequences incorporating both anti-off-target design and 5'-E-VP modification showed significantly reduced inhibition of off-target genes, indicating significant anti-off-target effects.

**I. The alternately modified sequences disclosed herein, when incorporating anti-off-target design, exhibited significant anti-off-target effects, reducing inhibition of off-target genes by 20%.**

**Table 36: Inhibition rates of off-target genes by alternately modified sequences incorporating anti-off-target design**

| **Basic SEQ ID NO:** | **Sequence ID** | **Off-target genes** | **Mean inhibition (%)** | **Reduction in inhibition vs without anti-off-target design (%)** |
|---|---|---|---|---|
| 3 | P92-si3 | AARSD1 | 43.5 | - |
| | P92-si3+ | | 23.5 | 20.0 |
| 3 | P92-si3 | ACAP2 | 56.0 | - |
| | P92-si3+ | | 35.5 | 20.5 |
| 8 | P92-si8 | AIFM2 | 17.3 | - |
| | P92-si8+ | | 10.1 | 7.2 |
| 31 | P92-si31 | ERG | 16.8 | - |
| | P92-si31+ | | 8.4 | 8.4 |
| 9 | P92-si9 | MACF1 | 24.9 | - |
| | P92-si9+ | | 20.1 | 4.8 |
| 8 | P92-si8 | RETREG2 | 14.1 | - |
| | P92-si8+ | | 8.2 | 5.9 |

As shown in Table 36, the alternately modified sequences disclosed herein, when incorporating anti-off-target design, showed significantly reduced inhibition of off-target genes, indicating anti-off-target effects. For example, compared to the sequence without anti-off-target design (P92-si3), the alternately modified sequence with anti-off-target design (P92-si3+) exhibited a 20.0% reduction in inhibition of the off-target gene AARSD1 and a 20.5% reduction in inhibition of the off-target gene ACAP2, indicating a significant decrease.

**II. The sequences modified using the templates disclosed herein, when incorporating anti-off-target design, exhibited significant anti-off-target effects, reducing inhibition of off-target genes by up to 73.6%.**

**Table 37: Inhibition rates of off-target genes by template-modified sequences incorporating anti-off-target or/and 5'-E-VP modifications**

| **Basic SEQ ID NO:** | **Sequence ID** | **Off-target genes** | **Inhibition against off-target genes (%)** | **Reduction in inhibition vs without anti-off-target design (%)** |
|---|---|---|---|---|
| 5 | D5-DV25 | DTWD1 | 26.4 | 0 |
| | D5-DV25+ | | 15.5 | 10.9 |
| | D5-DV25P | | 40 | -13.6 |
| | D5-DV25+P | | 18.7 | 7.7 |
| 51 | D51-DV26 | NEPRO | 37.8 | 0 |
| | D51-DV26+ | | 11.9 | 25.9 |
| | D51-DV26P | | 18.1 | 19.7 |
| | D51-DV26+P | | -4.8 | 42.6 |
| 51 | D51-DV26 | KIF1B | 35.9 | 0 |
| | D51-DV26+ | | 13.1 | 22.8 |
| | D51-DV26P | | 24.8 | 11.1 |
| | D51-DV26+P | | -5.7 | 41.6 |
| 81 | D81-DV25 | DSE | 56.2 | 0 |
| | D81-DV25+ | | 31.9 | 24.2 |
| | D81-DV25P | | 45.1 | 11.0 |
| | D81-DV25+P | | 51.0 | 5.1 |
| | D81-DV27 | | 44.5 | 0 |
| | D81-DV27+ | | 23.7 | 20.8 |
| | D81-DV27P | | 43.7 | 0.7 |
| | D81-DV27+P | | 29.6 | 14.9 |
| 81 | D81-DV25 | PCYOX1 | 64.1 | 0 |
| | D81-DV25+ | | 10.0 | 54.1 |
| | D81-DV25P | | 47.1 | 17.0 |
| | D81-DV25+P | | 34.2 | 29.9 |
| | D81-DV27 | | 54.4 | 0 |
| | D81-DV27+ | | 17.0 | 37.4 |
| | D81-DV27P | | 58.0 | -3.6 |
| | D81-DV27+P | | 23.3 | 31.1 |
| 84 | D84-DV26 | MXD1 | 69.3 | 0 |
| | D84-DV26+ | | 29.4 | 39.9 |
| | D84-DV26P | | 59.3 | 10.0 |
| | D84-DV26+P | | -4.3 | 73.6 |
| | D84-DV27 | | 44.6 | 0 |
| | D84-DV27+ | | 28.1 | 16.5 |
| | D84-DV27P | | 49.1 | -4.5 |
| | D84-DV27+P | | 37.2 | 7.4 |

### 1) Anti-off-target design alone (denoted as "+" in sequence identifiers)

The template-modified sequences incorporating anti-off-target design showed significantly reduced inhibition of off-target genes. For example, D84-DV26+ exhibited a 39.9% reduction in inhibition of the MXD1 gene, D81-DV25+ exhibited a 54.1% reduction in inhibition of the PCYOX1 gene, D51-DV26+ exhibited a 25.9% reduction in inhibition of the NEPRO gene, and D5-DV25+ exhibited a 10.9% reduction in inhibition of the DTWD1 gene.

These results demonstrated that for basic sequences 5, 51, 81, and 84, after modification using the templates designed in the present disclosure followed by anti-off-target design, the resulting sequences exhibited significant anti-off-target effects.

### 2) Anti-off-target design combined with 5'-E-VP modification (denoted as "+P" in sequence identifiers)

The sequences incorporating both template modification and 5'-E-VP modification showed significantly reduced inhibition of off-target genes, indicating significant anti-off-target effects. For example, D84-DV26+P exhibited a 73.6% reduction in inhibition of the off-target gene MXD1, D81-DV27+P exhibited a 31.1% reduction in inhibition of the off-target gene PCYOX1, and D51-DV26+P exhibited a 41.6% reduction in inhibition of the off-target gene KIF1B, indicating a significant decrease.

These results demonstrated that for basic sequences 5, 51, 81, and 84, after modification using the templates designed in the present disclosure followed by anti-off-target design combined with 5'-E-VP modification, the resulting sequences exhibited significant anti-off-target effects.

### Summary:

1. All modified sequences exhibited significant inhibitory effects on PCSK9 gene expression. For example, the alternately modified sequence P92-si3 exhibited an inhibition rate of 75.6%, while the template-modified and 5'-E-VP-modified sequence D84-DV27P exhibited an inhibition rate as high as 94.4%.
   (1) The alternately modified sequences exhibited significant inhibitory effects on PCSK9 gene expression, with inhibition rates exceeding 60%, among which P92-si3 exhibited an inhibition rate of 75.6%.
   (2) The template-modified sequences incorporating anti-off-target design or/and 5'-E-VP modification exhibited significant inhibitory effects on PCSK9 gene expression. For example, the template-modified and 5'-E-VP-modified sequence D84-DV27P exhibited an inhibition rate as high as 94.4%.
2. After adopting any one or more of the modification patterns disclosed herein followed by anti-off-target design, the resulting sequences exhibited significant anti-off-target effects on off-target genes, thereby reducing inhibition of off-target genes.
   (1) The alternately modified sequences disclosed herein, when incorporating anti-off-target design, exhibited significant anti-off-target effects, reducing inhibition of off-target genes by 20%.
   (2) The sequences modified using the templates disclosed herein, when incorporating anti-off-target design, exhibited significant anti-off-target effects, reducing inhibition of off-target genes by up to 73.6%.

### Example 7: Effect of sequences modified using the templates disclosed herein on serum PCSK9, low-density lipoprotein cholesterol (LDL-C), and total cholesterol (TC) in mice

In this example, several sequences were exemplarily selected, including basic sequences 5, 51, 81, 82, and 84. These sequences were modified, for example, by incorporating template modification alone, both template modification and anti-off-target design, both template modification and 5'-E-VP modification, or template modification combined with 5'-E-VP modification and anti-off-target design. Using transgenic mice expressing the human PCSK9 gene, the inhibitory effects of these sequences on serum PCSK9, low-density lipoprotein cholesterol (LDL-C), and total cholesterol (TC) at different time points were evaluated by ELISA.

### 1. Experimental materials

### Test compounds:

The sequences listed in Table 38, including those incorporating template modification alone, both template modification and 5'-E-VP modification, both template modification and anti-off-target design, or template modification combined with 5'-E-VP modification and anti-off-target design, wherein the 3' end of the sense strand of these sequences is conjugated with GalNAc ligands G4, G5, G6, or G7:

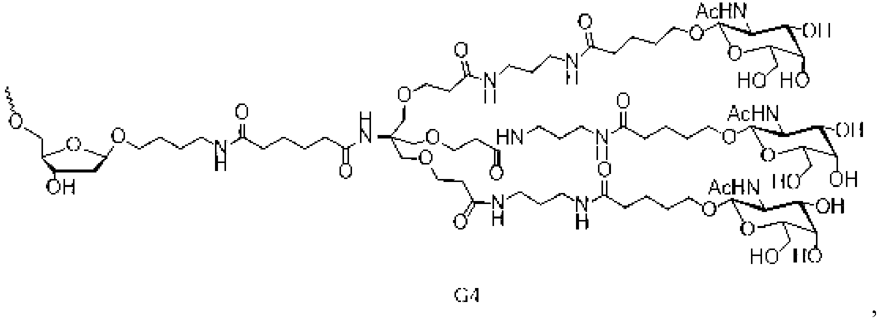

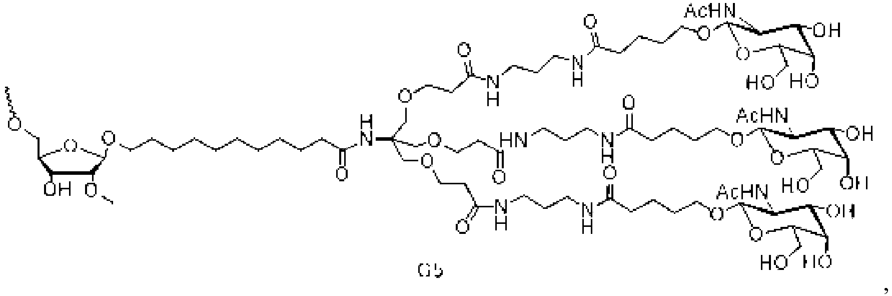

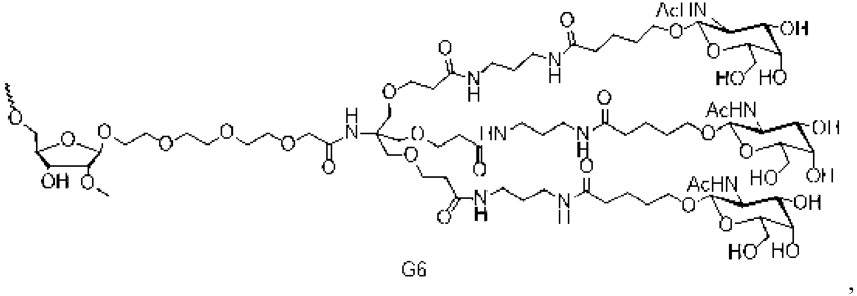

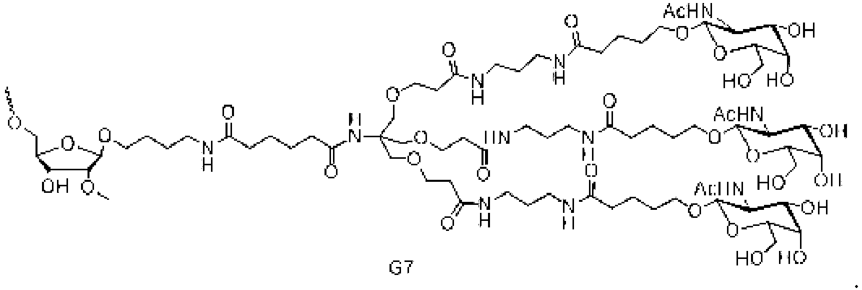

The conjugation method between oligonucleotides and ligands G4, G5, G6, or G7 was the same as that described for conjugates 4, 5, 6, and 7 in Example 3 of patent application CN116854754A, *i.e.*, conjugating YK-GAL-304, YK-GAL-305, YK-GAL-306, and YK-GAL-307 with oligonucleotides. YK-GAL-304, YK-GAL-305, YK-GAL-306, and YK-GAL-307 were synthesized as in Example 1 of CN116854754A.

The resulting oligonucleotide-ligand conjugate is shown below:

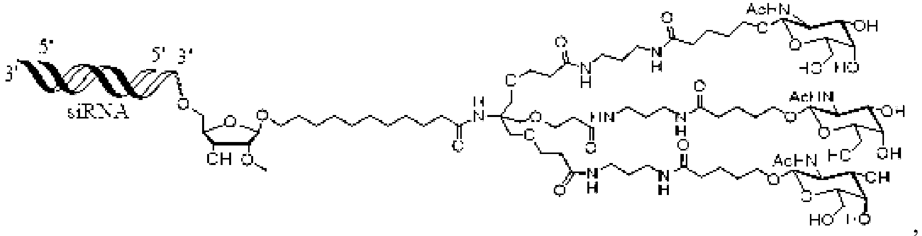

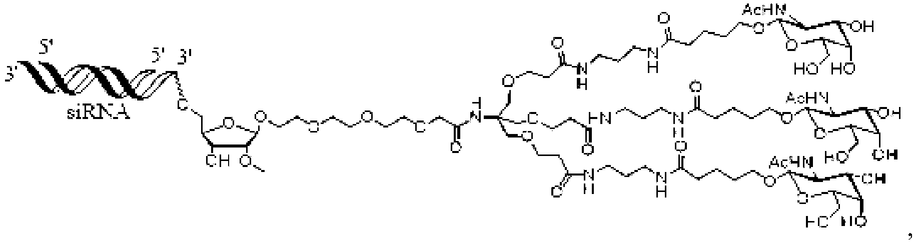

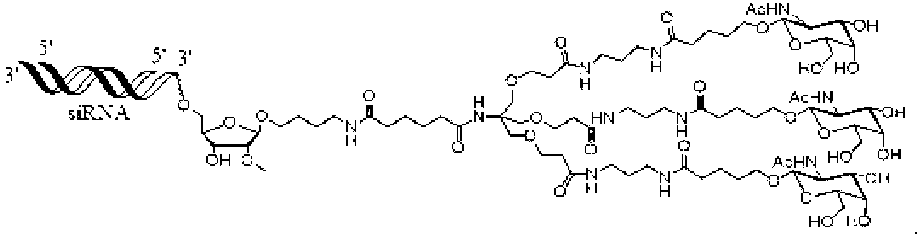

The specific sequences are shown in Table 38. In sequence identifiers, G4, G5, G6, and G7 indicate conjugation with GalNAc ligands G4, G5, G6, or G7, respectively.

**Table 38: Sequences used in animal experiments**

| **Sequence ID** | **Sense strand 5'-3'** | **Modified SEQ ID NO:/corr. basic SEQ ID NO:** | **Antisense strand 5'-3'** | **Modified SEQ ID NO:/corr. basic SEQ ID NO:** |
|---|---|---|---|---|
| D5-DV25+G4 | Ams-Ams-Gm-Am-Um-Cm-Cf- Um-Gf-Cf-Af-Um-Gm-Um-Cm- Um-Uf-Cm-Cm-Am-Um-G4 | 337/1 | Ams-Ufs-Gm-Gm-Am-Af-Ggna- Am-Cm-Am-Um-Gm-Cm-Af-Gm- Gf-Am-Um-Cm-Um-Ums-Gms-Gm | 427/13 |
| D5-DV25+G7 | Ams-Ams-Gm-Am-Um-Cm-Cf- Um-Gf-Cf-Af-Um-Gm-Um-Cm- Um-Uf-Cm-Cm-Am-Um-G7 | 337/1 | Ams-Ufs-Gm-Gm-Am-Af-Ggna- Am-Cm-Am-Um-Gm-Cm-Af-Gm- Gf-Am-Um-Cm-Um-Ums-Gms-Gm | 427/13 |
| D5-DV25PG4 | Ams-Ams-Gm-Am-Um-Cm-Cf- Um-Gf-Cf-Af-Um-Gm-Um-Cm- Um-Uf-Cm-Cm-Am-Um-G4 | 337/1 | AmsEVP-Ufs-Gm-Gm-Am-Af-Gm- Am-Cm-Am-Um-Gm-Cm-Af-Gm- Gf-Am-Um-Cm-Um-Ums-Gms-Gm | 428/13 |
| D5-DV25PG5 | Ams-Ams-Gm-Am-Um-Cm-Cf- Um-Gf-Cf-Af-Um-Gm-Um-Cm- Um-Uf-Cm-Cm-Am-Um-G5 | 337/1 | AmsEVP-Ufs-Gm-Gm-Am-Af-Gm- Am-Cm-Am-Um-Gm-Cm-Af-Gm- Gf-Am-Um-Cm-Um-Ums-Gms-Gm | 428/13 |
| D5-DV25PG7 | Ams-Ams-Gm-Am-Um-Cm-Cf- Um-Gf-Cf-Af-Um-Gm-Um-Cm- Um-Uf-Cm-Cm-Am-Um-G7 | 337/1 | AmsEVP-Ufs-Gm-Gm-Am-Af-Gm- Am-Cm-Am-Um-Gm-Cm-Af-Gm- Gf-Am-Um-Cm-Um-Ums-Gms-Gm | 428/13 |
| D51-DV26G4 | Ums-Gms-Gm-Am-Gm-Gm-Cf- Um-Uf-Af-Gf-Cm-Um-Um-Um- Cm-Uf-Gm-Gm-Am-Um-G4 | 342/2 | Ams-Ufs-Cf-Cf-Am-Gf-Am-Am- Am-Gm-Cm-Um-Am-Af-Gm-Cf- Cm-Um-Cm-Cm-Ams-Ums-Um | 381/14 |
| D51-DV26G6 | Ums-Gms-Gm-Am-Gm-Gm-Cf- Um-Uf-Af-Gf-Cm-Um-Um-Um- Cm-Uf-Gm-Gm-Am-Um-G6 | 342/2 | Ams-Ufs-Cf-Cf-Am-Gf-Am-Am- Am-Gm-Cm-Um-Am-Af-Gm-Cf- Cm-Um-Cm-Cm-Ams-Ums-Um | 381/14 |
| D51-DV26+G5 | Ums-Gms-Gm-Am-Gm-Gm-Cf- Um-Uf-Af-Gf-Cm-Um-Um-Um- Cm-Uf-Gm-Gm-Am-Um-G5 | 342/2 | Ams-Ufs-Cf-Cf-Am-Gf-Agna-Am- Am-Gm-Cm-Um-Am-Af-Gm-Cf- Cm-Um-Cm-Cm-Ams-Ums-Um | 430/14 |
| D51-DV26+G7 | Ums-Gms-Gm-Am-Gm-Gm-Cf- Um-Uf-Af-Gf-Cm-Um-Um-Um- Cm-Uf-Gm-Gm-Am-Um-G7 | 342/2 | Ams-Ufs-Cf-Cf-Am-Gf-Agna-Am- Am-Gm-Cm-Um-Am-Af-Gm-Cf- Cm-Um-Cm-Cm-Ams-Ums-Um | 430/14 |
| D51-DV26PG6 | Ums-Gms-Gm-Am-Gm-Gm-Cf- Um-Uf-Af-Gf-Cm-Um-Um-Um- Cm-Uf-Gm-Gm-Am-Um-G6 | 342/2 | AmsEVP-Ufs-Cf-Cf-Am-Gf-Am- Am-Am-Gm-Cm-Um-Am-Af-Gm- Cf-Cm-Um-Cm-Cm-Ams-Ums-Um | 431/14 |
| D51-DV26PG7 | Ums-Gms-Gm-Am-Gm-Gm-Cf- Um-Uf-Af-Gf-Cm-Um-Um-Um- Cm-Uf-Gm-Gm-Am-Um-G7 | 342/2 | AmsEVP-Ufs-Cf-Cf-Am-Gf-Am- Am-Am-Gm-Cm-Um-Am-Af-Gm- Cf-Cm-Um-Cm-Cm-Ams-Ums-Um | 431/14 |
| D81-DV25G4 | Cms-Ums-Um-Um-Um-Gm-Uf- Am-Af-Cf-Uf-Um-Gm-Am-Am- Gm-Af-Um-Am-Um-Um-G4 | 347/3 | Ams-Afs-Um-Am-Um-Cf-Um-Um- Cm-Am-Am-Gm-Um-Uf-Am-Cf- Am-Am-Am-Am-Gms-Cms-Am | 384/15 |
| D81-DV25G7 | Cms-Ums-Um-Um-Um-Gm-Uf- Am-Af-Cf-Uf-Um-Gm-Am-Am- Gm-Af-Um-Am-Um-Um-G7 | 347/3 | Ams-Afs-Um-Am-Um-Cf-Um-Um- Cm-Am-Am-Gm-Um-Uf-Am-Cf- Am-Am-Am-Am-Gms-Cms-Am | 384/15 |
| D81-DV27PG4 | Cms-Ums-Um-Um-Um-Gm-Uf- Am-Af-Cf-Uf-Um-Gm-Am-Am- Gm-Af-Um-Am-Um-Um-G4 | 347/3 | AmsEVP-Afs-Um-Af-Uf-Cf-Um- Um-Cm-Am-Am-Gm-Um-Uf-Am- Cf-Am-Am-Am-Am-Gms-Cms-Am | 437/15 |
| D81-DV27+PG 5 | Cms-Ums-Um-Um-Um-Gm-Uf- Am-Af-Cf-Uf-Um-Gm-Am-Am- Gm-Af-Um-Am-Um-Um-G5 | 347/3 | AmsEVP-Afs-Um-Af-Uf-Cf-Ugna- Um-Cm-Am-Am-Gm-Um-Uf-Am- Cf-Am-Am-Am-Am-Gms-Cms-Am | 438/15 |
| D81-DV27+PG 7 | Cms-Ums-Um-Um-Um-Gm-Uf- Am-Af-Cf-Uf-Um-Gm-Am-Am- Gm-Af-Um-Am-Um-Um-G7 | 347/3 | AmsEVP-Afs-Um-Af-Uf-Cf-Ugna- Um-Cm-Am-Am-Gm-Um-Uf-Am- Cf-Am-Am-Am-Am-Gms-Cms-Am | 438/15 |
| D81-DV28G6 | Cms-Ums-Um-Um-Um-Gm-Uf- Am-Af-Cm-Uf-Um-Gm-Am-Am- Gm-Af-Um-Am-Um-Um-G6 | 348/3 | Ams-Afs-Uf-Af-Um-Cf-Um-Um- Cm-Am-Am-Gm-Um-Uf-Am-Cf- Am-Am-Am-Am-Gms-Cms-Am | 385/15 |
| D82-DV27PG5 | Gms-Ams-Am-Gm-Am-Um-Af- Um-Uf-Uf-Af-Um-Um-Cm-Um- Gm-Gf-Gm-Um-Um-Um-G5 | 352/4 | AmsEVP-Afs-Am-Cf-Cf-Cf-Am- Gm-Am-Am-Um-Am-Am-Af-Um- Af-Um-Cm-Um-Um-Cms-Ams-Am | 448/16 |
| D82-DV27PG7 | Gms-Ams-Am-Gm-Am-Um-Af- Um-Uf-Uf-Af-Um-Um-Cm-Um- Gm-Gf-Gm-Um-Um-Um-G7 | 352/4 | AmsEVP-Afs-Am-Cf-Cf-Cf-Am- Gm-Am-Am-Um-Am-Am-Af-Um- Af-Um-Cm-Um-Um-Cms-Ams-Am | 448/16 |
| D82-DV29G4 | Gms-Ams-Am-Gm-Am-Um-Af- Um-Uf-Um-Af-Um-Um-Cm-Um- Gm-Gf-Gm-Um-Um-Um-G4 | 353/4 | Ams-Afs-Am-Cf-Cf-Cf-Am-Gm- Am-Am-Um-Am-Am-Af-Um-Af- Um-Cm-Um- Um-Cms-Ams-Am | 390/16 |
| D82-DV29G7 | Gms-Ams-Am-Gm-Am-Um-Af- Um-Uf-Um-Af-Um-Um-Cm-Um- Gm-Gf-Gm-Um-Um-Um-G7 | 353/4 | Ams-Afs-Am-Cf-Cf-Cf-Am-Gm- Am-Am-Um-Am-Am-Af-Um-Af- Um-Cm-Um- Um-Cms-Ams-Am | 390/16 |
| D82-DV29PG5 | Gms-Ams-Am-Gm-Am-Um-Af- Um-Uf-Um-Af-Um-Um-Cm-Um- Gm-Gf-Gm-Um-Um-Um-G5 | 353/4 | AmsEVP-Afs-Am-Cf-Cf-Cf-Am- Gm-Am-Am-Um-Am-Am-Af-Um- Af-Um-Cm-Um-Um-Cms-Ams-Am | 448/16 |
| D82-DV29PG6 | Gms-Ams-Am-Gm-Am-Um-Af- Um-Uf-Um-Af-Um-Um-Cm-Um- Gm-Gf-Gm-Um-Um-Um-G6 | 353/4 | AmsEVP-Afs-Am-Cf-Cf-Cf-Am- Gm-Am-Am-Um-Am-Am-Af-Um- Af-Um-Cm-Um-Um-Cms-Ams-Am | 448/16 |
| D82-DV29PG7 | Gms-Ams-Am-Gm-Am-Um-Af- Um-Uf-Um-Af-Um-Um-Cm-Um- Gm-Gf-Gm-Um-Um-Um-G7 | 353/4 | AmsEVP-Afs-Am-Cf-Cf-Cf-Am- Gm-Am-Am-Um-Am-Am-Af-Um- Af-Um-Cm-Um-Um-Cms-Ams-Am | 448/16 |
| D84-DV26G4 | Gms-Ums-Um-Um-Um-Gm-Uf- Am-Gf-Cf-Af-Um-Um-Um-Um- Um-Af-Um-Um-Am-Am-G4 | 357/5 | Ums-Ufs-Af-Af-Um-Af-Am-Am- Am-Am-Um-Gm-Cm-Uf-Am-Cf- Am-Am-Am-Am-Cms-Cms-Cm | 393/17 |
| D84-DV26G6 | Gms-Ums-Um-Um-Um-Gm-Uf- Am-Gf-Cf-Af-Um-Um-Um-Um- Um-Af-Um-Um-Am-Am-G6 | 357/5 | Ums-Ufs-Af-Af-Um-Af-Am-Am- Am-Am-Um-Gm-Cm-Uf-Am-Cf- Am-Am-Am-Am-Cms-Cms-Cm | 393/17 |
| D84-DV27PG4 | Gms-Ums-Um-Um-Um-Gm-Uf- Am-Gf-Cf-Af-Um-Um-Um-Um- Um-Af-Um-Um-Am-Am-G4 | 357/5 | UmsEVP-Ufs-Am-Af-Uf-Af-Am- Am-Am-Am-Um-Gm-Cm-Uf-Am- Cf-Am-Am-Am-Am-Cms-Cms-Cm | 446/17 |
| D84-DV27PG6 | Gms-Ums-Um-Um-Um-Gm-Uf- Am-Gf-Cf-Af-Um-Um-Um-Um- Um-Af-Um-Um-Am-Am-G6 | 357/5 | UmsEVP-Ufs-Am-Af-Uf-Af-Am- Am-Am-Am-Um-Gm-Cm-Uf-Am- Cf-Am-Am-Am-Am-Cms-Cms-Cm | 446/17 |
| D84-DV27+PG 4 | Gms-Ums-Um-Um-Um-Gm-Uf- Am-Gf-Cf-Af-Um-Um-Um-Um- Um-Af-Um-Um-Am-Am-G4 | 357/5 | UmsEVP-Ufs-Am-Af-Uf-Af-Agna- Am-Am-Am-Um-Gm-Cm-Uf-Am- Cf-Am-Am-Am-Cms-Cms-Cm | 447/17 |
| D84-DV27+PG 5 | Gms-Ums-Um-Um-Um-Gm-Uf- Am-Gf-Cf-Af-Um-Um-Um-Um- Um-Af-Um-Um-Am-Am-G5 | 357/5 | UmsEVP-Ufs-Am-Af-Uf-Af-Agna- Am-Am-Am-Um-Gm-Cm-Uf-Am- Cf-Am-Am-Am-Cms-Cms-Cm | 447/17 |
| D84-DV27+PG 7 | Gms-Ums-Um-Um-Um-Gm-Uf- Am-Gf-Cf-Af-Um-Um-Um-Um- Um-Af-Um-Um-Am-Am-G7 | 357/5 | UmsEVP-Ufs-Am-Af-Uf-Af-Agna- Am-Am-Am-Um-Gm-Cm-Uf-Am- Cf-Am-Am-Am-Cms-Cms-Cm | 447/17 |

### Preparation of test compounds:

Solvent: PBS buffer.

Preparation conditions: sterile environment.

Labeling method: each prepared dosage form was labeled with tags, with the outer packaging indicating the project number, name, concentration, quantity, preparation date, preparer, and storage conditions.

Storage conditions: freshly prepared before use; remaining samples stored at -80°C.

### Information on experimental animals:

Species/strain: hPCSK9 transgenic mice
Grade: SPF
Sex: Male
Quantity: 214
Age: 4 to 7 weeks
Weight: 18 to 20 g
Source: Jiangsu GemPharmatech Co., Ltd.
Production license No.: SCXK (Su) 2018-0008

### Review by Institutional Animal Care and Use Committee (IACUC):

Upon arrival, the experimental animals were housed at Tianjin Yugen Medtech Co., Ltd., under a project license No.: SYXK (Jinbin) 2019-0002. The project was approved by the IACUC of Tianjin Yugen Medtech Co., Ltd. The experimental procedures were strictly conducted in compliance with IACUC requirements to ensure animal welfare.

### Housing and management:

Housing conditions: Upon arrival, the experimental animals were housed at Tianjin Yugen Medtech Co., Ltd., under a project license No.: SYXK (Jinbin) 2019-0002. The animals were housed in rearing cages with dimensions of 29.0 cm (L) × 18.5 cm (W) × 13.0 cm (H). The environmental parameters were set as follows: a temperature ranging from 20°C to 26°C, a humidity ranging from 40% to 70%, a ventilation rate of at least 15 fresh air changes per hour, and a 12-hour light/12-hour dark cycle with artificial lighting.

The environmental conditions complied with the National Standard GB14925-2010 of the People's Republic of China, with environmental control provided by a modular air-conditioning system.

The animals had free access to food and water. Water bottles and drinking water in the bottles were replaced at least twice weekly. Used water bottles were sterilized in a pulse vacuum autoclave before reuse.

The cages and bedding were replaced at least once weekly. All cages and bedding were sterilized in a pulse vacuum autoclave before being introduced into a barrier environment. Cage racks were cleaned and disinfected at least once weekly.

Animal observation rooms were cleaned and disinfected daily, including shelves, floors, work surfaces, *etc*.

Disinfectants used in the barrier environment included: 6.67% benzalkonium bromide solution, 0.5% 84 disinfectant, 75% ethanol, and 0.08% didecyldimethylammonium bromide. These four disinfectants should be used alternately and should not be used in combination.

Animal feed: Maintenance diet for SPF-grade mice and rats: produced by SPF (Beijing) Biotechnology Co., Ltd.; production license No.: SCXK (Jing) 2019-0010, issued by the Beijing Municipal Science and Technology Commission.

Feed testing: Each batch of feed had a quality certificate. Microbial testing was conducted quarterly by the company. A recent third-party feed inspection report was provided semiannually by the supplier. Nutrient composition testing complied with the National Standard GB14924.3-2010 of the People's Republic of China, while contaminant testing complied with the National Standard GB14924.2-2001 of the People's Republic of China.

Water for experimental animals: Drinking water: sterile water prepared by a filtration system, directly filled into drinking bottles.

Drinking water testing: Microbial testing was conducted quarterly by the company. Water quality testing was conducted annually by a third-party testing agency. Drinking water testing complied with the National Standard GB5749-2006 of the People's Republic of China.

Animal bedding: Corncob bedding: produced by SPF (Beijing) Biotechnology Co., Ltd.; production license No.: SCXK (Jing) 2019-0004, issued by the Beijing Municipal Science and Technology Commission.

Bedding testing: Microbial testing was conducted quarterly by the company. At least one recent third-party bedding inspection report was provided semiannually by the supplier. Bedding testing complied with the National Standard GB14924.2-2001 of the People's Republic of China.

### 2. Experimental methods

### Dosage design and grouping

Definition of trial date: The day on which the animals were administered solvent or test compound was defined as Day 0.

Grouping and dosing: After acclimation, the experimental animals were randomly divided into a negative control group and a treatment group based on serum PSCK9 levels, with 5 animals per group. The test compounds were administered at a dose of 6 mg/kg via a single subcutaneous injection, with a dosing volume of 1 mL/kg and a dosing concentration of 6 mg/mL. The day of administration was designated as Day 0.

Individual animals were identified by ear tags. Cages were identified by hanging cage cards. A laboratory signage was hung at the entrance of the laboratory. Group information is shown in Table 39 below.

**Table 39**

| **Group** | **Test compound** | **Dose (mg/kg)** | **Route/frequency of administration** | **Duration (days)** | **Number of animals** |
|---|---|---|---|---|---|
| Negative control group | Solvent | / | / | 35 | 5 |
| Treatment group | siRNA | 6 | s.c./once | 35 | 5 |

### Measurement indicators

### (1) General observations

Animals were observed once daily from one week prior to administration until the end of the study.

Cage-side observation parameters included mortality or moribund status, mental state, behavior activities, fecal characteristics, food and water supply, *etc*.

Test animals: all animals in both the negative control group and the treatment group.

### (2) Serum PCSK9 expression

Time points for measurement: Day -3 (prior to dosing), Day 7 (1 week post-dosing), Day 14 (2 weeks post-dosing), Day 21 (3 weeks post-dosing), Day 28 (4 weeks post-dosing), and Day 35 (5 weeks post-dosing).

Method for measuring PCSK protein levels: measured using an ELISA kit; repeated freeze-thaw cycles of serum samples were avoided.

Test animals: all animals in both the negative control group and the treatment group.

### (3) Measurement of blood lipids

Time points for measurement: Day -3 (prior to dosing), Day 7 (1 week post-dosing), Day 14 (2 weeks post-dosing), Day 21 (3 weeks post-dosing), Day 28 (4 weeks post-dosing), and Day 35 (5 weeks post-dosing). Approximately 200 µL of blood was collected from the inner canthus. Whole blood samples were temporarily stored on ice before centrifugation at approximately 3000 g for 10 minutes at 2 to 8°C. The samples were dispensed into two tubes (one tube containing approximately 60 µL, with the remaining serum stored in the other tube) and stored at -70 to -86°C for measurement of blood lipids.

### (4) Data processing and statistical analysis

Data are expressed as mean ± standard deviation (Mean ± SD) and analyzed using GraphPad Prism 8.3 software. The LSD test was used for homogeneity of variance and Dunnett's T3 test was used for unequal variance. P < 0.05 was considered statistically significant.

### 3. Experimental results

The detailed experimental results are shown in Tables 40 to 42.

It can be seen that these sequences exhibited sustained and significant inhibition of serum PCSK9 expression, along with significantly reduced serum low-density lipoprotein cholesterol (LDL-C) and total cholesterol (TC) levels. For example, in the D81-DV25G7 group, the inhibition rates of serum PCSK9 expression were 83.1%, 82.2%, and 70.3% on Day 7, Day 14, and Day 28, respectively; in the D82-DV27PG5 group, serum LDL-C levels were reduced by 32.6%, 35.8%, and 21.7% on Day 7, Day 14, and Day 28, respectively; in the D82-DV29PG7 group, serum TC levels were reduced by 32.2%, 26.8%, and 19.6% on Day 7, Day 14, and Day 28, respectively.
**(1) The sequences disclosed herein, such as basic sequences 5, 51, 81, 82, and 84, when subjected to different modifications, exhibited significant inhibitory effects on serum PCSK9 expression.** F**or example, D81-DV25G7 exhibited inhibition rates of 83.1%, 82.2%, and 70.3% on Day 7, Day 14, and Day 28, respectively.**

**Table 40: Inhibition rates of serum PCSK9 expression by different sequences**

| **Basic SEQ ID NO:** | **Sequence ID** | **Inhibition rate (%)** | | |
|---|---|---|---|---|
| | | **D7** | **D14** | **D28** |
| 5 | D5-DV25PG4 | 78.1 | 70.5 | 53.8 |
| | D5-DV25PG5 | 77.0 | 64.7 | 58.4 |
| 51 | D51-DV26PG6 | 78.2 | 82.6 | 68.2 |
| | D51-DV26+G5 | 74.7 | 68.0 | 59.3 |
| | D51-DV26+G7 | 70.6 | 75.3 | 58.1 |
| 81 | D81-DV25G4 | 80.2 | 81.6 | 63.8 |
| | D81-DV25G7 | 83.1 | 82.2 | 70.3 |
| 82 | D82-DV27PG5 | 88.2 | 87.4 | 69.2 |
| | D82-DV27PG7 | 81.9 | 86.3 | 72.6 |
| | D82-DV29G4 | 74.2 | 81.6 | 72.6 |
| | D82-DV29PG5 | 83.8 | 90.0 | 76.4 |
| 84 | D84-DV26G4 | 81.7 | 77.4 | 65.8 |
| | D84-DV26G6 | 75.6 | 79.5 | 56.3 |
| | D84-DV27+PG5 | 84.0 | 76.1 | 63.4 |
| | D84-DV27+PG7 | 78.9 | 77.6 | 60.8 |

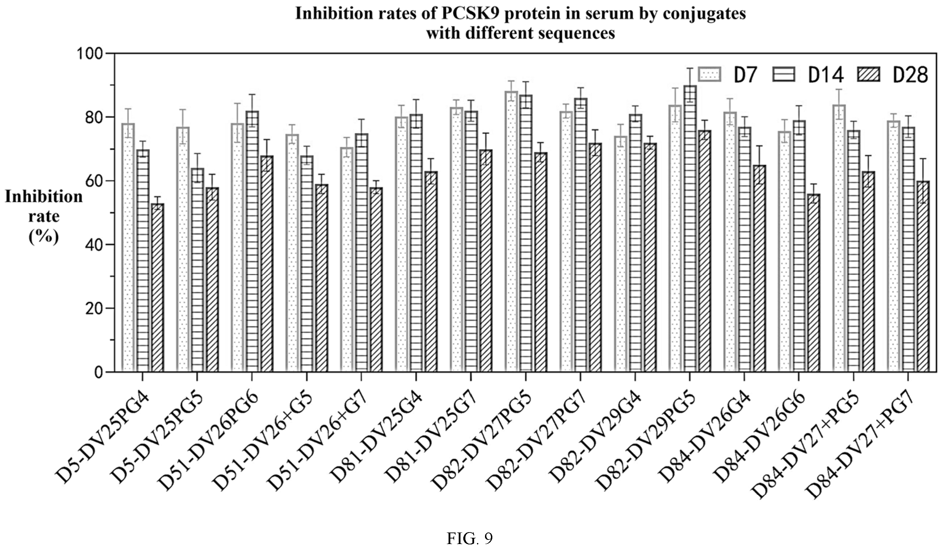

As shown in Table 40, the conjugates formed by conjugation of these sequences with GalNAc compounds exhibited significant inhibitory effects on serum PCSK9 expression. For example, D81-DV25G7 exhibited inhibition rates of 83.1%, 82.2%, and 70.3% on Day 7, Day 14, and Day 28, respectively (FIG. 9).

These findings indicate that the basic sequences designed in the present disclosure, such as 5, 51, 81, 82, and 84, when modified using the templates disclosed herein, or further incorporating 5'-E-VP modification or/and anti-off-target design, and conjugated with GalNAc compounds, can be efficiently delivered to the liver in animals, significantly inhibiting PCSK9 gene expression.

**(2) The sequences disclosed herein, such as basic sequences 5, 51, 81, 82, and 84, when subjected to different modifications, exhibited significantly reduced serum low-density lipoprotein cholesterol (LDL-C) levels. For example, D82-DV27PG5 exhibited reductions of 32.6%, 35.8%, and 21.7% on Day 7, Day 14, and Day 28, respectively.**

**Table 41: Reduction in serum low-density lipoprotein cholesterol (LDL-C) levels by different sequences**

| **Basic SEQ ID NO:** | **Sequence ID** | **Reduction (%)** | | |
|---|---|---|---|---|
| | | **D7** | **D14** | **D28** |
| 5 | D5-DV25PG5 | 21.2 | 16.9 | 15.4 |
| | D5-DV25PG7 | 20.7 | 18.7 | 19.2 |
| 51 | D51-DV26G4 | 19.7 | 21.9 | 17.7 |
| | D51-DV26G6 | 24.5 | 18.4 | 16.8 |
| 81 | D81-DV27PG4 | 25.2 | 25.3 | 17.3 |
| | D81-DV27+PG5 | 25.2 | 18.3 | 18.2 |
| | D81-DV27+PG7 | 21.9 | 17.9 | 16.8 |
| 82 | D82-DV27PG5 | 32.6 | 35.8 | 21.7 |
| | D82-DV29G4 | 29.3 | 34.1 | 22.5 |
| | D82-DV29G7 | 30.6 | 32.9 | 23.7 |
| | D82-DV29PG5 | 30.1 | 31.2 | 22.8 |
| 84 | D84-DV27PG4 | 30.1 | 26.1 | 20.7 |
| | D84-DV27PG6 | 28.9 | 29.6 | 18.5 |

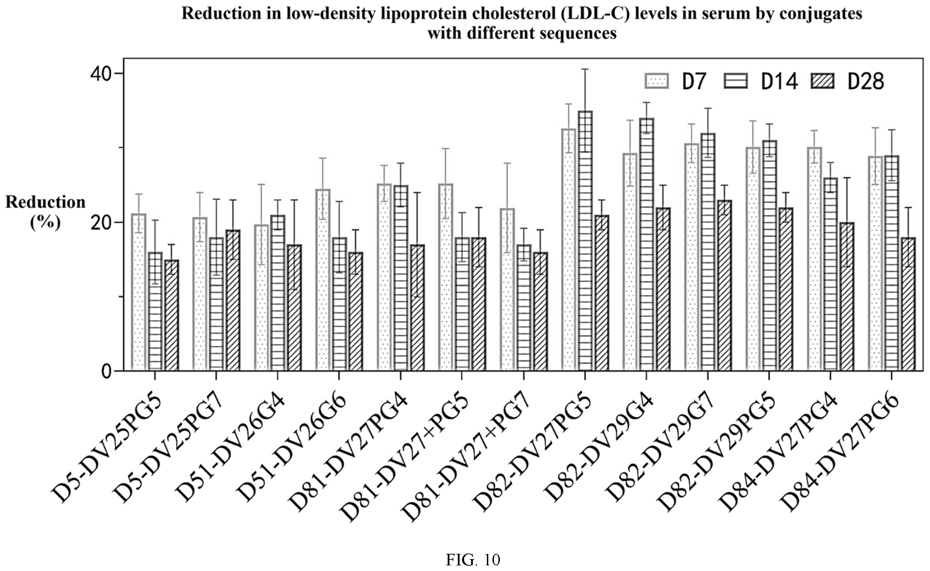

As shown in Table 41, serum LDL-C levels were significantly reduced in all experimental groups. For example, D82-DV27PG5 exhibited reductions of 32.6%, 35.8%, and 21.7% on Day 7, Day 14, and Day 28, respectively (FIG. 10).

These findings indicate that the basic sequences designed in the present disclosure, such as 5, 51, 81, 82, and 84, when modified using the templates disclosed herein, or further incorporating 5'-E-VP modification or/and anti-off-target design, and conjugated with GalNAc compounds, can be efficiently delivered to the liver in animals, significantly reducing serum low-density lipoprotein cholesterol (LDL-C) levels.

**(3) The sequences disclosed herein, such as basic sequences 5, 51, 81, 82, and 84, when subjected to different modifications, exhibited significantly reduced serum total cholesterol (TC) levels**. **For example, D82-DV29PG7 exhibited reductions of 32.2%, 26.8%, and 19.6% on Day 7, Day 14, and Day 28, respectively.**

**Table 42: Reduction in serum total cholesterol (TC) levels by different sequences**

| **Basic SEQ ID NO:** | **Sequence ID** | **Reduction (%)** | | |
|---|---|---|---|---|
| | | **D7** | **D14** | **D28** |
| 5 | D5-DV27+G4 | 21.5 | 20.8 | 13.2 |
| | D5-DV27+G7 | 19.7 | 18.7 | 9.2 |
| 51 | D51-DV26PG6 | 24.1 | 18.3 | 11.9 |
| | D51-DV26PG7 | 19.7 | 20.3 | 10.7 |
| 81 | D81-DV25G7 | 21.7 | 22.2 | 13.1 |
| | D81-DV27+PG5 | 21.1 | 16.1 | 15.6 |
| | D81-DV27+PG7 | 17.9 | 21.6 | 13.8 |
| | D81-DV28G6 | 22.9 | 19.1 | 13.4 |
| 82 | D82-DV29PG5 | 31.4 | 27.0 | 18.5 |
| | D82-DV29PG6 | 28.9 | 29.6 | 17.1 |
| | D82-DV29PG7 | 32.2 | 26.8 | 19.6 |
| | D84-DV26G6 | 28.9 | 29.6 | 18.5 |
| 84 | D84-DV27+PG4 | 28.4 | 25.7 | 14.9 |
| | D84-DV27+PG5 | 22.8 | 22.9 | 16.5 |

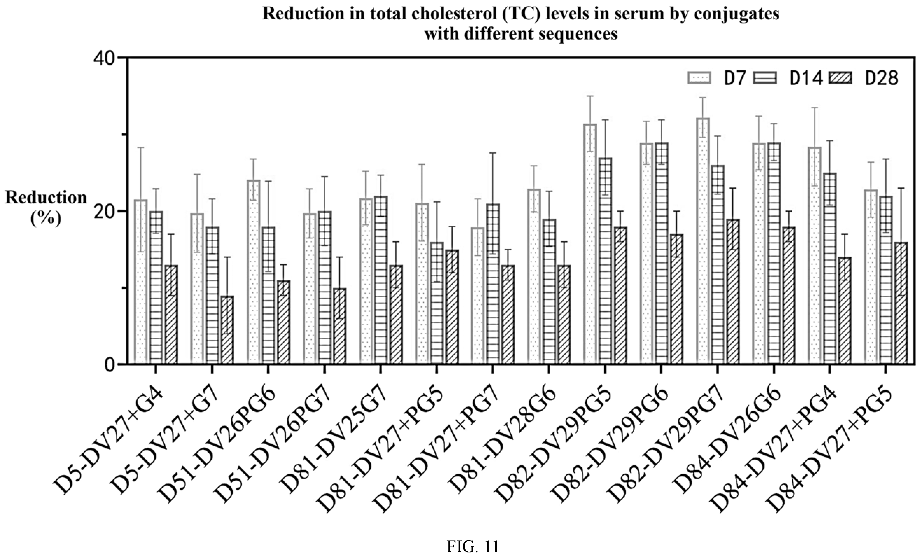

As shown in Table 42, serum total cholesterol (TC) levels were significantly reduced in all experimental groups. For example, D82-DV29PG7 exhibited reductions of 32.2%, 26.8%, and 19.6% on Day 7, Day 14, and Day 28, respectively (FIG. 11).

These findings indicate that the basic sequences designed in the present disclosure, such as 5, 51, 81, 82, and 84, when modified using the templates disclosed herein, or further incorporating 5'-E-VP modification or/and anti-off-target design, and conjugated with GalNAc compounds, can be efficiently delivered to the liver in animals, significantly reducing serum total cholesterol (TC) levels.

### Summary:

The basic sequences designed in the present disclosure, such as 5, 51, 81, 82, and 84, when modified using the templates disclosed herein, or further incorporating 5'-E-VP modification or/and anti-off-target design, and conjugated with GalNAc compounds, can be efficiently delivered to the liver in animals, exhibiting sustained and significant inhibition of serum PCSK9 expression, along with significantly reduced serum low-density lipoprotein cholesterol (LDL-C) and total cholesterol (TC) levels.

For example, in the D81-DV25G7 group, the inhibition rates of serum PCSK9 expression were 83.1%, 82.2%, and 70.3% on Day 7, Day 14, and Day 28, respectively; in the D82-DV27PG5 group, serum LDL-C levels were reduced by 32.6%, 35.8%, and 21.7% on Day 7, Day 14, and Day 28, respectively; in the D82-DV29PG7 group, serum TC levels were reduced by 32.2%, 26.8%, and 19.6% on Day 7, Day 14, and Day 28, respectively.

### Conclusion:

In the present disclosure, a series of siRNAs were designed based on the PCSK9 mRNA sequence, which were subjected to alternating modifications and modifications using a set of specific templates, with some of these sequences further incorporating anti-off-target design and 5'-E-VP modification. The results demonstrated that:
(1) The unmodified sequences, including basic sequences 5, 51, 81, 82, 84, 3, 8, 9, 21, 31, 73, and 83, exhibited significant inhibitory effects on PCSK9, with the highest inhibition rate exceeding 60%.
(2) The multiply modified sequences can achieve inhibition rates of up to greater than 90%. Furthermore, when conjugated with GalNAc compounds, the modified sequences can be efficiently delivered to the liver in animals, exhibiting significant inhibition of PCSK9 gene expression, along with significantly reduced serum low-density lipoprotein cholesterol (LDL-C) and total cholesterol (TC) levels.

### Specifically:

**1. The alternately modified sequences disclosed herein exhibited significant inhibitory effects on PCSK9 gene expression.**
   (1) Among the 84 designed sequences, 12 sequences exhibited significant inhibitory effects on PCSK9 gene expression, with inhibition rates exceeding 50%, including P92-si5, P92-si51, P92-si81, P92-si82, P92-si84, P92-si3, P92-si8, P92-si9, P92-si21, P92-si31, P92-si73, and P92-si83. Specifically, P92-si5, P92-si81, P92-si82, P92-si3, P92-si8, and P92-si31 exhibited inhibition rates exceeding 70%.
   (2) A total of 26 sequences exhibited inhibition rates of 30% to 50% on PCSK9 gene expression. For example, the inhibition rates of P92-si6 and P92-si7 were 38.3% and 35.7%, respectively.
   (3) A total of 46 sequences exhibited inhibition rates below 30% on PCSK9 gene expression. For example, the inhibition rates of P92-si1 and P92-si20 were only 9.2% and 6.0%, respectively.
   (4) siRNAs with similar sequences exhibited significant differences in activity. For example, the inhibition rate of P92-si5 was 61.8% higher than that of P92-si4, indicating a significant improvement.
**2. The unmodified sequences disclosed herein exhibited significant inhibitory effects on PCSK9 gene expression.**
   (1) A total of 12 unmodified sequences exhibited significant inhibitory effects on PCSK9 gene expression, with inhibition rates exceeding 50%, including si5, si51, si81, si82, si84, si3, si8, si9, si21, si31, si73, and si83. Specifically, si5, si81, si82, si3, and si8 exhibited inhibition rates exceeding 60%.
   (2) The other unmodified sequences exhibited inhibition rates below 40% on PCSK9 gene expression. For example, the inhibition rates of si52 and si74 were only 0.5% and 2.2%, respectively.
   (3) siRNAs with similar sequences exhibited significant differences in activity. For example, the inhibition rate of basic sequence 5 was 51.2% higher than that of basic sequence 4, indicating a significant improvement.
   (4) The effects of alternating modifications on activity varied across different sequences. For some sequences, the inhibition rate was significantly improved. For example, for basic sequence 5, the alternately modified sequence exhibited a 12.4% increase in inhibition rate compared to the unmodified sequence. For others, the improvement was minimal. For example, basic sequences 4, 22, and 52 exhibited little to no difference in inhibition rate between alternately modified and unmodified sequences.
**3. The sequences modified using the templates disclosed herein exhibited significant inhibitory effects on PCSK9 gene expression.**
   (1) The basic sequences 5, 51, 81, 82, 84, 3, 8, 9, 21, 31, 73, and 83, when modified using templates DV25 to 29 designed in the present disclosure, exhibited significant inhibitory effects on PCSK9 gene expression, with inhibition rates exceeding 70%. Specifically, P92-si81-DV26, P92-si82-DV27, and P92-si82-DV29 exhibited inhibition rates of 89.3%, 89.3%, and 89.1%, respectively.
   (2) The 12 sequences modified using templates DV25 to 29 disclosed herein showed significantly enhanced inhibition of PCSK9 gene expression as compared to the alternately modified sequences. For example, for basic sequence 51, the inhibition rate of P92-si51-DV27 (the sequence modified using template DV27) was 29.1% higher than that of the alternately modified sequence; for basic sequence 81, the inhibition rate of P92-si81-DV26 (the sequence modified using template DV26) was 26.9% higher than that of the alternately modified sequence.
   (3) The same sequence modified using templates DV25 to 29 disclosed herein showed significantly enhanced inhibition of PCSK9 gene expression as compared to modification using the templates disclosed in the prior art. For example, for basic sequence 51, the sequence modified using template DV27 disclosed herein exhibited a 21.3% increase in inhibition rate compared to the sequence modified using the previously disclosed Advanced ESC template DV21.
   (4) The same sequence modified using templates DV25 to 29 disclosed herein showed significantly enhanced inhibition of PCSK9 gene expression as compared to modification using templates DV30 and DV31 disclosed herein. For example, for basic sequence 51, the sequence modified using template DV27 disclosed herein exhibited a 22.8% increase in inhibition rate compared to the sequence modified using template DV31 disclosed herein.
   (5) The 12 sequences exhibited EC₅₀ values ranging from 0.0001 nM to 0.005 nM. Specifically, the EC₅₀ values for P92-si81-DV27, P92-si84-DV26, and P92-si82-DV27 were 0.0003 nM, 0.0004 nM, and 0.0006 nM, respectively. The results demonstrated that these sequences effectively inhibited PCSK9 gene expression at low concentrations.
**4. The alternately modified sequences and the sequences modified using the templates disclosed herein exhibited significantly enhanced PCSK9 inhibitory activity compared to the unmodified sequences that are identical to or differ slightly from those disclosed in the prior art.**
   (1) Compared to the identical unmodified sequences disclosed in the prior art, the alternately modified sequences and the sequences modified using specific templates disclosed herein showed significantly enhanced inhibition of PCSK9 gene expression, with an increase of up to 40%.
   (2) Compared to the unmodified sequences with minor differences disclosed in the prior art, the unmodified sequences, alternately modified sequences, and template-modified sequences disclosed herein showed significantly enhanced inhibition of PCSK9 gene expression, with an increase of up to 40%.
      I. The unmodified sequences disclosed herein exhibited significantly enhanced activity compared to the similar unmodified sequences disclosed in the prior art. For example, the unmodified sequence si31 disclosed herein exhibited a 10.4% increase in inhibition rate compared to the structurally similar 31P.
      II. The alternately modified sequences disclosed herein exhibited significantly enhanced activity compared to the similar unmodified sequences disclosed in the prior art. For example, the alternately modified sequence P92-si5 disclosed herein exhibited a 22.0% increase in inhibition rate compared to the sequence 5P.
      III. The sequences modified using the templates disclosed herein exhibited significantly enhanced activity compared to the similar unmodified sequences disclosed in the prior art. For example, the alternately modified sequence P92-si82-DV29 disclosed herein exhibited a 42.8% increase in inhibition rate compared to the sequence 82P.
**5. The alternately modified and template-modified sequences disclosed herein, when incorporating specific anti-off-target designs, exhibited significant inhibitory effects on PCSK9 gene expression, showing significantly reduced inhibition of off-target genes.**
   (1) All modified sequences exhibited significant inhibitory effects on PCSK9 gene expression. For example, the alternately modified sequence P92-si3 exhibited an inhibition rate of 75.6%, while the template-modified and 5'-E-VP-modified sequence D84-DV27P exhibited an inhibition rate as high as 94.4%.
      I. The alternately modified sequences exhibited significant inhibitory effects on PCSK9 gene expression, with inhibition rates exceeding 60%, among which P92-si3 exhibited an inhibition rate of 75.6%.
      II. The template-modified sequences incorporating anti-off-target design or/and 5'-E-VP modification exhibited significant inhibitory effects on PCSK9 gene expression. For example, the template-modified and 5'-E-VP-modified sequence D84-DV27P exhibited an inhibition rate as high as 94.4%.
   (2) After adopting any one or more of the modification patterns disclosed herein followed by anti-off-target design, the resulting sequences exhibited significant anti-off-target effects on off-target genes, thereby reducing inhibition of off-target genes.
      I. The alternately modified sequences disclosed herein, when incorporating anti-off-target design, exhibited significant anti-off-target effects, reducing inhibition of off-target genes by 20%.
      II. The sequences modified using the templates disclosed herein, when incorporating anti-off-target design, exhibited significant anti-off-target effects, reducing inhibition of off-target genes by up to 73.6%.
**6. The sequences modified using the templates disclosed herein exhibited significant inhibition of serum PCSK9 expression, along with significantly reduced serum low-density lipoprotein cholesterol (LDL-C) and total cholesterol (TC) levels in mice.**
   (1) The basic sequences 5, 51, 81, 82, and 84 disclosed herein, when subjected to different modifications, exhibited significant inhibitory effects on serum PCSK9 expression. For example, D81-DV25G7 exhibited inhibition rates of 83.1%, 82.2%, and 70.3% on Day 7, Day 14, and Day 28, respectively.
   (2) The basic sequences 5, 51, 81, 82, and 84 disclosed herein, when subjected to different modifications, exhibited significantly reduced serum low-density lipoprotein cholesterol (LDL-C) levels. For example, D82-DV27PG5 exhibited reductions of 32.6%, 35.8%, and 21.7% on Day 7, Day 14, and Day 28, respectively.
   (3) The basic sequences 5, 51, 81, 82, and 84 disclosed herein, when subjected to different modifications, exhibited significantly reduced serum total cholesterol (TC) levels. For example, D82-DV29PG7 exhibited reductions of 32.2%, 26.8%, and 19.6% on Day 7, Day 14, and Day 28, respectively.

### Example 8: Synthesis of DV29-modified small interfering oligonucleotides

A total of 36 siRNA basic sequences were designed. VIR-2218 from Alnylam Pharmaceuticals was used as a positive control (Patent No.: CN108064294B), designated as APC-VIR in this example; ANC-DV29P was used as a negative control. To enhance the inhibitory efficiency and stability of the sequences, the basic sequences were modified using DV29P or DV29SP. In the sense strand, positions 7, 9, 11, and 17 are modified with 2'-F, while the remaining positions are modified with 2'-OMe. In the antisense strand, positions 2, 4, 5, 6, 14, and 16 are modified with 2'-F, while the remaining positions are modified with 2'-OMe. In addition, there are two phosphorothioate modifications at the 5' end of the sense strand; there are two phosphorothioate modifications at both the 5' and 3' ends of the antisense strand; there is one EVP modification at the 5' end of the antisense strand. The DV29P-modified siRNA sequences are shown in Table 43.

### 1. Synthesis of DV29P-modified sequence A194-DV29P

The siRNA basic sequence designated as A194-DV29P in Table 43 is as follows:
Sense strand: 5'-UCGUGUUACAGGCGGGGUUUU-3' (SEQ ID NO: 463)
Antisense strand: 5'-AAAACCCCGCCUGUAACACGAGA-3' (SEQ ID NO: 495)

In the sense strand, positions 7, 9, 11, and 17 are modified with 2'-F, while the remaining positions are modified with 2'-OMe. In the antisense strand, positions 2, 4, 5, 6, 14, and 16 are modified with 2'-F, while the remaining positions are modified with 2'-OMe. In addition, there are two phosphorothioate modifications at the 5' end of the sense strand; there are two phosphorothioate modifications at both the 5' and 3' ends of the antisense strand; there is one EVP modification at the 5' end of the antisense strand.

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-OMe phosphoramidite monomer (Formula 1), DMT-C-OMe phosphoramidite monomer (Formula 2), DMT-G-OMe phosphoramidite monomer (Formula 3), DMT-U-OMe phosphoramidite monomer (Formula 4), DMT-A-F phosphoramidite monomer (Formula 5), DMT-C-F phosphoramidite monomer (Formula 6), DMT-G-F phosphoramidite monomer (Formula 7), DMT-U-F phosphoramidite monomer (Formula 8), vinyl-(E)-phosphonate-A-OMe phosphoramidite monomer (Formula 11), and vinyl-(E)-phosphonate-U-OMe phosphoramidite monomer (Formula 12).

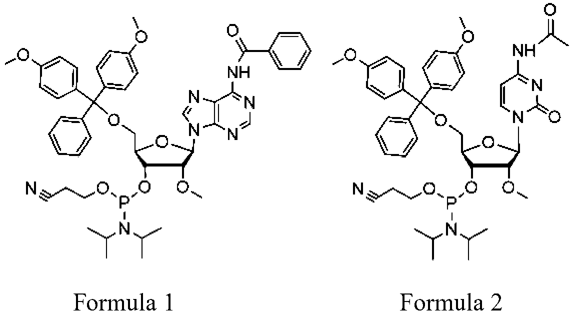

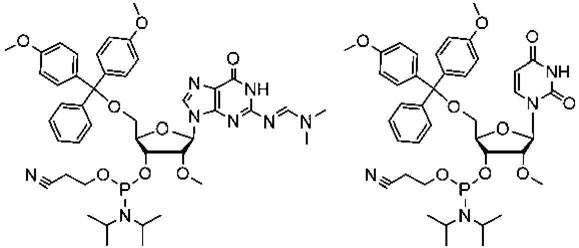

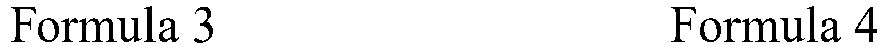

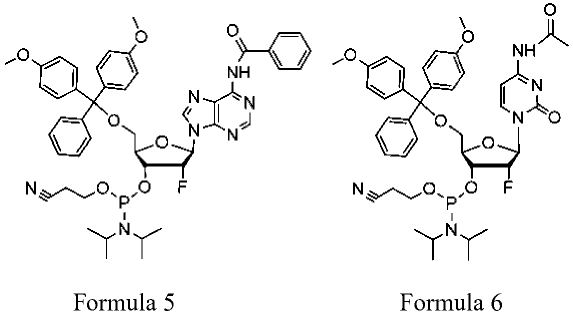

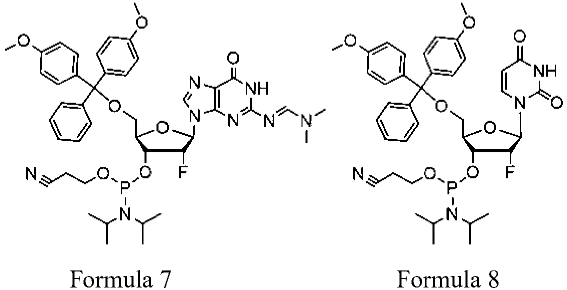

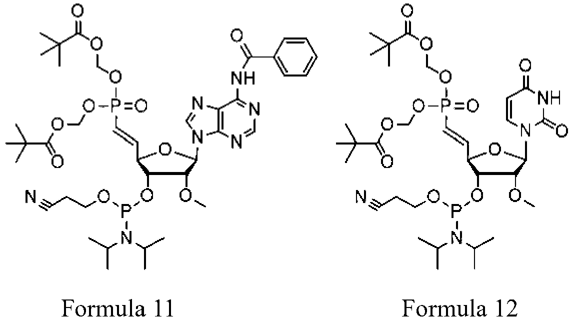

In the positive control sequence APC-VIR, the base at position 7 from the 5' end of the antisense strand contains a glycol nucleic acid (GNA) modification, and the structure of GNA monomer is as follows:

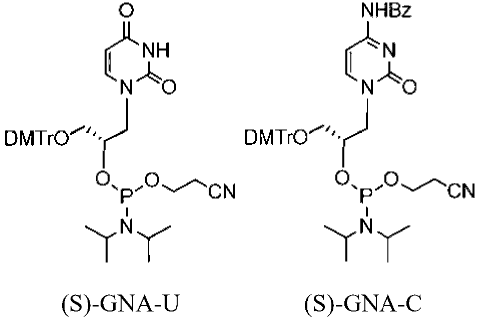

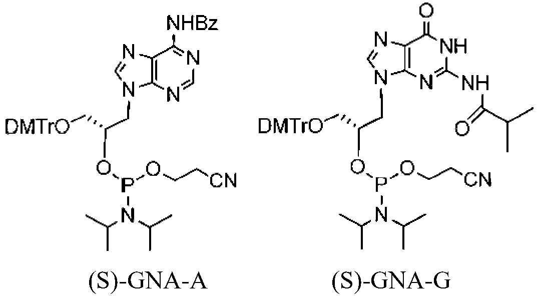

The preparation was carried out according to the following steps:
(1) Deprotection
   3% dichloroacetic acid in toluene was used as a deprotection reagent to remove the DMT protecting group, followed by washing with acetonitrile.
(2) Coupling
   Each nucleotide monomer in acetonitrile was coupled using 0.25 M 5-ethylthio-1*H-*tetrazole as an activator, followed by washing with acetonitrile.
(3) Oxidation/sulfurization
   Oxidation: 0.05 M iodine in pyridine/water (90/10) was used as an oxidant for oxidation, followed by washing with acetonitrile.
   Sulfurization: 3% xanthane hydride in pyridine was used as a sulfurizing agent for sulfurization, followed by washing with acetonitrile.
(4) Hydroxyl protection
   10% acetic anhydride in tetrahydrofuran (CAP A) and tetrahydrofuran/pyridine/*N-*methylimidazole (74/10/16, v/v/v) (CAP B) were used as hydroxyl protecting reagents for hydroxyl protection, followed by washing with acetonitrile.
   The above steps were repeated according to the set sequence to obtain a fully protected product.
(5) 3% dichloroacetic acid in toluene was used as a deprotection reagent to remove the DMT protecting group from the last nucleotide, followed by washing with acetonitrile.
(6) Ammonolysis and purification
   The solid-phase support was transferred to a reactor and treated with concentrated aqueous ammonia (25 to 28%) at 60°C for 12 hours. The system was then cooled to room temperature, and the mixture was filtered, followed by washing with a mixture of purified water and ethanol. The filtrates were combined, subjected to column chromatography, concentrated, and lyophilized to obtain a product.
(7) Annealing

The purified sense and antisense strands were mixed at a molar ratio of 1:1, heated to 95°C and maintained for 3 minutes, and slowly cooled to room temperature to form a duplex.

A194-DV29P exhibited a purity of 90.52% and a measured molecular weight of 14727.09.

### 2. Synthesis of other sequences

All other sequences listed in Table 43 were synthesized using the method described above.

**Table 43: DV29P-modified siRNA sequences**

| Basic sequence ID | Sequence ID | Sense sequence 5'-3' | SEQ ID NO: | Corr. basic SEQ ID NO: | Antisense sequence 5'-3' | SEQ ID NO: | Corr. basic SEQ ID NO: |
|---|---|---|---|---|---|---|---|
| B207S | B207S-DV29SP | Gms-Gms-Gm-Gm-Um- Uf-Um-Uf-Um-Cf-Um- Um-Gm-Um-Um-Gf- Am-Cm-Am-Am | 529 | 449 | UmsEVP-Ufs-Gm-Uf-Cf- Af-Am-Cm-Am-Am-Gm- Am-Am-Af-Am-Af-Cm- Cm-Cm-Cms-Gms-Cm | 627 | 456 |
| A1550 | A1550-DV29P | Cms-Cms-Cm-Gm-Um- Cm-Uf-Gm-Uf-Gm-Cf- Cm-Um-Um-Cm-Um-Cf- Am-Um-Cm-Um | 534 | 450 | AmsEVP-Gfs-Am-Uf-Gf- Af-Gm-Am-Am-Gm-Gm- Cm-Am-Cf-Am-Gf-Am- Cm-Gm-Gm-Gms-Gms-Am | 635 | 457 |
| B1572 | B1572-DV29P | Cms-Cms-Gm-Gm-Am- Cm-Cf-Gm-Uf-Gm-Uf- Gm-Cm-Am-Cm-Um-Uf- Cm-Gm-Cm-Um | 539 | 451 | AmsEVP-Gfs-Cm-Gf-Af- Af-Gm-Um-Gm-Cm-Am- Cm-Am-Cf-Gm-Gf-Um- Cm-Cm-Gm-Gms-Cms-Am | 643 | 458 |
| B1575 | B1575-DV29P | Gms-Ams-Cm-Cm-Gm- Um-Gf-Um-Gf-Cm-Af- Cm-Um-Um-Cm-Gm-Cf- Um-Um-Cm-Am | 544 | 452 | UmsEVP-Gfs-Am-Af-Gf- Cf-Gm-Am-Am-Gm-Um- Gm-Cm-Af-Cm-Af-Cm- Gm-Gm-Um-Cms-Cms-Gm | 649 | 459 |
| A1573 | A1573-DV29P | Cms-Gms-Gm-Am-Cm- Cm-Gf-Um-Gf-Um-Gf- Cm-Am-Cm-Um-Um-Cf- Gm-Cm-Um-Um | 549 | 453 | AmsEVP-Afs-Gm-Cf-Gf- Af-Am-Gm-Um-Gm-Cm- Am-Cm-Af-Cm-Gf-Gm- Um-Cm-Cm-Gms-Gms-Cm | 655 | 460 |
| B418S | B418S-DV29SP | Cms-Ums-Gm-Cm-Um- Af-Um-Gf-Cm-Cf-Um- Cm-Am-Um-Cm-Uf-Um- Cm-Um-Um | 554 | 454 | AmsEVP-Afs-Gm-Af-Af- Gf-Am-Um-Gm-Am-Gm- Gm-Cm-Af-Um-Af-Gm- Cm-Am-Gms-Cms-Am | 661 | 461 |
| A416 | A416-DV29P | Ums-Gms-Cm-Um-Gm- Cm-Uf-Am-Uf-Gm-Cf- Cm-Um-Cm-Am-Um-Cf- Um-Um-Cm-Um | 559 | 455 | AmsEVP-Gfs-Am-Af-Gf- Af-Um-Gm-Am-Gm-Gm- Cm-Am-Uf-Am-Gf-Cm- Am-Gm-Cm-Ams-Gms-Gm | 667 | 462 |
| A194 | A194-DV29P | Ums-Cms-Gm-Um-Gm- Um-Uf-Am-Cf-Am-Gf- Gm-Cm-Gm-Gm-Gm- Gf-Um-Um-Um-Um | 604 | 463 | AmsEVP-Afs-Am-Af-Cf- Cf-Cm-Cm-Gm-Cm-Cm- Um-Gm-Uf-Am-Af-Cm- Am-Cm-Gm-Ams-Gms-Am | 721 | 495 |
| A200 | A200-DV29P | Ums-Ams-Cm-Am-Gm- Gm-Cf-Gm-Gf-Gm-Gf- Um-Um-Um-Um-Um- Cf-Um-Um-Gm-Um | 605 | 464 | AmsEVP-Cfs-Am-Af-Gf- Af-Am-Am-Am-Am-Cm- Cm-Cm-Cf-Gm-Cf-Cm- Um-Gm-Um-Ams-Ams-Cm | 722 | 496 |
| A203 | A203-DV29P | Ams-Gms-Gm-Cm-Gm- Gm-Gf-Gm-Uf-Um-Uf- Um-Um-Cm-Um-Um- Gf-Um-Um-Gm-Am | 606 | 465 | UmsEVP-Cfs-Am-Af-Cf- Af-Am-Gm-Am-Am-Am- Am-Am-Cf-Cm-Cf-Cm- Gm-Cm-Cm-Ums-Gms-Um | 723 | 497 |
| A205 | A205-DV29P | Gms-Cms-Gm-Gm-Gm- Gm-Uf-Um-Uf-Um-Uf- Cm-Um-Um-Gm-Um- Uf-Gm-Am-Cm-Am | 564 | 466 | UmsEVP-Gfs-Um-Cf-Af- Af-Cm-Am-Am-Gm-Am- Am-Am-Af-Am-Cf-Cm- Cm-Cm-Gm-Cms-Cms-Um | 673 | 498 |
| A206 | A206-DV29P | Cms-Gms-Gm-Gm-Gm- Um-Uf-Um-Uf-Um-Cf- Um-Um-Gm-Um-Um- Gf-Am-Cm-Am-Am | 607 | 467 | UmsEVP-Ufs-Gm-Uf-Cf- Af-Am-Cm-Am-Am-Gm- Am-Am-Af-Am-Af-Cm- Cm-Cm-Cm-Gms-Cms-Cm | 724 | 499 |
| A248 | A248-DV29P | Gms-Ums-Cm-Um-Am- Gm-Af-Cm-Uf-Cm-Gf- Um-Gm-Gm-Um-Gm- Gf-Am-Cm-Um-Um | 608 | 468 | AmsEVP-Afs-Gm-Uf-Cf- Cf-Am-Cm-Cm-Am-Cm- Gm-Am-Gf-Um-Cf-Um- Am-Gm-Am-Cms-Ums-Cm | 725 | 500 |
| A255 | A255-DV29P | Cms-Ums-Cm-Gm-Um- Gm-Gf-Um-Gf-Gm-Af- Cm-Um-Um-Cm-Um-Cf- Um-Cm-Am-Am | 609 | 469 | UmsEVP-Ufs-Gm-Af-Gf- Af-Gm-Am-Am-Gm-Um- Cm-Cm-Af-Cm-Cf-Am- Cm-Gm-Am-Gms-Ums-Cm | 726 | 501 |
| A256 | A256-DV29P | Ums-Cms-Gm-Um-Gm- Gm-Uf-Gm-Gf-Am-Cf- Um-Um-Cm-Um-Cm-Uf- Cm-Am-Am-Um | 610 | 470 | AmsEVP-Ufs-Um-Gf-Af- Gf-Am-Gm-Am-Am-Gm- Um-Cm-Cf-Am-Cf-Cm- Am-Cm-Gm-Ams-Gms-Um | 727 | 502 |
| A258 | A258-DV29P | Gms-Ums-Gm-Gm-Um- Gm-Gf-Am-Cf-Um-Uf- Cm-Um-Cm-Um-Cm-Af- Am-Um-Um-Um | 611 | 471 | AmsEVP-Afs-Am-Uf-Uf- Gf-Am-Gm-Am-Gm-Am- Am-Gm-Uf-Cm-Cf-Am- Cm-Cm-Am-Cms-Gms-Am | 728 | 503 |
| A259 | A259-DV29P | Ums-Gms-Gm-Um-Gm- Gm-Af-Cm-Uf-Um-Cf- Um-Cm-Um-Cm-Am-Af- Um-Um-Um-Um | 612 | 472 | AmsEVP-Afs-Am-Af-Uf- Uf-Gm-Am-Gm-Am-Gm- Am-Am-Gf-Um-Cf-Cm- Am-Cm-Cm-Ams-Cms-Gm | 729 | 504 |
| A261 | A261-DV29P | Gms-Ums-Gm-Gm-Am- Cm-Uf-Um-Cf-Um-Cf- Um-Cm-Am-Am-Um- Uf-Um-Um-Cm-Um | 574 | 473 | AmsEVP-Gfs-Am-Af-Af- Af-Um-Um-Gm-Am-Gm- Am-Gm-Af-Am-Gf-Um- Cm-Cm-Am-Cms-Cms-Am | 685 | 505 |
| A381 | A381-DV29P | Gms-Ums-Gm-Um-Cm- Um-Gf-Cm-Gf-Gm-Cf- Gm-Um-Um-Um-Um- Af-Um-Cm-Am-Um | 613 | 474 | AmsEVP-Ufs-Gm-Af-Uf- Af-Am-Am-Am-Cm-Gm- Cm-Cm-Gf-Cm-Af-Gm- Am-Cm-Am-Cms-Ams-Um | 730 | 506 |
| A413 | A413-DV29P | Ums-Cms-Cm-Um-Gm- Cm-Uf-Gm-Cf-Um-Af- Um-Gm-Cm-Cm-Um-Cf- Am-Um-Cm-Um | 614 | 475 | AmsEVP-Gfs-Am-Uf-Gf- Af-Gm-Gm-Cm-Am-Um- Am-Gm-Cf-Am-Gf-Cm- Am-Gm-Gm-Ams-Ums-Gm | 731 | 507 |
| A463 | A463-DV29P | Ams-Ums-Gm-Um-Um- Gm-Cf-Cm-Cf-Gm-Uf- Um-Um-Gm-Um-Cm-Cf- Um-Cm-Um-Am | 615 | 476 | UmsEVP-Afs-Gm-Af-Gf- Gf-Am-Cm-Am-Am-Am- Cm-Gm-Gf-Gm-Cf-Am- Am-Cm-Am-Ums-Ams-Cm | 732 | 508 |
| A536 | A536-DV29P | Cms-Ums-Gm-Cm-Um- Cm-Af-Am-Gf-Gm-Af- Am-Cm-Cm-Um-Cm-Uf- Am-Um-Gm-Um | 616 | 477 | AmsEVP-Cfs-Am-Uf-Af- Gf-Am-Gm-Gm-Um-Um- Cm-Cm-Uf-Um-Gf-Am- Gm-Cm-Am-Gms-Ums-Am | 733 | 509 |
| A707 | A707-DV29P | Ums-Ams-Gm-Gm-Gm- Cm-Uf-Um-Uf-Cm-Cf- Cm-Cm-Cm-Am-Cm-Uf- Gm-Um-Um-Um | 617 | 478 | AmsEVP-Afs-Am-Cf-Af- Gf-Um-Gm-Gm-Gm-Gm- Gm-Am-Af-Am-Gf-Cm- Cm-Cm-Um-Ams-Cms-Gm | 734 | 510 |
| A734 | A734-DV29P | Cms-Ams-Gm-Um-Um- Am-Uf-Am-Uf-Gm-Gf- Am-Um-Gm-Am-Um- Gf-Um-Gm-Gm-Um | 618 | 479 | AmsEVP-Cfs-Cm-Af-Cf- Af-Um-Cm-Am-Um-Cm- Cm-Am-Uf-Am-Uf-Am- Am-Cm-Um-Gms-Ams-Am | 735 | 511 |
| A1260 | A1260-DV29P | Cms-Ums-Gm-Cm-Cm- Gm-Af-Um-Cf-Cm-Af- Um-Am-Cm-Um-Gm-Cf- Gm-Gm-Am-Am | 619 | 480 | UmsEVP-Ufs-Cm-Cf-Gf- Cf-Am-Gm-Um-Am-Um- Gm-Gm-Af-Um-Cf-Gm- Gm-Cm-Am-Gms-Ams-Gm | 736 | 512 |
| A1266 | A1266-DV29P | Ams-Ums-Cm-Cm-Am- Um-Af-Cm-Uf-Gm-Cf- Gm-Gm-Am-Am-Cm- Uf-Cm-Cm-Um-Am | 620 | 481 | UmsEVP-Afs-Gm-Gf-Af- Gf-Um-Um-Cm-Cm-Gm- Cm-Am-Gf-Um-Af-Um- Gm-Gm-Am-Ums-Cms-Gm | 737 | 513 |
| A1518 | A1518-DV29P | Ams-Cms-Cm-Am-Cm- Gm-Gf-Gm-Gf-Cm-Gf- Cm-Am-Cm-Cm-Um-Cf- Um-Cm-Um-Um | 621 | 482 | AmsEVP-Afs-Gm-Af-Gf- Af-Gm-Gm-Um-Gm-Cm- Gm-Cm-Cf-Cm-Cf-Gm- Um-Gm-Gm-Ums-Cms-Gm | 738 | 514 |
| A1520 | A1520-DV29P | Cms-Ams-Cm-Gm-Gm- Gm-Gf-Cm-Gf-Cm-Af- Cm-Cm-Um-Cm-Um-Cf- Um-Um-Um-Am | 622 | 483 | UmsEVP-Afs-Am-Af-Gf- Af-Gm-Am-Gm-Gm-Um- Gm-Cm-Gf-Cm-Cf-Cm- Cm-Gm-Um-Gms-Gms-Um | 739 | 515 |
| A1547 | A1547-DV29P | Cms-Ums-Cm-Cm-Cm- Cm-Gf-Um-Cf-Um-Gf- Um-Gm-Cm-Cm-Um-Uf- Cm-Um-Cm-Am | 623 | 484 | UmsEVP-Gfs-Am-Gf-Af- Af-Gm-Gm-Cm-Am-Cm- Am-Gm-Af-Cm-Gf-Gm- Gm-Gm-Am-Gms-Ums-Cm | 740 | 516 |
| A1548 | A1548-DV29P | Ums-Cms-Cm-Cm-Cm- Gm-Uf-Cm-Uf-Gm-Uf- Gm-Cm-Cm-Um-Um-Cf- Um-Cm-Am-Um | 624 | 485 | AmsEVP-Ufs-Gm-Af-Gf- Af-Am-Gm-Gm-Cm-Am- Cm-Am-Gf-Am-Cf-Gm- Gm-Gm-Gm-Ams-Gms-Um | 741 | 517 |
| B208 | B208-DV29P | Gms-Gms-Gm-Um-Um- Um-Uf-Um-Cf-Um-Uf- Gm-Um-Um-Gm-Am- Cf-Am-Am-Gm-Am | 569 | 486 | UmsEVP-Cfs-Um-Uf-Gf- Uf-Cm-Am-Am-Cm-Am- Am-Gm-Af-Am-Af-Am- Am-Cm-Cm-Cms-Cms-Gm | 679 | 518 |
| B262 | B262-DV29P | Ums-Gms-Gm-Am-Cm- Um-Uf-Cm-Uf-Cm-Uf- Cm-Am-Am-Um-Um- Uf-Um-Cm-Um-Am | 579 | 487 | UmsEVP-Afs-Gm-Af-Af- Af-Am-Um-Um-Gm-Am- Gm-Am-Gf-Am-Af-Gm- Um-Cm-Cm-Ams-Cms-Gm | 691 | 519 |
| B264 | B264-DV29P | Gms-Ams-Cm-Um-Um- Cm-Uf-Cm-Uf-Cm-Af- Am-Um-Um-Um-Um- Cf-Um-Am-Gm-Am | 584 | 488 | UmsEVP-Cfs-Um-Af-Gf- Af-Am-Am-Am-Um-Um- Gm-Am-Gf-Am-Gf-Am- Am-Gm-Um-Cms-Cms-Am | 697 | 520 |
| B1556 | B1556-DV29P | Ums-Gms-Um-Gm-Cm- Cm-Uf-Um-Cf-Um-Cf- Am-Um-Cm-Um-Gm-Cf- Cm-Gm-Gm-Am | 589 | 489 | UmsEVP-Cfs-Cm-Gf-Gf- Cf-Am-Gm-Am-Um-Gm- Am-Gm-Af-Am-Gf-Gm- Cm-Am-Cm-Ams-Gms-Am | 703 | 521 |
| B1560 | B1560-DV29P | Cms-Cms-Um-Um-Cm- Um-Cf-Am-Uf-Cm-Uf- Gm-Cm-Cm-Gm-Gm-Af- Cm-Cm-Gm-Um | 594 | 490 | AmsEVP-Cfs-Gm-Gf-Uf- Cf-Cm-Gm-Gm-Cm-Am- Gm-Am-Uf-Gm-Af-Gm- Am-Am-Gm-Gms-Cms-Am | 709 | 522 |
| B416S | B416S-DV29P | Ums-Gms-Cm-Um-Gm- Cf-Um-Af-Um-Gf-Cm- Cm-Um-Cm-Am-Uf-Cm- Um-Um-Am | 599 | 491 | UmsEVP-Afs-Am-Gf-Af- Uf-Gm-Am-Gm-Gm-Cm- Am-Um-Af-Gm-Cf-Am- Gm-Cm-Ams-Gms-Gm | 715 | 523 |
| APC | APC-VIR | Gms-Ums-Gm-Um-Gf- Cm-Af-Cf-Uf-Um-Cm- Gm-Cm-Um-Um-Cm- Am-Cm-Am | 625 | 493 | Ums-Gfs-Um-Gm-Am- A(gna)-Gm-Cf-Gf-Am-Am- Gm-Um-Gf-Cm-Af-Cm- Am-Cms-Ums-Um | 742 | 527 |
| ANC | ANC-DV29P | Gms-Gms-Um-Cm-Um- Cm-Uf-Um-Cf-Um-Af- Um-Cm-Gm-Am-Um-Cf- Um-Um-Am-Um | 626 | 494 | AmsEVP-Ufs-Am-Af-Gf- Af-Um-Cm-Gm-Am-Um- Am-Gm-Af-Am-Gf-Am- Gm-Am-Cm-Cms-Cms-Am | 743 | 528 |

### Example 9: Inhibitory effect of DV29P-modified sequences on HBsAg and HBeAg

The DV29P- or DV29SP-modified siRNA sequences synthesized in Example 8 were transfected into HepG2.2.15 cells via lipid nanoparticles (LNPs). The inhibitory effects of these sequences on HBsAg and HBeAg were then evaluated by ELISA.

### 1. Experimental materials

Test samples: The DV29P- or DV29SP-modified siRNA sequences listed in Table 43 (synthesized in Example 8).

Cell line: HepG2.2.15 cells.

Solvents: sterile nuclease-free water, Gibco Opti-MEM (Thermo Fisher Scientific).

### 2. Experimental methods

The inhibitory effects of the test samples on HBsAg and HBeAg in HepG2.2.15 cells were evaluated by ELISA.

### 2.1 Cell culture

Subculture: HepG2.2.15 cells were cultured in DMEM/F12 medium containing 10% fetal bovine serum, 370 µg/mL GENETICN, 1% L-glutamine, 1% non-essential amino acids, and 1% penicillin-streptomycin, and incubated in a cell incubator at 37°C with 5% CO₂. Cells were passaged every three days. The cells were digested with 0.25% trypsin, followed by centrifugation at 800 rpm for 3 minutes. The supernatant was discarded, and fresh medium was added for continued culture.

Seeding: HepG2.2.15 cells were seeded in DMEM/F12 medium containing 10% fetal bovine serum, 1% L-glutamine, 1% non-essential amino acids, and 1% penicillin-streptomycin.

### 2.2 Cell transfection

Preparation of transfection mixture: Lipofectamine RNAiMAX (Thermo Fisher Scientific) was mixed with Opti-MEM at a volume ratio of 2:98 and vortexed thoroughly.

Preparation of transfection complex: 30 µL of siRNA solution (diluted in Opti-MEM) was mixed with 30 µL of transfection mixture at a volume ratio of 1:1, vortexed thoroughly, followed by incubation at room temperature for 15 minutes to obtain a transfection complex.

Control preparation (transfection reagent): 15 µL of transfection mixture was added to 15 µL of Opti-MEM, vortexed thoroughly, followed by incubation at room temperature for 15 minutes.

The prepared transfection complex was added to a 96-well cell culture plate (15 µL per well, three replicates per sequence), resulting in a final siRNA concentration of 0.3 nM per well. Subsequently, 135 µL of cell suspension (2.25 × 10⁴ cells) was added to each well. After cross-mixing, the plate was incubated in a cell incubator at 37°C with 5% CO₂.

### 2.3 Measurement of HBsAg and HBeAg in cell supernatant

1) Collection of cell supernatant
   a. On day 3 post-transfection, the medium was exchanged by discarding the cell supernatant and adding 150 µL of fresh medium per well for continued culture.
   b. On day 6 post-transfection, the cell supernatant was collected for measurement of HBsAg and HBeAg levels.
2) Quantification of HBsAg and HBeAg
   HBsAg and HBeAg levels were measured using the HBeAg assay kit (Autobio, CL0310) and the HBsAg assay kit (Autobio, CL0312). The specific procedures are as follows:
   a. The kits and test samples were equilibrated to room temperature.
   b. 50 µL each of test samples, standards, negative control, and positive control were added to each well of a plate.
   c. 50 µL of enzyme conjugate was added to each well, mixed thoroughly, followed by incubation at 37°C for 60 minutes.
   d. The liquid was removed from the plate, which was wash 5 times with wash buffer and finally blotted dry on absorbent paper.
   e. Chemiluminescent substrates A and B were mixed at equal ratio, added to the plate at 50 µL per well, followed by incubation at room temperature in the dark for 3 minutes.
   f. Luminescence was measured using a microplate reader.

### 2.4 Data processing

The inhibition rates of HBsAg and HBeAg were calculated using the following formulas: 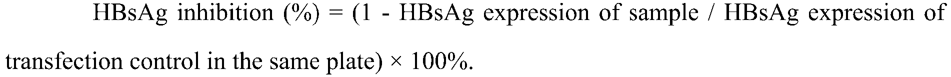 HBsAg inhibition (%) = (1 - HBsAg expression of sample / HBsAg expression of transfection control in the same plate) × 100%. 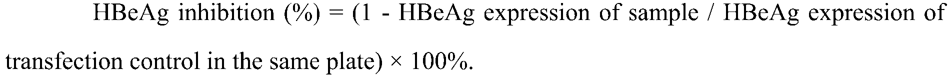 HBeAg inhibition (%) = (1 - HBeAg expression of sample / HBeAg expression of transfection control in the same plate) × 100%.

HBsAg inhibition relative to positive control (inhibition of positive control set as 1) = HBsAg inhibition of sample (%) / inhibition of positive control in the same plate (%). It should be understood that a value greater than 1 indicates that the inhibitory effect of the sequence on HBsAg is superior to that of the positive control, while a value less than 1 indicates that the inhibitory effect of the sequence on HBsAg is weaker than that of the positive control.

HBeAg inhibition relative to positive control (inhibition of positive control set as 1) = HBeAg inhibition of sample (%) / inhibition of positive control in the same plate (%). It should be understood that a value greater than 1 indicates that the inhibitory effect of the sequence on HBeAg is superior to that of the positive control, while a value less than 1 indicates that the inhibitory effect of the sequence on HBeAg is weaker than that of the positive control.

### 2.5 EC₅₀ assay

The sequences used for EC₅₀ determination were subjected to serial dilution starting from 10 nM with 4-fold dilutions, totaling 8 concentration points (10 nM, 2.5 nM, 0.625 nM, 0.15625 nM, 39.06 pM, 9.77 pM, 2.44 pM, and 0.61 pM). The inhibition rates for these sequences at each concentration were measured, and the resulting data were plotted to calculate EC₅₀ values for each sequence and the positive control.

### 2.6 Cytotoxicity assay

During the EC₅₀ assay, after collecting the cell supernatant, cell viability was measured using the CellTiter-Glo^{®} (Promega) assay kit. The procedure was briefly described as follows: CellTiter-Glo reagent was mixed with culture medium at a ratio of 1:1, added to the plate at 100 µL per well, followed by incubation at room temperature for 10 minutes. Luminescence was measured using a microplate reader. 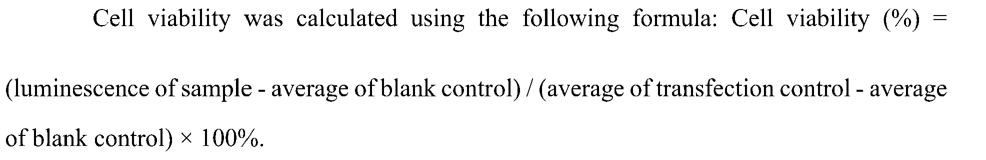Cell viability was calculated using the following formula: Cell viability (%) = (luminescence of sample - average of blank control) / (average of transfection control - average of blank control) × 100%.

### 3. Experimental results

The experimental results demonstrated that the sequences B207S-DV29SP, A1550-DV29P, B1572-DV29P, B1575-DV29P, A1573-DV29P, B418S-DV29SP, and A416-DV29P exhibited significantly superior inhibitory effects on HBsAg compared to other sequences, and significantly superior inhibitory effects on HBeAg compared to other sequences.

### Example 10: Inhibitory effect of unmodified sequences on HBV gene expression

In this example, certain unmodified sequences corresponding to the modified sequences in Example 8 were synthesized and transfected into HepG2.2.15 cells via lipid nanoparticles (LNPs). The inhibitory effects of these sequences on HBsAg and HBeAg were evaluated by ELISA to identify unmodified siRNA sequences with favorable inhibitory activity.

### 1. Experimental materials

### Test samples:

The unmodified siRNA sequences listed in Table 44. All sequences were synthesized using the method described in Example 8.

**Table 44: Unmodified siRNA sequences**

| Sequence ID | Sense sequence 5'-3' | SEQ ID NO: | Antisense sequence 5'-3' | SEQ ID NO: |
|---|---|---|---|---|
| B207S | GGGGUUUUUCUUGUUGACAA | 449 | UUGUCAACAAGAAAAACCCCGC | 456 |
| A1550 | CCCGUCUGUGCCUUCUCAUCU | 450 | AGAUGAGAAGGCACAGACGGGGA | 457 |
| B1572 | CCGGACCGUGUGCACUUCGCU | 451 | AGCGAAGUGCACACGGUCCGGCA | 458 |
| B1575 | GACCGUGUGCACUUCGCUUCA | 452 | UGAAGCGAAGUGCACACGGUCCG | 459 |
| A1573 | CGGACCGUGUGCACUUCGCUU | 453 | AAGCGAAGUGCACACGGUCCGGC | 460 |
| B418S | CUGCUAUGCCUCAUCUUCUU | 454 | AAGAAGAUGAGGCAUAGCAGCA | 461 |
| A416 | UGCUGCUAUGCCUCAUCUUCU | 455 | AGAAGAUGAGGCAUAGCAGCAGG | 462 |
| A194 | UCGUGUUACAGGCGGGGUUUU | 463 | AAAACCCCGCCUGUAACACGAGA | 495 |
| A200 | UACAGGCGGGGUUUUUCUUGU | 464 | ACAAGAAAAACCCCGCCUGUAAC | 496 |
| A203 | AGGCGGGGUUUUUCUUGUUGA | 465 | UCAACAAGAAAAACCCCGCCUGU | 497 |
| A205 | GCGGGGUUUUUCUUGUUGACA | 466 | UGUCAACAAGAAAAACCCCGCCU | 498 |
| A206 | CGGGGUUUUUCUUGUUGACAA | 467 | UUGUCAACAAGAAAAACCCCGCC | 499 |
| A248 | GUCUAGACUCGUGGUGGACUU | 468 | AAGUCCACCACGAGUCUAGACUC | 500 |
| A255 | CUCGUGGUGGACUUCUCUCAA | 469 | UUGAGAGAAGUCCACCACGAGUC | 501 |
| A256 | UCGUGGUGGACUUCUCUCAAU | 470 | AUUGAGAGAAGUCCACCACGAGU | 502 |
| A258 | GUGGUGGACUUCUCUCAAUUU | 471 | AAAUUGAGAGAAGUCCACCACGA | 503 |
| A259 | UGGUGGACUUCUCUCAAUUUU | 472 | AAAAUUGAGAGAAGUCCACCACG | 504 |
| A261 | GUGGACUUCUCUCAAUUUUCU | 473 | AGAAAAUUGAGAGAAGUCCACCA | 505 |
| A381 | GUGUCUGCGGCGUUUUAUCAU | 474 | AUGAUAAAACGCCGCAGACACAU | 506 |
| A413 | UCCUGCUGCUAUGCCUCAUCU | 475 | AGAUGAGGCAUAGCAGCAGGAUG | 507 |
| A463 | AUGUUGCCCGUUUGUCCUCUA | 476 | UAGAGGACAAACGGGCAACAUAC | 508 |
| A536 | CUGCUCAAGGAACCUCUAUGU | 477 | ACAUAGAGGUUCCUUGAGCAGUA | 509 |
| A707 | UAGGGCUUUCCCCCACUGUUU | 478 | AAACAGUGGGGGAAAGCCCUACG | 510 |
| A734 | CAGUUAUAUGGAUGAUGUGGU | 479 | ACCACAUCAUCCAUAUAACUGAA | 511 |
| A1260 | CUGCCGAUCCAUACUGCGGAA | 480 | UUCCGCAGUAUGGAUCGGCAGAG | 512 |
| A1266 | AUCCAUACUGCGGAACUCCUA | 481 | UAGGAGUUCCGCAGUAUGGAUCG | 513 |
| A1518 | ACCACGGGGCGCACCUCUCUU | 482 | AAGAGAGGUGCGCCCCGUGGUCG | 514 |
| A1520 | CACGGGGCGCACCUCUCUUUA | 483 | UAAAGAGAGGUGCGCCCCGUGGU | 515 |
| A1547 | CUCCCCGUCUGUGCCUUCUCA | 484 | UGAGAAGGCACAGACGGGGAGUC | 516 |
| A1548 | UCCCCGUCUGUGCCUUCUCAU | 485 | AUGAGAAGGCACAGACGGGGAGU | 517 |
| B208 | GGGUUUUUCUUGUUGACAAGA | 486 | UCUUGUCAACAAGAAAAACCCCG | 518 |
| B262 | UGGACUUCUCUCAAUUUUCUA | 487 | UAGAAAAUUGAGAGAAGUCCACG | 519 |
| B264 | GACUUCUCUCAAUUUUCUAGA | 488 | UCUAGAAAAUUGAGAGAAGUCCA | 520 |
| B1556 | UGUGCCUUCUCAUCUGCCGGA | 489 | UCCGGCAGAUGAGAAGGCACAGA | 521 |
| B1560 | CCUUCUCAUCUGCCGGACCGU | 490 | ACGGUCCGGCAGAUGAGAAGGCA | 522 |
| B416S | UGCUGCUAUGCCUCAUCUUA | 491 | UAAGAUGAGGCAUAGCAGCAGG | 523 |
| APC | GUGUGCACUUCGCUUCACA | 493 | UGUGAAGCGAAGUGCACACUU | 527 |
| ANC | GGUCUCUUCUAUCGAUCUUAU | 494 | AUAAGAUCGAUAGAAGAGACCCA | 528 |

Cell line: HepG2.2.15 cells.

Solvents: sterile nuclease-free water, Gibco Opti-MEM.

### 2. Experimental methods

Refer to Example 2.

### 3. Experimental results

The experimental results demonstrated that the sequences B207S, A1550, B1572, B1575, A1573, B418S, and A416 exhibited significantly superior inhibitory effects on HBsAg compared to other sequences, and significantly superior inhibitory effects on HBeAg compared to other sequences.

### Example 11: Inhibitory effect of sequences modified using different templates on HBsAg and HBeAg

In this example, 15 unmodified sequences, namely A205, B208, B207S, A261, B262, B264, A1550, B1556, B1560, B1575, A1573, B1572, B418S, A416, and B416S, were each modified using eight templates designed in the present disclosure, namely DV25P, DV26P, DV27P, DV28P, DV29P, DV32P, DV33P, and DV34P (DV25SP, DV26SP, DV27SP, DV28SP, DV29SP, DV32SP, DV33SP, and DV34SP for the shorter sequences), as well as the previously disclosed templates DV30P, DV31P, DV21P, DV22P, DV30SP, DV31SP, DV21SP, and DV22SP, resulting in a total of 154 sequences, which were transfected into HepG2.2.15 cells via lipid nanoparticles (LNPs). The inhibitory effects of these sequences on HBsAg and HBeAg were then evaluated by ELISA.

### 1. Experimental materials

Test samples: The sequences listed in Table 47, which were modified from 15 siRNA basic sequences using different templates (DV25P, DV26P, DV27P, DV28P, DV29P, DV32P, DV33P, DV34P, DV30P, DV31P, DV21P, DV22P, DV25SP, DV26SP, DV27SP, DV28SP, DV29SP, DV32SP, DV33SP, DV34SP, and DV22SP) (refers to Example 8 for the synthesis method).

Cell line: HepG2.2.15 cells.

Solvents: sterile nuclease-free water, Gibco Opti-MEM.

The modification principles for the templates disclosed herein are as follows:

### For modification templates DV25P to DV29P and DV32P to DV34P:

The antisense strand adopts one of the modification patterns shown in Table 45-1:

**Table 45-1: Modification of antisense strand**

| No. | **Antisense strand (AS) 5'-3'** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | **2'-OMe** +PS | **2'-OMe** +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |

The sense strand adopts one of the modification patterns shown in Table 45-2:

**Table 45-2: Modification of sense strand**

| No. | **Sense strand (SS) 5'-3'** | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |

In the above tables, 2'-OMe represents a 2'-O-methyl modification; 2'-F represents a 2'-fluoro modification; PS represents a phosphorothioate backbone; EVP represents 5'-vinyl-(E)-phosphonate.

The siRNA modification template, in which the antisense strand is modified according to modification pattern A and the sense strand is modified according to modification pattern a, is designated as DV25P.

The siRNA modification template, in which the antisense strand is modified according to modification pattern B and the sense strand is modified according to modification pattern a, is designated as DV26P.

The siRNA modification template, in which the antisense strand is modified according to modification pattern C and the sense strand is modified according to modification pattern a, is designated as DV27P.

The siRNA modification template, in which the antisense strand is modified according to modification pattern B and the sense strand is modified according to modification pattern b, is designated as DV28P.

The siRNA modification template, in which the antisense strand is modified according to modification pattern C and the sense strand is modified according to modification pattern b, is designated as DV29P.

The siRNA modification template, in which the antisense strand is modified according to modification pattern D and the sense strand is modified according to modification pattern b, is designated as DV32P.

The siRNA modification template, in which the antisense strand is modified according to modification pattern E and the sense strand is modified according to modification pattern b, is designated as DV33P.

The siRNA modification template, in which the antisense strand is modified according to modification pattern F and the sense strand is modified according to modification pattern b, is designated as DV34P.

The siRNA modification template, in which the antisense strand is modified according to modification pattern A and the sense strand is modified according to modification pattern c, is designated as DV22P.

### For modification templates DV25SP to DV29SP and DV32SP to DV34SP:

The antisense strand adopts one of the modification patterns shown in Table 46-1:

**Table 46-1: Modification of antisense strand**

| No. | **Antisense strand (AS) 5'-3'** | | | | | | | | | | |
|---|---|---|---|---|---|---|---|---|---|---|---|
| **A'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **B'** | **1** | **2** | **3** | **4** | **5** | **6** | **7** | **8** | **9** | **10** | **11** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **E'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

The sense strand adopts one of the modification patterns shown in Table 46-2:

**Table 46-2: Modification of sense strand**

| No. | **Sense strand (SS) 5'-3'** | | | | | | | | | |
|---|---|---|---|---|---|---|---|---|---|---|
| **a'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **c'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-OMe | 2'-OMe | 2'-OMe | 2'-OMe | 2'-OMe |

In the above tables, 2'-OMe represents a 2'-O-methyl modification; 2'-F represents a 2'-fluoro modification; PS represents a phosphorothioate backbone.

The siRNA modification template, in which the antisense strand is modified according to modification pattern A' and the sense strand is modified according to modification pattern a', is designated as DV25SP.

The siRNA modification template, in which the antisense strand is modified according to modification pattern B' and the sense strand is modified according to modification pattern a', is designated as DV26SP.

The siRNA modification template, in which the antisense strand is modified according to modification pattern C' and the sense strand is modified according to modification pattern a', is designated as DV27SP.

The siRNA modification template, in which the antisense strand is modified according to modification pattern B' and the sense strand is modified according to modification pattern b', is designated as DV28SP.

The siRNA modification template, in which the antisense strand is modified according to modification pattern C' and the sense strand is modified according to modification pattern b', is designated as DV29SP.

The siRNA modification template, in which the antisense strand is modified according to modification pattern D' and the sense strand is modified according to modification pattern b', is designated as DV32SP.

The siRNA modification template, in which the antisense strand is modified according to modification pattern E' and the sense strand is modified according to modification pattern b', is designated as DV33SP.

The siRNA modification template, in which the antisense strand is modified according to modification pattern F' and the sense strand is modified according to modification pattern b', is designated as DV34SP.

The siRNA modification template, in which the antisense strand is modified according to modification pattern A' and the sense strand is modified according to modification pattern c', is designated as DV22SP.

### For DV30SP:

### Antisense strand:

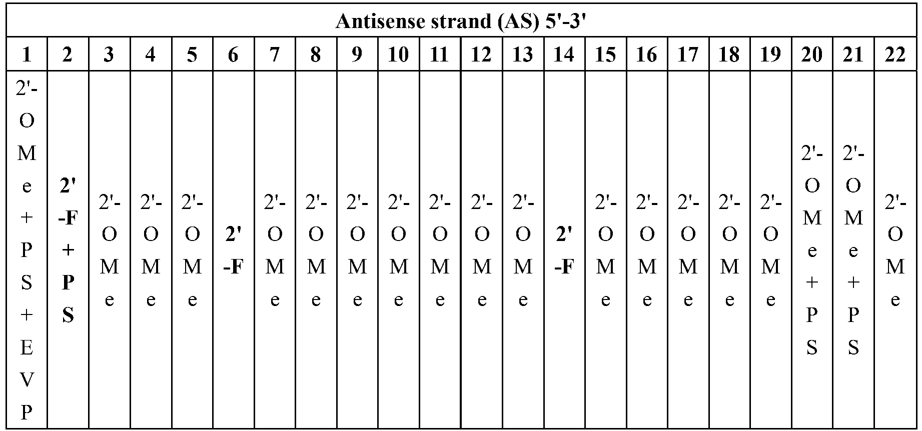

### Sense strand:

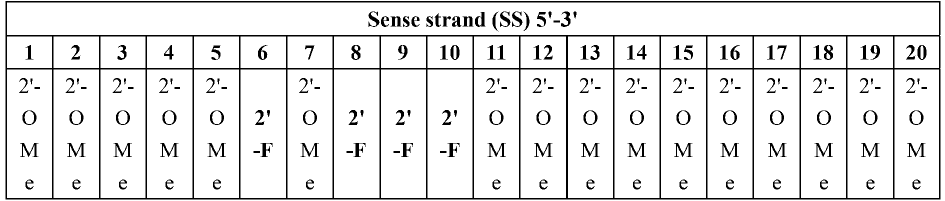

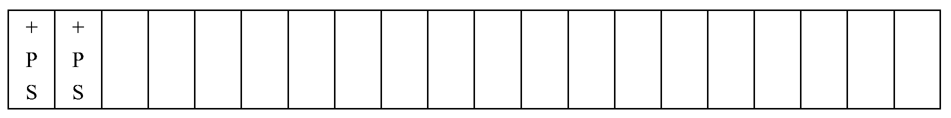

In the above tables, PS represents a phosphorothioate backbone.

### For DV31SP:

### Antisense strand:

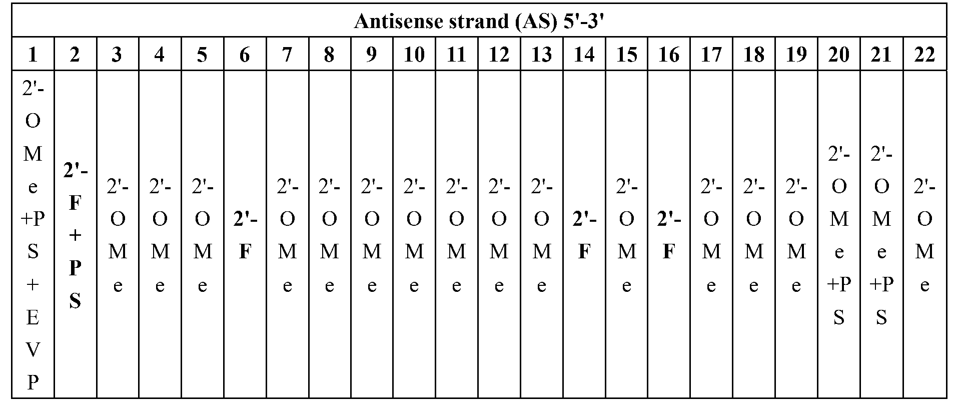

### Sense strand:

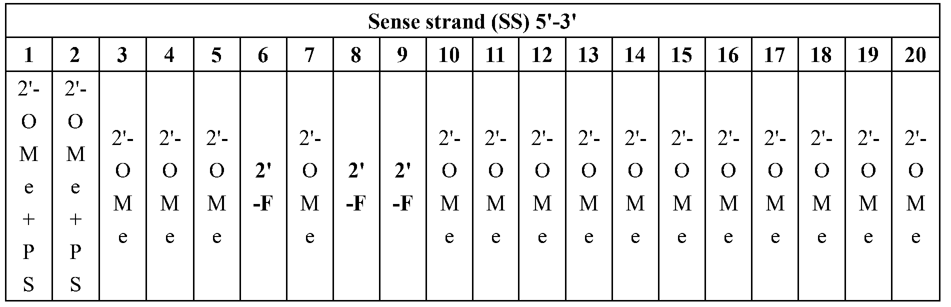

In the above tables, PS represents a phosphorothioate backbone.

These sequences were synthesized using the same method as described in Example 8.

**Table 47: Sequences modified using different templates**

| Sequence ID | Sense sequence 5'-3' | SEQ ID NO: | Corr. basic SEQ ID NO: | Antisense sequence 5'-3' | SEQ ID NO: | Corr. basic SEQ ID NO: |
|---|---|---|---|---|---|---|
| B207S-DV29SP | Gms-Gms-Gm-Gm-Um- Uf-Um-Uf-Um-Cf-Um- Um-Gm-Um-Um-Gf-Am- Cm-Am-Am | 529 | 449 | UmsEVP-Ufs-Gm-Uf-Cf-Af- Am-Cm-Am-Am-Gm-Am- Am-Af-Am-Af-Cm-Cm-Cm- Cms-Gms-Cm | 627 | 456 |
| B207S-DV26SP | Gms-Gms-Gm-Gm-Um- Uf-Um-Uf-Uf-Cf-Um-Um- Gm-Um-Um-Gf-Am-Cm- Am-Am | 530 | 449 | UmsEVP-Ufs-Gf-Uf-Cm-Af- Am-Cm-Am-Am-Gm-Am- Am-Af-Am-Af-Cm-Cm-Cm- Cms-Gms-Cm | 628 | 456 |
| B207S-DV27SP | Gms-Gms-Gm-Gm-Um- Uf-Um-Uf-Uf-Cf-Um-Um- Gm-Um-Um-Gf-Am-Cm- Am-Am | 530 | 449 | UmsEVP-Ufs-Gm-Uf-Cf-Af- Am-Cm-Am-Am-Gm-Am- Am-Af-Am-Af-Cm-Cm-Cm- Cms-Gms-Cm | 627 | 456 |
| B207S-DV28SP | Gms-Gms-Gm-Gm-Um- Uf-Um-Uf-Um-Cf-Um- Um-Gm-Um-Um-Gf-Am- Cm-Am-Am | 529 | 449 | UmsEVP-Ufs-Gf-Uf-Cm-Af- Am-Cm-Am-Am-Gm-Am- Am-Af-Am-Af-Cm-Cm-Cm- Cms-Gms-Cm | 628 | 456 |
| B207S-DV32SP | Gms-Gms-Gm-Gm-Um- Uf-Um-Uf-Um-Cf-Um- Um-Gm-Um-Um-Gf-Am- Cm-Am-Am | 529 | 449 | UmsEVP-Ufs-Gm-Um-Cf-Af- Am-Cm-Am-Am-Gm-Am- Am-Af-Am-Af-Cm-Cm-Cm- Cms-Gms-Cm | 629 | 456 |
| B207S-DV34SP | Gms-Gms-Gm-Gm-Um- Uf-Um-Uf-Um-Cf-Um- Um-Gm-Um-Um-Gf-Am- Cm-Am-Am | 529 | 449 | UmsEVP-Ufs-Gf-Um-Cm-Af- Am-Cm-Am-Am-Gm-Am- Am-Af-Am-Af-Cm-Cm-Cm- Cms-Gms-Cm | 630 | 456 |
| B207S-DV25SP | Gms-Gms-Gm-Gm-Um- Uf-Um-Uf-Uf-Cf-Um-Um- Gm-Um-Um-Gf-Am-Cm- Am-Am | 530 | 449 | UmsEVP-Ufs-Gm-Um-Cm- Af-Am-Cm-Am-Am-Gm-Am- Am-Af-Am-Af-Cm-Cm-Cm- Cms-Gms-Cm | 631 | 456 |
| B207S-DV33SP | Gms-Gms-Gm-Gm-Um- Uf-Um-Uf-Um-Cf-Um- Um-Gm-Um-Um-Gf-Am- Cm-Am-Am | 529 | 449 | UmsEVP-Ufs-Gf-Um-Cf-Af- Am-Cm-Am-Am-Gm-Am- Am-Af-Am-Af-Cm-Cm-Cm- Cms-Gms-Cm | 632 | 456 |
| B207S-DV30SP | Gms-Gms-Gm-Gm-Um- Uf-Um-Uf-Uf-Cf-Um-Um- Gm-Um-Um-Gm-Am-Cm- Am-Am | 531 | 449 | UmsEVP-Ufs-Gm-Um-Cm- Af-Am-Cm-Am-Am-Gm-Am- Am-Af-Am-Am-Cm-Cm-Cm- Cms-Gms-Cm | 633 | 456 |
| B207S-DV31SP | Gms-Gms-Gm-Gm-Um- Uf-Um-Uf-Uf-Cm-Um- Um-Gm-Um-Um-Gm-Am- Cm-Am-Am | 532 | 449 | UmsEVP-Ufs-Gm-Um-Cm- Af-Am-Cm-Am-Am-Gm-Am- Am-Af-Am-Af-Cm-Cm-Cm- Cms-Gms-Cm | 634 | 456 |
| B207S-DV21SP | Gms-Gms-Gm-Gm-Um- Uf-Um-Uf-Uf-Cf-Um-Um- Gm-Um-Uf-Gf-Am-Cm- Am-Am | 533 | 449 | UmsEVP-Ufs-Gm-Um-Cm- Af-Am-Cm-Am-Am-Gm-Am- Am-Af-Am-Af-Cm-Cm-Cm- Cms-Gms-Cm | 634 | 456 |
| B207S-DV22SP | Gms-Gms-Gm-Gm-Um- Uf-Um-Uf-Uf-Cf-Um-Um- Gm-Um-Um-Gm-Am-Cm- Am-Am | 531 | 449 | UmsEVP-Ufs-Gm-Um-Cm- Af-Am-Cm-Am-Am-Gm-Am- Am-Af-Am-Af-Cm-Cm-Cm- Cms-Gms-Cm | 634 | 456 |
| A1550-DV29P | Cms-Cms-Cm-Gm-Um- Cm-Uf-Gm-Uf-Gm-Cf- Cm-Um-Um-Cm-Um-Cf- Am-Um-Cm-Um | 534 | 450 | AmsEVP-Gfs-Am-Uf-Gf-Af- Gm-Am-Am-Gm-Gm-Cm- Am-Cf-Am-Gf-Am-Cm-Gm- Gm-Gms-Gms-Am | 635 | 457 |
| B1550-DV26P | Cms-Cms-Cm-Gm-Um- Cm-Uf-Gm-Uf-Gf-Cf-Cm- Um-Um-Cm-Um-Cf-Am- Um-Cm-Um | 535 | 450 | AmsEVP-Gfs-Af-Uf-Gm-Af- Gm-Am-Am-Gm-Gm-Cm- Am-Cf-Am-Gf-Am-Cm-Gm- Gm-Gms-Gms-Am | 636 | 457 |
| B1550-DV27P | Cms-Cms-Cm-Gm-Um- Cm-Uf-Gm-Uf-Gf-Cf-Cm- Um-Um-Cm-Um-Cf-Am- Um-Cm-Um | 535 | 450 | AmsEVP-Gfs-Am-Uf-Gf-Af- Gm-Am-Am-Gm-Gm-Cm- Am-Cf-Am-Gf-Am-Cm-Gm- Gm-Gms-Gms-Am | 635 | 457 |
| B1550-DV28P | Cms-Cms-Cm-Gm-Um- Cm-Uf-Gm-Uf-Gm-Cf- Cm-Um-Um-Cm-Um-Cf- Am-Um-Cm-Um | 534 | 450 | AmsEVP-Gfs-Af-Uf-Gm-Af- Gm-Am-Am-Gm-Gm-Cm- Am-Cf-Am-Gf-Am-Cm-Gm- Gm-Gms-Gms-Am | 636 | 457 |
| B1550-DV32P | Cms-Cms-Cm-Gm-Um- Cm-Uf-Gm-Uf-Gm-Cf- Cm-Um-Um-Cm-Um-Cf- Am-Um-Cm-Um | 534 | 450 | AmsEVP-Gfs-Am-Um-Gf-Af- Gm-Am-Am-Gm-Gm-Cm- Am-Cf-Am-Gf-Am-Cm-Gm- Gm-Gms-Gms-Am | 637 | 457 |
| B1550-DV34P | Cms-Cms-Cm-Gm-Um- Cm-Uf-Gm-Uf-Gm-Cf- Cm-Um-Um-Cm-Um-Cf- Am-Um-Cm-Um | 534 | 450 | AmsEVP-Gfs-Af-Um-Gm-Af- Gm-Am-Am-Gm-Gm-Cm- Am-Cf-Am-Gf-Am-Cm-Gm- Gm-Gms-Gms-Am | 638 | 457 |
| B1550-DV25P | Cms-Cms-Cm-Gm-Um- Cm-Uf-Gm-Uf-Gf-Cf-Cm- Um-Um-Cm-Um-Cf-Am- Um-Cm-Um | 535 | 450 | AmsEVP-Gfs-Am-Um-Gm- Af-Gm-Am-Am-Gm-Gm-Cm- Am-Cf-Am-Gf-Am-Cm-Gm- Gm-Gms-Gms-Am | 639 | 457 |
| B1550-DV33P | Cms-Cms-Cm-Gm-Um- Cm-Uf-Gm-Uf-Gm-Cf- Cm-Um-Um-Cm-Um-Cf- Am-Um-Cm-Um | 534 | 450 | AmsEVP-Gfs-Af-Um-Gf-Af- Gm-Am-Am-Gm-Gm-Cm- Am-Cf-Am-Gf-Am-Cm-Gm- Gm-Gms-Gms-Am | 640 | 457 |
| B1550-DV30P | Cms-Cms-Cm-Gm-Um- Cm-Uf-Gm-Uf-Gf-Cf-Cm- Um-Um-Cm-Um-Cm-Am- Um-Cm-Um | 536 | 450 | AmsEVP-Gfs-Am-Um-Gm- Af-Gm-Am-Am-Gm-Gm-Cm- Am-Cf-Am-Gm-Am-Cm-Gm- Gm-Gms-Gms-Am | 641 | 457 |
| B1550-DV31P | Cms-Cms-Cm-Gm-Um- Cm-Uf-Gm-Uf-Gf-Cm- Cm-Um-Um-Cm-Um-Cm- Am-Um-Cm-Um | 537 | 450 | AmsEVP-Gfs-Am-Um-Gm- Af-Gm-Am-Am-Gm-Gm-Cm- Am-Cf-Am-Gf-Am-Cm-Gm- Gm-Gms-Gms-Am | 642 | 457 |
| B1550-DV21P | Cms-Cms-Cm-Gm-Um- Cm-Uf-Gm-Uf-Gf-Cf-Cm- Um-Um-Cm-Uf-Cf-Am- Um-Cm-Um | 538 | 450 | AmsEVP-Gfs-Am-Um-Gm- Af-Gm-Am-Am-Gm-Gm-Cm- Am-Cf-Am-Gf-Am-Cm-Gm- Gm-Gms-Gms-Am | 642 | 457 |
| B1550-DV22P | Cms-Cms-Cm-Gm-Um- Cm-Uf-Gm-Uf-Gf-Cf-Cm- Um-Um-Cm-Um-Cm-Am- Um-Cm-Um | 536 | 450 | AmsEVP-Gfs-Am-Um-Gm- Af-Gm-Am-Am-Gm-Gm-Cm- Am-Cf-Am-Gf-Am-Cm-Gm- Gm-Gms-Gms-Am | 642 | 457 |
| B1572-DV29P | Cms-Cms-Gm-Gm-Am- Cm-Cf-Gm-Uf-Gm-Uf- Gm-Cm-Am-Cm-Um-Uf- Cm-Gm-Cm-Um | 539 | 451 | AmsEVP-Gfs-Cm-Gf-Af-Af- Gm-Um-Gm-Cm-Am-Cm- Am-Cf-Gm-Gf-Um-Cm-Cm- Gm-Gms-Cms-Am | 643 | 458 |
| B1572-DV26P | Cms-Cms-Gm-Gm-Am- Cm-Cf-Gm-Uf-Gf-Uf-Gm- Cm-Am-Cm-Um-Uf-Cm- Gm-Cm-Um | 540 | 451 | AmsEVP-Gfs-Cf-Gf-Am-Af- Gm-Um-Gm-Cm-Am-Cm- Am-Cf-Gm-Gf-Um-Cm-Cm- Gm-Gms-Cms-Am | 644 | 458 |
| B1572-DV27P | Cms-Cms-Gm-Gm-Am- Cm-Cf-Gm-Uf-Gf-Uf-Gm- Cm-Am-Cm-Um-Uf-Cm- Gm-Cm-Um | 540 | 451 | AmsEVP-Gfs-Cm-Gf-Af-Af- Gm-Um-Gm-Cm-Am-Cm- Am-Cf-Gm-Gf-Um-Cm-Cm- Gm-Gms-Cms-Am | 643 | 458 |
| B1572-DV28P | Cms-Cms-Gm-Gm-Am- Cm-Cf-Gm-Uf-Gm-Uf- Gm-Cm-Am-Cm-Um-Uf- Cm-Gm-Cm-Um | 539 | 451 | AmsEVP-Gfs-Cf-Gf-Am-Af- Gm-Um-Gm-Cm-Am-Cm- Am-Cf-Gm-Gf-Um-Cm-Cm- Gm-Gms-Cms-Am | 644 | 458 |
| B1572-DV32P | Cms-Cms-Gm-Gm-Am- Cm-Cf-Gm-Uf-Gm-Uf- Gm-Cm-Am-Cm-Um-Uf- Cm-Gm-Cm-Um | 539 | 451 | AmsEVP-Gfs-Cm-Gm-Af-Af- Gm-Um-Gm-Cm-Am-Cm- Am-Cf-Gm-Gf-Um-Cm-Cm- Gm-Gms-Cms-Am | 645 | 458 |
| B1572-DV34P | Cms-Cms-Gm-Gm-Am- Cm-Cf-Gm-Uf-Gm-Uf- Gm-Cm-Am-Cm-Um-Uf- Cm-Gm-Cm-Um | 539 | 451 | AmsEVP-Gfs-Cf-Gm-Am-Af- Gm-Um-Gm-Cm-Am-Cm- Am-Cf-Gm-Gf-Um-Cm-Cm- Gm-Gms-Cms-Am | 646 | 458 |
| B1572-DV30P | Cms-Cms-Gm-Gm-Am- Cm-Cf-Gm-Uf-Gf-Uf-Gm- Cm-Am-Cm-Um-Um-Cm- Gm-Cm-Um | 541 | 451 | AmsEVP-Gfs-Cm-Gm-Am- Af-Gm-Um-Gm-Cm-Am-Cm- Am-Cf-Gm-Gm-Um-Cm-Cm- Gm-Gms-Cms-Am | 647 | 458 |
| B1572-DV31P | Cms-Cms-Gm-Gm-Am- Cm-Cf-Gm-Uf-Gf-Um- Gm-Cm-Am-Cm-Um-Um- Cm-Gm-Cm-Um | 542 | 451 | AmsEVP-Gfs-Cm-Gm-Am- Af-Gm-Um-Gm-Cm-Am-Cm- Am-Cf-Gm-Gf-Um-Cm-Cm- Gm-Gms-Cms-Am | 648 | 458 |
| B1572-DV21P | Cms-Cms-Gm-Gm-Am- Cm-Cf-Gm-Uf-Gf-Uf-Gm- Cm-Am-Cm-Uf-Uf-Cm- Gm-Cm-Um | 543 | 451 | AmsEVP-Gfs-Cm-Gm-Am- Af-Gm-Um-Gm-Cm-Am-Cm- Am-Cf-Gm-Gf-Um-Cm-Cm- Gm-Gms-Cms-Am | 648 | 458 |
| B1572-DV22P | Cms-Cms-Gm-Gm-Am- Cm-Cf-Gm-Uf-Gf-Uf-Gm- Cm-Am-Cm-Um-Um-Cm- Gm-Cm-Um | 541 | 451 | AmsEVP-Gfs-Cm-Gm-Am- Af-Gm-Um-Gm-Cm-Am-Cm- Am-Cf-Gm-Gf-Um-Cm-Cm- Gm-Gms-Cms-Am | 648 | 458 |
| B1575-DV29P | Gms-Ams-Cm-Cm-Gm- Um-Gf-Um-Gf-Cm-Af- Cm-Um-Um-Cm-Gm-Cf- Um-Um-Cm-Am | 544 | 452 | UmsEVP-Gfs-Am-Af-Gf-Cf- Gm-Am-Am-Gm-Um-Gm- Cm-Af-Cm-Af-Cm-Gm-Gm- Um-Cms-Cms-Gm | 649 | 459 |
| B1575-DV26P | Gms-Ams-Cm-Cm-Gm- Um-Gf-Um-Gf-Cf-Af-Cm- Um-Um-Cm-Gm-Cf-Um- Um-Cm-Am | 545 | 452 | UmsEVP-Gfs-Af-Af-Gm-Cf- Gm-Am-Am-Gm-Um-Gm- Cm-Af-Cm-Af-Cm-Gm-Gm- Um-Cms-Cms-Gm | 650 | 459 |
| B1575-DV27P | Gms-Ams-Cm-Cm-Gm- Um-Gf-Um-Gf-Cf-Af-Cm- Um-Um-Cm-Gm-Cf-Um- Um-Cm-Am | 545 | 452 | UmsEVP-Gfs-Am-Af-Gf-Cf- Gm-Am-Am-Gm-Um-Gm- Cm-Af-Cm-Af-Cm-Gm-Gm- Um-Cms-Cms-Gm | 649 | 459 |
| B1575-DV28P | Gms-Ams-Cm-Cm-Gm- Um-Gf-Um-Gf-Cm-Af- Cm-Um-Um-Cm-Gm-Cf- Um-Um-Cm-Am | 544 | 452 | UmsEVP-Gfs-Af-Af-Gm-Cf- Gm-Am-Am-Gm-Um-Gm- Cm-Af-Cm-Af-Cm-Gm-Gm- Um-Cms-Cms-Gm | 650 | 459 |
| B1575-DV32P | Gms-Ams-Cm-Cm-Gm- Um-Gf-Um-Gf-Cm-Af- Cm-Um-Um-Cm-Gm-Cf- Um-Um-Cm-Am | 544 | 452 | UmsEVP-Gfs-Am-Am-Gf-Cf- Gm-Am-Am-Gm-Um-Gm- Cm-Af-Cm-Af-Cm-Gm-Gm- Um-Cms-Cms-Gm | 651 | 459 |
| B1575-DV34P | Gms-Ams-Cm-Cm-Gm- Um-Gf-Um-Gf-Cm-Af- Cm-Um-Um-Cm-Gm-Cf- Um-Um-Cm-Am | 544 | 452 | UmsEVP-Gfs-Af-Am-Gm-Cf- Gm-Am-Am-Gm-Um-Gm- Cm-Af-Cm-Af-Cm-Gm-Gm- Um-Cms-Cms-Gm | 652 | 459 |
| B1575-DV30P | Gms-Ams-Cm-Cm-Gm- Um-Gf-Um-Gf-Cf-Af-Cm- Um-Um-Cm-Gm-Cm-Um- Um-Cm-Am | 546 | 452 | UmsEVP-Gfs-Am-Am-Gm- Cf-Gm-Am-Am-Gm-Um-Gm- Cm-Af-Cm-Am-Cm-Gm-Gm- Um-Cms-Cms-Gm | 653 | 459 |
| B1575-DV31P | Gms-Ams-Cm-Cm-Gm- Um-Gf-Um-Gf-Cf-Am- Cm-Um-Um-Cm-Gm-Cm- Um-Um-Cm-Am | 547 | 452 | UmsEVP-Gfs-Am-Am-Gm- Cf-Gm-Am-Am-Gm-Um-Gm- Cm-Af-Cm-Af-Cm-Gm-Gm- Um-Cms-Cms-Gm | 654 | 459 |
| B1575-DV21P | Gms-Ams-Cm-Cm-Gm- Um-Gf-Um-Gf-Cf-Af-Cm- Um-Um-Cm-Gf-Cf-Um- Um-Cm-Am | 548 | 452 | UmsEVP-Gfs-Am-Am-Gm- Cf-Gm-Am-Am-Gm-Um-Gm- Cm-Af-Cm-Af-Cm-Gm-Gm- Um-Cms-Cms-Gm | 654 | 459 |
| B1575-DV22P | Gms-Ams-Cm-Cm-Gm- Um-Gf-Um-Gf-Cf-Af-Cm- Um-Um-Cm-Gm-Cm-Um- Um-Cm-Am | 546 | 452 | UmsEVP-Gfs-Am-Am-Gm- Cf-Gm-Am-Am-Gm-Um-Gm- Cm-Af-Cm-Af-Cm-Gm-Gm- Um-Cms-Cms-Gm | 654 | 459 |
| A1573-DV29P | Cms-Gms-Gm-Am-Cm- Cm-Gf-Um-Gf-Um-Gf- Cm-Am-Cm-Um-Um-Cf- Gm-Cm-Um-Um | 549 | 453 | AmsEVP-Afs-Gm-Cf-Gf-Af- Am-Gm-Um-Gm-Cm-Am- Cm-Af-Cm-Gf-Gm-Um-Cm- Cm-Gms-Gms-Cm | 655 | 460 |
| B1573-DV26P | Cms-Gms-Gm-Am-Cm- Cm-Gf-Um-Gf-Uf-Gf-Cm- Am-Cm-Um-Um-Cf-Gm- Cm-Um-Um | 550 | 453 | AmsEVP-Afs-Gf-Cf-Gm-Af- Am-Gm-Um-Gm-Cm-Am- Cm-Af-Cm-Gf-Gm-Um-Cm- Cm-Gms-Gms-Cm | 656 | 460 |
| B1573-DV27P | Cms-Gms-Gm-Am-Cm- Cm-Gf-Um-Gf-Uf-Gf-Cm- Am-Cm-Um-Um-Cf-Gm- Cm-Um-Um | 550 | 453 | AmsEVP-Afs-Gm-Cf-Gf-Af- Am-Gm-Um-Gm-Cm-Am- Cm-Af-Cm-Gf-Gm-Um-Cm- Cm-Gms-Gms-Cm | 655 | 460 |
| B1573-DV28P | Cms-Gms-Gm-Am-Cm- Cm-Gf-Um-Gf-Um-Gf- Cm-Am-Cm-Um-Um-Cf- Gm-Cm-Um-Um | 549 | 453 | AmsEVP-Afs-Gf-Cf-Gm-Af- Am-Gm-Um-Gm-Cm-Am- Cm-Af-Cm-Gf-Gm-Um-Cm- Cm-Gms-Gms-Cm | 656 | 460 |
| B1573-DV32P | Cms-Gms-Gm-Am-Cm- Cm-Gf-Um-Gf-Um-Gf- Cm-Am-Cm-Um-Um-Cf- Gm-Cm-Um-Um | 549 | 453 | AmsEVP-Afs-Gm-Cm-Gf-Af- Am-Gm-Um-Gm-Cm-Am- Cm-Af-Cm-Gf-Gm-Um-Cm- Cm-Gms-Gms-Cm | 657 | 460 |
| B1573-DV34P | Cms-Gms-Gm-Am-Cm- Cm-Gf-Um-Gf-Um-Gf- Cm-Am-Cm-Um-Um-Cf- Gm-Cm-Um-Um | 549 | 453 | AmsEVP-Afs-Gf-Cm-Gm-Af- Am-Gm-Um-Gm-Cm-Am- Cm-Af-Cm-Gf-Gm-Um-Cm- Cm-Gms-Gms-Cm | 658 | 460 |
| B1573-DV30P | Cms-Gms-Gm-Am-Cm- Cm-Gf-Um-Gf-Uf-Gf-Cm- Am-Cm-Um-Um-Cm-Gm- Cm-Um-Um | 551 | 453 | AmsEVP-Afs-Gm-Cm-Gm- Af-Am-Gm-Um-Gm-Cm-Am- Cm-Af-Cm-Gm-Gm-Um-Cm- Cm-Gms-Gms-Cm | 659 | 460 |
| B1573-DV31P | Cms-Gms-Gm-Am-Cm- Cm-Gf-Um-Gf-Uf-Gm- Cm-Am-Cm-Um-Um-Cm- Gm-Cm-Um-Um | 552 | 453 | AmsEVP-Afs-Gm-Cm-Gm- Af-Am-Gm-Um-Gm-Cm-Am- Cm-Af-Cm-Gf-Gm-Um-Cm- Cm-Gms-Gms-Cm | 660 | 460 |
| B1573-DV21P | Cms-Gms-Gm-Am-Cm- Cm-Gf-Um-Gf-Uf-Gf-Cm- Am-Cm-Um-Uf-Cf-Gm- Cm-Um-Um | 553 | 453 | AmsEVP-Afs-Gm-Cm-Gm- Af-Am-Gm-Um-Gm-Cm-Am- Cm-Af-Cm-Gf-Gm-Um-Cm- Cm-Gms-Gms-Cm | 660 | 460 |
| B1573-DV22P | Cms-Gms-Gm-Am-Cm- Cm-Gf-Um-Gf-Uf-Gf-Cm- Am-Cm-Um-Um-Cm-Gm- Cm-Um-Um | 551 | 453 | AmsEVP-Afs-Gm-Cm-Gm- Af-Am-Gm-Um-Gm-Cm-Am- Cm-Af-Cm-Gf-Gm-Um-Cm- Cm-Gms-Gms-Cm | 660 | 460 |
| B418S-DV29SP | Cms-Ums-Gm-Cm-Um-Af- Um-Gf-Cm-Cf-Um-Cm- Am-Um-Cm-Uf-Um-Cm- Um-Um | 554 | 454 | AmsEVP-Afs-Gm-Af-Af-Gf- Am-Um-Gm-Am-Gm-Gm- Cm-Af-Um-Af-Gm-Cm-Am- Gms-Cms-Am | 661 | 461 |
| B418S-DV26SP | Cms-Ums-Gm-Cm-Um-Af- Um-Gf-Cf-Cf-Um-Cm- Am-Um-Cm-Uf-Um-Cm- Um-Um | 555 | 454 | AmsEVP-Afs-Gf-Af-Am-Gf- Am-Um-Gm-Am-Gm-Gm- Cm-Af-Um-Af-Gm-Cm-Am- Gms-Cms-Am | 662 | 461 |
| B418S-DV27SP | Cms-Ums-Gm-Cm-Um-Af- Um-Gf-Cf-Cf-Um-Cm- Am-Um-Cm-Uf-Um-Cm- Um-Um | 555 | 454 | AmsEVP-Afs-Gm-Af-Af-Gf- Am-Um-Gm-Am-Gm-Gm- Cm-Af-Um-Af-Gm-Cm-Am- Gms-Cms-Am | 661 | 461 |
| B418S-DV28SP | Cms-Ums-Gm-Cm-Um-Af- Um-Gf-Cm-Cf-Um-Cm- Am-Um-Cm-Uf-Um-Cm- Um-Um | 554 | 454 | AmsEVP-Afs-Gf-Af-Am-Gf- Am-Um-Gm-Am-Gm-Gm- Cm-Af-Um-Af-Gm-Cm-Am- Gms-Cms-Am | 662 | 461 |
| B418S-DV32SP | Cms-Ums-Gm-Cm-Um-Af- Um-Gf-Cm-Cf-Um-Cm- Am-Um-Cm-Uf-Um-Cm- Um-Um | 554 | 454 | AmsEVP-Afs-Gm-Am-Af-Gf- Am-Um-Gm-Am-Gm-Gm- Cm-Af-Um-Af-Gm-Cm-Am- Gms-Cms-Am | 663 | 461 |
| B418S-DV34SP | Cms-Ums-Gm-Cm-Um-Af- Um-Gf-Cm-Cf-Um-Cm- Am-Um-Cm-Uf-Um-Cm- Um-Um | 554 | 454 | AmsEVP-Afs-Gf-Am-Am-Gf- Am-Um-Gm-Am-Gm-Gm- Cm-Af-Um-Af-Gm-Cm-Am- Gms-Cms-Am | 664 | 461 |
| B418S-DV30SP | Cms-Ums-Gm-Cm-Um-Af- Um-Gf-Cf-Cf-Um-Cm- Am-Um-Cm-Um-Um-Cm- Um-Um | 556 | 454 | AmsEVP-Afs-Gm-Am-Am- Gf-Am-Um-Gm-Am-Gm-Gm- Cm-Af-Um-Am-Gm-Cm-Am- Gms-Cms-Am | 665 | 461 |
| B418S-DV31SP | Cms-Ums-Gm-Cm-Um-Af- Um-Gf-Cf-Cm-Um-Cm- Am-Um-Cm-Um-Um-Cm- Um-Um | 557 | 454 | AmsEVP-Afs-Gm-Am-Am- Gf-Am-Um-Gm-Am-Gm-Gm- Cm-Af-Um-Af-Gm-Cm-Am- Gms-Cms-Am | 666 | 461 |
| B418S-DV21SP | Cms-Ums-Gm-Cm-Um-Af- Um-Gf-Cf-Cf-Um-Cm- Am-Um-Cf-Uf-Um-Cm- Um-Um | 558 | 454 | AmsEVP-Afs-Gm-Am-Am- Gf-Am-Um-Gm-Am-Gm-Gm- Cm-Af-Um-Af-Gm-Cm-Am- Gms-Cms-Am | 666 | 461 |
| B418S-DV22SP | Cms-Ums-Gm-Cm-Um-Af- Um-Gf-Cf-Cf-Um-Cm- Am-Um-Cm-Um-Um-Cm- Um-Um | 556 | 454 | AmsEVP-Afs-Gm-Am-Am- Gf-Am-Um-Gm-Am-Gm-Gm- Cm-Af-Um-Af-Gm-Cm-Am- Gms-Cms-Am | 666 | 461 |
| A416-DV29P | Ums-Gms-Cm-Um-Gm- Cm-Uf-Am-Uf-Gm-Cf- Cm-Um-Cm-Am-Um-Cf- Um-Um-Cm-Um | 559 | 455 | AmsEVP-Gfs-Am-Af-Gf-Af- Um-Gm-Am-Gm-Gm-Cm- Am-Uf-Am-Gf-Cm-Am-Gm- Cm-Ams-Gms-Gm | 667 | 462 |
| B416-DV26P | Ums-Gms-Cm-Um-Gm- Cm-Uf-Am-Uf-Gf-Cf-Cm- Um-Cm-Am-Um-Cf-Um- Um-Cm-Um | 560 | 455 | AmsEVP-Gfs-Af-Af-Gm-Af- Um-Gm-Am-Gm-Gm-Cm- Am-Uf-Am-Gf-Cm-Am-Gm- Cm-Ams-Gms-Gm | 668 | 462 |
| B416-DV27P | Ums-Gms-Cm-Um-Gm- Cm-Uf-Am-Uf-Gf-Cf-Cm- Um-Cm-Am-Um-Cf-Um- Um-Cm-Um | 560 | 455 | AmsEVP-Gfs-Am-Af-Gf-Af- Um-Gm-Am-Gm-Gm-Cm- Am-Uf-Am-Gf-Cm-Am-Gm- Cm-Ams-Gms-Gm | 667 | 462 |
| B416-DV28P | Ums-Gms-Cm-Um-Gm- Cm-Uf-Am-Uf-Gm-Cf- Cm-Um-Cm-Am-Um-Cf- Um-Um-Cm-Um | 559 | 455 | AmsEVP-Gfs-Af-Af-Gm-Af- Um-Gm-Am-Gm-Gm-Cm- Am-Uf-Am-Gf-Cm-Am-Gm- Cm-Ams-Gms-Gm | 668 | 462 |
| B416-DV32P | Ums-Gms-Cm-Um-Gm- Cm-Uf-Am-Uf-Gm-Cf- Cm-Um-Cm-Am-Um-Cf- Um-Um-Cm-Um | 559 | 455 | AmsEVP-Gfs-Am-Am-Gf-Af- Um-Gm-Am-Gm-Gm-Cm- Am-Uf-Am-Gf-Cm-Am-Gm- Cm-Ams-Gms-Gm | 669 | 462 |
| B416-DV34P | Ums-Gms-Cm-Um-Gm- Cm-Uf-Am-Uf-Gm-Cf- Cm-Um-Cm-Am-Um-Cf- Um-Um-Cm-Um | 559 | 455 | AmsEVP-Gfs-Af-Am-Gm-Af- Um-Gm-Am-Gm-Gm-Cm- Am-Uf-Am-Gf-Cm-Am-Gm- Cm-Ams-Gms-Gm | 670 | 462 |
| B416-DV30P | Ums-Gms-Cm-Um-Gm- Cm-Uf-Am-Uf-Gf-Cf-Cm- Um-Cm-Am-Um-Cm-Um- Um-Cm-Um | 561 | 455 | AmsEVP-Gfs-Am-Am-Gm- Af-Um-Gm-Am-Gm-Gm-Cm- Am-Uf-Am-Gm-Cm-Am-Gm- Cm-Ams-Gms-Gm | 671 | 462 |
| B416-DV31P | Ums-Gms-Cm-Um-Gm- Cm-Uf-Am-Uf-Gf-Cm- Cm-Um-Cm-Am-Um-Cm- Um-Um-Cm-Um | 562 | 455 | AmsEVP-Gfs-Am-Am-Gm- Af-Um-Gm-Am-Gm-Gm-Cm- Am-Uf-Am-Gm-Cf-Am-Gm- Cm-Ams-Gms-Gm | 672 | 462 |
| B416-DV21P | Ums-Gms-Cm-Um-Gm- Cm-Uf-Am-Uf-Gf-Cf-Cm- Um-Cm-Am-Uf-Cf-Um- Um-Cm-Um | 563 | 455 | AmsEVP-Gfs-Am-Am-Gm- Af-Um-Gm-Am-Gm-Gm-Cm- Am-Uf-Am-Gm-Cf-Am-Gm- Cm-Ams-Gms-Gm | 672 | 462 |
| B416-DV22P | Ums-Gms-Cm-Um-Gm- Cm-Uf-Am-Uf-Gf-Cf-Cm- Um-Cm-Am-Um-Cm-Um- Um-Cm-Um | 561 | 455 | AmsEVP-Gfs-Am-Am-Gm- Af-Um-Gm-Am-Gm-Gm-Cm- Am-Uf-Am-Gm-Cf-Am-Gm- Cm-Ams-Gms-Gm | 672 | 462 |
| A205-DV29P | Gms-Cms-Gm-Gm-Gm- Gm-Uf-Um-Uf-Um-Uf- Cm-Um-Um-Gm-Um-Uf- Gm-Am-Cm-Am | 564 | 466 | UmsEVP-Gfs-Um-Cf-Af-Af- Cm-Am-Am-Gm-Am-Am- Am-Af-Am-Cf-Cm-Cm-Cm- Gm-Cms-Cms-Um | 673 | 498 |
| B205-DV26P | Gms-Cms-Gm-Gm-Gm- Gm-Uf-Um-Uf-Uf-Uf-Cm- Um-Um-Gm-Um-Uf-Gm- Am-Cm-Am | 565 | 466 | UmsEVP-Gfs-Uf-Cf-Am-Af- Cm-Am-Am-Gm-Am-Am- Am-Af-Am-Cf-Cm-Cm-Cm- Gm-Cms-Cms-Um | 674 | 498 |
| B205-DV27P | Gms-Cms-Gm-Gm-Gm- Gm-Uf-Um-Uf-Uf-Uf-Cm- Um-Um-Gm-Um-Uf-Gm- Am-Cm-Am | 565 | 466 | UmsEVP-Gfs-Um-Cf-Af-Af- Cm-Am-Am-Gm-Am-Am- Am-Af-Am-Cf-Cm-Cm-Cm- Gm-Cms-Cms-Um | 673 | 498 |
| B205-DV28P | Gms-Cms-Gm-Gm-Gm- Gm-Uf-Um-Uf-Um-Uf- Cm-Um-Um-Gm-Um-Uf- Gm-Am-Cm-Am | 564 | 466 | UmsEVP-Gfs-Uf-Cf-Am-Af- Cm-Am-Am-Gm-Am-Am- Am-Af-Am-Cf-Cm-Cm-Cm- Gm-Cms-Cms-Um | 674 | 498 |
| B205-DV32P | Gms-Cms-Gm-Gm-Gm- Gm-Uf-Um-Uf-Um-Uf- Cm-Um-Um-Gm-Um-Uf- Gm-Am-Cm-Am | 564 | 466 | UmsEVP-Gfs-Um-Cm-Af-Af- Cm-Am-Am-Gm-Am-Am- Am-Af-Am-Cf-Cm-Cm-Cm- Gm-Cms-Cms-Um | 675 | 498 |
| B205-DV34P | Gms-Cms-Gm-Gm-Gm- Gm-Uf-Um-Uf-Um-Uf- Cm-Um-Um-Gm-Um-Uf- Gm-Am-Cm-Am | 564 | 466 | UmsEVP-Gfs-Uf-Cm-Am-Af- Cm-Am-Am-Gm-Am-Am- Am-Af-Am-Cf-Cm-Cm-Cm- Gm-Cms-Cms-Um | 676 | 498 |
| B205-DV30P | Gms-Cms-Gm-Gm-Gm- Gm-Uf-Um-Uf-Uf-Uf-Cm- Um-Um-Gm-Um-Um-Gm- Am-Cm-Am | 566 | 466 | UmsEVP-Gfs-Um-Cm-Am- Af-Cm-Am-Am-Gm-Am-Am- Am-Af-Am-Cm-Cm-Cm-Cm- Gm-Cms-Cms-Um | 677 | 498 |
| B205-DV31P | Gms-Cms-Gm-Gm-Gm- Gm-Uf-Um-Uf-Uf-Um- Cm-Um-Um-Gm-Um-Um- Gm-Am-Cm-Am | 567 | 466 | UmsEVP-Gfs-Um-Cm-Am- Af-Cm-Am-Am-Gm-Am-Am- Am-Af-Am-Cf-Cm-Cm-Cm- Gm-Cms-Cms-Um | 678 | 498 |
| B205-DV21P | Gms-Cms-Gm-Gm-Gm- Gm-Uf-Um-Uf-Uf-Uf-Cm- Um-Um-Gm-Uf-Uf-Gm- Am-Cm-Am | 568 | 466 | UmsEVP-Gfs-Um-Cm-Am- Af-Cm-Am-Am-Gm-Am-Am- Am-Af-Am-Cf-Cm-Cm-Cm- Gm-Cms-Cms-Um | 678 | 498 |
| B205-DV22P | Gms-Cms-Gm-Gm-Gm- Gm-Uf-Um-Uf-Uf-Uf-Cm- Um-Um-Gm-Um-Um-Gm- Am-Cm-Am | 566 | 466 | UmsEVP-Gfs-Um-Cm-Am- Af-Cm-Am-Am-Gm-Am-Am- Am-Af-Am-Cf-Cm-Cm-Cm- Gm-Cms-Cms-Um | 678 | 498 |
| A261-DV29P | Gms-Ums-Gm-Gm-Am- Cm-Uf-Um-Cf-Um-Cf- Um-Cm-Am-Am-Um-Uf- Um-Um-Cm-Um | 574 | 473 | AmsEVP-Gfs-Am-Af-Af-Af- Um-Um-Gm-Am-Gm-Am- Gm-Af-Am-Gf-Um-Cm-Cm- Am-Cms-Cms-Am | 685 | 505 |
| B261-DV26P | Gms-Ums-Gm-Gm-Am- Cm-Uf-Um-Cf-Uf-Cf-Um- Cm-Am-Am-Um-Uf-Um- Um-Cm-Um | 575 | 473 | AmsEVP-Gfs-Af-Af-Am-Af- Um-Um-Gm-Am-Gm-Am- Gm-Af-Am-Gf-Um-Cm-Cm- Am-Cms-Cms-Am | 686 | 505 |
| B261-DV27P | Gms-Ums-Gm-Gm-Am- Cm-Uf-Um-Cf-Uf-Cf-Um- Cm-Am-Am-Um-Uf-Um- Um-Cm-Um | 575 | 473 | AmsEVP-Gfs-Am-Af-Af-Af- Um-Um-Gm-Am-Gm-Am- Gm-Af-Am-Gf-Um-Cm-Cm- Am-Cms-Cms-Am | 685 | 505 |
| B261-DV28P | Gms-Ums-Gm-Gm-Am- Cm-Uf-Um-Cf-Um-Cf- Um-Cm-Am-Am-Um-Uf- Um-Um-Cm-Um | 574 | 473 | AmsEVP-Gfs-Af-Af-Am-Af- Um-Um-Gm-Am-Gm-Am- Gm-Af-Am-Gf-Um-Cm-Cm- Am-Cms-Cms-Am | 686 | 505 |
| B261-DV32P | Gms-Ums-Gm-Gm-Am- Cm-Uf-Um-Cf-Um-Cf- Um-Cm-Am-Am-Um-Uf- Um-Um-Cm-Um | 574 | 473 | AmsEVP-Gfs-Am-Am-Af-Af- Um-Um-Gm-Am-Gm-Am- Gm-Af-Am-Gf-Um-Cm-Cm- Am-Cms-Cms-Am | 687 | 505 |
| B261-DV34P | Gms-Ums-Gm-Gm-Am- Cm-Uf-Um-Cf-Um-Cf- Um-Cm-Am-Am-Um-Uf- Um-Um-Cm-Um | 574 | 473 | AmsEVP-Gfs-Af-Am-Am-Af- Um-Um-Gm-Am-Gm-Am- Gm-Af-Am-Gf-Um-Cm-Cm- Am-Cms-Cms-Am | 688 | 505 |
| B261-DV30P | Gms-Ums-Gm-Gm-Am- Cm-Uf-Um-Cf-Uf-Cf-Um- Cm-Am-Am-Um-Um-Um- Um-Cm-Um | 576 | 473 | AmsEVP-Gfs-Am-Am-Am- Af-Um-Um-Gm-Am-Gm-Am- Gm-Af-Am-Gm-Um-Cm-Cm- Am-Cms-Cms-Am | 689 | 505 |
| B261-DV31P | Gms-Ums-Gm-Gm-Am- Cm-Uf-Um-Cf-Uf-Cm- Um-Cm-Am-Am-Um-Um- Um-Um-Cm-Um | 577 | 473 | AmsEVP-Gfs-Am-Am-Am- Af-Um-Um-Gm-Am-Gm-Am- Gm-Af-Am-Gf-Um-Cm-Cm- Am-Cms-Cms-Am | 690 | 505 |
| B261-DV21P | Gms-Ums-Gm-Gm-Am- Cm-Uf-Um-Cf-Uf-Cf-Um- Cm-Am-Am-Uf-Uf-Um- Um-Cm-Um | 578 | 473 | AmsEVP-Gfs-Am-Am-Am- Af-Um-Um-Gm-Am-Gm-Am- Gm-Af-Am-Gf-Um-Cm-Cm- Am-Cms-Cms-Am | 690 | 505 |
| B261-DV22P | Gms-Ums-Gm-Gm-Am- Cm-Uf-Um-Cf-Uf-Cf-Um- Cm-Am-Am-Um-Um-Um- Um-Cm-Um | 576 | 473 | AmsEVP-Gfs-Am-Am-Am- Af-Um-Um-Gm-Am-Gm-Am- Gm-Af-Am-Gf-Um-Cm-Cm- Am-Cms-Cms-Am | 690 | 505 |
| B208-DV29P | Gms-Gms-Gm-Um-Um- Um-Uf-Um-Cf-Um-Uf- Gm-Um-Um-Gm-Am-Cf- Am-Am-Gm-Am | 569 | 486 | UmsEVP-Cfs-Um-Uf-Gf-Uf- Cm-Am-Am-Cm-Am-Am- Gm-Af-Am-Af-Am-Am-Cm- Cm-Cms-Cms-Gm | 679 | 518 |
| B208-DV26P | Gms-Gms-Gm-Um-Um- Um-Uf-Um-Cf-Uf-Uf-Gm- Um-Um-Gm-Am-Cf-Am- Am-Gm-Am | 570 | 486 | UmsEVP-Cfs-Uf-Uf-Gm-Uf- Cm-Am-Am-Cm-Am-Am- Gm-Af-Am-Af-Am-Am-Cm- Cm-Cms-Cms-Gm | 680 | 518 |
| B208-DV27P | Gms-Gms-Gm-Um-Um- Um-Uf-Um-Cf-Uf-Uf-Gm- Um-Um-Gm-Am-Cf-Am- Am-Gm-Am | 570 | 486 | UmsEVP-Cfs-Um-Uf-Gf-Uf- Cm-Am-Am-Cm-Am-Am- Gm-Af-Am-Af-Am-Am-Cm- Cm-Cms-Cms-Gm | 679 | 518 |
| B208-DV28P | Gms-Gms-Gm-Um-Um- Um-Uf-Um-Cf-Um-Uf- Gm-Um-Um-Gm-Am-Cf- Am-Am-Gm-Am | 569 | 486 | UmsEVP-Cfs-Uf-Uf-Gm-Uf- Cm-Am-Am-Cm-Am-Am- Gm-Af-Am-Af-Am-Am-Cm- Cm-Cms-Cms-Gm | 680 | 518 |
| B208-DV32P | Gms-Gms-Gm-Um-Um- Um-Uf-Um-Cf-Um-Uf- Gm-Um-Um-Gm-Am-Cf- Am-Am-Gm-Am | 569 | 486 | UmsEVP-Cfs-Um-Um-Gf-Uf- Cm-Am-Am-Cm-Am-Am- Gm-Af-Am-Af-Am-Am-Cm- Cm-Cms-Cms-Gm | 681 | 518 |
| B208-DV34P | Gms-Gms-Gm-Um-Um- Um-Uf-Um-Cf-Um-Uf- Gm-Um-Um-Gm-Am-Cf- Am-Am-Gm-Am | 569 | 486 | UmsEVP-Cfs-Uf-Um-Gm-Uf- Cm-Am-Am-Cm-Am-Am- Gm-Af-Am-Af-Am-Am-Cm- Cm-Cms-Cms-Gm | 682 | 518 |
| B208-DV30P | Gms-Gms-Gm-Um-Um- Um-Uf-Um-Cf-Uf-Uf-Gm- Um-Um-Gm-Am-Cm-Am- Am-Gm-Am | 571 | 486 | UmsEVP-Cfs-Um-Um-Gm- Uf-Cm-Am-Am-Cm-Am-Am- Gm-Af-Am-Am-Am-Am-Cm- Cm-Cms-Cms-Gm | 683 | 518 |
| B208-DV31P | Gms-Gms-Gm-Um-Um- Um-Uf-Um-Cf-Uf-Um- Gm-Um-Um-Gm-Am-Cm- Am-Am-Gm-Am | 572 | 486 | UmsEVP-Cfs-Um-Um-Gm- Uf-Cm-Am-Am-Cm-Am-Am- Gm-Af-Am-Af-Am-Am-Cm- Cm-Cms-Cms-Gm | 684 | 518 |
| B208-DV21P | Gms-Gms-Gm-Um-Um- Um-Uf-Um-Cf-Uf-Uf-Gm- Um-Um-Gm-Af-Cf-Am- Am-Gm-Am | 573 | 486 | UmsEVP-Cfs-Um-Um-Gm- Uf-Cm-Am-Am-Cm-Am-Am- Gm-Af-Am-Af-Am-Am-Cm- Cm-Cms-Cms-Gm | 684 | 518 |
| B208-DV22P | Gms-Gms-Gm-Um-Um- Um-Uf-Um-Cf-Uf-Uf-Gm- Um-Um-Gm-Am-Cm-Am- Am-Gm-Am | 571 | 486 | UmsEVP-Cfs-Um-Um-Gm- Uf-Cm-Am-Am-Cm-Am-Am- Gm-Af-Am-Af-Am-Am-Cm- Cm-Cms-Cms-Gm | 684 | 518 |
| B262-DV29P | Ums-Gms-Gm-Am-Cm- Um-Uf-Cm-Uf-Cm-Uf- Cm-Am-Am-Um-Um-Uf- Um-Cm-Um-Am | 579 | 487 | UmsEVP-Afs-Gm-Af-Af-Af- Am-Um-Um-Gm-Am-Gm- Am-Gf-Am-Af-Gm-Um-Cm- Cm-Ams-Cms-Gm | 691 | 519 |
| B262-DV26P | Ums-Gms-Gm-Am-Cm- Um-Uf-Cm-Uf-Cf-Uf-Cm- Am-Am-Um-Um-Uf-Um- Cm-Um-Am | 580 | 487 | UmsEVP-Afs-Gf-Af-Am-Af- Am-Um-Um-Gm-Am-Gm- Am-Gf-Am-Af-Gm-Um-Cm- Cm-Ams-Cms-Gm | 692 | 519 |
| B262-DV27P | Ums-Gms-Gm-Am-Cm- Um-Uf-Cm-Uf-Cf-Uf-Cm- Am-Am-Um-Um-Uf-Um- Cm-Um-Am | 580 | 487 | UmsEVP-Afs-Gm-Af-Af-Af- Am-Um-Um-Gm-Am-Gm- Am-Gf-Am-Af-Gm-Um-Cm- Cm-Ams-Cms-Gm | 691 | 519 |
| B262-DV28P | Ums-Gms-Gm-Am-Cm- Um-Uf-Cm-Uf-Cm-Uf- Cm-Am-Am-Um-Um-Uf- Um-Cm-Um-Am | 579 | 487 | UmsEVP-Afs-Gf-Af-Am-Af- Am-Um-Um-Gm-Am-Gm- Am-Gf-Am-Af-Gm-Um-Cm- Cm-Ams-Cms-Gm | 692 | 519 |
| B262-DV32P | Ums-Gms-Gm-Am-Cm- Um-Uf-Cm-Uf-Cm-Uf- Cm-Am-Am-Um-Um-Uf- Um-Cm-Um-Am | 579 | 487 | UmsEVP-Afs-Gm-Am-Af-Af- Am-Um-Um-Gm-Am-Gm- Am-Gf-Am-Af-Gm-Um-Cm- Cm-Ams-Cms-Gm | 693 | 519 |
| B262-DV34P | Ums-Gms-Gm-Am-Cm- Um-Uf-Cm-Uf-Cm-Uf- Cm-Am-Am-Um-Um-Uf- Um-Cm-Um-Am | 579 | 487 | UmsEVP-Afs-Gf-Am-Am-Af- Am-Um-Um-Gm-Am-Gm- Am-Gf-Am-Af-Gm-Um-Cm- Cm-Ams-Cms-Gm | 694 | 519 |
| B262-DV30P | Ums-Gms-Gm-Am-Cm- Um-Uf-Cm-Uf-Cf-Uf-Cm- Am-Am-Um-Um-Um-Um- Cm-Um-Am | 581 | 487 | UmsEVP-Afs-Gm-Am-Am- Af-Am-Um-Um-Gm-Am-Gm- Am-Gf-Am-Am-Gm-Um-Cm- Cm-Ams-Cms-Gm | 695 | 519 |
| B262-DV31P | Ums-Gms-Gm-Am-Cm- Um-Uf-Cm-Uf-Cf-Um- Cm-Am-Am-Um-Um-Um- Um-Cm-Um-Am | 582 | 487 | UmsEVP-Afs-Gm-Am-Am- Af-Am-Um-Um-Gm-Am-Gm- Am-Gf-Am-Af-Gm-Um-Cm- Cm-Ams-Cms-Gm | 696 | 519 |
| B262-DV21P | Ums-Gms-Gm-Am-Cm- Um-Uf-Cm-Uf-Cf-Uf-Cm- Am-Am-Um-Uf-Uf-Um- Cm-Um-Am | 583 | 487 | UmsEVP-Afs-Gm-Am-Am- Af-Am-Um-Um-Gm-Am-Gm- Am-Gf-Am-Af-Gm-Um-Cm- Cm-Ams-Cms-Gm | 696 | 519 |
| B262-DV22P | Ums-Gms-Gm-Am-Cm- Um-Uf-Cm-Uf-Cf-Uf-Cm- Am-Am-Um-Um-Um-Um- Cm-Um-Am | 581 | 487 | UmsEVP-Afs-Gm-Am-Am- Af-Am-Um-Um-Gm-Am-Gm- Am-Gf-Am-Af-Gm-Um-Cm- Cm-Ams-Cms-Gm | 696 | 519 |
| B264-DV29P | Gms-Ams-Cm-Um-Um- Cm-Uf-Cm-Uf-Cm-Af- Am-Um-Um-Um-Um-Cf- Um-Am-Gm-Am | 584 | 488 | UmsEVP-Cfs-Um-Af-Gf-Af- Am-Am-Am-Um-Um-Gm- Am-Gf-Am-Gf-Am-Am-Gm- Um-Cms-Cms-Am | 697 | 520 |
| B264-DV26P | Gms-Ams-Cm-Um-Um- Cm-Uf-Cm-Uf-Cf-Af-Am- Um-Um-Um-Um-Cf-Um- Am-Gm-Am | 585 | 488 | UmsEVP-Cfs-Uf-Af-Gm-Af- Am-Am-Am-Um-Um-Gm- Am-Gf-Am-Gf-Am-Am-Gm- Um-Cms-Cms-Am | 698 | 520 |
| B264-DV27P | Gms-Ams-Cm-Um-Um- Cm-Uf-Cm-Uf-Cf-Af-Am- Um-Um-Um-Um-Cf-Um- Am-Gm-Am | 585 | 488 | UmsEVP-Cfs-Um-Af-Gf-Af- Am-Am-Am-Um-Um-Gm- Am-Gf-Am-Gf-Am-Am-Gm- Um-Cms-Cms-Am | 697 | 520 |
| B264-DV28P | Gms-Ams-Cm-Um-Um- Cm-Uf-Cm-Uf-Cm-Af- Am-Um-Um-Um-Um-Cf- Um-Am-Gm-Am | 584 | 488 | UmsEVP-Cfs-Uf-Af-Gm-Af- Am-Am-Am-Um-Um-Gm- Am-Gf-Am-Gf-Am-Am-Gm- Um-Cms-Cms-Am | 698 | 520 |
| B264-DV32P | Gms-Ams-Cm-Um-Um- Cm-Uf-Cm-Uf-Cm-Af- Am-Um-Um-Um-Um-Cf- Um-Am-Gm-Am | 584 | 488 | UmsEVP-Cfs-Um-Am-Gf-Af- Am-Am-Am-Um-Um-Gm- Am-Gf-Am-Gf-Am-Am-Gm- Um-Cms-Cms-Am | 699 | 520 |
| B264-DV34P | Gms-Ams-Cm-Um-Um- Cm-Uf-Cm-Uf-Cm-Af- Am-Um-Um-Um-Um-Cf- Um-Am-Gm-Am | 584 | 488 | UmsEVP-Cfs-Uf-Am-Gm-Af- Am-Am-Am-Um-Um-Gm- Am-Gf-Am-Gf-Am-Am-Gm- Um-Cms-Cms-Am | 700 | 520 |
| B264-DV30P | Gms-Ams-Cm-Um-Um- Cm-Uf-Cm-Uf-Cf-Af-Am- Um-Um-Um-Um-Cm-Um- Am-Gm-Am | 586 | 488 | UmsEVP-Cfs-Um-Am-Gm- Af-Am-Am-Am-Um-Um-Gm- Am-Gf-Am-Gm-Am-Am-Gm- Um-Cms-Cms-Am | 701 | 520 |
| B264-DV31P | Gms-Ams-Cm-Um-Um- Cm-Uf-Cm-Uf-Cf-Am- Am-Um-Um-Um-Um-Cm- Um-Am-Gm-Am | 587 | 488 | UmsEVP-Cfs-Um-Am-Gm- Af-Am-Am-Am-Um-Um-Gm- Am-Gf-Am-Gf-Am-Am-Gm- Um-Cms-Cms-Am | 702 | 520 |
| B264-DV21P | Gms-Ams-Cm-Um-Um- Cm-Uf-Cm-Uf-Cf-Af-Am- Um-Um-Um-Uf-Cf-Um- Am-Gm-Am | 588 | 488 | UmsEVP-Cfs-Um-Am-Gm- Af-Am-Am-Am-Um-Um-Gm- Am-Gf-Am-Gf-Am-Am-Gm- Um-Cms-Cms-Am | 702 | 520 |
| B264-DV22P | Gms-Ams-Cm-Um-Um- Cm-Uf-Cm-Uf-Cf-Af-Am- Um-Um-Um-Um-Cm-Um- Am-Gm-Am | 586 | 488 | UmsEVP-Cfs-Um-Am-Gm- Af-Am-Am-Am-Um-Um-Gm- Am-Gf-Am-Gf-Am-Am-Gm- Um-Cms-Cms-Am | 702 | 520 |
| B1556-DV29P | Ums-Gms-Um-Gm-Cm- Cm-Uf-Um-Cf-Um-Cf- Am-Um-Cm-Um-Gm-Cf- Cm-Gm-Gm-Am | 589 | 489 | UmsEVP-Cfs-Cm-Gf-Gf-Cf- Am-Gm-Am-Um-Gm-Am- Gm-Af-Am-Gf-Gm-Cm-Am- Cm-Ams-Gms-Am | 703 | 521 |
| B1556-DV26P | Ums-Gms-Um-Gm-Cm- Cm-Uf-Um-Cf-Uf-Cf-Am- Um-Cm-Um-Gm-Cf-Cm- Gm-Gm-Am | 590 | 489 | UmsEVP-Cfs-Cf-Gf-Gm-Cf- Am-Gm-Am-Um-Gm-Am- Gm-Af-Am-Gf-Gm-Cm-Am- Cm-Ams-Gms-Am | 704 | 521 |
| B1556-DV27P | Ums-Gms-Um-Gm-Cm- Cm-Uf-Um-Cf-Uf-Cf-Am- Um-Cm-Um-Gm-Cf-Cm- Gm-Gm-Am | 590 | 489 | UmsEVP-Cfs-Cm-Gf-Gf-Cf- Am-Gm-Am-Um-Gm-Am- Gm-Af-Am-Gf-Gm-Cm-Am- Cm-Ams-Gms-Am | 703 | 521 |
| B1556-DV28P | Ums-Gms-Um-Gm-Cm- Cm-Uf-Um-Cf-Um-Cf- Am-Um-Cm-Um-Gm-Cf- Cm-Gm-Gm-Am | 589 | 489 | UmsEVP-Cfs-Cf-Gf-Gm-Cf- Am-Gm-Am-Um-Gm-Am- Gm-Af-Am-Gf-Gm-Cm-Am- Cm-Ams-Gms-Am | 704 | 521 |
| B1556-DV32P | Ums-Gms-Um-Gm-Cm- Cm-Uf-Um-Cf-Um-Cf- Am-Um-Cm-Um-Gm-Cf- Cm-Gm-Gm-Am | 589 | 489 | UmsEVP-Cfs-Cm-Gm-Gf-Cf- Am-Gm-Am-Um-Gm-Am- Gm-Af-Am-Gf-Gm-Cm-Am- Cm-Ams-Gms-Am | 705 | 521 |
| B1556-DV34P | Ums-Gms-Um-Gm-Cm- Cm-Uf-Um-Cf-Um-Cf- Am-Um-Cm-Um-Gm-Cf- Cm-Gm-Gm-Am | 589 | 489 | UmsEVP-Cfs-Cf-Gm-Gm-Cf- Am-Gm-Am-Um-Gm-Am- Gm-Af-Am-Gf-Gm-Cm-Am- Cm-Ams-Gms-Am | 706 | 521 |
| B1556-DV30P | Ums-Gms-Um-Gm-Cm- Cm-Uf-Um-Cf-Uf-Cf-Am- Um-Cm-Um-Gm-Cm-Cm- Gm-Gm-Am | 591 | 489 | UmsEVP-Cfs-Cm-Gm-Gm-Cf- Am-Gm-Am-Um-Gm-Am- Gm-Af-Am-Gm-Gm-Cm-Am- Cm-Ams-Gms-Am | 707 | 521 |
| B1556-DV31P | Ums-Gms-Um-Gm-Cm- Cm-Uf-Um-Cf-Uf-Cm- Am-Um-Cm-Um-Gm-Cm- Cm-Gm-Gm-Am | 592 | 489 | UmsEVP-Cfs-Cm-Gm-Gm-Cf- Am-Gm-Am-Um-Gm-Am- Gm-Af-Am-Gf-Gm-Cm-Am- Cm-Ams-Gms-Am | 708 | 521 |
| B1556-DV21P | Ums-Gms-Um-Gm-Cm- Cm-Uf-Um-Cf-Uf-Cf-Am- Um-Cm-Um-Gf-Cf-Cm- Gm-Gm-Am | 593 | 489 | UmsEVP-Cfs-Cm-Gm-Gm-Cf- Am-Gm-Am-Um-Gm-Am- Gm-Af-Am-Gf-Gm-Cm-Am- Cm-Ams-Gms-Am | 708 | 521 |
| B1556-DV22P | Ums-Gms-Um-Gm-Cm- Cm-Uf-Um-Cf-Uf-Cf-Am- Um-Cm-Um-Gm-Cm-Cm- Gm-Gm-Am | 591 | 489 | UmsEVP-Cfs-Cm-Gm-Gm-Cf- Am-Gm-Am-Um-Gm-Am- Gm-Af-Am-Gf-Gm-Cm-Am- Cm-Ams-Gms-Am | 708 | 521 |
| B1560-DV29P | Cms-Cms-Um-Um-Cm- Um-Cf-Am-Uf-Cm-Uf- Gm-Cm-Cm-Gm-Gm-Af- Cm-Cm-Gm-Um | 594 | 490 | AmsEVP-Cfs-Gm-Gf-Uf-Cf- Cm-Gm-Gm-Cm-Am-Gm- Am-Uf-Gm-Af-Gm-Am-Am- Gm-Gms-Cms-Am | 709 | 522 |
| B1560-DV26P | Cms-Cms-Um-Um-Cm- Um-Cf-Am-Uf-Cf-Uf-Gm- Cm-Cm-Gm-Gm-Af-Cm- Cm-Gm-Um | 595 | 490 | AmsEVP-Cfs-Gf-Gf-Um-Cf- Cm-Gm-Gm-Cm-Am-Gm- Am-Uf-Gm-Af-Gm-Am-Am- Gm-Gms-Cms-Am | 710 | 522 |
| B1560-DV27P | Cms-Cms-Um-Um-Cm- Um-Cf-Am-Uf-Cf-Uf-Gm- Cm-Cm-Gm-Gm-Af-Cm- Cm-Gm-Um | 595 | 490 | AmsEVP-Cfs-Gm-Gf-Uf-Cf- Cm-Gm-Gm-Cm-Am-Gm- Am-Uf-Gm-Af-Gm-Am-Am- Gm-Gms-Cms-Am | 709 | 522 |
| B1560-DV28P | Cms-Cms-Um-Um-Cm- Um-Cf-Am-Uf-Cm-Uf- Gm-Cm-Cm-Gm-Gm-Af- Cm-Cm-Gm-Um | 594 | 490 | AmsEVP-Cfs-Gf-Gf-Um-Cf- Cm-Gm-Gm-Cm-Am-Gm- Am-Uf-Gm-Af-Gm-Am-Am- Gm-Gms-Cms-Am | 710 | 522 |
| B1560-DV32P | Cms-Cms-Um-Um-Cm- Um-Cf-Am-Uf-Cm-Uf- Gm-Cm-Cm-Gm-Gm-Af- Cm-Cm-Gm-Um | 594 | 490 | AmsEVP-Cfs-Gm-Gm-Uf-Cf- Cm-Gm-Gm-Cm-Am-Gm- Am-Uf-Gm-Af-Gm-Am-Am- Gm-Gms-Cms-Am | 711 | 522 |
| B1560-DV34P | Cms-Cms-Um-Um-Cm- Um-Cf-Am-Uf-Cm-Uf- Gm-Cm-Cm-Gm-Gm-Af- Cm-Cm-Gm-Um | 594 | 490 | AmsEVP-Cfs-Gf-Gm-Um-Cf- Cm-Gm-Gm-Cm-Am-Gm- Am-Uf-Gm-Af-Gm-Am-Am- Gm-Gms-Cms-Am | 712 | 522 |
| B1560-DV30P | Cms-Cms-Um-Um-Cm- Um-Cf-Am-Uf-Cf-Uf-Gm- Cm-Cm-Gm-Gm-Am-Cm- Cm-Gm-Um | 596 | 490 | AmsEVP-Cfs-Gm-Gm-Um- Cf-Cm-Gm-Gm-Cm-Am-Gm- Am-Uf-Gm-Am-Gm-Am-Am- Gm-Gms-Cms-Am | 713 | 522 |
| B1560-DV31P | Cms-Cms-Um-Um-Cm- Um-Cf-Am-Uf-Cf-Um- Gm-Cm-Cm-Gm-Gm-Am- Cm-Cm-Gm-Um | 597 | 490 | AmsEVP-Cfs-Gm-Gm-Um- Cf-Cm-Gm-Gm-Cm-Am-Gm- Am-Uf-Gm-Af-Gm-Am-Am- Gm-Gms-Cms-Am | 714 | 522 |
| B1560-DV21P | Cms-Cms-Um-Um-Cm- Um-Cf-Am-Uf-Cf-Uf-Gm- Cm-Cm-Gm-Gf-Af-Cm- Cm-Gm-Um | 598 | 490 | AmsEVP-Cfs-Gm-Gm-Um- Cf-Cm-Gm-Gm-Cm-Am-Gm- Am-Uf-Gm-Af-Gm-Am-Am- Gm-Gms-Cms-Am | 714 | 522 |
| B1560-DV22P | Cms-Cms-Um-Um-Cm- Um-Cf-Am-Uf-Cf-Uf-Gm- Cm-Cm-Gm-Gm-Am-Cm- Cm-Gm-Um | 596 | 490 | AmsEVP-Cfs-Gm-Gm-Um- Cf-Cm-Gm-Gm-Cm-Am-Gm- Am-Uf-Gm-Af-Gm-Am-Am- Gm-Gms-Cms-Am | 714 | 522 |
| B416S-DV29SP | Ums-Gms-Cm-Um-Gm-Cf- Um-Af-Um-Gf-Cm-Cm- Um-Cm-Am-Uf-Cm-Um- Um-Am | 599 | 491 | UmsEVP-Afs-Am-Gf-Af-Uf- Gm-Am-Gm-Gm-Cm-Am- Um-Af-Gm-Cf-Am-Gm-Cm- Ams-Gms-Gm | 715 | 523 |
| B416S-DV26SP | Ums-Gms-Cm-Um-Gm-Cf- Um-Af-Uf-Gf-Cm-Cm- Um-Cm-Am-Uf-Cm-Um- Um-Am | 600 | 491 | UmsEVP-Afs-Af-Gf-Am-Uf- Gm-Am-Gm-Gm-Cm-Am- Um-Af-Gm-Cf-Am-Gm-Cm- Ams-Gms-Gm | 716 | 523 |
| B416S-DV27SP | Ums-Gms-Cm-Um-Gm-Cf- Um-Af-Uf-Gf-Cm-Cm- Um-Cm-Am-Uf-Cm-Um- Um-Am | 600 | 491 | UmsEVP-Afs-Am-Gf-Af-Uf- Gm-Am-Gm-Gm-Cm-Am- Um-Af-Gm-Cf-Am-Gm-Cm- Ams-Gms-Gm | 715 | 523 |
| B416S-DV28SP | Ums-Gms-Cm-Um-Gm-Cf- Um-Af-Um-Gf-Cm-Cm- Um-Cm-Am-Uf-Cm-Um- Um-Am | 599 | 491 | UmsEVP-Afs-Af-Gf-Am-Uf- Gm-Am-Gm-Gm-Cm-Am- Um-Af-Gm-Cf-Am-Gm-Cm- Ams-Gms-Gm | 716 | 523 |
| B416S-DV32SP | Ums-Gms-Cm-Um-Gm-Cf- Um-Af-Um-Gf-Cm-Cm- Um-Cm-Am-Uf-Cm-Um- Um-Am | 599 | 491 | UmsEVP-Afs-Am-Gm-Af-Uf- Gm-Am-Gm-Gm-Cm-Am- Um-Af-Gm-Cf-Am-Gm-Cm- Ams-Gms-Gm | 717 | 523 |
| B416S-DV34SP | Ums-Gms-Cm-Um-Gm-Cf- Um-Af-Um-Gf-Cm-Cm- Um-Cm-Am-Uf-Cm-Um- Um-Am | 599 | 491 | UmsEVP-Afs-Af-Gm-Am-Uf- Gm-Am-Gm-Gm-Cm-Am- Um-Af-Gm-Cf-Am-Gm-Cm- Ams-Gms-Gm | 718 | 523 |
| B416S-DV30SP | Ums-Gms-Cm-Um-Gm-Cf- Um-Af-Uf-Gf-Cm-Cm- Um-Cm-Am-Um-Cm-Um- Um-Am | 601 | 491 | UmsEVP-Afs-Am-Gm-Am- Uf-Gm-Am-Gm-Gm-Cm-Am- Um-Af-Gm-Cm-Am-Gm-Cm- Ams-Gms-Gm | 719 | 523 |
| B416S-DV31SP | Ums-Gms-Cm-Um-Gm-Cf- Um-Af-Uf-Gm-Cm-Cm- Um-Cm-Am-Um-Cm-Um- Um-Am | 602 | 491 | UmsEVP-Afs-Am-Gm-Am- Uf-Gm-Am-Gm-Gm-Cm-Am- Um-Af-Gm-Cf-Am-Gm-Cm- Ams-Gms-Gm | 720 | 523 |
| B416S-DV21SP | Ums-Gms-Cm-Um-Gm-Cf- Um-Af-Uf-Gf-Cm-Cm- Um-Cm-Af-Uf-Cm-Um- Um-Am | 603 | 491 | UmsEVP-Afs-Am-Gm-Am- Uf-Gm-Am-Gm-Gm-Cm-Am- Um-Af-Gm-Cf-Am-Gm-Cm- Ams-Gms-Gm | 720 | 523 |
| B416S-DV22SP | Ums-Gms-Cm-Um-Gm-Cf- Um-Af-Uf-Gf-Cm-Cm- Um-Cm-Am-Um-Cm-Um- Um-Am | 601 | 491 | UmsEVP-Afs-Am-Gm-Am- Uf-Gm-Am-Gm-Gm-Cm-Am- Um-Af-Gm-Cf-Am-Gm-Cm- Ams-Gms-Gm | 720 | 523 |

### 2. Experimental methods

Referring to Example 9, the concentration for the single-concentration screening assay was set to 0.3 nM.

### 3. Experimental results

The experimental results demonstrated that the sequences B207S-DV32SP, A1550-DV27P, B1572-DV27P, B1575-DV27P, B207S-DV29SP, A1573-DV29P, B1575-DV29P, B418S-DV34SP, and A416-DV27P exhibited significantly superior inhibitory effects on HBsAg compared to the positive control sequence, and significantly superior inhibitory effects on HBeAg compared to the positive control sequence.

### Example 12: Comparison of inhibitory effects of sequences disclosed in the prior art on HBV gene expression

In this example, unmodified sequences disclosed in the prior art that are identical or similar to basic sequences B207S, B1575, B1550, B1572, A416, B1573, and B418S disclosed herein were compared with the sequences modified using the previously disclosed templates DV30P, DV31P, DV21P, and DV22P (or DV30SP, DV31SP, DV21SP, and DV22SP), and the sequences modified using templates DV25P, DV26P, DV27P, DV28P, DV29P, DV32P, DV33P, and DV34P (or DV25SP, DV26SP, DV27SP, DV28SP, DV29SP, DV32SP, DV33SP, and DV34SP) disclosed herein, in terms of their inhibitory effects on HBV gene expression.

### 1. Experimental materials

### Test samples:

(1) Sequences disclosed in the prior art, as shown in Table 48.

**Table 48: Sequences disclosed in the prior art**

| Sequence ID | Position | Sense strand | SEQ ID NO: | Antisense strand | SEQ ID NO: | Source |
|---|---|---|---|---|---|---|
| P206 | 206-226 | CGGGGUUUUUCUU GUUGACAA | 467 | UUGUCAACAAGAA AAACCCCG | 524 | WO2023093896A1 |
| P413 | 413-433 | UCCUGCUGCUAUG CCUCAUCU | 475 | AUGAGGCAUAGCA GCAGGATT | 525 | US20060287266A1 |
| P1551 | 1551-1571 | CCGUCUGUGCCUU CUCAUCUA | 492 | UAGAUGAGAAGGC ACAGACGGGG | 526 | CN108064294B |
| APC | 1579-1597 | GUGUGCACUUCGC UUCACA | 493 | UGUGAAGCGAAGU GCACACUU | 527 | CN108064294B |

| | | | | | | |
|---|---|---|---|---|---|---|
| Note: The sequences disclosed in the above patent applications are all unmodified RNA sequences. | | | | | | |

(2) The siRNA sequences modified using templates DV25P, DV26P, DV27P, DV28P, DV29P, DV32P, DV33P, DV34P, DV30P, DV31P, DV21P, DV22P, DV25SP, DV26SP, DV27SP, DV28SP, DV29SP, DV32SP, DV33SP, DV34SP, DV30SP, DV31SP, DV21SP, and DV22SP in Example 11.

Cell line: HepG2.2.15 cells.

Solvents: sterile nuclease-free water, Gibco Opti-MEM.

### 2. Experimental methods

Referring to Example 9, the concentration for the single-concentration screening assay was set to 0.3 nM.

### 3. Experimental results

The experimental results demonstrated that the basic sequences B207S, A1550, B1572, B1575, A1573, B418S, and A416 disclosed herein exhibited significantly superior inhibition rates of HBsAg and HBeAg compared to the similar sequences P206, P413, P1551, and APC; after template modification, the inhibition rates were further significantly enhanced.

### Example 13: Inhibitory effect of sequences with specific anti-off-target designs on HBV and off-target genes

In practical applications of siRNA, there are numerous instances where the expression of non-target mRNAs, which are only partially complementary to the guide strand (antisense strand), is inhibited. Research by Alnylam Pharmaceuticals has demonstrated that the hepatotoxicity of N-acetylgalactosamine (GalNAc)-conjugated siRNAs is primarily attributed to off-target effects, which arise from a microRNA (miRNA)-like recognition mechanism leading to unintended gene silencing.

To address the issue, thermally destabilizing nucleotide modifications, such as glycol nucleic acid (GNA), unlocked nucleic acid (UNA), or DNA, may be introduced at positions 6 and 7 within the seed region of the antisense strand. These modifications disrupt the seed region of the antisense strand, thereby interfering with the binding of siRNA to non-target mRNA via seed-mediated recognition, which in turn significantly reduces off-target effects and mitigates hepatotoxicity. Alnylam Pharmaceuticals introduced a GNA modification at position 7 of the antisense strand of siRNA in their latest fifth-generation template design, thereby reducing off-target effects.

To reduce off-target effects of the sequences, in this example, the sequences B418S-DV34SP, B416-DV27P, B1550-DV27P, B207S-DV29SP, and B207S-DV32SP were employed, which incorporate DNA or GNA anti-off-target modifications at positions 6 and 7 of the antisense strand. These sequences were compared with sequences lacking anti-off-target modifications in terms of their activity and off-target effects.

### 1. Experimental materials

Test samples: The sequences incorporating template modification and anti-off-target design listed in Table 49 (refers to Example 8 for the synthesis method).

Cell line: HepG2.2.15 cells.

Solvents: sterile nuclease-free water, Gibco Opti-MEM.

**Table 49: Sequences incorporating template modification and anti-off-target design**

| Sequence ID | Sense sequence 5'-3' | SEQ ID NO: | Corr. basic SEQ ID NO: | Antisense sequence 5'-3' | SEQ ID NO: | Corr. basic SEQ ID NO: |
|---|---|---|---|---|---|---|
| B207S-DV29SP | Gms-Gms-Gm-Gm-Um- Uf-Um-Uf-Um-Cf-Um- Um-Gm-Um-Um-Gf-Am- Cm-Am-Am | 529 | 449 | UmsEVP-Ufs-Gm-Uf-Cf-Af- Am-Cm-Am-Am-Gm-Am- Am-Af-Am-Af-Cm-Cm-Cm- Cms-Gms-Cm | 627 | 456 |
| C207S-DV29SPd67B | Gms-Gms-Gm-Gm-Um- Uf-Um-Uf-Um-Cf-Um- Um-Gm-dT-dT-Gf-Am- Cm-Am-Am | 744 | 449 | UmsEVP-Ufs-Gm-Uf-Cf-dA- dA-Cm-Am-Am-Gm-Am-Am- Af-Am-Af-Cm-Cm-Cm-Cms- Gms-Cm | 759 | 456 |
| C207S-DV29SPd7B | Gms-Gms-Gm-Gm-Um- Uf-Um-Uf-Um-Cf-Um- Um-Gm-dT-Um-Gf-Am- Cm-Am-Am | 745 | 449 | UmsEVP-Ufs-Gm-Uf-Cf-Af- dA-Cm-Am-Am-Gm-Am-Am- Af-Am-Af-Cm-Cm-Cm-Cms- Gms-Cm | 760 | 456 |
| C207S-DV29SP+ | Gms-Gms-Gm-Gm-Um- Uf-Um-Uf-Um-Cf-Um- Um-Gm-Um-Um-Gf-Am- Cm-Am-Am | 746 | 449 | UmsEVP-Ufs-Gm-Uf-Cf- A(gna)-Am-Cm-Am-Am-Gm- Am-Am-Af-Am-Af-Cm-Cm- Cm-Cms-Gms-Cm | 761 | 456 |
| B207S-DV32SP | Gms-Gms-Gm-Gm-Um- Uf-Um-Uf-Um-Cf-Um- Um-Gm-Um-Um-Gf-Am- Cm-Am-Am | 529 | 449 | UmsEVP-Ufs-Gm-Um-Cf-Af- Am-Cm-Am-Am-Gm-Am- Am-Af-Am-Af-Cm-Cm-Cm- Cms-Gms-Cm | 629 | 456 |
| C207S-DV32SPd67B | Gms-Gms-Gm-Gm-Um- Uf-Um-Uf-Um-Cf-Um- Um-Gm-dT-dT-Gf-Am- Cm-Am-Am | 747 | 449 | UmsEVP-Ufs-Gm-Um-Cf-dA- dA-Cm-Am-Am-Gm-Am-Am- Af-Am-Af-Cm-Cm-Cm-Cms- Gms-Cm | 762 | 456 |
| C207S-DV32SPd7B | Gms-Gms-Gm-Gm-Um- Uf-Um-Uf-Um-Cf-Um- Um-Gm-dT-Um-Gf-Am- Cm-Am-Am | 748 | 449 | UmsEVP-Ufs-Gm-Um-Cf-Af- dA-Cm-Am-Am-Gm-Am-Am- Af-Am-Af-Cm-Cm-Cm-Cms- Gms-Cm | 763 | 456 |
| C207S-DV32SP+ | Gms-Gms-Gm-Gm-Um- Uf-Um-Uf-Um-Cf-Um- Um-Gm-Um-Um-Gf-Am- Cm-Am-Am | 749 | 449 | UmsEVP-Ufs-Gm-Um-Cf- A(gna)-Am-Cm-Am-Am-Gm- Am-Am-Af-Am-Af-Cm-Cm- Cm-Cms-Gms-Cm | 764 | 456 |
| B1550-DV27P | Cms-Cms-Cm-Gm-Um- Cm-Uf-Gm-Uf-Gf-Cf-Cm- Um-Um-Cm-Um-Cf-Am- Um-Cm-Um | 535 | 450 | AmsEVP-Gfs-Am-Uf-Gf-Af- Gm-Am-Am-Gm-Gm-Cm- Am-Cf-Am-Gf-Am-Cm-Gm- Gm-Gms-Gms-Am | 635 | 457 |
| C1550-DV27Pd67B | Cms-Cms-Cm-Gm-Um- Cm-Uf-Gm-Uf-Gf-Cf-Cm- Um-Um-dC-dT-Cf-Am- Um-Cm-Um | 750 | 450 | AmsEVP-Gfs-Am-Uf-Gf-dA- dG-Am-Am-Gm-Gm-Cm-Am- Cf-Am-Gf-Am-Cm-Gm-Gm- Gms-Gms-Am | 765 | 457 |
| C1550-DV27Pd7B | Cms-Cms-Cm-Gm-Um- Cm-Uf-Gm-Uf-Gf-Cf-Cm- Um-Um-dC-Um-Cf-Am- Um-Cm-Um | 751 | 450 | AmsEVP-Gfs-Am-Uf-Gf-Af- dG-Am-Am-Gm-Gm-Cm-Am- Cf-Am-Gf-Am-Cm-Gm-Gm- Gms-Gms-Am | 766 | 457 |
| C1550-DV27P+ | Cms-Cms-Cm-Gm-Um- Cm-Uf-Gm-Uf-Gf-Cf-Cm- Um-Um-Cm-Um-Cf-Am- Um-Cm-Um | 752 | 450 | AmsEVP-Gfs-Am-Uf-Gf- A(gna)-Gm-Am-Am-Gm-Gm- Cm-Am-Cf-Am-Gf-Am-Cm- Gm-Gm-Gms-Gms-Am | 767 | 457 |
| B418S-DV34SP | Cms-Ums-Gm-Cm-Um-Af- Um-Gf-Cm-Cf-Um-Cm- Am-Um-Cm-Uf-Um-Cm- Um-Um | 554 | 454 | AmsEVP-Afs-Gf-Am-Am-Gf- Am-Um-Gm-Am-Gm-Gm- Cm-Af-Um-Af-Gm-Cm-Am- Gms-Cms-Am | 664 | 461 |
| C418S-DV34SPd67B | Cms-Ums-Gm-Cm-Um-Af- Um-Gf-Cm-Cf-Um-Cm- Am-dT-dC-Uf-Um-Cm- Um-Um | 753 | 454 | AmsEVP-Afs-Gf-Am-Am-dG- dA-Um-Gm-Am-Gm-Gm-Cm- Af-Um-Af-Gm-Cm-Am-Gms- Cms-Am | 768 | 461 |
| C418S-DV34SPd7B | Cms-Ums-Gm-Cm-Um-Af- Um-Gf-Cm-Cf-Um-Cm- Am-dT-Cm-Uf-Um-Cm- Um-Um | 754 | 454 | AmsEVP-Afs-Gf-Am-Am-Gf- dA-Um-Gm-Am-Gm-Gm-Cm- Af-Um-Af-Gm-Cm-Am-Gms- Cms-Am | 769 | 461 |
| C418S-DV34SP+ | Cms-Ums-Gm-Cm-Um-Af- Um-Gf-Cm-Cf-Um-Cm- Am-Um-Cm-Uf-Um-Cm- Um-Um | 755 | 454 | AmsEVP-Afs-Gf-Am-Am- G(gna)-Am-Um-Gm-Am-Gm- Gm-Cm-Af-Um-Af-Gm-Cm- Am-Gms-Cms-Am | 770 | 461 |
| B416-DV27P | Ums-Gms-Cm-Um-Gm- Cm-Uf-Am-Uf-Gf-Cf-Cm- Um-Cm-Am-Um-Cf-Um- Um-Cm-Um | 560 | 455 | AmsEVP-Gfs-Am-Af-Gf-Af- Um-Gm-Am-Gm-Gm-Cm- Am-Uf-Am-Gf-Cm-Am-Gm- Cm-Ams-Gms-Gm | 667 | 462 |
| C416-DV27Pd67B | Ums-Gms-Cm-Um-Gm- Cm-Uf-Am-Uf-Gf-Cf-Cm- Um-Cm-dA-dT-Cf-Um- Um-Cm-Um | 756 | 455 | AmsEVP-Gfs-Am-Af-Gf-dA- dT-Gm-Am-Gm-Gm-Cm-Am- Uf-Am-Gf-Cm-Am-Gm-Cm- Ams-Gms-Gm | 771 | 462 |
| C416-DV27Pd7B | Ums-Gms-Cm-Um-Gm- Cm-Uf-Am-Uf-Gf-Cf-Cm- Um-Cm-dA-Um-Cf-Um- Um-Cm-Um | 757 | 455 | AmsEVP-Gfs-Am-Af-Gf-Af- dT-Gm-Am-Gm-Gm-Cm-Am- Uf-Am-Gf-Cm-Am-Gm-Cm- Ams-Gms-Gm | 772 | 462 |
| C416-DV27P+ | Ums-Gms-Cm-Um-Gm- Cm-Uf-Am-Uf-Gf-Cf-Cm- Um-Cm-Am-Um-Cf-Um- Um-Cm-Um | 758 | 455 | AmsEVP-Gfs-Am-Af-Gf- A(gna)-Um-Gm-Am-Gm-Gm- Cm-Am-Uf-Am-Gf-Cm-Am- Gm-Cm-Ams-Gms-Gm | 773 | 462 |

### 2. Experimental methods

The inhibitory effects of the test samples on HBV and off-target genes in HepG2.2.15 cells were evaluated by qRT-PCR.

### 2.1 Cell culture

Subculture: HepG2.2.15 cells were cultured in DMEM/F12 medium containing 10% fetal bovine serum, 370 µg/mL GENETICN, 1% L-glutamine, 1% non-essential amino acids, and 1% penicillin-streptomycin, and incubated in a cell incubator at 37°C with 5% CO₂. Cells were passaged every three days. The cells were digested with 0.25% trypsin, followed by centrifugation at 800 rpm for 3 minutes. The supernatant was discarded, and fresh medium was added for continued culture.

Seeding: HepG2.2.15 cells were seeded in DMEM/F12 medium containing 10% fetal bovine serum, 1% L-glutamine, 1% non-essential amino acids, and 1% penicillin-streptomycin.

### 2.2 Cell transfection

Preparation of transfection reagent: Lipofectamine RNAiMAX was mixed with Opti-MEM at a volume ratio of 2:98 and vortexed thoroughly.

Preparation of transfection complex: 30 µL of siRNA solution (diluted in Opti-MEM) was mixed with 30 µL of transfection mixture at a volume ratio of 1:1, vortexed thoroughly, followed by incubation at room temperature for 15 minutes to obtain a transfection complex.

Control preparation (transfection reagent): 15 µL of transfection mixture was added to 15 µL of Opti-MEM, vortexed thoroughly, followed by incubation at room temperature for 15 minutes.

The prepared transfection complex was added to a 96-well cell culture plate (15 µL per well, three replicates per sequence), such that the final siRNA concentrations per well were 0.065 nM, 0.14 nM, and 0.28 nM, respectively. Each siRNA was tested at three concentrations, with three replicate per concentration. Subsequently, 135 µL of cell suspension (2.25 × 10⁴ cells) was added to each well. After cross-mixing, the plate was incubated in a cell incubator at 37°C with 5% CO₂.

### 2.3 RNA extraction and reverse transcription

At 48 hours post-transfection, the culture medium was removed and the cells were collected for RNA extraction. Total RNA was extracted using the RNeasy^{®} 96 Kit (QIAGEN, 74182) according to the instructions provided by the manufacturer. Subsequently, RNA was reverse transcribed into cDNA using HiScript III RT SuperMix for qPCR (Vazyme) according to the instructions provided by the manufacturer.

### 2.4 RT-qPCR

cDNA of HBV and off-target genes was tested by qPCR, with GAPDH cDNA used as an internal control in parallel. 8 µL of prepared qPCR reaction mixture and 2 µL of sample cDNA were added to a 384-well plate. SYBR qPCR was performed according to the following program: 50°C for 2 minutes, 95°C for 2 minutes, followed by 40 cycles of 95°C for 5 seconds and 60°C for 30 seconds; finally, a melting curve analysis was performed at 95°C for 15 seconds, 60°C for 1 minute, and 95°C for 15 seconds.

### 2.5 Data analysis

The RNA expression levels of the target gene in each sample were calculated based on Ct values using the ΔΔCt relative quantification method. Relative expression of the target gene was expressed as 2^{-ΔΔCt}.

The calculation formula is as follows:
$ΔCt = mean Ct value of the target gene - mean Ct value of GAPDH ;$
$ΔΔCt = ΔCt \left(experimental group\right) - ΔCt \left(transfection control group\right) ;$

Relative mRNA expression of the target gene = 2^{-ΔΔCt}; 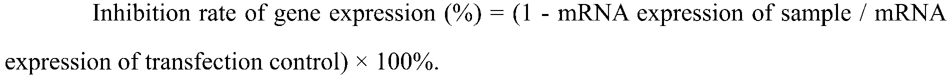 Inhibition rate of gene expression (%) = (1 - mRNA expression of sample / mRNA expression of transfection control) × 100%.

### 3. Experimental results

The experimental results demonstrated that the sequences B207S-DV32SP, A1550-DV27P, and B207S-DV29SP exhibited no off-target effects. After incorporating anti-off-target design, the sequences B418S-DV34SP and A416-DV27P showed significantly reduced inhibition of off-target genes, without compromising the inhibitory effects on the target gene HBV. The findings indicate that anti-off-target design does not compromise the inhibitory effects of the template-modified sequences disclosed herein on HBV gene expression, but can significantly inhibit off-target effects.

### Example 14: Effects of sequences modified using the templates disclosed herein on serum HBsAg, HBeAg, and HBV DNA in mice and their impact on reconstruction of adaptive immune function (high dose)

In this example, several sequences were exemplarily selected, including basic sequences B418S, A416, B1550, B207S, B1575, B1572, and B1573. These sequences were modified, for example, by incorporating template modification alone, or both template modification and anti-off-target design, and were further conjugated with GalNAc ligands. The *in vivo* efficacy of these RNAi agents was analyzed using an AAV-HBV mouse model. After infection with a recombinant adeno-associated virus (AAV) carrying a replicable HBV genome, the mouse model can continuously produce HBV viral particles and HBV antigens for more than one year without seroconversion, thereby replicating certain immunological features observed in patients with chronic hepatitis B. Therefore, novel immune-based therapies and antiviral treatments were also evaluated using the model. In this example, using the AAV-HBV mouse model, the inhibitory effects of these sequences on serum HBsAg, HBeAg, and HBV DNA at different time points, as well as their impact on reconstruction of adaptive immune function in mice at specific time points were evaluated.

### 1. Experimental materials

### Test compounds:

The sequences listed in Table 50, including those incorporating template modification alone and those incorporating both template modification and anti-off-target design, wherein the 3' end of the sense strand of these sequences is conjugated with GalNAc ligand G5. The structural formula of G5 is as follows:

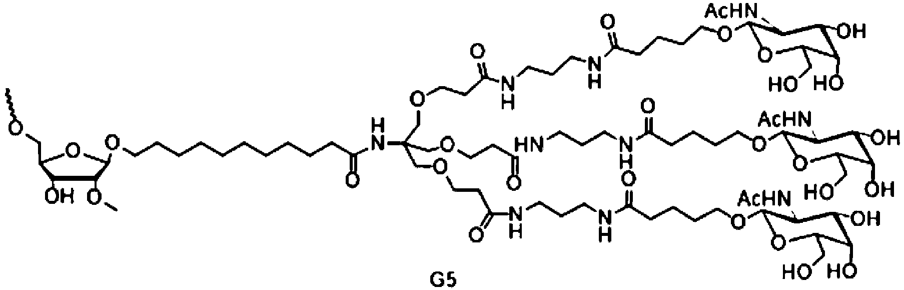

The conjugation method between oligonucleotides and ligand G5 was the same as that described in Example 10 of patent application CN116854754A.

The resulting oligonucleotide-G5 conjugate is shown below:

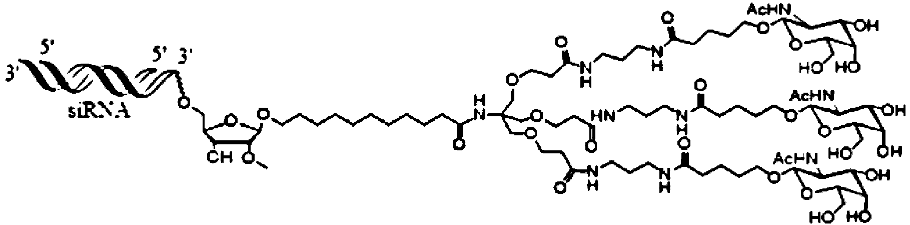

The specific sequences are shown in Table 50. In sequence identifiers, G5 indicates conjugation with GalNAc ligand G5, and GL indicates conjugation with GalNAc ligand L96.

The structural formula of L96 is as follows:

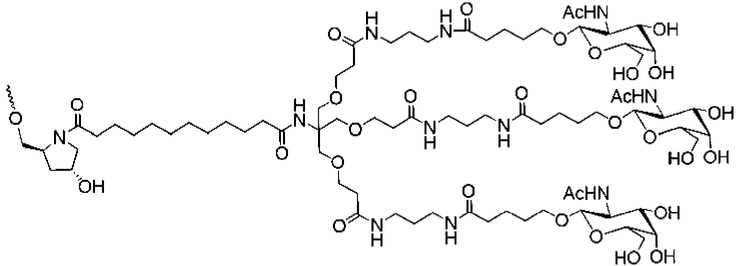

**Table 50: siRNA sequences used in animal experiments**

| Basic sequence ID | Sequence ID | Sense sequence 5'-3' | SEQ ID NO: | Corr. basic SEQ ID NO: | Antisense sequence 5'-3' | SEQ ID NO: | Corr. basic SEQ ID NO: |
|---|---|---|---|---|---|---|---|
| B207S | C207S-DV29SPG5 | Gms-Gms-Gm-Gm-Um- Uf-Um-Uf-Um-Cf-Um- Um-Gm-Um-Um-Gf-Am- Cm-Am-Am-G5 | 774 | 449 | UmsEVP-Ufs-Gm-Uf-Cf- Af-Am-Cm-Am-Am-Gm- Am-Am-Af-Am-Af-Cm- Cm-Cm-Cms-Gms-Cm | 627 | 456 |
| B207S | C207S-DV32SPG5 | Gms-Gms-Gm-Gm-Um- Uf-Um-Uf-Um-Cf-Um- Um-Gm-Um-Um-Gf-Am- Cm-Am-Am-G5 | 775 | 449 | UmsEVP-Ufs-Gm-Um-Cf- Af-Am-Cm-Am-Am-Gm- Am-Am-Af-Am-Af-Cm- Cm-Cm-Cms-Gms-Cm | 629 | 456 |
| A1550 | C1550-DV27PG5 | Cms-Cms-Cm-Gm-Um- Cm-Uf-Gm-Uf-Gf-Cf-Cm- Um-Um-Cm-Um-Cf-Am- Um-Cm-Um-G5 | 776 | 450 | AmsEVP-Gfs-Am-Uf-Gf- Af-Gm-Am-Am-Gm-Gm- Cm-Am-Cf-Am-Gf-Am- Cm-Gm-Gm-Gms-Gms-Am | 635 | 457 |
| B1572 | C1572-DV27PG5 | Cms-Cms-Gm-Gm-Am- Cm-Cf-Gm-Uf-Gf-Uf-Gm- Cm-Am-Cm-Um-Uf-Cm- Gm-Cm-Um-G5 | 777 | 451 | AmsEVP-Gfs-Cm-Gf-Af- Af-Gm-Um-Gm-Cm-Am- Cm-Am-Cf-Gm-Gf-Um- Cm-Cm-Gm-Gms-Cms-Am | 643 | 458 |
| B1575 | C1575-DV27PG5 | Gms-Ams-Cm-Cm-Gm- Um-Gf-Um-Gf-Cf-Af-Cm- Um-Um-Cm-Gm-Cf-Um- Um-Cm-Am-G5 | 778 | 452 | UmsEVP-Gfs-Am-Af-Gf- Cf-Gm-Am-Am-Gm-Um- Gm-Cm-Af-Cm-Af-Cm- Gm-Gm-Um-Cms-Cms-Gm | 649 | 459 |
| B1575 | C1575-DV29PG5 | Gms-Ams-Cm-Cm-Gm- Um-Gf-Um-Gf-Cm-Af- Cm-Um-Um-Cm-Gm-Cf- Um-Um-Cm-Am-G5 | 779 | 452 | UmsEVP-Gfs-Am-Af-Gf- Cf-Gm-Am-Am-Gm-Um- Gm-Cm-Af-Cm-Af-Cm- Gm-Gm-Um-Cms-Cms-Gm | 649 | 459 |
| A1573 | C1573-DV29PG5 | Cms-Gms-Gm-Am-Cm- Cm-Gf-Um-Gf-Um-Gf- Cm-Am-Cm-Um-Um-Cf- Gm-Cm-Um-Um-G5 | 780 | 453 | AmsEVP-Afs-Gm-Cf-Gf- Af-Am-Gm-Um-Gm-Cm- Am-Cm-Af-Cm-Gf-Gm- Um-Cm-Cm-Gms-Gms-Cm | 655 | 460 |
| B418S | C418S-DV34SPd7B G5 | Cms-Ums-Gm-Cm-Um- Af-Um-Gf-Cm-Cf-Um- Cm-Am-dT-Cm-Uf-Um- Cm-Um-Um-G5 | 781 | 454 | AmsEVP-Afs-Gf-Am-Am- Gf-dA-Um-Gm-Am-Gm- Gm-Cm-Af-Um-Af-Gm- Cm-Am-Gms-Cms-Am | 769 | 461 |
| A416 | C416-DV27Pd7B G5 | Ums-Gms-Cm-Um-Gm- Cm-Uf-Am-Uf-Gf-Cf-Cm- Um-Cm-dA-Um-Cf-Um- Um-Cm-Um-G5 | 782 | 455 | AmsEVP-Gfs-Am-Af-Gf- Af-dT-Gm-Am-Gm-Gm- Cm-Am-Uf-Am-Gf-Cm- Am-Gm-Cm-Ams-Gms-Gm | 772 | 462 |
| APC | VIR-2218-GL | Gms-Ums-Gm-Um-Gf- Cm-Af-Cf-Uf-Um-Cm- Gm-Cm-Um-Um-Cm-Am- Cm-Am-GL | 783 | 493 | Ums-Gfs-Um-Gm-Am- A(gna)-Gm-Cf-Gf-Am-Am- Gm-Um-Gf-Cm-Af-Cm- Am-Cms-Ums-Um | 742 | 527 |
| ANC | CNC-G5 | Gms-Gms-Um-Cm-Um- Cm-Uf-Um-Cf-Um-Af- Um-Cm-Gm-Am-Um-Cm- Um-Um-Am-Um-G5 | 784 | 494 | AmsEVP-Ufs-Am-Af-Gf- Af-Um-Cm-Gm-Am-Um- Am-Gm-Af-Am-Gf-Am- Gm-Am-Cm-Cms-Cms-Am | 743 | 528 |

### Preparation of test compounds:

Solvent: PBS buffer.

Preparation conditions: sterile environment.

Labeling method: each prepared dosage form was labeled with tags, with the outer packaging indicating the project number, name, concentration, quantity, preparation date, preparer, and storage conditions.

Storage conditions: freshly prepared before use; remaining samples stored at -80°C.

### Information on experimental animals:

Species/strain: AAV-HBV mice
Grade: SPF
Sex: Male
Quantity: 100
Age: 7 weeks
Weight: 19 to 24 g
Source: Guangdong Zhiyuan Biomedical Technology Co., Ltd.
Production license No.: SCXK (Yue) 2021-0057

### Review by Institutional Animal Care and Use Committee (IACUC):

Upon arrival, the experimental animals were housed at Guangzhou Jennio Biotech Co., Ltd., under a project license No.: JENNIO (Jinbin) 2019-0002. The project was approved by the IACUC of Guangzhou Jennio Biotech Co., Ltd., with IACUC approval No.: JENNIO-IACUC-2024-A005. The experimental procedures were strictly conducted in compliance with IACUC requirements to ensure animal welfare.

### Housing and management:

Housing conditions: Upon arrival, the experimental animals were housed at Guangzhou Jennio Biotech Co., Ltd. The animals were housed in rearing cages with dimensions of 29.0 cm (L) × 18.5 cm (W) × 13.0 cm (H). The environmental parameters were set as follows: a temperature ranging from 20°C to 26°C, a humidity ranging from 40% to 70%, and a 12-hour light/12-hour dark cycle with automatic lighting.

The environmental conditions complied with the National Standard GB14925-2010 of the People's Republic of China.

The animals had free access to food and water. Animal feed: Irradiated, sterilized maintenance diet for laboratory mice, supplied by Jiangsu Xietong Pharmaceutical BioEngineering Co., Ltd., with production license No.: Su Feed Certificate (2019) 01008. Nutrient composition testing complied with the National Standard GB14924.3-2010 of the People's Republic of China, while contaminant testing complied with the National Standard GB14924.2-2001 of the People's Republic of China. Batch-specific testing reports were provided by the feed supplier. Drinking water was reverse osmosis water, provided in water bottles. Drinking water testing complied with the National Standard GB5749-2006 of the People's Republic of China, which was conducted annually by a third-party testing agency.

Animal bedding: Corncob bedding, supplied by Guangzhou SeBiona Bio-Tech Co., Ltd., with production license No.: SCXK (Jing) 2019-0004. Contaminant testing complied with the National Standard GB14924.2-2001 of the People's Republic of China. Batch-specific testing reports were provided by the bedding supplier.

The cages and bedding were replaced at least once weekly. All cages and bedding were sterilized in a pulse vacuum autoclave before being introduced into a barrier environment. Cage racks were cleaned and disinfected at least once weekly.

Animal observation rooms were cleaned and disinfected daily, including shelves, floors, work surfaces, *etc*.

Disinfectants used in the barrier environment included: 6.67% benzalkonium bromide solution, 0.5% 84 disinfectant, 75% ethanol, and 0.08% didecyldimethylammonium bromide. These four disinfectants should be used alternately and should not be used in combination.

### 2. Experimental methods

### 2.1 Establishment of AAV-HBV mouse model

Definition of trial date: The day on which the animals were administered solvent or test compound was defined as Day 0.

A total of 220 SPF-grade C57BL/6 male mice were acclimatized for 7 days in a barrier facility and monitored daily to confirm health status before model establishment. The mice were randomly divided into two batches. One batch of 180 mice received tail vein injection of rAAV8-1.3HBV (Guangzhou PackGene Biotech Co., Ltd., product: AAV8[HBV-D, ayw] (D#2012), batch No.: HBV101-6) at a dose of 1 × 10¹¹ GC/100 µL per mouse, serving as the low-baseline group. The remaining 40 mice received tail vein injection of rAAV-HBV1.3-mer WT replicon (Wuhan BrainVTA Co., Ltd., batch No.: ayw1-P4-230516) at a dose of 1 × 10¹⁰ vg/100 µL per mouse, serving as the high-baseline group. At weeks 5 and 6 post-modeling (D-14 and D-7), blood samples were collected, followed by centrifugation, and plasma was analyzed for HBV DNA, HBsAg, and HBeAg. Modeling criteria: HBV DNA > 10⁵ IU/mL with fluctuation within 2 log₁₀; HBsAg fluctuation within 1 log₁₀; mean HBsAg level for the low-baseline group: 2000 to 4000 IU/mL; mean HBsAg level for the high-baseline group: 10,000 to 15,000 IU/mL.

### 2.2 Animal grouping and dosing

Definition of trial date: The day on which the animals were administered solvent or test compound was defined as Day 0 (D0).

Grouping was performed based on the indicators from week 6 post-modeling (D-7). From the low-baseline group, 72 mice were selected and randomly divided into 12 groups of 6 each based on HBsAg levels. From the high-baseline group, 18 mice were selected and randomly divided into 3 groups of 6 each based on HBsAg levels. Groups were balanced to ensure comparable mean HBsAg levels and no statistical differences in HBV DNA or HBeAg. Dosing was initiated at week 7 post-modeling (D0). The details of grouping and dosing are shown in Table 51.

**Table 51: Details of grouping and dosing**

| Group | | Dosing regimen | Route of administration | Number of animals |
|---|---|---|---|---|
| Low-baseline group | siRNA (11 groups) | 3 mg/kg, D0/7/14 | s.c. | 6 per group × 11 |
| | Vehicle control (1 group) | PBS, once on D0/7/14 | s.c. | 6 per group |
| High-baseline group | siRNA (2 groups: C207S-DV29SPG5 and positive control VIR-2218-GL) | 3 mg/kg, D0/7/14/35/42/49 | s.c. | 6 per group × 2 |
| | Vehicle control (1 group) | PBS, D0/7/14/35/42/49 | s.c. | 6 per group |

| | | | | |
|---|---|---|---|---|
| Note: s.c.: subcutaneous injection. | | | | |

### 2.3 Secondary challenge

On D60, two groups (3 mice per group, 6 mice in total) were selected for hydrodynamic tail vein injection of 8 µg pAAV-HBV 1.2 plasmid (Fenghui Biotechnology), with an injection volume of 100 µL/g. The remaining 6 mice were left untreated.

### 2.4 Observation and measurement

### (1) General observations

Daily monitoring during modeling and trial periods, including mortality or moribund status, food and water intake, injuries, fecal characteristics, physical appearance and coat condition, mental state, activity levels, *etc*.

### (2) Body weight

Acclimation: Animals were weighed and recorded upon arrival and at the end of acclimation.

Trial period: Animals were weighed and recorded weekly during the trial period. If dosing or blood collection was scheduled for the same day, weighing was performed prior to the procedure and prior to euthanasia.

### (3) Measurement of serum HBsAg, HBeAg, HBV DNA, HBsAb, and ALT levels

At week 4 (D-14) and week 5 (D-7) post-modeling, on the day prior to dosing (D0), and weekly post-dosing, 200 µL of blood was collected from the inner canthus and added to an EDTA-K2 tube, followed by centrifugation at 1000 g for 10 minutes to obtain plasma. The 12 mice in the two groups were subjected to challenge on D60. 200 µL of blood was collected from the inner canthus on D63, D67, D74, and D81, respectively, and added to an EDTA-K2 tube, followed by centrifugation at 1000 g for 10 minutes to obtain plasma. 20 µL of plasma was added to 980 µL of PBS, vortexed thoroughly, and analyzed for HBsAg, HBV DNA, HBeAg, and ALT levels. Separately, 15 µL of plasma was added to 210 µL of PBS, vortexed thoroughly, and analyzed for HBsAb levels. All processed samples to be tested were analyzed by Guangzhou Huayin Medical Research Institute. The remaining plasma was stored at - 80°C.

### 2.5 In vitro immunoassay

At designated time points, mononuclear cells were isolated from the liver and spleen for immunoassays. IFN-γ secretion levels in liver mononuclear cells were measured using ELISpot assays. IFN-γ, IFN-α, and IL-2 secretion levels in spleen mononuclear cells were measured using ELISpot assays. HBsAg-specific IgG secretion levels in effector B cells and memory B cells within spleen mononuclear cells were measured using ELISpot assays. The proportion of HBsAg-specific memory B cells in the spleen were measured using flow cytometry. The proportions of CD4⁺ T cells and CD8⁺ T cells in the spleen, as well as the proportions of cells secreting IFN-γ, IFN-α, and IL-2 were measured using flow cytometry.

### 2.6 Data processing and statistical analysis

Data are expressed as mean ± standard deviation (Mean ± SD) and analyzed using GraphPad Prism 8.3 software. Data conforming to normal distribution and homogeneity of variance were tested using one-way ANOVA. When variances were unequal, Welch's ANOVA was employed. When data did not conform to normal distribution, the non-parametric Kruskal-Wallis H test was employed. *P* < 0.05 was considered statistically significant.

### 3. Experimental results

The experimental results demonstrated that, compared to the vehicle control group and the negative control group, the sequences B207S-DV32SP, A1550-DV27P, B1572-DV27P, B1575-DV27P, B207S-DV29SP, A1573-DV29P, B1575-DV29P, B418S-DV34SPd7B, and A416-DV27Pd7B, when conjugated with G5, can be specifically delivered to the liver, exhibiting sustained and significant inhibition of serum HBsAg, HBeAg, and HBV DNA levels.

Specifically, the sequences C207S-DV32SPG5, C207S-DV29SPG5, and C418S-DV34SPd7BG5 demonstrated superior efficacy in reducing HBsAg, HBV DNA, and HBeAg levels, significantly outperforming the positive control sequence VIR-2218-GL. Furthermore, the sequences C207S-DV32SPG5 and C207S-DV29SPG5 ultimately achieved HBsAg and HBV DNA clearance in all mice within their respective groups, and both induced high levels of antibodies. In the C418S-DV34SPd7BG5 group, 3 out of 6 mice ultimately achieved HBsAg clearance, also accompanied by the production of high levels of antibodies.

The results of secondary challenge demonstrated that the blank control group (which had not been infected with HBV prior to D56) exhibited the presence of HBsAg, HBeAg, and HBV DNA after challenge, indicating that the challenge was effective. In the challenged mice of the C207S-DV32SPG5 group, HBV DNA levels initially increased after challenge and then decreased to the detection limit, while serum HBsAb levels increased significantly and HBsAg levels remained below the detection limit, indicating that the challenge was effective and that HBV-infected mice treated with the oligonucleotide sequence were protected from reinfection by HBV. These findings confirm that HBV-infected mice treated with the oligonucleotide sequence are resistant to subsequent HBV infection.

### (i) Changes over time in HBsAg, HBeAg, HBV DNA, HBsAb, and ALT levels in mice from each sequence group

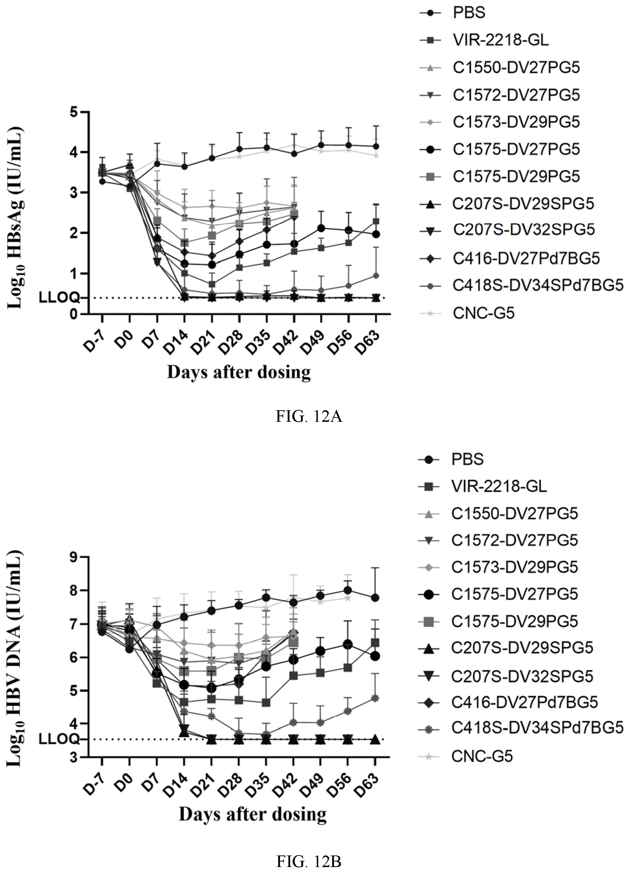

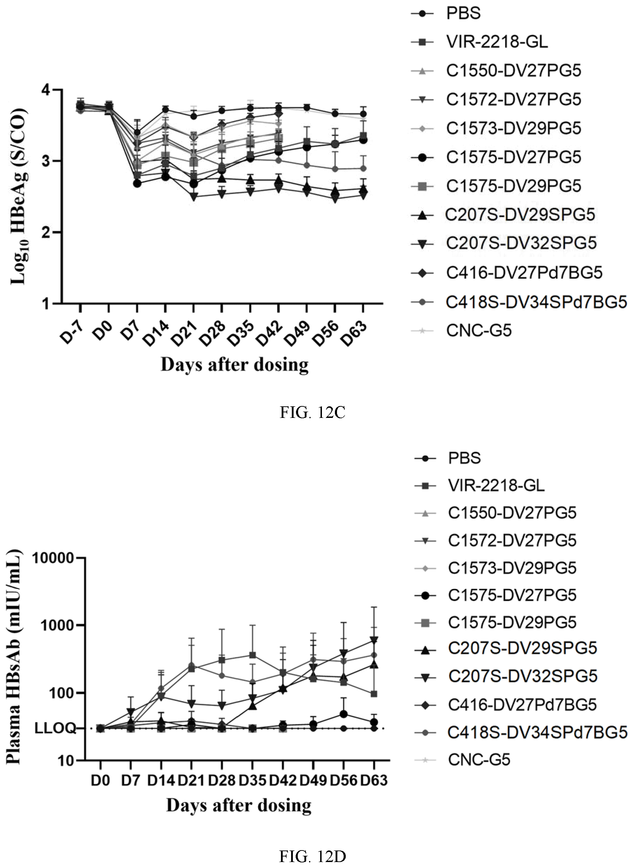

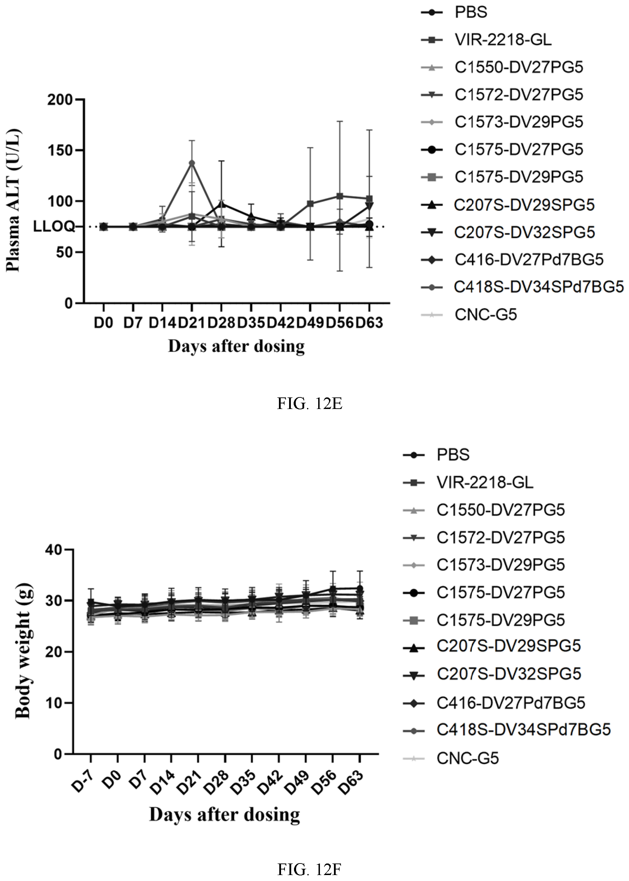

The changes over time in HBsAg, HBeAg, HBV DNA, HBsAb, ALT, and body weight in each group are shown in Tables 51-1 to 51-9 and FIGs. 12A to 12F. As shown in the tables, the sequences C207S-DV32SPG5, C207S-DV29SPG5, and C418S-DV34SPd7BG5 demonstrated superior efficacy in reducing HBsAg, HBV DNA, and HBeAg levels, significantly outperforming the positive control sequence VIR-2218-GL. Furthermore, the sequences C207S-DV32SPG5 and C207S-DV29SPG5 ultimately achieved HBsAg and HBV DNA clearance in all mice within their respective groups, and both induced high levels of antibodies. Specifically, the C207S-DV32SPG5 group achieved HBsAg and HBV DNA clearance in all mice on Day 14 and Day 21, respectively, which persisted until the end of the study, indicating that functional cure was achieved in these mice. In the C418S-DV34SPd7BG5 group, 3 out of 6 mice ultimately achieved HBsAg clearance, also accompanied by the production of high levels of antibodies. These data demonstrate the superior efficacy of the sequences C207S-DV32SPG5, C207S-DV29SPG5, and C418S-DV34SPd7BG5.

**Table 51-1: Changes in plasma HBsAg levels in mice from each sequence group**

| Basic seq ID | Seq ID | **Log₁₀ HBsAg (IU/mL)** | | | | | | | | | | |
|---|---|---|---|---|---|---|---|---|---|---|---|---|
| | | **D-7** | **D0** | **D7** | **D14** | **D21** | **D28** | **D35** | **D42** | **D49** | **D56** | **D63** |
| | PBS | 3.27 ± 0.60 | 3.16 ± 0.50 | 3.71 ± 0.51 | 3.64 + 0.34 | 3.85 ± 0.35 | 4.08 + 0.40 | 4.12 + 0.36 | 3.96 + 0.48 | 4.17 + 0.35 | 4.17 + 0.44 | 4.14 + 0.51 |
| APC | VIR-2218-GL | 3.49 ± 0.16 | 3.09 + 0.38 | 1.61 + 0.17** | 1.01 + 0.19** | 0.74 + 0.28** | 1.15 ± 0.23** | 1.26 + 0.22** | 1.54 + 0.45** | 1.63 + 0.25 | 1.75 + 0.29 | 2.29 ± 0.44 |
| A1550 | C1550-DV27PG5 | 3.49 ± 0.15 | 3.48 ± 0.18 | 2.83 + 0.21 | 2.38 + 0.40 | 2.18 + 0.47 | 2.28 ± 0.48 | 2.51 + 0.65 | 2.63 + 0.52 | - | - | - |
| B1572 | C1572-DV27PG5 | 3.50 + 0.13 | 3.44 + 0.36 | 2.76 + 0.54 | 2.39 + 0.58 | 2.29 ± 0.52 | 2.49 ± 0.50 | 2.58 + 0.77 | 2.65 + 0.73 | - | - | - |
| A1573 | C1573-DV29PG5 | 3.50 + 0.13 | 3.44 + 0.30 | 2.98 + 0.56 | 2.64 + 0.44 | 2.67 + 0.38 | 2.62 + 0.35 | 2.76 + 0.39 | 2.67 + 0.51 | - | - | - |
| B1575 | C1575-DV27PG5 | 3.49 ± 0.15 | 3.45 ± 0.24 | 1.62 + 0.51** | 1.24 + 0.56* | 1.22 + 0.50* | 1.47 ± 0.61* | 1.71 + 0.66* | 1.73 + 0.68* | 2.12 ± 0.43 | 2.07 + 0.45 | 1.97 + 0.74 |
| B1575 | C1575-DV29PG5 | 3.50 + 0.12 | 3.40 ± 0.18 | 2.31 ± 0.29 | 1.76 + 0.34 | 1.93 + 0.44 | 2.23 ± 0.36 | 2.29 ± 0.31 | 2.48 ± 0.29 | - | - | - |
| B207S | C207S-DV29SPG5 | 3.50 + 0.11 | 3.69 ± 0.27 | 1.88 ± 0.32* | 0.43 ± 0.08** | 0.41 ± 0.03** ## | 0.43 ± 0.08** ## | 0.45 ± 0.12** ## | 0.45 ± 0.12** ## | 0.40 ± 0.00** ## | 0.41 ± 0.03** ## | 0.40 ± 0.00** ## |
| B207S | C207S-DV32SPG5 | 3.50 + 0.13 | 3.35 ± 0.26 | 1.29 + 0.53** | 0.40 ± 0.00** | 0.40 ± 0.00** ## | 0.40 ± 0.00** ## | 0.40 ± 0.00** ## | 0.40 ± 0.00** ## | 0.40 ± 0.00** ## | 0.40 ± 0.00** ## | / |
| A416 | C416-DV27Pd7B G5 | 3.47 ± 0.20 | 3.47 ± 0.16 | 1.90 ± 0.30* | 1.52 ± 0.31 | 1.44 ± 0.34 | 1.79 ± 0.31 | 2.08 ± 0.27 | 2.41 ± 0.13 | - | - | - |
| B418S | C418S-DV34SPd7 BG5 | 3.47 ± 0.23 | 3.41 ± 0.26 | 1.27 ± 0.50** | 0.60 + 0.40** | 0.51 ± 0.27** # | 0.53 ± 0.31 ** ## | 0.49 ± 0.22** ## | 0.61 ± 0.45** # | 0.59 ± 0.35** # | 0.70 ± 0.51** # | 0.95 ± 0.69** # |
| ANC | CNC-G5 | 3.47 ± 0.22 | 3.43 + 0.23 | 3.83 ± 0.21 | 3.66 + 0.29 | 3.82 ± 0.32 | 3.88 ± 0.20 | 4.02 ± 0.30 | 4.17 ± 0.30 | 4.02 ± 0.35 | 4.04 ± 0.35 | - |

| | | | | | | | | | | | | |
|---|---|---|---|---|---|---|---|---|---|---|---|---|
| Note: "-" indicates termination of measurement; "/" indicates that the C207S-DV32SPG5 group was selected for challenge on D56. Compared with the PBS group, * indicates *P* < 0.05; ** indicates *P* < 0.01. Compared with the positive control group VIR-2218-GL, ^{#} indicates *P* < 0.05; ^{##} indicates *P* < 0.01. | | | | | | | | | | | | |

**Table 51-2: Changes in plasma HBV DNA levels in mice from each sequence group**

| Basic seq ID | Seq ID | **Log₁₀ HBV DNA (IU/mL)** | | | | | | | | | | |
|---|---|---|---|---|---|---|---|---|---|---|---|---|
| | | **D-7** | **DO** | **D7** | **D14** | **D21** | **D28** | **D35** | **D42** | **D49** | **D56** | **D63** |
| | PBS | 6.76 ± 0.75 | 6.25 ± 0.61 | 6.98 ± 0.54 | 7.22 ± 0.35 | 7.40 ± 0.30 | 7.55 ± 0.17 | 7.79 ± 0.24 | 7.64 ± 0.11 | 7.84 ± 0.16 | 8.01 ± 0.28 | 7.78 ± 0.90 |
| APC | VIR-2218-GL | 6.93 ± 0.30 | 6.47 ± 0.63 | 5.22 ± 0.36** | 4.66 ± 0.33** | 4.75 ± 0.53** | 4.72 ± 0.69* | 4.64 ± 0.76** | 5.45 ± 0.64* | 5.54 ± 0.48 | 5.69 ± 0.69 | 6.44 ± 0.69 |
| A1550 | C1550-DV27PG5 | 7.01 ± 0.47 | 7.12 ± 0.33 | 6.92 ± 0.41 | 6.20 ± 0.67 | 5.94 ± 0.80 | 6.04 ± 0.99 | 6.21 ± 1.00 | 6.79 ± 0.69 | - | - | - |
| B1572 | C1572-DV27PG5 | 6.83 ± 0.50 | 6.30 ± 0.78 | 6.10 ± 1.16 | 5.86 ± 1.03 | 5.90 ± 0.92 | 5.82 ± 1.19 | 6.09 ± 1.31 | 6.69 ± 1.16 | - | - | - |
| A1573 | C1573-DV29PG5 | 7.14 ± 0.22 | 6.63 ± 0.78 | 6.56 ± | 6.43 ± 0.69 | 6.37 ± 0.38 | 6.37 ± 0.32 | 6.59 ± 0.4 | 6.63 ± 0.67 | - | - | - |
| B1575 | C1575-DV27PG5 | 6.98 ± 0.39 | 6.81 ± 0.50 | 5.53 ± 0.44** | 5.18 ± 0.49 | 5.08 ± 0.51* | 5.34 ± 0.30 | 5.73 ± 0.42* | 5.93 ± 0.33 | 6.19 + 0.40 | 6.39 ± 0.71 | 6.04 ± 0.81 |
| B1575 | C1575-DV29PG5 | 6.94 ± 0.29 | 6.65 ± 0.27 | 5.96 ± 0.53* | 5.59 ± 0.55 | 5.59 ± 0.75 | 5.99 ± 0.70 | 5.96 ± 0.43 | 6.46 ± 0.61 | - | - | - |
| B207S | C207S-DV29SPG5 | 6.96 ± 0.34 | 7.14 ± 0.46 | 5.61 ± 0.56** | 3.76 ± 0.19** | 3.54 ± 0.00**^{# #} | 3.54 ± 0.00**^{# #} | 3.54 ± 0.00**^{# #} | 3.54 ± 0.00**^{# #} | 3.54 ± 0.00**^{# #} | 3.54 ± 0.00**^{# #} | 3.54 ± 0.00**^{# #} |
| B207S | C207S-DV32SPG5 | 6.96 ± 0.39 | 6.65 ± 0.50 | 5.92 ± 0.38* | 3.84 ± 0.45** | 3.54 ± 0.00**^{# #} | 3.54 ± 0.00**^{# #} | 3.54 ± 0.00**^{# #} | 3.54 ± 0.00**^{# #} | 3.54 ± 0.00**^{# #} | 3.54 ± 0.00**^{# #} | / |
| A416 | C416-DV27Pd7B G5 | 6.99 ± 0.40 | 6.90 ± 0.40 | 5.94 ± 0.51* | 5.16 ± 0.39 | 5.14 ± 0.59* | 5.21 ± 0.49* | 6.10 ± 0.39 | 6.73 ± 0.42 | - | - | - |
| B418S | C418S-DV34SPd7 BG5 | 6.94 ± 0.52 | 6.95 ± 0.50 | 5.47 ± 0.38** | 4.38 ± 0.52** | 4.23 ± 0.24** | 3.72 ± 0.44**^{#} | 3.70 ± 0.32** | 4.04 ± 0.59** | 4.03 ± 0.52* | 4.39 ± 0.41* | 4.78 ± 0.74* |
| ANC | CNC-G5 | 6.76 ± 0.89 | 6.65 ± 0.90 | 7.14 ± 0.63 | 7.33 ± 0.56 | 7.41 ± 0.54 | 7.53 ± 0.44 | 7.48 ± 0.51 | 7.79 ± 0.67 | 7.66 ± 0.47 | 7.77 ± 0.70 | / |

| | | | | | | | | | | | | |
|---|---|---|---|---|---|---|---|---|---|---|---|---|
| Note: "-" indicates termination of measurement; "/" indicates that the C207S-DV32SPG5 group was selected for challenge on D56. Compared with the PBS group, * indicates *P* < 0.05; ** indicates *P* < 0.01. Compared with the positive control group VIR-2218-GL, ^{#} indicates *P* < 0.05; ^{##} indicates *P* < 0.01. | | | | | | | | | | | | |

**Table 51-3: Changes in plasma HBeAg levels in mice from each sequence group**

| Basic seq ID | Seq ID | **Log₁₀ HBeAg (S/CO)** | | | | | | | | | | |
|---|---|---|---|---|---|---|---|---|---|---|---|---|
| | | **D-7** | **D0** | **D7** | **D14** | **D21** | **D28** | **D35** | **D42** | **D49** | **D56** | **D63** |
| / | PBS | 3.76 ± 0.06 | 3.76 ± 0.06 | 3.40 ± 0.17 | 3.72 ± 0.05 | 3.63 ± 0.08 | 3.70 ± 0.06 | 3.74 ± 0.05 | 3.75 ± 0.06 | 3.75 ± 0.04 | 3.66 ± 0.06 | 3.66 ± 0.10 |
| APC | VIR-2218-GL | 3.80 ± 0.08 | 3.76 ± 0.05 | 2.80 ± 0.28** | 2.96 ± 0.27** | 2.79 ± 0.25** | 2.90 ± 0.24** | 3.09 ± 0.26* | 3.18 ± 0.22* | 3.27 ± 0.20** | 3.24 ± 0.22* | 3.36 ± 0.20 |
| A1550 | C1550-DV27PG5 | 3.77 ± 0.06 | 3.72 ± 0.09 | 3.00 ± 0.31 | 3.26 ± 0.19 | 3.09 ± 0.18** | 3.22 ± 0.19 | 3.34 ± 0.18 | 3.37 ± 0.17 | - | - | - |
| B1572 | C1572-DV27PG5 | 3.74 ± 0.08 | 3.75 ± 0.06 | 3.25 ± 0.31 | 3.33 ± 0.28 | 3.12 ± 0.26* | 3.25 ± 0.26 | 3.33 ± 0.26 | 3.39 ± 0.27 | - | - | - |
| A1573 | C1573-DV29PG5 | 3.78 ± 0.04 | 3.75 ± 0.09 | 3.33 ± 0.18 | 3.51 ± 0.07 | 3.34 ± 0.07** | 3.46 ± 0.05 | 3.56 ± 0.03 | 3.52 ± 0.06 | - | - | - |
| B1575 | C1575-DV27PG5 | 3.77 ± 0.05 | 3.71 ± 0.05 | 2.69 ± 0.33** | 2.78 ± 0.26** | 2.68 ± 0.14** | 2.87 ± 0.17** | 3.04 ± 0.17** | 3.14 ± 0.14** | 3.20 ± 0.10** | 3.24 ± 0.12** | 3.30 ± 0.07 |
| B1575 | C1575-DV29PG5 | 3.76 ± 0.07 | 3.73 ± 0.04 | 2.95 ± 0.23 | 3.07 ± 0.26** | 2.98 ± 0.24** | 3.17 ± 0.20 | 3.25 ± 0.17 | 3.32 ± 0.13 | - | - | - |
| B207S | C207S-DV29SPG5 | 3.75 ± 0.03 | 3.71 ± 0.09 | 2.99 ± 0.16 | 3.02 ± 0.07** | 2.75 ± 0.12** | 2.76 ± 0.10** | 2.73 ± 0.08** ^{##} | 2.73 ± 0.09** ^{##} | 2.64 ± 0.13** ^{##} | 2.59 ± 0.11** ^{##} | 2.61 ± 0.14**^{# #} |
| B207S | C207S-DV32SPG5 | 3.75 ± 0.03 | 3.75 ± 0.05 | 2.79 ± 0.23** | 2.83 ± 0.11** | 2.50 ± 0.19** ^{#} | 2.54 ± 0.11** ^{##} | 2.57 ± 0.09** ^{##} | 2.61 ± 0.10** ^{##} | 2.56 ± 0.09** ^{##} | 2.47 ± 0.09** ^{##} | / |
| A416 | C416-DV27Pd7B G5 | 3.75 ± 0.05 | 3.77 ± 0.06 | 3.25 ± 0.14 | 3.49 ± 0.09 | 3.33 ± 0.06** | 3.51 ± 0.06 | 3.61 ± 0.06 | 3.67 ± 0.04 | - | - | - |
| B418S | C418S-DV34SPd7 BG5 | 3.70 + 0.10 | 3.70 + 0.09 | 3.17 + 0.18 | 3.29 + 0.11 | 3.09 + 0.15** | 2.94 + 0.22** | 3.02 + 0.22** | 3.01 ± 0.23** | 2.94 + 0.24** # | 2.89 + 0.24** ## | 2.90 + 0.18**^{,} # |
| ANC | CNC-G5 | 3.76 + 0.07 | 3.71 + 0.07 | 3.32 ± 0.14 | 3.66 + 0.06 | 3.70 + 0.06 | 3.69 + 0.05 | 3.76 + 0.09 | 3.74 + 0.08 | 3.71 ± 0.08 | 3.64 + 0.06 | / |

| | | | | | | | | | | | | |
|---|---|---|---|---|---|---|---|---|---|---|---|---|
| Note: "-" indicates termination of measurement; "/" indicates that the C207S-DV32SPG5 group was selected for challenge on D56. Compared with the PBS group, * indicates *P* < 0.05; ** indicates *P* < 0.01. Compared with the positive control group VIR-2218-GL, ^{#} indicates P < 0.05; ^{##} indicates *P* < 0.01. | | | | | | | | | | | | |

**Table 51-4: Changes in the number of mice with HBsAg clearance in each sequence group**

| Basic sequence ID | Sequence ID | Number of mice with HBsAg clearance | | | | | | | | |
|---|---|---|---|---|---|---|---|---|---|---|
| | | D7 | D14 | D21 | D28 | D35 | D42 | D49 | D56 | D63 |
| B418S | C418S-DV34SPd7BG5 | 0/6 | 4/6 | 5/6 | 5/6 | 5/6 | 4/6 | 4/6 | 4/6 | 3/6 |
| B207S | C207S-DV32SPG5 | 0/6 | 6/6 | 6/6 | 6/6 | 6/6 | 6/6 | 6/6 | 6/6 | 6/6 |
| B1575 | C1575-DV27PG5 | 0/6 | 0/6 | 0/6 | 0/6 | 0/6 | 0/6 | 0/6 | 0/6 | 0/6 |
| B207S | C207S-DV29SPG5 | 0/6 | 5/6 | 5/6 | 5/6 | 5/6 | 5/6 | 6/6 | 5/6 | 6/6 |
| APC | VIR-2218-GL | 0/6 | 0/6 | 0/6 | 0/6 | 0/6 | 0/6 | 0/6 | 0/6 | 0/6 |
| A416 | C416-DV27Pd7BG5 | 0/6 | 0/6 | 0/6 | 0/6 | 0/6 | 0/6 | NA | NA | NA |
| B1575 | C1575-DV29PG5 | 0/6 | 0/6 | 0/6 | 0/6 | 0/6 | 0/6 | NA | NA | NA |
| B1572 | C1572-DV27PG5 | 0/6 | 0/6 | 0/6 | 0/6 | 0/6 | 0/6 | NA | NA | NA |
| A1550 | C1550-DV27PG5 | 0/6 | 0/6 | 0/6 | 0/6 | 0/6 | 0/6 | NA | NA | NA |
| A1573 | C1573-DV29PG5 | 0/6 | 0/6 | 0/6 | 0/6 | 0/6 | 0/6 | NA | NA | NA |
| ANC | CNC-G5 | 0/6 | 0/6 | 0/6 | 0/6 | 0/6 | 0/6 | 0/6 | 0/6 | 0/6 |
| | PBS | 0/6 | 0/6 | 0/6 | 0/6 | 0/6 | 0/6 | 0/6 | 0/6 | 0/6 |

**Table 51-5: Changes in the number of mice with HBV DNA clearance in each sequence group**

| Basic sequence ID | Sequence ID | Number of mice with HBV DNA clearance | | | | | | | | |
|---|---|---|---|---|---|---|---|---|---|---|
| | | D7 | D14 | D21 | D28 | D35 | D42 | D49 | D56 | D63 |
| B418S | C418S-DV34SPd7BG5 | 0/6 | 0/6 | 0/6 | 5/6 | 4/6 | 2/6 | 0/6 | 0/6 | 0/6 |
| B207S | C207S-DV32SPG5 | 0/6 | 3/6 | 6/6 | 6/6 | 6/6 | 6/6 | 6/6 | 6/6 | 2/6 |
| B1575 | C1575-DV27PG5 | 0/6 | 0/6 | 0/6 | 0/6 | 0/6 | 0/6 | 0/6 | 0/6 | 0/6 |
| B207S | C207S-DV29SPG5 | 0/6 | 1/6 | 6/6 | 6/6 | 6/6 | 6/6 | 6/6 | 6/6 | 6/6 |
| APC | VIR-2218-GL | 0/6 | 0/6 | 0/6 | 1/6 | 1/6 | 0/6 | 0/6 | 0/6 | 0/6 |
| A416 | C416-DV27Pd7BG5 | 0/6 | 0/6 | 0/6 | 0/6 | 0/6 | 0/6 | NA | NA | NA |
| B1575 | C1575-DV29PG5 | 0/6 | 0/6 | 0/6 | 0/6 | 0/6 | 0/6 | NA | NA | NA |
| B1572 | C1572-DV27PG5 | 0/6 | 0/6 | 0/6 | 1/6 | 0/6 | 0/6 | NA | NA | NA |
| A1550 | C1550-DV27PG5 | 0/6 | 0/6 | 0/6 | 0/6 | 0/6 | 0/6 | NA | NA | NA |
| A1573 | C1573-DV29PG5 | 0/6 | 0/6 | 0/6 | 0/6 | 0/6 | 0/6 | NA | NA | NA |
| ANC | CNC-G5 | 0/6 | 0/6 | 0/6 | 0/6 | 0/6 | 0/6 | 0/6 | 0/6 | 0/6 |
| | PBS | 0/6 | 0/6 | 0/6 | 0/6 | 0/6 | 0/6 | 0/6 | 0/6 | 0/6 |

**Table 51-6: Changes in the number of mice producing antibodies in each sequence group**

| Basic sequence ID | Sequence ID | Number of mice producing antibodies | | | | | | | | |
|---|---|---|---|---|---|---|---|---|---|---|
| | | D7 | D14 | D21 | D28 | D35 | D42 | D49 | D56 | D63 |
| B418S | C418S-DV34SPd7BG5 | 2/6 | 4/6 | 4/6 | 4/6 | 6/6 | 6/6 | 6/6 | 6/6 | 4/6 |
| B207S | C207S-DV32SPG5 | 2/6 | 2/6 | 3/6 | 3/6 | 4/6 | 4/6 | 4/6 | 5/6 | 5/6 |
| B1575 | C1575-DV27PG5 | 0/6 | 1/6 | 2/6 | 0/6 | 0/6 | 2/6 | 2/6 | 2/6 | 2/6 |
| B207S | C207S-DV29SPG5 | 1/6 | 4/6 | 1/6 | 2/6 | 3/6 | 5/6 | 5/6 | 4/6 | 5/6 |
| APC | VIR-2218-GL | 2/6 | 3/6 | 3/6 | 3/6 | 3/6 | 3/6 | 2/6 | 2/6 | 1/6 |
| A416 | C416-DV27Pd7BG5 | 2/6 | 2/6 | 3/6 | 2/6 | 0/6 | 0/6 | NA | NA | NA |
| B1575 | C1575-DV29PG5 | 0/6 | 0/6 | 0/6 | 0/6 | 0/6 | 0/6 | NA | NA | NA |
| B1572 | C1572-DV27PG5 | 0/6 | 0/6 | 0/6 | 0/6 | 0/6 | 0/6 | NA | NA | NA |
| A1550 | C1550-DV27PG5 | 0/6 | 0/6 | 0/6 | 0/6 | 0/6 | 0/6 | NA | NA | NA |
| A1573 | C1573-DV29PG5 | 0/6 | 0/6 | 0/6 | 0/6 | 0/6 | 1/6 | NA | NA | NA |
| ANC | CNC-G5 | 0/6 | 0/6 | 0/6 | 0/6 | 0/6 | 0/6 | 0/6 | 0/6 | 0/6 |
| | PBS | 0/6 | 0/6 | 0/6 | 0/6 | 0/6 | 0/6 | 0/6 | 0/6 | 0/6 |

**Table 51-7: Changes in plasma HBsAb levels in mice from each sequence group**

| Basic sequence ID | Sequence ID | HBsAb (mIU/mL) | | | | | | | | |
|---|---|---|---|---|---|---|---|---|---|---|
| | | D7 | D14 | D21 | D28 | D35 | D42 | D49 | D56 | D63 |
| B418S | C418S-DV34SPd7BG5 | 35 | 117 | 257 | 180 | 145 | 191 | 311 | 293 | 363 |
| B207S | C207S-DV32SPG5 | 52 | 88 | 69 | 65 | 84 | 111 | 237 | 383 | 597 |
| B1575 | C1575-DV27PG5 | 30 | 30 | 34 | 30 | 30 | 33 | 34 | 49 | 37 |
| B207S | C207S-DV29SPG5 | 37 | 39 | 31 | 31 | 64 | 119 | 179 | 172 | 264 |
| APC | VIR-2218-GL | 31 | 92 | 226 | 306 | 362 | 203 | 161 | 142 | 97 |
| A416 | C416-DV27Pd7BG5 | 33 | 36 | 39 | 34 | 30 | 30 | NA | NA | NA |
| B1575 | C1575-DV29PG5 | 30 | 30 | 30 | 30 | 30 | 30 | NA | NA | NA |
| B1572 | C1572-DV27PG5 | 30 | 30 | 30 | 30 | 30 | 30 | NA | NA | NA |
| A1550 | C1550-DV27PG5 | 30 | 30 | 30 | 30 | 30 | 30 | NA | NA | NA |
| A1573 | C1573-DV29PG5 | 30 | 30 | 30 | 30 | 30 | 31 | NA | NA | NA |
| ANC | CNC-G5 | 30 | 30 | 30 | 30 | 30 | 30 | 30 | 30 | 30 |
| | PBS | 30 | 30 | 30 | 30 | 30 | 30 | 30 | 30 | 30 |

**Table 51-8: Changes in plasma ALT levels in mice from each sequence group**

| Basic sequence ID | Sequence ID | ALT (U/L) | | | | | | | | |
|---|---|---|---|---|---|---|---|---|---|---|
| | | D7 | D14 | D21 | D28 | D35 | D42 | D49 | D56 | D63 |
| B418S | C418S-DV34SPd7BG5 | 75 | 83 | 138 | 75 | 75 | 80 | 75 | 80 | 75 |
| B207S | C207S-DV32SPG5 | 75 | 78 | 75 | 78 | 75 | 78 | 75 | 75 | 95 |
| B1575 | C1575-DV27PG5 | 75 | 75 | 75 | 75 | 75 | 75 | 75 | 75 | 78 |
| B207S | C207S-DV29SPG5 | 75 | 75 | 75 | 98 | 75 | 78 | 223 | 75 | 75 |
| APC | VIR-2218-GL | 75 | 75 | 85 | 75 | 75 | 75 | 98 | 105 | 103 |
| A416 | C416-DV27Pd7BG5 | 75 | 75 | 75 | 75 | 75 | 75 | NA | NA | NA |
| B1575 | C1575-DV29PG5 | 75 | 75 | 75 | 75 | 75 | 75 | NA | NA | NA |
| B1572 | C1572-DV27PG5 | 75 | 75 | 75 | 83 | 75 | 75 | NA | NA | NA |
| A1550 | C1550-DV27PG5 | 75 | 80 | 88 | 83 | 75 | 75 | NA | NA | NA |
| A1573 | C1573-DV29PG5 | 75 | 75 | 75 | 75 | 75 | 75 | NA | NA | NA |
| ANC | CNC-G5 | 75 | 75 | 75 | 75 | 75 | 75 | 75 | 75 | 83 |
| | PBS | 75 | 75 | 75 | 75 | 75 | 75 | 75 | 75 | 78 |

**Table 51-9: Changes in body weight of mice from each sequence group**

| Basic sequence ID | Sequence ID | Body weight (g) | | | | | | | | |
|---|---|---|---|---|---|---|---|---|---|---|
| | | D7 | D14 | D21 | D28 | D35 | D42 | D49 | D56 | D63 |
| B418S | C418S-DV34SPd7BG5 | 28.9 | 29.2 | 28.8 | 28.8 | 29.1 | 29.9 | 30.3 | 30.5 | 29.8 |
| B207S | C207S-DV32SPG5 | 29.2 | 29.7 | 29.9 | 30.0 | 30.2 | 30.8 | 31.0 | 31.2 | 31.2 |
| B1575 | C1575-DV27PG5 | 27.7 | 28.3 | 28.2 | 28.4 | 28.7 | 28.6 | 29.0 | 29.0 | 28.6 |
| B207S | C207S-DV29SPG5 | 27.4 | 27.6 | 27.7 | 27.6 | 27.7 | 28.1 | 28.3 | 28.5 | 28.1 |
| APC | VIR-2218-GL | 28.4 | 28.9 | 29.0 | 28.6 | 29.4 | 29.6 | 30.0 | 30.2 | 29.9 |
| A416 | C416-DV27Pd7BG5 | 29.7 | 30.6 | 30.0 | 30.0 | 29.8 | 30.8 | NA | NA | NA |
| B1575 | C1575-DV29PG5 | 29.4 | 30.0 | 30.0 | 29.4 | 29.8 | 29.8 | NA | NA | NA |
| B1572 | C1572-DV27PG5 | 29.6 | 29.9 | 30.2 | 30.1 | 29.2 | 29.2 | NA | NA | NA |
| A1550 | C1550-DV27PG5 | 28.1 | 28.7 | 28.5 | 29.0 | 29.2 | 29.2 | NA | NA | NA |
| A1573 | C1573-DV29PG5 | 27.5 | 27.6 | 27.3 | 27.3 | 27.0 | 27.6 | NA | NA | NA |
| ANC | CNC-G5 | 28.4 | 29.0 | 29.2 | 28.6 | 28.9 | 29.4 | 29.5 | 29.7 | 29.8 |
| | PBS | 29.2 | 29.8 | 30.2 | 30.0 | 30.2 | 30.2 | 31.0 | 32.3 | 32.4 |

### (ii) Protective effect of sequence CS7S-DV32PG5 against secondary challenge in mice

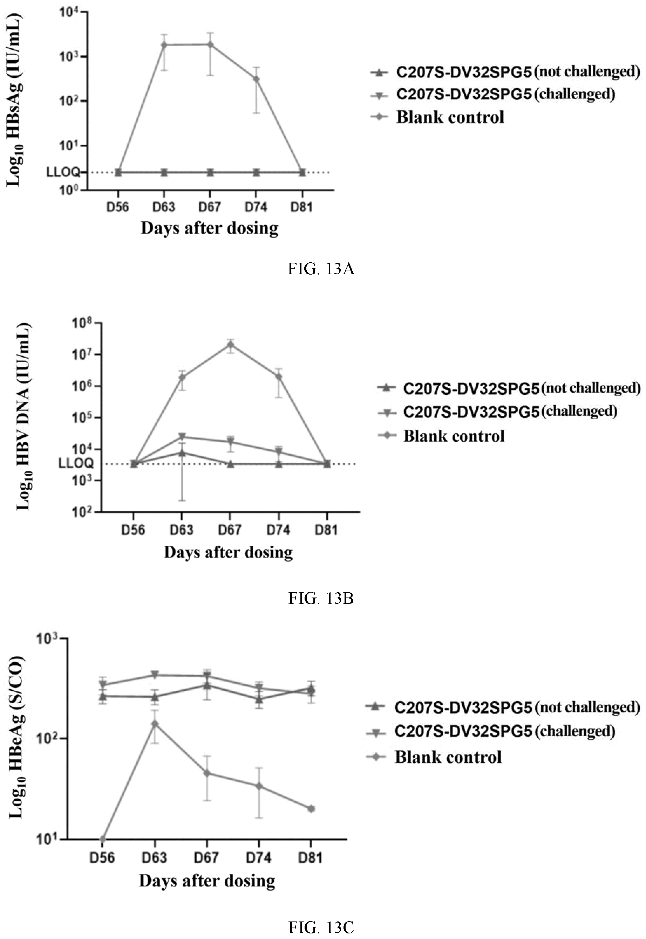

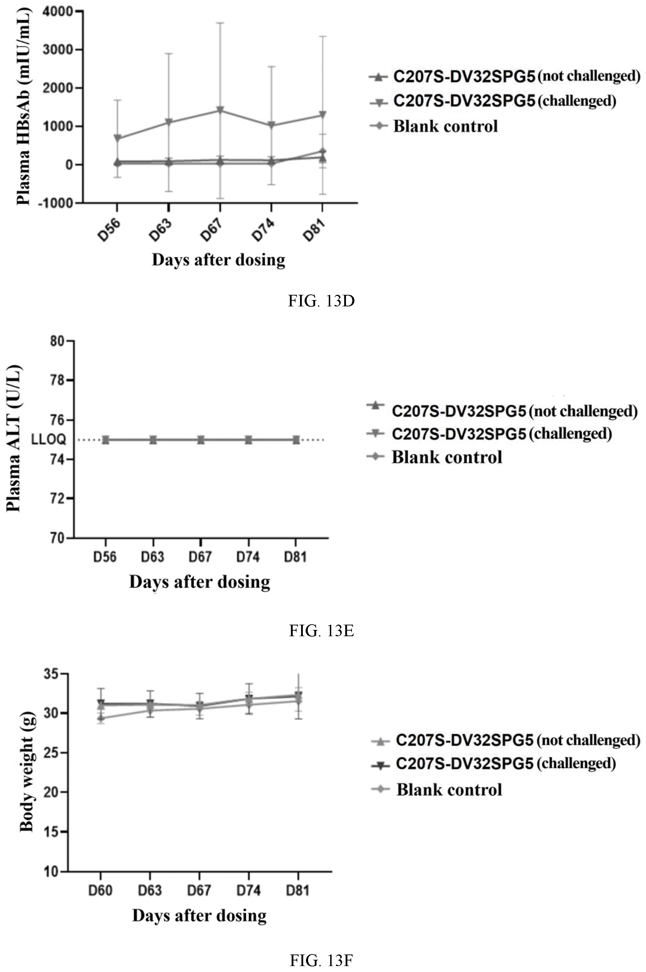

Mice in the CS7S-DV32SPG5 group were subjected to hydrodynamic tail vein injection of an HBV plasmid on D60 to simulate secondary HBV infection. The experimental results, as shown in FIGs. 13A to 13F, demonstrated that the blank control group (which had not been infected with HBV prior to D56) exhibited the presence of HBsAg, HBeAg, and HBV DNA after hydrodynamic injection of the HBV plasmid, indicating that the challenge was effective. In the challenged mice of the C207S-DV32SPG5 group, HBV DNA levels initially increased after challenge and then decreased to the detection limit, while serum HBsAb levels increased significantly and HBsAg levels remained below the detection limit, indicating that the challenge was effective and that HBV-infected mice treated with the oligonucleotide sequence were protected from reinfection by HBV.

### Example 15: Inhibition of HBV in mice by sequences modified using the templates disclosed herein (low dose)

In this example, several sequences were exemplarily selected, such as the sequences C207S-DV32SPG5, C207S-DV29SPG5, C418S-DV34SPd7BG5, and C1575-DV27PG5 from Example 14, as shown in Table 49. The *in vivo* efficacy of these RNAi agents at a low dose (*e.g.*, 0.5 mg/kg) was analyzed using an AAV-HBV mouse model.

### 1. Experimental materials

The sequences C207S-DV32SPG5, C207S-DV29SPG5, C418S-DV34SPd7BG5, C1575-DV27PG5, and VIR-2218-GL from Example 14, as shown in Table 50, along with entecavir (ETV).

### 2. Experimental methods

Refer to Example 7. The details of grouping and dosing are shown in Table 51-10.

**Table 51-10: Details of grouping and dosing**

| Group | Dosing regimen | Route of administration | Number of animals |
|---|---|---|---|
| siRNA (6 groups) | 0.5 mg/kg, D0/7/14 | s.c. | 6 per group × 6 |
| ETV (1 group) | 0.1 mg/kg, daily | i.g. | 6 per group × 1 |
| Vehicle control (1 group) | PBS, once on D0/7/14 | s.c. | 6 per group × 1 |

| | | | |
|---|---|---|---|
| Note: s.c.: subcutaneous injection; i.g.: intragastric administration. | | | |

### 3. Experimental results

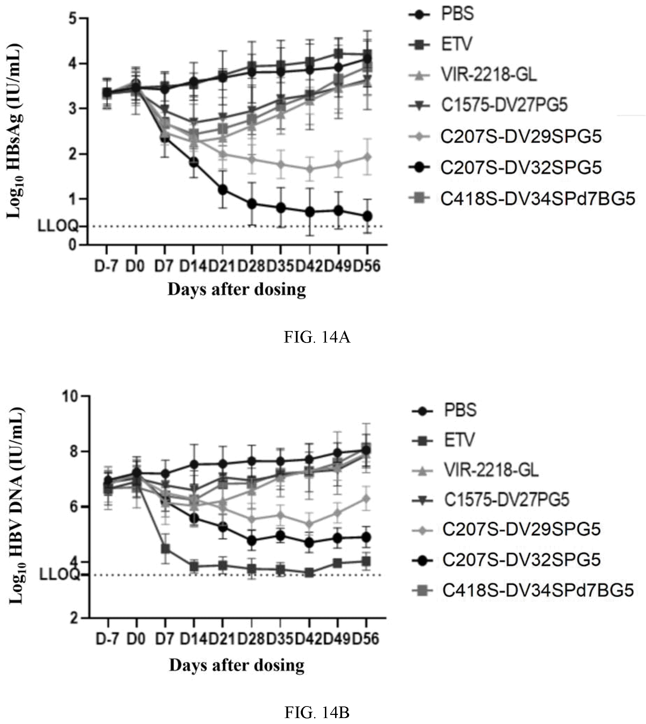

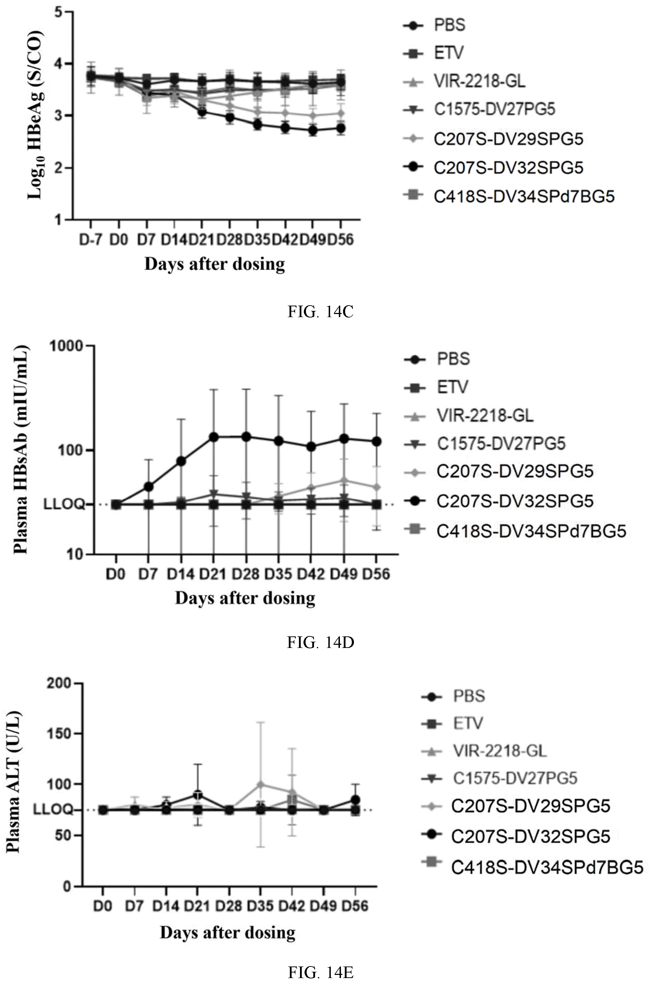

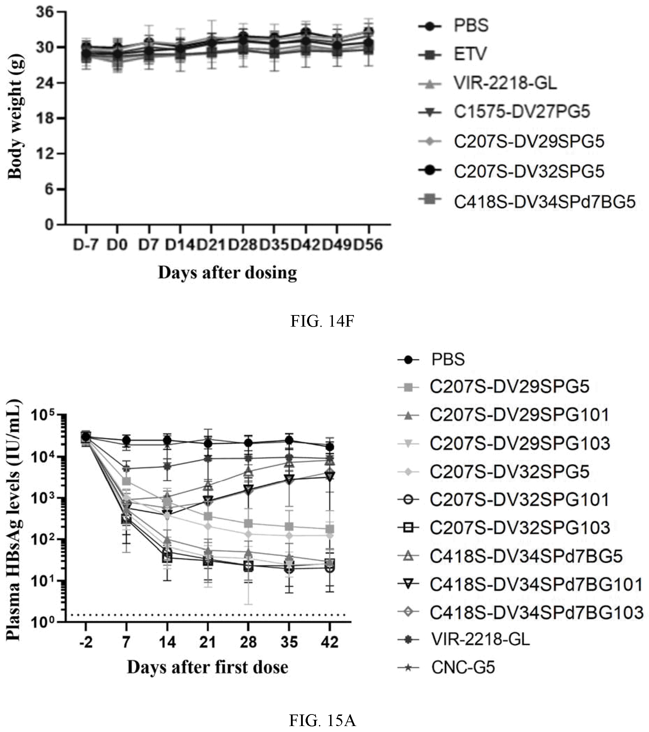

The changes over time in HBsAg, HBeAg, HBV DNA, HBsAb, and ALT levels in each group are shown in Tables 51-11 to 51-18 and FIGs. 14A to 14F. The experimental results demonstrated that the sequences C207S-DV32SPG5 and C207S-DV29SPG5 exhibited significantly superior efficacy in inhibiting HBsAg, HBV DNA, and HBeAg compared to the positive reference sequence VIR-2218-GL. Specifically, C207S-DV32SPG5 exhibited the best efficacy, reducing HBsAg by approximately 2.9 log₁₀ on Day 56, which was approximately 1.4 log₁₀ higher than that of C207S-DV29SPG5. Furthermore, on Day 56, HBsAg clearance was observed in 3 mice, and production of antibodies was observed in 4 mice in the C207S-DV32SPG5 group, whereas no clearance was observed in the C207S-DV29SPG5 group. These data further demonstrate the superior efficacy of the sequence C207S-DV32SPG5.

**Table 51-11: Changes in plasma HBsAg levels in mice from each sequence group**

| Basic seq ID | Seq ID | **Log₁₀ HBsAg (IU/mL)** | | | | | | | | | |
|---|---|---|---|---|---|---|---|---|---|---|---|
| | | **D-7** | **D0** | **D7** | **D14** | **D21** | **D28** | **D35** | **D42** | **D49** | **D56** |
| / | PBS | 3.36 ± 0.30 | 3.46 ± 0.27 | 3.43 ± 0.36 | 3.61 ± 0.41 | 3.69 ± 0.59 | 3.81 ± 0.68 | 3.81 ± 0.70 | 3.86 ± 0.67 | 3.92 ± 0.65 | 4.10 ± 0.62 |
| / | ETV | 3.36 ± 0.26 | 3.47 ± 0.34 | 3.50 ± 0.33 | 3.54 ± 0.25 | 3.74 ± 0.14 | 3.94 ± 0.27 | 3.96 ± 0.39 | 4.04 ± 0.40 | 4.22 ± 0.33 | 4.20 ± 0.30 |
| APC | VIR-2218-GL | 3.33 ± 0.33 | 3.60 ± 0.24 | 2.47 ± 0.27** | 2.26 ± 0.45* | 2.36 ± 0.42** | 2.62 ± 0.28 | 2.88 ± 0.34 | 3.18 ± 0.29 | 3.46 ± 0.47 | 3.59 ± 0.30 |
| B1575 | C1575-DV27PG5 | 3.34 ± 0.34 | 3.40 ± 0.52 | 2.96 ± 0.49 | 2.69 ± 0.57 | 2.80 ± 0.62* | 2.95 ± 0.52 | 3.21 ± 0.65 | 3.23 ± 0.92 | 3.46 ± 0.68 | 3.64 ± 0.66 |
| B207S | C207S-DV29SPG5 | 3.33 ± 0.33 | 3.51 ± 0.30 | 2.70 ± 0.22** | 2.36 ± 0.29* | 2.00 ± 0.33** | 1.88 ± 0.32 | 1.77 ± 0.31* | 1.67 ± 0.27* | 1.77 ± 0.29* | 1.94 ± 0.40 |
| B207S | C207S-DV32SPG5 | 3.33 ± 0.32 | 3.55 ± 0.34 | 2.37 ± 0.43** | 1.82 ± 0.34** | 1.22 ± 0.41** | 0.90 ± 0.47** | 0.82 ± 0.44** | 0.79 ± 0.67** | 0.75 ± 0.41** | 0.63 ± 0.37** |
| B418S | C418S-DV34SPd7B G5 | 3.33 ± 0.33 | 3.40 ± 0.32 | 2.68 ± 0.40** | 2.43 ± 0.66* | 2.56 ± 0.69** | 2.76 ± 0.77 | 3.06 ± 0.63 | 3.30 ± 0.65 | 3.65 ± 0.75 | 3.93 ± 0.61 |

| | | | | | | | | | | | |
|---|---|---|---|---|---|---|---|---|---|---|---|
| Note: Compared with the PBS group, * indicates *P* < 0.05; ** indicates *P* < 0.01. | | | | | | | | | | | |

**Table 51-12: Changes in plasma HBV DNA levels in mice from each sequence group**

| Basic seq ID | Seq ID | **Log₁₀ HBV DNA (IU/mL)** | | | | | | | | | |
|---|---|---|---|---|---|---|---|---|---|---|---|
| | | **D-7** | **D0** | **D7** | **D14** | **D21** | **D28** | **D35** | **D42** | **D49** | **D56** |
| / | PBS | 6.97 ± 0.28 | 7.22 ± 0.41 | 7.20 ± 0.49 | 7.54 ± 0.72 | 7.55 ± 0.63 | 7.65 ± 0.57 | 7.65 ± 0.46 | 7.72 ± 0.56 | 7.96 ± 0.35 | 8.05 ± 0.57 |
| / | ETV | 6.65 ± 0.58 | 6.91 ± 0.58 | 4.49 ± 0.54** | 3.85 ± 0.24** | 3.89 ± 0.31** | 3.77 ± 0.37** | 3.74 ± 0.25** | 3.63 ± 0.08** | 3.97 ± 0.13** | 4.04 ± 0.32** |
| APC | VIR-2218-GL | 6.81 ± 0.63 | 7.20 ± 0.15 | 6.16 ± 0.78 | 6.03 ± 0.76 | 6.22 ± 0.88 | 6.59 ± 0.46 | 7.07 ± 0.31 | 7.31 ± 0.27 | 7.43 ± 0.47 | 7.92 ± 0.29 |
| B1575 | C1575-DV27PG5 | 6.87 ± 0.43 | 7.04 ± 0.66 | 6.78 ± 0.48 | 6.60 ± 0.70 | 7.07 ± 0.40 | 6.95 ± 0.44 | 7.15 ± 0.52 | 7.09 ± 1.14 | 7.34 ± 0.58 | 7.86 ± 0.44 |
| B207S | C207S-DV29SPG5 | 6.98 ± 0.23 | 7.07 ± 0.39 | 6.51 ± 0.25 | 6.29 ± 0.33 | 5.95 ± 0.19 | 5.56 ± 0.36 | 5.70 ± 0.37 | 5.38 ± 0.40 | 5.78 ± 0.37 | 6.31 ± 0.44 |
| B207S | C207S-DV32SPG5 | 6.83 ± 0.42 | 7.23 ± 0.58 | 6.20 ± 0.35 | 5.59 ± 0.28 | 5.29 ± 0.43 | 4.79 ± 0.36 | 4.97 ± 0.26 | 4.83 ± 0.57 | 4.88 ± 0.36* | 4.91 ± 0.38* |
| B418S | C418S-DV34SPd7B G5 | 6.68 ± 0.78 | 6.71 ± 0.75 | 6.39 ± 0.75 | 6.25 ± 0.88 | 6.84 ± 0.67 | 6.85 ± 0.91 | 7.21 ± 0.84 | 7.28 ± 1.00 | 7.58 ± 1.12 | 8.12 ± 0.89 |

| | | | | | | | | | | | |
|---|---|---|---|---|---|---|---|---|---|---|---|
| Note: Compared with the PBS group, * indicates *P* < 0.05; ** indicates *P* < 0.01. | | | | | | | | | | | |

**Table 51-13: Changes in plasma HBeAg levels in mice from each sequence group**

| Basic seq ID | Seq ID | **Log₁₀ HBeAg (S/CO)** | | | | | | | | | |
|---|---|---|---|---|---|---|---|---|---|---|---|
| | | **D-7** | **D0** | **D7** | **D14** | **D21** | **D28** | **D35** | **D42** | **D49** | **D56** |
| / | PBS | 3.76 ± 0.17 | 3.73 + 0.10 | 3.61 ± 0.07 | 3.69 + 0.13 | 3.67 + 0.14 | 3.69 + 0.14 | 3.66 + 0.15 | 3.65 ± 0.13 | 3.62 + 0.19 | 3.24 ± 1.01 |
| / | ETV | 3.77 + 0.06 | 3.73 + 0.06 | 3.72 + 0.05 | 3.72 ± 0.03 | 3.66 + 0.05 | 3.71 + 0.06 | 3.66 + 0.05 | 3.68 + 0.05 | 3.69 + 0.08 | 3.70 + 0.04 |
| APC | VIR-2218-GL | 3.78 ± 0.11 | 3.77 + 0.13 | 3.35 ± 0.15* | 3.39 + 0.15 | 3.32 ± 0.19 | 3.39 + 0.19 | 3.46 ± 0.17 | 3.52 + 0.12 | 3.60 + 0.13 | 3.59 + 0.13 |
| B1575 | C1575-DV27PG5 | 3.75 ± 0.19 | 3.68 + 0.13 | 3.48 ± 0.12 | 3.51 ± 0.18 | 3.43 + 0.20 | 3.50 + 0.15 | 3.51 ± 0.17 | 3.37 ± 0.38 | 3.60 + 0.16 | 3.59 + 0.20 |
| B207S | C207S-DV29SPG5 | 3.78 + 0.06 | 3.70 + 0.07 | 3.50 ± 0.13 | 3.47 ± 0.09 | 3.31 ± 0.12 | 3.20 ± 0.13 | 3.07 + 0.11 | 3.06 + 0.15 | 3.00 + 0.14 | 3.05 ± 0.18 |
| B207S | C207S-DV32SPG5 | 3.77 + 0.04 | 3.72 + 0.04 | 3.45 ± 0.04** | 3.40 ± 0.12 | 3.08 + 0.13 | 2.97 + 0.12 | 2.84 + 0.10* | 2.94 + 0.35 | 2.73 + 0.11* | 2.77 ± 0.13 |
| B418S | C418S-DV34SPd7BG5 | 3.74 + 0.30 | 3.66 + 0.26 | 3.38 + 0.33 | 3.49 ± 0.31 | 3.46 ± 0.30 | 3.55 ± 0.33 | 3.49 ± 0.34 | 3.51 ± 0.31 | 3.52 ± 0.32 | 3.59 + 0.28 |

| | | | | | | | | | | | |
|---|---|---|---|---|---|---|---|---|---|---|---|
| Note: Compared with the PBS group, * indicates P < 0.05; ** indicates P < 0.01. | | | | | | | | | | | |

**Table 51-14: Number of mice with plasma HBsAg clearance in each sequence group**

| Basic sequence ID | Sequence ID | Number of mice with HBsAg clearance | | | | | | | |
|---|---|---|---|---|---|---|---|---|---|
| | | D7 | D14 | D21 | D28 | D35 | D42 | D49 | D56 |
| B207S | C207S-DV32SPG5 | 0 | 0 | 0 | 1 | 1 | 2 | 1 | 3 |
| B207S | C207S-DV29SPG5 | 0 | 0 | 0 | 0 | 0 | 0 | 0 | 0 |
| B418S | C418S-DV34SPd7BG5 | 0 | 0 | 0 | 0 | 0 | 0 | 0 | 0 |
| B1575 | C1575-DV27PG5 | 0 | 0 | 0 | 0 | 0 | 0 | 0 | 0 |
| APC | VIR-2218-GL | 0 | 0 | 0 | 0 | 0 | 0 | 0 | 0 |
| / | ETV | 0 | 0 | 0 | 0 | 0 | 0 | 0 | 0 |
| / | PBS | 0 | 0 | 0 | 0 | 0 | 0 | 0 | 0 |

**Table 51-15: Number of mice with plasma HBV DNA clearance in each sequence group**

| Basic sequence ID | Sequence ID | Number of mice with HBV DNA clearance | | | | | | | |
|---|---|---|---|---|---|---|---|---|---|
| | | D7 | D14 | D21 | D28 | D35 | D42 | D49 | D56 |
| B207S | C207S-DV32SPG5 | 0 | 0 | 0 | 0 | 0 | 0 | 0 | 0 |
| B207S | C207S-DV29SPG5 | 0 | 0 | 0 | 0 | 0 | 0 | 0 | 0 |
| B418S | C418S-DV34SPd7BG5 | 0 | 0 | 0 | 0 | 0 | 0 | 0 | 0 |
| B1575 | C1575-DV27PG5 | 0 | 0 | 0 | 0 | 0 | 0 | 0 | 0 |
| APC | VIR-2218-GL | 0 | 0 | 0 | 0 | 0 | 0 | 0 | 0 |
| / | ETV | 1 | 1 | 1 | 3 | 3 | 2 | 0 | 0 |
| / | PBS | 0 | 0 | 0 | 0 | 0 | 0 | 0 | 0 |

**Table 51-16: Number of mice producing HBsAb in each sequence group**

| Basic sequence ID | Sequence ID | Number of mice producing HBsAb | | | | | | | |
|---|---|---|---|---|---|---|---|---|---|
| | | D7 | D14 | D21 | D28 | D35 | D42 | D49 | D56 |
| B207S | C207S-DV32SPG5 | 1 | 1 | 2 | 3 | 4 | 4 | 4 | 4 |
| B207S | C207S-DV29SPG5 | 0 | 0 | 0 | 0 | 2 | 3 | 3 | 2 |
| B418S | C418S-DV34SPd7BG5 | 0 | 0 | 0 | 0 | 0 | 0 | 0 | 0 |
| B1575 | C1575-DV27PG5 | 0 | 1 | 1 | 1 | 1 | 1 | 1 | 0 |
| APC | VIR-2218-GL | 0 | 0 | 0 | 0 | 0 | 0 | 0 | 0 |
| / | ETV | 0 | 0 | 0 | 0 | 0 | 0 | 0 | 0 |
| / | PBS | 0 | 0 | 0 | 0 | 0 | 0 | 0 | 0 |

**Table 51-17: Changes in plasma HBsAb levels in mice from each sequence group**

| Basic sequence ID | Sequence ID | HBsAb (mIU/mL) | | | | | | | |
|---|---|---|---|---|---|---|---|---|---|
| | | D7 | D14 | D21 | D28 | D35 | D42 | D49 | D56 |
| B207S | C207S-DV32SPG5 | 45 | 79 | 135 | 136 | 124 | 109 | 130 | 122 |
| B207S | C207S-DV29SPG5 | 30 | 30 | 30 | 30 | 36 | 44 | 52 | 44 |
| B418S | C418S-DV34SPd7BG5 | 30 | 30 | 30 | 30 | 30 | 30 | 30 | 30 |
| B1575 | C1575-DV27PG5 | 30 | 32 | 38 | 36 | 33 | 34 | 35 | 30 |
| APC | VIR-2218-GL | 30 | 30 | 30 | 30 | 30 | 30 | 30 | 30 |
| | ETV | 30 | 30 | 30 | 30 | 30 | 30 | 30 | 30 |
| | PBS | 30 | 30 | 30 | 30 | 30 | 30 | 30 | 30 |

**Table 51-18: Changes in plasma ALT levels in mice from each sequence group**

| Basic sequence ID | Sequence ID | ALT (U/L) | | | | | | | |
|---|---|---|---|---|---|---|---|---|---|
| | | D7 | D14 | D21 | D28 | D35 | D42 | D49 | D56 |
| B207S | C207S-DV32SPG5 | 75 | 80 | 90 | 75 | 78 | 75 | 75 | 85 |
| B207S | C207S-DV29SPG5 | 75 | 78 | 80 | 75 | 100 | 93 | 75 | 75 |
| B418S | C418S-DV34SPd7BG5 | 75 | 75 | 75 | 75 | 75 | 85 | 75 | 75 |
| B1575 | C1575-DV27PG5 | 75 | 75 | 75 | 75 | 75 | 75 | 75 | 75 |
| APC | VIR-2218-GL | 80 | 75 | 75 | 75 | 75 | 75 | 75 | 75 |
| | ETV | 75 | 75 | 75 | 75 | 75 | 75 | | |
| | PBS | 75 | 75 | 75 | 75 | 75 | 75 | | |

### Example 16: Inhibition of HBV in mice by sequences modified using the delivery ligands disclosed herein

In this example, several sequences were exemplarily selected, including basic sequences B418S and B207S. These sequences were modified, for example, by incorporating template modification alone, or both template modification and anti-off-target design, and were further conjugated with different delivery ligands. The *in vivo* efficacy of these RNAi agents was analyzed using an AAV-HBV mouse model.

### 1. Experimental materials

### Test compounds:

The sequences used in this example, including those incorporating template modification alone and those incorporating both template modification and anti-off-target design, wherein the 3' end of the sense strand of these sequences is conjugated with delivery ligands G5, G101, or G103, with the structural formula as follows:

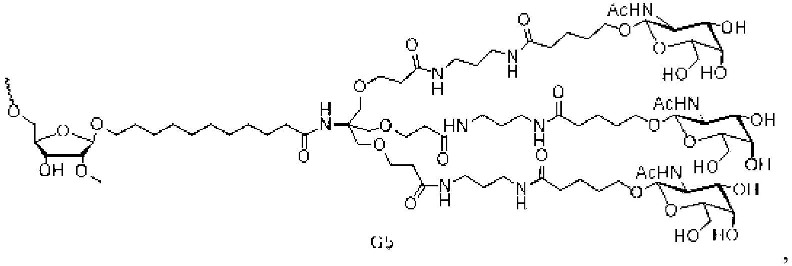

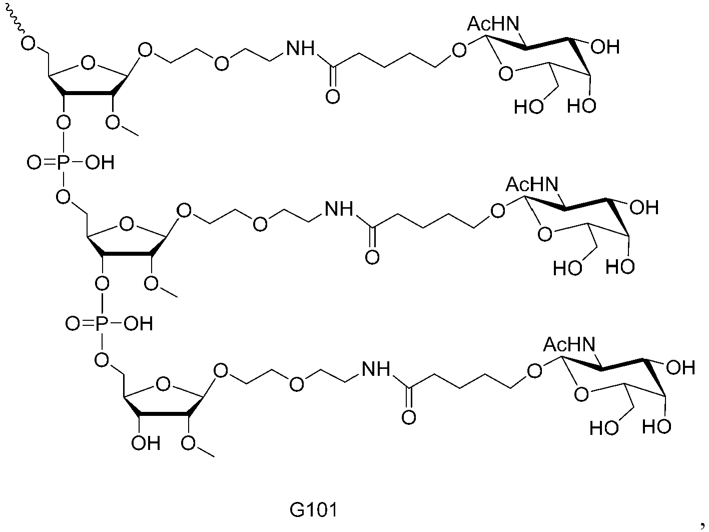

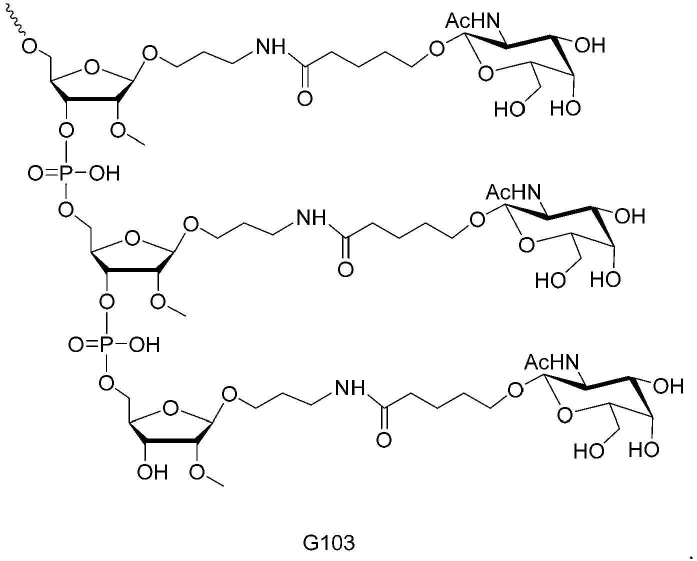

The conjugation method between oligonucleotides and ligands G5, G101, or G103 was the same as that described in Example 3 of patent application CN116854754A.

The resulting oligonucleotide-ligand (G5, G101, or G103) conjugate is shown below:

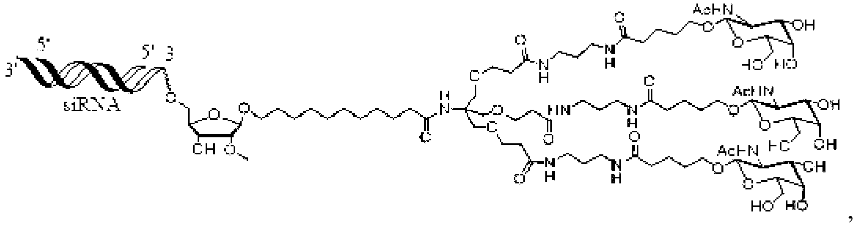

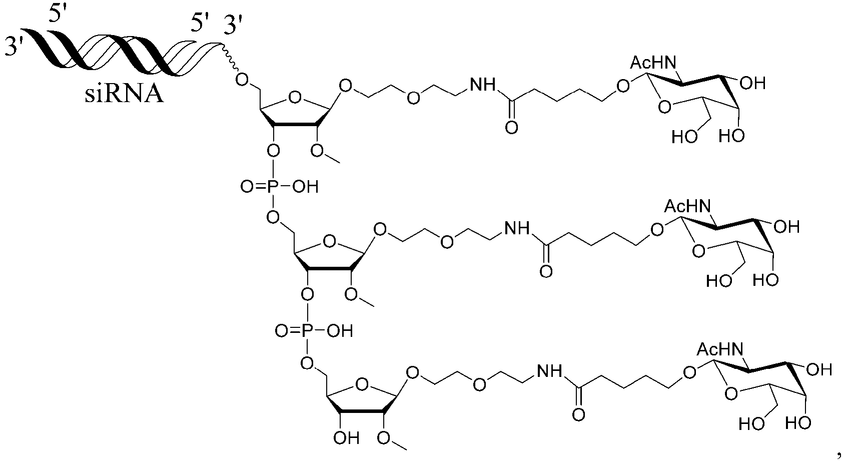

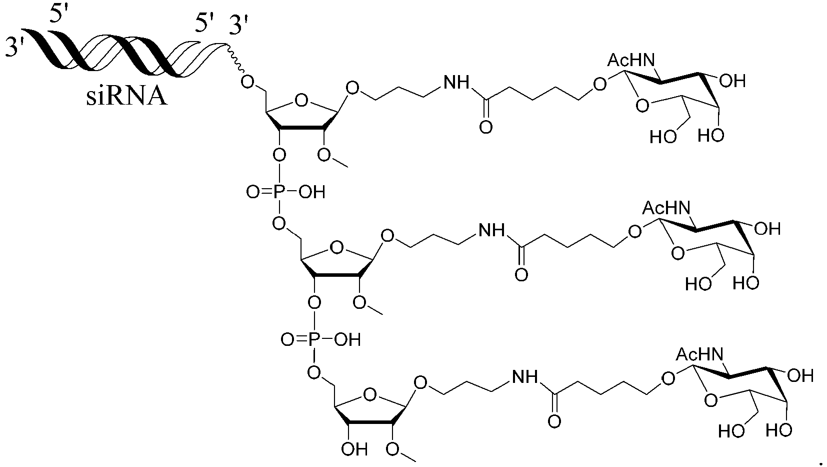

In this example, six siRNA sequences were designed for the basic sequence B207S based on the modification templates DV25SP to DV34SP, including: C207S-DV29SPG5, C207S-DV29SPG101, and C207S-DV29SPG103 designed based on D29SP, as well as C207S-DV32SPG5, C207S-DV32SPG101, and C207S-DV32SPG103 designed based on D32SP (see Table 50 for the sequences of C207S-DV29SP and C207S-DV32SP). For the basic sequence B418S, three siRNA sequences, namely C418S-DV34SPd7BG5, C418S-DV34SPd7BG101, and C418S-DV34SPd7BG103, were designed based on D34SP (see Table 50 for the sequence of C418S-DV34SPd7B). In this example, VIR-2218-GL based on the positive control sequence APC and CNC-G5 based on the basic sequence ANC (see Table 50) were also employed. In sequence identifiers, G5 indicates conjugation with delivery ligand G5, G101 indicates conjugation with delivery ligand G101, G103 indicates conjugation with delivery ligand G103, and GL indicates conjugation with delivery ligand L96. The structural formula of L96 is as follows:

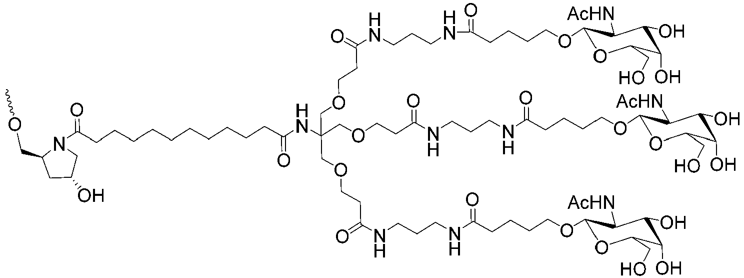

### Preparation of test compounds:

Refer to Example 14.

### Information on experimental animals:

Species/strain: AAV-HBV mice
Grade: SPF
Sex: Male
Quantity: 82
Age: 5 weeks
Weight: 19 to 24 g
Source: Beijing Vital River Laboratory Animal Technology Co., Ltd.
Production license No.: SCXK (Jing) 2021-0006

### Review by Institutional Animal Care and Use Committee (IACUC):

Upon arrival, the experimental animals were housed at Beijing Vitalstar Biotechnology Co., Ltd., under a project license No.: SCXK (Jing) 2022-0013. The project was approved by the IACUC of Beijing Vitalstar Biotechnology Co., Ltd, with IACUC approval No.: VST-SY-24062701. The experimental procedures were strictly conducted in compliance with IACUC requirements to ensure animal welfare.

### Housing and management:

The animals were housed in a negative-pressure barrier environment using plastic (polycarbonate) IVCs (dimensions: 370 × 157 × 180 mm). Due to the aggressive nature of male animals, they were housed individually. The housing was managed by Beijing Vitalstar Biotechnology Co., Ltd.

The use and testing of animal feed, bedding, and drinking water were conducted in accordance with GB14925-2010 ("Laboratory animal - Environment and housing facilities"). Environmental control parameters, including temperature, humidity, pressure differential, noise, illumination, air exchange rate, and ammonia concentration, were maintained in accordance with GB50447-2008 ("Architectural and technical code for laboratory animal facility"). The environmental parameters for the animal facility were set as follows: a room temperature ranging from 20°C to 26°C (with a daily fluctuation of 4°C or less); a relative humidity ranging from 40% to 70%; and a 12-hour light/12-hour dark cycle with artificial lighting. Compressed wood shaving bedding was purchased from Beijing Keao Xieli Feed Co., Ltd. (batch No.: 23109613). Growth and reproduction feed for mice and rats was purchased from Beijing Keao Xieli Feed Co., Ltd. (batch No.: 23103313). All records related to animal housing and management as well as environmental control during the study were maintained by Beijing Vitalstar Biotechnology Co., Ltd.

### 2. Experimental methods

### 2.1 Establishment of AAV-HBV mouse model

Definition of trial date: The day on which the animals were administered solvent or test compound was defined as Day 0.

A total of 100 SPF-grade C57BL/6 male mice were acclimatized for 7 days in a barrier facility and monitored daily to confirm health status before model establishment. A mouse model of persistent HBV infection was established by tail vein injection of rAAV8-1.3HBV at a dose of 1.00 × 10¹⁰ vg/mouse. The AAV was diluted with sterile PBS to 5.00 × 10¹⁰ vg/mL, and each mouse was injected with 200 µL. Four weeks after virus injection, serum HBV DNA, HBeAg, and HBsAg levels were measured.

### 2.2 Animal grouping and dosing

Definition of trial date: The day on which the animals were administered solvent or test compound was defined as Day 0 (D0).

On Day -2, blood was collected from all mice via submandibular venipuncture. The collected blood samples were anticoagulated with EDTA, centrifuged at 5000 rpm for 10 minutes, and the supernatant was used for confirming HBV model establishment. On Day 0, 72 mice were selected based on the test results from Day -2 and divided into 12 groups, with 6 mice per group. The details of grouping and dosing are shown in Table 51-19.

**Table 51-19: Details of grouping and dosing**

| Group | Dosing regimen | Route of administration | Number of animals |
|---|---|---|---|
| siRNA (11 groups) | 3 mg/kg, D0 | s.c. | 6 per group × 11 |
| Vehicle control (1 group) | PBS, D0 | s.c. | 6 per group × 1 |

| | | | |
|---|---|---|---|
| Note: s.c.: subcutaneous injection. | | | |

### 2.4 Observation and measurement

### (1) General observations

Daily monitoring during modeling and trial periods, including mortality or moribund status, food and water intake, injuries, fecal characteristics, physical appearance and coat condition, mental state, activity levels, *etc*.

### (2) Body weight

Acclimation: Animals were weighed and recorded upon arrival and at the end of acclimation.

Trial period: Animals were weighed and recorded weekly during the trial period. If dosing or blood collection was scheduled for the same day, weighing was performed prior to the procedure and prior to euthanasia.

### (3) Measurement of serum HBsAg, HBeAg, HBV DNA, and ALT levels

Blood was collected from all animals via submandibular venipuncture on Days -2, 6, 13, 20, 27, and 34. The collected blood samples were anticoagulated with EDTA and centrifuged at 5000 rpm for 10 minutes to obtain serum. After blood collection, the serum was separated, diluted with PBS, and sent for analysis. A 10 µL aliquot of serum was taken from each sample, diluted to 500 µL with PBS (50-fold dilution), and sent to Beijing DIAN Medical Laboratory Co., Ltd. for analysis of serum HBV DNA, HBeAg, and HBsAg. A 30 µL aliquot of serum was taken from each sample, diluted to 120 µL with PBS (4-fold dilution), and sent to Beijing DIAN Medical Laboratory Co., Ltd. for analysis of serum ALT. The remaining plasma was stored at -80°C.

### 3. Experimental results

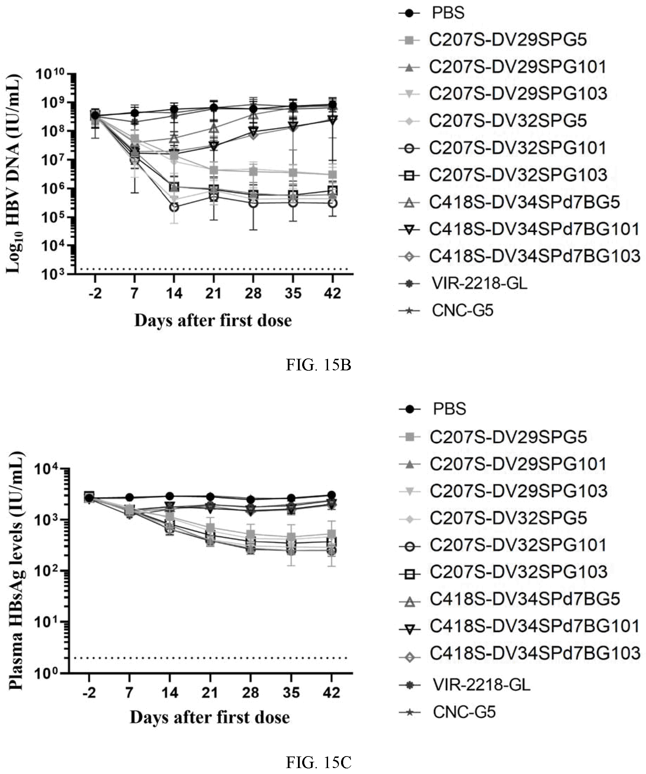

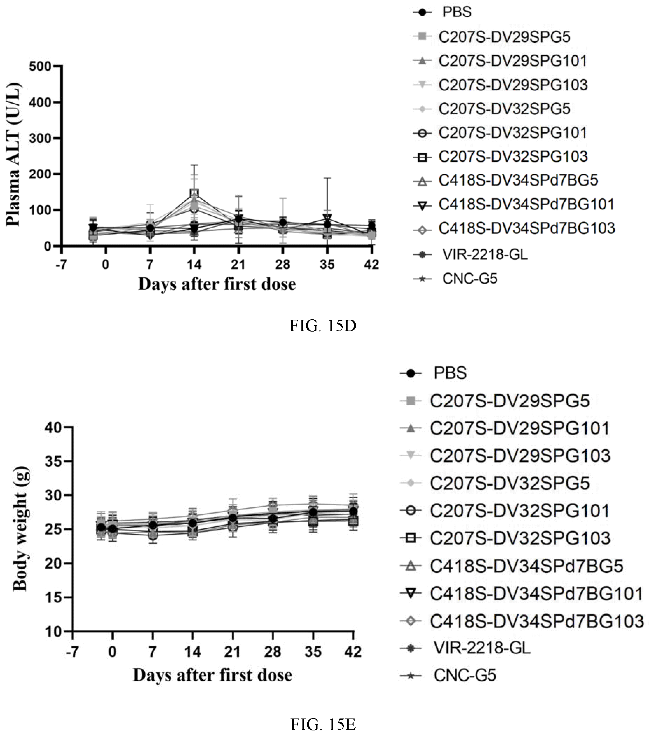

The changes over time in HBsAg, HBeAg, HBV DNA, ALT, and body weight in each group are shown in Tables 51-20 to 51-24 and FIGs. 15A to 15E. The experimental results demonstrated that all sequences exhibited superior efficacy compared to the positive control sequence VIR-2218-GL. Specifically, C207S-DV29SPG101, C207S-DV29SPG103, C207S-DV32SPG101, and C207S-DV32SPG103 demonstrated superior efficacy in reducing HBsAg, HBV DNA, and HBeAg levels, significantly outperforming the positive control sequence VIR-2218-GL. On Day 28, these sequences still reduced HBsAg by more than 3 log₁₀ and HBV DNA by more than 3 log₁₀, with no rebound observed, demonstrating excellent anti-HBV efficacy.

**Table 51-20: Changes in plasma HBsAg levels in mice from each sequence group**

| Basic sequence ID | Sequence ID | **Log₁₀ HBsAg (IU/mL)** | | | | | | |
|---|---|---|---|---|---|---|---|---|
| | | **D-2** | **D7** | **D14** | **D21** | **D28** | **D35** | **D42** |
| / | PBS | 4.47 ± 0.12 | 4.38 ± 0.15 | 4.36 ± 0.21 | 4.26 ± 0.27 | 4.3 ± 0.21 | 4.37 ± 0.17 | 4.22 ± 0.16 |
| B207S | C207S-DV29SPG5 | 4.46 ± 0.13 | 3.11 ± 0.67 | 2.54 ± 0.79 | 2.16 ± 0.8 | 1.94 ± 0.9 | 1.78 ± 0.95 | 1.56 ± 0.98 |
| B207S | C207S-DV29SPG101 | 4.43 ± 0.16 | 2.65 ± 0.32 | 1.8 ± 0.59 | 1.47 ± 0.7 | 1.44 ± 0.69 | 1.19 ± 0.77 | 1 ± 0.83 |
| B207S | C207S-DV29SPG103 | 4.46 ± 0.15 | 2.54 ± 0.37 | 1.66 ± 0.47 | 1.36 ± 0.58 | 1.22 ± 0.71 | 1.04 ± 0.71 | 1.02 ± 0.73 |
| B207S | C207S-DV32SPG5 | 4.45 ± 0.15 | 2.86 ± 0.49 | 2.34 ± 0.7 | 2.08 ± 0.66 | 1.88 ± 0.74 | 1.75 ± 0.83 | 1.56 ± 1 |
| B207S | C207S-DV32SPG101 | 4.46 ± 0.12 | 2.5 ± 0.2 | 1.66 ± 0.22 | 1.47 ± 0.31 | 1.27 ± 0.39 | 1.12 ± 0.51 | 1.19 ± 0.43 |
| B207S | C207S-DV32SPG103 | 4.42 ± 0.22 | 2.25 ± 0.64 | 1.33 ± 0.64 | 1.3 ± 0.59 | 1.21 ± 0.53 | 1.18 ± 0.55 | 1.18 ± 0.62 |
| B418S | C418S-DV34SPd7BG5 | 4.43 ± 0.15 | 2.92 ± 0.19 | 2.99 ± 0.19 | 3.23 ± 0.31 | 3.58 ± 0.31 | 3.83 ± 0.22 | 3.81 ± 0.42 |
| B418S | C418S-DV34SPd7BG103 | 4.46 ± 0.11 | 2.68 ± 0.55 | 2.51 ± 0.56 | 2.69 ± 0.51 | 2.94 ± 0.6 | 3.26 ± 0.46 | 3.52 ± 0.35 |
| APC | VIR-2218-GL | 4.46 ± 0.14 | 3.66 ± 0.24 | 3.71 ± 0.23 | 3.84 ± 0.37 | 3.86 ± 0.39 | 3.87 ± 0.41 | 3.82 ± 0.45 |
| ANC | CNC-G5 | 4.44 ± 0.21 | 4.27 ± 0.13 | 4.21 ± 0.3 | 4.33 ± 0.32 | 4.25 ± 0.3 | 4.26 ± 0.4 | 4.22 ± 0.33 |

**Table 51-21: Changes in plasma HBV DNA levels in mice from each sequence group**

| Basic sequence ID | Sequence ID | **Log₁₀ HBV DNA (IU/mL)** | | | | | | |
|---|---|---|---|---|---|---|---|---|
| | | **D-2** | **D7** | **D14** | **D21** | **D28** | **D35** | **D42** |
| / | PBS | 8.47 ± 0.27 | 8.58 ± 0.21 | 8.68 ± 0.28 | 8.71 ± 0.38 | 8.57 ± 0.53 | 8.73 ± 0.46 | 8.7 ± 0.64 |
| B207S | C207S-DV29SPG5 | 8.47 ± 0.23 | 7.46 ± 0.67 | 6.74 ± 0.97 | 6.32 ± 0.77 | 5.97 ± 1.43 | 6.01 ± 1.09 | 5.69 ± 1.16 |
| B207S | C207S-DV29SPG101 | 8.5 ± 0.17 | 7.21 ± 0.32 | 5.78 ± 0.51 | 5.53 ± 0.85 | 5.3 ± 0.94 | 5.33 ± 0.69 | 4.91 ± 1.22 |
| B207S | C207S-DV29SPG103 | 8.51 ± 0.25 | 6.8 ± 0.32 | 5.41 ± 0.51 | 5.48 ± 0.8 | 4.97 ± 1.14 | 4.98 ± 1.17 | 5.09 ± 1.09 |
| B207S | C207S-DV32SPG5 | 8.49 ± 0.21 | 7.5 ± 0.5 | 6.7 ± 0.74 | 6.24 ± 0.88 | 6.39 ± 0.78 | 6.29 ± 0.8 | 6.15 ± 0.78 |
| B207S | C207S-DV32SPG101 | 8.47 0.27 ± | 6.88 ± 0.47 | 5.1 ± 0.5 | 5.3 ± 1.07 | 5.06 ± 1 | 5.24 ± 0.7 | 5.29 ± 0.61 |
| B207S | C207S-DV32SPG103 | 8.49 0.28 ± | 6.84 ± 0.64 | 5.64 ± 0.73 | 5.31 ± 0.91 | 5.41 ± 0.67 | 5.25 ± 1.1 | 5.51 ± 0.86 |
| B418S | C418S-DV34SPd7BG5 | 8.5 ± 0.15 | 7.57 ± 0.18 | 7.68 ± 0.26 | 8.02 ± 0.34 | 8.48 ± 0.37 | 8.77 ± 0.22 | 8.78 ± 0.47 |
| B418S | C418S-DV34SPd7BG101 | 8.53 ± 0.12 | 7.17 ± 0.27 | 6.89 ± 0.69 | 7.12 ± 0.73 | 7.66 ± 0.67 | 7.94 ± 0.5 | 8.07 ± 0.68 |
| B418S | C418S-DV34SPd7BG103 | 8.53 ± 0.2 | 7.13 ± 0.44 | 7.01 ± 0.58 | 7.18 ± 0.66 | 7.56 ± 0.65 | 7.94 ± 0.47 | 8.29 ± 0.38 |
| APC | VIR-2218-GL | 8.42 ± 0.3 | 8.24 ± 0.27 | 8.43 ± 0.31 | 8.62 ± 0.41 | 8.57 ± 0.47 | 8.49 ± 0.66 | 8.57 ± 0.57 |
| ANC | CNC-G5 | 8.43 ± 0.29 | 8.58 ± 0.29 | 8.55 ± 0.29 | 8.76 ± 0.21 | 8.83 ± 0.31 | 8.72 ± 0.41 | 8.79 ± 0.33 |

**Table 51-22: Changes in plasma HBeAg levels in mice from each sequence group**

| Basic sequence ID | Sequence ID | **Log₁₀ HBeAg (IU/mL)** | | | | | | |
|---|---|---|---|---|---|---|---|---|
| | | **D-2** | **D7** | **D14** | **D21** | **D28** | **D35** | **D42** |
| / | PBS | 3.43 ± 0.06 | 3.44 ± 0.02 | 3.46 ± 0.03 | 3.45 ± 0.03 | 3.39 ± 0.06 | 3.43 ± 0.04 | 3.49 ± 0.03 |
| B207S | C207S-DV29SPG5 | 3.42 ± 0.03 | 3.18 ± 0.1 | 3.04 ± 0.17 | 2.81 ± 0.21 | 2.67 ± 0.24 | 2.59 ± 0.27 | 2.65 ± 0.28 |
| B207S | C207S-DV29SPG101 | 3.43 ± 0.02 | 3.18 ± 0.04 | 2.87 ± 0.14 | 2.58 ± 0.11 | 2.43 ± 0.1 | 2.39 ± 0.07 | 2.39 ± 0.1 |
| B207S | C207S-DV29SPG103 | 3.44 ± 0.04 | 3.22 ± 0.06 | 2.82 ± 0.08 | 2.61 ± 0.08 | 2.47 ± 0.1 | 2.46 ± 0.06 | 2.45 ± 0.06 |
| B207S | C207S-DV32SPG5 | 3.44 ± 0.03 | 3.24 ± 0.04 | 3.03 ± 0.09 | 2.77 ± 0.14 | 2.63 ± 0.12 | 2.6 ± 0.14 | 2.65 ± 0.15 |
| B207S | C207S-DV32SPG101 | 3.44 ± 0.03 | 3.17 ± 0.06 | 2.81 ± 0.1 | 2.58 ± 0.07 | 2.42 ± 0.06 | 2.39 ± 0.06 | 2.4 ± 0.06 |
| B207S | C207S-DV32SPG103 | 3.46 ± 0.03 | 3.19 ± 0.07 | 2.91 ± 0.1 | 2.69 ± 0.13 | 2.57 ± 0.12 | 2.52 ± 0.13 | 2.56 ± 0.12 |
| B418S | C418S-DV34SPd7BG5 | 3.43 ± 0.03 | 3.18 ± 0.09 | 3.25 ± 0.06 | 3.3 ± 0.04 | 3.24 ± 0.06 | 3.3 ± 0.06 | 3.38 ± 0.04 |
| B418S | C418S-DV34SPd7BG101 | 3.41 ± 0.05 | 3.19 ± 0.07 | 3.25 ± 0.09 | 3.22 ± 0.07 | 3.18 ± 0.07 | 3.2 ± 0.08 | 3.3 ± 0.06 |
| B418S | C418S-DV34SPd7BG103 | 3.43 ± 0.02 | 3.17 ± 0.03 | 3.21 ± 0.04 | 3.26 ± 0.06 | 3.16 ± 0.08 | 3.19 ± 0.1 | 3.28 ± 0.08 |
| APC | VIR-2218-GL | 3.4 ± 0.07 | 3.09 ± 0.07 | 3.2 ± 0.09 | 3.29 ± 0.05 | 3.25 ± 0.08 | 3.27 ± 0.1 | 3.35 ± 0.17 |
| ANC | CNC-G5 | 3.42 ± 0.03 | 3.42 ± 0.04 | 3.46 ± 0.05 | 3.47 ± 0.04 | 3.42 ± 0.05 | 3.41 ± 0.05 | 3.47 ± 0.04 |

**Table 51-23: Changes in plasma ALT levels in mice from each sequence group**

| Basic sequence ID | Sequence ID | ALT (U/L) | | | | | |
|---|---|---|---|---|---|---|---|
| | | D7 | D14 | D21 | D28 | D35 | D42 |
| B207S | C207S-DV29SPG5 | 30 | 53 | 111 | 68 | 71 | 41 |
| | C207S-DV29SPG101 | 50 | 45 | 129 | 83 | 50 | 61 |
| | C207S-DV29SPG103 | 44 | 63 | 129 | 57 | 49 | 35 |
| | C207S-DV32SPG5 | 51 | 49 | 122 | 71 | 43 | 40 |
| | C207S-DV32SPG101 | 41 | 61 | 103 | 61 | 48 | 43 |
| | C207S-DV32SPG103 | 29 | 45 | 146 | 56 | 49 | 33 |
| B418S | C418S-DV34SPd7BG5 | 34 | 49 | 41 | 48 | 49 | 36 |
| | C418S-DV34SPd7BG101 | 48 | 30 | 48 | 75 | 44 | 76 |
| | C418S-DV34SPd7BG103 | 42 | 30 | 62 | 69 | 41 | 32 |
| APC | VIR-2218-GL | 47 | 35 | 37 | 79 | 50 | 49 |
| CNC | CNC-G5 | 48 | 51 | 60 | 60 | 61 | 58 |
| | PBS | 51 | 53 | 49 | 75 | 65 | 59 |

**Table 51-24: Changes in body weight of mice from each sequence group**

| Basic sequence ID | Sequence ID | Body weight (g) | | | | | |
|---|---|---|---|---|---|---|---|
| | | D7 | D14 | D21 | D28 | D35 | D42 |
| B207S | C207S-DV29SPG5 | 25.8 | 25.6 | 25.6 | 26 | 27.0 | 27.5 |
| | C207S-DV29SPG101 | 24.8 | 24.4 | 24.5 | 24.4 | 25.6 | 26.0 |
| | C207S-DV29SPG103 | 25.7 | 25.4 | 25.2 | 25.5 | 26.6 | 27.0 |
| | C207S-DV32SPG5 | 25.6 | 25.7 | 25.7 | 25.5 | 26.4 | 26.4 |
| | C207S-DV32SPG101 | 24.6 | 24.4 | 24.1 | 24.4 | 25.3 | 26.0 |
| | C207S-DV32SPG103 | 25 | 25 | 24.6 | 24.7 | 25.8 | 26.1 |
| B418S | C418S-DV34SPd7BG5 | 25.6 | 25.7 | 25.8 | 26.2 | 26.6 | 27.1 |
| | C418S-DV34SPd7BG101 | 25.4 | 25.6 | 25.4 | 25.9 | 26.8 | 27.4 |
| | C418S-DV34SPd7BG103 | 26.1 | 26.2 | 26.5 | 27 | 27.8 | 28.5 |
| APC | VIR-2218-GL | 25.5 | 25.9 | 26 | 26.3 | 27.0 | 27.3 |
| CNC | CNC-G5 | 25.4 | 25.5 | 25.5 | 26.4 | 26.9 | 27.1 |
| | PBS | 25.3 | 25.1 | 25.6 | 25.9 | 26.7 | 26.6 |

### Example 17: Synthesis of alternately modified small interfering oligonucleotides

A total of 99 unmodified siRNA sequences were designed based on the AGT mRNA sequence (NM_001384479.1). To enhance the inhibitory efficiency and stability of the sequences, the unmodified sequences were alternately modified with 2'-O-methyl (2'-OMe) and 2'-fluoro (2'-F), with terminal phosphorothioate modifications. In the sense strand of odd-numbered length, all odd-numbered positions are modified with 2'-F, and all even-numbered positions are modified with 2'-OMe. However, in the sense strand of even-numbered length, all odd-numbered positions are modified with 2'-OMe, and all even-numbered positions are modified with 2'-F. In the antisense strand, all odd-numbered positions are modified with 2'-OMe, and all even-numbered positions are modified with 2'-F. In addition, there are two phosphorothioate modifications at the 5' end of the sense strand, while there are two phosphorothioate modifications at both the 5' and 3' ends of the antisense strand. The alternately modified siRNA sequences are shown in Table 52.

### 1. Synthesis of alternately modified sequence B1579-AL

The unmodified siRNA sequence designated as B1579-AL in Table 52 is as follows:
Sense strand: 5'-CCUUUUCUUCUAAUGAGUCGA-3' (SEQ ID NO: 788)
Antisense strand: 5'-UCGACUCAUUAGAAGAAAAGGUG-3' (SEQ ID NO: 801)

Odd-numbered positions of the sense strand and even-numbered positions of the antisense strand are modified with 2'-F, while all other positions are modified with 2'-OMe. In addition, there are two phosphorothioate modifications at the 5' end of the sense strand, while there are two phosphorothioate modifications at both the 5' and 3' ends of the antisense strand.

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-OMe phosphoramidite monomer (Formula 1), DMT-C-OMe phosphoramidite monomer (Formula 2), DMT-G-OMe phosphoramidite monomer (Formula 3), DMT-U-OMe phosphoramidite monomer (Formula 4), DMT-A-F phosphoramidite monomer (Formula 5), DMT-C-F phosphoramidite monomer (Formula 6), DMT-G-F phosphoramidite monomer (Formula 7), and DMT-U-F phosphoramidite monomer (Formula 8).

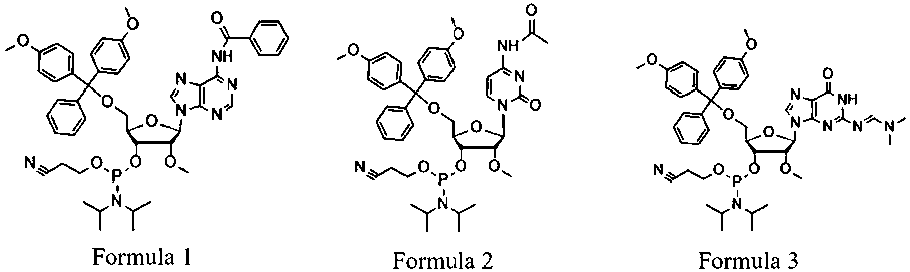

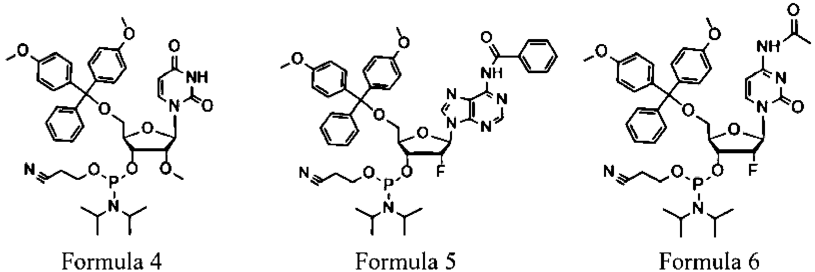

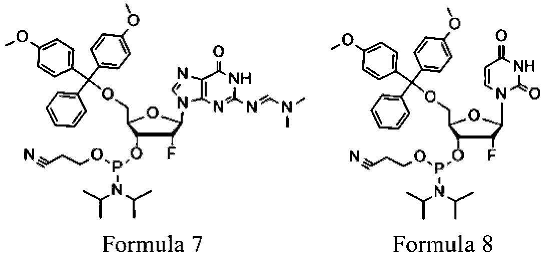

The preparation was carried out according to the following steps:

The solid-phase support was loaded into the designated position of the synthesizer, followed by multiple synthesis cycles to obtain a desired fully protected product, each cycle consisting of (1) deprotection, (2) coupling, (3) oxidation/sulfurization, and (4) hydroxyl protection. The cycle process and reagents used are described as follows:
(1) Deprotection
   3% dichloroacetic acid in toluene was used as a deprotection reagent to remove the DMT protecting group, followed by washing with acetonitrile.
(2) Coupling
   Each nucleotide monomer in acetonitrile was coupled using 0.25 M 5-ethylthio-1*H-*tetrazole as an activator, followed by washing with acetonitrile.
(3) Oxidation/sulfurization
   Oxidation: 0.05 M iodine in pyridine/water (90/10) was used as an oxidant for oxidation, followed by washing with acetonitrile.
   Sulfurization: 3% xanthane hydride in pyridine was used as a sulfurizing agent for sulfurization, followed by washing with acetonitrile.
(4) Hydroxyl protection
   10% acetic anhydride in tetrahydrofuran (CAP A) and tetrahydrofuran/pyridine/*N-*methylimidazole (74/10/16, v/v/v) (CAP B) were used as hydroxyl protecting reagents for hydroxyl protection, followed by washing with acetonitrile.
   The above steps were repeated according to the set sequence to obtain a fully protected product.
(5) 3% dichloroacetic acid in toluene was used as a deprotection reagent to remove the DMT protecting group from the last nucleotide, followed by washing with acetonitrile.
(6) Ammonolysis and purification
   The solid-phase support was transferred to a reactor and treated with concentrated aqueous ammonia (25 to 28%) at 60°C for 12 hours. The system was then cooled to room temperature, and the mixture was transferred to a pressure filter, followed by washing with a mixture of purified water and ethanol. The filtrates were combined, subjected to column chromatography, concentrated, and lyophilized to obtain a product.
(7) Annealing

The purified sense and antisense strands were mixed at a molar ratio of 1:1, heated to 95°C and maintained for 3 minutes, and slowly cooled to room temperature to form a duplex.

B1579-AL exhibited a purity of 90.61% and a measured molecular weight of 14437.86.

### 2. Synthesis of other sequences

All other sequences listed in Table 52 were synthesized using the method described above, resulting in a total of 99 siRNA sequences.

**Table 52: Alternately modified siRNA sequences**

| **Sequence ID** | **Sense sequence 5'-3'** | **SEQ ID NO:/corr. unmodified SEQ ID NO:** | **Antisense sequence 5'-3'** | **SEQ ID NO:/corr. unmodified SEQ ID NO:** |
|---|---|---|---|---|
| B1835-AL | Ufs-Ums-Gf-Gm-Gf-Um-Uf- Um-Uf-Am-Af-Am-Af-Um-Uf- Am-Af-Am-Gf-Um-Af | 983/785 | Ums-Afs-Cm-Uf-Um-Uf-Am-Af- Um-Uf-Um-Uf-Am-Af-Am-Af-Cm- Cf-Cm-Af-Ams-Ufs-Um | 996/798 |
| B1816-AL | Ufs-Ums-Cf-Am-Af-Gm-Uf- Um-Gf-Am-Gf-Am-Af-Cm-Af- Am-Af-Am-Af-Um-Uf | 984/786 | Ams-Afs-Um-Uf-Um-Uf-Um-Gf- Um-Uf-Cm-Uf-Cm-Af-Am-Cf-Um- Uf-Gm-Af-Ams-Afs-Am | 997/799 |
| B1812-AL | Cfs-Cms-Uf-Um-Uf-Um-Cf- Am-Af-Gm-Uf-Um-Gf-Am-Gf- Am-Af-Cm-Af-Am-Af | 985/787 | Ums-Ufs-Um-Gf-Um-Uf-Cm-Uf- Cm-Af-Am-Cf-Um-Uf-Gm-Af-Am- Af-Am-Gf-Gms-Gfs-Am | 998/800 |
| B1579-AL | Cfs-Cms-Uf-Um-Uf-Um-Cf- Um-Uf-Cm-Uf-Am-Af-Um-Gf- Am-Gf-Um-Cf-Gm-Af | 986/788 | Ums-Cfs-Gm-Af-Cm-Uf-Cm-Af- Um-Uf-Am-Gf-Am-Af-Gm-Af-Am- Af-Am-Gf-Gms-Ufs-Gm | 999/801 |
| B1836-AL | Ufs-Gms-Gf-Gm-Uf-Um-Uf- Um-Af-Am-Af-Am-Uf-Um-Af- Am-Af-Gm-Uf-Am-Uf | 987/789 | Ams-Ufs-Am-Cf-Um-Uf-Um-Af- Am-Uf-Um-Uf-Um-Af-Am-Af-Am- Cf-Cm-Cf-Ams-Afs-Um | 1000/802 |
| B1789-AL | Gfs-Ums-Uf-Um-Gf-Um-Gf- Am-Af-Am-Cf-Am-Af-Am-Af- Am-Af-Gm-Uf-Gm-Uf | 988/790 | Ams-Cfs-Am-Cf-Um-Uf-Um-Uf- Um-Uf-Gm-Uf-Um-Uf-Cm-Af-Cm- Af-Am-Af-Cms-Afs-Am | 1001/803 |
| B1839-AL | Gfs-Ums-Uf-Um-Uf-Am-Af- Am-Af-Um-Uf-Am-Af-Am-Gf- Um-Af-Um-Af-Cm-Af | 989/791 | Ums-Gfs-Um-Af-Um-Af-Cm-Uf- Um-Uf-Am-Af-Um-Uf-Um-Uf-Am- Af-Am-Af-Cms-Cfs-Cm | 1002/804 |
| B1791-AL | Ufs-Ums-Gf-Um-Gf-Am-Af- Am-Cf-Am-Af-Am-Af-Am-Af- Gm-Uf-Gm-Uf-Um-Af | 990/792 | Ums-Afs-Am-Cf-Am-Cf-Um-Uf- Um-Uf-Um-Uf-Gm-Uf-Um-Uf-Cm- Af-Cm-Af-Ams-Afs-Cm | 1003/805 |
| B1795-AL | Gfs-Ams-Af-Am-Cf-Am-Af- Am-Af-Am-Af-Gm-Uf-Gm-Uf- Um-Cf-Cm-Cf-Um-Uf | 991/793 | Ams-Afs-Gm-Gf-Gm-Af-Am-Cf- Am-Cf-Um-Uf-Um-Uf-Um-Uf-Gm- Uf-Um-Uf-Cms-Afs-Cm | 1004/806 |
| B1585sAL | Cms-Ufs-Um-Cf-Um-Af-Am- Uf-Gm-Af-Gm-Uf-Cm-Gf-Am- Cf-Um-Uf-Um-Af | 992/794 | Ums-Afs-Am-Af-Gm-Uf-Cm-Gf- Am-Cf-Um-Cf-Am-Uf-Um-Af-Gm- Af-Am-Gfs-Ams-Af | 1005/807 |
| B1576sAL | Cms-Cfs-Am-Cf-Cm-Uf-Um-Uf- Um-Cf-Um-Uf-Cm-Uf-Am-Af- Um-Gf-Am-Af | 993/795 | Ums-Ufs-Cm-Af-Um-Uf-Am-Gf- Am-Af-Gm-Af-Am-Af-Am-Gf-Gm- Uf-Gm-Gfs-Gms-Af | 1006/808 |
| B1578sAL | Ams-Cfs-Cm-Uf-Um-Uf-Um- Cf-Um-Uf-Cm-Uf-Am-Af-Um- Gf-Am-Gf-Um-Af | 994/796 | Ums-Afs-Cm-Uf-Cm-Af-Um-Uf- Am-Gf-Am-Af-Gm-Af-Am-Af-Am- Gf-Gm-Ufs-Gms-Gf | 1007/809 |
| B1838-AL | Gfs-Gms-Uf-Um-Uf-Um-Af- Am-Af-Am-Uf-Um-Af-Am-Af- Gm-Uf-Am-Uf-Am-Af | 995/797 | Ums-Ufs-Am-Uf-Am-Cf-Um-Uf- Um-Af-Am-Uf-Um-Uf-Um-Af-Am- Af-Am-Cf-Cms-Cfs-Am | 1008/810 |
| B1008-AL | Cfs-Ums-Uf-Cm-Af-Cm-Uf- Gm-Af-Gm-Af-Gm-Cf-Gm-Cf- Cm-Uf-Gm-Cf-Cm-Uf | 1009/811 | Ams-Gfs-Gm-Cf-Am-Gf-Gm-Cf- Gm-Cf-Um-Cf-Um-Cf-Am-Gf-Um- Gf-Am-Af-Gms-Gfs-Gm - | 1095/897 |
| B1011-AL | Cfs-Ams-Cf-Um-Gf-Am-Gf- Am-Gf-Cm-Gf-Cm-Cf-Um-Gf- Cm-Cf-Um-Gf-Cm-Uf | 1010/812 | Ams-Gfs-Cm-Af-Gm-Gf-Cm-Af- Gm-Gf-Cm-Gf-Cm-Uf-Cm-Uf-Cm- Af-Gm-Uf-Gms-Afs-Am | 1096/898 |
| B1014-AL | Ufs-Gms-Af-Gm-Af-Gm-Cf- Gm-Cf-Cm-Uf-Gm-Cf-Cm-Uf- Gm-Cf-Um-Gf-Cm-Uf | 1011/813 | Ams-Gfs-Cm-Af-Gm-Cf-Am-Gf- Gm-Cf-Am-Gf-Gm-Cf-Gm-Cf-Um- Cf-Um-Cf-Ams-Gfs-Um - | 1097/899 |
| B1016-AL | Afs-Gms-Af-Gm-Cf-Gm-Cf- Cm-Uf-Gm-Cf-Cm-Uf-Gm-Cf- Um-Gf-Cm-Uf-Gm-Af | 1012/814 | Ums-Cfs-Am-Gf-Cm-Af-Gm-Cf- Am-Gf-Gm-Cf-Am-Gf-Gm-Cf-Gm- Cf-Um-Cf-Ums-Cfs-Am | - 1098/900 |
| B1017-AL | Gfs-Ams-Gf-Cm-Gf-Cm-Cf- Um-Gf-Cm-Cf-Um-Gf-Cm-Uf- Gm-Cf-Um-Gf-Am-Uf | 1013/815 | Ams-Ufs-Cm-Af-Gm-Cf-Am-Gf- Cm-Af-Gm-Gf-Cm-Af-Gm-Gf-Cm- Gf-Cm-Uf-Cms-Ufs-Cm - | 1099/901 |
| B1020-AL | Cfs-Gms-Cf-Cm-Uf-Gm-Cf-Cm- Uf-Gm-Cf-Um-Gf-Cm-Uf-Gm- Af-Um-Cf-Cm-Af | 1014/816 | Ums-Gfs-Gm-Af-Um-Cf-Am-Gf- Cm-Af-Gm-Cf-Am-Gf-Gm-Cf-Am- Gf-Gm-Cf-Gms-Cfs-Um - | 1100/902 |
| B1024-AL | Ufs-Gms-Cf-Cm-Uf-Gm-Cf- Um-Gf-Cm-Uf-Gm-Af-Um-Cf- Cm-Af-Gm-Cf-Cm-Uf | 1015/817 | Ams-Gfs-Gm-Cf-Um-Gf-Gm-Af- Um-Cf-Am-Gf-Cm-Af-Gm-Cf-Am- Gf-Gm-Cf-Ams-Gfs-Gm - | 1101/903 |
| B1026-AL | Cfs-Cms-Uf-Gm-Cf-Um-Gf-Cm- Uf-Gm-Af-Um-Cf-Cm-Af-Gm- Cf-Cm-Uf-Cm-Af | 1016/818 | Ums-Gfs-Am-Gf-Gm-Cf-Um-Gf- Gm-Af-Um-Cf-Am-Gf-Cm-Af-Gm- Cf-Am-Gf-Gms-Cfs-Am | - 1102/904 |
| B1028-AL | Ufs-Gms-Cf-Um-Gf-Cm-Uf- Gm-Af-Um-Cf-Cm-Af-Gm-Cf- Cm-Uf-Cm-Af-Cm-Uf | 1017/819 | Ams-Gfs-Um-Gf-Am-Gf-Gm-Cf- Um-Gf-Gm-Af-Um-Cf-Am-Gf-Cm- Af-Gm-Cf-Ams-Gfs-Gm | - 1103/905 |
| B1029-AL | Gfs-Cms-Uf-Gm-Cf-Um-Gf- Am-Uf-Cm-Cf-Am-Gf-Cm-Cf- Um-Cf-Am-Cf-Um-Af | 1018/820 | Ums-Afs-Gm-Uf-Gm-Af-Gm-Gf- Cm-Uf-Gm-Gf-Am-Uf-Cm-Af-Gm- Cf-Am-Gf-Cms-Afs-Gm | - 1104/906 |
| B1030-AL | Cfs-Ums-Gf-Cm-Uf-Gm-Af- Um-Cf-Cm-Af-Gm-Cf-Cm-Uf- Cm-Af-Cm-Uf-Am-Uf | 1019/821 | Ams-Ufs-Am-Gf-Um-Gf-Am-Gf- Gm-Cf-Um-Gf-Gm-Af-Um-Cf-Am- Gf-Cm-Af-Gms-Cfs-Am | - 1105/907 |
| B1038-AL | Cfs-Cms-Af-Gm-Cf-Cm-Uf-Cm- Af-Cm-Uf-Am-Uf-Gm-Cf-Cm- Uf-Cm-Uf-Gm-Af | 1020/822 | Ums-Cfs-Am-Gf-Am-Gf-Gm-Cf- Am-Uf-Am-Gf-Um-Gf-Am-Gf-Gm- Cf-Um-Gf-Gms-Afs-Um | - 1106/908 |
| B1041-AL | Gfs-Cms-Cf-Um-Cf-Am-Cf-Um- Af-Um-Gf-Cm-Cf-Um-Cf-Um- Gf-Am-Cf-Cm-Uf | 1021/823 | Ams-Gfs-Gm-Uf-Cm-Af-Gm-Af- Gm-Gf-Cm-Af-Um-Af-Gm-Uf-Gm- Af-Gm-Gf-Cms-Ufs-Gm | - 1107/909 |
| B1044-AL | Ufs-Cms-Af-Cm-Uf-Am-Uf- Gm-Cf-Cm-Uf-Cm-Uf-Gm-Af- Cm-Cf-Um-Gf-Gm-Af | 1022/824 | Ums-Cfs-Cm-Af-Gm-Gf-Um-Cf- Am-Gf-Am-Gf-Gm-Cf-Am-Uf-Am- Gf-Um-Gf-Ams-Gfs-Gm | - 1108/910 |
| B1046-AL | Afs-Cms-Uf-Am-Uf-Gm-Cf- Cm-Uf-Cm-Uf-Gm-Af-Cm-Cf- Um-Gf-Gm-Af-Cm-Af | 1023/825 | Ums-Gfs-Um-Cf-Cm-Af-Gm-Gf- Um-Cf-Am-Gf-Am-Gf-Gm-Cf-Am- Uf-Am-Gf-Ums-Gfs-Am | 1109/911 |
| B1047-AL | Cfs-Ums-Af-Um-Gf-Cm-Cf- Um-Cf-Um-Gf-Am-Cf-Cm-Uf- Gm-Gf-Am-Cf-Am-Af | 1024/826 | Ums-Ufs-Gm-Uf-Cm-Cf-Am-Gf- Gm-Uf-Cm-Af-Gm-Af-Gm-Gf-Cm- Af-Um-Af-Gms-Ufs-Gm | 1110/912 |
| B1053-AL | Cfs-Ums-Cf-Um-Gf-Am-Cf-Cm- Uf-Gm-Gf-Am-Cf-Am-Af-Gm- Gf-Um-Gf-Gm-Af | 1025/827 | Ums-Cfs-Cm-Af-Cm-Cf-Um-Uf- Gm-Uf-Cm-Cf-Am-Gf-Gm-Uf-Cm- Af-Gm-Af-Gms-Gfs-Cm | 1111/913 |
| B1057-AL | Gfs-Ams-Cf-Cm-Uf-Gm-Gf- Am-Cf-Am-Af-Gm-Gf-Um-Gf- Gm-Af-Gm-Gf-Gm-Uf | 1026/828 | Ams-Cfs-Cm-Cf-Um-Cf-Cm-Af- Cm-Cf-Um-Uf-Gm-Uf-Cm-Cf-Am- Gf-Gm-Uf-Cms-Afs-Gm | 1112/914 |
| B1061-AL | Ufs-Gms-Gf-Am-Cf-Am-Af- Gm-Gf-Um-Gf-Gm-Af-Gm-Gf- Gm-Uf-Cm-Uf-Cm-Af | 1027/829 | Ums-Gfs-Am-Gf-Am-Cf-Cm-Cf- Um-Cf-Cm-Af-Cm-Cf-Um-Uf-Gm- Uf-Cm-Cf-Ams-Gfs-Gm | 1113/915 |
| B1065sAL | Cms-Afs-Am-Gf-Gm-Uf-Gm- Gf-Am-Gf-Gm-Gf-Um-Cf-Um- Cf-Am-Cf-Um-Uf | 1028/830 | Ams-Afs-Gm-Uf-Gm-Af-Gm-Af- Cm-Cf-Cm-Uf-Cm-Cf-Am-Cf-Cm- Uf-Um-Gfs-Ums-Cf | 1114/916 |
| B1071-AL | Gfs-Gms-Af-Gm-Gf-Gm-Uf- Cm-Uf-Cm-Af-Cm-Uf-Um-Uf- Cm-Cf-Am-Gf-Cm-Af | 1029/831 | Ums-Gfs-Cm-Uf-Gm-Gf-Am-Af- Am-Gf-Um-Gf-Am-Gf-Am-Cf-Cm- Cf-Um-Cf-Cms-Afs-Cm | 1115/917 |
| B1260-AL | Cfs-Ams-Gf-Gm-Gf-Um-Gf- Gm-Gf-Gm-Gf-Am-Gf-Gm-Uf- Gm-Cf-Um-Gf-Am-Af | 1030/832 | Ums-Ufs-Cm-Af-Gm-Cf-Am-Cf- Cm-Uf-Cm-Cf-Cm-Cf-Cm-Af-Cm- Cf-Cm-Uf-Gms-Afs-Um | 1116/918 |
| B1262-AL | Gfs-Gms-Gf-Um-Gf-Gm-Gf- Gm-Gf-Am-Gf-Gm-Uf-Gm-Cf- Um-Gf-Am-Af-Cm-Af | 1031/833 | Ums-Gfs-Um-Uf-Cm-Af-Gm-Cf- Am-Cf-Cm-Uf-Cm-Cf-Cm-Cf-Cm- Af-Cm-Cf-Cms-Ufs-Gm | 1117/919 |
| B1265-AL | Ufs-Gms-Gf-Gm-Gf-Gm-Af- Gm-Gf-Um-Gf-Cm-Uf-Gm-Af- Am-Cf-Am-Gf-Cm-Af | 1032/834 | Ums-Gfs-Cm-Uf-Gm-Uf-Um-Cf- Am-Gf-Cm-Af-Cm-Cf-Um-Cf-Cm- Cf-Cm-Cf-Ams-Cfs-Cm | 1118/920 |
| B1266-AL | Gfs-Gms-Gf-Gm-Gf-Am-Gf- Gm-Uf-Gm-Cf-Um-Gf-Am-Af- Cm-Af-Gm-Cf-Am-Uf | 1033/835 | Ams-Ufs-Gm-Cf-Um-Gf-Um-Uf- Cm-Af-Gm-Cf-Am-Cf-Cm-Uf-Cm- Cf-Cm-Cf-Cms-Afs-Cm | 1119/921 |
| B1267-AL | Gfs-Gms-Gf-Gm-Af-Gm-Gf- Um-Gf-Cm-Uf-Gm-Af-Am-Cf- Am-Gf-Cm-Af-Um-Uf | 1034/836 | Ams-Afs-Um-Gf-Cm-Uf-Gm-Uf- Um-Cf-Am-Gf-Cm-Af-Cm-Cf-Um- Cf-Cm-Cf-Cms-Cfs-Am | 1120/922 |
| B1268-AL | Gfs-Gms-Gf-Am-Gf-Gm-Uf- Gm-Cf-Um-Gf-Am-Af-Cm-Af- Gm-Cf-Am-Uf-Um-Uf | 1035/837 | Ams-Afs-Am-Uf-Gm-Cf-Um-Gf- Um-Uf-Cm-Af-Gm-Cf-Am-Cf-Cm- Uf-Cm-Cf-Cms-Cfs-Cm | 1121/923 |
| B1279-AL | Afs-Ams-Cf-Am-Gf-Cm-Af- Um-Uf-Um-Uf-Um-Uf-Um-Uf- Gm-Af-Gm-Cf-Um-Uf | 1036/838 | Ams-Afs-Gm-Cf-Um-Cf-Am-Af- Am-Af-Am-Af-Am-Af-Um-Gf-Cm- Uf-Gm-Uf-Ums-Cfs-Am | 1122/924 |
| B1281-AL | Cfs-Ams-Gf-Cm-Af-Um-Uf- Um-Uf-Um-Uf-Um-Uf-Gm-Af- Gm-Cf-Um-Uf-Gm-Af | 1037/839 | Ums-Cfs-Am-Af-Gm-Cf-Um-Cf- Am-Af-Am-Af-Am-Af-Am-Af-Um- Gf-Cm-Uf-Gms-Ufs-Um | 1123/925 |
| B1282-AL | Afs-Gms-Cf-Am-Uf-Um-Uf- Um-Uf-Um-Uf-Um-Gf-Am-Gf- Cm-Uf-Um-Gf-Am-Af | 1038/840 | Ums-Ufs-Cm-Af-Am-Gf-Cm-Uf- Cm-Af-Am-Af-Am-Af-Am-Af-Am- Uf-Gm-Cf-Ums-Gfs-Um | 1124/926 |
| B1362-AL | Gfs-Gms-Uf-Gm-Af-Cm-Cf- Cm-Uf-Gm-Af-Am-Cf-Cm-Gf- Cm-Cf-Cm-Af-Um-Uf | 1039/841 | Ams-Afs-Um-Gf-Gm-Gf-Cm-Gf- Gm-Uf-Um-Cf-Am-Gf-Gm-Gf-Um- Cf-Am-Cf-Cms-Ufs-Cm | 1125/927 |
| B1365-AL | Gfs-Ams-Cf-Cm-Cf-Um-Gf- Am-Af-Cm-Cf-Gm-Cf-Cm-Cf- Am-Uf-Um-Cf-Cm-Uf | 1040/842 | Ams-Gfs-Gm-Af-Am-Uf-Gm-Gf- Gm-Cf-Gm-Gf-Um-Uf-Cm-Af-Gm- Gf-Gm-Uf-Cms-Afs-Cm | 1126/928 |
| C B1367-AL | Cfs-Cms-Cf-Um-Gf-Am-Af-Cm- Cf-Gm-Cf-Cm-Cf-Am-Uf-Um- Cf-Cm-Uf-Gm-Uf | 1041/843 | Ams-Cfs-Am-Gf-Gm-Af-Am-Uf- Gm-Gf-Gm-Cf-Gm-Gf-Um-Uf-Cm- Af-Gm-Gf-Gms-Ufs-Cm | 1127/929 |
| B1369-AL | Cfs-Ums-Gf-Am-Af-Cm-Cf- Gm-Cf-Cm-Cf-Am-Uf-Um-Cf- Cm-Uf-Gm-Uf-Um-Uf | 1042/844 | Ams-Afs-Am-Cf-Am-Gf-Gm-Af- Am-Uf-Gm-Gf-Gm-Cf-Gm-Gf-Um- Uf-Cm-Af-Gms-Gfs-Gm | 1128/930 |
| B1393-AL | Gfs-Ums-Gf-Um-Af-Um-Gf- Am-Uf-Cm-Af-Am-Af-Gm-Cf- Gm-Cf-Cm-Af-Cm-Uf | 1043/845 | Ams-Gfs-Um-Gf-Gm-Cf-Gm-Cf- Um-Uf-Um-Gf-Am-Uf-Cm-Af-Um- Af-Cm-Af-Cms-Afs-Gm | 1129/931 |
| B1401-AL | Ufs-Cms-Af-Am-Af-Gm-Cf- Gm-Cf-Cm-Af-Cm-Uf-Gm-Cf- Cm-Cf-Um-Gf-Cm-Af | 1044/846 | Ums-Gfs-Cm-Af-Gm-Gf-Gm-Cf- Am-Gf-Um-Gf-Gm-Cf-Gm-Cf-Um- Uf-Um-Gf-Ams-Ufs-Cm | 1130/932 |
| B1404-AL | Afs-Ams-Gf-Cm-Gf-Cm-Cf- Am-Cf-Um-Gf-Cm-Cf-Cm-Uf- Gm-Cf-Am-Cf-Um-Uf | 1045/847 | Ams-Afs-Gm-Uf-Gm-Cf-Am-Gf- Gm-Gf-Cm-Af-Gm-Uf-Gm-Gf-Cm- Gf-Cm-Uf-Ums-Ufs-Gm | 1131/933 |
| C B1407-AL | Cfs-Gms-Cf-Cm-Af-Cm-Uf-Gm- Cf-Cm-Cf-Um-Gf-Cm-Af-Cm- Uf-Um-Cf-Cm-Uf | 1046/848 | Ams-Gfs-Gm-Af-Am-Gf-Um-Gf- Cm-Af-Gm-Gf-Gm-Cf-Am-Gf-Um- Gf-Gm-Cf-Gms-Cfs-Um | 1132/934 |
| B1554-AL | Gfs-Cms-Gf-Am-Uf-Gm-Uf- Gm-Uf-Cm-Af-Cm-Cf-Cm-Cf- Cm-Af-Gm-Uf-Cm-Uf | 1047/849 | Ams-Gfs-Am-Cf-Um-Gf-Gm-Gf- Gm-Gf-Um-Gf-Am-Cf-Am-Cf-Am- Uf-Cm-Gf-Cms-Ufs-Gm | 1133/935 |
| B1558-AL | Ufs-Gms-Uf-Gm-Uf-Cm-Af- Cm-Cf-Cm-Cf-Cm-Af-Gm-Uf- Cm-Uf-Cm-Cf-Cm-Af | 1048/850 | Ums-Gfs-Gm-Gf-Am-Gf-Am-Cf- Um-Gf-Gm-Gf-Gm-Gf-Um-Gf-Am- Cf-Am-Cf-Ams-Ufs-Cm | 1134/936 |
| B1561-AL | Gfs-Ums-Cf-Am-Cf-Cm-Cf-Cm- Cf-Am-Gf-Um-Cf-Um-Cf-Cm- Cf-Am-Cf-Cm-Uf | 1049/851 | Ams-Gfs-Gm-Uf-Gm-Gf-Gm-Af- Gm-Af-Cm-Uf-Gm-Gf-Gm-Gf-Gm- Uf-Gm-Af-Cms-Afs-Cm | 1135/937 |
| B1562-AL | Ufs-Cms-Af-Cm-Cf-Cm-Cf-Cm- Af-Gm-Uf-Cm-Uf-Cm-Cf-Cm- Af-Cm-Cf-Um-Uf | 1050/852 | Ams-Afs-Gm-Gf-Um-Gf-Gm-Gf- Am-Gf-Am-Cf-Um-Gf-Gm-Gf-Gm- Gf-Um-Gf-Ams-Cfs-Am | 1136/938 |
| B1564-AL | Afs-Cms-Cf-Cm-Cf-Cm-Af-Gm- Uf-Cm-Uf-Cm-Cf-Cm-Af-Cm- Cf-Um-Uf-Um-Uf | - 1051/853 G | Ams-Afs-Am-Af-Gm-Gf-Um-Gf- Gm-Gf-Am-Gf-Am-Cf-Um-Gf-Gm- Gf-Gm-Gf-Ums-Gfs-Am | 1137/939 |
| B1591-AL | Afs-Ums-Gf-Am-Gf-Um-Cf- Gm-Af-Cm-Uf-Um-Uf-Gm-Af- Gm-Cf-Um-Gf-Gm-Af | 1052/854 A | Ums-Cfs-Cm-Af-Gm-Cf-Um-Cf- Am-Af-Am-Gf-Um-Cf-Gm-Af-Cm- Uf-Cm-Af-Ums-Ufs-Am | 1138/940 |
| B1592-AL | Ufs-Gms-Af-Gm-Uf-Cm-Gf- Am-Cf-Um-Uf-Um-Gf-Am-Gf- Cm-Uf-Gm-Gf-Am-Af | 1053/855 C | Ums-Ufs-Cm-Cf-Am-Gf-Cm-Uf- Cm-Af-Am-Af-Gm-Uf-Cm-Gf-Am- Cf-Um-Cf-Ams-Ufs-Um | 1139/941 |
| B1593-AL | Gfs-Ams-Gf-Um-Cf-Gm-Af- Cm-Uf-Um-Uf-Gm-Af-Gm-Cf- Um-Gf-Gm-Af-Am-Af | 1054/856 U | Ums-Ufs-Um-Cf-Cm-Af-Gm-Cf- Um-Cf-Am-Af-Am-Gf-Um-Cf-Gm- Af-Cm-Uf-Cms-Afs-Um | 1140/942 |
| B1596-AL | Ufs-Cms-Gf-Am-Cf-Um-Uf- Um-Gf-Am-Gf-Cm-Uf-Gm-Gf- Am-Af-Am-Gf-Cm-Af | 1055/857 A | Ums-Gfs-Cm-Uf-Um-Uf-Cm-Cf- Am-Gf-Cm-Uf-Cm-Af-Am-Af-Gm- Uf-Cm-Gf-Ams-Cfs-Um | 1141/943 |
| B1602-AL | Ufs-Ums-Gf-Am-Gf-Cm-Uf- Gm-Gf-Am-Af-Am-Gf-Cm-Af- Gm-Cf-Cm-Gf-Um-Uf | 1056/858 C | Ams-Afs-Cm-Gf-Gm-Cf-Um-Gf- Cm-Uf-Um-Uf-Cm-Cf-Am-Gf-Cm- Uf-Cm-Af-Ams-Afs-Gm | 1142/944 |
| B1603-AL | Ufs-Gms-Af-Gm-Cf-Um-Gf- Gm-Af-Am-Af-Gm-Cf-Am-Gf- Cm-Cf-Gm-Uf-Um-Uf | 1057/859 G | Ams-Afs-Am-Cf-Gm-Gf-Cm-Uf- Gm-Cf-Um-Uf-Um-Cf-Cm-Af-Gm- Cf-Um-Cf-Ams-Afs-Am | 1143/945 |
| B1608-AL | Ufs-Gms-Gf-Am-Af-Am-Gf- Cm-Af-Gm-Cf-Cm-Gf-Um-Uf- Um-Cf-Um-Cf-Cm-Uf | 1058/860 C | Ams-Gfs-Gm-Af-Gm-Af-Am-Af- Cm-Gf-Gm-Cf-Um-Gf-Cm-Uf-Um- Uf-Cm-Cf-Ams-Gfs-Cm | 1144/946 |
| B1612-AL | Afs-Ams-Gf-Cm-Af-Gm-Cf- Cm-Gf-Um-Uf-Um-Cf-Um-Cf- Cm-Uf-Um-Gf-Gm-Uf | 1059/861 G | Ams-Cfs-Cm-Af-Am-Gf-Gm-Af- Gm-Af-Am-Af-Cm-Gf-Gm-Cf-Um- Gf-Cm-Uf-Ums-Ufs-Cm | 1145/947 |
| B1775-AL | Cfs-Ams-Af-Cm-Cf-Gm-Af-Cm- Cf-Am-Gf-Cm-Uf-Um-Gf-Um- Uf-Um-Gf-Um-Af | - 1060/862 A | Ums-Afs-Cm-Af-Am-Af-Cm-Af- Am-Gf-Cm-Uf-Gm-Gf-Um-Cf-Gm- Gf-Um-Uf-Gms-Gfs-Am | 1146/948 |
| B1790-AL | Ufs-Ums-Uf-Gm-Uf-Gm-Af- Am-Af-Cm-Af-Am-Af-Am-Af- Am-Gf-Um-Gf-Um-Uf | 1061/863 U | Ams-Afs-Cm-Af-Cm-Uf-Um-Uf- Um-Uf-Um-Gf-Um-Uf-Um-Cf-Am- Cf-Am-Af-Ams-Cfs-Am | 1147/949 |
| B1796-AL | Afs-Ams-Af-Cm-Af-Am-Af- Am-Af-Am-Gf-Um-Gf-Um-Uf- Cm-Cf-Cm-Uf-Um-Uf | 1062/864 C | Ams-Afs-Am-Gf-Gm-Gf-Am-Af- Cm-Af-Cm-Uf-Um-Uf-Um-Uf-Um- Gf-Um-Uf-Ums-Cfs-Am | 1148/950 |
| B1798sAL | Ams-Cfs-Am-Af-Am-Af-Am- Af-Gm-Uf-Gm-Uf-Um-Cf-Cm- Cf-Um-Uf-Um-Uf | 1063/865 A | Ams-Afs-Am-Af-Gm-Gf-Gm-Af- Am-Cf-Am-Cf-Um-Uf-Um-Uf-Um- Uf-Gm-Ufs-Ums-Uf | 1149/951 |
| B1799-AL | Cfs-Ams-Af-Am-Af-Am-Af- Gm-Uf-Gm-Uf-Um-Cf-Cm-Cf- Um-Uf-Um-Uf-Cm-Af | 1064/866 G | Ums-Gfs-Am-Af-Am-Af-Gm-Gf- Gm-Af-Am-Cf-Am-Cf-Um-Uf-Um- Uf-Um-Uf-Gms-Ufs-Um | 1150/952 |
| B1810-AL | Ufs-Cms-Cf-Cm-Uf-Um-Uf- Um-Cf-Am-Af-Gm-Uf-Um-Gf- Am-Gf-Am-Af-Cm-Af | 1065/867 | Ums-Gfs-Um-Uf-Cm-Uf-Cm-Af- Am-Cf-Um-Uf-Gm-Af-Am-Af-Am- Gf-Gm-Gf-Ams-Afs-Cm | 1151/953 |
| B1811-AL | Cfs-Cms-Cf-Um-Uf-Um-Uf-Cm- Af-Am-Gf-Um-Uf-Gm-Af-Gm- Af-Am-Cf-Am-Af | - 1066/868 | Ums-Ufs-Gm-Uf-Um-Cf-Um-Cf- Am-Af-Cm-Uf-Um-Gf-Am-Af-Am- Af-Gm-Gf-Gms-Afs-Am | 1152/954 |
| B1814-AL | Ufs-Ums-Uf-Um-Cf-Am-Af- Gm-Uf-Um-Gf-Am-Gf-Am-Af- Cm-Af-Am-Af-Am-Af | 1067/869 | Ums-Ufs-Um-Uf-Um-Gf-Um-Uf- Cm-Uf-Cm-Af-Am-Cf-Um-Uf-Gm- Af-Am-Af-Ams-Gfs-Gm | 1153/955 |
| B1815-AL | Ufs-Ums-Uf-Cm-Af-Am-Gf- Um-Uf-Gm-Af-Gm-Af-Am-Cf- Am-Af-Am-Af-Am-Uf | 1068/870 | Ams-Ufs-Um-Uf-Um-Uf-Gm-Uf- Um-Cf-Um-Cf-Am-Af-Cm-Uf-Um- Gf-Am-Af-Ams-Afs-Gm | 1154/956 |
| B1817-AL | Ufs-Cms-Af-Am-Gf-Um-Uf- Gm-Af-Gm-Af-Am-Cf-Am-Af- Am-Af-Am-Uf-Um-Gf | 1069/871 | Cms-Afs-Am-Uf-Um-Uf-Um-Uf- Gm-Uf-Um-Cf-Um-Cf-Am-Af-Cm- Uf-Um-Gf-Ams-Afs-Am | 1155/957 |
| B1828-AL | Afs-Cms-Af-Am-Af-Am-Af- Um-Uf-Gm-Gf-Gm-Uf-Um-Uf- Um-Af-Am-Af-Am-Uf | 1070/872 | Ams-Ufs-Um-Uf-Um-Af-Am-Af- Am-Cf-Cm-Cf-Am-Af-Um-Uf-Um- Uf-Um-Gf-Ums-Ufs-Cm | 1156/958 |
| B1838sAL | Gms-Gfs-Um-Uf-Um-Uf-Am- Af-Am-Af-Um-Uf-Am-Af-Am- Gf-Um-Af-Um-Af | 1071/873 | Ums-Afs-Um-Af-Cm-Uf-Um-Uf- Am-Af-Um-Uf-Um-Uf-Am-Af-Am- Af-Cm-Cfs-Cms-Af | 1157/959 |
| B2018-AL | Afs-Ums-Af-Gm-Cf-Um-Gf- Gm-Uf-Um-Af-Um-Uf-Um-Cf- Um-Cf-Cm-Cf-Um-Uf | 1072/874 | Ams-Afs-Gm-Gf-Gm-Af-Gm-Af- Am-Af-Um-Af-Am-Cf-Cm-Af-Gm- Cf-Um-Af-Ums-Gfs-Gm | 1158/960 |
| B2020-AL | Afs-Gms-Cf-Um-Gf-Gm-Uf- Um-Af-Um-Uf-Um-Cf-Um-Cf- Cm-Cf-Um-Uf-Gm-Uf | 1073/875 | Ams-Cfs-Am-Af-Gm-Gf-Gm-Af- Gm-Af-Am-Af-Um-Af-Am-Cf-Cm- Af-Gm-Cf-Ums-Afs-Um | 1159/961 |
| B2025-AL | Gfs-Ums-Uf-Am-Uf-Um-Uf- Cm-Uf-Cm-Cf-Cm-Uf-Um-Gf- Um-Gf-Um-Uf-Am-Af | 1074/876 | Ums-Ufs-Am-Af-Cm-Af-Cm-Af- Am-Gf-Gm-Gf-Am-Gf-Am-Af-Am- Uf-Am-Af-Cms-Cfs-Am | 1160/962 |
| B2030-AL | Ufs-Ums-Cf-Um-Cf-Cm-Cf-Um- Uf-Gm-Uf-Gm-Uf-Um-Af-Gm- Uf-Am-Af-Um-Af | - 1075/877 | Ums-Afs-Um-Uf-Am-Cf-Um-Af- Am-Cf-Am-Cf-Am-Af-Gm-Gf-Gm- Af-Gm-Af-Ams-Afs-Um | 1161/963 |
| B2031-AL | Ufs-Cms-Uf-Cm-Cf-Cm-Uf-Um- Gf-Um-Gf-Um-Uf-Am-Gf-Um- Af-Am-Uf-Am-Af | - 1076/878 | Ums-Ufs-Am-Uf-Um-Af-Cm-Uf- Am-Af-Cm-Af-Cm-Af-Am-Gf-Gm- Gf-Am-Gf-Ams-Afs-Am | 1162/964 |
| B2032-AL | Cfs-Ums-Cf-Cm-Cf-Um-Uf-Gm- Uf-Gm-Uf-Um-Af-Gm-Uf-Am- Af-Um-Af-Am-Af | - 1077/879 | Ums-Ufs-Um-Af-Um-Uf-Am-Cf- Um-Af-Am-Cf-Am-Cf-Am-Af-Gm- Gf-Gm-Af-Gms-Afs-Am | 1163/965 |
| B2033-AL | Ufs-Cms-Cf-Cm-Uf-Um-Gf- Um-Gf-Um-Uf-Am-Gf-Um-Af- Am-Uf-Am-Af-Am-Af | 1078/880 | Ums-Ufs-Um-Uf-Am-Uf-Um-Af- Cm-Uf-Am-Af-Cm-Af-Cm-Af-Am- Gf-Gm-Gf-Ams-Gfs-Am | 1164/966 |
| B729-AL | Afs-Gms-Af-Am-Cf-Um-Gf- Gm-Af-Um-Gf-Um-Uf-Gm-Cf- Um-Gf-Cm-Uf-Gm-Af | 1079/881 | Ums-Cfs-Am-Gf-Cm-Af-Gm-Cf- Am-Af-Cm-Af-Um-Cf-Cm-Af-Gm- Uf-Um-Cf-Ums-Gfs-Um | 1165/967 |
| B731-AL | Afs-Ams-Cf-Um-Gf-Gm-Af- Um-Gf-Um-Uf-Gm-Cf-Um-Gf- Cm-Uf-Gm-Af-Gm-Af | 1080/882 | Ums-Cfs-Um-Cf-Am-Gf-Cm-Af- Gm-Cf-Am-Af-Cm-Af-Um-Cf-Cm- Af-Gm-Uf-Ums-Cfs-Um | 1166/968 |
| B732-AL | Afs-Cms-Uf-Gm-Gf-Am-Uf- Gm-Uf-Um-Gf-Cm-Uf-Gm-Cf- Um-Gf-Am-Gf-Am-Af | 1081/883 | Ums-Ufs-Cm-Uf-Cm-Af-Gm-Cf- Am-Gf-Cm-Af-Am-Cf-Am-Uf-Cm- Cf-Am-Gf-Ums-Ufs-Cm | 1167/969 |
| B734-AL | Ufs-Gms-Gf-Am-Uf-Gm-Uf- Um-Gf-Cm-Uf-Gm-Cf-Um-Gf- Am-Gf-Am-Af-Gm-Af | 1082/884 | Ums-Cfs-Um-Uf-Cm-Uf-Cm-Af- Gm-Cf-Am-Gf-Cm-Af-Am-Cf-Am- Uf-Cm-Cf-Ams-Gfs-Um | 1168/970 |
| B734s-AL | Ums-Gfs-Gm-Af-Um-Gf-Um- Uf-Gm-Cf-Um-Gf-Cm-Uf-Gm- Af-Gm-Af-Am-Af | 1083/885 | Ums-Ufs-Um-Cf-Um-Cf-Am-Gf- Cm-Af-Gm-Cf-Am-Af-Cm-Af-Um- Cf-Cm-Afs-Gms-Uf | 1169/971 |
| B736-AL | Gfs-Ams-Uf-Gm-Uf-Um-Gf- Cm-Uf-Gm-Cf-Um-Gf-Am-Gf- Am-Af-Gm-Af-Um-Uf | 1084/886 | Ams-Afs-Um-Cf-Um-Uf-Cm-Uf- Cm-Af-Gm-Cf-Am-Gf-Cm-Af-Am- Cf-Am-Uf-Cms-Cfs-Am | 1170/972 |
| B743-AL | Cfs-Ums-Gf-Cm-Uf-Gm-Af- Gm-Af-Am-Gf-Am-Uf-Um-Gf- Am-Cf-Am-Gf-Gm-Uf | 1085/887 | Ams-Cfs-Cm-Uf-Gm-Uf-Cm-Af- Am-Uf-Cm-Uf-Um-Cf-Um-Cf-Am- Gf-Cm-Af-Gms-Cfs-Am | 1171/973 |
| B753s-AL | Gms-Afs-Um-Uf-Gm-Af-Cm- Af-Gm-Gf-Um-Uf-Cm-Af-Um- Gf-Cm-Af-Gm-Af | 1086/888 | Ums-Cfs-Um-Gf-Cm-Af-Um-Gf- Am-Af-Cm-Cf-Um-Gf-Um-Cf-Am- Af-Um-Cfs-Ums-Uf | 1172/974 |
| B963-AL | Gfs-Cms-Af-Cm-Uf-Gm-Gf- Am-Gf-Um-Gf-Am-Cf-Am-Uf- Cm-Cf-Am-Gf-Gm-Af | 1087/889 | Ums-Cfs-Cm-Uf-Gm-Gf-Am-Uf- Gm-Uf-Cm-Af-Cm-Uf-Cm-Cf-Am- Gf-Um-Gf-Cms-Ufs-Gm | 1173/975 |
| B966-AL | Cfs-Ums-Gf-Gm-Af-Gm-Uf- Gm-Af-Cm-Af-Um-Cf-Cm-Af- Gm-Gf-Am-Cf-Am-Af | 1088/890 | Ums-Ufs-Gm-Uf-Cm-Cf-Um-Gf- Gm-Af-Um-Gf-Um-Cf-Am-Cf-Um- Cf-Cm-Af-Gms-Ufs-Gm | 1174/976 |
| B969-AL | Gfs-Ams-Gf-Um-Gf-Am-Cf- Am-Uf-Cm-Cf-Am-Gf-Gm-Af- Cm-Af-Am-Cf-Um-Uf | 1089/891 | Ams-Afs-Gm-Uf-Um-Gf-Um-Cf- Cm-Uf-Gm-Gf-Am-Uf-Gm-Uf-Cm- Af-Cm-Uf-Cms-Cfs-Am | 1175/977 |
| B971-AL | Gfs-Ums-Gf-Am-Cf-Am-Uf- Cm-Cf-Am-Gf-Gm-Af-Cm-Af- Am-Cf-Um-Uf-Cm-Uf | 1090/892 | Ams-Gfs-Am-Af-Gm-Uf-Um-Gf- Um-Cf-Cm-Uf-Gm-Gf-Am-Uf-Gm- Uf-Cm-Af-Cms-Ufs-Cm | 1176/978 |
| B982-AL | Gfs-Ams-Cf-Am-Af-Cm-Uf- Um-Cf-Um-Cf-Gm-Gf-Um-Gf- Am-Cf-Um-Cf-Am-Af | 1091/893 | Ums-Ufs-Gm-Af-Gm-Uf-Cm-Af- Cm-Cf-Gm-Af-Gm-Af-Am-Gf-Um- Uf-Gm-Uf-Cms-Cfs-Um | 1177/979 |
| B984-AL | Cfs-Ams-Af-Cm-Uf-Um-Cf- Um-Cf-Gm-Gf-Um-Gf-Am-Cf- Um-Cf-Am-Af-Gm-Uf | 1092/894 | Ams-Cfs-Um-Uf-Gm-Af-Gm-Uf- Cm-Af-Cm-Cf-Gm-Af-Gm-Af-Am- Gf-Um-Uf-Gms-Ufs-Cm | 1178/980 |
| B992-AL | Cfs-Gms-Gf-Um-Gf-Am-Cf- Um-Cf-Am-Af-Gm-Uf-Gm-Cf- Cm-Cf-Um-Uf-Cm-Af | 1093/895 | Ums-Gfs-Am-Af-Gm-Gf-Gm-Cf- Am-Cf-Um-Uf-Gm-Af-Gm-Uf-Cm- Af-Cm-Cf-Gms-Afs-Gm | 1179/981 |
| B994-AL | Gfs-Ums-Gf-Am-Cf-Um-Cf- Am-Af-Gm-Uf-Gm-Cf-Cm-Cf- Um-Uf-Cm-Af-Cm-Uf | 1094/896 | Ams-Gfs-Um-Gf-Am-Af-Gm-Gf- Gm-Cf-Am-Cf-Um-Uf-Gm-Af-Gm- Uf-Cm-Af-Cms-Cfs-Gm | 1180/982 |

### Example 18: Inhibitory effect of alternately modified sequences on AGT gene expression

The siRNA sequences alternately modified with 2'-OMe and 2'-F synthesized in Example 17 were transfected into HepG2 cells via lipid nanoparticles (LNPs). The inhibitory effects of these sequences on AGT gene expression were then evaluated using qPCR.

### 1. Experimental materials

Test sample: the alternately modified siRNA sequences listed in Table 52 (synthesized in Example 17).

Cell line: HepG2 cells.

Solvents: sterile nuclease-free water, Gibco Opti-MEM.

### 2. Experimental methods

The inhibitory effects of the test samples on AGT mRNA expression in HepG2 cells were evaluated by qRT-PCR.

### 2.1 Cell culture

Subcultured HepG2 cells in the logarithmic growth phase were cultured in DMEM culture medium containing 10% fetal bovine serum (supplemented with 100× penicillin and 100× streptomycin at 10 µL/mL), and incubated in a cell incubator at 37°C with 5% CO₂. The medium was exchanged once daily. The cells were digested with 0.25% trypsin, followed by centrifugation at 800 rpm for 3 minutes. The supernatant was discarded, and fresh medium was added for continued culture.

### 2.2 Cell transfection

Preparation of transfection mixture: Lipofectamine RNAiMAX was mixed with Opti-MEM at a volume ratio of 2:98 and vortexed thoroughly.

Preparation of transfection reagent: 65 µL of siRNA solution (diluted in Opti-MEM) was mixed with 65 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 65 µL of transfection mixture was added to 65 µL of Opti-MEM, vortexed thoroughly, followed by incubation at room temperature for 15 minutes.

The prepared transfection reagent was added to a 24-well cell culture plate (100 µL per well), resulting in a final siRNA concentration of 0.07 nM per well. Subsequently, 500 µL of cell suspension (1.5 × 10⁵ cells per mL) was added to each well. After cross-mixing, the plate was incubated in a cell incubator at 37°C with 5% CO₂ for 40 hours.

### 2.3 AGT mRNA analysis

1) RNA extraction
   a. The culture medium was removed from a 12-well plate. 0.5 mL of 1× PBS was then added to each well to wash the cells, and the PBS was discarded. 0.5 mL of TRIzol reagent was added to each well, followed by pipetting to ensure complete lysis of the cells. The lysate was transferred to a 1.5 mL RNase-free EP tube, followed by incubation at room temperature for 5 minutes.
   b. 0.1 mL of chloroform was added to each tube, shaken vigorously for 15 seconds, followed by incubation at room temperature for 5 minutes. After centrifugation at 12000 × g for 15 minutes at 4°C, 200 µL of the supernatant was transferred to a new EP tube.
   c. An equal volume of isopropanol was added to the tube, which was gently mixed by inversion, followed by incubation at -20°C for 10 minutes. After centrifugation at 12000 × g for 15 minutes at 4°C, the supernatant was discarded.
   d. 0.5 mL of 75% ethanol was added to gently wash the RNA pellet, followed by centrifugation at 12000 × g for 5 minutes at 4°C, and the supernatant was discarded. The washing step was repeated once, followed by centrifugation at 12000 × g for 1 minute at 4°C, and residual ethanol was thoroughly removed using a micropipette tip.
   e. The residual ethanol was air-dried at room temperature for 2 to 3 minutes, followed by addition of 40 µL of RNase-free ddH₂O for dissolution.
2) Measurement of RNA concentration
   RNA concentration was measured using a NanoDrop spectrophotometer, using 2 µL of RNase-free ddH₂O as a blank control and 2 µL of RNA sample for each measurement. The sample concentration was recorded.
3) Reverse transcription of AGT mRNA
   Reverse transcription reagent, RNA solution, and water were mixed at a volume ratio of 2:5:3, followed by incubation in a PCR instrument at 37°C for 15 minutes and 85°C for 5 seconds, and finally held at 4°C. The resulting cDNA was diluted 5-fold with sterile nuclease-free water.
4) Quantitative analysis of AGT mRNA

In a 15 mL centrifuge tube, qPCR reagent, forward primer, and reverse primer were mixed at a volume ratio of 5:0.1:0.1 to obtain solution A.

In a labeled 1.5 mL EP tube, diluted cDNA and water were mixed at a volume ratio of 1:3.8 to obtain solution B.

5.2 µL of solution A and 4.8 µL of solution B were added to each well of a 96-well PCR plate, which was sealed, then centrifuged at 3000 rpm for 1 minute, and subjected to instrumental analysis.

*The step was performed on ice under low-temperature conditions.

The plate was placed into the qPCR instrument and run according to the following program:
Pre-denaturation: 95°C for 30 seconds;
Cycling: 95°C for 5 seconds; 60°C for 34 seconds; 40 cycles;
Melting curve: 95°C for 15 seconds; 60°C for 60 seconds; 95°C for 15 seconds.

Total run time: approximately 2 hours. After analysis of the experimental results, 2^{-ΔΔCt} was calculated.

### 2.4 Data processing

AGT mRNA expression rate (%) was calculated using the following formula: 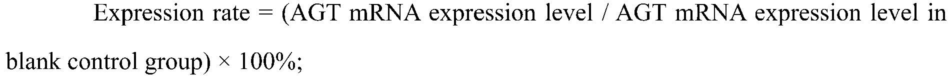 Expression rate = (AGT mRNA expression level / AGT mRNA expression level in blank control group) × 100%;
$Inhibition rate of AGT gene expression = 100 % - expression rate \left(%\right) .$

### 3. Experimental results

In this example, the inhibition rate represents the average of three independent experiments. The inhibition rates of AGT mRNA expression in HepG2 cells by these sequences are shown in Tables 53 to 56.

The experimental results demonstrated that certain sequences with alternating 2'-OMe and 2'-F modifications, including B1789-AL, exhibited significant inhibitory effects on AGT mRNA expression in HepG2 cells, with inhibition rates exceeding 45%. Specifically, B1576s-AL exhibited an inhibition rate exceeding 60%.

The other sequences exhibited relatively weak inhibitory effects on AGT mRNA expression, with inhibition rates below 45%.
**(1) Among the 99 designed sequences, 33 sequences exhibited significant inhibitory effects AGT gene expression, with inhibition rates exceeding 40%. Some of these sequences exhibited inhibition rates exceeding 60%.**

**Table 53: Alternately modified sequences with significant inhibitory effects on AGT gene expression (inhibition rate > 40%)**

| **Unmodified sequence ID** | **Alternately modified sequence ID** | **Inhibition rate (%)** | **Unmodified sequence ID** | **Alternately modified sequence ID** | **Inhibition rate (%)** |
|---|---|---|---|---|---|
| 1576s | B1576s-AL | 64.6 | 734s | B734s-AL | 56.4 |
| 1578s | B1578s-AL | 63.9 | 1798s | B1798s-AL | 54.4 |
| 1838 | B1838-AL | 63.6 | 736 | B736-AL | 53.3 |
| 1789 | B 1789-AL | 63.5 | 1608 | B1608-AL | 52.5 |
| 1812 | B1812-AL | 62.9 | 1282 | B1282-AL | 51.8 |
| 1835 | B1835-AL | 62.3 | 1811 | B1811-AL | 51.5 |
| 1791 | B1791-AL | 61.8 | 1281 | B1281-AL | 50.4 |
| 1579 | B1579-AL | 61.1 | 1799 | B1799-AL | 49.2 |
| 1816 | B1816-AL | 60.9 | 1603 | B1603-AL | 48.6 |
| 1790 | B 1790-AL | 60.3 | 1828 | B1828-AL | 46.6 |
| 1836 | B1836-AL | 59.8 | 1592 | B1592-AL | 44.4 |
| 1585s | B1585s-AL | 59.3 | 1814 | B1814-AL | 43.2 |
| 1839 | B1839-AL | 59.1 | 1593 | B1593-AL | 42.4 |
| 1838s | B1838s-AL | 58.4 | 1810 | B1810-AL | 42.1 |
| 1796 | B 1796-AL | 57.9 | 1365 | B1365-AL | 42.0 |
| 1795 | B 1795-AL | 57.5 | 1279 | B1279-AL | 41.4 |
| 1817 | B1817-AL | 57.3 | | | |

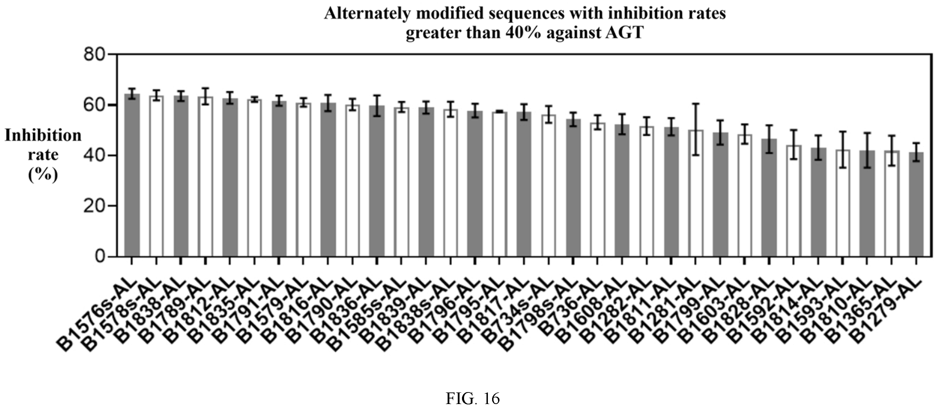

As shown in Table 53, the alternately modified sequences listed in the above table (see Table 52 for specific sequences) exhibited significant inhibitory effects on AGT mRNA expression, with inhibition rates exceeding 40%. Specifically, the top 10 sequences exhibited inhibition rates exceeding 60% (FIG. 16).

The sequences of the compounds listed in Table 53 are shown in Table 54 below.

**Table 54: siRNA sequences with significant inhibitory effects on AGT gene expression**

| **Sequence ID** | **Sense sequence 5'-3'** | **SEQ ID NO:/corr. unmodified SEQ ID NO:** | **Antisense sequence 5'-3'** | **SEQ ID NO:/corr. unmodified SEQ ID NO:** |
|---|---|---|---|---|
| B1576sAL | Cms-Cfs-Am-Cf-Cm-Uf-Um- Uf-Um-Cf-Um-Uf-Cm-Uf- Am-Af-Um-Gf-Am-Af | 993/795 | Ums-Ufs-Cm-Af-Um-Uf-Am-Gf- Am-Af-Gm-Af-Am-Af-Am-Gf- Gm-Uf-Gm-Gfs-Gms-Af | 1006/808 |
| B1578sAL | Ams-Cfs-Cm-Uf-Um-Uf-Um- Cf-Um-Uf-Cm-Uf-Am-Af- Um-Gf-Am-Gf-Um-Af | 994/796 | Ums-Afs-Cm-Uf-Cm-Af-Um-Uf- Am-Gf-Am-Af-Gm-Af-Am-Af- Am-Gf-Gm-Ufs-Gms-Gf | 1007/809 |
| B1838-AL | Gfs-Gms-Uf-Um-Uf-Um-Af- Am-Af-Am-Uf-Um-Af-Am- Af-Gm-Uf-Am-Uf-Am-Af | 995/797 | Ums-Ufs-Am-Uf-Am-Cf-Um-Uf- Um-Af-Am-Uf-Um-Uf-Um-Af- Am-Af-Am-Cf-Cms-Cfs-Am | 1008/810 |
| B1789-AL | Gfs-Ums-Uf-Um-Gf-Um-Gf- Am-Af-Am-Cf-Am-Af-Am- Af-Am-Af-Gm-Uf-Gm-Uf | 988/1238 | Ams-Cfs-Am-Cf-Um-Uf-Um-Uf- Um-Uf-Gm-Uf-Um-Uf-Cm-Af- Cm-Af-Am-Af-Cms-Afs-Am | 1001/803 |
| B1812-AL | Cfs-Cms-Uf-Um-Uf-Um-Cf- Am-Af-Gm-Uf-Um-Gf-Am- Gf-Am-Af-Cm-Af-Am-Af | 985/787 | Ums-Ufs-Um-Gf-Um-Uf-Cm-Uf- Cm-Af-Am-Cf-Um-Uf-Gm-Af- Am-Af-Am-Gf-Gms-Gfs-Am | 998/800 |
| B1835-AL | Ufs-Ums-Gf-Gm-Gf-Um-Uf- Um-Uf-Am-Af-Am-Af-Um- Uf-Am-Af-Am-Gf-Um-Af | 983/785 | Ums-Afs-Cm-Uf-Um-Uf-Am-Af- Um-Uf-Um-Uf-Am-Af-Am-Af- Cm-Cf-Cm-Af-Ams-Ufs-Um | 996/798 |
| B1791-AL | Ufs-Ums-Gf-Um-Gf-Am-Af- Am-Cf-Am-Af-Am-Af-Am- Af-Gm-Uf-Gm-Uf-Um-Af | 990/792 | Ums-Afs-Am-Cf-Am-Cf-Um-Uf- Um-Uf-Um-Uf-Gm-Uf-Um-Uf- Cm-Af-Cm-Af-Ams-Afs-Cm | 1003/805 |
| B1579-AL | Cfs-Cms-Uf-Um-Uf-Um-Cf- Um-Uf-Cm-Uf-Am-Af-Um- Gf-Am-Gf-Um-Cf-Gm-Af | 986/788 | Ums-Cfs-Gm-Af-Cm-Uf-Cm-Af- Um-Uf-Am-Gf-Am-Af-Gm-Af- Am-Af-Am-Gf-Gms-Ufs-Gm | 999/801 |
| B1816-AL | Ufs-Ums-Cf-Am-Af-Gm-Uf- Um-Gf-Am-Gf-Am-Af-Cm- Af-Am-Af-Am-Af-Um-Uf | 984/786 | Ams-Afs-Um-Uf-Um-Uf-Um-Gf- Um-Uf-Cm-Uf-Cm-Af-Am-Cf- Um-Uf-Gm-Af-Ams-Afs-Am | 997/799 |
| B1790-AL | Ufs-Ums-Uf-Gm-Uf-Gm-Af- Am-Af-Cm-Af-Am-Af-Am- Af-Am-Gf-Um-Gf-Um-Uf | 1061/863 | Ams-Afs-Cm-Af-Cm-Uf-Um-Uf- Um-Uf-Um-Gf-Um-Uf-Um-Cf- Am-Cf-Am-Af-Ams-Cfs-Am | 1147/949 |
| B1836-AL | Ufs-Gms-Gf-Gm-Uf-Um-Uf- Um-Af-Am-Af-Am-Uf-Um- Af-Am-Af-Gm-Uf-Am-Uf | 987/789 | Ams-Ufs-Am-Cf-Um-Uf-Um-Af- Am-Uf-Um-Uf-Um-Af-Am-Af- Am-Cf-Cm-Cf-Ams-Afs-Um | 1000/802 |
| B1585sAL | Cms-Ufs-Um-Cf-Um-Af-Am- Uf-Gm-Af-Gm-Uf-Cm-Gf- Am-Cf-Um-Uf-Um-Af | 992/794 | Ums-Afs-Am-Af-Gm-Uf-Cm-Gf- Am-Cf-Um-Cf-Am-Uf-Um-Af- Gm-Af-Am-Gfs-Ams-Af | 1005/807 |
| B1839-AL | Gfs-Ums-Uf-Um-Uf-Am-Af- Am-Af-Um-Uf-Am-Af-Am- Gf-Um-Af-Um-Af-Cm-Af | 989/791 | Ums-Gfs-Um-Af-Um-Af-Cm-Uf- Um-Uf-Am-Af-Um-Uf-Um-Uf- Am-Af-Am-Af-Cms-Cfs-Cm | 1002/804 |
| B1838sAL | Gms-Gfs-Um-Uf-Um-Uf-Am- Af-Am-Af-Um-Uf-Am-Af- Am-Gf-Um-Af-Um-Af | 1071/873 | Ums-Afs-Um-Af-Cm-Uf-Um-Uf- Am-Af-Um-Uf-Um-Uf-Am-Af- Am-Af-Cm-Cfs-Cms-Af | 1157/959 |
| B1796-AL | Afs-Ams-Af-Cm-Af-Am-Af- Am-Af-Am-Gf-Um-Gf-Um- Uf-Cm-Cf-Cm-Uf-Um-Uf | 1062/864 | Ams-Afs-Am-Gf-Gm-Gf-Am-Af- Cm-Af-Cm-Uf-Um-Uf-Um-Uf- Um-Gf-Um-Uf-Ums-Cfs-Am | 1148/950 |
| B1795-AL | Gfs-Ams-Af-Am-Cf-Am-Af- Am-Af-Am-Af-Gm-Uf-Gm- Uf-Um-Cf-Cm-Cf-Um-Uf | 991/793 | Ams-Afs-Gm-Gf-Gm-Af-Am-Cf- Am-Cf-Um-Uf-Um-Uf-Um-Uf- Gm-Uf-Um-Uf-Cms-Afs-Cm | 1004/806 |
| B1817-AL | Ufs-Cms-Af-Am-Gf-Um-Uf- Gm-Af-Gm-Af-Am-Cf-Am- Af-Am-Af-Am-Uf-Um-Gf | 1069/871 | Cms-Afs-Am-Uf-Um-Uf-Um-Uf- Gm-Uf-Um-Cf-Um-Cf-Am-Af- Cm-Uf-Um-Gf-Ams-Afs-Am | 1155/957 |
| B734s-AL | Ums-Gfs-Gm-Af-Um-Gf-Um- Uf-Gm-Cf-Um-Gf-Cm-Uf- Gm-Af-Gm-Af-Am-Af | 1083/885 | Ums-Ufs-Um-Cf-Um-Cf-Am-Gf- Cm-Af-Gm-Cf-Am-Af-Cm-Af- Um-Cf-Cm-Afs-Gms-Uf | 1169/971 |
| B1798sAL | Ams-Cfs-Am-Af-Am-Af-Am- Af-Gm-Uf-Gm-Uf-Um-Cf- Cm-Cf-Um-Uf-Um-Uf | 1063/865 | Ams-Afs-Am-Af-Gm-Gf-Gm-Af- Am-Cf-Am-Cf-Um-Uf-Um-Uf- Um-Uf-Gm-Ufs-Ums-Uf | 1149/951 |
| B736-AL | Gfs-Ams-Uf-Gm-Uf-Um-Gf- Cm-Uf-Gm-Cf-Um-Gf-Am- Gf-Am-Af-Gm-Af-Um-Uf | 1084/886 | Ams-Afs-Um-Cf-Um-Uf-Cm-Uf- Cm-Af-Gm-Cf-Am-Gf-Cm-Af- Am-Cf-Am-Uf-Cms-Cfs-Am | 1170/972 |
| B1608-AL | Ufs-Gms-Gf-Am-Af-Am-Gf- Cm-Af-Gm-Cf-Cm-Gf-Um- Uf-Um-Cf-Um-Cf-Cm-Uf | 1058/860 | Ams-Gfs-Gm-Af-Gm-Af-Am-Af- Cm-Gf-Gm-Cf-Um-Gf-Cm-Uf- Um-Uf-Cm-Cf-Ams-Gfs-Cm | 1144/946 |
| B1282-AL | Afs-Gms-Cf-Am-Uf-Um-Uf- Um-Uf-Um-Uf-Um-Gf-Am- Gf-Cm-Uf-Um-Gf-Am-Af | 1038/840 | Ums-Ufs-Cm-Af-Am-Gf-Cm-Uf- Cm-Af-Am-Af-Am-Af-Am-Af- Am-Uf-Gm-Cf-Ums-Gfs-Um | 1124/926 |
| B1811-AL | Cfs-Cms-Cf-Um-Uf-Um-Uf- Cm-Af-Am-Gf-Um-Uf-Gm- Af-Gm-Af-Am-Cf-Am-Af | 1066/868 | Ums-Ufs-Gm-Uf-Um-Cf-Um-Cf- Am-Af-Cm-Uf-Um-Gf-Am-Af- Am-Af-Gm-Gf-Gms-Afs-Am | 1152/954 |
| B1281-AL | Cfs-Ams-Gf-Cm-Af-Um-Uf- Um-Uf-Um-Uf-Um-Uf-Gm- Af-Gm-Cf-Um-Uf-Gm-Af | 1037/839 | Ums-Cfs-Am-Af-Gm-Cf-Um-Cf- Am-Af-Am-Af-Am-Af-Am-Af- Um-Gf-Cm-Uf-Gms-Ufs-Um | 1123/925 |
| B1799-AL | Cfs-Ams-Af-Am-Af-Am-Af- Gm-Uf-Gm-Uf-Um-Cf-Cm- Cf-Um-Uf-Um-Uf-Cm-Af | 1064/866 | Ums-Gfs-Am-Af-Am-Af-Gm-Gf- Gm-Af-Am-Cf-Am-Cf-Um-Uf- Um-Uf-Um-Uf-Gms-Ufs-Um | 1150/952 |
| B1603-AL | Ufs-Gms-Af-Gm-Cf-Um-Gf- Gm-Af-Am-Af-Gm-Cf-Am- Gf-Cm-Cf-Gm-Uf-Um-Uf | 1057/859 | Ams-Afs-Am-Cf-Gm-Gf-Cm-Uf- Gm-Cf-Um-Uf-Um-Cf-Cm-Af- Gm-Cf-Um-Cf-Ams-Afs-Am | 1143/945 |
| B1828-AL | Afs-Cms-Af-Am-Af-Am-Af- Um-Uf-Gm-Gf-Gm-Uf-Um- Uf-Um-Af-Am-Af-Am-Uf | 1070/872 | Ams-Ufs-Um-Uf-Um-Af-Am-Af- Am-Cf-Cm-Cf-Am-Af-Um-Uf- Um-Uf-Um-Gf-Ums-Ufs-Cm | 1156/958 |
| B1592-AL | Ufs-Gms-Af-Gm-Uf-Cm-Gf- Am-Cf-Um-Uf-Um-Gf-Am- Gf-Cm-Uf-Gm-Gf-Am-Af | 1053/855 | Ums-Ufs-Cm-Cf-Am-Gf-Cm-Uf- Cm-Af-Am-Af-Gm-Uf-Cm-Gf- Am-Cf-Um-Cf-Ams-Ufs-Um | 1139/941 |
| B1814-AL | Ufs-Ums-Uf-Um-Cf-Am-Af- Gm-Uf-Um-Gf-Am-Gf-Am- Af-Cm-Af-Am-Af-Am-Af | 1067/869 | Ums-Ufs-Um-Uf-Um-Gf-Um-Uf- Cm-Uf-Cm-Af-Am-Cf-Um-Uf- Gm-Af-Am-Af-Ams-Gfs-Gm | 1153/955 |
| B1593-AL | Gfs-Ams-Gf-Um-Cf-Gm-Af- Cm-Uf-Um-Uf-Gm-Af-Gm- Cf-Um-Gf-Gm-Af-Am-Af | 1054/856 | Ums-Ufs-Um-Cf-Cm-Af-Gm-Cf- Um-Cf-Am-Af-Am-Gf-Um-Cf- Gm-Af-Cm-Uf-Cms-Afs-Um | 1140/942 |
| B1810-AL | Ufs-Cms-Cf-Cm-Uf-Um-Uf- Um-Cf-Am-Af-Gm-Uf-Um- Gf-Am-Gf-Am-Af-Cm-Af | 1065/867 | Ums-Gfs-Um-Uf-Cm-Uf-Cm-Af- Am-Cf-Um-Uf-Gm-Af-Am-Af- Am-Gf-Gm-Gf-Ams-Afs-Cm | 1151/953 |
| B1365-AL | Gfs-Ams-Cf-Cm-Cf-Um-Gf- Am-Af-Cm-Cf-Gm-Cf-Cm-Cf- Am-Uf-Um-Cf-Cm-Uf | 1040/842 | Ams-Gfs-Gm-Af-Am-Uf-Gm-Gf- Gm-Cf-Gm-Gf-Um-Uf-Cm-Af- Gm-Gf-Gm-Uf-Cms-Afs-Cm | 1126/928 |
| B1279-AL | Afs-Ams-Cf-Am-Gf-Cm-Af- Um-Uf-Um-Uf-Um-Uf-Um- Uf-Gm-Af-Gm-Cf-Um-Uf | 1036/838 | Ams-Afs-Gm-Cf-Um-Cf-Am-Af- Am-Af-Am-Af-Am-Af-Um-Gf- Cm-Uf-Gm-Uf-Ums-Cfs-Am | 1122/924 |

**(2) A total of 23 sequences exhibited inhibition rates of 25% to 40% on AGT gene expression. For example, the inhibition rates of B734-AL and B994-AL were 39.2% and 37.3%, respectively.**

**Table 55: Alternately modified sequences with inhibition rates of 25% to 40% on AGT gene expression**

| **Unmodified sequence ID** | **Sequence ID** | **Inhibition rate (%)** | **Unmodified sequence ID** | **Sequence ID** | **Inhibition rate (%)** |
|---|---|---|---|---|---|
| 734 | B734-AL | 39.2 | 2020 | B2020-AL | 30.4 |
| 1591 | B1591-AL | 37.9 | 753s | B753s-AL | 30.2 |
| 969 | B969-AL | 37.3 | 2032 | B2032-AL | 30.2 |
| 994 | B994-AL | 37.3 | 1602 | B1602-AL | 29.0 |
| 984 | B984-AL | 36.9 | 1029 | B1029-AL | 28.6 |
| 743 | B743-AL | 33.8 | 1612 | B1612-AL | 28.6 |
| 1393 | B1393-AL | 33.4 | 2018 | B2018-AL | 27.9 |
| 2030 | B2030-AL | 33.0 | 2025 | B2025-AL | 26.6 |
| 1267 | B1267-AL | 31.5 | 731 | B731-AL | 26.4 |
| 2033 | B2033-AL | 31.5 | 1011 | B1011-AL | 26.2 |
| 2031 | B2031-AL | 31.0 | 732 | B732-AL | 25.4 |
| 1815 | B1815-AL | 30.4 | | | |

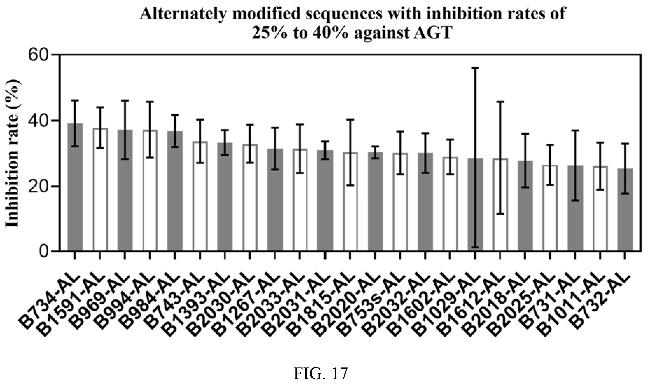

The 22 sequences listed in Table 55 exhibited relatively weak inhibitory effects on AGT gene expression, with inhibition rates below 40%, ranging from 25% to 40%. For example, the inhibition rates of B734-AL and B994-AL were 39.2% and 37.3%, respectively (FIG. 17).

**(3) A total of 43 sequences exhibited inhibition rates below 25% on AGT gene expression. For example, the inhibition rate of B1017-AL was only 23.1%.**

**Table 56: Alternately modified sequences with inhibition rates below 25% on AGT gene expression**

| **Unmodified sequence ID** | **Sequence ID** | **Inhibition rate (%)** | **Unmodified sequence ID** | **Sequence ID** | **Inhibition rate (%)** |
|---|---|---|---|---|---|
| 1030 | B1030-AL | 23.5 | 1041 | B1041-AL | 6.0 |
| 1017 | B1017-AL | 23.1 | 1008 | B1008-AL | 5.6 |
| 992 | B992-AL | 20.5 | 966 | B966-AL | 4.8 |
| 1362 | B1362-AL | 17.5 | 1046 | B1046-AL | 4.5 |
| 1061 | B1061-AL | 17.1 | 1071 | B1071-AL | 4.1 |
| 982 | B982-AL | 17.1 | 1038 | B1038-AL | 3.9 |
| 729 | B729-AL | 16.4 | 1016 | B1016-AL | 3.5 |
| 1020 | B1020-AL | 16.3 | 1026 | B1026-AL | 3.4 |
| 1775 | B1775-AL | 15.5 | 1554 | B1554-AL | 1.7 |
| 1596 | B1596-AL | 15.0 | 1367 | B1367-AL | 1.6 |
| 971 | B971-AL | 15.0 | 1407 | B1407-AL | 1.3 |
| 1057 | B1057-AL | 14.8 | 1260 | B1260-AL | 0.9 |
| 1268 | B1268-AL | 14.2 | 1558 | B1558-AL | 0.9 |
| 1564 | B1564-AL | 13.8 | 1401 | B1401-AL | -0.6 |
| 1028 | B1028-AL | 10.4 | 1053 | B1053-AL | -1.6 |
| 1065s | B1065s-AL | 10.0 | 1561 | B1561-AL | -2.0 |
| 1024 | B1024-AL | 9.9 | 1404 | B1404-AL | -2.4 |
| 1044 | B1044-AL | 9.9 | 1369 | B1369-AL | -2.8 |
| 963 | B963-AL | 9.8 | 1562 | B1562-AL | -3.3 |
| 1266 | B1266-AL | 8.2 | 1262 | B1262-AL | -6.0 |
| 1014 | B1014-AL | 6.5 | 1265 | B1265-AL | -8.8 |
| 1047 | B1047-AL | 6.4 | | | |

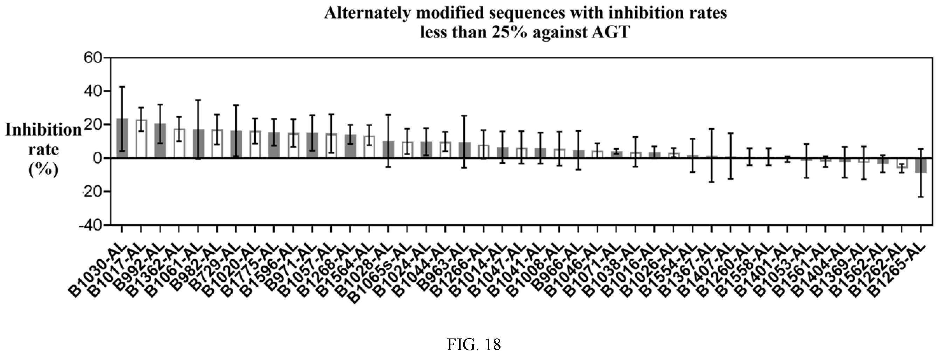

The 43 sequences listed in Table 56 exhibited very weak inhibitory effects on AGT mRNA expression, with inhibition rates below 25%. For example, the inhibition rate of B1017-AL was only 23.1% (FIG. 18).

**(4) siRNAs with similar sequences exhibited significant differences in activity. For example, the inhibition rate of B1365-AL was 40.4% higher than that of B1367-AL, indicating a significant improvement.**

A comparison of the inhibitory effects of certain siRNAs with similar sequences on AGT mRNA expression is shown in Table 57.

**Table 57: Comparison of inhibition rates of AGT gene expression by siRNAs with similar sequences**

| **Group of similar sequences** | **Unmodified seq ID** | **Seq ID** | **Corr. unmodified sense strand 5'-3'** | **Corr. unmodified SEQ ID NO:** | **Corr. unmodified antisense strand 5'-3'** | **Corr. unmodified SEQ ID NO:** | **Inh (%)** |
|---|---|---|---|---|---|---|---|
| A | 1367 | B1367-AL | CCCUGAACCGC CCAUUCCUGU | 843 | ACAGGAAUGGGC GGUUCAGGUC | 929 | 1.6 |
| | 1365 | B1365-AL | GACCCUGAACC GCCCAUUCCU | 842 | AGGAAUGGGCGG UUCAGGGUCAC | 928 | 42.0 |
| B | 1815 | B1815-AL | UUUCAAGUUGA GAACAAAAU | 870 | AUUUUUGUUCUC AACUUGAAAAG | 956 | 30.4 |
| | 1816 | B1816-AL | UUCAAGUUGAG AACAAAAAUU | 786 | AAUUUUUGUUCU CAACUUGAAAA | 799 | 60.9 |
| C | 729 | B729-AL | AGAACUGGAUG UUGCUGCUGA | 881 | UCAGCAGCAACA UCCAGUUCUGU | 967 | 16.4 |
| | 734s | B734sAL | UGGAUGUUGCU GCUGAGAAA | 885 | UUUCUCAGCAGC AACAUCCAGU | 971 | 56.4 |

As shown in Table 57, certain siRNAs with similar sequences exhibited vastly different inhibitory effects on AGT mRNA expression.

Compared with unmodified sequence 1365, unmodified sequence 1367 differs only in two bases at the terminal positions. For the sense strand, the 5' end of unmodified sequence 58 is GA, while the 3' end of unmodified sequence 59 is GU, with all other bases being identical. For the antisense strand, the 3' end of unmodified sequence 144 is AC, while the 5' end of unmodified sequence 145 is AC, with all other bases being identical. However, the inhibition rate of the alternately modified sequence B1365-AL was 40.4% higher than that of B1367-AL, indicating a significant improvement.

Compared with unmodified sequence 1816, unmodified sequence 1815 differs only in one base at the terminal position. For the sense strand, the 5' end of unmodified sequence 86 is U, while the 3' end of unmodified sequence 2 is U, with all other bases being identical. For the antisense strand, the 3' end of unmodified sequence 172 is G, while the 5' end of unmodified sequence 15 is A, with all other bases being identical. However, the inhibition rate of the alternately modified sequence B1816-AL was 30.5% higher than that of B1815-AL, indicating a significant improvement.

Compared with unmodified sequence 734s, unmodified sequence 729 differs only in five bases at the terminal positions. For the sense strand, the 5' end of unmodified sequence 97 is AGAAC, while the 3' end of unmodified sequence 101 is GAAA, with all other bases being identical. For the antisense strand, the 3' end of unmodified sequence 183 is UCUGU, while the 5' end of unmodified sequence 187 is UUUC, with all other bases being identical. However, the inhibition rate of the alternately modified sequence B734s-AL was 40.0% higher than that of B729-AL, indicating a significant improvement.

Therefore, identifying oligonucleotide sequences with significant inhibitory activity from a vast number of candidate sequences designed against the AGT mRNA sequence is not straightforward and requires substantial creative effort.

### Summary:

(1) Among the 99 designed sequences, 33 sequences exhibited significant inhibitory effects AGT gene expression, with inhibition rates exceeding 40%. Ten of these sequences exhibited inhibition rates exceeding 60%.
(2) A total of 23 sequences exhibited inhibition rates of 25% to 40% on AGT gene expression. For example, the inhibition rates of B734-AL and B994-AL were 39.2% and 37.3%, respectively.
(3) A total of 43 sequences exhibited inhibition rates below 25% on AGT gene expression. For example, the inhibition rate of B1017-AL was only 23.1%.
(4) siRNAs with similar sequences exhibited significant differences in activity. For example, the inhibition rate of B1365-AL was 40.4% higher than that of B1367-AL, indicating a significant improvement. Therefore, identifying oligonucleotide sequences with significant inhibitory activity from a vast number of candidate sequences designed against the AGT mRNA sequence is not straightforward and requires substantial creative effort.

### Example 19: Inhibitory effect of unmodified sequences on AGT gene expression

In this example, certain unmodified sequences corresponding to the modified sequences in Example 18 were synthesized and transfected into HepG2 cells via lipid nanoparticles (LNPs). The inhibitory effects of these unmodified sequences on AGT gene expression were evaluated using qPCR to identify unmodified siRNA sequences with favorable inhibitory activity.

### 1. Experimental materials

Test samples: The unmodified siRNA sequences listed in Table 58.

### 2. Synthesis of unmodified sequence 1579

The siRNA sequence designated as 1579 in Table 58 is as follows:
Sense strand: 5'-CCUUUUCUUCUAAUGAGUCGA-3' (SEQ ID NO: 788)
Antisense strand: 5'-UCGACUCAUUAGAAGAAAAGGUG-3' (SEQ ID NO: 801)

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-2'-O-TBDMS phosphoramidite monomer (Formula 13), DMT-C-2'-O-TBDMS phosphoramidite monomer (Formula 14), DMT-G-2'-O-TBDMS phosphoramidite monomer (Formula 15), and DMT-U-2'-O-TBDMS phosphoramidite monomer (Formula 16).

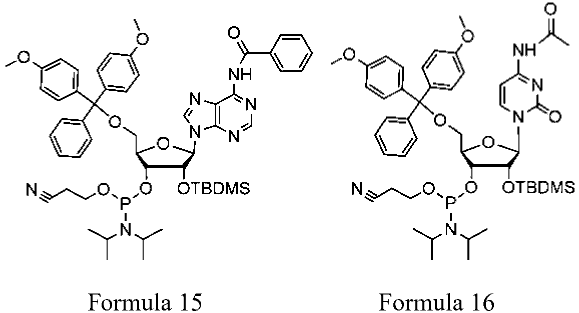

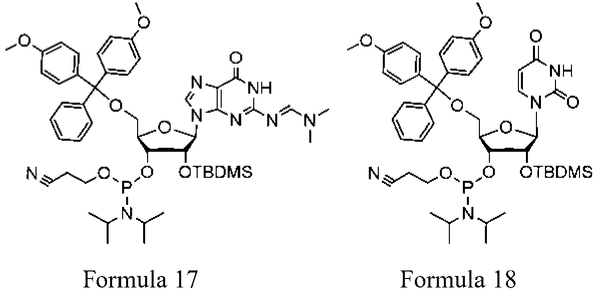

The preparation was carried out according to the following steps:
The solid-phase support was loaded into the designated position of the synthesizer, followed by multiple synthesis cycles to obtain a desired fully protected product, each cycle consisting of (1) deprotection, (2) coupling, (3) oxidation/sulfurization, and (4) hydroxyl protection. The cycle process and reagents used are described as follows:
(1) Deprotection
   3% dichloroacetic acid in toluene was used as a deprotection reagent to remove the DMT protecting group, followed by washing with acetonitrile.
(2) Coupling
   Each nucleotide monomer in acetonitrile was coupled using 0.25 M 5-ethylthio-1*H-*tetrazole as an activator, followed by washing with acetonitrile.
(3) Oxidation/sulfurization
   Oxidation: 0.05 M iodine in pyridine/water (90/10) was used as an oxidant for oxidation, followed by washing with acetonitrile.
   Sulfurization: 3% xanthane hydride in pyridine was used as a sulfurizing agent for sulfurization, followed by washing with acetonitrile.
(4) Hydroxyl protection
   10% acetic anhydride in tetrahydrofuran (CAP A) and tetrahydrofuran/pyridine/*N-*methylimidazole (74/10/16, v/v/v) (CAP B) were used as hydroxyl protecting reagents for hydroxyl protection, followed by washing with acetonitrile.
   The above steps were repeated according to the set sequence to obtain a fully protected product.
(5) 3% dichloroacetic acid in toluene was used as a deprotection reagent to remove the DMT protecting group from the last nucleotide, followed by washing with acetonitrile.
(6) Ammonolysis and purification
   The solid-phase support was transferred to a reactor and treated with concentrated aqueous ammonia (25 to 28%) at 60°C for 12 hours. The system was then cooled to room temperature, and the mixture was transferred to a pressure filter, followed by washing with a mixture of purified water and ethanol. The filtrates were combined, subjected to column chromatography, concentrated, and lyophilized to obtain a 2'-O-TBDMS-protected product.
(7) Removal of TBDMS protecting groups
   The resulting product was treated with DMSO and triethylamine hydrofluoride at 60°C for 2 hours. An aqueous ammonium acetate solution was then added to the reaction mixture and vortexed thoroughly. The resulting mixture was added with anhydrous ethanol, vortexed thoroughly, followed by crystallization at -20°C for 8 to 12 hours. After centrifugation, the supernatant was discarded, and the precipitate was washed with anhydrous ethanol to obtain an unmodified single-stranded product.
(8) Annealing

The resulting unmodified sense and antisense strands were mixed at a molar ratio of 1:1, heated to 95°C and maintained for 3 minutes, and slowly cooled to room temperature to form an unmodified duplex.

Unmodified sequence 1579 exhibited a purity of 97.4% and a measured molecular weight of 13990.9.

### 3. Synthesis of other sequences

All other sequences listed in Table 58 were synthesized using the method described above.

**Table 58: Unmodified siRNA sequences**

| **Seq ID** | **Sense sequence (unmodified sequence) 5'-3'** | **SEQ ID NO:** | **Antisense sequence (unmodified sequence) 5'-3'** | **SEQ ID NO:** |
|---|---|---|---|---|
| 1835 | UUGGGUUUUAAAAUUAAAGUA | 785 | UACUUUAAUUUUAAAACCCAAUU | 798 |
| 1816 | UUCAAGUUGAGAACAAAAAUU | 786 | AAUUUUUGUUCUCAACUUGAAAA | 799 |
| 1812 | CCUUUUCAAGUUGAGAACAAA | 787 | UUUGUUCUCAACUUGAAAAGGGA | 800 |
| 1579 | CCUUUUCUUCUAAUGAGUCGA | 788 | UCGACUCAUUAGAAGAAAAGGUG | 801 |
| 1836 | UGGGUUUUAAAAUUAAAGUAU | 789 | AUACUUUAAUUUUAAAACCCAAU | 802 |
| 1789 | GUUUGUGAAACAAAAAAGUGU | 790 | ACACUUUUUUGUUUCACAAACAA | 803 |
| 1839 | GUUUUAAAAUUAAAGUAUACA | 791 | UGUAUACUUUAAUUUUAAAACCC | 804 |
| 1791 | UUGUGAAACAAAAAAGUGUUA | 792 | UAACACUUUUUUGUUUCACAAAC | 805 |
| 1795 | GAAACAAAAAAGUGUUCCCUU | 793 | AAGGGAACACUUUUUUGUUUCAC | 806 |
| 1585s | CUUCUAAUGAGUCGACUUUA | 794 | UAAAGUCGACUCAUUAGAAGAA | 807 |
| 1576s | CCACCUUUUCUUCUAAUGAA | 795 | UUCAUUAGAAGAAAAGGUGGGA | 808 |
| 1578s | ACCUUUUCUUCUAAUGAGUA | 796 | UACUCAUUAGAAGAAAAGGUGG | 809 |
| 1838 | GGUUUUAAAAUUAAAGUAUAA | 797 | UUAUACUUUAAUUUUAAAACCCA | 810 |
| 1008 | CUUCACUGAGAGCGCCUGCCU | 811 | AGGCAGGCGCUCUCAGUGAAGGG | 897 |
| 1011 | CACUGAGAGCGCCUGCCUGCU | 812 | AGCAGGCAGGCGCUCUCAGUGAA | 898 |
| 1014 | UGAGAGCGCCUGCCUGCUGCU | 813 | AGCAGCAGGCAGGCGCUCUCAGU | 899 |
| 1016 | AGAGCGCCUGCCUGCUGCUGA | 814 | UCAGCAGCAGGCAGGCGCUCUCA | 900 |
| 1017 | GAGCGCCUGCCUGCUGCUGAU | 815 | AUCAGCAGCAGGCAGGCGCUCUC | 901 |
| 1020 | CGCCUGCCUGCUGCUGAUCCA | 816 | UGGAUCAGCAGCAGGCAGGCGCU | 902 |
| 1024 | UGCCUGCUGCUGAUCCAGCCU | 817 | AGGCUGGAUCAGCAGCAGGCAGG | 903 |
| 1026 | CCUGCUGCUGAUCCAGCCUCA | 818 | UGAGGCUGGAUCAGCAGCAGGCA | 904 |
| 1028 | UGCUGCUGAUCCAGCCUCACU | 819 | AGUGAGGCUGGAUCAGCAGCAGG | 905 |
| 1029 | GCUGCUGAUCCAGCCUCACUA | 820 | UAGUGAGGCUGGAUCAGCAGCAG | 906 |
| 1030 | CUGCUGAUCCAGCCUCACUAU | 821 | AUAGUGAGGCUGGAUCAGCAGCA | 907 |
| 1038 | CCAGCCUCACUAUGCCUCUGA | 822 | UCAGAGGCAUAGUGAGGCUGGAU | 908 |
| 1041 | GCCUCACUAUGCCUCUGACCU | 823 | AGGUCAGAGGCAUAGUGAGGCUG | 909 |
| 1044 | UCACUAUGCCUCUGACCUGGA | 824 | UCCAGGUCAGAGGCAUAGUGAGG | 910 |
| 1046 | ACUAUGCCUCUGACCUGGACA | 825 | UGUCCAGGUCAGAGGCAUAGUGA | 911 |
| 1047 | CUAUGCCUCUGACCUGGACAA | 826 | UUGUCCAGGUCAGAGGCAUAGUG | 912 |
| 1053 | CUCUGACCUGGACAAGGUGGA | 827 | UCCACCUUGUCCAGGUCAGAGGC | 913 |
| 1057 | GACCUGGACAAGGUGGAGGGU | 828 | ACCCUCCACCUUGUCCAGGUCAG | 914 |
| 1061 | UGGACAAGGUGGAGGGUCUCA | 829 | UGAGACCCUCCACCUUGUCCAGG | 915 |
| 1065s | CAAGGUGGAGGGUCUCACUU | 830 | AAGUGAGACCCUCCACCUUGUC | 916 |
| 1071 | GGAGGGUCUCACUUUCCAGCA | 831 | UGCUGGAAAGUGAGACCCUCCAC | 917 |
| 1260 | CAGGGUGGGGGAGGUGCUGAA | 832 | UUCAGCACCUCCCCCACCCUGAU | 918 |
| 1262 | GGGUGGGGGAGGUGCUGAACA | 833 | UGUUCAGCACCUCCCCCACCCUG | 919 |
| 1265 | UGGGGGAGGUGCUGAACAGCA | 834 | UGCUGUUCAGCACCUCCCCCACC | 920 |
| 1266 | GGGGGAGGUGCUGAACAGCAU | 835 | AUGCUGUUCAGCACCUCCCCCAC | 921 |
| 1267 | GGGGAGGUGCUGAACAGCAUU | 836 | AAUGCUGUUCAGCACCUCCCCCA | 922 |
| 1268 | GGGAGGUGCUGAACAGCAUUU | 837 | AAAUGCUGUUCAGCACCUCCCCC | 923 |
| 1279 | AACAGCAUUUUUUUUGAGCUU | 838 | AAGCUCAAAAAAAAUGCUGUUCA | 924 |
| 1281 | CAGCAUUUUUUUUGAGCUUGA | 839 | UCAAGCUCAAAAAAAAUGCUGUU | 925 |
| 1282 | AGCAUUUUUUUUGAGCUUGAA | 840 | UUCAAGCUCAAAAAAAAUGCUGU | 926 |
| 1362 | GGUGACCCUGAACCGCCCAUU | 841 | AAUGGGCGGUUCAGGGUCACCUC | 927 |
| 1365 | GACCCUGAACCGCCCAUUCCU | 842 | AGGAAUGGGCGGUUCAGGGUCAC | 928 |
| 1367 | CCCUGAACCGCCCAUUCCUGU | 843 | ACAGGAAUGGGCGGUUCAGGGUC | 929 |
| 1369 | CUGAACCGCCCAUUCCUGUUU | 844 | AAACAGGAAUGGGCGGUUCAGGG | 930 |
| 1393 | GUGUAUGAUCAAAGCGCCACU | 845 | AGUGGCGCUUUGAUCAUACACAG | 931 |
| 1401 | UCAAAGCGCCACUGCCCUGCA | 846 | UGCAGGGCAGUGGCGCUUUGAUC | 932 |
| 1404 | AAGCGCCACUGCCCUGCACUU | 847 | AAGUGCAGGGCAGUGGCGCUUUG | 933 |
| 1407 | CGCCACUGCCCUGCACUUCCU | 848 | AGGAAGUGCAGGGCAGUGGCGCU | 934 |
| 1554 | GCGAUGUGUCACCCCCAGUCU | 849 | AGACUGGGGGUGACACAUCGCUG | 935 |
| 1558 | UGUGUCACCCCCAGUCUCCCA | 850 | UGGGAGACUGGGGGUGACACAUC | 936 |
| 1561 | GUCACCCCCAGUCUCCCACCU | 851 | AGGUGGGAGACUGGGGGUGACAC | 937 |
| 1562 | UCACCCCCAGUCUCCCACCUU | 852 | AAGGUGGGAGACUGGGGGUGACA | 938 |
| 1564 | ACCCCCAGUCUCCCACCUUUU | 853 | AAAAGGUGGGAGACUGGGGGUGA | 939 |
| 1591 | AUGAGUCGACUUUGAGCUGGA | 854 | UCCAGCUCAAAGUCGACUCAUUA | 940 |
| 1592 | UGAGUCGACUUUGAGCUGGAA | 855 | UUCCAGCUCAAAGUCGACUCAUU | 941 |
| 1593 | GAGUCGACUUUGAGCUGGAAA | 856 | UUUCCAGCUCAAAGUCGACUCAU | 942 |
| 1596 | UCGACUUUGAGCUGGAAAGCA | 857 | UGCUUUCCAGCUCAAAGUCGACU | 943 |
| 1602 | UUGAGCUGGAAAGCAGCCGUU | 858 | AACGGCUGCUUUCCAGCUCAAAG | 944 |
| 1603 | UGAGCUGGAAAGCAGCCGUUU | 859 | AAACGGCUGCUUUCCAGCUCAAA | 945 |
| 1608 | UGGAAAGCAGCCGUUUCUCCU | 860 | AGGAGAAACGGCUGCUUUCCAGC | 946 |
| 1612 | AAGCAGCCGUUUCUCCUUGGU | 861 | ACCAAGGAGAAACGGCUGCUUUC | 947 |
| 1775 | CAACCGACCAGCUUGUUUGUA | 862 | UACAAACAAGCUGGUCGGUUGGA | 948 |
| 1790 | UUUGUGAAACAAAAAAGUGUU | 863 | AACACUUUUUUGUUUCACAAACA | 949 |
| 1796 | AAACAAAAAAGUGUUCCCUUU | 864 | AAAGGGAACACUUUUUUGUUUCA | 950 |
| 1798s | ACAAAAAAGUGUUCCCUUUU | 865 | AAAAGGGAACACUUUUUUGUUU | 951 |
| 1799 | CAAAAAAGUGUUCCCUUUUCA | 866 | UGAAAAGGGAACACUUUUUUGUU | 952 |
| 1810 | UCCCUUUUCAAGUUGAGAACA | 867 | UGUUCUCAACUUGAAAAGGGAAC | 953 |
| 1811 | CCCUUUUCAAGUUGAGAACAA | 868 | UUGUUCUCAACUUGAAAAGGGAA | 954 |
| 1814 | UUUUCAAGUUGAGAACAAAAA | 869 | UUUUUGUUCUCAACUUGAAAAGG | 955 |
| 1815 | UUUCAAGUUGAGAACAAAAAU | 870 | AUUUUUGUUCUCAACUUGAAAAG | 956 |
| 1817 | UCAAGUUGAGAACAAAAAUUG | 871 | CAAUUUUUGUUCUCAACUUGAAA | 957 |
| 1828 | ACAAAAAUUGGGUUUUAAAAU | 872 | AUUUUAAAACCCAAUUUUUGUUC | 958 |
| 1838s | GGUUUUAAAAUUAAAGUAUA | 873 | UAUACUUUAAUUUUAAAACCCA | 959 |
| 2018 | AUAGCUGGUUAUUUCUCCCUU | 874 | AAGGGAGAAAUAACCAGCUAUGG | 960 |
| 2020 | AGCUGGUUAUUUCUCCCUUGU | 875 | ACAAGGGAGAAAUAACCAGCUAU | 961 |
| 2025 | GUUAUUUCUCCCUUGUGUUAA | 876 | UUAACACAAGGGAGAAAUAACCA | 962 |
| 2030 | UUCUCCCUUGUGUUAGUAAUA | 877 | UAUUACUAACACAAGGGAGAAAU | 963 |
| 2031 | UCUCCCUUGUGUUAGUAAUAA | 878 | UUAUUACUAACACAAGGGAGAAA | 964 |
| 2032 | CUCCCUUGUGUUAGUAAUAAA | 879 | UUUAUUACUAACACAAGGGAGAA | 965 |
| 2033 | UCCCUUGUGUUAGUAAUAAAA | 880 | UUUUAUUACUAACACAAGGGAGA | 966 |
| 729 | AGAACUGGAUGUUGCUGCUGA | 881 | UCAGCAGCAACAUCCAGUUCUGU | 967 |
| 731 | AACUGGAUGUUGCUGCUGAGA | 882 | UCUCAGCAGCAACAUCCAGUUCU | 968 |
| 732 | ACUGGAUGUUGCUGCUGAGAA | 883 | UUCUCAGCAGCAACAUCCAGUUC | 969 |
| 734 | UGGAUGUUGCUGCUGAGAAGA | 884 | UCUUCUCAGCAGCAACAUCCAGU | 970 |
| 734s | UGGAUGUUGCUGCUGAGAAA | 885 | UUUCUCAGCAGCAACAUCCAGU | 971 |
| 736 | GAUGUUGCUGCUGAGAAGAUU | 886 | AAUCUUCUCAGCAGCAACAUCCA | 972 |
| 743 | CUGCUGAGAAGAUUGACAGGU | 887 | ACCUGUCAAUCUUCUCAGCAGCA | 973 |
| 753s | GAUUGACAGGUUCAUGCAGA | 888 | UCUGCAUGAACCUGUCAAUCUU | 974 |
| 963 | GCACUGGAGUGACAUCCAGGA | 889 | UCCUGGAUGUCACUCCAGUGCUG | 975 |
| 966 | CUGGAGUGACAUCCAGGACAA | 890 | UUGUCCUGGAUGUCACUCCAGUG | 976 |
| 969 | GAGUGACAUCCAGGACAACUU | 891 | AAGUUGUCCUGGAUGUCACUCCA | 977 |
| 971 | GUGACAUCCAGGACAACUUCU | 892 | AGAAGUUGUCCUGGAUGUCACUC | 978 |
| 982 | GACAACUUCUCGGUGACUCAA | 893 | UUGAGUCACCGAGAAGUUGUCCU | 979 |
| 984 | CAACUUCUCGGUGACUCAAGU | 894 | ACUUGAGUCACCGAGAAGUUGUC | 980 |
| 992 | CGGUGACUCAAGUGCCCUUCA | 895 | UGAAGGGCACUUGAGUCACCGAG | 981 |
| 994 | GUGACUCAAGUGCCCUUCACU | 896 | AGUGAAGGGCACUUGAGUCACCG | 982 |

Cell line: hepatocellular carcinoma cell line, HepG2 cells.

Solvents: sterile nuclease-free water, Gibco Opti-MEM.

### 3. Experimental methods

Refer to Example 18 for the experimental procedures and siRNA concentrations.

### 4. Experimental results

The inhibition rates of AGT mRNA expression by these unmodified sequences are shown in Tables 59 and 60.

The unmodified sequences 1576s, 1578s, 1838, 1835, 1816, 1812, 1579, 1836, 1789, 1839, 1791, 1795, and 1585s exhibited significant inhibitory effects on AGT mRNA expression in HepG2 cells, with inhibition rates exceeding 45%. Specifically, 1576s, 1578s, 1838, 1835, 1812, 1579, and 1789 exhibited inhibition rates exceeding 50%. A comparison of the inhibitory effects between the unmodified sequences disclosed herein and the sequences disclosed in the prior art is shown in Table 80.
**(1) A total of 13 unmodified sequences exhibited significant inhibitory effects on AGT gene expression, with inhibition rates exceeding 45%, including 1576s, 1578s, 1838, 1835, 1816, 1812, 1579, 1836, 1789, 1839, 1791, 1795, and 1585s.** Specifically, 1576s, 1578s, 1838, 1835, 1812, 1579, and 1789 exhibited inhibition rates exceeding 50%.

**Table 59: Unmodified sequences with significant inhibitory effects on AGT gene expression (inhibition rate > 45%)**

| **Sequence ID** | **Inhibition rate (%)** | **Sequence ID** | **Inhibition rate (%)** |
|---|---|---|---|
| 1576s | 59.1 | 1836 | 46.5 |
| 1578s | 55.2 | 1789 | 54.5 |
| 1838 | 50.6 | 1839 | 45.7 |
| 1835 | 51.6 | 1791 | 46.5 |
| 1816 | 46.8 | 1795 | 49.6 |
| 1812 | 56.8 | 1585s | 48.9 |
| 1579 | 58.2 | | |

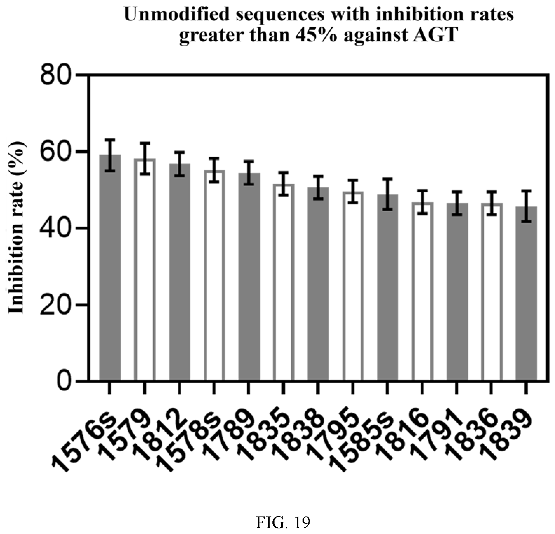

The unmodified sequences listed in Table 59, including 1576s, 1578s, 1838, 1835, 1816, 1812, 1579, 1836, 1789, 1839, 1791, 1795, and 1585s, exhibited significant inhibitory effects on AGT mRNA expression, with inhibition rates exceeding 45%. Specifically, 1576s, 1578s, 1838, 1835, 1812, 1579, and 1789 exhibited inhibition rates exceeding 50% (FIG. 19).

**(2) Some of these unmodified sequences exhibited inhibition rates below 45% on AGT gene expression.** For example, the inhibition rates of 731 and 1011 were only 1.3% and 2.1%, respectively.

**Table 60: Unmodified sequences with inhibition rates below 45% on AGT gene expression**

| **Sequence ID** | **Inhibition rate (%)** | **Sequence ID** | **Inhibition rate (%)** |
|---|---|---|---|
| 1608 | 39.3 | 1393 | 38.2 |
| 1811 | 37.7 | 2030 | 36.3 |
| 1281 | 26.2 | 1267 | 36.8 |
| 1799 | 34.9 | 2033 | 34.9 |
| 1603 | 26.7 | 2031 | 36.4 |
| 1828 | 31.5 | 1815 | 37.1 |
| 1592 | 38.6 | 2020 | 32.5 |
| 1593 | 41.6 | 753s | 27.8 |
| 1810 | 24.5 | 2032 | 17.6 |
| 1365 | 24.3 | 1602 | 22.3 |
| 1279 | 41.5 | 1029 | 18.5 |
| 734 | 43.0 | 1612 | 9.6 |
| 1591 | 37.4 | 2018 | 14.1 |
| 969 | 27.3 | 2025 | 7.2 |
| 994 | 36.6 | 731 | 1.3 |
| 984 | 31.6 | 1011 | 2.1 |
| 743 | 29.3 | | |

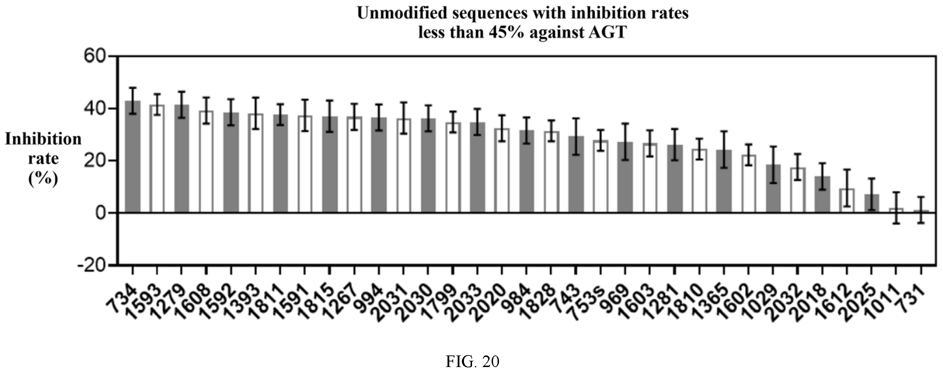

The sequences listed in Table 60 exhibited very weak inhibitory effects on AGT mRNA expression, with inhibition rates below 45%. For example, the inhibition rates of 731 and 1011 were only 1.3% and 2.1%, respectively (FIG. 20).

**(3) siRNAs with similar unmodified sequences exhibited significant differences in activity. For example, the inhibition rate of unmodified sequence 1812 was 32.3% higher than that of unmodified sequence 1810, indicating a significant improvement.**

A comparison of the inhibitory effects of certain siRNAs with similar sequences on AGT mRNA expression is shown in Table 61.

**Table 61: Comparison of inhibition rates of unmodified sequences with similar sequences on AGT gene expression**

| **Group of similar sequences** | **Basic SEQ ID NO:** | **Sense strand 5'-3'** | **Unmodified SEQ ID NO:** | **Antisense strand 5'-3'** | **Unmodified SEQ ID NO:** | **Inh (%)** |
|---|---|---|---|---|---|---|
| A | 1608 | UGGAAAGCAGCC GUUUCUCCU | 860 | AGGAGAAACGGCUG CUUUCCAGC | 946 | 39.3 |
| | 1612 | AAGCAGCCGUUU CUCCUUGGU | 861 | ACCAAGGAGAAACG GCUGCUUUC | 947 | 9.6 |
| B | 2025 | GUUAUUUCUCCC UUGUGUUAA | 876 | UUAACACAAGGGAG AAAUAACCA | 962 | 7.2 |
| | 2030 | UUCUCCCUUGUG UUAGUAAUA | 877 | UAUUACUAACACAA GGGAGAAAU | 963 | 36.3 |
| C | 731 | AACUGGAUGUUG CUGCUGAGA | 882 | UCUCAGCAGCAACA UCCAGUUCU | 968 | 1.3 |
| | 734 | UGGAUGUUGCUG CUGAGAAGA | 884 | UCUUCUCAGCAGCA ACAUCCAGU | 970 | 43.0 |
| D | 1812 | CCUUUUCAAGUU GAGAACAAA | 787 | UUUGUUCUCAACUU GAAAAGGA | 800 | 56.8 |
| | 1810 | UCCCUUUUCAAG UUGAGAACA | 867 | UGUUCUCAACUUGA AAAGGAAC | 953 | 24.5 |

As shown in Table 61, certain siRNAs with similar sequences exhibited vastly different inhibitory effects on AGT mRNA expression.

Compared with unmodified sequence 1612, unmodified sequence 1608 differs only in four bases at the terminal positions. For the sense strand, the 5' end of unmodified sequence 76 is UGGA, while the 3' end of unmodified sequence 77 is UGGU, with all other bases being identical. For the antisense strand, the 3' end of unmodified sequence 162 is CAGC, while the 5' end of unmodified sequence 163 is ACCA, with all other bases being identical. However, the inhibition rate of unmodified sequence 1608 was 29.7% higher than that of 1612, indicating a significant improvement.

Compared with unmodified sequence 2030, unmodified sequence 2025 differs only in six bases at the terminal positions. For the sense strand, the 5' end of unmodified sequence 92 is GUUAU and the 3' end is A, while the 3' end of sequence 93 is GUAAUA, with all other bases being identical. For the antisense strand, the 3' end of unmodified sequence 178 is AACCA and the 5' end is U, while the 5' end of unmodified sequence 179 is UAUUAC, with all other bases being identical. However, the inhibition rate of unmodified sequence 2030 was 29.1% higher than that of 2025, indicating a significant improvement.

Compared with unmodified sequence 734, unmodified sequence 731 differs only in three bases at the terminal positions. For the sense strand, the 5' end of unmodified sequence 98 is AAC, while the 3' end of unmodified sequence 100 is AGA, with all other bases being identical. For the antisense strand, the 3' end of unmodified sequence 184 is UCU, while the 5' end of unmodified sequence 186 is UCU, with all other bases being identical. However, the inhibition rate of unmodified sequence 734 was 41.7% higher than that of 731, indicating a significant improvement.

Compared with unmodified sequence 1810, unmodified sequence 1812 differs only in two bases at the terminal positions. For the sense strand, the 3' end of unmodified sequence 3 is AA, while the 5' end of unmodified sequence 83 is UC, with all other bases being identical. For the antisense strand, the 5' end of unmodified sequence 16 is UU, while the 3' end of unmodified sequence 169 is AC, with all other bases being identical. The inhibition rate of unmodified sequence 1812 was 32.3% higher than that of unmodified sequence 1810, indicating a significant improvement.

Therefore, identifying unmodified oligonucleotide sequences with significant inhibitory activity from a vast number of candidate sequences designed against AGT mRNA sequences is not straightforward and requires substantial creative effort.

**(4) The effects of alternating modifications on activity enhancement varied across different sequences. For some sequences, the inhibition rate was significantly improved. For example, for unmodified sequence 731, the alternately modified sequence exhibited a 25.1% increase in inhibition rate compared to the unmodified sequence. For others, the improvement was minimal. For example, unmodified sequences 994, 1279, and 1591 exhibited little to no difference in inhibition rate between alternately modified and unmodified sequences.**

**Table 62: Comparison of inhibition rates of AGT gene expression between alternately modified and unmodified sequences**

| **Sequence ID** | **Inhibition rate of unmodified sequences (%)** | **Alternately modified sequence ID** | **Inhibition rate of alternately modified sequences (%)** | **Increase (%)** |
|---|---|---|---|---|
| 731 | 1.3 | B731-AL | 26.4 | 25.1 |
| 734 | 43.0 | B734-AL | 39.2 | -3.8 |
| 743 | 29.3 | B743-AL | 33.8 | 4.5 |
| 753s | 27.8 | B753s-AL | 30.2 | 2.4 |
| 969 | 27.3 | B969-AL | 37.3 | 10.0 |
| 984 | 31.6 | B984-AL | 36.9 | 5.3 |
| 994 | 36.6 | B994-AL | 37.3 | 0.7 |
| 1011 | 2.1 | B1011-AL | 26.2 | 24.1 |
| 1029 | 18.5 | B1029-AL | 28.6 | 10.1 |
| 1267 | 36.8 | B1267-AL | 31.5 | -5.3 |
| 1279 | 41.5 | B1279-AL | 41.4 | -0.1 |
| 1281 | 26.2 | B1281-AL | 50.4 | 24.2 |
| 1365 | 24.3 | B1365-AL | 42.0 | 17.7 |
| 1393 | 38.2 | B1393-AL | 33.4 | -4.8 |
| 1576s | 59.1 | B1576s-AL | 64.6 | 5.5 |
| 1578s | 55.2 | B1578s-AL | 63.9 | 8.7 |
| 1579 | 58.2 | B1579-AL | 61.1 | 2.9 |
| 1585s | 48.9 | B1585s-AL | 59.3 | 10.4 |
| 1591 | 37.4 | B1591-AL | 37.9 | 0.5 |
| 1592 | 38.6 | B1592-AL | 44.4 | 5.8 |
| 1593 | 41.6 | B1593-AL | 42.4 | 0.8 |
| 1602 | 22.3 | B1602-AL | 29.0 | 6.7 |
| 1603 | 26.7 | B1603-AL | 48.6 | 21.9 |
| 1608 | 39.3 | B1608-AL | 52.5 | 13.2 |
| 1612 | 9.6 | B1612-AL | 28.6 | 19.0 |
| 1789 | 54.5 | B1789-AL | 63.5 | 9.0 |
| 1791 | 46.5 | B1791-AL | 61.8 | 15.3 |
| 1795 | 49.6 | B1795-AL | 57.5 | 7.9 |
| 1799 | 34.9 | B1799-AL | 49.2 | 14.3 |
| 1810 | 24.5 | B1810-AL | 42.1 | 17.6 |
| 1811 | 37.7 | B1811-AL | 51.5 | 13.8 |
| 1812 | 56.8 | B1812-AL | 62.9 | 6.1 |
| 1815 | 37.1 | B1815-AL | 30.4 | -6.7 |
| 1816 | 46.8 | B1816-AL | 60.9 | 14.1 |
| 1828 | 31.5 | B1828-AL | 46.6 | 15.1 |
| 1835 | 51.6 | B1835-AL | 62.3 | 10.7 |
| 1836 | 46.5 | B1836-AL | 59.8 | 13.3 |
| 1838 | 50.6 | B1838-AL | 63.6 | 13.0 |
| 1839 | 45.7 | B1839-AL | 59.1 | 13.4 |
| 2018 | 14.1 | B2018-AL | 27.9 | 13.8 |
| 2020 | 32.5 | B2020-AL | 30.4 | -2.1 |
| 2025 | 7.2 | B2025-AL | 26.6 | 19.4 |
| 2030 | 36.3 | B2030-AL | 33.0 | -3.3 |
| 2031 | 36.4 | B2031-AL | 31.0 | -5.4 |
| 2032 | 17.6 | B2032-AL | 30.2 | 12.6 |
| 2033 | 34.9 | B2033-AL | 31.5 | -3.4 |

As shown in Table 62, the effects of alternating modifications on the inhibition rate of AGT mRNA expression varied across different sequences. For some sequences, the inhibition rate was significantly improved. For example, for unmodified sequence 731, the alternately modified sequence exhibited a 25.1% increase in inhibition rate compared to the unmodified sequence. For others, the improvement was minimal. For example, unmodified sequences 994, 1279, and 1591 exhibited little to no difference in inhibition rate between alternately modified and unmodified sequences.

Therefore, not all unmodified sequences exhibited significantly improved activity after alternating modifications, and the effects of alternating modifications on activity varied across different sequences.

### Summary:

(1) A total of 13 unmodified sequences exhibited significant inhibitory effects on AGT gene expression, with inhibition rates exceeding 45%, including 1576s, 1579, 1812, 1578s, 1789, 1835, 1838, 1795, 1585s, 1816, 1791, 1836, and 1839. Specifically, 1576s, 1578s, 1812, 1579, 1789, 1835, and 1838 exhibited inhibition rates exceeding 50%.
(2) The other unmodified sequences exhibited inhibition rates below 45% on AGT gene expression. For example, the inhibition rates of 731 and 1011 were only 1.3% and 2.1%, respectively.
(3) siRNAs with similar sequences exhibited significant differences in activity. For example, the inhibition rate of unmodified sequence 734 was 41.7% higher than that of unmodified sequence 731, indicating a significant improvement.
(4) The effects of alternating modifications on activity varied across different sequences. For some sequences, the inhibition rate was significantly improved. For example, for unmodified sequence 731, the alternately modified sequence exhibited a 25.1% increase in inhibition rate compared to the unmodified sequence. For others, the improvement was minimal. For example, unmodified sequences 994, 1279, and 1591 exhibited little to no difference in inhibition rate between alternately modified and unmodified sequences.

### Example 20: Inhibitory effect of template-modified sequences on AGT gene expression

In this example, a total of 13 sequences identified in Example 19, namely unmodified sequences 1576s, 1578s, 1838, 1835, 1816, 1812, 1579, 1836, 1789, 1839, 1791, 1795, and 1585s, were modified using different modification templates. Specifically, DV25P, DV26P, DV27P, DV28P, DV29P, DV32P, DV33P, DV34P, DV25SP, DV26SP, DV27SP, DV28SP, DV29SP, DV32SP, DV33SP, and DV34SP are novel modification templates designed in the present disclosure, while DV22 is a previously disclosed Advanced ESC modification template (Foster, D. J., et al. (2018). "Advanced siRNA Designs Further Improve In Vivo Performance of GalNAc-siRNA Conjugates." Mol Ther 26(3): 708-717).

Natural 5'-phosphorylation or directly introduced 5'-phosphorylation may undergo dephosphorylation in cells. Directly 5'-phosphorylated oligonucleotide strands may undergo 90% dephosphorylation after 2 hours of circulation in blood and be completely dephosphorylated after 24 hours. The 5'-phosphorylation design (5'-E-VP), in which E-vinylphosphonate replaces the bridging oxygen, provides improved phosphorylation efficiency and enhanced stability. In the modification templates DV25-29P and DV32-34P disclosed herein, the antisense strand incorporates the 5'-E-VP phosphorylation design.

### 1. Experimental materials

### Test samples:

The sequences listed in Table 63, including sequences 1576s, 1578s, 1838, 1835, 1816, 1812, 1579, 1836, 1789, 1839, 1791, 1795, and 1585s from Example 19 modified using different templates (DV25P, DV26P, DV27P, DV28P, DV29P, DV30P, DV32P, DV33P, DV34P, DV22, DV25SP, DV26SP, DV27SP, DV28SP, DV29SP, DV30SP, DV32SP, DV33SP, DV34SP, and DV22S), as well as their corresponding alternately modified sequences B1576s-AL, B1578s-AL, B1838-AL, B1835-AL, B1816-AL, B1812-AL, B1579-AL, B1836-AL, B1789-AL, B1839-AL, B1791-AL, B1795-AL, and B1585s-AL (synthesized in Example 17).

### Synthesis of sequences:

The siRNA sequences were synthesized using the method described in Example 17, except that the base at the 5' end of the antisense strand (the last base) was synthesized using a monomer containing a phosphonate group at the 5' end, such as vinyl-(E)-phosphonate-A-OMe phosphoramidite monomer (Formula 13) or vinyl-(E)-phosphonate-U-OMe phosphoramidite monomer (Formula 14), the structures of which are as follows:

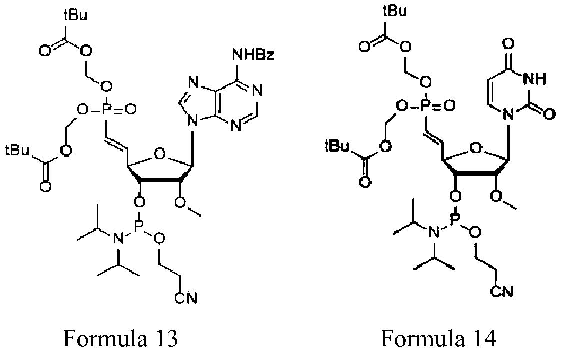

The modification principles for the templates disclosed herein are as follows:

### For DV22, DV25P to D29P, and D32P to 34P:

The antisense strand adopts one of the modification patterns shown in Table 63-1:

**Table 63-1: Modification of antisense strand**

| No. | **Antisense strand (AS) 5'-3'** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | 2'-F | 2'-OMe | 2'-F | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | 2'-F | 2'-F | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **G** | **1** | **2** | **3** | **4** | **5** | **6** | **7** | **8** | **9** | **10** | **11** | **12** |
| | 2'-OMe +PS | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |

The sense strand adopts one of the modification patterns shown in Table 63-2:

**Table 63-2: Modification of sense strand**

| No. | **Sense strand (SS) 5'-3'** | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-F | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **c** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-OMe | 2'-OMe | 2'-OMe | 2'-OMe | 2'-OMe | |

In the above tables, PS represents a phosphorothioate backbone.

The siRNA modification template, in which the antisense strand is modified according to modification pattern A and the sense strand is modified according to modification pattern a, is designated as DV25P.

The siRNA modification template, in which the antisense strand is modified according to modification pattern B and the sense strand is modified according to modification pattern a, is designated as DV26P.

The siRNA modification template, in which the antisense strand is modified according to modification pattern C and the sense strand is modified according to modification pattern a, is designated as DV27P.

The siRNA modification template, in which the antisense strand is modified according to modification pattern B and the sense strand is modified according to modification pattern b, is designated as DV28P.

The siRNA modification template, in which the antisense strand is modified according to modification pattern C and the sense strand is modified according to modification pattern b, is designated as DV29P.

The siRNA modification template, in which the antisense strand is modified according to modification pattern D and the sense strand is modified according to modification pattern b, is designated as DV32P.

The siRNA modification template, in which the antisense strand is modified according to modification pattern E and the sense strand is modified according to modification pattern b, is designated as DV33P.

The siRNA modification template, in which the antisense strand is modified according to modification pattern F and the sense strand is modified according to modification pattern b, is designated as DV34P.

The siRNA modification template, in which the antisense strand is modified according to modification pattern G and the sense strand is modified according to modification pattern c, is designated as DV22.

### For DV22S, DV25P to DV29SP, and DV32P to DV34SP:

For compounds in which the sense and antisense strands are 20 and 22 nucleotides in length, respectively, the antisense strand adopts one of the modification patterns shown in Table 63-3:

**Table 63-3: Modification of antisense strand**

| No. | **Antisense strand (AS) 5'-3'** | | | | | | | | | | |
|---|---|---|---|---|---|---|---|---|---|---|---|
| **A'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **B'** | **1** | **2** | **3** | **4** | **5** | **6** | **7** | **8** | **9** | **10** | **11** |
| | 2'-OMe +PS +EVP | **2'-F +PS** | 2'-F | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | 2'-F | 2'-OMe | **2'-F** | 2'-OMe | 2'-OMe | 2'-OMe | 2'-OMe +PS | 2'-OMe +PS | 2'-OMe |
| **C'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **E'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe+PS | 2'-OMe+PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

The sense strand adopts one of the modification patterns shown in Table 63-4:

**Table 63-4: Modification of sense strand**

| No. | **Sense strand (SS) 5'-3'** | | | | | | | | | |
|---|---|---|---|---|---|---|---|---|---|---|
| **a'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **c'** | **1** | **2** | **3** | **4** | **5** | **6** | **7** | **8** | **9** | **10** |
| | 2'-OMe +PS | 2'-OMe +PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-OMe | 2'-OMe | 2'-OMe | 2'-OMe | 2'-OMe |

In the above tables, 2'-OMe represents a 2'-O-methyl modification; 2'-F represents a 2'-fluoro modification; PS represents a phosphorothioate backbone.

The siRNA modification template, in which the antisense strand is modified according to modification pattern A' and the sense strand is modified according to modification pattern a', is designated as DV25SP.

The siRNA modification template, in which the antisense strand is modified according to modification pattern B' and the sense strand is modified according to modification pattern a', is designated as DV26SP.

The siRNA modification template, in which the antisense strand is modified according to modification pattern C' and the sense strand is modified according to modification pattern a', is designated as DV27SP.

The siRNA modification template, in which the antisense strand is modified according to modification pattern B' and the sense strand is modified according to modification pattern b', is designated as DV28SP.

The siRNA modification template, in which the antisense strand is modified according to modification pattern C' and the sense strand is modified according to modification pattern b', is designated as DV29SP.

The siRNA modification template, in which the antisense strand is modified according to modification pattern D' and the sense strand is modified according to modification pattern b', is designated as DV32SP.

The siRNA modification template, in which the antisense strand is modified according to modification pattern E' and the sense strand is modified according to modification pattern b', is designated as DV33SP.

The siRNA modification template, in which the antisense strand is modified according to modification pattern F' and the sense strand is modified according to modification pattern b', is designated as DV34SP.

The siRNA modification template, in which the antisense strand is modified according to modification pattern G' and the sense strand is modified according to modification pattern c', is designated as DV22S.

The template-modified sequences are detailed in Table 63-5. These sequences were synthesized using the same method as described in Example 17.

**Table 63-5: Sequences modified using different templates**

| Unmodified seq ID | Template -modified seq ID | Sense sequence 5'-3' | Modified SEQ ID NO:/corr. unmodified SEQ ID NO: | Antisense sequence 5'-3' | Modified SEQ ID NO:/corr. unmodified SEQ ID NO: |
|---|---|---|---|---|---|
| 1835 | C1835-DV29P | Ums-Ums-Gm-Gm-Gm-Um-Uf- Um-Uf-Am-Af-Am-Am-Um- Um-Am-Af-Am-Gm-Um-Am | 1181/785 | UmsEVP-Afs-Cm-Uf-Uf-Uf- Am-Am-Um-Um-Um-Um- Am-Af-Am-Af-Cm-Cm-Cm- Am-Ams-Ums-Um | 1216/1582 |
| | C1835-DV26P | Ums-Ums-Gm-Gm-Gm-Um-Uf- Um-Uf-Af-Af-Am-Am-Um- Um-Am-Af-Am-Gm-Um-Am | 1182/785 | UmsEVP-Afs-Cf-Uf-Um-Uf- Am-Am-Um-Um-Um-Um- Am-Af-Am-Af-Cm-Cm-Cm- Am-Ams-Ums-Um | 1217/1582 |
| | C1835-DV27P | Ums-Ums-Gm-Gm-Gm-Um-Uf- Um-Uf-Af-Af-Am-Am-Um- Um-Am-Af-Am-Gm-Um-Am | 1182/785 | UmsEVP-Afs-Cm-Uf-Uf-Uf- Am-Am-Um-Um-Um-Um- Am-Af-Am-Af-Cm-Cm-Cm- Am-Ams-Ums-Um | 1216/1582 |
| | C1835-DV28P | Ums-Ums-Gm-Gm-Gm-Um-Uf- Um-Uf-Am-Af-Am-Am-Um- Um-Am-Af-Am-Gm-Um-Am | 1181/785 | UmsEVP-Afs-Cf-Uf-Um-Uf- Am-Am-Um-Um-Um-Um- Am-Af-Am-Af-Cm-Cm-Cm- Am-Ams-Ums-Um | 1217/1582 |
| | C1835-DV32P | Ums-Ums-Gm-Gm-Gm-Um-Uf- Um-Uf-Am-Af-Am-Am-Um- Um-Am-Af-Am-Gm-Um-Am | 1181/785 | UmsEVP-Afs-Cm-Um-Uf-Uf- Am-Am-Um-Um-Um-Um- Am-Af-Am-Af-Cm-Cm-Cm- Am-Ams-Ums-Um | 1218/1582 |
| | C1835-DV34P | Ums-Ums-Gm-Gm-Gm-Um-Uf- Um-Uf-Am-Af-Am-Am-Um- Um-Am-Af-Am-Gm-Um-Am | 1181/785 | UmsEVP-Afs-Cf-Um-Um-Uf- Am-Am-Um-Um-Um-Um- Am-Af-Am-Af-Cm-Cm-Cm- Am-Ams-Ums-Um | 1219/1582 |
| | C1835-DV25P | Ums-Ums-Gm-Gm-Gm-Um-Uf- Um-Uf-Af-Af-Am-Am-Um- Um-Am-Af-Am-Gm-Um-Am | 1182/785 | UmsEVP-Afs-Cm-Um-Um- Uf-Am-Am-Um-Um-Um-Um- Am-Af-Am-Af-Cm-Cm-Cm- Am-Ams-Ums-Um | 1220/1582 |
| | C1835-DV33P | Ums-Ums-Gm-Gm-Gm-Um-Uf- Um-Uf-Am-Af-Am-Am-Um- Um-Am-Af-Am-Gm-Um-Am | 1181/785 | UmsEVP-Afs-Cf-Um-Uf-Uf- Am-Am-Um-Um-Um-Um- Am-Af-Am-Af-Cm-Cm-Cm- Am-Ams-Ums-Um | 1221/1582 |
| | C1835-DV22 | Ums-Ums-Gm-Gm-Gm-Um-Uf- Um-Uf-Af-Af-Am-Am-Um- Um-Am-Am-Am-Gm-Um-Am | 1183/785 | Ums-Afs-Cm-Um-Um-Uf- Am-Am-Um-Um-Um-Um- Am-Af-Am-Af-Cm-Cm-Cm- Am-Ams-Ums-Um | 1222/1582 |
| 1816 | C1816-DV29P | Ums-Ums-Cm-Am-Am-Gm-Uf- Um-Gf-Am-Gf-Am-Am-Cm- Am-Am-Af-Am-Am-Um-Um | 1184/786 | AmsEVP-Afs-Um-Uf-Uf-Uf- Um-Gm-Um-Um-Cm-Um- Cm-Af-Am-Cf-Um-Um-Gm- Am-Ams-Ams-Am | 1223/1583 |
| | C1816-DV26P | Ums-Ums-Cm-Am-Am-Gm-Uf- Um-Gf-Af-Gf-Am-Am-Cm- Am-Am-Af-Am-Am-Um-Um | 1185/786 | AmsEVP-Afs-Uf-Uf-Um-Uf- Um-Gm-Um-Um-Cm-Um- Cm-Af-Am-Cf-Um-Um-Gm- Am-Ams-Ams-Am | 1224/1583 |
| | C1816-DV27P | Ums-Ums-Cm-Am-Am-Gm-Uf- Um-Gf-Af-Gf-Am-Am-Cm- Am-Am-Af-Am-Am-Um-Um | 1185/786 | AmsEVP-Afs-Um-Uf-Uf-Uf- Um-Gm-Um-Um-Cm-Um- Cm-Af-Am-Cf-Um-Um-Gm- Am-Ams-Ams-Am | 1223/1583 |
| | C1816-DV28P | Ums-Ums-Cm-Am-Am-Gm-Uf- Um-Gf-Am-Gf-Am-Am-Cm- Am-Am-Af-Am-Am-Um-Um | 1184/786 | AmsEVP-Afs-Uf-Uf-Um-Uf- Um-Gm-Um-Um-Cm-Um- Cm-Af-Am-Cf-Um-Um-Gm- Am-Ams-Ams-Am | 1224/1583 |
| | C1816-DV32P | Ums-Ums-Cm-Am-Am-Gm-Uf- Um-Gf-Am-Gf-Am-Am-Cm- Am-Am-Af-Am-Am-Um-Um | 1184/786 | AmsEVP-Afs-Um-Um-Uf-Uf- Um-Gm-Um-Um-Cm-Um- Cm-Af-Am-Cf-Um-Um-Gm- Am-Ams-Ams-Am | 1225/1583 |
| | C1816-DV34P | Ums-Ums-Cm-Am-Am-Gm-Uf- Um-Gf-Am-Gf-Am-Am-Cm- Am-Am-Af-Am-Am-Um-Um | 1184/786 | AmsEVP-Afs-Uf-Um-Um-Uf- Um-Gm-Um-Um-Cm-Um- Cm-Af-Am-Cf-Um-Um-Gm- Am-Ams-Ams-Am | 1226/1583 |
| | C1816-DV25P | Ums-Ums-Cm-Am-Am-Gm-Uf- Um-Gf-Af-Gf-Am-Am-Cm- Am-Am-Af-Am-Am-Um-Um | 1185/786 | AmsEVP-Afs-Um-Um-Um- Uf-Um-Gm-Um-Um-Cm-Um- Cm-Af-Am-Cf-Um-Um-Gm- Am-Ams-Ams-Am | 1227/1583 |
| | C1816-DV33P | Ums-Ums-Cm-Am-Am-Gm-Uf- Um-Gf-Am-Gf-Am-Am-Cm- Am-Am-Af-Am-Am-Um-Um | 1184/786 | AmsEVP-Afs-Uf-Um-Uf-Uf- Um-Gm-Um-Um-Cm-Um- Cm-Af-Am-Cf-Um-Um-Gm- Am-Ams-Ams-Am | 1228/1583 |
| | C1816-DV22 | Ums-Ums-Cm-Am-Am-Gm-Uf- Um-Gf-Af-Gf-Am-Am-Cm- Am-Am-Am-Am-Am-Um-Um | 1186/786 | Ams-Afs-Um-Um-Um-Uf- Um-Gm-Um-Um-Cm-Um- Cm-Af-Am-Cf-Um-Um-Gm- Am-Ams-Ams-Am | 1229/1583 |
| 1812 | C1812-DV29P | Cms-Cms-Um-Um-Um-Um-Cf- Am-Af-Gm-Uf-Um-Gm-Am- Gm-Am-Af-Cm-Am-Am-Am | 1187/787 | UmsEVP-Ufs-Um-Gf-Uf-Uf- Cm-Um-Cm-Am-Am-Cm- Um-Uf-Gm-Af-Am-Am-Am- Gm-Gms-Gms-Am | 1230/1584 |
| | C1812-DV26P | Cms-Cms-Um-Um-Um-Um-Cf- Am-Af-Gf-Uf-Um-Gm-Am- Gm-Am-Af-Cm-Am-Am-Am | 1188/787 | UmsEVP-Ufs-Uf-Gf-Um-Uf- Cm-Um-Cm-Am-Am-Cm- Um-Uf-Gm-Af-Am-Am-Am- Gm-Gms-Gms-Am | 1231/1584 |
| | C1812-DV27P | Cms-Cms-Um-Um-Um-Um-Cf- Am-Af-Gf-Uf-Um-Gm-Am- Gm-Am-Af-Cm-Am-Am-Am | 1188/787 | UmsEVP-Ufs-Um-Gf-Uf-Uf- Cm-Um-Cm-Am-Am-Cm- Um-Uf-Gm-Af-Am-Am-Am- Gm-Gms-Gms-Am | 1230/1584 |
| | C1812-DV28P | Cms-Cms-Um-Um-Um-Um-Cf- Am-Af-Gm-Uf-Um-Gm-Am- Gm-Am-Af-Cm-Am-Am-Am | 1187/787 | UmsEVP-Ufs-Uf-Gf-Um-Uf- Cm-Um-Cm-Am-Am-Cm- Um-Uf-Gm-Af-Am-Am-Am- Gm-Gms-Gms-Am | 1231/1584 |
| | C1812-DV32P | Cms-Cms-Um-Um-Um-Um-Cf- Am-Af-Gm-Uf-Um-Gm-Am- Gm-Am-Af-Cm-Am-Am-Am | 1187/787 | UmsEVP-Ufs-Um-Gm-Uf-Uf- Cm-Um-Cm-Am-Am-Cm- Um-Uf-Gm-Af-Am-Am-Am- Gm-Gms-Gms-Am | 1232/1584 |
| | C1812-DV34P | Cms-Cms-Um-Um-Um-Um-Cf- Am-Af-Gm-Uf-Um-Gm-Am- Gm-Am-Af-Cm-Am-Am-Am | 1187/787 | UmsEVP-Ufs-Uf-Gm-Um-Uf- Cm-Um-Cm-Am-Am-Cm- Um-Uf-Gm-Af-Am-Am-Am- Gm-Gms-Gms-Am | 1233/1584 |
| | C1812-DV25P | Cms-Cms-Um-Um-Um-Um-Cf- Am-Af-Gf-Uf-Um-Gm-Am- Gm-Am-Af-Cm-Am-Am-Am | 1188/787 | UmsEVP-Ufs-Um-Gm-Um- Uf-Cm-Um-Cm-Am-Am-Cm- Um-Uf-Gm-Af-Am-Am-Am- Gm-Gms-Gms-Am | 1234/1584 |
| | C1812-DV33P | Cms-Cms-Um-Um-Um-Um-Cf- Am-Af-Gm-Uf-Um-Gm-Am- Gm-Am-Af-Cm-Am-Am-Am | 1187/787 | UmsEVP-Ufs-Uf-Gm-Uf-Uf- Cm-Um-Cm-Am-Am-Cm- Um-Uf-Gm-Af-Am-Am-Am- Gm-Gms-Gms-Am | 1235/1584 |
| | C1812-DV22 | Cms-Cms-Um-Um-Um-Um-Cf- Am-Af-Gf-Uf-Um-Gm-Am- Gm-Am-Am-Cm-Am-Am-Am | 1189/787 | Ums-Ufs-Um-Gm-Um-Uf- Cm-Um-Cm-Am-Am-Cm- Um-Uf-Gm-Af-Am-Am-Am- Gm-Gms-Gms-Am | 1236/1584 |
| 1579 | C1579-DV29P | Cms-Cms-Um-Um-Um-Um-Cf- Um-Uf-Cm-Uf-Am-Am-Um- Gm-Am-Gf-Um-Cm-Gm-Am | 1190/788 | UmsEVP-Cfs-Gm-Af-Cf-Uf- Cm-Am-Um-Um-Am-Gm- Am-Af-Gm-Af-Am-Am-Am- Gm-Gms-Ums-Gm | 1237/1585 |
| | C1579-DV26P | Cms-Cms-Um-Um-Um-Um-Cf- Um-Uf-Cf-Uf-Am-Am-Um- Gm-Am-Gf-Um-Cm-Gm-Am | 1191/788 | UmsEVP-Cfs-Gf-Af-Cm-Uf- Cm-Am-Um-Um-Am-Gm- Am-Af-Gm-Af-Am-Am-Am- Gm-Gms-Ums-Gm | 1238/1585 |
| | C1579-DV27P | Cms-Cms-Um-Um-Um-Um-Cf- Um-Uf-Cf-Uf-Am-Am-Um- Gm-Am-Gf-Um-Cm-Gm-Am | 1191/788 | UmsEVP-Cfs-Gm-Af-Cf-Uf- Cm-Am-Um-Um-Am-Gm- Am-Af-Gm-Af-Am-Am-Am- Gm-Gms-Ums-Gm | 1237/1585 |
| | C1579-DV28P | Cms-Cms-Um-Um-Um-Um-Cf- Um-Uf-Cm-Uf-Am-Am-Um- Gm-Am-Gf-Um-Cm-Gm-Am | 1190/788 | UmsEVP-Cfs-Gf-Af-Cm-Uf- Cm-Am-Um-Um-Am-Gm- Am-Af-Gm-Af-Am-Am-Am- Gm-Gms-Ums-Gm | 1238/1585 |
| | C1579-DV32P | Cms-Cms-Um-Um-Um-Um-Cf- Um-Uf-Cm-Uf-Am-Am-Um- Gm-Am-Gf-Um-Cm-Gm-Am | 1190/788 | UmsEVP-Cfs-Gm-Am-Cf-Uf- Cm-Am-Um-Um-Am-Gm- Am-Af-Gm-Af-Am-Am-Am- Gm-Gms-Ums-Gm | 1239/1585 |
| | C1579-DV34P | Cms-Cms-Um-Um-Um-Um-Cf- Um-Uf-Cm-Uf-Am-Am-Um- Gm-Am-Gf-Um-Cm-Gm-Am | 1190/788 | UmsEVP-Cfs-Gf-Am-Cm-Uf- Cm-Am-Um-Um-Am-Gm- Am-Af-Gm-Af-Am-Am-Am- Gm-Gms-Ums-Gm | 1240/1585 |
| | C1579-DV25P | Cms-Cms-Um-Um-Um-Um-Cf- Um-Uf-Cf-Uf-Am-Am-Um- Gm-Am-Gf-Um-Cm-Gm-Am | 1191/788 | UmsEVP-Cfs-Gm-Am-Cm- Uf-Cm-Am-Um-Um-Am-Gm- Am-Af-Gm-Af-Am-Am-Am- Gm-Gms-Ums-Gm | 1241/1585 |
| | C1579-DV33P | Cms-Cms-Um-Um-Um-Um-Cf- Um-Uf-Cm-Uf-Am-Am-Um- Gm-Am-Gf-Um-Cm-Gm-Am | 1190/788 | UmsEVP-Cfs-Gf-Am-Cf-Uf- Cm-Am-Um-Um-Am-Gm- Am-Af-Gm-Af-Am-Am-Am- Gm-Gms-Ums-Gm | 1242/1585 |
| | C1579-DV22 | Cms-Cms-Um-Um-Um-Um-Cf- Um-Uf-Cf-Uf-Am-Am-Um- Gm-Am-Gm-Um-Cm-Gm-Am | 1192/788 | Ums-Cfs-Gm-Am-Cm-Uf-Cm- Am-Um-Um-Am-Gm-Am-Af- Gm-Af-Am-Am-Am-Gm- Gms-Ums-Gm | 1243/1585 |
| 1836 | C1836-DV29P | Ums-Gms-Gm-Gm-Um-Um-Uf- Um-Af-Am-Af-Am-Um-Um- Am-Am-Af-Gm-Um-Am-Um | 1193/789 | AmsEVP-Ufs-Am-Cf-Uf-Uf- Um-Am-Am-Um-Um-Um- Um-Af-Am-Af-Am-Cm-Cm- Cm-Ams-Ams-Um | 1244/1586 |
| | C1836-DV26P | Ums-Gms-Gm-Gm-Um-Um-Uf- Um-Af-Af-Af-Am-Um-Um- Am-Am-Af-Gm-Um-Am-Um | 1194/789 | AmsEVP-Ufs-Af-Cf-Um-Uf- Um-Am-Am-Um-Um-Um- Um-Af-Am-Af-Am-Cm-Cm- Cm-Ams-Ams-Um | 1245/1586 |
| | C1836-DV27P | Ums-Gms-Gm-Gm-Um-Um-Uf- Um-Af-Af-Af-Am-Um-Um- Am-Am-Af-Gm-Um-Am-Um | 1194/789 | AmsEVP-Ufs-Am-Cf-Uf-Uf- Um-Am-Am-Um-Um-Um- Um-Af-Am-Af-Am-Cm-Cm- Cm-Ams-Ams-Um | 1244/1586 |
| | C1836-DV28P | Ums-Gms-Gm-Gm-Um-Um-Uf- Um-Af-Am-Af-Am-Um-Um- Am-Am-Af-Gm-Um-Am-Um | 1193/789 | AmsEVP-Ufs-Af-Cf-Um-Uf- Um-Am-Am-Um-Um-Um- Um-Af-Am-Af-Am-Cm-Cm- Cm-Ams-Ams-Um | 1245/1586 |
| | C1836-DV32P | Ums-Gms-Gm-Gm-Um-Um-Uf- Um-Af-Am-Af-Am-Um-Um- Am-Am-Af-Gm-Um-Am-Um | 1193/789 | AmsEVP-Ufs-Am-Cm-Uf-Uf- Um-Am-Am-Um-Um-Um- Um-Af-Am-Af-Am-Cm-Cm- Cm-Ams-Ams-Um | 1246/1586 |
| | C1836-DV34P | Ums-Gms-Gm-Gm-Um-Um-Uf- Um-Af-Am-Af-Am-Um-Um- Am-Am-Af-Gm-Um-Am-Um | 1193/789 | AmsEVP-Ufs-Af-Cm-Um-Uf- Um-Am-Am-Um-Um-Um- Um-Af-Am-Af-Am-Cm-Cm- Cm-Ams-Ams-Um | 1247/1586 |
| | C1789-DV29P | Gms-Ums-Um-Um-Gm-Um-Gf- Am-Af-Am-Cf-Am-Am-Am- Am-Am-Af-Gm-Um-Gm-Um | 1195/789 | AmsEVP-Cfs-Am-Cf-Uf-Uf- Um-Um-Um-Um-Gm-Um- Um-Uf-Cm-Af-Cm-Am-Am- Am-Cms-Ams-Am | 1248/1586 |
| | C1789-DV26P | Gms-Ums-Um-Um-Gm-Um-Gf- Am-Af-Af-Cf-Am-Am-Am- Am-Am-Af-Gm-Um-Gm-Um | 1196/789 | AmsEVP-Cfs-Af-Cf-Um-Uf- Um-Um-Um-Um-Gm-Um- Um-Uf-Cm-Af-Cm-Am-Am- Am-Cms-Ams-Am | 1249/1586 |
| | C1789-DV27P | Gms-Ums-Um-Um-Gm-Um-Gf- Am-Af-Af-Cf-Am-Am-Am- Am-Am-Af-Gm-Um-Gm-Um | 1196/789 | AmsEVP-Cfs-Am-Cf-Uf-Uf- Um-Um-Um-Um-Gm-Um- Um-Uf-Cm-Af-Cm-Am-Am- Am-Cms-Ams-Am | 1248/1586 |
| 1789 | C1789-DV28P | Gms-Ums-Um-Um-Gm-Um-Gf- Am-Af-Am-Cf-Am-Am-Am- Am-Am-Af-Gm-Um-Gm-Um | 1195/790 | AmsEVP-Cfs-Af-Cf-Um-Uf- Um-Um-Um-Um-Gm-Um- Um-Uf-Cm-Af-Cm-Am-Am- Am-Cms-Ams-Am | 1249/1587 |
| | C1789-DV32P | Gms-Ums-Um-Um-Gm-Um-Gf- Am-Af-Am-Cf-Am-Am-Am- Am-Am-Af-Gm-Um-Gm-Um | 1195/790 | AmsEVP-Cfs-Am-Cm-Uf-Uf- Um-Um-Um-Um-Gm-Um- Um-Uf-Cm-Af-Cm-Am-Am- Am-Cms-Ams-Am | 1250/1587 |
| | C1789-DV34P | Gms-Ums-Um-Um-Gm-Um-Gf- Am-Af-Am-Cf-Am-Am-Am- Am-Am-Af-Gm-Um-Gm-Um | 1195/790 | AmsEVP-Cfs-Af-Cm-Um-Uf- Um-Um-Um-Um-Gm-Um- Um-Uf-Cm-Af-Cm-Am-Am- Am-Cms-Ams-Am | 1251/1587 |
| | C1789-DV25P | Gms-Ums-Um-Um-Gm-Um-Gf- Am-Af-Af-Cf-Am-Am-Am- Am-Am-Af-Gm-Um-Gm-Um | 1196/790 | AmsEVP-Cfs-Am-Cm-Um- Uf-Um-Um-Um-Um-Gm-Um- Um-Uf-Cm-Af-Cm-Am-Am- Am-Cms-Ams-Am | 1252/1587 |
| | C1789-DV33P | Gms-Ums-Um-Um-Gm-Um-Gf- Am-Af-Am-Cf-Am-Am-Am- Am-Am-Af-Gm-Um-Gm-Um | 1195/790 | AmsEVP-Cfs-Af-Cm-Uf-Uf- Um-Um-Um-Um-Gm-Um- Um-Uf-Cm-Af-Cm-Am-Am- Am-Cms-Ams-Am | 1253/1587 |
| | C1789-DV22 | Gms-Ums-Um-Um-Gm-Um-Gf- Am-Af-Af-Cf-Am-Am-Am- Am-Am-Am-Gm-Um-Gm-Um | 1197/790 | Ams-Cfs-Am-Cm-Um-Uf-Um- Um-Um-Um-Gm-Um-Um-Uf- Cm-Af-Cm-Am-Am-Am-Cms- Ams-Am | 1254/1587 |
| 1839 | C1839-DV29P | Gms-Ums-Um-Um-Um-Am-Af- Am-Af-Um-Uf-Am-Am-Am- Gm-Um-Af-Um-Am-Cm-Am | 1198/791 | UmsEVP-Gfs-Um-Af-Uf-Af- Cm-Um-Um-Um-Am-Am- Um-Uf-Um-Uf-Am-Am-Am- Am-Cms-Cms-Cm | 1255/1588 |
| | C1839-DV26P | Gms-Ums-Um-Um-Um-Am-Af- Am-Af-Uf-Uf-Am-Am-Am- Gm-Um-Af-Um-Am-Cm-Am | 1199/791 | UmsEVP-Gfs-Uf-Af-Um-Af- Cm-Um-Um-Um-Am-Am- Um-Uf-Um-Uf-Am-Am-Am- Am-Cms-Cms-Cm | 1256/1588 |
| | C1839-DV27P | Gms-Ums-Um-Um-Um-Am-Af- Am-Af-Uf-Uf-Am-Am-Am- Gm-Um-Af-Um-Am-Cm-Am | 1199/791 | UmsEVP-Gfs-Um-Af-Uf-Af- Cm-Um-Um-Um-Am-Am- Um-Uf-Um-Uf-Am-Am-Am- Am-Cms-Cms-Cm | 1255/1588 |
| | C1839-DV28P | Gms-Ums-Um-Um-Um-Am-Af- Am-Af-Um-Uf-Am-Am-Am- Gm-Um-Af-Um-Am-Cm-Am | 1198/791 | UmsEVP-Gfs-Uf-Af-Um-Af- Cm-Um-Um-Um-Am-Am- Um-Uf-Um-Uf-Am-Am-Am- Am-Cms-Cms-Cm | 1256/1588 |
| | C1839-DV32P | Gms-Ums-Um-Um-Um-Am-Af- Am-Af-Um-Uf-Am-Am-Am- Gm-Um-Af-Um-Am-Cm-Am | 1198/791 | UmsEVP-Gfs-Um-Am-Uf-Af- Cm-Um-Um-Um-Am-Am- Um-Uf-Um-Uf-Am-Am-Am- Am-Cms-Cms-Cm | 1257/1588 |
| | C1839-DV34P | Gms-Ums-Um-Um-Um-Am-Af- Am-Af-Um-Uf-Am-Am-Am- Gm-Um-Af-Um-Am-Cm-Am | 1198/791 | UmsEVP-Gfs-Uf-Am-Um-Af- Cm-Um-Um-Um-Am-Am- Um-Uf-Um-Uf-Am-Am-Am- Am-Cms-Cms-Cm | 1258/1588 |
| 1791 | C1791-DV29P | Ums-Ums-Gm-Um-Gm-Am-Af- Am-Cf-Am-Af-Am-Am-Am- Am-Gm-Uf-Gm-Um-Um-Am | 1200/792 | UmsEVP-Afs-Am-Cf-Af-Cf- Um-Um-Um-Um-Um-Um- Gm-Uf-Um-Uf-Cm-Am-Cm- Am-Ams-Ams-Cm | 1259/1589 |
| | C1791-DV26P | Ums-Ums-Gm-Um-Gm-Am-Af- Am-Cf-Af-Af-Am-Am-Am- Am-Gm-Uf-Gm-Um-Um-Am | 1201/792 | UmsEVP-Afs-Af-Cf-Am-Cf- Um-Um-Um-Um-Um-Um- Gm-Uf-Um-Uf-Cm-Am-Cm- Am-Ams-Ams-Cm | 1260/1589 |
| | C1791-DV27P | Ums-Ums-Gm-Um-Gm-Am-Af- Am-Cf-Af-Af-Am-Am-Am- Am-Gm-Uf-Gm-Um-Um-Am | 1201/792 | UmsEVP-Afs-Am-Cf-Af-Cf- Um-Um-Um-Um-Um-Um- Gm-Uf-Um-Uf-Cm-Am-Cm- Am-Ams-Ams-Cm | 1259/1589 |
| | C1791-DV28P | Ums-Ums-Gm-Um-Gm-Am-Af- Am-Cf-Am-Af-Am-Am-Am- Am-Gm-Uf-Gm-Um-Um-Am | 1200/792 | UmsEVP-Afs-Af-Cf-Am-Cf- Um-Um-Um-Um-Um-Um- Gm-Uf-Um-Uf-Cm-Am-Cm- Am-Ams-Ams-Cm | 1260/1589 |
| | C1791-DV32P | Ums-Ums-Gm-Um-Gm-Am-Af- Am-Cf-Am-Af-Am-Am-Am- Am-Gm-Uf-Gm-Um-Um-Am | 1200/792 | UmsEVP-Afs-Am-Cm-Af-Cf- Um-Um-Um-Um-Um-Um- Gm-Uf-Um-Uf-Cm-Am-Cm- Am-Ams-Ams-Cm | 1261/1589 |
| | C1791-DV34P | Ums-Ums-Gm-Um-Gm-Am-Af- Am-Cf-Am-Af-Am-Am-Am- Am-Gm-Uf-Gm-Um-Um-Am | 1200/792 | UmsEVP-Afs-Af-Cm-Am-Cf- Um-Um-Um-Um-Um-Um- Gm-Uf-Um-Uf-Cm-Am-Cm- Am-Ams-Ams-Cm | 1262/1589 |
| 1795 | C1795-DV29P | Gms-Ams-Am-Am-Cm-Am-Af- Am-Af-Am-Af-Gm-Um-Gm- Um-Um-Cf-Cm-Cm-Um-Um | 1202/793 | AmsEVP-Afs-Gm-Gf-Gf-Af- Am-Cm-Am-Cm-Um-Um- Um-Uf-Um-Uf-Gm-Um-Um- Um-Cms-Ams-Cm | 1263/1590 |
| | C1795-DV26P | Gms-Ams-Am-Am-Cm-Am-Af- Am-Af-Af-Af-Gm-Um-Gm- Um-Um-Cf-Cm-Cm-Um-Um | 1203/793 | AmsEVP-Afs-Gf-Gf-Gm-Af- Am-Cm-Am-Cm-Um-Um- Um-Uf-Um-Uf-Gm-Um-Um- Um-Cms-Ams-Cm | 1264/1590 |
| | C1795-DV27P | Gms-Ams-Am-Am-Cm-Am-Af- Am-Af-Af-Af-Gm-Um-Gm- Um-Um-Cf-Cm-Cm-Um-Um | 1203/793 | AmsEVP-Afs-Gm-Gf-Gf-Af- Am-Cm-Am-Cm-Um-Um- Um-Uf-Um-Uf-Gm-Um-Um- Um-Cms-Ams-Cm | 1263/1590 |
| | C1795-DV28P | Gms-Ams-Am-Am-Cm-Am-Af- Am-Af-Am-Af-Gm-Um-Gm- Um-Um-Cf-Cm-Cm-Um-Um | 1202/793 | AmsEVP-Afs-Gf-Gf-Gm-Af- Am-Cm-Am-Cm-Um-Um- Um-Uf-Um-Uf-Gm-Um-Um- Um-Cms-Ams-Cm | 1264/1590 |
| | C1795-DV32P | Gms-Ams-Am-Am-Cm-Am-Af- Am-Af-Am-Af-Gm-Um-Gm- Um-Um-Cf-Cm-Cm-Um-Um | 1202/793 | AmsEVP-Afs-Gm-Gm-Gf-Af- Am-Cm-Am-Cm-Um-Um- Um-Uf-Um-Uf-Gm-Um-Um- Um-Cms-Ams-Cm | 1265/1590 |
| | C1795-DV34P | Gms-Ams-Am-Am-Cm-Am-Af- Am-Af-Am-Af-Gm-Um-Gm- Um-Um-Cf-Cm-Cm-Um-Um | 1202/793 | AmsEVP-Afs-Gf-Gm-Gm-Af- Am-Cm-Am-Cm-Um-Um- Um-Uf-Um-Uf-Gm-Um-Um- Um-Cms-Ams-Cm | 1266/1590 |
| 1585s | C1585s-DV29SP | Cms-Ums-Um-Cm-Um-Af-Am- Uf-Gm-Af-Gm-Um-Cm-Gm- Am-Cf-Um-Um-Um-Am | 1204/794 | UmsEVP-Afs-Am-Af-Gf-Uf- Cm-Gm-Am-Cm-Um-Cm- Am-Uf-Um-Af-Gm-Am-Am- Gms-Ams-Am | 1267/1591 |
| | C1585s-DV26SP | Cms-Ums-Um-Cm-Um-Af-Am- Uf-Gf-Af-Gm-Um-Cm-Gm- Am-Cf-Um-Um-Um-Am | 1205/794 | UmsEVP-Afs-Af-Af-Gm-Uf- Cm-Gm-Am-Cm-Um-Cm- Am-Uf-Um-Af-Gm-Am-Am- Gms-Ams-Am | 1268/1591 |
| | C1585s-DV27SP | Cms-Ums-Um-Cm-Um-Af-Am- Uf-Gf-Af-Gm-Um-Cm-Gm- Am-Cf-Um-Um-Um-Am | 1205/794 | UmsEVP-Afs-Am-Af-Gf-Uf- Cm-Gm-Am-Cm-Um-Cm- Am-Uf-Um-Af-Gm-Am-Am- Gms-Ams-Am | 1267/1591 |
| | C1585s-DV28SP | Cms-Ums-Um-Cm-Um-Af-Am- Uf-Gm-Af-Gm-Um-Cm-Gm- Am-Cf-Um-Um-Um-Am | 1204/794 | UmsEVP-Afs-Af-Af-Gm-Uf- Cm-Gm-Am-Cm-Um-Cm- Am-Uf-Um-Af-Gm-Am-Am- Gms-Ams-Am | 1268/1591 |
| | C1585s-DV32SP | Cms-Ums-Um-Cm-Um-Af-Am- Uf-Gm-Af-Gm-Um-Cm-Gm- Am-Cf-Um-Um-Um-Am | 1204/794 | UmsEVP-Afs-Am-Am-Gf-Uf- Cm-Gm-Am-Cm-Um-Cm- Am-Uf-Um-Af-Gm-Am-Am- Gms-Ams-Am | 1269/1591 |
| | C1585s-DV34SP | Cms-Ums-Um-Cm-Um-Af-Am- Uf-Gm-Af-Gm-Um-Cm-Gm- Am-Cf-Um-Um-Um-Am | 1204/794 | UmsEVP-Afs-Af-Am-Gm-Uf- Cm-Gm-Am-Cm-Um-Cm- Am-Uf-Um-Af-Gm-Am-Am- Gms-Ams-Am | 1270/1591 |
| | C1585s-DV22S | Cms-Ums-Um-Cm-Um-Af-Am- Uf-Gf-Af-Gm-Um-Cm-Gm- Am-Cm-Um-Um-Um-Am | 1206/794 | Ums-Afs-Am-Am-Gm-Uf- Cm-Gm-Am-Cm-Um-Cm- Am-Uf-Um-Af-Gm-Am-Am- Gms-Ams-Am | 1271/1591 |
| 1576s | C1576s-DV29SP | Cms-Cms-Am-Cm-Cm-Uf-Um- Uf-Um-Cf-Um-Um-Cm-Um- Am-Af-Um-Gm-Am-Am | 1207/795 | UmsEVP-Ufs-Cm-Af-Uf-Uf- Am-Gm-Am-Am-Gm-Am- Am-Af-Am-Gf-Gm-Um-Gm- Gms-Gms-Am | 1272/1592 |
| | C1576s-DV28SP | Cms-Cms-Am-Cm-Cm-Uf-Um- Uf-Um-Cf-Um-Um-Cm-Um- Am-Af-Um-Gm-Am-Am | 1207/795 | UmsEVP-Ufs-Cf-Af-Um-Uf- Am-Gm-Am-Am-Gm-Am- Am-Af-Am-Gf-Gm-Um-Gm- Gms-Gms-Am | 1273/1592 |
| | C1576s-DV26SP | Cms-Cms-Am-Cm-Cm-Uf-Um- Uf-Uf-Cf-Um-Um-Cm-Um- Am-Af-Um-Gm-Am-Am | 1208/795 | UmsEVP-Ufs-Cf-Af-Um-Uf- Am-Gm-Am-Am-Gm-Am- Am-Af-Am-Gf-Gm-Um-Gm- Gms-Gms-Am | 1273/1592 |
| | C1576s-DV25SP | Cms-Cms-Am-Cm-Cm-Uf-Um- Uf-Uf-Cf-Um-Um-Cm-Um- Am-Af-Um-Gm-Am-Am | 1208/795 | UmsEVP-Ufs-Cm-Am-Um- Uf-Am-Gm-Am-Am-Gm-Am- Am-Af-Am-Gf-Gm-Um-Gm- Gms-Gms-Am | 1274/1592 |
| | C1576s-DV27SP | Cms-Cms-Am-Cm-Cm-Uf-Um- Uf-Uf-Cf-Um-Um-Cm-Um- Am-Af-Um-Gm-Am-Am | 1208/795 | UmsEVP-Ufs-Cm-Af-Uf-Uf- Am-Gm-Am-Am-Gm-Am- Am-Af-Am-Gf-Gm-Um-Gm- Gms-Gms-Am | 1272/1592 |
| | C1576s-DV22S | Cms-Cms-Am-Cm-Cm-Uf-Um- Uf-Uf-Cf-Um-Um-Cm-Um- Am-Am-Um-Gm-Am-Am | 1209/795 | Ums-Ufs-Cm-Am-Um-Uf- Am-Gm-Am-Am-Gm-Am- Am-Af-Am-Gf-Gm-Um-Gm- Gms-Gms-Am | 1275/1592 |
| 1578s | C1578s-DV29SP | Ams-Cms-Cm-Um-Um-Uf-Um- Cf-Um-Uf-Cm-Um-Am-Am- Um-Gf-Am-Gm-Um-Am | 1210/796 | UmsEVP-Afs-Cm-Uf-Cf-Af- Um-Um-Am-Gm-Am-Am- Gm-Af-Am-Af-Am-Gm-Gm- Ums-Gms-Gm | 1276/1593 |
| | C1578s-DV28SP | Ams-Cms-Cm-Um-Um-Uf-Um- Cf-Um-Uf-Cm-Um-Am-Am- Um-Gf-Am-Gm-Um-Am | 1210/796 | UmsEVP-Afs-Cf-Uf-Cm-Af- Um-Um-Am-Gm-Am-Am- Gm-Af-Am-Af-Am-Gm-Gm- Ums-Gms-Gm | 1277/1593 |
| | C1578s-DV26SP | Ams-Cms-Cm-Um-Um-Uf-Um- Cf-Uf-Uf-Cm-Um-Am-Am- Um-Gf-Am-Gm-Um-Am | 1211/796 | UmsEVP-Afs-Cf-Uf-Cm-Af- Um-Um-Am-Gm-Am-Am- Gm-Af-Am-Af-Am-Gm-Gm- Ums-Gms-Gm | 1277/1593 |
| | C1578s-DV25SP | Ams-Cms-Cm-Um-Um-Uf-Um- Cf-Uf-Uf-Cm-Um-Am-Am- Um-Gf-Am-Gm-Um-Am | 1211/796 | UmsEVP-Afs-Cm-Um-Cm- Af-Um-Um-Am-Gm-Am-Am- Gm-Af-Am-Af-Am-Gm-Gm- Ums-Gms-Gm | 1278/1593 |
| | C1578s-DV27SP | Ams-Cms-Cm-Um-Um-Uf-Um- Cf-Uf-Uf-Cm-Um-Am-Am- Um-Gf-Am-Gm-Um-Am | 1211/796 | UmsEVP-Afs-Cm-Uf-Cf-Af- Um-Um-Am-Gm-Am-Am- Gm-Af-Am-Af-Am-Gm-Gm- Ums-Gms-Gm | 1276/1593 |
| | C1578s-DV22S | Ams-Cms-Cm-Um-Um-Uf-Um- Cf-Uf-Uf-Cm-Um-Am-Am- Um-Gm-Am-Gm-Um-Am | 1212/796 | Ums-Afs-Cm-Um-Cm-Af-Um- Um-Am-Gm-Am-Am-Gm-Af- Am-Af-Am-Gm-Gm-Ums- Gms-Gm | 1279/1593 |
| 1838 | C1838-DV29P | Gms-Gms-Um-Um-Um-Um-Af- Am-Af-Am-Uf-Um-Am-Am- Am-Gm-Uf-Am-Um-Am-Am | 1213/797 | UmsEVP-Ufs-Am-Uf-Af-Cf- Um-Um-Um-Am-Am-Um- Um-Uf-Um-Af-Am-Am-Am- Cm-Cms-Cms-Am | 1280/1594 |
| | C1838-DV28P | Gms-Gms-Um-Um-Um-Um-Af- Am-Af-Am-Uf-Um-Am-Am- Am-Gm-Uf-Am-Um-Am-Am | 1213/797 | UmsEVP-Ufs-Af-Uf-Am-Cf- Um-Um-Um-Am-Am-Um- Um-Uf-Um-Af-Am-Am-Am- Cm-Cms-Cms-Am | 1 1281/1594 |
| | C1838-DV26P | Gms-Gms-Um-Um-Um-Um-Af- Am-Af-Af-Uf-Um-Am-Am- Am-Gm-Uf-Am-Um-Am-Am | 1214/797 | UmsEVP-Ufs-Af-Uf-Am-Cf- Um-Um-Um-Am-Am-Um- Um-Uf-Um-Af-Am-Am-Am- Cm-Cms-Cms-Am | 1 1281/1594 |
| | C1838-DV25P | Gms-Gms-Um-Um-Um-Um-Af- Am-Af-Af-Uf-Um-Am-Am- Am-Gm-Uf-Am-Um-Am-Am | 1214/797 | UmsEVP-Ufs-Am-Um-Am- Cf-Um-Um-Um-Am-Am-Um- Um-Uf-Um-Af-Am-Am-Am- Cm-Cms-Cms-Am | 1 1282/1594 |
| | C1838-DV27P | Gms-Gms-Um-Um-Um-Um-Af- Am-Af-Af-Uf-Um-Am-Am- Am-Gm-Uf-Am-Um-Am-Am | 1214/797 | UmsEVP-Ufs-Am-Uf-Af-Cf- Um-Um-Um-Am-Am-Um- Um-Uf-Um-Af-Am-Am-Am- Cm-Cms-Cms-Am | 1 1280/1594 |
| | C1838-DV22 | Gms-Gms-Um-Um-Um-Um-Af- Am-Af-Af-Uf-Um-Am-Am- Am-Gm-Um-Am-Um-Am-Am | 1215/797 | Ums-Ufs-Am-Um-Am-Cf- Um-Um-Um-Am-Am-Um- Um-Uf-Um-Af-Am-Am-Am- Cm-Cms-Cms-Am | 1 1283/1594 |

Cell line: HepG2 cells.

Solvents: sterile nuclease-free water, Gibco Opti-MEM.

### 2. Experimental methods

The inhibitory effects of the test samples on AGT mRNA expression in HepG2 cells were evaluated by qRT-PCR.

### 2.1 Cell culture

Subcultured HepG2 cells in the logarithmic growth phase were cultured in DMEM culture medium containing 10% fetal bovine serum (supplemented with penicillin and streptomycin at 100 µL/mL), and incubated in a cell incubator at 37°C with 5% CO₂. The medium was exchanged once daily. The cells were digested with 0.25% trypsin, followed by centrifugation at 1000 rpm for 5 minutes. The supernatant was discarded, and fresh medium was added for continued culture.

### 2.2 Cell transfection

Preparation of transfection mixture: Lipofectamine RNAiMAX was mixed with Opti-MEM at a volume ratio of 2:98 and vortexed thoroughly.

Preparation of transfection reagent: 60 µL of siRNA solution (diluted in Opti-MEM) was mixed with 60 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 60 µL of transfection mixture was added to 60 µL of Opti-MEM, vortexed thoroughly, followed by incubation at room temperature for 15 minutes.

The prepared transfection reagent was added to a 24-well cell culture plate (100 µL per well), resulting in a final siRNA concentration of 0.02 nM per well. Subsequently, 500 µL of cell suspension (1.5 × 10⁵ cells per mL) was added to each well. After cross-mixing, the plate was incubated in a cell incubator at 37°C with 5% CO₂ for 40 hours.

### 2.3 AGT mRNA analysis

The procedure is the same as described in "2.3 AGT mRNA analysis" of Example 18.

### 2.4 Data processing

AGT mRNA expression rate (%) was calculated using the following formula: 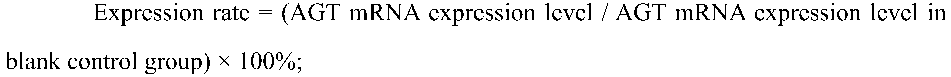 Expression rate = (AGT mRNA expression level / AGT mRNA expression level in blank control group) × 100%;
$Inhibition rate of AGT gene expression = 100 % - expression rate \left(%\right) .$

### 2.5 IC₅₀ assay

The siRNAs used for IC₅₀ determination were subjected to serial dilution starting from 1.0 nM with 4-fold dilutions, totaling 8 concentration points (1.0 nM, 0.25 nM, 0.0625 nM, 15.6 pM, 3.9 pM, 0.977 pM, 0.244 Pm, and 0.061 pM). The inhibition rates for these sequences at each concentration were measured, and the resulting data were plotted to calculate IC₅₀ values.

### 3. Experimental results

Based on three independent experiments, the inhibition rates of AGT gene expression in HepG2 cells by these modified sequences are shown in Tables 64 to 76.

The activity assay results demonstrated that all 13 candidate sequences (unmodified sequences 1576s, 1578s, 1838, 1835, 1816, 1812, 1579, 1836, 1789, 1839, 1791, 1795, and 1585s), when modified using templates DV25P to DV29P, DV32P to DV34P, DV25SP to DV29SP, and DV32SP to DV34SP designed in the present disclosure, exhibited significant inhibitory effects on AGT gene expression, with inhibition rates exceeding 40%. Specifically, C1812-DV25P, C1579-DV33P, and C1789-DV26P exhibited inhibition rates of 74.4%, 74.0%, and 73.6%, respectively.

The sequences modified using templates DV25P to DV29P, DV32P to DV34P, DV25SP to DV29SP, and DV32SP to DV34SP disclosed herein showed significantly enhanced inhibition of AGT gene expression as compared to the alternately modified sequences. For example, for unmodified sequence 1579, the inhibition rate of C1579-DV33 (the sequence modified using template DV33P) was 12.9% higher than that of the alternately modified sequence; for unmodified sequence 1812, the inhibition rate of C1812-DV25P (the sequence modified using template DV25P) was 11.5% higher than that of the alternately modified sequence.

Moreover, the same siRNA sequence exhibited significant differences in activity when modified using different modification templates. For example, for unmodified sequence 1579, the sequence modified using template DV33P disclosed herein exhibited a 29.8% increase in inhibition rate compared to the sequence modified using template DV28P disclosed herein, and a 18.4% increase in inhibition rate compared to the sequence modified using the previously disclosed Advanced ESC template DV22.

IC₅₀ assays demonstrated that the sequences modified using the templates designed in the present disclosure exhibited IC₅₀ values ranging from 0.0019 to 0.0615 nM. For example, the IC₅₀ values of C1789-DV25P, C1812-DV25P, and C1789-DV26P were 0.0019 nM, 0.0022 nM, and 0.0026 nM, respectively. These sequences effectively inhibited AGT gene expression at low concentrations.

### (i) Inhibition of AGT gene expression by unmodified sequences modified using different templates

### (1) Unmodified sequence 1576s

**Table 64: Inhibition rates of AGT gene expression by unmodified sequence 1576s modified using different templates**

| **Unmodified sequence ID** | **Template-modified sequence ID** | **Inhibition rate (%) of template-modified sequence** | **Inhibition rate (%) of corr. alternately modified sequence** | **Increase vs alternately modified sequence (%)** |
|---|---|---|---|---|
| | C1576s-DV26SP | 73.4 | | 8.8 |
| | C1576s-DV28SP | 58.8 | | -5.8 |
| 1576s | C1576s-DV29SP | 79.3 | 64.6 | 14.7 |
| | C1576s-DV25SP | 73.3 | | 8.7 |
| | C1576s-DV27SP | 69.7 | | 5.1 |
| | C1576s-DV22S | 63.9 | | -0.7 |

### I. Templates DV25SP to DV29SP disclosed herein

The unmodified sequence 84 modified using templates DV25SP, DV26SP, and DV29SP designed in the present disclosure exhibited inhibition rates exceeding 70% on AGT gene expression, indicating significantly enhanced inhibition of AGT gene expression as compared to alternating 2'-O-methyl and 2'-fluoro modifications. For example, after modification using templates DV25SP and DV29SP, the inhibition rates of C1576s-DV25SP and C1576s-DV29SP were increased by 8.7% and 14.7%, respectively.

### II. Advanced ESC templates DV22 (fluorinated sites: positions 2, 6, 14, and 16 of the antisense strand; positions 7, 9, 10, and 11 of the sense strand) disclosed in the prior art

For unmodified sequence 1576s, after modification using template DV22S, the resulting sequence C1576s-DV22SP exhibited an inhibition rate of 63.9% on AGT gene expression, which was 0.7% lower than that of the alternately modified sequence.

After modification using templates DV25SP and DV29SP disclosed herein, the inhibition rates of C1576s-DV25SP and C1576s-DV29SP were increased by 9.4% and 15.4%, respectively, compared to C1576s-DV22S, indicating a significant improvement.

It can be concluded that:
1) The unmodified sequence 1576s modified using templates DV25SP to DV29SP disclosed herein showed significantly enhanced inhibition of AGT gene expression as compared to the alternately modified sequences. For example, when the unmodified sequence 1576s was modified using DV26SP, the inhibition rate was increased by 8.8% compared to the alternately modified sequence.
2) The same siRNA sequence exhibited significant differences in activity when modified using different modification templates. For example, when the unmodified sequence 1576s was modified using templates DV25SP and DV29SP disclosed herein, the inhibition rate was increased by up to 20.5% compared to modification using template DV28SP disclosed herein, indicating a significant improvement; the inhibition rate was increased by up to 15.4% compared to modification using the previously disclosed Advanced ESC templates DV22S, indicating a significant improvement.
3) The siRNA sequences modified using different templates varied greatly in activity, with differences of up to 20.5%. Thus, it is uncertain which modification template would confer high activity to a given siRNA sequence.

### (2) Unmodified sequence 1578s

**Table 65: Inhibition rates of AGT gene expression by unmodified sequence 1578s modified using different templates**

| **Unmodified sequence ID** | **Template-modified sequence ID** | **Inhibition rate (%) of template-modified sequence** | **Inhibition rate (%) of corr. alternately modified sequence** | **Increase vs alternately modified sequence (%)** |
|---|---|---|---|---|
| | C1578s-DV26SP | 69.7 | | 5.8 |
| | C1578s-DV28SP | 56.9 | | -7.0 |
| 1578s | C1578s-DV29SP | 77.1 | 63.9 | 13.2 |
| | C1578s-DV25SP | 75.8 | | 11.9 |
| | C1578s-DV27SP | 73.1 | | 9.2 |
| | C1578s-DV22S | 61.1 | | -2.8 |

### I. Templates DV25SP to DV29SP disclosed herein

The unmodified sequence 1578s modified using templates DV25SP, DV27SP, and DV29SP designed in the present disclosure exhibited inhibition rates exceeding 70% on AGT gene expression, indicating significantly enhanced inhibition of AGT gene expression as compared to alternating 2'-O-methyl and 2'-fluoro modifications. For example, after modification using templates DV25SP and DV29SP, the inhibition rates of C1578s-DV25SP and C1578s-DV29SP were increased by 11.9% and 13.2%, respectively.

### II. Advanced ESC templates DV22 (fluorinated sites: positions 2, 6, 14, and 16 of the antisense strand; positions 7, 9, 10, and 11 of the sense strand) disclosed in the prior art

For unmodified sequence 1578s, after modification using template DV22S, the resulting sequence C1578s-DV22S exhibited an inhibition rate of 61.1% on AGT gene expression, which was 2.8% lower than that of the alternately modified sequence.

After modification using templates DV25SP and DV29SP disclosed herein, the inhibition rates of C1578s-DV25SP and C1578s-DV29SP were increased by 14.7% and 16.0%, respectively, compared to C1578s-DV22S, indicating a significant improvement.

It can be concluded that:
1) The unmodified sequence 1578s modified using templates DV25SP to DV29SP disclosed herein showed significantly enhanced inhibition of AGT gene expression as compared to the alternately modified sequences. For example, when the unmodified sequence 1578s was modified using DV27SP, the inhibition rate was increased by 9.2% compared to the alternately modified sequence.
2) The same siRNA sequence exhibited significant differences in activity when modified using different modification templates. For example, when the unmodified sequence 1578s was modified using templates DV25SP and DV29SP disclosed herein, the inhibition rate was increased by up to 20.2% compared to modification using template DV28SP disclosed herein, indicating a significant improvement; the inhibition rate was increased by up to 16.0% compared to modification using the previously disclosed Advanced ESC templates DV22S, indicating a significant improvement.
3) The siRNA sequences modified using different templates varied greatly in activity, with differences of up to 20.2%. Thus, it is uncertain which modification template would confer high activity to a given siRNA sequence.

### (3) Unmodified sequence 1838

**Table 66: Inhibition rates of AGT gene expression by unmodified sequence 1838 modified using different templates**

| **Unmodified sequence ID** | **Template-modified sequence ID** | **Inhibition rate (%) of template-modified sequence** | **Inhibition rate (%) of corr. alternately modified sequence** | **Increase vs alternately modified sequence (%)** |
|---|---|---|---|---|
| | C1838-DV26P | 67.2 | | 3.6 |
| | C1838-DV28P | 60.3 | | -3.3 |
| 1838 | C1838-DV29P | 75.6 | 63.6 | 12.0 |
| | C1838-DV25P | 69.3 | | 5.7 |
| | C1838-DV27P | 72.3 | | 8.7 |
| | C1838-DV22 | 62.7 | | -0.9 |

### I. Templates DV25P to DV29P disclosed herein

The unmodified sequence 1838 modified using templates DV27P and DV29P designed in the present disclosure exhibited inhibition rates exceeding 70% on AGT gene expression, indicating significantly enhanced inhibition of AGT gene expression as compared to alternating 2'-O-methyl and 2'-fluoro modifications. For example, after modification using templates DV27P and DV29P, the inhibition rates of C1838-DV27P and C1838-DV29P were increased by 8.7% and 12.0%, respectively.

### II. Advanced ESC templates DV22 (fluorinated sites: positions 2, 6, 14, and 16 of the antisense strand; positions 7, 9, 10, and 11 of the sense strand) disclosed in the prior art

For unmodified sequence 1838, after modification using template DV22, the resulting sequence C1838-DV22 exhibited an inhibition rate of 62.7% on AGT gene expression, which was 0.9% lower than that of the alternately modified sequence.

After modification using templates DV27P and DV29P disclosed herein, the inhibition rates of C1838-DV27P and C1838-DV29P were increased by 9.6% and 12.9%, respectively, compared to C1838-DV22, indicating a significant improvement.

It can be concluded that:
1) The unmodified sequence 1838 modified using templates DV25P to DV29P disclosed herein showed significantly enhanced inhibition of AGT gene expression as compared to the alternately modified sequences. For example, when the unmodified sequence 1838 was modified using DV27P, the inhibition rate was increased by 8.7% compared to the alternately modified sequence.
2) The same siRNA sequence exhibited significant differences in activity when modified using different modification templates. For example, when the unmodified sequence 1838 was modified using templates DV27P and DV29P disclosed herein, the inhibition rate was increased by up to 15.3% compared to modification using template DV28P disclosed herein, indicating a significant improvement; the inhibition rate was increased by up to 12.9% compared to modification using the previously disclosed Advanced ESC templates DV22, indicating a significant improvement.
3) The siRNA sequences modified using different templates varied greatly in activity, with differences of up to 15.3%. Thus, it is uncertain which modification template would confer high activity to a given siRNA sequence.

### (4) Unmodified sequence 1579

**Table 67: Inhibition rates of AGT gene expression by unmodified sequence 1579 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1579-DV25P | 72.2 | | 11.1 |
| | C1579-DV26P | 70.8 | | 9.7 |
| | C1579-DV27P | 63.3 | | 2.2 |
| | C1579-DV28P | 44.2 | | -16.9 |
| 1579 | C1579-DV29P | 71.7 | 61.1 | 10.6 |
| | C1579-DV32P | 68.9 | | 7.8 |
| | C1579-DV33P | 74.0 | | 12.9 |
| | C1579-DV34P | 63.9 | | 2.8 |
| | C1579-DV22 | 55.6 | | -5.5 |

### I. Templates DV25P to DV29P disclosed herein

The unmodified sequence 1579 modified using templates DV25P, DV26P, and DV29P designed in the present disclosure exhibited inhibition rates exceeding 70% on AGT gene expression, indicating significantly enhanced inhibition of AGT gene expression as compared to alternating 2'-O-methyl and 2'-fluoro modifications. For example, after modification using templates DV25P and DV29P, the inhibition rates of C1579-DV25P and C1579-DV29P were increased by 11.1% and 10.6%, respectively.

### II. Other modification templates disclosed herein

For unmodified sequence 1579, after modification using templates DV32P, DV33P, and DV34P, the resulting sequences C1579-DV32P, C1579-DV33P, and C1579-DV34P exhibited inhibition rates of 68.9%, 74.0%, and 63.9% on AGT gene expression, respectively, which were 7.8%, 12.9%, and 2.8% higher than those of the corresponding alternately modified sequences, respectively.

After modification using templates DV25P and DV29P disclosed herein, the inhibition rates of C1579-DV25P and C1579-DV29P were increased by 3.3% and 2.8% compared to C1579-DV32P, decreased by 1.8% and 2.3% compared to C1579-DV33P, and increased by 8.3% and 7.8% compared to C1579-DV34P.

### III. Advanced ESC templates DV22 (fluorinated sites: positions 2, 6, 14, and 16 of the antisense strand; positions 7, 9, 10, and 11 of the sense strand) disclosed in the prior art

For unmodified sequence 1579, after modification using template DV22, the resulting sequence C1579-DV22 exhibited an inhibition rate of 55.6% on AGT gene expression, which was 5.5% lower than that of the alternately modified sequence.

After modification using templates DV25P and DV29P disclosed herein, the inhibition rates of C1579-DV25P and C1579-DV29P were increased by 16.6% and 16.1%, respectively, compared to C1579-DV22, indicating a significant improvement.

It can be concluded that:
1) The unmodified sequence 1579 modified using templates DV25P to DV29P disclosed herein showed significantly enhanced inhibition of AGT gene expression as compared to the alternately modified sequences. For example, when the unmodified sequence 1579 was modified using DV26, the inhibition rate was increased by 9.7% compared to the alternately modified sequence.
2) The same siRNA sequence exhibited significant differences in activity when modified using different modification templates. For example, when the unmodified sequence 1579 was modified using templates DV25P and DV29P disclosed herein, the inhibition rate was increased by up to 28.0% compared to modification using template DV28P disclosed herein, indicating a significant improvement; the inhibition rate was increased by up to 16.6% compared to modification using the previously disclosed Advanced ESC templates DV22, indicating a significant improvement.
3) The siRNA sequences modified using different templates varied greatly in activity, with differences of up to 28.0%. Thus, it is uncertain which modification template would confer high activity to a given siRNA sequence.

### (5) Unmodified sequence 1585s

**Table 68: Inhibition rates of AGT gene expression by unmodified sequence 1585s modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1585s-DV26SP | 62.6 | | 3.3 |
| | C1585s-DV27SP | 64.6 | | 5.3 |
| | C1585s-DV28SP | 49.5 | | -9.8 |
| 1585s | C1585s-DV29SP | 59.2 | 59.3 | -0.1 |
| | C1585s-DV32SP | 61.8 | | 2.5 |
| | C1585s-DV34SP | 54.1 | | -5.2 |
| | C1585s-DV22S | 55.2 | | -4.1 |

### I. Templates DV26SP to DV29SP disclosed herein

The unmodified sequence 1585s modified using templates DV26SP, DV27SP, and DV29SP designed in the present disclosure exhibited inhibition rates exceeding 50% on AGT gene expression, indicating significantly enhanced inhibition of AGT gene expression as compared to alternating 2'-O-methyl and 2'-fluoro modifications. For example, after modification using templates DV26SP and DV27SP, the inhibition rates of C1585s-DV26SP and C1585s-DV27SP were increased by 3.3% and 5.3%, respectively.

### II. Other modification templates disclosed herein

For unmodified sequence 1585s, after modification using templates DV32SP and DV34SP, the resulting sequences C1585s-DV32SP and C1585s-DV34SP exhibited inhibition rates of 61.8% and 54.1% on AGT gene expression, respectively, which were 2.5% and -5.2% higher than those of the corresponding alternately modified sequences, respectively.

After modification using templates DV26SP and DV27SP disclosed herein, the inhibition rates of C1585s-DV26SP and C1585s-DV27SP were increased by 0.8% and 2.8% compared to C1585s-DV32SP, and increased by 8.5% and 10.5% compared to C1585s-DV34SP.

### III. Advanced ESC templates DV22S (fluorinated sites: positions 2, 6, 14, and 16 of the antisense strand; positions 7, 9, 10, and 11 of the sense strand) disclosed in the prior art

For unmodified sequence 1585s, after modification using template DV22S, the resulting sequence C1585s-DV22S exhibited an inhibition rate of 55.2% on AGT gene expression, which was 4.1% lower than that of the alternately modified sequence.

After modification using templates DV26SP and DV27SP disclosed herein, the inhibition rates of C1585s-DV26SP and C1585s-DV27SP were increased by 7.4% and 9.4%, respectively, compared to C1585s-DV22S, indicating a significant improvement.

It can be concluded that:
1) The unmodified sequence 1585s modified using templates DV26SP to DV29SP disclosed herein showed significantly enhanced inhibition of AGT gene expression as compared to the alternately modified sequences. For example, when the unmodified sequence 1585s was modified using DV27SP, the inhibition rate was increased by 5.3% compared to the alternately modified sequence.
2) The same siRNA sequence exhibited significant differences in activity when modified using different modification templates. For example, when the unmodified sequence 51 was modified using template DV27SP disclosed herein, the inhibition rate was increased by up to 15.1% compared to modification using template DV28SP disclosed herein, indicating a significant improvement; the inhibition rate was increased by up to 9.4% compared to modification using the previously disclosed Advanced ESC templates DV22S, indicating a significant improvement.
3) The siRNA sequences modified using different templates varied greatly in activity, with differences of up to 15.1%. Thus, it is uncertain which modification template would confer high activity to a given siRNA sequence.

### (6) Unmodified sequence 1789

**Table 69: Inhibition rates of AGT gene expression by unmodified sequence 1789 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1789-DV25P | 69.0 | | 5.5 |
| | C1789-DV26P | 73.6 | | 10.1 |
| | C1789-DV27P | 66.3 | | 2.8 |
| | C1789-DV28P | 63.1 | | -0.4 |
| 1789 | C1789-DV29P | 64.5 | 63.5 | 1 |
| | C1789-DV32P | 61.8 | | -1.7 |
| | C1789-DV33P | 57.9 | | -5.6 |
| | C1789-DV34P | 56.9 | | -6.6 |
| | C1789-DV22 | 60.3 | | -3.2 |

### I. Templates DV25P to DV29P disclosed herein

The unmodified sequence 1789 modified using templates DV25P to DV29P designed in the present disclosure exhibited inhibition rates exceeding 60% on AGT gene expression, indicating significantly enhanced inhibition of AGT gene expression as compared to alternating 2'-O-methyl and 2'-fluoro modifications. For example, after modification using templates DV25P and DV26P, the inhibition rates of C1789-DV25P and C1789-DV26P were increased by 5.5% and 10.1%, respectively.

### II. Other modification templates disclosed herein

For unmodified sequence 1789, after modification using templates DV32P to DV34P, the resulting sequences C1789-DV32P, C1789-DV33P, and C1789-DV34P exhibited inhibition rates of 61.8%, 57.9, and 56.9% on AGT gene expression, respectively, which were 1.7%, 5.6, and 6.6% lower than those of the corresponding alternately modified sequences, respectively.

After modification using templates DV25P and DV26P disclosed herein, the inhibition rates of C1789-DV25P and C1789-DV26P were increased by 7.2% and 11.8% compared to C1789-DV32P, increased by 11.1% and 15.7% compared to C1789-DV33P, and increased by 12.1% and 16.7% compared to C1789-DV34P.

### III. Advanced ESC templates DV22 (fluorinated sites: positions 2, 6, 14, and 16 of the antisense strand; positions 7, 9, 10, and 11 of the sense strand) disclosed in the prior art

For unmodified sequence 1789, after modification using template DV22, the resulting sequence C1789-DV22 exhibited an inhibition rate of 60.3% on AGT gene expression, which was 3.2% lower than that of the alternately modified sequence.

After modification using templates DV25P and DV26P disclosed herein, the inhibition rates of C1789-DV25P and C1789-DV26P were increased by 8.7% and 13.3%, respectively, compared to C1789-DV22, indicating a significant improvement.

It can be concluded that:
1) The unmodified sequence 1789 modified using templates DV25P to DV29P disclosed herein showed significantly enhanced inhibition of AGT gene expression as compared to the alternately modified sequences. For example, when the unmodified sequence 81 was modified using DV26P, the inhibition rate was increased by 10.1% compared to the alternately modified sequence.
2) The same siRNA sequence exhibited significant differences in activity when modified using different modification templates. For example, when the unmodified sequence 1789 was modified using templates DV25P and DV26P disclosed herein, the inhibition rate was increased by up to 16.7% compared to modification using templates DV32P to DV34P disclosed herein, indicating a significant improvement; the inhibition rate was increased by up to 13.3% compared to modification using the previously disclosed Advanced ESC templates DV22, indicating a significant improvement.
3) The siRNA sequences modified using different templates varied greatly in activity, with differences of up to 16.7%. Thus, it is uncertain which modification template would confer high activity to a given siRNA sequence.

### (7) Unmodified sequence 1791

**Table 70: Inhibition rates of AGT gene expression by unmodified sequence 1791 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1791-DV26P | 70.3 | | 8.5 |
| | C1791-DV27P | 63.6 | | 1.8 |
| 1791 | C1791-DV28P | 64.6 | 61.8 | 2.8 |
| | C1791-DV29P | 59.4 | | -2.4 |
| | C1791-DV32P | 65.9 | | 4.1 |
| | C1791-DV34P | 66.7 | | 4.9 |

### I. Templates DV26P to 29P disclosed herein

The unmodified sequence 1791 modified using templates DV26P to DV29P designed in the present disclosure exhibited inhibition rates exceeding 55% on AGT gene expression, indicating significantly enhanced inhibition of AGT gene expression as compared to alternating 2'-O-methyl and 2'-fluoro modifications. For example, after modification using templates DV26P and DV28P, the inhibition rates of C1791-DV26P and C1791-DV28P were increased by 8.5% and 2.8%, respectively.

### II. Other modification templates disclosed herein

For unmodified sequence 1791, after modification using templates DV32P and DV34P, the resulting sequences C1791-DV32P and C1791-DV34P exhibited inhibition rates of 65.9% and 66.7% on AGT gene expression, respectively, which were 4.1% and 4.9% higher than those with alternating 2'-O-methyl and 2'-fluoro modifications, respectively.

After modification using template DV26P disclosed herein, the inhibition rate of C1791-DV26P was increased by 4.4% compared to C1791-DV32P, and increased by 3.6% compared to C1791-DV34P.

It can be concluded that:
1) The unmodified sequence 1791 modified using templates DV26P to DV29P disclosed herein showed significantly enhanced inhibition of AGT gene expression as compared to the alternately modified sequences. For example, when the unmodified sequence 1791 was modified using DV26P, the inhibition rate was increased by 8.5% compared to the alternately modified sequence.
2) The same siRNA sequence exhibited significant differences in activity when modified using different modification templates. For example, for unmodified sequence 1791, the sequence modified using template DV26P disclosed herein exhibited a 10.9% increase in inhibition rate compared to the sequence modified using template DV29P disclosed herein, indicating a significant improvement.
3) The siRNA sequences modified using different templates varied greatly in activity, with differences of up to 10.9%. Thus, it is uncertain which modification template would confer high activity to a given siRNA sequence.

### (8) Unmodified sequence 1795

**Table 71: Inhibition rates of AGT gene expression by unmodified sequence 1795 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1795-DV26P | 59.3 | | 1.8 |
| | C1795-DV27P | 63.3 | | 5.8 |
| 1795 | C1795-DV28P | 51.9 | 57.5 | -5.6 |
| | C1795-DV29P | 61.6 | | 4.1 |
| | C1795-DV32P | 55.8 | | -1.7 |
| | C1795-DV34P | 55.0 | | -2.5 |

### I. Templates DV25 to 29P disclosed herein

The unmodified sequence 1795 modified using templates DV26P to DV29P designed in the present disclosure exhibited inhibition rates exceeding 50% on AGT gene expression, indicating significantly enhanced inhibition of AGT gene expression as compared to alternating 2'-O-methyl and 2'-fluoro modifications. For example, after modification using templates DV27P and DV29P, the inhibition rates of C1795-DV27P and C1795-DV29P were increased by 5.8% and 4.1%, respectively.

### II. Other modification templates disclosed herein

For unmodified sequence 1795, after modification using templates DV32P and DV34P, the resulting sequences C1795-DV32P and C1795-DV34P exhibited inhibition rates of 55.8% and 55.0% on AGT gene expression, respectively, which were 1.7% and 2.5% lower than those of the corresponding alternately modified sequences, respectively.

After modification using templates DV27P and DV29P disclosed herein, the inhibition rates of C1795-DV27P and C1795-DV29P were increased by 7.5% and 5.8% compared to C1795-DV32P, and increased by 8.3% and 6.6% compared to C1795-DV34P.

It can be concluded that:
1) The unmodified sequence 1795 modified using templates DV25P to DV29P disclosed herein showed significantly enhanced inhibition of AGT gene expression as compared to the alternately modified sequences. For example, when the unmodified sequence 84 was modified using DV27P, the inhibition rate was increased by 5.8% compared to the alternately modified sequence.
2) The same siRNA sequence exhibited significant differences in activity when modified using different modification templates. For example, for unmodified sequence 1795, the sequence modified using template DV27P disclosed herein exhibited a 11.4% increase in inhibition rate compared to the sequence modified using template DV28P disclosed herein, indicating a significant improvement.
3) The siRNA sequences modified using different templates varied greatly in activity, with differences of up to 11.4%. Thus, it is uncertain which modification template would confer high activity to a given siRNA sequence.

### (9) Unmodified sequence 1812

**Table 72: Inhibition rates of AGT gene expression by unmodified sequence 1812 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1812-DV25P | 74.4 | | 11.5 |
| | C1812-DV26P | 58.0 | | -4.9 |
| | C1812-DV27P | 63.9 | | 1.0 |
| | C1812-DV28P | 55.3 | | -7.6 |
| 1812 | C1812-DV29P | 66.9 | 62.9 | 4.0 |
| | C1812-DV32P | 63.6 | | 0.7 |
| | C1812-DV33P | 54.2 | | -8.7 |
| | C1812-DV34P | 60.7 | | -2.2 |
| | C1812-DV22 | 63.6 | | 0.7 |

### I. Templates DV25P to 29P disclosed herein

The unmodified sequence 1812 modified using templates DV25P to DV29P designed in the present disclosure exhibited inhibition rates exceeding 55% on AGT gene expression, indicating significantly enhanced inhibition of AGT gene expression as compared to alternating 2'-O-methyl and 2'-fluoro modifications. For example, after modification using template DV25P, the inhibition rate of C1812-DV25P was increased by 11.5%.

### II. Other modification templates disclosed herein

For unmodified sequence 1812, after modification using templates DV32P to DV34P, the resulting sequences C1812-DV32P, C1812-DV33P, and C1812-DV34P exhibited inhibition rates of 63.6%, 54.2%, and 60.7% on AGT gene expression, respectively, which were 0.7%, - 8.7%, and -2.2% higher than those of the corresponding alternately modified sequences, respectively.

After modification using template DV25P disclosed herein, the inhibition rate of C1812-DV25P was increased by 10.8% compared to C1812-DV32P, increased by 20.2% compared to C1812-DV33P, and increased by 13.7% compared to C1812-DV34P, indicating a significant improvement.

### III. Advanced ESC templates DV22 (fluorinated sites: positions 2, 6, 14, and 16 of the antisense strand; positions 7, 9, 10, and 11 of the sense strand) disclosed in the prior art

For unmodified sequence 1812, after modification using template DV22, the resulting sequence C812-DV22 exhibited an inhibition rate of 63.6% on AGT gene expression, which was 0.7% higher than that of the alternately modified sequence.

After modification using template DV25P disclosed herein, the inhibition rate of C1812-DV25P was increased by 10.8% compared to C1812-DV22, indicating a significant improvement.

It can be concluded that:
1) The unmodified sequence 1812 modified using templates DV25P to DV29P disclosed herein showed significantly enhanced inhibition of AGT gene expression as compared to the alternately modified sequences. For example, when the unmodified sequence 1812 was modified using DV25P, the inhibition rate was increased by 11.5% compared to the alternately modified sequence.
2) The same siRNA sequence exhibited significant differences in activity when modified using different modification templates. For example, when the unmodified sequence 1812 was modified using template DV25P disclosed herein, the inhibition rate was increased by up to 20.2% compared to modification using template DV33P disclosed herein, indicating a significant improvement; the inhibition rate was increased by up to 10.8% compared to modification using the previously disclosed Advanced ESC templates DV22, indicating a significant improvement.
3) The siRNA sequences modified using different templates varied greatly in activity, with differences of up to 20.2%. Thus, it is uncertain which modification template would confer high activity to a given siRNA sequence.

### (10) Unmodified sequence 1816

**Table 73: Inhibition rates of AGT gene expression by unmodified sequence 1816 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1816-DV25P | 68.1 | | 7.2 |
| | C1816-DV26P | 58.5 | | -2.4 |
| | C1816-DV27P | 64.5 | | 3.6 |
| | C1816-DV28P | 49.2 | | -11.7 |
| 1816 | C1816-DV29P | 68.5 | 60.9 | 7.6 |
| | C1816-DV32P | 58.6 | | -2.3 |
| | C1816-DV33P | 48.4 | | -12.5 |
| | C1816-DV34P | 38.5 | | -22.4 |
| | C1816-DV22 | 66.7 | | 5.8 |

### I. Templates DV25P to DV29P disclosed herein

The unmodified sequence 1816 modified using templates DV25P, DV26P, DV27P, and DV29P designed in the present disclosure exhibited inhibition rates exceeding 55% on AGT gene expression, indicating significantly enhanced inhibition of AGT gene expression as compared to alternating 2'-O-methyl and 2'-fluoro modifications. For example, after modification using template DV29P, the inhibition rate of C1812-DV25P was increased by 7.6%.

### II. Other modification templates disclosed herein

For unmodified sequence 1816, after modification using templates DV32P to DV34P, the resulting sequences C1816-DV32P, C1816-DV33P, and C1816-DV34P exhibited inhibition rates of 58.6%, 48.4%, and 38.5% on AGT gene expression, respectively, which were 2.3%, 12.5%, and 22.4% lower than those of the corresponding alternately modified sequences, respectively.

After modification using template DV29P disclosed herein, the inhibition rate of C1816-DV25P was increased by 9.9% compared to C1816-DV32P, increased by 20.1% compared to C1816-DV33P, and increased by 30.0% compared to C1816-DV34P, indicating a significant improvement.

### III. Advanced ESC templates DV22 (fluorinated sites: positions 2, 6, 14, and 16 of the antisense strand; positions 7, 9, 10, and 11 of the sense strand) disclosed in the prior art

For unmodified sequence 1816, after modification using template DV22, the resulting sequence C1816-DV22 exhibited an inhibition rate of 66.7% on AGT gene expression, which was 5.8% higher than that of the alternately modified sequence.

After modification using template DV29P disclosed herein, the inhibition rate of C1816-DV29P was increased by 1.8% compared to C1816-DV22.

It can be concluded that:
1) The unmodified sequence 1816 modified using templates DV25P to DV29P disclosed herein showed significantly enhanced inhibition of AGT gene expression as compared to the alternately modified sequences. For example, when the unmodified sequence 1816 was modified using DV29P, the inhibition rate was increased by 7.6% compared to the alternately modified sequence.
2) The same siRNA sequence exhibited significant differences in activity when modified using different modification templates. For example, when the unmodified sequence 1816 was modified using template DV29P disclosed herein, the inhibition rate was increased by up to 30.0% compared to modification using template DV34P disclosed herein, indicating a significant improvement; the inhibition rate was increased by 1.8% compared to modification using the previously disclosed Advanced ESC templates DV22.
3) The siRNA sequences modified using different templates varied greatly in activity, with differences of up to 30.0%. Thus, it is uncertain which modification template would confer high activity to a given siRNA sequence.

### (11) Unmodified sequence 1835

**Table 74: Inhibition rates of AGT gene expression by unmodified sequence 1835 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1835-DV25P | 68.2 | | 5.9 |
| | C1835-DV26P | 71.8 | | 9.5 |
| | C1835-DV27P | 51.2 | | -11.1 |
| | C1835-DV28P | 70.0 | | 7.7 |
| 1835 | C1835-DV29P | 64.9 | 62.3 | 2.6 |
| | C1835-DV32P | 53.3 | | -9.0 |
| | C1835-DV33P | 64.9 | | 2.6 |
| | C1835-DV34P | 71.8 | | 9.5 |
| | C1835-DV22 | 58.2 | | -4.1 |

### I. Templates DV25P to 29P disclosed herein

The unmodified sequence 1835 modified using templates DV25P to DV29P designed in the present disclosure exhibited inhibition rates exceeding 50% on AGT gene expression, except for DV27P, indicating significantly enhanced inhibition of AGT gene expression as compared to alternating 2'-O-methyl and 2'-fluoro modifications. For example, after modification using template DV26P, the inhibition rate of C1835-DV26P was increased by 9.5%.

### II. Other modification templates disclosed herein

For unmodified sequence 1835, after modification using templates DV32P to DV34P, the resulting sequences C1835-DV32P, C1835-DV33P, and C1835-DV34P exhibited inhibition rates of 53.3%, 64.9%, and 71.8% on AGT gene expression, respectively, which were -9.0%, 2.6%, and 9.5% higher than those of the corresponding alternately modified sequences, respectively.

After modification using template DV26P disclosed herein, the inhibition rate of C1835-DV26P was increased by 18.5% compared to C1835-DV32P, increased by 10.7% compared to C1835-DV33P, and increased by 0.0% compared to C1835-DV34P.

### III. Advanced ESC templates DV22 (fluorinated sites: positions 2, 6, 14, and 16 of the antisense strand; positions 7, 9, 10, and 11 of the sense strand) disclosed in the prior art

For unmodified sequence 1835, after modification using template DV22, the resulting sequence C1835-DV22 exhibited an inhibition rate of 58.2% on AGT gene expression, which was 4.1% lower than that of the alternately modified sequence.

After modification using template DV25P disclosed herein, the inhibition rate of C1835-DV26P was increased by 13.6% compared to C1835-DV22, indicating a significant improvement.

It can be concluded that:
1) The unmodified sequence 1835 modified using templates DV25P to DV29P disclosed herein showed significantly enhanced inhibition of AGT gene expression as compared to the alternately modified sequences. For example, when the unmodified sequence 1835 was modified using DV26P, the inhibition rate was increased by 9.5% compared to the alternately modified sequence.
2) The same siRNA sequence exhibited significant differences in activity when modified using different modification templates. For example, when the unmodified sequence 1835 was modified using template DV26P disclosed herein, the inhibition rate was increased by up to 20.6% compared to modification using template DV27P disclosed herein, indicating a significant improvement; the inhibition rate was increased by up to 13.6% compared to modification using the previously disclosed Advanced ESC templates DV22, indicating a significant improvement.
3) The siRNA sequences modified using different templates varied greatly in activity, with differences of up to 20.6%. Thus, it is uncertain which modification template would confer high activity to a given siRNA sequence.

### (12) Unmodified sequence 1836

**Table 75: Inhibition rates of AGT gene expression by unmodified sequence 1836 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1836-DV26P | 63.8 | | 4.0 |
| | C1836-DV27P | 58.9 | | -0.9 |
| 1836 | C1836-DV28P | 63.8 | 59.8 | 4.0 |
| | C1836-DV29P | 56.9 | | -2.9 |
| | C1836-DV32P | 49.2 | | -10.6 |
| | C1836-DV34P | 63.0 | | 3.2 |

### I. Templates DV26P to DV29P disclosed herein

The unmodified sequence 1836 modified using templates DV26P to DV29P designed in the present disclosure exhibited inhibition rates exceeding 55% on AGT gene expression, indicating significantly enhanced inhibition of AGT gene expression as compared to alternating 2'-O-methyl and 2'-fluoro modifications. For example, after modification using templates DV26P and DV28P, the inhibition rates of C1836-DV26P and C1836-DV28P were increased by 4.0%.

### II. Other modification templates disclosed herein

For unmodified sequence 1836, after modification using templates DV32P and DV34P, the resulting sequences C1836-DV32P and C1836-DV34P exhibited inhibition rates of 49.2% and 63.0% on AGT gene expression, respectively, which were 2.9% and 10.6% lower than those of the corresponding alternately modified sequences, respectively.

After modification using templates DV26P and DV28P disclosed herein, the inhibition rates of C1836-DV26P and C1836-DV28P were increased by 14.6% compared to C1836-DV32P, and increased by 0.8% compared to C1836-DV34P.

It can be concluded that:
1) The unmodified sequence 1836 modified using templates DV26P to DV29P disclosed herein showed significantly enhanced inhibition of AGT gene expression as compared to the alternately modified sequences. For example, when the unmodified sequence 1836 was modified using DV26P and DV28P, the inhibition rate was increased by 4.0% compared to the alternately modified sequence.
2) The same siRNA sequence exhibited significant differences in activity when modified using different modification templates. For example, for unmodified sequence 1836, the sequence modified using templates DV26P and DV28P disclosed herein exhibited a 14.6% increase in inhibition rate compared to the sequence modified using template DV32P disclosed herein, indicating a significant improvement.
3) The siRNA sequences modified using different templates varied greatly in activity, with differences of up to 14.6%. Thus, it is uncertain which modification template would confer high activity to a given siRNA sequence.

### (13) Unmodified sequence 1839

**Table 76: Inhibition rates of AGT gene expression by unmodified sequence 1839 modified using different templates**

| **Unmodified sequence ID** | **Template-modified sequence ID** | **Inhibition rate (%) of template-modified sequence** | **Inhibition rate (%) of corr. alternately modified sequence** | **Increase (%)** |
|---|---|---|---|---|
| | C1839-DV26P | 48.6 | | -10.5 |
| | C1839-DV27P | 63.9 | | 4.8 |
| 1839 | C1839-DV28P | 51.4 | 59.1 | -7.7 |
| | C1839-DV29P | 64.2 | | 5.1 |
| | C1839-DV32P | 63.7 | | 4.6 |
| | C1839-DV34P | 55.9 | | -3.2 |

### I. Templates DV26P to DV29P disclosed herein

The unmodified sequence 1839 modified using templates DV26P to DV29P designed in the present disclosure exhibited inhibition rates exceeding 50% on AGT gene expression, indicating significantly enhanced inhibition of AGT gene expression as compared to alternating 2'-O-methyl and 2'-fluoro modifications. For example, after modification using template DV29P, the inhibition rate of C1839-DV29P was increased by 5.1%.

### II. Other modification templates disclosed herein

For unmodified sequence 1839, after modification using templates DV32P and DV34P, the resulting sequences C1839-DV32P and C1839-DV34P exhibited inhibition rates of 63.7% and 55.9% on AGT gene expression, respectively, which were 4.6% and -3.2% higher than those of the corresponding alternately modified sequences, respectively.

After modification using template DV29P disclosed herein, the inhibition rate of C1839-DV29P was increased by 0.5% compared to C1839-DV32P, and increased by 8.3% compared to C1839-DV34P.

It can be concluded that:
1) The unmodified sequence 1839 modified using templates DV26P to DV29P disclosed herein showed significantly enhanced inhibition of AGT gene expression as compared to the alternately modified sequences. For example, when the unmodified sequence 1839 was modified using DV29P, the inhibition rate was increased by 5.1% compared to the alternately modified sequence.
2) The same siRNA sequence exhibited significant differences in activity when modified using different modification templates. For example, for unmodified sequence 1839, the sequence modified using template DV29P disclosed herein exhibited a 15.6% increase in inhibition rate compared to the sequence modified using template DV26P disclosed herein, indicating a significant improvement.
3) The siRNA sequences modified using different templates varied greatly in activity, with differences of up to 15.6%. Thus, it is uncertain which modification template would confer high activity to a given siRNA sequence.

### Summary:

(1) The identified unmodified sequences 1576s, 1578s, 1838, 1835, 1816, 1812, 1579, 1836, 1789, 1839, 1791, 1795, and 1585s, when modified using templates DV25P to DV29P or DV25SP to DV29SP designed in the present disclosure, exhibited significant inhibitory effects on AGT gene expression, with inhibition rates exceeding 40%. Specifically, C1812-DV25P, C1579-DV33P, and C1789-DV26P exhibited inhibition rates of 74.4%, 74.0%, and 73.6%, respectively.
(2) The 12 sequences modified using templates DV25P to DV29P or DV25SP to DV29SP disclosed herein showed significantly enhanced inhibition of AGT gene expression as compared to the alternately modified sequences. For example, for unmodified sequence 1812, the inhibition rate of C1812-DV25P (the sequence modified using template DV25P) was 11.5% higher than that of the alternately modified sequence; for unmodified sequence 1791, the inhibition rate of C1791-DV26P (the sequence modified using template DV26P) was 8.5% higher than that of the alternately modified sequence.
(3) The same sequence modified using templates DV25P to DV29P or DV25SP to DV29SP disclosed herein showed significantly enhanced inhibition of AGT gene expression as compared to modification using the templates disclosed in the prior art. For example, for unmodified sequence 1579, the sequence modified using template DV25P disclosed herein exhibited a 16.6% increase in inhibition rate compared to the sequence modified using the previously disclosed Advanced ESC template DV22.
(4) The same sequence modified using templates DV25P to DV29P or DV25SP to DV29SP disclosed herein showed significantly enhanced inhibition of AGT gene expression as compared to modification using templates DV32P to DV34P disclosed herein. For example, for unmodified sequence 1812, the sequence modified using template DV25P disclosed herein exhibited a 20.2% increase in inhibition rate compared to the sequence modified using template DV33P disclosed herein.
(5) The sequences modified using different templates varied greatly in activity. For example, when the unmodified sequence 1816 was modified using DV29P, the inhibition rate was increased by 30.0% compared to modification using DV34; when the unmodified sequence 1579 was modified using DV33P, the inhibition rate was increased by 29.8% compared to modification using DV28P. Thus, it is uncertain which modification template would confer high activity to a given siRNA sequence.

### (ii) IC₅₀ assay

A total of 24 template-modified sequences, including C1816-DV29P, C1816-DV25P, C1812-DV25P, C1579-DV32P, C1789-DV26P, C1789-DV25P, C1839-DV29P, C1795-DV27P, C1585s-DV27SP, C1835-DV26P, C1835-DV25P, C1579-DV25P, C1836-DV26P, C1791-DV26P, C1585s-DV29SP, C1604-DV29P, C1606-DV29P, C1607-DV29P, C1835-DV29P, C1838-DV29P, C1579-DV29P, C1576s-DV29SP, C1578s-DV29SP, and C1579s-DV29SP, were selected for IC₅₀ determination. The IC₅₀ values of these sequences were determined. The experimental results are shown in Table 77.

**Table 77: IC₅₀ values of modified sequences**

| Sequence ID | IC₅₀ (pM) | Sequence ID | IC₅₀ (pM) |
|---|---|---|---|
| C1816-DV29P | 27.925 | C1836-DV26P | 12.838 |
| C1816-DV25P | 3.530 | C1791-DV26P | 7.812 |
| C1812-DV25P | 2.160 | C1585s-DV29SP | 13.333 |
| C1579-DV32P | 4.712 | C1604-DV29P | 23.160 |
| C1789-DV26P | 2.643 | C1606-DV29P | 46.237 |
| C1789-DV25P | 1.884 | C1607-DV29P | 61.498 |
| C1839-DV29P | 3.301 | C1835-DV29P | 53.143 |
| C1795-DV27P | 3.570 | C1838-DV29P | 15.393 |
| C1585s-DV27SP | 2.863 | C1579-DV29P | 15.837 |
| C1835-DV26P | 14.565 | C1576s-DV29SP | 11.214 |
| C1835-DV25P | 14.683 | C1578s-DV29SP | 8.568 |
| C1579-DV25P | 10.532 | C1579s-DV29SP | 30.601 |

As shown in Table 77, these 24 sequences exhibited IC₅₀ values ranging from 1.884 pM to 61.498 pM. Specifically, the IC₅₀ values for C1789-DV25P, C1812-DV25P, and C1789-DV26P were 1.884 pM, 2.160 pM, and 2.643 pM, respectively. The results demonstrated that these sequences effectively inhibited AGT gene expression at low concentrations.

### Example 21: Comparison of inhibitory effects of sequences disclosed in the prior art on AGT gene expression

In this example, the unmodified sequences 1579, 1789, 1812, 1576s, 1578s, 1585s, 1816, 1835, 1836, 1838, 1839, and 1791 disclosed herein were compared with similar unmodified sequences disclosed in the prior art, the alternately modified sequences disclosed herein, and the sequences modified using templates DV25P to DV29P or DV25SP to DV29SP, in terms of their inhibitory effects on AGT gene expression.

### 1. Experimental materials

### Test samples:

(1) Sequences disclosed in the prior art, as shown in Table 78.

**Table 78: Sequences disclosed in the prior art**

| Sequence ID | Sense strand | **SEQ ID NO:** | Antisense strand | **SEQ ID NO:** | Source |
|---|---|---|---|---|---|
| 579P | ACCUUUUCUUCUAAU GAGUCU | 1284 | AGACUCAUUAGAAGAAA AGGUGG | 1297 | CN117751189A |
| 789P | UUUGUGAAACAAAA AAGUGA | 1285 | UCACUUUUUGUUCAC AAACA | 1298 | WO2024031101A1 |
| 812P | UUGUUCUCAACUUGA AAAGGGA | 1286 | CCUUUUCAAGUUGAGAA CAA | 1299 | WO2024031101A1 |
| 576P | CCACCUUUUCUUCUA AUGAGU | 1287 | ACUCAUUAGAAGAAAAG GUGGGA | 1300 | CN112313335A |
| 578P | CACCUUUUCUUCUAA UGAGUA | 1288 | UACUCAUUAGAAGAAAA GGUGGG | 1301 | CN112313335A |
| 585P | UUCUAAUGAGUCGAC UUUA | 1289 | UAAAGUCGACUCAUUAG AA | 1302 | CN112852809A |
| AD-60771 | AGAACAAAAAUUGG GUUUUAA | 1290 | UUAAAACCCAAUUUUG UUCUCA | 1303 | CN112852809A |
| AD-85481 | GUCAUCCACAAUGAG AGUACA | 1291 | UGUACUCUCAUUGUGGA UGACGA | 1304 | CN112852809A |
| 816P | UCAAGUUGAGAACAA AAAUA | 1292 | UAUUUUUGUUCUCAACU UGAAA | 1305 | WO2024031101A1 |
| 835P | GGGUUUUAAAUUA AAGUAUA | 1293 | UACUUUAAUUUAAAAC CCAA | 1306 | CN113862268A |
| 836P | GGUUUUAAAAUUAA AGUAUAC | 1294 | AUACUUUAAUUUUAAAA CCCA | 1307 | CN113862268A |
| 839P | GUUUUAAAAUUAAA GUAUACA | 1295 | UAUACUUUAAUUUUAAA ACCC | 1308 | CN113862268A |
| 791P | UUGUGAAACAAAAA AGUGUUU | 1296 | ACACUUUUUUGUUUCAC AAUU | 1309 | CN106574268B |

(2) siRNA sequences with alternating 2'-OMe and 2'-F modifications and terminal phosphorothioate modifications in Example 18.
(3) siRNA sequences modified using templates DV25P to DV29P or DV25SP to DV29SP (see Table 79 for specific sequences).

**Table 79: Sequences modified using templates DV25P to DV29P**

| Unmodified seq ID | Template-modified seq ID | Sense sequence 5'-3' | Modified SEQ ID NO:/corr. unmodified SEQ ID NO: | Antisense sequence 5'-3' | Modified SEQ ID NO:/corr. unmodified SEQ ID NO: |
|---|---|---|---|---|---|
| 1579 | C1579-DV25P | Cms-Cms-Um-Um-Um-Um-Cf- Um-Uf-Cf-Uf-Am-Am-Um- Gm-Am-Gf-Um-Cm-Gm-Am | 1191/788 | UmsEVP-Cfs-Gm-Am-Cm-Uf- Cm-Am-Um-Um-Am-Gm-Am- Af-Gm-Af-Am-Am-Am-Gm- Gms-Ums-Gm | 1241/801 |
| 1579 | C1579-DV26P | Cms-Cms-Um-Um-Um-Um-Cf- Um-Uf-Cf-Uf-Am-Am-Um- Gm-Am-Gf-Um-Cm-Gm-Am | 1191/788 | UmsEVP-Cfs-Gf-Af-Cm-Uf- Cm-Am-Um-Um-Am-Gm-Am- Af-Gm-Af-Am-Am-Am-Gm- Gms-Ums-Gm | 1238/801 |
| 1579 | C1579-DV27P | Cms-Cms-Um-Um-Um-Um-Cf- Um-Uf-Cf-Uf-Am-Am-Um- Gm-Am-Gf-Um-Cm-Gm-Am | 1191/788 | UmsEVP-Cfs-Gm-Af-Cf-Uf- Cm-Am-Um-Um-Am-Gm-Am- Af-Gm-Af-Am-Am-Am-Gm- Gms-Ums-Gm | 1237/801 |
| 1579 | C1579-DV28P | Cms-Cms-Um-Um-Um-Um-Cf- Um-Uf-Cm-Uf-Am-Am-Um- Gm-Am-Gf-Um-Cm-Gm-Am | 1190/788 | UmsEVP-Cfs-Gf-Af-Cm-Uf- Cm-Am-Um-Um-Am-Gm-Am- Af-Gm-Af-Am-Am-Am-Gm- Gms-Ums-Gm | 1238/801 |
| 1579 | C1579-DV29P | Cms-Cms-Um-Um-Um-Um-Cf- Um-Uf-Cm-Uf-Am-Am-Um- Gm-Am-Gf-Um-Cm-Gm-Am | 1190/788 | UmsEVP-Cfs-Gm-Af-Cf-Uf- Cm-Am-Um-Um-Am-Gm-Am- Af-Gm-Af-Am-Am-Am-Gm- Gms-Ums-Gm | 1237/801 |
| 1789 | C1789-DV25P | Gms-Ums-Um-Um-Gm-Um-Gf- Am-Af-Af-Cf-Am-Am-Am- Am-Am-Af-Gm-Um-Gm-Um | 1196/790 | AmsEVP-Cfs-Am-Cm-Um-Uf- Um-Um-Um-Um-Gm-Um-Um- Uf-Cm-Af-Cm-Am-Am-Am- Cms-Ams-Am | 1252/803 |
| 1789 | C1789-DV26P | Gms-Ums-Um-Um-Gm-Um-Gf- Am-Af-Af-Cf-Am-Am-Am- Am-Am-Af-Gm-Um-Gm-Um | 1196/789 | AmsEVP-Cfs-Af-Cf-Um-Uf- Um-Um-Um-Um-Gm-Um-Um- Uf-Cm-Af-Cm-Am-Am-Am- Cms-Ams-Am | 1249/802 |
| 1789 | C1789-DV27P | Gms-Ums-Um-Um-Gm-Um-Gf- Am-Af-Af-Cf-Am-Am-Am- Am-Am-Af-Gm-Um-Gm-Um | 1196/789 | AmsEVP-Cfs-Am-Cf-Uf-Uf- Um-Um-Um-Um-Gm-Um-Um- Uf-Cm-Af-Cm-Am-Am-Am- Cms-Ams-Am | 1248/802 |
| 1789 | C1789-DV28P | Gms-Ums-Um-Um-Gm-Um-Gf- Am-Af-Am-Cf-Am-Am-Am- Am-Am-Af-Gm-Um-Gm-Um | 1195/790 | AmsEVP-Cfs-Af-Cf-Um-Uf- Um-Um-Um-Um-Gm-Um-Um- Uf-Cm-Af-Cm-Am-Am-Am- Cms-Ams-Am | 1249/803 |
| 1789 | C1789-DV29P | Gms-Ums-Um-Um-Gm-Um-Gf- Am-Af-Am-Cf-Am-Am-Am- Am-Am-Af-Gm-Um-Gm-Um | 1195/789 | AmsEVP-Cfs-Am-Cf-Uf-Uf- Um-Um-Um-Um-Gm-Um-Um- Uf-Cm-Af-Cm-Am-Am-Am- Cms-Ams-Am | 1248/802 |
| 1812 | C1812-DV25P | Cms-Cms-Um-Um-Um-Um-Cf- Am-Af-Gf-Uf-Um-Gm-Am- Gm-Am-Af-Cm-Am-Am-Am | 1188/787 | UmsEVP-Ufs-Um-Gm-Um-Uf- Cm-Um-Cm-Am-Am-Cm-Um- Uf-Gm-Af-Am-Am-Am-Gm- Gms-Gms-Am | 1234/800 |
| 1812 | C1812-DV26P | Cms-Cms-Um-Um-Um-Um-Cf- Am-Af-Gf-Uf-Um-Gm-Am- Gm-Am-Af-Cm-Am-Am-Am | 1188/787 | UmsEVP-Ufs-Uf-Gf-Um-Uf- Cm-Um-Cm-Am-Am-Cm-Um- Uf-Gm-Af-Am-Am-Am-Gm- Gms-Gms-Am | 1231/800 |
| 1812 | C1812-DV27P | Cms-Cms-Um-Um-Um-Um-Cf- Am-Af-Gf-Uf-Um-Gm-Am- Gm-Am-Af-Cm-Am-Am-Am | 1188/787 | UmsEVP-Ufs-Um-Gf-Uf-Uf- Cm-Um-Cm-Am-Am-Cm-Um- Uf-Gm-Af-Am-Am-Am-Gm- Gms-Gms-Am | 1230/800 |
| 1812 | C1812-DV28P | Cms-Cms-Um-Um-Um-Um-Cf- Am-Af-Gm-Uf-Um-Gm-Am- Gm-Am-Af-Cm-Am-Am-Am | 1187/787 | UmsEVP-Ufs-Uf-Gf-Um-Uf- Cm-Um-Cm-Am-Am-Cm-Um- Uf-Gm-Af-Am-Am-Am-Gm- Gms-Gms-Am | 1231/800 |
| 1812 | C1812-DV29P | Cms-Cms-Um-Um-Um-Um-Cf- Am-Af-Gm-Uf-Um-Gm-Am- Gm-Am-Af-Cm-Am-Am-Am | 1187/787 | UmsEVP-Ufs-Um-Gf-Uf-Uf- Cm-Um-Cm-Am-Am-Cm-Um- Uf-Gm-Af-Am-Am-Am-Gm- Gms-Gms-Am | 1230/800 |
| 1576s | C1576s-DV29SP | Cms-Cms-Am-Cm-Cm-Uf-Um- Uf-Um-Cf-Um-Um-Cm-Um- Am-Af-Um-Gm-Am-Am | 1207/795 | UmsEVP-Ufs-Cm-Af-Uf-Uf- Am-Gm-Am-Am-Gm-Am-Am- Af-Am-Gf-Gm-Um-Gm-Gms- Gms-Am | 1272/808 |
| 1576s | C1576s-DV28SP | Cms-Cms-Am-Cm-Cm-Uf-Um- Uf-Um-Cf-Um-Um-Cm-Um- Am-Af-Um-Gm-Am-Am | 1207/795 | UmsEVP-Ufs-Cf-Af-Um-Uf- Am-Gm-Am-Am-Gm-Am-Am- Af-Am-Gf-Gm-Um-Gm-Gms- Gms-Am | 1273/808 |
| 1576s | C1576s-DV26SP | Cms-Cms-Am-Cm-Cm-Uf-Um- Uf-Uf-Cf-Um-Um-Cm-Um- Am-Af-Um-Gm-Am-Am | 1208/795 | UmsEVP-Ufs-Cf-Af-Um-Uf- Am-Gm-Am-Am-Gm-Am-Am- Af-Am-Gf-Gm-Um-Gm-Gms- Gms-Am | 1273/808 |
| 1578s | C1578s-DV29SP | Ams-Cms-Cm-Um-Um-Uf-Um- Cf-Um-Uf-Cm-Um-Am-Am- Um-Gf-Am-Gm-Um-Am | 1210/796 | UmsEVP-Afs-Cm-Uf-Cf-Af- Um-Um-Am-Gm-Am-Am-Gm- Af-Am-Af-Am-Gm-Gm-Ums- Gms-Gm | 1276/809 |
| 1578s | C1578s-DV28SP | Ams-Cms-Cm-Um-Um-Uf-Um- Cf-Um-Uf-Cm-Um-Am-Am- Um-Gf-Am-Gm-Um-Am | 1210/796 | UmsEVP-Afs-Cf-Uf-Cm-Af- Um-Um-Am-Gm-Am-Am-Gm- Af-Am-Af-Am-Gm-Gm-Ums- Gms-Gm | 1277/809 |
| 1578s | C1578s-DV26SP | Ams-Cms-Cm-Um-Um-Uf-Um- Cf-Uf-Uf-Cm-Um-Am-Am- Um-Gf-Am-Gm-Um-Am | 1211/796 | UmsEVP-Afs-Cf-Uf-Cm-Af- Um-Um-Am-Gm-Am-Am-Gm- Af-Am-Af-Am-Gm-Gm-Ums- Gms-Gm | 1277/809 |
| 1585s | C1585s-DV26SP | Cms-Ums-Um-Cm-Um-Af-Am- Uf-Gf-Af-Gm-Um-Cm-Gm- Am-Cf-Um-Um-Um-Am | 1205/794 | UmsEVP-Afs-Af-Af-Gm-Uf- Cm-Gm-Am-Cm-Um-Cm-Am- Uf-Um-Af-Gm-Am-Am-Gms- Ams-Am | 1268/807 |
| 1585s | C1585s-DV27SP | Cms-Ums-Um-Cm-Um-Af-Am- Uf-Gf-Af-Gm-Um-Cm-Gm- Am-Cf-Um-Um-Um-Am | 1205/794 | UmsEVP-Afs-Am-Af-Gf-Uf- Cm-Gm-Am-Cm-Um-Cm-Am- Uf-Um-Af-Gm-Am-Am-Gms- Ams-Am | 1267/807 |
| 1585s | C1585s-DV28SP | Cms-Ums-Um-Cm-Um-Af-Am- Uf-Gm-Af-Gm-Um-Cm-Gm- Am-Cf-Um-Um-Um-Am | 1204/794 | UmsEVP-Afs-Af-Af-Gm-Uf- Cm-Gm-Am-Cm-Um-Cm-Am- Uf-Um-Af-Gm-Am-Am-Gms- Ams-Am | 1268/807 |
| 1585s | C1585s-DV29SP | Cms-Ums-Um-Cm-Um-Af-Am- Uf-Gm-Af-Gm-Um-Cm-Gm- Am-Cf-Um-Um-Um-Am | 1204/794 | UmsEVP-Afs-Am-Af-Gf-Uf- Cm-Gm-Am-Cm-Um-Cm-Am- Uf-Um-Af-Gm-Am-Am-Gms- Ams-Am | 1267/807 |
| 1789 | C1789-DV26P | Gms-Ums-Um-Um-Gm-Um-Gf- Am-Af-Af-Cf-Am-Am-Am- Am-Am-Af-Gm-Um-Gm-Um | 1196/789 | AmsEVP-Cfs-Af-Cf-Um-Uf- Um-Um-Um-Um-Gm-Um-Um- Uf-Cm-Af-Cm-Am-Am-Am- Cms-Ams-Am | 1249/802 |
| 1789 | C1789-DV27P | Gms-Ums-Um-Um-Gm-Um-Gf- Am-Af-Af-Cf-Am-Am-Am- Am-Am-Af-Gm-Um-Gm-Um | 1196/789 | AmsEVP-Cfs-Am-Cf-Uf-Uf- Um-Um-Um-Um-Gm-Um-Um- Uf-Cm-Af-Cm-Am-Am-Am- Cms-Ams-Am | 1248/802 |
| 1816 | C1816-DV25P | Ums-Ums-Cm-Am-Am-Gm-Uf- Um-Gf-Af-Gf-Am-Am-Cm- Am-Am-Af-Am-Am-Um-Um | 1185/786 | AmsEVP-Afs-Um-Um-Um-Uf- Um-Gm-Um-Um-Cm-Um-Cm- Af-Am-Cf-Um-Um-Gm-Am- Ams-Ams-Am | 1227/799 |
| 1816 | C1816-DV26P | Ums-Ums-Cm-Am-Am-Gm-Uf- Um-Gf-Af-Gf-Am-Am-Cm- Am-Am-Af-Am-Am-Um-Um | 1185/786 | AmsEVP-Afs-Uf-Uf-Um-Uf- Um-Gm-Um-Um-Cm-Um-Cm- Af-Am-Cf-Um-Um-Gm-Am- Ams-Ams-Am | 1224/799 |
| 1816 | C1816-DV27P | Ums-Ums-Cm-Am-Am-Gm-Uf- Um-Gf-Af-Gf-Am-Am-Cm- Am-Am-Af-Am-Am-Um-Um | 1185/786 | AmsEVP-Afs-Um-Uf-Uf-Uf- Um-Gm-Um-Um-Cm-Um-Cm- Af-Am-Cf-Um-Um-Gm-Am- Ams-Ams-Am | 1223/799 |
| 1816 | C1816-DV28P | Ums-Ums-Cm-Am-Am-Gm-Uf- Um-Gf-Am-Gf-Am-Am-Cm- Am-Am-Af-Am-Am-Um-Um | 1184/786 | AmsEVP-Afs-Uf-Uf-Um-Uf- Um-Gm-Um-Um-Cm-Um-Cm- Af-Am-Cf-Um-Um-Gm-Am- Ams-Ams-Am | 1224/799 |
| 1816 | C1816-DV29P | Ums-Ums-Cm-Am-Am-Gm-Uf- Um-Gf-Am-Gf-Am-Am-Cm- Am-Am-Af-Am-Am-Um-Um | 1184/786 | AmsEVP-Afs-Um-Uf-Uf-Uf- Um-Gm-Um-Um-Cm-Um-Cm- Af-Am-Cf-Um-Um-Gm-Am- Ams-Ams-Am | 1223/799 |
| 1835 | C1835-DV25P | Ums-Ums-Gm-Gm-Gm-Um-Uf- Um-Uf-Af-Af-Am-Am-Um- Um-Am-Af-Am-Gm-Um-Am | 1182/785 | UmsEVP-Afs-Cm-Um-Um-Uf- Am-Am-Um-Um-Um-Um-Am- Af-Am-Af-Cm-Cm-Cm-Am- Ams-Ums-Um | 1220/798 |
| 1835 | C1835-DV26P | Ums-Ums-Gm-Gm-Gm-Um-Uf- Um-Uf-Af-Af-Am-Am-Um- Um-Am-Af-Am-Gm-Um-Am | 1182/785 | UmsEVP-Afs-Cf-Uf-Um-Uf- Am-Am-Um-Um-Um-Um-Am- Af-Am-Af-Cm-Cm-Cm-Am- Ams-Ums-Um | 1217/798 |
| 1835 | C1835-DV27P | Ums-Ums-Gm-Gm-Gm-Um-Uf- Um-Uf-Af-Af-Am-Am-Um- Um-Am-Af-Am-Gm-Um-Am | 1182/785 | UmsEVP-Afs-Cm-Uf-Uf-Uf- Am-Am-Um-Um-Um-Um-Am- Af-Am-Af-Cm-Cm-Cm-Am- Ams-Ums-Um | 1216/798 |
| 1835 | C1835-DV28P | Ums-Ums-Gm-Gm-Gm-Um-Uf- Um-Uf-Am-Af-Am-Am-Um- Um-Am-Af-Am-Gm-Um-Am | 1181/785 | UmsEVP-Afs-Cf-Uf-Um-Uf- Am-Am-Um-Um-Um-Um-Am- Af-Am-Af-Cm-Cm-Cm-Am- Ams-Ums-Um | 1217/798 |
| 1835 | C1835-DV29P | Ums-Ums-Gm-Gm-Gm-Um-Uf- Um-Uf-Am-Af-Am-Am-Um- Um-Am-Af-Am-Gm-Um-Am | 1181/785 | UmsEVP-Afs-Cm-Uf-Uf-Uf- Am-Am-Um-Um-Um-Um-Am- Af-Am-Af-Cm-Cm-Cm-Am- Ams-Ums-Um | 1216/798 |
| 1838 | C1838-DV29P | Gms-Gms-Um-Um-Um-Um-Af- Am-Af-Am-Uf-Um-Am-Am- Am-Gm-Uf-Am-Um-Am-Am | 1213/797 | UmsEVP-Ufs-Am-Uf-Af-Cf- Um-Um-Um-Am-Am-Um-Um- Uf-Um-Af-Am-Am-Am-Cm- Cms-Cms-Am | 1280/810 |
| 1838 | C1838-DV28P | Gms-Gms-Um-Um-Um-Um-Af- Am-Af-Am-Uf-Um-Am-Am- Am-Gm-Uf-Am-Um-Am-Am | 1213/797 | UmsEVP-Ufs-Af-Uf-Am-Cf- Um-Um-Um-Am-Am-Um-Um- Uf-Um-Af-Am-Am-Am-Cm- Cms-Cms-Am | 1281/810 |
| 1838 | C1838-DV26P | Gms-Gms-Um-Um-Um-Um-Af- Am-Af-Af-Uf-Um-Am-Am- Am-Gm-Uf-Am-Um-Am-Am | 1214/797 | UmsEVP-Ufs-Af-Uf-Am-Cf- Um-Um-Um-Am-Am-Um-Um- Uf-Um-Af-Am-Am-Am-Cm- Cms-Cms-Am | 1281/810 |
| 1836 | C1836-DV26P | Ums-Gms-Gm-Gm-Um-Um-Uf- Um-Af-Af-Af-Am-Um-Um- Am-Am-Af-Gm-Um-Am-Um | 1194/789 | AmsEVP-Ufs-Af-Cf-Um-Uf- Um-Am-Am-Um-Um-Um-Um- Af-Am-Af-Am-Cm-Cm-Cm- Ams-Ams-Um | 1245/802 |
| 1836 | C1836-DV27P | Ums-Gms-Gm-Gm-Um-Um-Uf- Um-Af-Af-Af-Am-Um-Um- Am-Am-Af-Gm-Um-Am-Um | 1194/789 | AmsEVP-Ufs-Am-Cf-Uf-Uf- Um-Am-Am-Um-Um-Um-Um- Af-Am-Af-Am-Cm-Cm-Cm- Ams-Ams-Um | 1244/802 |
| 1836 | C1836-DV28P | Ums-Gms-Gm-Gm-Um-Um-Uf- Um-Af-Am-Af-Am-Um-Um- Am-Am-Af-Gm-Um-Am-Um | 1193/789 | AmsEVP-Ufs-Af-Cf-Um-Uf- Um-Am-Am-Um-Um-Um-Um- Af-Am-Af-Am-Cm-Cm-Cm- Ams-Ams-Um | 1245/802 |
| 1836 | C1836-DV29P | Ums-Gms-Gm-Gm-Um-Um-Uf- Um-Af-Am-Af-Am-Um-Um- Am-Am-Af-Gm-Um-Am-Um | 1193/789 | AmsEVP-Ufs-Am-Cf-Uf-Uf- Um-Am-Am-Um-Um-Um-Um- Af-Am-Af-Am-Cm-Cm-Cm- Ams-Ams-Um | 1244/802 |
| 1839 | C1839-DV26P | Gms-Ums-Um-Um-Um-Am-Af- Am-Af-Uf-Uf-Am-Am-Am- Gm-Um-Af-Um-Am-Cm-Am | 1199/791 | UmsEVP-Gfs-Uf-Af-Um-Af- Cm-Um-Um-Um-Am-Am-Um- Uf-Um-Uf-Am-Am-Am-Am- Cms-Cms-Cm | 1256/804 |
| 1839 | C1839-DV27P | Gms-Ums-Um-Um-Um-Am-Af- Am-Af-Uf-Uf-Am-Am-Am- Gm-Um-Af-Um-Am-Cm-Am | 1199/791 | UmsEVP-Gfs-Um-Af-Uf-Af- Cm-Um-Um-Um-Am-Am-Um- Uf-Um-Uf-Am-Am-Am-Am- Cms-Cms-Cm | 1255/804 |
| 1839 | C1839-DV28P | Gms-Ums-Um-Um-Um-Am-Af- Am-Af-Um-Uf-Am-Am-Am- Gm-Um-Af-Um-Am-Cm-Am | 1198/791 | UmsEVP-Gfs-Uf-Af-Um-Af- Cm-Um-Um-Um-Am-Am-Um- Uf-Um-Uf-Am-Am-Am-Am- Cms-Cms-Cm | 1256/804 |
| 1839 | C1839-DV29P | Gms-Ums-Um-Um-Um-Am-Af- Am-Af-Um-Uf-Am-Am-Am- Gm-Um-Af-Um-Am-Cm-Am | 1198/791 | UmsEVP-Gfs-Um-Af-Uf-Af- Cm-Um-Um-Um-Am-Am-Um- Uf-Um-Uf-Am-Am-Am-Am- Cms-Cms-Cm | 1255/804 |
| 1791 | C1791-DV26P | Ums-Ums-Gm-Um-Gm-Am-Af- Am-Cf-Af-Af-Am-Am-Am- Am-Gm-Uf-Gm-Um-Um-Am | 1201/792 | UmsEVP-Afs-Af-Cf-Am-Cf- Um-Um-Um-Um-Um-Um-Gm- Uf-Um-Uf-Cm-Am-Cm-Am- Ams-Ams-Cm | 1260/805 |
| 1791 | C1791-DV27P | Ums-Ums-Gm-Um-Gm-Am-Af- Am-Cf-Af-Af-Am-Am-Am- Am-Gm-Uf-Gm-Um-Um-Am | 1201/792 | UmsEVP-Afs-Am-Cf-Af-Cf- Um-Um-Um-Um-Um-Um-Gm- Uf-Um-Uf-Cm-Am-Cm-Am- Ams-Ams-Cm | 1259/805 |
| 1791 | C1791-DV28P | Ums-Ums-Gm-Um-Gm-Am-Af- Am-Cf-Am-Af-Am-Am-Am- Am-Gm-Uf-Gm-Um-Um-Am | 1200/792 | UmsEVP-Afs-Af-Cf-Am-Cf- Um-Um-Um-Um-Um-Um-Gm- Uf-Um-Uf-Cm-Am-Cm-Am- Ams-Ams-Cm | 1260/805 |
| 1791 | C1791-DV29P | Ums-Ums-Gm-Um-Gm-Am-Af- Am-Cf-Am-Af-Am-Am-Am- Am-Gm-Uf-Gm-Um-Um-Am | 1200/792 | UmsEVP-Afs-Am-Cf-Af-Cf- Um-Um-Um-Um-Um-Um-Gm- Uf-Um-Uf-Cm-Am-Cm-Am- Ams-Ams-Cm | 1259/805 |

Cell line: HepG2 cells, provided by Cyagen Biosciences (H1-1701).

HepG2 cells were cultured in DMEM culture medium (ATCC-30-2003) containing 10% fetal bovine serum (FBS, ExCell Bio-FSP500) and 1% penicillin-streptomycin (HyClone-SV30010).

Solvents: sterile nuclease-free water, Gibco Opti-MEM.

### 2. Experimental methods

The inhibitory effects of the test samples on AGT mRNA expression in HepG2 cells were evaluated by qRT-PCR.

### 2.1 Cell culture

Subcultured HepG2 cells in the logarithmic growth phase were cultured in DMEM culture medium containing 10% fetal bovine serum (supplemented with penicillin and streptomycin at 100 µL/mL), and incubated in a cell incubator at 37°C with 5% CO₂. The medium was exchanged once daily. The cells were digested with 0.25% trypsin, followed by centrifugation at 1000 rpm for 5 minutes. The supernatant was discarded, and fresh medium was added for continued culture.

### 2.2 Cell transfection

Preparation of transfection mixture: Lipofectamine RNAiMAX was mixed with Opti-MEM at a volume ratio of 2:98 and vortexed thoroughly.

Preparation of transfection reagent: 60 µL of siRNA solution (diluted in Opti-MEM) was mixed with 60 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 60 µL of transfection mixture was added to 60 µL of Opti-MEM, vortexed thoroughly, followed by incubation at room temperature for 15 minutes.

The prepared transfection reagent was added to a 24-well cell culture plate (100 µL per well), resulting in a final siRNA concentration of 0.02 nM per well. Subsequently, 500 µL of cell suspension (1.5 × 10⁵ cells per mL) was added to each well. After cross-mixing, the plate was incubated in a cell incubator at 37°C with 5% CO₂ for 40 hours.

### 2.3 AGT mRNA analysis

1) RNA extraction
   The procedure was the same as "1) RNA extraction" described in "2.3 AGT mRNA analysis" of Example 18.
2) Measurement of RNA concentration
   The procedure was the same as "2) Measurement of RNA concentration" described in "2.3 AGT mRNA analysis" of Example 18.
3) Quantitative analysis of AGT mRNA

In a 15 mL centrifuge tube, all components except primers and templates were added in 7.5 × 84 = 630 aliquots (labeled as A).

1.5 mL EP tubes were labeled. 77 µL of A and 420 ng of total RNA were added in 7 aliquots per tube, and mixed well for later use.

The forward and reverse primers for both the internal reference gene GAPDH and the target gene AGT were mixed well for later use.

11 µL of B and 1 µL of C were added to each well of a PCR plate, which was sealed, then centrifuged at 3000 rpm for 1 minute, and subjected to instrumental analysis.

*The step was performed on ice under low-temperature conditions.

The plate was placed into the qPCR instrument and run according to the following program:
Reverse transcription: 55°C for 15 minutes;
Pre-denaturation: 95°C for 30 seconds;
Cycling: 95°C for 10 seconds; 60°C for 35 seconds; 40 cycles;
Melting curve: 95°C for 15 seconds; 60°C for 60 seconds; 95°C for 15 seconds.

Total run time: approximately 2 hours. After analysis of the experimental results, 2^{-ΔΔCt} was calculated.

### 2.4 Data processing

AGT mRNA expression rate (%) was calculated using the following formula: 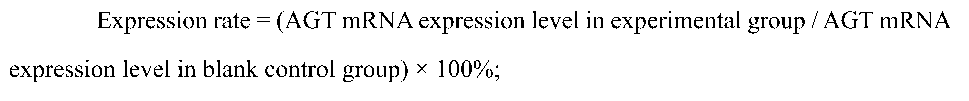
Expression rate = (AGT mRNA expression level in experimental group / AGT mRNA expression level in blank control group) × 100%;

Inhibition rate of AGT gene expression = 100% - expression rate (%).

### 3. Experimental results

The detailed experimental results are shown in Table 80.

The experimental results demonstrated that both the alternately modified sequences and the sequences modified using specific templates disclosed herein exhibited significantly enhanced AGT inhibitory activity compared to similar unmodified sequences disclosed in the prior art. For example, compared to the sequence 1812 disclosed herein, the sequence 812P disclosed in the prior art differs in that the sense strand lacks one base A at the 3' end; the antisense strand lacks one base U at the 5' end, with all other bases being identical. However, the unmodified sequence 1812 disclosed herein exhibited a 14.3% increase in inhibition rate compared to 812P. The alternately modified sequence exhibited an inhibition rate of 62.9%, which was 20.4% higher than that of 812P. After modification using template DV25P disclosed herein, the inhibition rate was increased to 74.4%, which was 31.9% higher than that of 31P.
**(1) Compared to the relatively similar unmodified sequences disclosed in the prior art, the unmodified sequences, alternately modified sequences, and template-modified sequences disclosed herein showed significantly enhanced inhibition of AGT gene expression, with an increase of up to 91.0%.**

Compared to the relatively similar sequences disclosed in the prior art, the unmodified sequences, alternately modified sequences, and template-modified sequences disclosed herein showed significantly enhanced inhibition of AGT gene expression. For example, the unmodified sequence 812P exhibited an inhibition rate of 42.5%. The similar unmodified sequence 1812 disclosed herein exhibited an inhibition rate of 56.8%, which was 14.3% higher than that of 812P. The alternately modified sequence exhibited an inhibition rate of 62.9%, which was 20.4% higher than that of 812P. After modification using template DV25 disclosed herein, the inhibition rate was increased to 74.4%, which was 31.9% higher than that of 812P.

**Table 80: Inhibition rates of AGT gene expression by similar sequences disclosed in the prior art and disclosed herein**

| **Sequences disclosed in the prior art** | | **Unmodified sequences disclosed herein** | | | **Alternately modified disclosed herein** | | | **Template-modified sequences disclosed herein** | | |
|---|---|---|---|---|---|---|---|---|---|---|
| **Name** | **Inh (%)** | **Seq ID** | **Inh (%)** | **Increase vs sequences disclosed in the prior art (%)** | **Seq ID** | **Inh (%)** | **Increase vs sequences disclosed in the prior art (%)** | **Seq ID** | **Inh (%)** | **Increase vs sequences disclosed in the prior art (%)** |
| 579P | 47 | 1579 | 58.2 | 11.2 | B1579-AL | 61.1 | 14.1 | C1579-DV25P | 55.6 | 8.6 |
| | | | | | | | | C1579-DV26P | 72.2 | 25.2 |
| | | | | | | | | C1579-DV27P | 70.8 | 23.8 |
| | | | | | | | | C1579-DV28P | 63.3 | 16.3 |
| | | | | | | | | C1579-DV29P | 44.2 | -2.8 |
| 789P | 51.9 | 1789 | 54.5 | 2.6 | B1789-AL | 63.5 | 11.6 | C1789-DV25P | 69 | 17.1 |
| | | | | | | | | C1789-DV26P | 73.6 | 21.7 |
| | | | | | | | | C1789-DV27P | 66.3 | 14.4 |
| | | | | | | | | C1789-DV28P | 63.1 | 11.2 |
| | | | | | | | | C1789-DV29P | 64.5 | 12.6 |
| 812P | 42.5 | 1812 | 56.8 | 14.3 | B1812-AL | 62.9 | 20.4 | C1812-DV25P | 74.4 | 31.9 |
| | | | | | | | | C1812-DV26P | 58 | 15.5 |
| | | | | | | | | C1812-DV27P | 63.9 | 21.4 |
| | | | | | | | | C1812-DV28P | 55.3 | 12.8 |
| | | | | | | | | C1812-DV29P | 66.9 | 24.4 |
| 576P | 38.2 | 1576s | 59.1 | 20.9 | B1576sAL | 64.6 | 26.4 | C1576s-DV26SP | 73.4 | 35.2 |
| | | | | | | | | C1576s-DV28SP | 58.8 | 20.6 |
| | | | | | | | | C1576s-DV29SP | 79.3 | 41.1 |
| | | | | | | | | C1576s-DV25SP | 73.3 | 35.1 |
| | | | | | | | | C1576s-DV27SP | 69.7 | 31.5 |
| 578P | 33.6 | 1578s | 55.2 | 21.6 | B1578sAL | 63.9 | 30.3 | C1578s-DV26SP | 69.7 | 36.1 |
| | | | | | | | | C1578s-DV28SP | 56.9 | 23.3 |
| | | | | | | | | C1578s-DV29SP | 77.1 | 43.5 |
| | | | | | | | | C1578s-DV25SP | 75.8 | 42.2 |
| | | | | | | | | C1578s-DV27SP | 73.1 | 39.5 |
| 585P | 47.33 | 1585s | 48.9 | 1.57 | B1585sAL | 59.3 | 11.97 | C1585s-DV26SP | 62.6 | 15.27 |
| | | | | | | | | C1585s-DV27SP | 64.6 | 17.27 |
| | | | | | | | | C1585s-DV28SP | 49.5 | 2.17 |
| | | | | | | | | C1585s-DV29SP | 59.2 | 11.87 |
| 816P | 64.5 | 1816 | 46.8 | -17.7 | B1816-AL | 60.9 | -3.6 | C1816-DV25P | 68.1 | 3.6 |
| | | | | | | | | C1816-DV26P | 58.5 | -6 |
| | | | | | | | | C1816-DV27P | 64.5 | 0 |
| | | | | | | | | C1816-DV28P | 49.2 | -15.3 |
| | | | | | | | | C1816-DV29P | 68.5 | 4 |
| 835P | 13.9 | 1835 | 51.6 | 37.7 | B1835-AL | 62.3 | 48.4 | C1835-DV25P | 68.2 | 54.3 |
| | | | | | | | | C1835-DV26P | 71.8 | 57.9 |
| | | | | | | | | C1835-DV27P | 51.2 | 37.3 |
| | | | | | | | | C1835-DV28P | 70 | 56.1 |
| | | | | | | | | C1835-DV29P | 64.9 | 51 |
| 836P | 41.9 | 1836 | 46.5 | 4.6 | B1836-AL | 59.8 | 17.9 | C1836-DV26P | 63.8 | 21.9 |
| | | | | | | | | C1836-DV27P | 58.9 | 17 |
| | | | | | | | | C1836-DV28P | 63.8 | 21.9 |
| | | | | | | | | C1836-DV29P | 56.9 | 15 |
| 839P | -15.4 | 1839 | 45.7 | 61.1 | B1839-AL | 59.1 | 74.5 | C1839-DV26P | 48.6 | 64 |
| | | | | | | | | C1839-DV27P | 63.9 | 79.3 |
| | | | | | | | | C1839-DV28P | 51.4 | 66.8 |
| | | | | | | | | C1839-DV29P | 64.2 | 79.6 |
| | | 1838 | 50.6 | 66.0 | B1838-AL | 63.6 | 79.0 | C1838-DV26P | 67.2 | 82.6 |
| | | | | | | | | C1838-DV28P | 60.3 | 75.7 |
| | | | | | | | | C1838-DV29P | 75.6 | 91 |
| | | | | | | | | C1838-DV25P | 69.3 | 84.7 |
| | | | | | | | | C1838-DV27P | 72.3 | 87.7 |
| 791P | 50.8 | 1791 | 46.5 | -4.3 | B1791-AL | 61.8 | 11 | C1791-DV26P | 70.3 | 19.5 |
| | | | | | | | | C1791-DV27P | 63.6 | 12.8 |
| | | | | | | | | C1791-DV28P | 64.6 | 13.8 |
| | | | | | | | | C1791-DV29P | 59.4 | 8.6 |

### I. Comparison of sequence structures

As shown in Table 81, the sequences disclosed in the prior art are highly similar to the sequences disclosed herein, with only minor differences. See the table below for a detailed comparison.

**Table 81: Comparison between sequences disclosed in the prior art and sequences disclosed herein**

| Sequence ID | **Sense strand 5'-3'** | **SEQ ID NO:** | **Antisense strand 5'-3'** | **SEQ ID NO:** | Source |
|---|---|---|---|---|---|
| 579P | ACCUUUUCUUCUAAUG AGUCU | 1284 | AGACUCAUUAGAAGAA AAGGUGG | 1297 | CN117751189A |
| 1579 | CCUUUUCUUCUAAUGA GUCGA | 788 | UCGACUCAUUAGAAGA AAAGGUG | 801 | Sequences disclosed herein |
| 789P | UUUGUGAAACAAAAAA GUGA | 1285 | UCACUUUUUGUUUCA CAAACA | 1298 | WO2024031101A1 |
| 1789 | GUUUGUGAAACAAAAA AGUGU | 790 | ACACUUUUUGUUUCA CAAACAA | 803 | Sequences disclosed herein |
| 812P | CCUUUUCAAGUUGAGA ACAA | 1286 | UUGUUCUCAACUUGAA AAGGGA | 1299 | WO2024031101A1 |
| 1812 | CCUUUUCAAGUUGAGA ACAAA | 787 | UUUGUUCUCAACUUGA AAAGGA | 800 | Sequences disclosed herein |
| 576P | CCACCUUUUCUUCUAA UGAGU | 1287 | ACUCAUUAGAAGAAAA GGUGGGA | 1300 | CN112313335A |
| 1576s | CCACCUUUUCUUCUAA UGAA | 795 | UUCAUUAGAAGAAAAG GUGGGA | 808 | Sequences disclosed herein |
| 578P | CACCUUUUCUUCUAAU GAGUA | 1288 | UACUCAUUAGAAGAAA AGGUGGG | 1301 | CN112313335A |
| 1578s | ACCUUUUCUUCUAAUG AGUA | 796 | UACUCAUUAGAAGAAA AGGUGG | 809 | Sequences disclosed herein |
| 585P | UUCUAAUGAGUCGACU UUA | 1289 | UAAAGUCGACUCAUUA GAA | 1302 | CN112852809A |
| 1585s | CUUCUAAUGAGUCGAC UUUA | 794 | UAAAGUCGACUCAUUA GAAGAA | 807 | Sequences disclosed herein |
| 816P | UCAAGUUGAGAACAAA AAUA | 1292 | UAUUUUUGUUCUCAAC UUGAAA | 1305 | WO2024031101A1 |
| 1816 | UUCAAGUUGAGAACAA AAAUU | 786 | AAUUUUUGUUCUCAAC UUGAAAA | 799 | Sequences disclosed herein |
| 835P | GGGUUUUAAAUUAAA GUAUA | 1293 | UACUUUAAUUUAAAA CCCAA | 1306 | CN113862268A |
| 1835 | UUGGGUUUUAAAAUUA AAGUA | 785 | UACUUUAAUUUAAAA CCCAAUU | 798 | Sequences disclosed herein |
| 836P | GGUUUUAAAUUAAAG UAUAC | 1294 | AUACUUUAAUUUUAAA ACCCA | 1307 | CN113862268A |
| 1836 | UGGGUUUUAAAAUUAA AGUAU | 789 | AUACUUUAAUUUUAAA ACCCAAU | 802 | Sequences disclosed herein |
| 839P | GUUUUAAAAUUAAAGU AUACA | 1295 | UAUACUUUAAUUUUAA AACCC | 1308 | CN113862268A |
| 1839 | GUUUUAAAAUUAAAGU AUACA | 791 | UGUAUACUUUAAUUUU AAAACCC | 804 | Sequences disclosed herein |
| 1838 | GGUUUUAAAAUUAAAG UAUAA | 797 | UUAUACUUUAAUUUUA AAACCCA | 810 | Sequences disclosed herein |
| 791P | UUGUGAAACAAAAAG UGUUU | 1296 | ACACUUUUUUGUUUCA CAAUU | 1309 | CN106574268B |
| 1791 | UUGUGAAACAAAAAG UGUUA | 792 | UAACACUUUUUUGUUU CACAAAC | 805 | Sequences disclosed herein |

As shown in Table 81:
Compared to the sequence 1579 disclosed herein, the sequence 579P disclosed in the prior art differs in that the sense strand has one additional base A at the 5' end and one different base U at the 3' end; the antisense strand has one different base A at the 5' end and one additional base G at the 3' end.
Compared to the sequence 1789 disclosed herein, the sequence 789P disclosed in the prior art differs in that the sense strand lacks one base G at the 5' end and has one different base A at the 3' end; the antisense strand has one different base U at the 5' end and lacks one base A at the 3' end.
Compared to the sequence 1812 disclosed herein, the sequence 812P disclosed in the prior art differs in that the sense strand lacks one base A at the 3' end; the antisense strand lacks one base U at the 5' end.
Compared to the sequence 1576s disclosed herein, the sequence 576P disclosed in the prior art differs in that the sense strand has two different bases GU at the 3' end; the antisense strand has two different bases AC at the 5' end.
Compared to the sequence 1578s disclosed herein, the sequence 578P disclosed in the prior art differs in that the sense strand has one additional base C at the 5' end; the antisense strand has one additional base G at the 3' end.
Compared to the sequence 1585s disclosed herein, the sequence 585P disclosed in the prior art differs in that the sense strand lacks one base C at the 5' end; the antisense strand lacks three bases GAA at the 3' end.
Compared to the sequence 1816 disclosed herein, the sequence 816P disclosed in the prior art differs in that the sense strand lacks one base U at the 5' end; the antisense strand has one different base U at the 5' end and lacks one base A at the 3' end.
Compared to the sequence 1835 disclosed herein, the sequence 835P disclosed in the prior art differs in that the sense strand lacks two bases UU at the 5' end; the antisense strand lacks two bases UU at the 3' end.
Compared to the sequence 1836 disclosed herein, the sequence 836P disclosed in the prior art differs in that the sense strand lacks two bases UG at the 5' end; the antisense strand lacks two bases AU at the 3' end.
Compared to the sequence 1839 disclosed herein, the sequence 839P disclosed in the prior art has an identical sense strand; differs in that the antisense strand lacks two bases UG at the 5' end. Compared to the sequence 1838 disclosed herein, the sequence 839P disclosed in the prior art differs in that the sense strand lacks one base G at the 5' end and has two different bases CA at the 3' end; the antisense strand lacks one base U at the 5' end and lacks one base A at the 3' end.
Compared to the sequence 1791 disclosed herein, the sequence 791P disclosed in the prior art differs in that the sense strand has one different base U at the 3' end; the antisense strand lacks two bases UA at the 5' end and has two different bases UU at the 3' end.

### II. Activity comparison

**1) The unmodified sequences disclosed herein exhibited significantly enhanced activity compared to the similar unmodified sequences disclosed in the prior art. For example, the unmodified sequence 1576s disclosed herein exhibited a 20.9% increase in inhibition rate compared to the structurally similar 576P.**

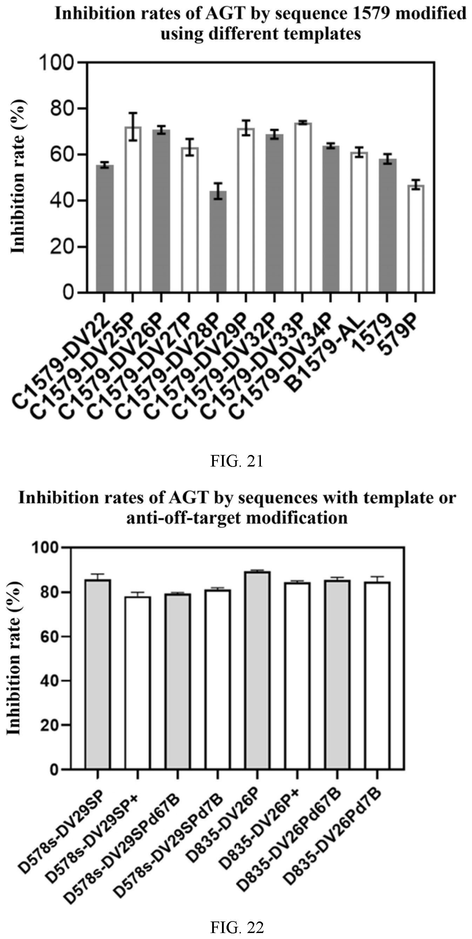

The unmodified sequence 1579 disclosed herein exhibited an inhibition rate of 58.2% on AGT gene expression, which was 11.2% higher than that of the similar sequence 579P disclosed in the prior art (inhibition rate of 47%), indicating a significant improvement (FIG. 21).

The unmodified sequence 1789 disclosed herein exhibited an inhibition rate of 54.5% on AGT gene expression, which was 2.6% higher than that of the similar sequence 789P disclosed in the prior art (inhibition rate of 51.9%).

The unmodified sequence 1812 disclosed herein exhibited an inhibition rate of 56.8% on AGT gene expression, which was 14.3% higher than that of the similar sequence 812P disclosed in the prior art (inhibition rate of 42.5%), indicating a significant improvement.

The unmodified sequence 1576s disclosed herein exhibited an inhibition rate of 59.1% on AGT gene expression, which was 20.9% higher than that of the similar sequence 576P disclosed in the prior art (inhibition rate of 38.2%), indicating a significant improvement.

The unmodified sequence 1578s disclosed herein exhibited an inhibition rate of 55.2% on AGT gene expression, which was 21.6% higher than that of the similar sequence 578P disclosed in the prior art (inhibition rate of 33.6%), indicating a significant improvement.

The unmodified sequence 1585s disclosed herein exhibited an inhibition rate of 48.9% on AGT gene expression, which was 1.57% higher than that of the similar sequence 585P disclosed in the prior art (inhibition rate of 47.33%).

The unmodified sequence 1816 disclosed herein exhibited an inhibition rate of 46.8% on AGT gene expression, which was -17.7% higher than that of the similar sequence 816P disclosed in the prior art (inhibition rate of 64.5%), indicating a significant improvement.

The unmodified sequence 1835 disclosed herein exhibited an inhibition rate of 51.6% on AGT gene expression, which was 37.7% higher than that of the similar sequence 835P disclosed in the prior art (inhibition rate of 13.9%), indicating a significant improvement.

The unmodified sequence 1836 disclosed herein exhibited an inhibition rate of 46.5% on AGT gene expression, which was 4.6% higher than that of the similar sequence 836P disclosed in the prior art (inhibition rate of 41.9%).

The unmodified sequence 1839 disclosed herein exhibited an inhibition rate of 45.7% on AGT gene expression, which was 61.1% higher than that of the similar sequence 839P disclosed in the prior art (inhibition rate of -15.4%), indicating a significant improvement.

The unmodified sequence 1838 disclosed herein exhibited an inhibition rate of 50.6% on AGT gene expression, which was 66.0% higher than that of the similar sequence 839P disclosed in the prior art (inhibition rate of -15.4%), indicating a significant improvement.

The unmodified sequence 1791 disclosed herein exhibited an inhibition rate of 46.5% on AGT gene expression, which was 4.3% lower than that of the similar sequence 791P disclosed in the prior art (inhibition rate of 50.8%).

**2) The alternately modified sequences disclosed herein exhibited significantly enhanced activity compared to the similar unmodified sequences disclosed in the prior art.** F**or example, the alternately modified sequence B1579-AL disclosed herein exhibited a 14.1% increase in inhibition rate compared to the sequence 579P.**

For unmodified sequence 1579 disclosed herein, after alternating modification, the resulting sequence B1579-AL exhibited an inhibition rate of 61.1% on AGT gene expression, which was 14.1% higher than that of the unmodified sequence 579P disclosed in the prior art, indicating a significant improvement.

For unmodified sequence 1789 disclosed herein, after alternating modification, the resulting sequence B1789-AL exhibited an inhibition rate of 63.5% on AGT gene expression, which was 11.6% higher than that of the unmodified sequence 789P disclosed in the prior art, indicating a significant improvement.

For unmodified sequence 1812 disclosed herein, after alternating modification, the resulting sequence B1812-AL exhibited an inhibition rate of 62.9% on AGT gene expression, which was 20.4% higher than that of the unmodified sequence 812P disclosed in the prior art, indicating a significant improvement.

For unmodified sequence 1576s disclosed herein, after alternating modification, the resulting sequence B1576s-AL exhibited an inhibition rate of 64.6% on AGT gene expression, which was 26.4% higher than that of the unmodified sequence 576P disclosed in the prior art, indicating a significant improvement.

For unmodified sequence 1578s disclosed herein, after alternating modification, the resulting sequence B1578s-AL exhibited an inhibition rate of 63.9% on AGT gene expression, which was 30.3% higher than that of the unmodified sequence 578P disclosed in the prior art, indicating a significant improvement.

For unmodified sequence 1585s disclosed herein, after alternating modification, the resulting sequence B1585s-AL exhibited an inhibition rate of 59.3% on AGT gene expression, which was 11.97% higher than that of the unmodified sequence 585P disclosed in the prior art, indicating a significant improvement.

For unmodified sequence 1816 disclosed herein, after alternating modification, the resulting sequence B1816-AL exhibited an inhibition rate of 60.9% on AGT gene expression, which was 3.6% lower than that of the unmodified sequence 816P disclosed in the prior art.

For unmodified sequence 1835 disclosed herein, after alternating modification, the resulting sequence B1835-AL exhibited an inhibition rate of 62.3% on AGT gene expression, which was 48.4% higher than that of the unmodified sequence 835P disclosed in the prior art, indicating a significant improvement.

For unmodified sequence 1836 disclosed herein, after alternating modification, the resulting sequence B1836-AL exhibited an inhibition rate of 59.8% on AGT gene expression, which was 17.9% higher than that of the unmodified sequence 836P disclosed in the prior art, indicating a significant improvement.

For unmodified sequence 1839 disclosed herein, after alternating modification, the resulting sequence B1839-AL exhibited an inhibition rate of 59.1% on AGT gene expression, which was 74.5% higher than that of the unmodified sequence 839P disclosed in the prior art, indicating a significant improvement.

For unmodified sequence 1838 disclosed herein, after alternating modification, the resulting sequence B1838-AL exhibited an inhibition rate of 63.6% on AGT gene expression, which was 79% higher than that of the unmodified sequence 839P disclosed in the prior art, indicating a significant improvement.

For unmodified sequence 1791 disclosed herein, after alternating modification, the resulting sequence B1791-AL exhibited an inhibition rate of 61.8% on AGT gene expression, which was 11% higher than that of the unmodified sequence 791P disclosed in the prior art, indicating a significant improvement.

**3) The sequences modified using the templates disclosed herein exhibited significantly enhanced activity compared to the similar unmodified sequences disclosed in the prior art. For example, the modified sequence C1579-DV26P disclosed herein exhibited a 25.2% increase in inhibition rate compared to the sequence 579P.**

For unmodified sequence 1579 disclosed herein, after modification using templates DV25P to DV29P, the inhibition rate was increased by more than 20% compared to the unmodified sequence 579P disclosed in the prior art. For example, C1579-DV26P exhibited a 25.2% increase, indicating a significant improvement.

For unmodified sequence 1789 disclosed herein, after modification using templates DV25P to DV29P, the inhibition rate was increased by more than 20% compared to the unmodified sequence 789P disclosed in the prior art. For example, C1789-DV26P exhibited a 21.7% increase, indicating a significant improvement.

For unmodified sequence 1812 disclosed herein, after modification using templates DV25P to DV29P, the inhibition rate was increased by more than 30% compared to the unmodified sequence 812P disclosed in the prior art. For example, C1812-DV25P exhibited a 31.9% increase, indicating a significant improvement.

For unmodified sequence 1576s disclosed herein, after modification using templates DV25P to DV29P, the inhibition rate was increased by more than 40% compared to the unmodified sequence 576P disclosed in the prior art. For example, C1576s-DV29SP exhibited a 41.1% increase, indicating a significant improvement.

For unmodified sequence 1578s disclosed herein, after modification using templates DV25P to DV29P, the inhibition rate was increased by more than 40% compared to the unmodified sequence 578P disclosed in the prior art. For example, C1578s-DV29SP exhibited a 43.5% increase, indicating a significant improvement.

For unmodified sequence 1585s disclosed herein, after modification using templates DV25P to DV29P, the inhibition rate was increased by more than 10% compared to the unmodified sequence 585P disclosed in the prior art. For example, C1585s-DV27SP exhibited a 17.27% increase, indicating a significant improvement.

For unmodified sequence 1816 disclosed herein, after modification using templates DV25P to DV29P, the inhibition rate was not significantly improved compared to the unmodified sequence 816P disclosed in the prior art.

For unmodified sequence 1835 disclosed herein, after modification using templates DV25P to DV29P, the inhibition rate was increased by more than 50% compared to the unmodified sequence 835P disclosed in the prior art. For example, C1835-DV26P exhibited a 57.9% increase, indicating a significant improvement.

For unmodified sequence 1836 disclosed herein, after modification using templates DV25P to DV29P, the inhibition rate was increased by more than 20% compared to the unmodified sequence 836P disclosed in the prior art. For example, C1836-DV26P exhibited a 21.9% increase, indicating a significant improvement.

For unmodified sequence 1839 disclosed herein, after modification using templates DV25P to DV29P, the inhibition rate was increased by more than 70% compared to the unmodified sequence 839P disclosed in the prior art. For example, C1839-DV29P exhibited a 79.6% increase, indicating a significant improvement.

For unmodified sequence 1838 disclosed herein, after modification using templates DV25P to DV29P, the inhibition rate was increased by more than 90% compared to the unmodified sequence 839P disclosed in the prior art. For example, C1838-DV29P exhibited a 91% increase, indicating a significant improvement.

For unmodified sequence 1791 disclosed herein, after modification using templates DV25P to DV29P, the inhibition rate was increased by more than 10% compared to the unmodified sequence 791P disclosed in the prior art. For example, C1791-DV26P exhibited a 19.5% increase, indicating a significant improvement.

It can be concluded that, compared to the similar unmodified sequences disclosed in the prior art, the unmodified sequences, alternately modified sequences, and template-modified sequences disclosed herein showed significantly enhanced inhibition of AGT gene expression, with an increase of up to 91%.

### Summary:

Compared to the similar sequences disclosed in the prior art, the unmodified sequences, alternately modified sequences, and template-modified sequences disclosed herein showed significantly enhanced inhibition of AGT gene expression. For example, the unmodified sequence 1839 disclosed herein exhibited a 61.1% increase in inhibition rate compared to the similar sequence 839P disclosed in the prior art. For the sequence 1835 disclosed herein, the alternately modified sequence B1835-AL exhibited a 48.4% increase in inhibition rate compared to the unmodified sequence 835P disclosed in the prior art, which was similar to 1835. For the sequence 1812 disclosed herein, after modification using template DV25P disclosed herein, the resulting sequence C1812-DV25P exhibited a 31.9% increase in inhibition rate compared to the unmodified sequence 812P disclosed in the prior art, which was similar to 1812.

### Example 22: Off-target effect assay of modified sequences

In practical applications of siRNA, there are numerous instances where the expression of non-target mRNAs, which are only partially complementary to the guide strand (antisense strand), is inhibited. Research by Alnylam Pharmaceuticals has demonstrated that the hepatotoxicity of N-acetylgalactosamine (GalNAc)-conjugated siRNAs is primarily attributed to off-target effects, which arise from a microRNA (miRNA)-like recognition mechanism leading to unintended gene silencing.

To investigate whether the sequences disclosed herein would produce off-target effects, in this example, the inhibitory effects of the highly active sequences from Example 20 on potential off-target genes in HepG2 cells were evaluated, and compared them with Zilebesiran, a drug currently in Phase II clinical trials. For C1576s-DV29SP, C1578s-DV29SP, C1579-DV25P, C1789-DV25P, C1789-DV26P, C1791-DV26P, C1795-DV27P, C1812-DV25P, C1816-DV25P, C1835-DV26P, C1838-DV29P, and C1585s-DV27SP, potential off-target genes with high sequence similarity were identified via BLAST analysis. C1585s-DV27SP showed no significant sequence similarity to any known genes and was therefore excluded from off-target testing. The remaining 12 sequences exhibited significant inhibitory effects on AGT expression at concentrations ranging from 16 pM to 10 nM; all of which except C1578s-DV29SP and C1835-DV26P showed no significant inhibitory effects on potential off-target genes (IC₅₀ values differed by more than 200-fold).

### 1. Experimental materials

### 1) Test samples:

The 12 highly active sequences from Example 20, along with the positive control sequence APC-ZL (Table 82). APC-ZL is the Zilebesiran sequence without a GalNAc delivery vector.

**Table 82: Template-modified sequences**

| **Sequence ID** | **Sense sequence 5'-3'** | **SEQ ID NO:** | **Antisense sequence 5'-3'** | **SEQ ID NO:** |
|---|---|---|---|---|
| C1576s-DV29SP | Cms-Cms-Am-Cm-Cm-Uf-Um-Uf- Um-Cf-Um-Um-Cm-Um-Am-Af- Um-Gm-Am-Am | 1207 | UmsEVP-Ufs-Cm-Af-Uf-Uf-Am-Gm- Am-Am-Gm-Am-Am-Af-Am-Gf-Gm- Um-Gm-Gms-Gms-Am | 1272 |
| C1578s-DV29SP | Ams-Cms-Cm-Um-Um-Uf-Um-Cf- Um-Uf-Cm-Um-Am-Am-Um-Gf- Am-Gm-Um-Am | 1210 | UmsEVP-Afs-Cm-Uf-Cf-Af-Um-Um- Am-Gm-Am-Am-Gm-Af-Am-Af-Am- Gm-Gm-Ums-Gms-Gm | 1276 |
| C1579-DV25P | Cms-Cms-Um-Um-Um-Um-Cf-Um- Uf-Cf-Uf-Am-Am-Um-Gm-Am-Gf- Um-Cm-Gm-Am | 1191 | UmsEVP-Cfs-Gm-Am-Cm-Uf-Cm- Am-Um-Um-Am-Gm-Am-Af-Gm-Af- Am-Am-Am-Gm-Gms-Ums-Gm | 1241 |
| C1789-DV25P | Gms-Ums-Um-Um-Gm-Um-Gf-Am- Af-Af-Cf-Am-Am-Am-Am-Am-Af- Gm-Um-Gm-Um | 1196 | AmsEVP-Cfs-Am-Cm-Um-Uf-Um- Um-Um-Um-Gm-Um-Um-Uf-Cm-Af- Cm-Am-Am-Am-Cms-Ams-Am | 1252 |
| C1789-DV26P | Gms-Ums-Um-Um-Gm-Um-Gf-Am- Af-Af-Cf-Am-Am-Am-Am-Am-Af- Gm-Um-Gm-Um | 1196 | AmsEVP-Cfs-Af-Cf-Um-Uf-Um-Um- Um-Um-Gm-Um-Um-Uf-Cm-Af-Cm- Am-Am-Am-Cms-Ams-Am | 1249 |
| C1791-DV26P | Ums-Ums-Gm-Um-Gm-Am-Af-Am- Cf-Af-Af-Am-Am-Am-Am-Gm-Uf- Gm-Um-Um-Am | 1201 | UmsEVP-Afs-Af-Cf-Am-Cf-Um-Um- Um-Um-Um-Um-Gm-Uf-Um-Uf-Cm- Am-Cm-Am-Ams-Ams-Cm | 1260 |
| C1795-DV27P | Gms-Ams-Am-Am-Cm-Am-Af-Am- Af-Af-Af-Gm-Um-Gm-Um-Um-Cf- Cm-Cm-Um-Um | 1203 | AmsEVP-Afs-Gm-Gf-Gf-Af-Am-Cm- Am-Cm-Um-Um-Um-Uf-Um-Uf-Gm- Um-Um-Um-Cms-Ams-Cm | 1263 |
| C1812-DV25P | Cms-Cms-Um-Um-Um-Um-Cf-Am- Af-Gf-Uf-Um-Gm-Am-Gm-Am-Af- Cm-Am-Am-Am | 1188 | UmsEVP-Ufs-Um-Gm-Um-Uf-Cm- Um-Cm-Am-Am-Cm-Um-Uf-Gm-Af- Am-Am-Am-Gm-Gms-Gms-Am | 1234 |
| C1816-DV25P | Ums-Ums-Cm-Am-Am-Gm-Uf-Um- Gf-Af-Gf-Am-Am-Cm-Am-Am-Af- Am-Am-Um-Um | 1185 | AmsEVP-Afs-Um-Um-Um-Uf-Um- Gm-Um-Um-Cm-Um-Cm-Af-Am-Cf- Um-Um-Gm-Am-Ams-Ams-Am | 1227 |
| C1835-DV26P | Ums-Ums-Gm-Gm-Gm-Um-Uf-Um- Uf-Af-Af-Am-Am-Um-Um-Am-Af- Am-Gm-Um-Am | 1182 | UmsEVP-Afs-Cf-Uf-Um-Uf-Am-Am- Um-Um-Um-Um-Am-Af-Am-Af-Cm- Cm-Cm-Am-Ams-Ums-Um | 1217 |
| C1838-DV29P | Gms-Gms-Um-Um-Um-Um-Af-Am- Af-Am-Uf-Um-Am-Am-Am-Gm-Uf- Am-Um-Am-Am | 1213 | UmsEVP-Ufs-Am-Uf-Af-Cf-Um-Um- Um-Am-Am-Um-Um-Uf-Um-Af-Am- Am-Am-Cm-Cms-Cms-Am | 1280 |
| C1839-DV29P | Gms-Ums-Um-Um-Um-Am-Af-Am- Af-Um-Uf-Am-Am-Am-Gm-Um-Af- Um-Am-Cm-Am | 1198 | UmsEVP-Gfs-Um-Af-Uf-Af-Cm-Um- Um-Um-Am-Am-Um-Uf-Um-Uf-Am- Am-Am-Am-Cms-Cms-Cm | 1255 |
| APC-ZL | Gms-Ums-Cm-Am-Um-Cm-Cf-Am- Cf-Af-Af-Um-Gm-Am-Gm-Am-Gm- Um-Am-Cm-Am | 1347 | Ums-Gfs-Um-Am-Cm-Tgna-Cm-Um- Cm-Am-Um-Um-Gm-Uf-Gm-Gf-Am- Um-Gm-Am-Cms-Gms-Am | 1371 |

Cell line: HepG2 cells, provided by Cyagen Biosciences (H1-1701).

HepG2 cells were cultured in DMEM culture medium (ATCC-30-2003) containing 10% fetal bovine serum (FBS, ExCell Bio-FSP500) and 1% penicillin-streptomycin (HyClone-SV30010).

Solvents: sterile nuclease-free water, Gibco Opti-MEM.

### 2. Experimental methods

The inhibitory effects of the test samples on AGT mRNA expression in HepG2 cells were evaluated by qRT-PCR.

### 2.1 Cell culture

Subcultured HepG2 cells in the logarithmic growth phase were cultured in DMEM culture medium containing 10% fetal bovine serum (supplemented with penicillin and streptomycin at 100 µL/mL), and incubated in a cell incubator at 37°C with 5% CO₂. The medium was exchanged once daily. The cells were digested with 0.25% trypsin, followed by centrifugation at 1000 rpm for 5 minutes. The supernatant was discarded, and fresh medium was added for continued culture.

### 2.2 Cell transfection

Preparation of transfection mixture: Lipofectamine RNAiMAX was mixed with Opti-MEM at a volume ratio of 2:98 and vortexed thoroughly.

Preparation of transfection reagent: 60 µL of siRNA solution (diluted in Opti-MEM) was mixed with 60 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 60 µL of transfection mixture was added to 60 µL of Opti-MEM, vortexed thoroughly, followed by incubation at room temperature for 15 minutes.

The prepared transfection reagent was added to a 24-well cell culture plate (100 µL per well), such that the final siRNA concentrations per well were 0.016 nM, 0.08 nM, 0.4 nM, 2 nM, and 10 nM, respectively. Subsequently, 500 µL of cell suspension (1.5 × 10⁵ cells per mL) was added to each well. After cross-mixing, the plate was incubated in a cell incubator at 37°C with 5% CO₂ for 40 hours.

### 2.3 RNA extraction and reverse transcription

At 24 hours post-transfection, the culture medium was removed and the cells were collected for RNA extraction. Total RNA was extracted using the RNeasy^{®} 96 Kit (QIAGEN, 74182) according to the instructions provided by the manufacturer. Subsequently, cDNA was synthesized using the FastKing RT Kit (with gDNase) (TIANGEN, KR116-02) according to the instructions provided by the manufacturer.

### 2.4 RT-qPCR

Refer to "Quantitative analysis of AGT mRNA" in Section 16.3 of Example 18.

### 2.5 Data analysis

The RNA expression levels of the target gene in each sample were calculated based on Ct values using the ΔΔCt relative quantification method. Relative expression of the target gene was expressed as 2^{-ΔΔCt}.

The calculation formula is as follows:
$ΔCt = mean Ct value of the target gene - mean Ct value of the reference gene ;$
$ΔΔCt = ΔCt \left(treatment group\right) - ΔCt \left(RNAiMAX control group\right) ;$

Relative mRNA expression of the target gene = 2^{-ΔΔCt}; 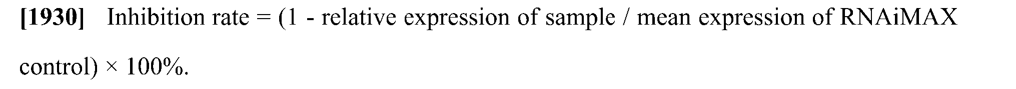Inhibition rate = (1 - relative expression of sample / mean expression of RNAiMAX control) × 100%.

### 3. Experimental results

**3.1 All sequences exhibited significant inhibitory effects on AGT gene expression. For example, C1576s-DV29SP exhibited an inhibition rate of 83.8% against AGT at 10 nM.**

The inhibition rates of these sequences against AGT at various concentrations are shown in Table 83 below. All sequences exhibited significant inhibitory effects against AGT. For example, C1576s-DV29SP exhibited an inhibition rate of 83.8% against AGT at 10 nM.

**Table 83: Inhibition rates of AGT by sequences at various concentrations**

| **Sequence name** | **AGT inhibition rate (%)** | | | | |
|---|---|---|---|---|---|
| | **10.0 nM** | **2.0 nM** | **0.4 nM** | **0.08 nM** | **0.016 nM** |
| C1576s-DV29SP | 83.8 | 81.3 | 82.4 | 68.9 | 56.1 |
| C1578s-DV29SP | 85.7 | 84.9 | 82.6 | 77.5 | 66.7 |
| C1579-DV25P | 85.0 | 84.9 | 82.7 | 77.9 | 58.3 |
| C1789-DV25P | 82.6 | 84.2 | 82.1 | 79.6 | 66.1 |
| C1789-DV26P | 79.3 | 82.8 | 81.3 | 78.8 | 64.5 |
| C1791-DV26P | 86.3 | 87.4 | 83.7 | 76.0 | 57.9 |
| C1795-DV27P | 87.4 | 84.1 | 83.1 | 74.2 | 57.8 |
| C1812-DV25P | 88.2 | 88.0 | 86.8 | 82.5 | 66.4 |
| C1816-DV25P | 84.5 | 83.8 | 80.2 | 73.5 | 56.3 |
| C1835-DV26P | 89.2 | 88.3 | 88.4 | 82.4 | 72.2 |
| C1838-DV29P | 80.0 | 79.3 | 80.8 | 74.1 | 52.4 |
| C1839-DV29P | 82.2 | 83.2 | 81.8 | 73.8 | 64.9 |
| APC-ZL | 90.7 | 87.4 | 76.5 | 76.4 | 53.9 |

**3.2 C1578s-DV29SP and C1835-DV26P exhibited certain off-target effects, while other sequences showed no significant off-target activity.**

The inhibition rates and IC₅₀ values of these sequences against AGT and potential off-target genes at various concentrations are shown in Table 84 below.

**Table 84: Inhibition rates and IC₅₀ values of sequences against potential off-target genes at various concentrations**

| **Sequence name** | **Gene name** | **Inhibition rate (%)** | | | | | | |
|---|---|---|---|---|---|---|---|---|
| | | **10.0 nM** | **2.0 nM** | **0.4 nM** | **0.08 nM** | **0.016 nM** | **IC₅₀/nM** | **Ratio** |
| C1576s-DV29SP | AGT | 83.8 | 81.3 | 82.4 | 68.9 | 56.1 | <0.016 | 1.0 |
| | DSE | -138.9 | -89.7 | -79.0 | -7.1 | -70.7 | >10.0 | >625.0 |
| C1578s-DV29SP | AGT | 85.7 | 84.9 | 82.6 | 77.5 | 66.7 | 0.004 | 1.0 |
| | MIA2 | -12.9 | -4.3 | -13.5 | 4.0 | -3.8 | >10.0 | >2500.0 |
| | CHST15 | 57.9 | 63.1 | 64.0 | 63.6 | 5.0 | 0.019 | 4.8 |
| | ERRFI1 | 66.9 | 65.9 | 69.9 | 74.1 | 16.3 | 0.021 | 5.3 |
| C1579-DV25P | AGT | 85.0 | 84.9 | 82.7 | 77.9 | 58.3 | 0.011 | 1.0 |
| | GLTP | 42.3 | 17.4 | -43.6 | -91.0 | -136.7 | >10.0 | >909.1 |
| C1789-DV25P | AGT | 82.6 | 84.2 | 82.1 | 79.6 | 66.1 | 0.008 | 1.0 |
| | MRPL42 | 2.7 | -2.0 | -19.6 | 3.6 | -16.6 | >10.0 | >1250.0 |
| | NSD3 | 15.0 | 9.1 | -15.4 | 8.5 | -4.5 | >10.0 | >1250.0 |
| | LUC7L | 15.9 | 13.0 | -2.2 | 1.8 | -8.9 | >10.0 | >1250.0 |
| C1789-DV26P | AGT | 79.3 | 82.8 | 81.3 | 78.8 | 64.5 | <0.016 | 1.0 |
| | MRPL42 | 14.6 | -7.5 | -21.2 | -22.6 | -21.4 | >10.0 | >625.0 |
| | NSD3 | 28.2 | 16.7 | -7.0 | -25.8 | -24.4 | >10.0 | >625.0 |
| | LUC7L | 21.8 | 10.7 | -0.5 | -13.4 | -12.8 | >10.0 | >625.0 |
| C1791-DV26P | AGT | 86.3 | 87.4 | 83.7 | 76.0 | 57.9 | 0.008 | 1.0 |
| | ORC2 | -58.0 | -69.3 | -105.6 | -54.9 | -4.4 | >10.0 | >1250.0 |
| | RBM23 | -24.7 | -81.8 | -97.9 | -48.2 | 1.3 | >10.0 | >1250.0 |
| | SLC2A13 | 2.1 | -2.0 | -8.7 | 8.0 | -5.9 | >10.0 | >1250.0 |
| | KANK1 | 21.8 | 25.7 | 1.1 | 20.7 | -11.8 | >10.0 | >1250.0 |
| | LUC7L | 25.5 | 22.0 | 14.9 | 8.8 | -13.6 | >10.0 | >1250.0 |
| C1795-DV27P | AGT | 87.4 | 84.1 | 83.1 | 74.2 | 57.8 | 0.010 | 1.0 |
| | BCKDHB | 16.6 | -2.3 | -5.8 | -14.2 | -7.6 | >10.0 | >1000.0 |
| | ACBD5 | 25.7 | 5.5 | 1.4 | -1.7 | -1.0 | >10.0 | >1000.0 |
| | MPDZ | 12.0 | -8.2 | 7.9 | -10.9 | 1.6 | >10.0 | >1000.0 |
| | TRIP11 | 2.7 | -23.4 | -30.1 | -23.8 | -0.7 | >10.0 | >1000.0 |
| | WDPCP | 14.4 | 6.2 | -16.2 | -6.2 | -3.2 | >10.0 | >1000.0 |
| C1812-DV25P | AGT | 88.2 | 88.0 | 86.8 | 82.5 | 66.4 | 0.008 | 1.0 |
| | ISOC1 | -11.1 | -15.4 | -34.8 | -20.6 | -14.5 | >10.0 | >1250.0 |
| | ATXN10 | -21.9 | -19.0 | -30.1 | -11.7 | -10.3 | >10.0 | >1250.0 |
| | PARP14 | -58.2 | -102.4 | -110.3 | -20.2 | -24.9 | >10.0 | >1250.0 |
| | SRGAP1 | -53.4 | -45.6 | -46.1 | -14.9 | -3.2 | >10.0 | >1250.0 |
| C1816-DV25P | AGT | 84.5 | 83.8 | 80.2 | 73.5 | 56.3 | 0.011 | 1.0 |
| | FOXN3 | 48.6 | 40.8 | 25.8 | 16.7 | 18.0 | >10.0 | >909.1 |
| C1835-DV26P | AGT | 89.2 | 88.3 | 88.4 | 82.4 | 72.2 | <0.016 | 1.0 |
| | MTF2 | 15.3 | -5.6 | 0.2 | -8.1 | -1.8 | 12.227 | 764.2 |
| | CCNA2 | 74.6 | 50.3 | 22.0 | -9.8 | 4.3 | 1.766 | 110.4 |
| | SMNDC1 | -24.5 | -30.7 | -22.1 | -30.3 | -12.9 | >10.0 | >625.0 |
| | ZNF107 | 14.1 | 3.3 | -4.6 | -19.6 | 0.2 | >10.0 | >625.0 |
| | RFC3 | 43.3 | 18.3 | 5.2 | -8.7 | 7.7 | >10.0 | >625.0 |
| C1838-DV29P | AGT | 80.0 | 79.3 | 80.8 | 74.1 | 52.4 | <0.016 | 1.0 |
| | AKAP9 | -45.3 | -55.3 | -37.0 | -9.6 | -13.0 | >10.0 | >625.0 |
| | DENR | 19.2 | -3.6 | -26.3 | -7.9 | -31.1 | >10.0 | >625.0 |
| | PGRMC1 | 40.9 | 6.1 | -27.2 | -13.9 | -18.9 | >10.0 | >625.0 |
| | ZNF626 | 20.3 | -0.6 | -5.2 | -6.8 | 3.1 | >10.0 | >625.0 |
| C1839-DV29P | AGT | 82.2 | 83.2 | 81.8 | 73.8 | 64.9 | <0.016 | 1.0 |
| | GRB14 | 5.8 | -11.9 | -4.7 | -12.4 | -42.1 | >10.0 | >625.0 |
| | JADE1 | -38.2 | -26.1 | -40.0 | -46.0 | -43.5 | >10.0 | >625.0 |
| | STAT5B | 13.2 | 0.3 | -6.5 | -25.4 | -0.4 | >10.0 | >625.0 |
| | ABCD3 | 35.4 | 18.2 | -0.8 | -3.2 | -0.8 | >10.0 | >625.0 |
| APC-ZL | AGT | 90.7 | 87.4 | 76.5 | 76.4 | 53.9 | 0.012 | 1.0 |
| | BAP1 | 24.4 | 14.1 | 14.0 | 2.1 | 21.7 | >10.0 | >833.3 |
| | NFAT5 | 2.4 | -8.0 | 10.4 | 0.6 | -17.5 | >10.0 | >833.3 |
| | ZNF460 | 40.6 | 42.1 | 30.3 | 27.1 | 12.8 | >10.0 | >833.3 |

As shown in the table above:
(1) Some sequences exhibited minimal inhibitory effects on potential off-target genes. For example, C1789-DV25P exhibited an inhibition rate of only 2.7% against MRPL42 at 10 nM, indicating no significant inhibitory effects.
(2) Some sequences exhibited certain inhibitory effects on potential off-target genes at high concentrations, but the IC₅₀ for inhibiting off-target genes was significantly higher than the IC₅₀ for inhibiting AGT. For example, C1579-DV25P exhibited an inhibition rate of 42.3% against GLTP at 10 nM, but the IC₅₀ against GLTP was greater than 10 nM, while the IC₅₀ against AGT was 0.011 nM, with an IC₅₀ ratio much greater than 200, indicating no significant off-target effects.
(3) C1578s-DV29SP exhibited an inhibition rate of 66.9% against ERRFI1 at 10 nM with an IC₅₀ value of 0.021 nM, while the IC₅₀ against AGT was 0.004 nM, with an IC₅₀ ratio of only 5.3; C1835-DV26P exhibited an inhibition rate of 74.6% against CCNA2 at 10 nM with an IC₅₀ value of 1.766 nM, while the IC₅₀ against AGT was less than 0.016 nM, with a minimum IC₅₀ ratio of 110.4. These two sequences were considered to have certain off-target effects, requiring anti-off-target modifications to reduce their inhibition of off-target genes.

### Summary:

(1) All sequences exhibited significant inhibitory effects against AGT. For example, C1576s-DV29SP exhibited an inhibition rate of 83.8% against AGT at 10 nM.
(2) C1578s-DV29SP and C1835-DV26P exhibited certain off-target effects, while other sequences showed no significant off-target activity.

### Example 23: Inhibitory effect of sequences with specific anti-off-target designs on AGT and off-target genes

In practical applications of siRNA, there are numerous instances where the expression of non-target mRNAs, which are only partially complementary to the guide strand (antisense strand), is inhibited. Research by Alnylam Pharmaceuticals has demonstrated that the hepatotoxicity of N-acetylgalactosamine (GalNAc)-conjugated siRNAs is primarily attributed to off-target effects, which arise from a microRNA (miRNA)-like recognition mechanism leading to unintended gene silencing.

To address the issue, Alnylam Pharmaceuticals introduced a glycol nucleic acid (GNA) modification at position 7 of the antisense strand of siRNA in their latest fifth-generation template design to disrupt the seed region of the antisense strand, thereby significantly reducing off-target effects and mitigating hepatotoxicity. Such modifications can interfere with the binding of siRNA to unintended targets via seed-mediated recognition, thereby inhibiting off-target effects.

In addition, a study in 2008 revealed that replacing all eight base pairs at the 5' end of the antisense strand of the siRNA duplex with DNA significantly reduced the off-target effects without compromising the activity of siRNA. Research on mRNA cleavage sites revealed that DNA substitution did not interfere with RISC-mediated mRNA cleavage between nucleotides at positions 10 and 11 of the antisense strand but did interfere with cleavage at non-canonical sites, possibly by inhibiting non-specific cleavage of double-stranded RNA by RNase.

Based on the above, to reduce off-target effects of the sequences, in this example, the unmodified sequences 1578s and 1835 were modified using DV25 to DV29P, by replacing the nucleotides at position 7 or positions 6 and 7 of the antisense strand with DNA, while also replacing the nucleotides at complementary positions of the sense strand with DNA, thereby reducing the off-target effects. These two anti-off-target modifications were compared with the anti-off-target design in which position 7 of the antisense strand was replaced with (S)-GNA.

The experimental results demonstrated that, at concentrations ranging from 10 nM to 16 pM, the sequences incorporating anti-off-target design exhibited significant inhibitory effects on the target gene AGT, while certain sequences exhibited significantly reduced inhibitory effects on off-target genes. The findings indicate that anti-off-target design does not compromise the inhibitory effects of the alternately modified and template-modified sequences disclosed herein on AGT gene expression, but can effectively inhibit the off-target effects of certain sequences.

### 1. Experimental materials

### 1) Test samples:

The unmodified sequences 1578s, 1835, and 1838 incorporating template modification alone or both template modification and anti-off-target design (Table 85).

### 2) Sequence synthesis

### I. Template-modified sequences

The sequences were synthesized using the method described in Example 17, concerning synthesis of alternately modified sequences.

### II. siRNA sequences with DNA-based anti-off-target design

The siRNA sequences were synthesized using the method described in Example 17, except that the nucleotide at position 6 or 7 from the 5' end of the antisense strand was synthesized using the following DNA phosphoramidite monomers, including DMT-dA phosphoramidite monomer (Formula 19), DMT-dT phosphoramidite monomer (Formula 20), DMT-dC phosphoramidite monomer (Formula 21), and DMT-dG phosphoramidite monomer (Formula 22), the structures of which are as follows:

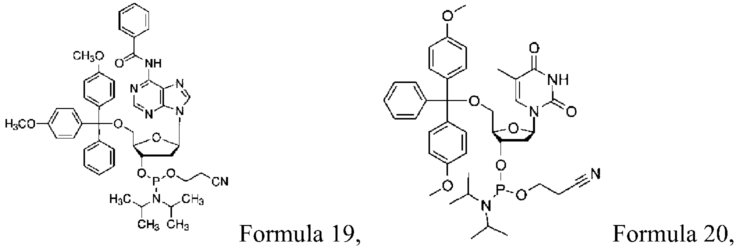

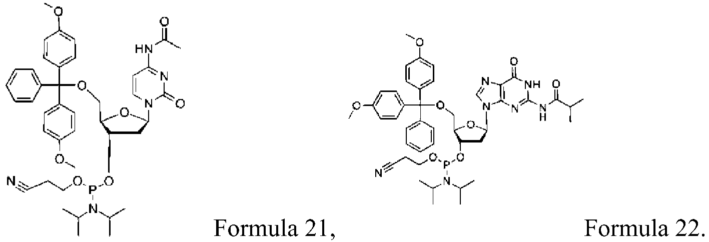

### III. siRNA sequences with GNA-based anti-off-target design

The siRNA sequences were synthesized using the method described in Example 17, except that the base at position 7 from the 5' end of the antisense strand was synthesized using a GNA monomer, whose structure is as follows:

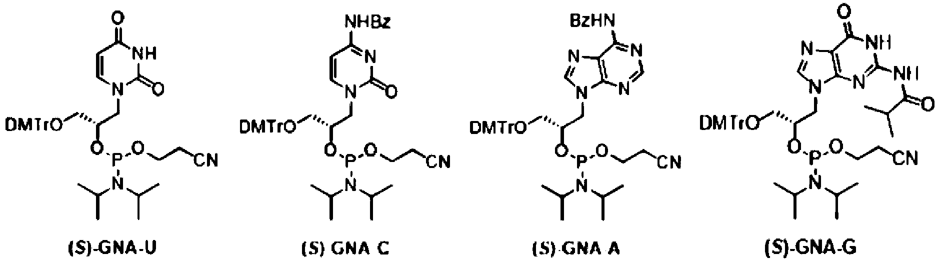

**Table 85: Sequences incorporating template modification and anti-off-target design**

| **Sequence ID** | **Sense strand 5'-3'** | **Modified SEQ ID NO:/corr. unmodified SEQ ID NO:** | **Antisense strand 5'-3'** | **Modified SEQ ID NO:/corr. unmodified SEQ ID NO:** |
|---|---|---|---|---|
| D835-DV26P | Ums-Ums-Gm-Gm-Gm-Um- Uf-Um-Uf-Af-Af-Am-Am-Um- Um-Am-Af-Am-Gm-Um-Am | 1182/785 | UmsEVP-Afs-Cf-Uf-Um-Uf-Am- Am-Um-Um-Um-Um-Am-Af-Am- Af-Cm-Cm-Cm-Am-Ams-Ums-Um | 1217/798 |
| D835-DV26P+ | Ums-Ums-Gm-Gm-Gm-Um- Uf-Um-Uf-Af-Af-Am-Am-Um- Um-Am-Af-Am-Gm-Um-Am | 1182/785 | UmsEVP-Afs-Cf-Uf-Um-Tgna-Am- Am-Um-Um-Um-Um-Am-Af-Am- Af-Cm-Cm-Cm-Am-Ams-Ums-Um | 1348/798 |
| D835-DV26Pd7B | Ums-Ums-Gm-Gm-Gm-Um- Uf-Um-Uf-Af-Af-Am-Am-Um- Td-Am-Af-Am-Gm-Um-Am | 1310/785 | UmsEVP-Afs-Cf-Uf-Um-Uf-Ad- Am-Um-Um-Um-Um-Am-Af-Am- Af-Cm-Cm-Cm-Am-Ams-Ums-Um | 1349/798 |
| D835-DV26Pd67B | Ums-Ums-Gm-Gm-Gm-Um- Uf-Um-Uf-Af-Af-Am-Am-Um- Td-Ad-Af-Am-Gm-Um-Am | 1311/785 | UmsEVP-Afs-Cf-Uf-Um-Td-Ad- Am-Um-Um-Um-Um-Am-Af-Am- Af-Cm-Cm-Cm-Am-Ams-Ums-Um | 1350/798 |
| D578s-DV29SP+ | Ams-Cms-Cm-Um-Um-Uf- Um-Cf-Um-Uf-Cm-Um-Am- Am-Um-Gf-Am-Gm-Um-Am | 1210/796 | UmsEVP-Afs-Cm-Uf-Cf-Agna-Um- Um-Am-Gm-Am-Am-Gm-Af-Am- Af-Am-Gm-Gm-Ums-Gms-Gm | 1351/809 |
| D578s-DV29SPd67 B | Ams-Cms-Cm-Um-Um-Uf- Um-Cf-Um-Uf-Cm-Um-Am- Ad-Td-Gf-Am-Gm-Um-Am | 1312/796 | UmsEVP-Afs-Cm-Uf-Cf-Ad-Td- Um-Am-Gm-Am-Am-Gm-Af-Am- Af-Am-Gm-Gm-Ums-Gms-Gm | 1352/809 |
| D578s-DV29SPd7B | Ams-Cms-Cm-Um-Um-Uf- Um-Cf-Um-Uf-Cm-Um-Am- Ad-Um-Gf-Am-Gm-Um-Am | 1313/796 | UmsEVP-Afs-Cm-Uf-Cf-Af-Td- Um-Am-Gm-Am-Am-Gm-Af-Am- Af-Am-Gm-Gm-Ums-Gms-Gm | 1353/809 |
| D578s-DV29SP | Ams-Cms-Cm-Um-Um-Uf- Um-Cf-Um-Uf-Cm-Um-Am- Am-Um-Gf-Am-Gm-Um-Am | 1210/796 | UmsEVP-Afs-Cm-Uf-Cf-Af-Um- Um-Am-Gm-Am-Am-Gm-Af-Am- Af-Am-Gm-Gm-Ums-Gms-Gm | 1276/809 |

Cell line: HepG2 cells, provided by Cyagen Biosciences (H1-1701).

HepG2 cells were cultured in DMEM culture medium (ATCC-30-2003) containing 10% fetal bovine serum (FBS, ExCell Bio-FSP500) and 1% penicillin-streptomycin (HyClone-SV30010).

Solvents: sterile nuclease-free water, Gibco Opti-MEM.

### 2. Experimental methods

The inhibitory effects of the test samples on the mRNA expression of AGT and potential off-target genes in PHHs were evaluated by qRT-PCR.

### 2.1 Cell culture

Subcultured HepG2 cells in the logarithmic growth phase were cultured in DMEM culture medium containing 10% fetal bovine serum (supplemented with penicillin and streptomycin at 100 µL/mL), and incubated in a cell incubator at 37°C with 5% CO₂. The medium was exchanged once daily. The cells were digested with 0.25% trypsin, followed by centrifugation at 1000 rpm for 5 minutes. The supernatant was discarded, and fresh medium was added for continued culture.

### 2.2 Cell transfection

Preparation of transfection mixture: Lipofectamine RNAiMAX was mixed with Opti-MEM at a volume ratio of 2:98 and vortexed thoroughly.

Preparation of transfection reagent: 60 µL of siRNA solution (diluted in Opti-MEM) was mixed with 60 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 60 µL of transfection mixture was added to 60 µL of Opti-MEM, vortexed thoroughly, followed by incubation at room temperature for 15 minutes.

The prepared transfection reagent was added to a 24-well cell culture plate (100 µL per well), such that the final siRNA concentrations per well were 16 pM, 80 pM, 400 pM, 2 nM, and 10 nM, respectively. Subsequently, 500 µL of cell suspension (1.5 × 10⁵ cells per mL) was added to each well. After cross-mixing, the plate was incubated in a cell incubator at 37°C with 5% CO₂ for 40 hours.

### 2.3 RNA extraction and reverse transcription

At 24 hours post-transfection, the culture medium was removed and the cells were collected for RNA extraction. Total RNA was extracted using the RNeasy^{®} 96 Kit (QIAGEN, 74182) according to the instructions provided by the manufacturer. Subsequently, cDNA was synthesized using the FastKing RT Kit (with gDNase) (TIANGEN, KR116-02) according to the instructions provided by the manufacturer.

### 2.4 RT-qPCR

Target cDNA was tested by qPCR, with GAPDH cDNA used as an internal control in parallel. 8 µL of prepared qPCR reaction mixture and 2 µL of sample cDNA were added to a 384-well plate. TaqMan qPCR was performed according to the following program: 95°C for 10 minutes, followed by 40 cycles of 95°C for 15 seconds and 60°C for 1 minute. SYBR qPCR was performed according to the following program: 50°C for 2 minutes, 95°C for 10 minutes, followed by 40 cycles of 95°C for 15 seconds and 60°C for 1 minute; finally, a melting curve analysis was performed at 95°C for 15 seconds, 60°C for 1 minute, and 95°C for 15 seconds.

### 2.5 Data analysis

The RNA expression levels of the target gene in each sample were calculated based on Ct values using the ΔΔCt relative quantification method. Relative expression of the target gene was expressed as 2^{-ΔΔCt}.

The calculation formula is as follows:
$ΔCt = mean Ct value of the target gene - mean Ct value of the reference gene ;$
$ΔΔCt = ΔCt \left(treatment group\right) - ΔCt \left(RNAiMAX control group\right) ;$

Relative mRNA expression of the target gene = 2^{-ΔΔCt}; 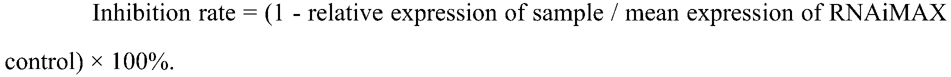 Inhibition rate = (1 - relative expression of sample / mean expression of RNAiMAX control) × 100%.

### 3. Experimental results

**(1) All modified sequences exhibited significant inhibitory effects on AGT gene expression. For example, the template-modified sequence D578s-DV29P exhibited an inhibition rate of 85.7%, while the sequence D578s-DV29Pd7B incorporating both template and anti-off-target modifications exhibited an inhibition rate of 81.3%.**

The inhibition rates of AGT gene expression by all test samples are shown in Table 86 and FIG. 22.

All template-modified sequences except D838-DV29P+ exhibited significant inhibitory effects on AGT at a concentration of 10 nM. For example, the template-modified sequence D578s-DV29P exhibited an inhibition rate of 85.7%.

**Table 86: Inhibition rates of AGT gene expression by sequences incorporating template and anti-off-target modifications**

| **Unmodified sequence** | **Sequence ID** | **Modification pattern** | **Inhibition rate (%)** |
|---|---|---|---|
| 1578s | D578s-DV29SP | Template | 85.7 |
| | D578s-DV29SP+ | Template + GNA anti-off-target | 78.2 |
| | D578s-DV29SPd67B | Template + d67B anti-off-target | 79.3 |
| | D578s-DV29SPd7B | Template + d7B anti-off-target | 81.3 |
| 1835 | D835-DV26P | Template | 89.2 |
| | D835-DV26P+ | Template + GNA anti-off-target | 84.4 |
| | D835-DV26Pd67B | Template + d67B anti-off-target | 85.4 |
| | D835-DV26Pd7B | Template + d7B anti-off-target | 84.6 |

After template modification followed by anti-off-target design, the resulting sequence exhibited significant inhibitory activity on AGT gene expression. For example, for unmodified sequence 1578s, after modification using template DV29SP followed by anti-off-target design, the resulting sequence D578s-DV29SPd7B exhibited an inhibition rate as high as 81.3%.

**(2) After adopting any one or more of the modification patterns disclosed herein followed by anti-off-target design, the resulting sequences exhibited significant anti-off-target effects on certain off-target genes, thereby reducing inhibition of off-target genes.**

As shown in Table 87, the experimental results demonstrated that:
The template-modified sequences disclosed herein, when incorporating anti-off-target design, can reduce inhibition of off-target genes, thereby achieving anti-off-target effects. The sequences modified using the templates disclosed herein, when incorporating anti-off-target design, exhibited anti-off-target effects, reducing inhibition of off-target genes by up to 72.535%.

**Table 87: Inhibition rates of off-target genes by template-modified sequences incorporating anti-off-target design**

| **Unmodified sequence ID** | **Sequence ID** | **Off-target genes** | **Inhibition against off-target genes (%)** | **Reduction in inhibition vs without anti-off-target design (%)** |
|---|---|---|---|---|
| 1578s | D578s-DV29SP | ERRFI1 | 66.894 | 0.000 |
| | D578s-DV29SP+ | | 19.447 | 47.447 |
| | D578s-DV29SPd67B | | 12.678 | 54.216 |
| | D578s-DV29SPd7B | | -5.641 | 72.535 |
| 1835 | D835-DV26P | CCNA2 | 74.619 | 0.000 |
| | D835-DV26P+ | | 35.133 | 39.486 |
| | D835-DV26Pd67B | | 33.215 | 41.404 |
| | D835-DV26Pd7B | | 20.953 | 53.666 |

### 1) Template modification alone

Certain template-modified sequences exhibited measurable off-target effects. For example, for unmodified sequence 1578s, after modification using template DV29SP, the resulting sequence D578s-DV29SP exhibited an inhibition rate of 66.894% against the potential off-target gene ERRFI1; for unmodified sequence 1835, after modification using template DV26P, the resulting sequence D835-DV26P exhibited an inhibition rate of 74.619% against the potential off-target gene CCNA2.

### 2) Template modification combined with anti-off-target design (denoted as "d67B" or "d7B" in sequence identifiers)

Certain sequences incorporating both template and anti-off-target modifications showed significantly reduced inhibition of off-target genes, indicating significant anti-off-target effects. For example, D578s-DV25SPd7B exhibited a 72.535% reduction in inhibition of ERRFI1, indicating a significant decrease; D835-DV26Pd7B exhibited a 53.666% reduction in inhibition of CCNA2, indicating a significant decrease.

### Summary:

1. All modified sequences exhibited significant inhibitory effects on AGT gene expression. For example, the template-modified sequence D578s-DV29SP exhibited an inhibition rate of 85.7%, while the sequence D578s-DV29SPd7B incorporating both template and anti-off-target modifications exhibited an inhibition rate of 81.3%.
2. After adopting any one or more of the modification patterns disclosed herein followed by anti-off-target design, the resulting sequences exhibited significant anti-off-target effects on certain off-target genes, thereby reducing inhibition of off-target genes.
   1) Certain template-modified sequences exhibited measurable off-target effects. For example, for unmodified sequence 1578s, after modification using template DV29SP, the resulting sequence D578s-DV29SP exhibited an inhibition rate of 66.894% against the potential off-target gene ERRFI1; for unmodified sequence 1835, after modification using template DV26P, the resulting sequence D835-DV26P exhibited an inhibition rate of 74.619% against the potential off-target gene CCNA2.
   2) The template-modified sequences disclosed herein, when incorporating anti-off-target design, can reduce inhibition of off-target genes, thereby achieving anti-off-target effects, reducing inhibition of off-target genes by up to 72.535%.

### Example 24: Inhibitory effect of sequences modified using the templates disclosed herein on AGT in primary human hepatocytes

In this example, the inhibitory effects of the highly active sequences from Example 20 on potential off-target genes in primary human hepatocytes were evaluated, and compared them with Zilebesiran, a drug currently in Phase II clinical trials. Five sequences, namely C1576s-DV29SP, C1578s-DV29SP, C1579-DV25P, C1812-DV25P, and C1585s-DV27SP5, were synthesized using their respective modification templates or incorporating both template and anti-off-target modifications, and conjugated with GalNAc to enable free uptake by hepatocytes. The sequences listed in Table 88 were applied to primary human hepatocytes at concentrations ranging from 0.0064 nM to 100 nM via free uptake. Dose-response curves were fitted, and the IC₅₀ values of these sequences were calculated. The results demonstrated that the IC₅₀ values of some sequences were superior to that of the positive control Zilebesiran. For example, D576s-DV29SPG5 exhibited an IC₅₀ value of 4.698 nM, outperforming that of the positive control Zilebesiran at 7.071 nM.

### 1. Experimental materials

### 1) Test samples:

The sequences listed in Table 88, including those incorporating template modification alone and those incorporating both template modification and anti-off-target design, wherein the 3' end of the sense strand of these sequences is conjugated with GalNAc ligand G5:

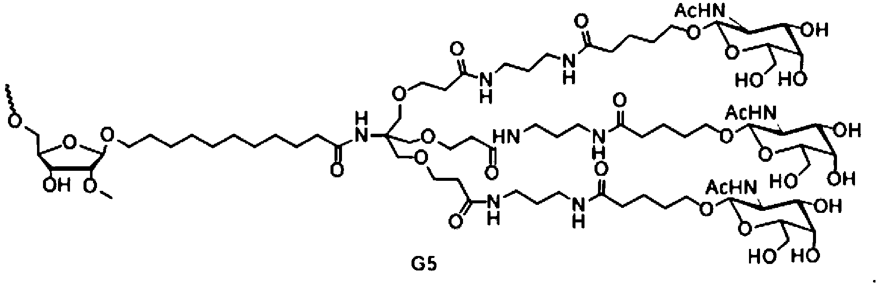

The conjugation method between oligonucleotides and ligand G5 was the same as that described for conjugates 4, 5, 6, and 7 in Example 19 of patent application CN116854754A, *i.e.*, conjugating YK-GAL-304, YK-GAL-305, YK-GAL-306, and YK-GAL-307 with oligonucleotides. YK-GAL-304, YK-GAL-305, YK-GAL-306, and YK-GAL-307 were synthesized as in Example 17 of CN116854754A.

The resulting oligonucleotide-ligand conjugate is shown below:

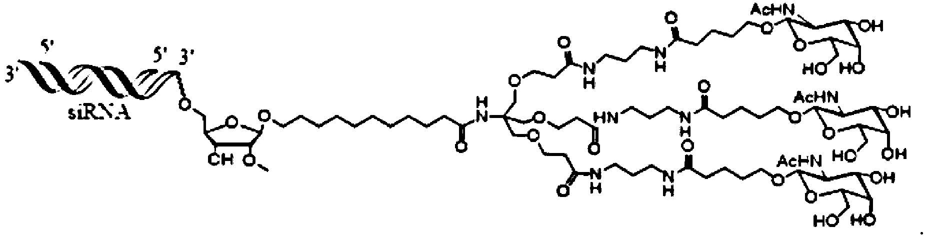

The specific sequences are shown in Table 88. In sequence identifiers, G5 indicates conjugation with GalNAc ligand G5, and GL indicates conjugation with GalNAc ligand L96.

**Table 88: Template-modified sequences**

| Sequence ID | **Sense strand 5'-3'** | Modified SEQ ID NO:/corr. unmodified SEQ ID NO: | **Antisense strand 5'-3'** | Modified SEQ ID NO:/corr. unmodified SEQ ID NO: |
|---|---|---|---|---|
| D579-DV25PG5 | Cms-Cms-Um-Um-Um-Um-Cf- Jm-Uf-Cf-Uf-Am-Am-Um-Gm- Am-Gf-Um-Cm-Gm-Am-G5 | 1314/788 | UmsEVP-Cfs-Gm-Am-Cm-Uf-Cm- Am-Um-Um-Am-Gm-Am-Af-Gm- Af-Am-Am-Am-Gm-Gms-Ums-Gm | 1241/801 |
| D579-DV25Pd7B G5 | Cms-Cms-Um-Um-Um-Um-Cf- Um-Uf-Cf-Uf-Am-Am-Um-Gd- Am-Gf-Um-Cm-Gm-Am-G5 | 1315/788 | UmsEVP-Cfs-Gm-Am-Cm-Uf-Cd- Am-Um-Um-Am-Gm-Am-Af-Gm- Af-Am-Am-Am-Gm-Gms-Ums-Gm | 1354/801 |
| D579-DV25Pd67 BG5 | Cms-Cms-Um-Um-Um-Um-Cf- Um-Uf-Cf-Uf-Am-Am-Um-Gd- Ad-Gf-Um-Cm-Gm-Am-G5 | 1316/788 | UmsEVP-Cfs-Gm-Am-Cm-Td-Cd- Am-Um-Um-Am-Gm-Am-Af-Gm- Af-Am-Am-Am-Gm-Gms-Ums-Gm | 1355/801 |
| D812-DV25PG5 | Cms-Cms-Um-Um-Um-Um-Cf- Am-Af-Gf-Uf-Um-Gm-Am-Gm- Am-Af-Cm-Am-Am-Am-G5 | 1325/787 | UmsEVP-Ufs-Um-Gm-Um-Uf-Cm- Um-Cm-Am-Am-Cm-Um-Uf-Gm- Af-Am-Am-Am-Gm-Gms-Gms-Am | 1234/800 |
| D576s-DV29SPG5 | Cms-Cms-Am-Cm-Cm-Uf-Um- Uf-Um-Cf-Um-Um-Cm-Um- Am-Af-Um-Gm-Am-Am-G5 | 1338/795 | UmsEVP-Ufs-Cm-Af-Uf-Uf-Am- Gm-Am-Am-Gm-Am-Am-Af-Am- Gf-Gm-Um-Gm-Gms-Gms-Am | 1272/808 |
| D578s-DV29SPd7 BG5 | Ams-Cms-Cm-Um-Um-Uf-Um- Cf-Um-Uf-Cm-Um-Am-Ad- Um-Gf-Am-Gm-Um-Am-G5 | 1342/796 | UmsEVP-Afs-Cm-Uf-Cf-Af-Td- Um-Am-Gm-Am-Am-Gm-Af-Am- Af-Am-Gm-Gm-Ums-Gms-Gm | 1353/809 |
| D585s-DV27SPG5 | Cms-Ums-Um-Cm-Um-Af-Am- Uf-Gf-Af-Gm-Um-Cm-Gm-Am- Cf-Um-Um-Um-Am-G5 | 1344/794 | UmsEVP-Afs-Am-Af-Gf-Uf-Cm- Gm-Am-Cm-Um-Cm-Am-Uf-Um- Af-Gm-Am-Am-Gms-Ams-Am | 1267/807 |
| Zilebesiran | Gms-Ums-Cm-Am-Um-Cm-Cf- Am-Cf-Af-Af-Um-Gm-Am-Gm- Am-Gm-Um-Am-Cm-Am-GL | 1345/1291 | Ums-Gfs-Um-Am-Cm-Tgna-Cm- Um-Cm-Am-Um-Um-Gm-Uf-Gm- Gf-Am-Um-Gm-Am-Cms-Gms-Am | 1371/1304 |

Cell line: primary human hepatocytes, provided by Liver Biotechnology Co., Ltd. (LV-PHH001).

Solvents: sterile nuclease-free water, primary human hepatocyte maintenance medium (LV-WEM001).

### 2. Experimental methods

The inhibitory effects of the test samples on AGT mRNA expression in primary human hepatocytes were evaluated by qRT-PCR.

### 2.1 Thawing and culturing of primary human hepatocytes

The culture medium was pre-warmed at a constant temperature of 37°C for at least 30 minutes. 300 µL of PBS was added to each well of a collagen-coated 24-well plate, which was shaken several times, and the PBS was aspirated. A cryovial containing hepatocytes was removed from liquid nitrogen, placed in a 37°C water bath, and gently shaken until only a small amount of ice crystals remained in the vial. The cell suspension was poured into thawing medium in one step. 1 mL of thawing medium was pipetted to rinse the inner wall of the cryovial 2 to 3 times. The cell suspension in thawing medium was mixed by inversion. The vial was centrifuged at 50 g for 5 minutes at room temperature, with acceleration set to 5 and deceleration set to 3. The cell suspension was diluted to 3 × 10⁵ cells/mL in seeding medium, seeded into a 24-well plate at 0.5 mL per well, followed by incubation in an incubator for 24 hours.

### 2.2 Free uptake by primary human hepatocytes

The test samples were diluted to 100 to 0.0064 nM in primary human hepatocyte maintenance medium. After incubation, the primary human hepatocytes were removed, and the seeding medium was aspirated. 500 µL of diluted test sample was added to each well. The plate was returned to the cell incubator at 37°C with 5% CO₂ and incubated for an additional 40 hours.

### 2.3 RNA extraction and reverse transcription

At 24 hours post-transfection, the culture medium was removed and the cells were collected for RNA extraction. Total RNA was extracted using the RNeasy^{®} 96 Kit (QIAGEN, 74182) according to the instructions provided by the manufacturer. Subsequently, cDNA was synthesized using the FastKing RT Kit (with gDNase) (TIANGEN, KR116-02) according to the instructions provided by the manufacturer.

### 2.4 RT-qPCR

Refer to "Quantitative analysis of AGT mRNA" in Section 16.3 of Example 18.

### 2.5 Data analysis

The RNA expression levels of the target gene in each sample were calculated based on Ct values using the ΔΔCt relative quantification method. Relative expression of the target gene was expressed as 2^{-ΔΔCt}.

The calculation formula is as follows:
$ΔCt = mean Ct value of the target gene - mean Ct value of the reference gene ;$
$ΔΔCt = ΔCt \left(treatment group\right) - ΔCt \left(RNAiMAX control group\right) ;$

Relative mRNA expression of the target gene = 2^{-ΔΔCt}; 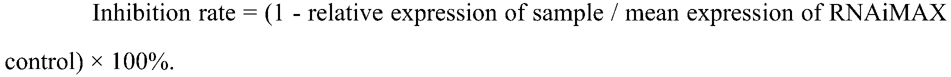 Inhibition rate = (1 - relative expression of sample / mean expression of RNAiMAX control) × 100%.

### 3. Experimental results

The inhibition rates of these sequences against AGT at various concentrations are shown in Table 89 below. All sequences exhibited significant inhibitory effects against AGT. For example, D576s-DV29PG5 exhibited an inhibition rate of 83.1% against AGT at 100 nM. In addition, the IC₅₀ values of some sequences were superior to that of the positive control Zilebesiran. For example, D576s-DV29PG5 exhibited an IC₅₀ value of 4.698 nM, outperforming that of the positive control Zilebesiran at 7.071 nM.

**Table 89: Inhibition rates and IC₅₀ values of sequences against AGT at various concentrations**

| **Sequence name** | **AGT inhibition rate (%)** | | | | | | | **IC₅₀ (nM)** |
|---|---|---|---|---|---|---|---|---|
| | **100 nM** | **20 nM** | **4 nM** | **0.8 nM** | **0.16 nM** | **0.032 nM** | **0.0064 nM** | |
| Zilebesiran | 65.5 | 57.8 | 42.8 | 22.6 | 9.9 | 5.2 | 14.9 | 7.071 |
| D579-DV25Pd7BG5 | 71.7 | 58.8 | 63.2 | 12.7 | 2.1 | -5.5 | -6.2 | 1.998 |
| D576s-DV29SPG5 | 83.1 | 66.8 | 46.4 | -0.5 | 3.0 | -1.2 | -2.8 | 4.698 |
| D576s-DV29SPG5 | 74.4 | 63.3 | 45.4 | 10.9 | 7.8 | 1.4 | 1.1 | 5.659 |
| D812-DV25PG5 | 61.5 | 43.0 | 17.3 | -7.7 | -12.5 | -27.5 | -13.0 | 30.720 |
| D578s-DV29SPd7BG5 | 56.9 | 47.5 | 33.9 | 13.5 | -3.8 | -8.7 | 2.5 | 20.470 |
| D585s-DV27SPG5 | 76.1 | 73.3 | 42.0 | 19.9 | 24.4 | 2.3 | -12.2 | 5.719 |
| D579-DV25PG5 | 72.6 | 60.2 | 32.9 | 12.5 | -0.9 | 6.3 | 8.6 | 9.818 |

### Example 25: Inhibitory effect of sequences modified using the templates disclosed herein on serum AGT in mice

In this example, several sequences were exemplarily selected, including unmodified sequences 1576s, 1578s, 1579, 1585s, 1789, 1791, 1795, 1812, 1816, 1835, 1838, and 1839. These sequences were modified, for example, by incorporating template modification alone, or both template modification and anti-off-target design. The positive control was Zilebesiran, an siRNA drug currently in Phase II clinical trials. Using transgenic mice expressing the human AGT gene, the inhibitory effects of these sequences on serum AGT at different time points were evaluated by ELISA.

### 1. Experimental materials

### Test compounds:

The sequences listed in Table 90, including those incorporating template modification alone and those incorporating both template modification and anti-off-target design, wherein the 3' end of the sense strand of these sequences is conjugated with GalNAc ligand G5:

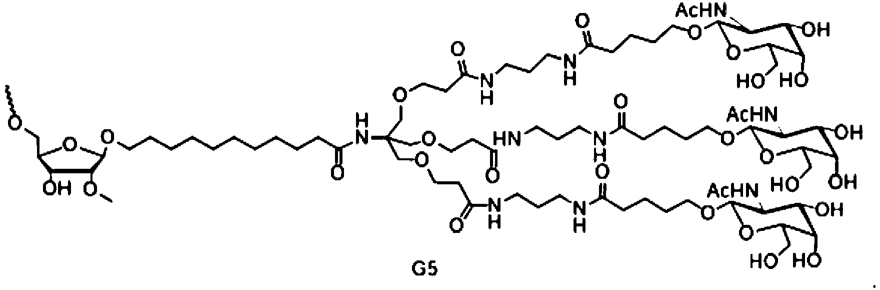

The conjugation method between oligonucleotides and ligand G5 was the same as that described for conjugates 4, 5, 6, and 7 in Example 19 of patent application CN116854754A, *i.e.,* conjugating YK-GAL-304, YK-GAL-305, YK-GAL-306, and YK-GAL-307 with oligonucleotides. YK-GAL-304, YK-GAL-305, YK-GAL-306, and YK-GAL-307 were synthesized as in Example 17 of CN116854754A.

The resulting oligonucleotide-ligand conjugate is shown below:

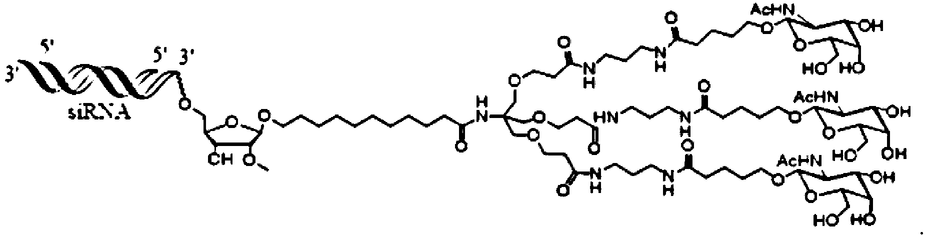

The specific sequences are shown in Table 90. In sequence identifiers, G5 indicates conjugation with GalNAc ligand G5, and GL indicates conjugation with GalNAc ligand L96.

The structure of G5 is as follows: 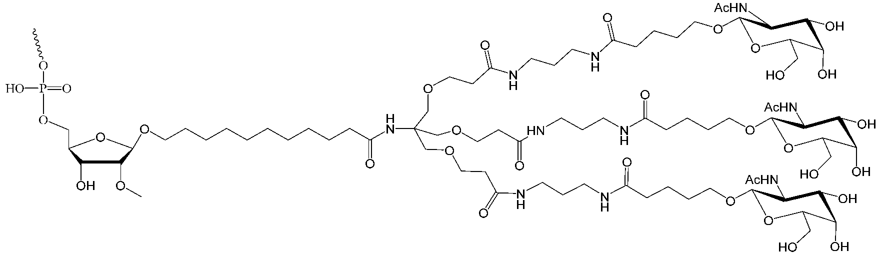
[[(1R,2R,3R,4R)-1-[[28-[[2-(acetylamino)-2-deoxy-β-D-galactopyranosyl]oxy]-13,13-bis[[3-[[3-[[5-[[2-(acetylamino)-2-deoxy-β-D-galactopyranosyl]oxy]-1-oxopentyl]amino]propyl]amino]-3-oxopropoxy]methyl]-11,18,24-trioxo-15-oxa-12,19,23-triazaoctacosan-1-yl]oxy]-2-O-methyl-5-β-D-ribofuranosyl]hydrogen phosphate]
[[(1R,2R,3R,4R)-1-[[28-[[2-(acetylamino)-2-deoxy-β-D-galactopyranosyl]oxy]-13,13-bis[[3-[[3-[[5-[[2-(acetylamino)-2-deoxy-β-D-galactopyranosyl]oxy]-1-oxopentyl]amino]propyl]amino]-3-oxopropoxy]methyl]-11,18,24-trioxo-15-oxa-12,19,23-triazaoctacosan-1-yl]oxy]-2-O-methyl-5-β-D-ribofuranosyl]hydrogen phosphate]

The structure of GL is as follows: 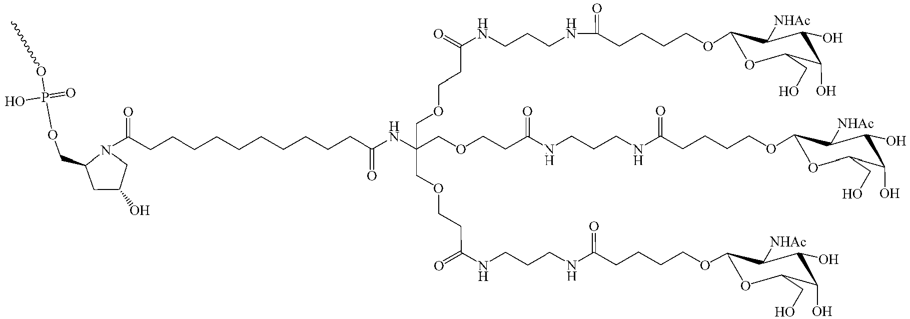
[(2S,4R)-1-[29-[[2-(acetylamino)-2-deoxy-β-D-galactopyranosyl]oxy]-14,14-bis[[3-[[3-[[5-[[2-(acetylamino)-2-deoxy-β-D-galactopyranosyl]oxy]-1-oxopentyl]amino]propyl]amino]-3-oxopropoxy]methyl]-1,12,19,25-tetraoxo-16-oxa-13,20,24-triazanonacos-1-yl]-4-hydroxy-2-pyrrolidinyl]methyl hydrogen phosphate]
[(2S,4R)-1-[29-[[2-(acetylamino)-2-deoxy-β-D-galactopyranosyl]oxy]-14,14-bis[[3-[[3-[[5-[[2-(acetylamino)-2-deoxy-β-D-galactopyranosyl]oxy]-1-oxopentyl]amino]propyl]amino]-3-oxopropoxy]methyl]-1,12,19,25-tetraoxo-16-oxa-13,20,24-triazanonacos-1-yl]-4-hydroxy-2-pyrrolidinyl]methyl hydrogen phosphate]

**Table 90: Sequences used in animal experiments**

| Sequence ID | **Sense strand 5'-3'** | Modified SEQ ID NO:/corr. unmodified SEQ ID NO: | **Antisense strand 5'-3'** | Modified SEQ ID NO:/corr. unmodified SEQ ID NO: |
|---|---|---|---|---|
| D579-DV25PG5 | Cms-Cms-Um-Um-Um-Um-Cf- Um-Uf-Cf-Uf-Am-Am-Um-Gm- Am-Gf-Um-Cm-Gm-Am-G5 | 1314/788 | UmsEVP-Cfs-Gm-Am-Cm-Uf-Cm- Am-Um-Um-Am-Gm-Am-Af-Gm- Af-Am-Am-Am-Gm-Gms-Ums-Gm | 1241/801 |
| D579-DV25Pd7B G5 | Cms-Cms-Um-Um-Um-Um-Cf- Um-Uf-Cf-Uf-Am-Am-Um-Gd- Am-Gf-Um-Cm-Gm-Am-G5 | 1315/788 | UmsEVP-Cfs-Gm-Am-Cm-Uf-Cd- Am-Um-Um-Am-Gm-Am-Af-Gm- Af-Am-Am-Am-Gm-Gms-Ums-Gm | 1354/801 |
| D789-DV26PG5 | Gms-Ums-Um-Um-Gm-Um-Gf- Am-Af-Af-Cf-Am-Am-Am-Am- Am-Af-Gm-Um-Gm-Um-G5 | 1317/790 | AmsEVP-Cfs-Af-Cf-Um-Uf-Um- Um-Um-Um-Gm-Um-Um-Uf-Cm- Af-Cm-Am-Am-Am-Cms-Ams-Am | 1249/803 |
| D789-DV25PG5 | Gms-Ums-Um-Um-Gm-Um-Gf- Am-Af-Af-Cf-Am-Am-Am-Am- Am-Af-Gm-Um-Gm-Um-G5 | 1317/790 | AmsEVP-Cfs-Am-Cm-Um-Uf-Um- Um-Um-Um-Gm-Um-Um-Uf-Cm- Af-Cm-Am-Am-Am-Cms-Ams-Am | 1252/803 |
| D789-DV26Pd7B G5 | Gms-Ums-Um-Um-Gm-Um-Gf- Am-Af-Af-Cf-Am-Am-Am-Ad- Am-Af-Gm-Um-Gm-Um-G5 | 1318/790 | AmsEVP-Cfs-Af-Cf-Um-Uf-Td- Um-Um-Um-Gm-Um-Um-Uf-Cm- Af-Cm-Am-Am-Am-Cms-Ams-Am | 1356/803 |
| D789-DV25Pd7B G5 | Gms-Ums-Um-Um-Gm-Um-Gf- Am-Af-Af-Cf-Am-Am-Am-Ad- Am-Af-Gm-Um-Gm-Um-G5 | 1318/790 | AmsEVP-Cfs-Am-Cm-Um-Uf-Td- Um-Um-Um-Gm-Um-Um-Uf-Cm- Af-Cm-Am-Am-Am-Cms-Ams-Am | 1357/803 |
| D791-DV26PG5 | Ums-Ums-Gm-Um-Gm-Am-Af- Am-Cf-Af-Af-Am-Am-Am-Am- Gm-Uf-Gm-Um-Um-Am-G5 | 1320/792 | UmsEVP-Afs-Af-Cf-Am-Cf-Um- Um-Um-Um-Um-Um-Gm-Uf-Um- Uf-Cm-Am-Cm-Am-Ams-Ams-Cm | 1260/805 |
| D791-DV26Pd7B G5 | Ums-Ums-Gm-Um-Gm-Am-Af- Am-Cf-Af-Af-Am-Am-Am-Ad- Gm-Uf-Gm-Um-Um-Am-G5 | 1321/792 | UmsEVP-Afs-Af-Cf-Am-Cf-Td- Um-Um-Um-Um-Um-Gm-Uf-Um- Uf-Cm-Am-Cm-Am-Ams-Ams-Cm | 1360/805 |
| D795-DV27PG5 | Gms-Ams-Am-Am-Cm-Am-Af- Am-Af-Af-Af-Gm-Um-Gm-Um- Um-Cf-Cm-Cm-Um-Um-G5 | 1323/793 | AmsEVP-Afs-Gm-Gf-Gf-Af-Am- Cm-Am-Cm-Um-Um-Um-Uf-Um- Uf-Gm-Um-Um-Um-Cms-Ams-Cm | 1263/806 |
| D812-DV25PG5 | Cms-Cms-Um-Um-Um-Um-Cf- Am-Af-Gf-Uf-Um-Gm-Am-Gm- Am-Af-Cm-Am-Am-Am-G5 | 1325/787 | UmsEVP-Ufs-Um-Gm-Um-Uf-Cm- Um-Cm-Am-Am-Cm-Um-Uf-Gm- Af-Am-Am-Am-Gm-Gms-Gms-Am | 1234/800 |
| D816-DV25PG5 | Ums-Ums-Cm-Am-Am-Gm-Uf- Um-Gf-Af-Gf-Am-Am-Cm-Am- Am-Af-Am-Am-Um-Um-G5 | 1327/786 | AmsEVP-Afs-Um-Um-Um-Uf-Um- Gm-Um-Um-Cm-Um-Cm-Af-Am- Cf-Um-Um-Gm-Am-Ams-Ams-Am | 1227/799 |
| D835-DV26PG5 | Ums-Ums-Gm-Gm-Gm-Um-Uf- Um-Uf-Af-Af-Am-Am-Um-Um- Am-Af-Am-Gm-Um-Am-G5 | 1329/785 | UmsEVP-Afs-Cf-Uf-Um-Uf-Am- Am-Um-Um-Um-Um-Am-Af-Am- Af-Cm-Cm-Cm-Am-Ams-Ums-Um | 1217/798 |
| D835-DV26Pd7B G5 | Ums-Ums-Gm-Gm-Gm-Um-Uf- Um-Uf-Af-Af-Am-Am-Um-Td- Am-Af-Am-Gm-Um-Am-G5 | 1330/785 | UmsEVP-Afs-Cf-Uf-Um-Uf-Ad- Am-Um-Um-Um-Um-Am-Af-Am- Af-Cm-Cm-Cm-Am-Ams-Ums-Um | 1349/798 |
| D838-DV29PG5 | Gms-Gms-Um-Um-Um-Um-Af- Am-Af-Am-Uf-Um-Am-Am- Am-Gm-Uf-Am-Um-Am-Am-G5 | 1332/797 | UmsEVP-Ufs-Am-Uf-Af-Cf-Um- Um-Um-Am-Am-Um-Um-Uf-Um- Af-Am-Am-Am-Cm-Cms-Cms-Am | 1280/810 |
| D838-DV29Pd7B G5 | Gms-Gms-Um-Um-Um-Um-Af- Am-Af-Am-Uf-Um-Am-Am-Ad- Gm-Uf-Am-Um-Am-Am-G5 | 1333/797 | UmsEVP-Ufs-Am-Uf-Af-Cf-Td- Um-Um-Am-Am-Um-Um-Uf-Um- Af-Am-Am-Am-Cm-Cms-Cms-Am | 1365/810 |
| D839-DV29PG5 | Gms-Ums-Um-Um-Um-Am-Af- Am-Af-Um-Uf-Am-Am-Am- Gm-Um-Af-Um-Am-Cm-Am-G5 | 1335/791 | UmsEVP-Gfs-Um-Af-Uf-Af-Cm- Um-Um-Um-Am-Am-Um-Uf-Um- Uf-Am-Am-Am-Am-Cms-Cms-Cm | 1255/804 |
| D839-DV29Pd7B G5 | Gms-Ums-Um-Um-Um-Am-Af- Am-Af-Um-Uf-Am-Am-Am-Gd- Um-Af-Um-Am-Cm-Am-G5 | 1336/791 | UmsEVP-Gfs-Um-Af-Uf-Af-Cd- Um-Um-Um-Am-Am-Um-Uf-Um- Uf-Am-Am-Am-Am-Cms-Cms-Cm | 1367/804 |
| D576s-DV29SPG5 | Cms-Cms-Am-Cm-Cm-Uf-Um- Uf-Um-Cf-Um-Um-Cm-Um-Am- Af-Um-Gm-Am-Am-G5 | 1338/795 | UmsEVP-Ufs-Cm-Af-Uf-Uf-Am- Gm-Am-Am-Gm-Am-Am-Af-Am- Gf-Gm-Um-Gm-Gms-Gms-Am | 1272/808 |
| D576s-DV29SPd7 BG5 | Cms-Cms-Am-Cm-Cm-Uf-Um- Uf-Um-Cf-Um-Um-Cm-Td-Am- Af-Um-Gm-Am-Am-G5 | 1339/795 | UmsEVP-Ufs-Cm-Af-Uf-Uf-Ad- Gm-Am-Am-Gm-Am-Am-Af-Am- Gf-Gm-Um-Gm-Gms-Gms-Am | 1369/808 |
| D578s-DV29SPG5 | Ams-Cms-Cm-Um-Um-Uf-Um- Cf-Um-Uf-Cm-Um-Am-Am-Um- Gf-Am-Gm-Um-Am-G5 | 1341/796 | UmsEVP-Afs-Cm-Uf-Cf-Af-Um- Um-Am-Gm-Am-Am-Gm-Af-Am- Af-Am-Gm-Gm-Ums-Gms-Gm | 1276/809 |
| D578s-DV29SPd7 BG5 | Ams-Cms-Cm-Um-Um-Uf-Um- Cf-Um-Uf-Cm-Um-Am-Ad-Um- Gf-Am-Gm-Um-Am-G5 | 1342/796 | UmsEVP-Afs-Cm-Uf-Cf-Af-Td- Um-Am-Gm-Am-Am-Gm-Af-Am- Af-Am-Gm-Gm-Ums-Gms-Gm | 1353/809 |
| D585s-DV27SPG5 | Cms-Ums-Um-Cm-Um-Af-Am- Uf-Gf-Af-Gm-Um-Cm-Gm-Am- Cf-Um-Um-Um-Am-G5 | 1344/794 | UmsEVP-Afs-Am-Af-Gf-Uf-Cm- Gm-Am-Cm-Um-Cm-Am-Uf-Um- Af-Gm-Am-Am-Gms-Ams-Am | 1267/807 |
| Zilebesiran | Gms-Ums-Cm-Am-Um-Cm-Cf- Am-Cf-Af-Af-Um-Gm-Am-Gm- Am-Gm-Um-Am-Cm-Am-GL | 1345/1291 | Ums-Gfs-Um-Am-Cm-Tgna-Cm- Um-Cm-Am-Um-Um-Gm-Uf-Gm- Gf-Am-Um-Gm-Am-Cms-Gms-Am | 1371/1304 |
| DNC-DV29PG5 | Gms-Ums-Gm-Am-Am-Am-Cf- Am-Af-Cm-Uf-Am-Gm-Um- Gm-Cm-Af-Cm-Cm-Um-Cm-G5 | 1346/NA | UmsEVP-Afs-Gm-Af-Gf-Gf-Um- Gm-Cm-Am-Cm-Um-Am-Gf-Um- Uf-Gm-Um-Um-Um-Cms-Ams-Cm | 1372/NA |

### Preparation of test compounds:

Solvent: saline.

Preparation conditions: sterile environment.

Labeling method: each prepared dosage form was labeled with tags, with the outer packaging indicating the project number, name, concentration, quantity, preparation date, preparer, and storage conditions.

Storage conditions: freshly prepared before use; remaining samples stored at -80°C.

### Information on experimental animals:

Species/strain: hAGT transgenic mice
Grade: SPF
Sex: Male
Quantity: 150
Age: 6 to 8 weeks
Weight: 18 to 28 g
Source: Jiangsu GemPharmatech Co., Ltd.
Production license No.: SCXK (Su) 2018-0008

### Review by Institutional Animal Care and Use Committee (IACUC):

Upon arrival, the experimental animals were housed at Beijing BrightShines Technology Co., Ltd., under a project license No.: SYXK (Jing) 2022-0025. The project was approved by the IACUC of Beijing BrightShines Technology Co., Ltd. The experimental procedures were strictly conducted in compliance with IACUC requirements to ensure animal welfare.

### Housing and management:

Housing conditions: Upon arrival, the experimental animals were housed at Beijing BrightShines Technology Co., Ltd., under a project license No.: SYXK (Jing) 2022-0025. The animals were housed in rearing cages with dimensions of 29.0 cm (L) × 18.5 cm (W) × 13.0 cm (H). The environmental parameters were set as follows: a temperature ranging from 20°C to 26°C, a humidity ranging from 40% to 70%, a ventilation rate of at least 15 fresh air changes per hour, and a 12-hour light/12-hour dark cycle with artificial lighting.

The environmental conditions complied with the National Standard GB14925-2010 of the People's Republic of China, with environmental control provided by a modular air-conditioning system.

The animals had free access to food and water. Water bottles and drinking water in the bottles were replaced at least twice weekly. Used water bottles were sterilized in a pulse vacuum autoclave before reuse.

The cages and bedding were replaced at least once weekly. All cages and bedding were sterilized in a pulse vacuum autoclave before being introduced into a barrier environment. Cage racks were cleaned and disinfected at least once weekly.

Animal observation rooms were cleaned and disinfected daily, including shelves, floors, work surfaces, *etc*.

Disinfectants used in the barrier environment included: 6.67% benzalkonium bromide solution, 0.5% 84 disinfectant, 75% ethanol, and 0.08% didecyldimethylammonium bromide. These four disinfectants should be used alternately and should not be used in combination.

Animal feed: Maintenance diet for SPF-grade mice and rats: produced by SPF (Beijing) Biotechnology Co., Ltd.; production license No.: SCXK (Jing) 2019-0010, issued by the Beijing Municipal Science and Technology Commission.

Feed testing: Each batch of feed had a quality certificate. Microbial testing was conducted quarterly by the company. A recent third-party feed inspection report was provided semiannually by the supplier. Nutrient composition testing complied with the National Standard GB14924.3-2010 of the People's Republic of China, while contaminant testing complied with the National Standard GB14924.2-2001 of the People's Republic of China.

Water for experimental animals: Drinking water: sterile water prepared by a filtration system, directly filled into drinking bottles.

Drinking water testing: Microbial testing was conducted quarterly by the company. Water quality testing was conducted annually by a third-party testing agency. Drinking water testing complied with the National Standard GB5749-2006 of the People's Republic of China.

Animal bedding: Corncob bedding: produced by SPF (Beijing) Biotechnology Co., Ltd.; production license No.: SCXK (Jing) 2019-0004, issued by the Beijing Municipal Science and Technology Commission.

Bedding testing: Microbial testing was conducted quarterly by the company. At least one recent third-party bedding inspection report was provided semiannually by the supplier. Bedding testing complied with the National Standard GB14924.2-2001 of the People's Republic of China.

### 2. Experimental methods

### Dosage design and grouping

Definition of trial date: The day on which the animals were administered solvent or test compound was defined as Day 0.

Grouping and dosing: After 3 days of acclimation, the experimental animals were randomly divided into a negative control group and a treatment group based on serum AGT levels, with 6 animals per group. The test compounds were administered at a dose of 3 mg/kg via a single subcutaneous injection, with a dosing volume of 5 mL/kg and a dosing concentration of 0.6 mg/mL. The day of administration was designated as Day 0.

Individual animals were identified by ear tags. Cages were identified by hanging cage cards. A laboratory signage was hung at the entrance of the laboratory. Group information is shown in Table 91 below.

**Table 91: Group information**

| **Group** | **Test compound** | **Dose (mg/kg)** | **Route/frequency of administration** | **Duration (days)** | **Number of animals** |
|---|---|---|---|---|---|
| Negative control group | Solvent | / | / | 56 | 6 |
| Treatment group | siRNA | 3 | s.c./once | 56 | 6 |

### Measurement indicators

### (1) General observations

Animals were observed once daily from one week prior to administration until the end of the study.

Cage-side observation parameters included mortality or moribund status, mental state, behavior activities, fecal characteristics, food and water supply, *etc*.

Test animals: all animals in both the negative control group and the treatment group.

### (2) Serum AGT expression

Time points for measurement: Day -3 (prior to dosing), Day 7 (1 week post-dosing), Day 14 (2 weeks post-dosing), Day 21 (3 weeks post-dosing), Day 28 (4 weeks post-dosing), and Day 35 (5 weeks post-dosing).

Method for measuring AGT protein levels: measured using an ELISA kit; repeated freeze-thaw cycles of serum samples were avoided.

Test animals: all animals in both the negative control group and the treatment group.

### (4) Data processing and statistical analysis

Data are expressed as mean ± standard deviation (Mean ± SD) and analyzed using GraphPad Prism 8.3 software. Statistical analysis was performed using two-way ANOVA followed by post hoc analysis, including the LSD test for homogeneity of variance and Dunnett's T3 test for unequal variance. P < 0.05 was considered statistically significant.

### 3. Experimental results

The detailed experimental results are shown in Table 92.

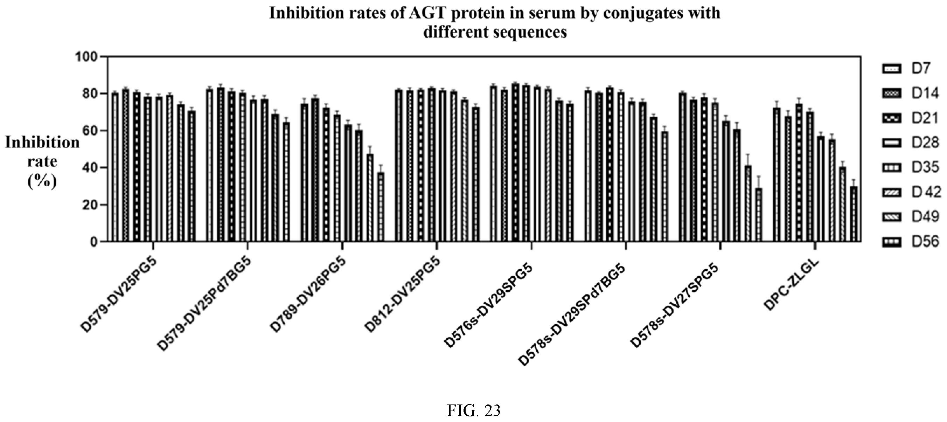

It can be seen that these sequences exhibited sustained and significant inhibition of serum AGT expression. For example, D576s-DV29PG5 exhibited inhibition rates of 84.09%, 82.11%, and 85.25% on Day 7, Day 14, and Day 28, respectively.
(1) The sequences disclosed herein, such as unmodified sequences 1579, 1789, 1812, 1576s, 1578s, and 1585s, when subjected to different modifications, exhibited significant inhibitory effects on serum AGT expression. For example, D576s-DV29SPG5 exhibited inhibition rates of 84.09%, 82.11%, and 85.25% on Day 7, Day **14,** and Day 21, respectively (FIG. 23).

**Table 92: Inhibition rates of serum AGT expression by different sequences**

| **Unmodified sequence ID** | **Sequence ID** | **Inhibition rate (%)** | | | | | | | |
|---|---|---|---|---|---|---|---|---|---|
| | | **D7** | **D14** | **D21** | **D28** | **D35** | **D42** | **D49** | **D56** |
| | Saline | -12.05 | -4.17 | -14.50 | -17.18 | -6.21 | -5.65 | -13.98 | -8.02 |
| | DNC-DV29PG5 | 4.14 | -17.92 | 9.78 | 3.04 | | | | |
| | Zilebesiran | 72.52 | 67.96 | 74.49 | 70.53 | 57.13 | 55.50 | 40.41 | 29.90 |
| 1576s | D576s-DV29SPd7BG5 | 75.91 | 61.16 | 54.05 | 32.94 | | | | |
| 1576s | D576s-DV29SPG5 | 84.09 | 82.11 | 85.25 | 84.61 | 83.67 | 82.63 | 76.08 | 74.43 |
| 1578s | D578s-DV29SPd7BG5 | 81.76 | 80.52 | 83.37 | 80.74 | 75.71 | 75.38 | 67.58 | 59.76 |
| 1578s | D578s-DV29SPG5 | 84.16 | 81.73 | 85.02 | 83.70 | | | | |
| 1579 | D579-DV25PG5 | 80.18 | 82.30 | 80.62 | 78.42 | 78.42 | 79.22 | 74.17 | 70.95 |
| 1579 | D579-DV25Pd7BG5 | 82.45 | 83.37 | 81.28 | 80.19 | 76.74 | 76.95 | 69.05 | 64.51 |
| 1585s | D585s-DV27SPG5 | 80.37 | 76.55 | 77.88 | 75.02 | 65.47 | 60.63 | 41.16 | 29.04 |
| 1789 | D789-DV25Pd7BG5 | 63.75 | 64.84 | 51.52 | 40.12 | | | | |
| 1789 | D789-DV26Pd7BG5 | 72.49 | 70.00 | 55.31 | 42.63 | | | | |
| 1789 | D789-DV25PG5 | 69.90 | 73.61 | 69.45 | 61.94 | | | | |
| 1789 | D789-DV26PG5 | 74.52 | 77.51 | 72.40 | 68.56 | 63.15 | 60.50 | 47.62 | 37.35 |
| 1791 | D791-DV26Pd7BG5 | 74.54 | 70.80 | 62.16 | 49.77 | | | | |
| 1791 | D791-DV26PG5 | 73.28 | 73.36 | 66.01 | 59.54 | | | | |
| 1795 | D795-DV27PG5 | 60.21 | 60.31 | 62.70 | 55.58 | | | | |
| 1812 | D812-DV25PG5 | 81.87 | 81.67 | 82.22 | 82.83 | 81.77 | 81.17 | 76.58 | 72.98 |
| 1816 | D816-DV25PG5 | 73.58 | 72.79 | 63.17 | 60.38 | | | | |
| 1835 | D835-DV26Pd7BG5 | 52.91 | 35.34 | 18.61 | 25.60 | | | | |
| 1835 | D835-DV26PG5 | 65.38 | 63.25 | 59.95 | 59.09 | | | | |
| 1838 | D838-DV29Pd7BG5 | 69.83 | 64.03 | 59.38 | 56.69 | | | | |
| 1838 | D838-DV29PG5 | 69.85 | 73.02 | 73.01 | 70.69 | | | | |
| 1839 | D839-DV29PG5 | 67.89 | 62.97 | 65.43 | 63.36 | | | | |
| 1839 | D839-DV29Pd7BG5 | 75.62 | 72.52 | 73.96 | 67.02 | | | | |

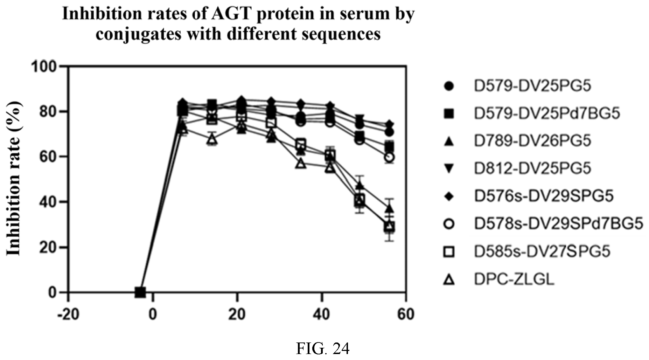

As shown in Table 92, the conjugates formed by conjugation of these sequences with GalNAc compounds exhibited significant inhibitory effects on serum AGT expression. For example, D576s-DV29SPG5 exhibited inhibition rates of 84.09%, 82.11%, and 85.25% on Day 7, Day 14, and Day 21, respectively (FIG. 24).

These findings indicate that the unmodified sequences designed in the present disclosure, such as 1579, 1789, 1812, 1576s, 1578s, and 1585s, when modified using the templates disclosed herein, or further incorporating anti-off-target design, and conjugated with GalNAc compounds, can be efficiently delivered to the liver in animals, significantly inhibiting AGT gene expression.

### Summary:

The unmodified sequences designed in the present disclosure, such as 1579, 1789, 1812, 1576s, 1578s, and 1585s, when modified using the templates disclosed herein, or further incorporating anti-off-target design, and conjugated with GalNAc compounds, can be efficiently delivered to the liver in animals, exhibiting sustained and significant inhibition of serum AGT expression.

For example, D576s-DV29SPG5 exhibited inhibition rates of 84.09%, 82.11%, and 85.25% on Day 7, Day 14, and Day 28, respectively.

### Conclusion:

In the present disclosure, a series of siRNAs were designed based on the AGT mRNA sequence, which were subjected to alternating modifications and modifications using a set of specific templates, with some of these sequences further incorporating anti-off-target design. The results demonstrated that:
(1) A total of 13 unmodified sequences exhibited significant inhibitory effects on AGT gene expression, with inhibition rates exceeding 45%, including 1576s, 1579, 1812, 1578s, 1789, 1835, 1838, 1795, 1585s, 1816, 1791, 1836, and 1839.
(2) The multiply modified sequences can achieve inhibition rates of up to greater than 70%. Furthermore, conjugation of the modified sequences with GalNAc compounds enables efficient delivery to the liver in animals, significantly inhibiting AGT gene expression.

### Specifically:

**1. The alternately modified sequences disclosed herein exhibited significant inhibitory effects on AGT gene expression.**
   (1) Among the 99 designed sequences, 33 sequences exhibited significant inhibitory effects AGT gene expression, with inhibition rates exceeding 40%. Ten of these sequences exhibited inhibition rates exceeding 60%.
   (2) A total of 23 sequences exhibited inhibition rates of 25% to 40% on AGT gene expression. For example, the inhibition rates of B734-AL and B994-AL were 39.2% and 37.3%, respectively.
   (3) A total of 43 sequences exhibited inhibition rates below 25% on AGT gene expression. For example, the inhibition rate of B1017-AL was only 23.1%.
   (4) siRNAs with similar sequences exhibited significant differences in activity. For example, the inhibition rate of B1365-AL was 40.4% higher than that of B1367-AL, indicating a significant improvement, despite a difference of only two bases at the terminal positions; the inhibition rate of B1816-AL was 30.5% higher than that of B1815-AL, indicating a significant improvement, despite a difference of only one base at the terminal position. Therefore, identifying oligonucleotide sequences with significant inhibitory activity from a vast number of candidate sequences designed against the AGT mRNA sequence is not straightforward and requires substantial creative effort.
**2. The unmodified sequences disclosed herein exhibited significant inhibitory effects on AGT gene expression.**
   (1) A total of 13 unmodified sequences exhibited significant inhibitory effects on AGT gene expression, with inhibition rates exceeding 45%, including 1576s, 1579, 1812, 1578s, 1789, 1835, 1838, 1795, 1585s, 1816, 1791, 1836, and 1839. Specifically, 1576s, 1578s, 1812, 1579, 1789, 1835, and 1838 exhibited inhibition rates exceeding 50%.
   (2) The other unmodified sequences exhibited inhibition rates below 45% on AGT gene expression. For example, the inhibition rates of 731 and 1011 were only 1.3% and 2.1%, respectively.
   (3) siRNAs with similar sequences exhibited significant differences in activity. For example, the inhibition rate of unmodified sequence 734 was 41.7% higher than that of unmodified sequence 731, indicating a significant improvement.
   (4) The effects of alternating modifications on activity varied across different sequences. For some sequences, the inhibition rate was significantly improved. For example, for unmodified sequence 731, the alternately modified sequence exhibited a 25.1% increase in inhibition rate compared to the unmodified sequence. For others, the improvement was minimal. For example, unmodified sequences 994, 1279, and 1591 exhibited little to no difference in inhibition rate between alternately modified and unmodified sequences.
**3. The sequences modified using the templates disclosed herein exhibited significant inhibitory effects on AGT gene expression.**
   (1) The identified unmodified sequences 1576s, 1578s, 1838, 1835, 1816, 1812, 1579, 1836, 1789, 1839, 1791, 1795, and 1585s, when modified using templates DV25P to 29P or DV25SP to 29SP designed in the present disclosure, exhibited significant inhibitory effects on AGT gene expression, with inhibition rates exceeding 40%. Specifically, C1812-DV25P, C1579-DV33P, and C1789-DV26P exhibited inhibition rates of 74.4%, 74.0%, and 73.6%, respectively.
   (2) The 12 sequences modified using templates DV25P to DV29P or DV25SP to DV29SP disclosed herein showed significantly enhanced inhibition of AGT gene expression as compared to the alternately modified sequences. For example, for unmodified sequence 1812, the inhibition rate of C1812-DV25P (the sequence modified using template DV25P) was 11.5% higher than that of the alternately modified sequence; for unmodified sequence 1791, the inhibition rate of C1791-DV26P (the sequence modified using template DV26P) was 8.5% higher than that of the alternately modified sequence.
   (3) The same sequence modified using templates DV25P to DV29P or DV25SP to DV29SP disclosed herein showed significantly enhanced inhibition of AGT gene expression as compared to modification using the templates disclosed in the prior art. For example, for unmodified sequence 1579, the sequence modified using template DV25P disclosed herein exhibited a 16.6% increase in inhibition rate compared to the sequence modified using the previously disclosed Advanced ESC template DV22.
   (4) The same sequence modified using templates DV25P to DV29P or DV25SP to DV29SP disclosed herein showed significantly enhanced inhibition of AGT gene expression as compared to modification using templates DV32P to DV34P or DV32SP to DV34SP disclosed herein. For example, for unmodified sequence 1812, the sequence modified using template DV25P disclosed herein exhibited a 20.2% increase in inhibition rate compared to the sequence modified using template DV33P disclosed herein.
   (5) The sequences modified using different templates varied greatly in activity. For example, when the unmodified sequence 1816 was modified using DV29P, the inhibition rate was increased by 30.0% compared to modification using DV34; when the unmodified sequence 1579 was modified using DV33P, the inhibition rate was increased by 29.8% compared to modification using DV28P. Thus, it is uncertain which modification template would confer high activity to a given siRNA sequence.
   (6) The 24 sequences exhibited IC₅₀ values ranging from 1.884 pM to 61.498 pM. Specifically, the IC₅₀ values for C1789-DV25P, C1812-DV25P, and C1789-DV26P were 1.884 pM, 2.160 pM, and 2.643 pM, respectively. The results demonstrated that these sequences effectively inhibited AGT gene expression at low concentrations.
**4. The alternately modified sequences and the sequences modified using the templates disclosed herein exhibited significantly enhanced AGT inhibitory activity compared to the unmodified sequences with minor differences disclosed in the prior art.**

Compared to the unmodified sequences with minor differences disclosed in the prior art, the unmodified sequences, alternately modified sequences, and template-modified sequences disclosed herein showed significantly enhanced inhibition of AGT gene expression, with an increase of up to 91%.
I. The unmodified sequences disclosed herein exhibited significantly enhanced activity compared to the similar unmodified sequences disclosed in the prior art. For example, the unmodified sequence 1835 disclosed herein exhibited a 37.7% increase in inhibition rate compared to the structurally similar 835P.
II. The alternately modified sequences disclosed herein exhibited significantly enhanced activity compared to the similar unmodified sequences disclosed in the prior art. For example, the alternately modified sequence B1812-AL disclosed herein exhibited a 20.4% increase in inhibition rate compared to the sequence 812P.
III. The sequences modified using the templates disclosed herein exhibited significantly enhanced activity compared to the similar unmodified sequences disclosed in the prior art. For example, the alternately modified sequence C1576s-DV29SP disclosed herein exhibited a 41.1% increase in inhibition rate compared to the sequence 576P.

**5. The alternately modified and template-modified sequences disclosed herein, when incorporating specific anti-off-target designs, exhibited significant inhibitory effects on AGT gene expression, showing significantly reduced inhibition of off-target genes.**
(1) All modified sequences exhibited significant inhibitory effects on AGT gene expression. For example, the template-modified sequence D576s-DV29SP exhibited an inhibition rate of 83.8%, while the sequence D576s-DV29SPd7B incorporating both template and anti-off-target modifications exhibited an inhibition rate of 85.4%.
(2) After adopting any one or more of the modification patterns disclosed herein followed by anti-off-target design, the resulting sequences exhibited significant anti-off-target effects on certain off-target genes, thereby reducing inhibition of off-target genes, *i.e.*, achieving anti-off-target effects, reducing inhibition of off-target genes by up to 72.535%.

### 6. The sequences modified using the templates disclosed herein exhibited significant inhibition of serum AGT expression in mice.

The unmodified sequences designed in the present disclosure, such as 1579, 1789, 1812, 1576s, 1578s, and 1585s, when modified using the templates disclosed herein, or further incorporating anti-off-target design, and conjugated with GalNAc compounds, can be efficiently delivered to the liver in animals, exhibiting sustained and significant inhibition of serum AGT expression.

For example, D576s-DV29SPG5 exhibited inhibition rates of 84.09%, 82.11%, and 85.25% on Day 7, Day 14, and Day 21, respectively.

### Sequence Listing

| **Sequence ID** | **Sense sequence 5'-3'** | **SEQ ID NO:** | **Antisense sequence 5'-3'** | **SEQ ID NO:** |
|---|---|---|---|---|
| 1835 | UUGGGUUUUAAAAUUAAAGUA | 785 | UACUUUAAUUUUAAAACCCAAUU | 798 |
| 1816 | UUCAAGUUGAGAACAAAAAUU | 786 | AAUUUUUGUUCUCAACUUGAAAA | 799 |
| 1812 | CCUUUUCAAGUUGAGAACAAA | 787 | UUUGUUCUCAACUUGAAAAGGGA | 800 |
| 1579 | CCUUUUCUUCUAAUGAGUCGA | 788 | UCGACUCAUUAGAAGAAAAGGUG | 801 |
| 1836 | UGGGUUUUAAAAUUAAAGUAU | 789 | AUACUUUAAUUUUAAAACCCAAU | 802 |
| 1789 | GUUUGUGAAACAAAAAAGUGU | 790 | ACACUUUUUUGUUUCACAAACAA | 803 |
| 1839 | GUUUUAAAAUUAAAGUAUACA | 791 | UGUAUACUUUAAUUUUAAAACCC | 804 |
| 1791 | UUGUGAAACAAAAAAGUGUUA | 792 | UAACACUUUUUUGUUUCACAAAC | 805 |
| 1795 | GAAACAAAAAAGUGUUCCCUU | 793 | AAGGGAACACUUUUUUGUUUCAC | 806 |
| 1585s | CUUCUAAUGAGUCGACUUUA | 794 | UAAAGUCGACUCAUUAGAAGAA | 807 |
| 1576s | CCACCUUUUCUUCUAAUGAA | 795 | UUCAUUAGAAGAAAAGGUGGGA | 808 |
| 1578s | ACCUUUUCUUCUAAUGAGUA | 796 | UACUCAUUAGAAGAAAAGGUGG | 809 |
| 1838 | GGUUUUAAAAUUAAAGUAUAA | 797 | UUAUACUUUAAUUUUAAAACCCA | 810 |
| 1008 | CUUCACUGAGAGCGCCUGCCU | 811 | AGGCAGGCGCUCUCAGUGAAGGG | 897 |
| 1011 | CACUGAGAGCGCCUGCCUGCU | 812 | AGCAGGCAGGCGCUCUCAGUGAA | 898 |
| 1014 | UGAGAGCGCCUGCCUGCUGCU | 813 | AGCAGCAGGCAGGCGCUCUCAGU | 899 |
| 1016 | AGAGCGCCUGCCUGCUGCUGA | 814 | UCAGCAGCAGGCAGGCGCUCUCA | 900 |
| 1017 | GAGCGCCUGCCUGCUGCUGAU | 815 | AUCAGCAGCAGGCAGGCGCUCUC | 901 |
| 1020 | CGCCUGCCUGCUGCUGAUCCA | 816 | UGGAUCAGCAGCAGGCAGGCGCU | 902 |
| 1024 | UGCCUGCUGCUGAUCCAGCCU | 817 | AGGCUGGAUCAGCAGCAGGCAGG | 903 |
| 1026 | CCUGCUGCUGAUCCAGCCUCA | 818 | UGAGGCUGGAUCAGCAGCAGGCA | 904 |
| 1028 | UGCUGCUGAUCCAGCCUCACU | 819 | AGUGAGGCUGGAUCAGCAGCAGG | 905 |
| 1029 | GCUGCUGAUCCAGCCUCACUA | 820 | UAGUGAGGCUGGAUCAGCAGCAG | 906 |
| 1030 | CUGCUGAUCCAGCCUCACUAU | 821 | AUAGUGAGGCUGGAUCAGCAGCA | 907 |
| 1038 | CCAGCCUCACUAUGCCUCUGA | 822 | UCAGAGGCAUAGUGAGGCUGGAU | 908 |
| 1041 | GCCUCACUAUGCCUCUGACCU | 823 | AGGUCAGAGGCAUAGUGAGGCUG | 909 |
| 1044 | UCACUAUGCCUCUGACCUGGA | 824 | UCCAGGUCAGAGGCAUAGUGAGG | 910 |
| 1046 | ACUAUGCCUCUGACCUGGACA | 825 | UGUCCAGGUCAGAGGCAUAGUGA | 911 |
| 1047 | CUAUGCCUCUGACCUGGACAA | 826 | UUGUCCAGGUCAGAGGCAUAGUG | 912 |
| 1053 | CUCUGACCUGGACAAGGUGGA | 827 | UCCACCUUGUCCAGGUCAGAGGC | 913 |
| 1057 | GACCUGGACAAGGUGGAGGGU | 828 | ACCCUCCACCUUGUCCAGGUCAG | 914 |
| 1061 | UGGACAAGGUGGAGGGUCUCA | 829 | UGAGACCCUCCACCUUGUCCAGG | 915 |
| 1065s | CAAGGUGGAGGGUCUCACUU | 830 | AAGUGAGACCCUCCACCUUGUC | 916 |
| 1071 | GGAGGGUCUCACUUUCCAGCA | 831 | UGCUGGAAAGUGAGACCCUCCAC | 917 |
| 1260 | CAGGGUGGGGGAGGUGCUGAA | 832 | UUCAGCACCUCCCCCACCCUGAU | 918 |
| 1262 | GGGUGGGGGAGGUGCUGAACA | 833 | UGUUCAGCACCUCCCCCACCCUG | 919 |
| 1265 | UGGGGGAGGUGCUGAACAGCA | 834 | UGCUGUUCAGCACCUCCCCCACC | 920 |
| 1266 | GGGGGAGGUGCUGAACAGCAU | 835 | AUGCUGUUCAGCACCUCCCCCAC | 921 |
| 1267 | GGGGAGGUGCUGAACAGCAUU | 836 | AAUGCUGUUCAGCACCUCCCCCA | 922 |
| 1268 | GGGAGGUGCUGAACAGCAUUU | 837 | AAAUGCUGUUCAGCACCUCCCCC | 923 |
| 1279 | AACAGCAUUUUUUUUGAGCUU | 838 | AAGCUCAAAAAAAAUGCUGUUCA | 924 |
| 1281 | CAGCAUUUUUUUUGAGCUUGA | 839 | UCAAGCUCAAAAAAAAUGCUGUU | 925 |
| 1282 | AGCAUUUUUUUUGAGCUUGAA | 840 | UUCAAGCUCAAAAAAAAUGCUGU | 926 |
| 1362 | GGUGACCCUGAACCGCCCAUU | 841 | AAUGGGCGGUUCAGGGUCACCUC | 927 |
| 1365 | GACCCUGAACCGCCCAUUCCU | 842 | AGGAAUGGGCGGUUCAGGGUCAC | 928 |
| 1367 | CCCUGAACCGCCCAUUCCUGU | 843 | ACAGGAAUGGGCGGUUCAGGGUC | 929 |
| 1369 | CUGAACCGCCCAUUCCUGUUU | 844 | AAACAGGAAUGGGCGGUUCAGGG | 930 |
| 1393 | GUGUAUGAUCAAAGCGCCACU | 845 | AGUGGCGCUUUGAUCAUACACAG | 931 |
| 1401 | UCAAAGCGCCACUGCCCUGCA | 846 | UGCAGGGCAGUGGCGCUUUGAUC | 932 |
| 1404 | AAGCGCCACUGCCCUGCACUU | 847 | AAGUGCAGGGCAGUGGCGCUUUG | 933 |
| 1407 | CGCCACUGCCCUGCACUUCCU | 848 | AGGAAGUGCAGGGCAGUGGCGCU | 934 |
| 1554 | GCGAUGUGUCACCCCCAGUCU | 849 | AGACUGGGGGUGACACAUCGCUG | 935 |
| 1558 | UGUGUCACCCCCAGUCUCCCA | 850 | UGGGAGACUGGGGGUGACACAUC | 936 |
| 1561 | GUCACCCCCAGUCUCCCACCU | 851 | AGGUGGGAGACUGGGGGUGACAC | 937 |
| 1562 | UCACCCCCAGUCUCCCACCUU | 852 | AAGGUGGGAGACUGGGGGUGACA | 938 |
| 1564 | ACCCCCAGUCUCCCACCUUUU | 853 | AAAAGGUGGGAGACUGGGGGUGA | 939 |
| 1591 | AUGAGUCGACUUUGAGCUGGA | 854 | UCCAGCUCAAAGUCGACUCAUUA | 940 |
| 1592 | UGAGUCGACUUUGAGCUGGAA | 855 | UUCCAGCUCAAAGUCGACUCAUU | 941 |
| 1593 | GAGUCGACUUUGAGCUGGAAA | 856 | UUUCCAGCUCAAAGUCGACUCAU | 942 |
| 1596 | UCGACUUUGAGCUGGAAAGCA | 857 | UGCUUUCCAGCUCAAAGUCGACU | 943 |
| 1602 | UUGAGCUGGAAAGCAGCCGUU | 858 | AACGGCUGCUUUCCAGCUCAAAG | 944 |
| 1603 | UGAGCUGGAAAGCAGCCGUUU | 859 | AAACGGCUGCUUUCCAGCUCAAA | 945 |
| 1608 | UGGAAAGCAGCCGUUUCUCCU | 860 | AGGAGAAACGGCUGCUUUCCAGC | 946 |
| 1612 | AAGCAGCCGUUUCUCCUUGGU | 861 | ACCAAGGAGAAACGGCUGCUUUC | 947 |
| 1775 | CAACCGACCAGCUUGUUUGUA | 862 | UACAAACAAGCUGGUCGGUUGGA | 948 |
| 1790 | UUUGUGAAACAAAAAAGUGUU | 863 | AACACUUUUUUGUUUCACAAACA | 949 |
| 1796 | AAACAAAAAAGUGUUCCCUUU | 864 | AAAGGGAACACUUUUUUGUUUCA | 950 |
| 1798s | ACAAAAAAGUGUUCCCUUUU | 865 | AAAAGGGAACACUUUUUUGUUU | 951 |
| 1799 | CAAAAAAGUGUUCCCUUUUCA | 866 | UGAAAAGGGAACACUUUUUUGUU | 952 |
| 1810 | UCCCUUUUCAAGUUGAGAACA | 867 | UGUUCUCAACUUGAAAAGGGAAC | 953 |
| 1811 | CCCUUUUCAAGUUGAGAACAA | 868 | UUGUUCUCAACUUGAAAAGGGAA | 954 |
| 1814 | UUUUCAAGUUGAGAACAAAAA | 869 | UUUUUGUUCUCAACUUGAAAAGG | 955 |
| 1815 | UUUCAAGUUGAGAACAAAAAU | 870 | AUUUUUGUUCUCAACUUGAAAAG | 956 |
| 1817 | UCAAGUUGAGAACAAAAAUUG | 871 | CAAUUUUUGUUCUCAACUUGAAA | 957 |
| 1828 | ACAAAAAUUGGGUUUUAAAAU | 872 | AUUUUAAAACCCAAUUUUUGUUC | 958 |
| 1838s | GGUUUUAAAAUUAAAGUAUA | 873 | UAUACUUUAAUUUUAAAACCCA | 959 |
| 2018 | AUAGCUGGUUAUUUCUCCCUU | 874 | AAGGGAGAAAUAACCAGCUAUGG | 960 |
| 2020 | AGCUGGUUAUUUCUCCCUUGU | 875 | ACAAGGGAGAAAUAACCAGCUAU | 961 |
| 2025 | GUUAUUUCUCCCUUGUGUUAA | 876 | UUAACACAAGGGAGAAAUAACCA | 962 |
| 2030 | UUCUCCCUUGUGUUAGUAAUA | 877 | UAUUACUAACACAAGGGAGAAAU | 963 |
| 2031 | UCUCCCUUGUGUUAGUAAUAA | 878 | UUAUUACUAACACAAGGGAGAAA | 964 |
| 2032 | CUCCCUUGUGUUAGUAAUAAA | 879 | UUUAUUACUAACACAAGGGAGAA | 965 |
| 2033 | UCCCUUGUGUUAGUAAUAAAA | 880 | UUUUAUUACUAACACAAGGGAGA | 966 |
| 729 | AGAACUGGAUGUUGCUGCUGA | 881 | UCAGCAGCAACAUCCAGUUCUGU | 967 |
| 731 | AACUGGAUGUUGCUGCUGAGA | 882 | UCUCAGCAGCAACAUCCAGUUCU | 968 |
| 732 | ACUGGAUGUUGCUGCUGAGAA | 883 | UUCUCAGCAGCAACAUCCAGUUC | 969 |
| 734 | UGGAUGUUGCUGCUGAGAAGA | 884 | UCUUCUCAGCAGCAACAUCCAGU | 970 |
| 734s | UGGAUGUUGCUGCUGAGAAA | 885 | UUUCUCAGCAGCAACAUCCAGU | 971 |
| 736 | GAUGUUGCUGCUGAGAAGAUU | 886 | AAUCUUCUCAGCAGCAACAUCCA | 972 |
| 743 | CUGCUGAGAAGAUUGACAGGU | 887 | ACCUGUCAAUCUUCUCAGCAGCA | 973 |
| 753s | GAUUGACAGGUUCAUGCAGA | 888 | UCUGCAUGAACCUGUCAAUCUU | 974 |
| 963 | GCACUGGAGUGACAUCCAGGA | 889 | UCCUGGAUGUCACUCCAGUGCUG | 975 |
| 966 | CUGGAGUGACAUCCAGGACAA | 890 | UUGUCCUGGAUGUCACUCCAGUG | 976 |
| 969 | GAGUGACAUCCAGGACAACUU | 891 | AAGUUGUCCUGGAUGUCACUCCA | 977 |
| 971 | GUGACAUCCAGGACAACUUCU | 892 | AGAAGUUGUCCUGGAUGUCACUC | 978 |
| 982 | GACAACUUCUCGGUGACUCAA | 893 | UUGAGUCACCGAGAAGUUGUCCU | 979 |
| 984 | CAACUUCUCGGUGACUCAAGU | 894 | ACUUGAGUCACCGAGAAGUUGUC | 980 |
| 992 | CGGUGACUCAAGUGCCCUUCA | 895 | UGAAGGGCACUUGAGUCACCGAG | 981 |
| 994 | GUGACUCAAGUGCCCUUCACU | 896 | AGUGAAGGGCACUUGAGUCACCG | 982 |
| B1835-AL | Ufs-Ums-Gf-Gm-Gf-Um-Uf-Um-Uf- Am-Af-Am-Af-Um-Uf-Am-Af-Am- Gf-Um-Af | 983 | Ums-Afs-Cm-Uf-Um-Uf-Am-Af-Um- Uf-Um-Uf-Am-Af-Am-Af-Cm-Cf-Cm- Af-Ams-Ufs-Um | 996 |
| B1816-AL | Ufs-Ums-Cf-Am-Af-Gm-Uf-Um-Gf- Am-Gf-Am-Af-Cm-Af-Am-Af-Am- Af-Um-Uf | 984 | Ams-Afs-Um-Uf-Um-Uf-Um-Gf-Um- Uf-Cm-Uf-Cm-Af-Am-Cf-Um-Uf-Gm- Af-Ams-Afs-Am | 997 |
| B1812-AL | Cfs-Cms-Uf-Um-Uf-Um-Cf-Am-Af- Gm-Uf-Um-Gf-Am-Gf-Am-Af-Cm- Af-Am-Af | 985 | Ums-Ufs-Um-Gf-Um-Uf-Cm-Uf-Cm- Af-Am-Cf-Um-Uf-Gm-Af-Am-Af-Am- Gf-Gms-Gfs-Am | 998 |
| B1579-AL | Cfs-Cms-Uf-Um-Uf-Um-Cf-Um-Uf- Cm-Uf-Am-Af-Um-Gf-Am-Gf-Um- Cf-Gm-Af | 986 | Ums-Cfs-Gm-Af-Cm-Uf-Cm-Af-Um-Uf- Am-Gf-Am-Af-Gm-Af-Am-Af-Am-Gf- Gms-Ufs-Gm | 999 |
| B1836-AL | Ufs-Gms-Gf-Gm-Uf-Um-Uf-Um-Af- Am-Af-Am-Uf-Um-Af-Am-Af-Gm- Uf-Am-Uf | 987 | Ams-Ufs-Am-Cf-Um-Uf-Um-Af-Am- Uf-Um-Uf-Um-Af-Am-Af-Am-Cf-Cm- Cf-Ams-Afs-Um | 1000 |
| B1789-AL | Gfs-Ums-Uf-Um-Gf-Um-Gf-Am-Af- Am-Cf-Am-Af-Am-Af-Am-Af-Gm- Uf-Gm-Uf | 988 | Ams-Cfs-Am-Cf-Um-Uf-Um-Uf-Um- Uf-Gm-Uf-Um-Uf-Cm-Af-Cm-Af-Am- Af-Cms-Afs-Am | 1001 |
| B1839-AL | Gfs-Ums-Uf-Um-Uf-Am-Af-Am-Af- Um-Uf-Am-Af-Am-Gf-Um-Af-Um- Af-Cm-Af | 989 | Ums-Gfs-Um-Af-Um-Af-Cm-Uf-Um- Uf-Am-Af-Um-Uf-Um-Uf-Am-Af-Am- Af-Cms-Cfs-Cm | 1002 |
| B1791-AL | Ufs-Ums-Gf-Um-Gf-Am-Af-Am-Cf- Am-Af-Am-Af-Am-Af-Gm-Uf-Gm- Uf-Um-Af | 990 | Ums-Afs-Am-Cf-Am-Cf-Um-Uf-Um- Uf-Um-Uf-Gm-Uf-Um-Uf-Cm-Af-Cm- Af-Ams-Afs-Cm | 1003 |
| B1795-AL | Gfs-Ams-Af-Am-Cf-Am-Af-Am-Af- Am-Af-Gm-Uf-Gm-Uf-Um-Cf-Cm- Cf-Um-Uf | 991 | Ams-Afs-Gm-Gf-Gm-Af-Am-Cf-Am- Cf-Um-Uf-Um-Uf-Um-Uf-Gm-Uf-Um- Uf-Cms-Afs-Cm | 1004 |
| B1585sAL | Cms-Ufs-Um-Cf-Um-Af-Am-Uf-Gm- Af-Gm-Uf-Cm-Gf-Am-Cf-Um-Uf- Um-Af | 992 | Ums-Afs-Am-Af-Gm-Uf-Cm-Gf-Am- Cf-Um-Cf-Am-Uf-Um-Af-Gm-Af-Am- Gfs-Ams-Af | 1005 |
| B1576sAL | Cms-Cfs-Am-Cf-Cm-Uf-Um-Uf-Um- Cf-Um-Uf-Cm-Uf-Am-Af-Um-Gf- Am-Af | 993 | Ums-Ufs-Cm-Af-Um-Uf-Am-Gf-Am- Af-Gm-Af-Am-Af-Am-Gf-Gm-Uf-Gm- Gfs-Gms-Af | 1006 |
| B1578sAL | Ams-Cfs-Cm-Uf-Um-Uf-Um-Cf-Um- Uf-Cm-Uf-Am-Af-Um-Gf-Am-Gf- Um-Af | 994 | Ums-Afs-Cm-Uf-Cm-Af-Um-Uf-Am- Gf-Am-Af-Gm-Af-Am-Af-Am-Gf-Gm- Ufs-Gms-Gf | 1007 |
| B1838-AL | Gfs-Gms-Uf-Um-Uf-Um-Af-Am-Af- Am-Uf-Um-Af-Am-Af-Gm-Uf-Am- Uf-Am-Af | 995 | Ums-Ufs-Am-Uf-Am-Cf-Um-Uf-Um- Af-Am-Uf-Um-Uf-Um-Af-Am-Af-Am- Cf-Cms-Cfs-Am | 1008 |
| B1008-AL | Cfs-Ums-Uf-Cm-Af-Cm-Uf-Gm-Af- Gm-Af-Gm-Cf-Gm-Cf-Cm-Uf-Gm- Cf-Cm-Uf | 1009 | Ams-Gfs-Gm-Cf-Am-Gf-Gm-Cf-Gm-Cf- Um-Cf-Um-Cf-Am-Gf-Um-Gf-Am-Af- Gms-Gfs-Gm | 1095 |
| B1011-AL | Cfs-Ams-Cf-Um-Gf-Am-Gf-Am-Gf- Cm-Gf-Cm-Cf-Um-Gf-Cm-Cf-Um- Gf-Cm-Uf | 1010 | Ams-Gfs-Cm-Af-Gm-Gf-Cm-Af-Gm- Gf-Cm-Gf-Cm-Uf-Cm-Uf-Cm-Af-Gm- Uf-Gms-Afs-Am | 1096 |
| B1014-AL | Ufs-Gms-Af-Gm-Af-Gm-Cf-Gm-Cf- Cm-Uf-Gm-Cf-Cm-Uf-Gm-Cf-Um- Gf-Cm-Uf | 1011 | Ams-Gfs-Cm-Af-Gm-Cf-Am-Gf-Gm-Cf- Am-Gf-Gm-Cf-Gm-Cf-Um-Cf-Um-Cf- Ams-Gfs-Um | 1097 |
| B1016-AL | Afs-Gms-Af-Gm-Cf-Gm-Cf-Cm-Uf- Gm-Cf-Cm-Uf-Gm-Cf-Um-Gf-Cm- Uf-Gm-Af | 1012 | Ums-Cfs-Am-Gf-Cm-Af-Gm-Cf-Am-Gf- Gm-Cf-Am-Gf-Gm-Cf-Gm-Cf-Um-Cf- Ums-Cfs-Am | 1098 |
| B1017-AL | Gfs-Ams-Gf-Cm-Gf-Cm-Cf-Um-Gf- Cm-Cf-Um-Gf-Cm-Uf-Gm-Cf-Um- Gf-Am-Uf | 1013 | Ams-Ufs-Cm-Af-Gm-Cf-Am-Gf-Cm-Af- Gm-Gf-Cm-Af-Gm-Gf-Cm-Gf-Cm-Uf- Cms-Ufs-Cm | 1099 |
| B1020-AL | Cfs-Gms-Cf-Cm-Uf-Gm-Cf-Cm-Uf- Gm-Cf-Um-Gf-Cm-Uf-Gm-Af-Um- Cf-Cm-Af | 1014 | Ums-Gfs-Gm-Af-Um-Cf-Am-Gf-Cm- Af-Gm-Cf-Am-Gf-Gm-Cf-Am-Gf-Gm- Cf-Gms-Cfs-Um | 1100 |
| B1024-AL | Ufs-Gms-Cf-Cm-Uf-Gm-Cf-Um-Gf- Cm-Uf-Gm-Af-Um-Cf-Cm-Af-Gm- Cf-Cm-Uf | 1015 | Ams-Gfs-Gm-Cf-Um-Gf-Gm-Af-Um- Cf-Am-Gf-Cm-Af-Gm-Cf-Am-Gf-Gm- Cf-Ams-Gfs-Gm | 1101 |
| B1026-AL | Cfs-Cms-Uf-Gm-Cf-Um-Gf-Cm-Uf- Gm-Af-Um-Cf-Cm-Af-Gm-Cf-Cm- Uf-Cm-Af | 1016 | Ums-Gfs-Am-Gf-Gm-Cf-Um-Gf-Gm- Af-Um-Cf-Am-Gf-Cm-Af-Gm-Cf-Am- Gf-Gms-Cfs-Am | 1102 |
| B1028-AL | Ufs-Gms-Cf-Um-Gf-Cm-Uf-Gm-Af- Um-Cf-Cm-Af-Gm-Cf-Cm-Uf-Cm- Af-Cm-Uf | 1017 | Ams-Gfs-Um-Gf-Am-Gf-Gm-Cf-Um- Gf-Gm-Af-Um-Cf-Am-Gf-Cm-Af-Gm- Cf-Ams-Gfs-Gm | 1103 |
| B1029-AL | Gfs-Cms-Uf-Gm-Cf-Um-Gf-Am-Uf- Cm-Cf-Am-Gf-Cm-Cf-Um-Cf-Am- Cf-Um-Af | 1018 | Ums-Afs-Gm-Uf-Gm-Af-Gm-Gf-Cm- Uf-Gm-Gf-Am-Uf-Cm-Af-Gm-Cf-Am- Gf-Cms-Afs-Gm | 1104 |
| B1030-AL | Cfs-Ums-Gf-Cm-Uf-Gm-Af-Um-Cf- Cm-Af-Gm-Cf-Cm-Uf-Cm-Af-Cm- Uf-Am-Uf | 1019 | Ams-Ufs-Am-Gf-Um-Gf-Am-Gf-Gm- Cf-Um-Gf-Gm-Af-Um-Cf-Am-Gf-Cm- Af-Gms-Cfs-Am | 1105 |
| B1038-AL | Cfs-Cms-Af-Gm-Cf-Cm-Uf-Cm-Af- Cm-Uf-Am-Uf-Gm-Cf-Cm-Uf-Cm- Uf-Gm-Af | 1020 | Ums-Cfs-Am-Gf-Am-Gf-Gm-Cf-Am- Uf-Am-Gf-Um-Gf-Am-Gf-Gm-Cf-Um- Gf-Gms-Afs-Um | 1106 |
| B1041-AL | Gfs-Cms-Cf-Um-Cf-Am-Cf-Um-Af- Um-Gf-Cm-Cf-Um-Cf-Um-Gf-Am- Cf-Cm-Uf | 1021 | Ams-Gfs-Gm-Uf-Cm-Af-Gm-Af-Gm- Gf-Cm-Af-Um-Af-Gm-Uf-Gm-Af-Gm- Gf-Cms-Ufs-Gm | 1107 |
| B1044-AL | Ufs-Cms-Af-Cm-Uf-Am-Uf-Gm-Cf- Cm-Uf-Cm-Uf-Gm-Af-Cm-Cf-Um- Gf-Gm-Af | 1022 | Ums-Cfs-Cm-Af-Gm-Gf-Um-Cf-Am-Gf- Am-Gf-Gm-Cf-Am-Uf-Am-Gf-Um-Gf- Ams-Gfs-Gm | 1108 |
| B1046-AL | Afs-Cms-Uf-Am-Uf-Gm-Cf-Cm-Uf- Cm-Uf-Gm-Af-Cm-Cf-Um-Gf-Gm- Af-Cm-Af | 1023 | Ums-Gfs-Um-Cf-Cm-Af-Gm-Gf-Um-Cf- Am-Gf-Am-Gf-Gm-Cf-Am-Uf-Am-Gf- Ums-Gfs-Am | 1109 |
| B1047-AL | Cfs-Ums-Af-Um-Gf-Cm-Cf-Um-Cf- Um-Gf-Am-Cf-Cm-Uf-Gm-Gf-Am- Cf-Am-Af | 1024 | Ums-Ufs-Gm-Uf-Cm-Cf-Am-Gf-Gm- Uf-Cm-Af-Gm-Af-Gm-Gf-Cm-Af-Um- Af-Gms-Ufs-Gm | 1110 |
| B1053-AL | Cfs-Ums-Cf-Um-Gf-Am-Cf-Cm-Uf- Gm-Gf-Am-Cf-Am-Af-Gm-Gf-Um- Gf-Gm-Af | 1025 | Ums-Cfs-Cm-Af-Cm-Cf-Um-Uf-Gm-Uf- Cm-Cf-Am-Gf-Gm-Uf-Cm-Af-Gm-Af- Gms-Gfs-Cm | 1111 |
| B1057-AL | Gfs-Ams-Cf-Cm-Uf-Gm-Gf-Am-Cf- Am-Af-Gm-Gf-Um-Gf-Gm-Af-Gm- Gf-Gm-Uf | 1026 | Ams-Cfs-Cm-Cf-Um-Cf-Cm-Af-Cm-Cf- Um-Uf-Gm-Uf-Cm-Cf-Am-Gf-Gm-Uf- Cms-Afs-Gm | 1112 |
| B1061-AL | Ufs-Gms-Gf-Am-Cf-Am-Af-Gm-Gf- Um-Gf-Gm-Af-Gm-Gf-Gm-Uf-Cm- Uf-Cm-Af | 1027 | Ums-Gfs-Am-Gf-Am-Cf-Cm-Cf-Um-Cf- Cm-Af-Cm-Cf-Um-Uf-Gm-Uf-Cm-Cf- Ams-Gfs-Gm | 1113 |
| B1065sAL | Cms-Afs-Am-Gf-Gm-Uf-Gm-Gf- Am-Gf-Gm-Gf-Um-Cf-Um-Cf-Am- Cf-Um-Uf | 1028 | Ams-Afs-Gm-Uf-Gm-Af-Gm-Af-Cm- Cf-Cm-Uf-Cm-Cf-Am-Cf-Cm-Uf-Um- Gfs-Ums-Cf | 1114 |
| B1071-AL | Gfs-Gms-Af-Gm-Gf-Gm-Uf-Cm-Uf- Cm-Af-Cm-Uf-Um-Uf-Cm-Cf-Am- Gf-Cm-Af | 1029 | Ums-Gfs-Cm-Uf-Gm-Gf-Am-Af-Am- Gf-Um-Gf-Am-Gf-Am-Cf-Cm-Cf-Um- Cf-Cms-Afs-Cm | 1115 |
| B1260-AL | Cfs-Ams-Gf-Gm-Gf-Um-Gf-Gm-Gf- Gm-Gf-Am-Gf-Gm-Uf-Gm-Cf-Um- Gf-Am-Af | 1030 | Ums-Ufs-Cm-Af-Gm-Cf-Am-Cf-Cm-Uf- Cm-Cf-Cm-Cf-Cm-Af-Cm-Cf-Cm-Uf- Gms-Afs-Um | 1116 |
| B1262-AL | Gfs-Gms-Gf-Um-Gf-Gm-Gf-Gm-Gf- Am-Gf-Gm-Uf-Gm-Cf-Um-Gf-Am- Af-Cm-Af | 1031 | Ums-Gfs-Um-Uf-Cm-Af-Gm-Cf-Am-Cf- Cm-Uf-Cm-Cf-Cm-Cf-Cm-Af-Cm-Cf- Cms-Ufs-Gm | 1117 |
| B1265-AL | Ufs-Gms-Gf-Gm-Gf-Gm-Af-Gm-Gf- Um-Gf-Cm-Uf-Gm-Af-Am-Cf-Am- Gf-Cm-Af | 1032 | Ums-Gfs-Cm-Uf-Gm-Uf-Um-Cf-Am- Gf-Cm-Af-Cm-Cf-Um-Cf-Cm-Cf-Cm- Cf-Ams-Cfs-Cm | 1118 |
| B1266-AL | Gfs-Gms-Gf-Gm-Gf-Am-Gf-Gm-Uf- Gm-Cf-Um-Gf-Am-Af-Cm-Af-Gm- Cf-Am-Uf | 1033 | Ams-Ufs-Gm-Cf-Um-Gf-Um-Uf-Cm- Af-Gm-Cf-Am-Cf-Cm-Uf-Cm-Cf-Cm- Cf-Cms-Afs-Cm | 1119 |
| B1267-AL | Gfs-Gms-Gf-Gm-Af-Gm-Gf-Um-Gf- Cm-Uf-Gm-Af-Am-Cf-Am-Gf-Cm- Af-Um-Uf | 1034 | Ams-Afs-Um-Gf-Cm-Uf-Gm-Uf-Um- Cf-Am-Gf-Cm-Af-Cm-Cf-Um-Cf-Cm- Cf-Cms-Cfs-Am | 1120 |
| B1268-AL | Gfs-Gms-Gf-Am-Gf-Gm-Uf-Gm-Cf- Um-Gf-Am-Af-Cm-Af-Gm-Cf-Am- Uf-Um-Uf | 1035 | Ams-Afs-Am-Uf-Gm-Cf-Um-Gf-Um- Uf-Cm-Af-Gm-Cf-Am-Cf-Cm-Uf-Cm- Cf-Cms-Cfs-Cm | 1121 |
| B1279-AL | Afs-Ams-Cf-Am-Gf-Cm-Af-Um-Uf- Um-Uf-Um-Uf-Um-Uf-Gm-Af-Gm- Cf-Um-Uf | 1036 | Ams-Afs-Gm-Cf-Um-Cf-Am-Af-Am- Af-Am-Af-Am-Af-Um-Gf-Cm-Uf-Gm- Uf-Ums-Cfs-Am | 1122 |
| B1281-AL | Cfs-Ams-Gf-Cm-Af-Um-Uf-Um-Uf- Um-Uf-Um-Uf-Gm-Af-Gm-Cf-Um- Uf-Gm-Af | 1037 | Ums-Cfs-Am-Af-Gm-Cf-Um-Cf-Am-Af- Am-Af-Am-Af-Am-Af-Um-Gf-Cm-Uf- Gms-Ufs-Um | 1123 |
| B1282-AL | Afs-Gms-Cf-Am-Uf-Um-Uf-Um-Uf- Um-Uf-Um-Gf-Am-Gf-Cm-Uf-Um- Gf-Am-Af | 1038 | Ums-Ufs-Cm-Af-Am-Gf-Cm-Uf-Cm-Af- Am-Af-Am-Af-Am-Af-Am-Uf-Gm-Cf- Ums-Gfs-Um | 1124 |
| B1362-AL | Gfs-Gms-Uf-Gm-Af-Cm-Cf-Cm-Uf- Gm-Af-Am-Cf-Cm-Gf-Cm-Cf-Cm- Af-Um-Uf | 1039 | Ams-Afs-Um-Gf-Gm-Gf-Cm-Gf-Gm- Uf-Um-Cf-Am-Gf-Gm-Gf-Um-Cf-Am- Cf-Cms-Ufs-Cm | 1125 |
| B1365-AL | Gfs-Ams-Cf-Cm-Cf-Um-Gf-Am-Af- Cm-Cf-Gm-Cf-Cm-Cf-Am-Uf-Um- Cf-Cm-Uf | 1040 | Ams-Gfs-Gm-Af-Am-Uf-Gm-Gf-Gm- Cf-Gm-Gf-Um-Uf-Cm-Af-Gm-Gf-Gm- Uf-Cms-Afs-Cm | 1126 |
| B1367-AL | Cfs-Cms-Cf-Um-Gf-Am-Af-Cm-Cf- Gm-Cf-Cm-Cf-Am-Uf-Um-Cf-Cm- Uf-Gm-Uf | 1041 | Ams-Cfs-Am-Gf-Gm-Af-Am-Uf-Gm- Gf-Gm-Cf-Gm-Gf-Um-Uf-Cm-Af-Gm- Gf-Gms-Ufs-Cm | 1127 |
| B1369-AL | Cfs-Ums-Gf-Am-Af-Cm-Cf-Gm-Cf- Cm-Cf-Am-Uf-Um-Cf-Cm-Uf-Gm- Uf-Um-Uf | 1042 | Ams-Afs-Am-Cf-Am-Gf-Gm-Af-Am- Uf-Gm-Gf-Gm-Cf-Gm-Gf-Um-Uf-Cm- Af-Gms-Gfs-Gm | 1128 |
| B1393-AL | Gfs-Ums-Gf-Um-Af-Um-Gf-Am-Uf- Cm-Af-Am-Af-Gm-Cf-Gm-Cf-Cm- Af-Cm-Uf | 1043 | Ams-Gfs-Um-Gf-Gm-Cf-Gm-Cf-Um- Uf-Um-Gf-Am-Uf-Cm-Af-Um-Af-Cm- Af-Cms-Afs-Gm | 1129 |
| B1401-AL | Ufs-Cms-Af-Am-Af-Gm-Cf-Gm-Cf- Cm-Af-Cm-Uf-Gm-Cf-Cm-Cf-Um- Gf-Cm-Af | 1044 | Ums-Gfs-Cm-Af-Gm-Gf-Gm-Cf-Am- Gf-Um-Gf-Gm-Cf-Gm-Cf-Um-Uf-Um- Gf-Ams-Ufs-Cm | 1130 |
| B1404-AL | Afs-Ams-Gf-Cm-Gf-Cm-Cf-Am-Cf- Um-Gf-Cm-Cf-Cm-Uf-Gm-Cf-Am- Cf-Um-Uf | 1045 | Ams-Afs-Gm-Uf-Gm-Cf-Am-Gf-Gm- Gf-Cm-Af-Gm-Uf-Gm-Gf-Cm-Gf-Cm- Uf-Ums-Ufs-Gm | 1131 |
| B1407-AL | Cfs-Gms-Cf-Cm-Af-Cm-Uf-Gm-Cf- Cm-Cf-Um-Gf-Cm-Af-Cm-Uf-Um- Cf-Cm-Uf | 1046 | Ams-Gfs-Gm-Af-Am-Gf-Um-Gf-Cm- Af-Gm-Gf-Gm-Cf-Am-Gf-Um-Gf-Gm- Cf-Gms-Cfs-Um | 1132 |
| B1554-AL | Gfs-Cms-Gf-Am-Uf-Gm-Uf-Gm-Uf- Cm-Af-Cm-Cf-Cm-Cf-Cm-Af-Gm- Uf-Cm-Uf | 1047 | Ams-Gfs-Am-Cf-Um-Gf-Gm-Gf-Gm- Gf-Um-Gf-Am-Cf-Am-Cf-Am-Uf-Cm- Gf-Cms-Ufs-Gm | 1133 |
| B1558-AL | Ufs-Gms-Uf-Gm-Uf-Cm-Af-Cm-Cf- Cm-Cf-Cm-Af-Gm-Uf-Cm-Uf-Cm- Cf-Cm-Af | 1048 | Ums-Gfs-Gm-Gf-Am-Gf-Am-Cf-Um- Gf-Gm-Gf-Gm-Gf-Um-Gf-Am-Cf-Am- Cf-Ams-Ufs-Cm | 1134 |
| B1561-AL | Gfs-Ums-Cf-Am-Cf-Cm-Cf-Cm-Cf- Am-Gf-Um-Cf-Um-Cf-Cm-Cf-Am- Cf-Cm-Uf | 1049 | Ams-Gfs-Gm-Uf-Gm-Gf-Gm-Af-Gm- Af-Cm-Uf-Gm-Gf-Gm-Gf-Gm-Uf-Gm- Af-Cms-Afs-Cm | 1135 |
| B1562-AL | Ufs-Cms-Af-Cm-Cf-Cm-Cf-Cm-Af- Gm-Uf-Cm-Uf-Cm-Cf-Cm-Af-Cm- Cf-Um-Uf | 1050 | Ams-Afs-Gm-Gf-Um-Gf-Gm-Gf-Am- Gf-Am-Cf-Um-Gf-Gm-Gf-Gm-Gf-Um- Gf-Ams-Cfs-Am | 1136 |
| B1564-AL | Afs-Cms-Cf-Cm-Cf-Cm-Af-Gm-Uf- Cm-Uf-Cm-Cf-Cm-Af-Cm-Cf-Um- Uf-Um-Uf | 1051 | Ams-Afs-Am-Af-Gm-Gf-Um-Gf-Gm- Gf-Am-Gf-Am-Cf-Um-Gf-Gm-Gf-Gm- Gf-Ums-Gfs-Am | 1137 |
| B1591-AL | Afs-Ums-Gf-Am-Gf-Um-Cf-Gm-Af- Cm-Uf-Um-Uf-Gm-Af-Gm-Cf-Um- Gf-Gm-Af | 1052 | Ums-Cfs-Cm-Af-Gm-Cf-Um-Cf-Am-Af- Am-Gf-Um-Cf-Gm-Af-Cm-Uf-Cm-Af- Ums-Ufs-Am | 1138 |
| B1592-AL | Ufs-Gms-Af-Gm-Uf-Cm-Gf-Am-Cf- Um-Uf-Um-Gf-Am-Gf-Cm-Uf-Gm- Gf-Am-Af | 1053 | Ums-Ufs-Cm-Cf-Am-Gf-Cm-Uf-Cm-Af- Am-Af-Gm-Uf-Cm-Gf-Am-Cf-Um-Cf- Ams-Ufs-Um | 1139 |
| B1593-AL | Gfs-Ams-Gf-Um-Cf-Gm-Af-Cm-Uf- Um-Uf-Gm-Af-Gm-Cf-Um-Gf-Gm- Af-Am-Af | 1054 | Ums-Ufs-Um-Cf-Cm-Af-Gm-Cf-Um-Cf- Am-Af-Am-Gf-Um-Cf-Gm-Af-Cm-Uf- Cms-Afs-Um | 1140 |
| B1596-AL | Ufs-Cms-Gf-Am-Cf-Um-Uf-Um-Gf- Am-Gf-Cm-Uf-Gm-Gf-Am-Af-Am- Gf-Cm-Af | 1055 | Ums-Gfs-Cm-Uf-Um-Uf-Cm-Cf-Am-Gf- Cm-Uf-Cm-Af-Am-Af-Gm-Uf-Cm-Gf- Ams-Cfs-Um | 1141 |
| B1602-AL | Ufs-Ums-Gf-Am-Gf-Cm-Uf-Gm-Gf- Am-Af-Am-Gf-Cm-Af-Gm-Cf-Cm- Gf-Um-Uf | 1056 | Ams-Afs-Cm-Gf-Gm-Cf-Um-Gf-Cm-Uf- Um-Uf-Cm-Cf-Am-Gf-Cm-Uf-Cm-Af- Ams-Afs-Gm | 1142 |
| B1603-AL | Ufs-Gms-Af-Gm-Cf-Um-Gf-Gm-Af- Am-Af-Gm-Cf-Am-Gf-Cm-Cf-Gm- Uf-Um-Uf | 1057 | Ams-Afs-Am-Cf-Gm-Gf-Cm-Uf-Gm-Cf- Um-Uf-Um-Cf-Cm-Af-Gm-Cf-Um-Cf- Ams-Afs-Am | 1143 |
| B1608-AL | Ufs-Gms-Gf-Am-Af-Am-Gf-Cm-Af- Gm-Cf-Cm-Gf-Um-Uf-Um-Cf-Um- Cf-Cm-Uf | 1058 | Ams-Gfs-Gm-Af-Gm-Af-Am-Af-Cm- Gf-Gm-Cf-Um-Gf-Cm-Uf-Um-Uf-Cm- Cf-Ams-Gfs-Cm | 1144 |
| B1612-AL | Afs-Ams-Gf-Cm-Af-Gm-Cf-Cm-Gf- Um-Uf-Um-Cf-Um-Cf-Cm-Uf-Um- Gf-Gm-Uf | 1059 | Ams-Cfs-Cm-Af-Am-Gf-Gm-Af-Gm- Af-Am-Af-Cm-Gf-Gm-Cf-Um-Gf-Cm- Uf-Ums-Ufs-Cm | 1145 |
| B1775-AL | Cfs-Ams-Af-Cm-Cf-Gm-Af-Cm-Cf- Am-Gf-Cm-Uf-Um-Gf-Um-Uf-Um- Gf-Um-Af | 1060 | Ums-Afs-Cm-Af-Am-Af-Cm-Af-Am- Gf-Cm-Uf-Gm-Gf-Um-Cf-Gm-Gf-Um- Uf-Gms-Gfs-Am | 1146 |
| B1790-AL | Ufs-Ums-Uf-Gm-Uf-Gm-Af-Am-Af- Cm-Af-Am-Af-Am-Af-Am-Gf-Um- Gf-Um-Uf | 1061 | Ams-Afs-Cm-Af-Cm-Uf-Um-Uf-Um- Uf-Um-Gf-Um-Uf-Um-Cf-Am-Cf-Am- Af-Ams-Cfs-Am | 1147 |
| B1796-AL | Afs-Ams-Af-Cm-Af-Am-Af-Am-Af- Am-Gf-Um-Gf-Um-Uf-Cm-Cf-Cm- Uf-Um-Uf | 1062 | Ams-Afs-Am-Gf-Gm-Gf-Am-Af-Cm- Af-Cm-Uf-Um-Uf-Um-Uf-Um-Gf-Um- Uf-Ums-Cfs-Am | 1148 |
| B1798sAL | Ams-Cfs-Am-Af-Am-Af-Am-Af- Gm-Uf-Gm-Uf-Um-Cf-Cm-Cf-Um- Uf-Um-Uf | 1063 | Ams-Afs-Am-Af-Gm-Gf-Gm-Af-Am- Cf-Am-Cf-Um-Uf-Um-Uf-Um-Uf-Gm- Ufs-Ums-Uf | 1149 |
| B1799-AL | Cfs-Ams-Af-Am-Af-Am-Af-Gm-Uf- Gm-Uf-Um-Cf-Cm-Cf-Um-Uf-Um- Uf-Cm-Af | 1064 | Ums-Gfs-Am-Af-Am-Af-Gm-Gf-Gm- Af-Am-Cf-Am-Cf-Um-Uf-Um-Uf-Um- Uf-Gms-Ufs-Um | 1150 |
| B1810-AL | Ufs-Cms-Cf-Cm-Uf-Um-Uf-Um-Cf- Am-Af-Gm-Uf-Um-Gf-Am-Gf-Am- Af-Cm-Af | 1065 | Ums-Gfs-Um-Uf-Cm-Uf-Cm-Af-Am-Cf- Um-Uf-Gm-Af-Am-Af-Am-Gf-Gm-Gf- Ams-Afs-Cm | 1151 |
| B1811-AL | Cfs-Cms-Cf-Um-Uf-Um-Uf-Cm-Af- Am-Gf-Um-Uf-Gm-Af-Gm-Af-Am- Cf-Am-Af | 1066 | Ums-Ufs-Gm-Uf-Um-Cf-Um-Cf-Am- Af-Cm-Uf-Um-Gf-Am-Af-Am-Af-Gm- Gf-Gms-Afs-Am | 1152 |
| B1814-AL | Ufs-Ums-Uf-Um-Cf-Am-Af-Gm-Uf- Um-Gf-Am-Gf-Am-Af-Cm-Af-Am- Af-Am-Af | 1067 | Ums-Ufs-Um-Uf-Um-Gf-Um-Uf-Cm- Uf-Cm-Af-Am-Cf-Um-Uf-Gm-Af-Am- Af-Ams-Gfs-Gm | 1153 |
| B1815-AL | Ufs-Ums-Uf-Cm-Af-Am-Gf-Um-Uf- Gm-Af-Gm-Af-Am-Cf-Am-Af-Am- Af-Am-Uf | 1068 | Ams-Ufs-Um-Uf-Um-Uf-Gm-Uf-Um- Cf-Um-Cf-Am-Af-Cm-Uf-Um-Gf-Am- Af-Ams-Afs-Gm | 1154 |
| B1817-AL | Ufs-Cms-Af-Am-Gf-Um-Uf-Gm-Af- Gm-Af-Am-Cf-Am-Af-Am-Af-Am- Uf-Um-Gf | 1069 | Cms-Afs-Am-Uf-Um-Uf-Um-Uf-Gm- Uf-Um-Cf-Um-Cf-Am-Af-Cm-Uf-Um- Gf-Ams-Afs-Am | 1155 |
| B1828-AL | Afs-Cms-Af-Am-Af-Am-Af-Um-Uf- Gm-Gf-Gm-Uf-Um-Uf-Um-Af-Am- Af-Am-Uf | 1070 | Ams-Ufs-Um-Uf-Um-Af-Am-Af-Am- Cf-Cm-Cf-Am-Af-Um-Uf-Um-Uf-Um- Gf-Ums-Ufs-Cm | 1156 |
| B1838sAL | Gms-Gfs-Um-Uf-Um-Uf-Am-Af- Am-Af-Um-Uf-Am-Af-Am-Gf-Um- Af-Um-Af | 1071 | Ums-Afs-Um-Af-Cm-Uf-Um-Uf-Am- Af-Um-Uf-Um-Uf-Am-Af-Am-Af-Cm- Cfs-Cms-Af | 1157 |
| B2018-AL | Afs-Ums-Af-Gm-Cf-Um-Gf-Gm-Uf- Um-Af-Um-Uf-Um-Cf-Um-Cf-Cm- Cf-Um-Uf | 1072 | Ams-Afs-Gm-Gf-Gm-Af-Gm-Af-Am- Af-Um-Af-Am-Cf-Cm-Af-Gm-Cf-Um- Af-Ums-Gfs-Gm | 1158 |
| B2020-AL | Afs-Gms-Cf-Um-Gf-Gm-Uf-Um-Af- Um-Uf-Um-Cf-Um-Cf-Cm-Cf-Um- Uf-Gm-Uf | 1073 | Ams-Cfs-Am-Af-Gm-Gf-Gm-Af-Gm- Af-Am-Af-Um-Af-Am-Cf-Cm-Af-Gm- Cf-Ums-Afs-Um | 1159 |
| B2025-AL | Gfs-Ums-Uf-Am-Uf-Um-Uf-Cm-Uf- Cm-Cf-Cm-Uf-Um-Gf-Um-Gf-Um- Uf-Am-Af | 1074 | Ums-Ufs-Am-Af-Cm-Af-Cm-Af-Am- Gf-Gm-Gf-Am-Gf-Am-Af-Am-Uf-Am- Af-Cms-Cfs-Am | 1160 |
| B2030-AL | Ufs-Ums-Cf-Um-Cf-Cm-Cf-Um-Uf- Gm-Uf-Gm-Uf-Um-Af-Gm-Uf-Am- Af-Um-Af | 1075 | Ums-Afs-Um-Uf-Am-Cf-Um-Af-Am- Cf-Am-Cf-Am-Af-Gm-Gf-Gm-Af-Gm- Af-Ams-Afs-Um | 1161 |
| B2031-AL | Ufs-Cms-Uf-Cm-Cf-Cm-Uf-Um-Gf- Um-Gf-Um-Uf-Am-Gf-Um-Af-Am- Uf-Am-Af | 1076 | Ums-Ufs-Am-Uf-Um-Af-Cm-Uf-Am- Af-Cm-Af-Cm-Af-Am-Gf-Gm-Gf-Am- Gf-Ams-Afs-Am | 1162 |
| B2032-AL | Cfs-Ums-Cf-Cm-Cf-Um-Uf-Gm-Uf- Gm-Uf-Um-Af-Gm-Uf-Am-Af-Um- Af-Am-Af | 1077 | Ums-Ufs-Um-Af-Um-Uf-Am-Cf-Um- Af-Am-Cf-Am-Cf-Am-Af-Gm-Gf-Gm- Af-Gms-Afs-Am | 1163 |
| B2033-AL | Ufs-Cms-Cf-Cm-Uf-Um-Gf-Um-Gf- Um-Uf-Am-Gf-Um-Af-Am-Uf-Am- Af-Am-Af | 1078 | Ums-Ufs-Um-Uf-Am-Uf-Um-Af-Cm- Uf-Am-Af-Cm-Af-Cm-Af-Am-Gf-Gm- Gf-Ams-Gfs-Am | 1164 |
| B729-AL | Afs-Gms-Af-Am-Cf-Um-Gf-Gm-Af- Um-Gf-Um-Uf-Gm-Cf-Um-Gf-Cm- Uf-Gm-Af | 1079 | Ums-Cfs-Am-Gf-Cm-Af-Gm-Cf-Am-Af- Cm-Af-Um-Cf-Cm-Af-Gm-Uf-Um-Cf- Ums-Gfs-Um | 1165 |
| B731-AL | Afs-Ams-Cf-Um-Gf-Gm-Af-Um-Gf- Um-Uf-Gm-Cf-Um-Gf-Cm-Uf-Gm- Af-Gm-Af | 1080 | Ums-Cfs-Um-Cf-Am-Gf-Cm-Af-Gm-Cf- Am-Af-Cm-Af-Um-Cf-Cm-Af-Gm-Uf- Ums-Cfs-Um | 1166 |
| B732-AL | Afs-Cms-Uf-Gm-Gf-Am-Uf-Gm-Uf- Um-Gf-Cm-Uf-Gm-Cf-Um-Gf-Am- Gf-Am-Af | 1081 | Ums-Ufs-Cm-Uf-Cm-Af-Gm-Cf-Am-Gf- Cm-Af-Am-Cf-Am-Uf-Cm-Cf-Am-Gf- Ums-Ufs-Cm | 1167 |
| B734-AL | Ufs-Gms-Gf-Am-Uf-Gm-Uf-Um-Gf- Cm-Uf-Gm-Cf-Um-Gf-Am-Gf-Am- Af-Gm-Af | 1082 | Ums-Cfs-Um-Uf-Cm-Uf-Cm-Af-Gm-Cf- Am-Gf-Cm-Af-Am-Cf-Am-Uf-Cm-Cf- Ams-Gfs-Um | 1168 |
| B734s-AL | Ums-Gfs-Gm-Af-Um-Gf-Um-Uf- Gm-Cf-Um-Gf-Cm-Uf-Gm-Af-Gm- Af-Am-Af | 1083 | Ums-Ufs-Um-Cf-Um-Cf-Am-Gf-Cm-Af- Gm-Cf-Am-Af-Cm-Af-Um-Cf-Cm-Afs- Gms-Uf | 1169 |
| B736-AL | Gfs-Ams-Uf-Gm-Uf-Um-Gf-Cm-Uf- Gm-Cf-Um-Gf-Am-Gf-Am-Af-Gm- Af-Um-Uf | 1084 | Ams-Afs-Um-Cf-Um-Uf-Cm-Uf-Cm-Af- Gm-Cf-Am-Gf-Cm-Af-Am-Cf-Am-Uf- Cms-Cfs-Am | 1170 |
| B743-AL | Cfs-Ums-Gf-Cm-Uf-Gm-Af-Gm-Af- Am-Gf-Am-Uf-Um-Gf-Am-Cf-Am- Gf-Gm-Uf | 1085 | Ams-Cfs-Cm-Uf-Gm-Uf-Cm-Af-Am-Uf- Cm-Uf-Um-Cf-Um-Cf-Am-Gf-Cm-Af- Gms-Cfs-Am | 1171 |
| B753s-AL | Gms-Afs-Um-Uf-Gm-Af-Cm-Af- Gm-Gf-Um-Uf-Cm-Af-Um-Gf-Cm- Af-Gm-Af | 1086 | Ums-Cfs-Um-Gf-Cm-Af-Um-Gf-Am- Af-Cm-Cf-Um-Gf-Um-Cf-Am-Af-Um- Cfs-Ums-Uf | 1172 |
| B963-AL | Gfs-Cms-Af-Cm-Uf-Gm-Gf-Am-Gf- Um-Gf-Am-Cf-Am-Uf-Cm-Cf-Am- Gf-Gm-Af | 1087 | Ums-Cfs-Cm-Uf-Gm-Gf-Am-Uf-Gm- Uf-Cm-Af-Cm-Uf-Cm-Cf-Am-Gf-Um- Gf-Cms-Ufs-Gm | 1173 |
| B966-AL | Cfs-Ums-Gf-Gm-Af-Gm-Uf-Gm-Af- Cm-Af-Um-Cf-Cm-Af-Gm-Gf-Am- Cf-Am-Af | 1088 | Ums-Ufs-Gm-Uf-Cm-Cf-Um-Gf-Gm- Af-Um-Gf-Um-Cf-Am-Cf-Um-Cf-Cm- Af-Gms-Ufs-Gm | 1174 |
| B969-AL | Gfs-Ams-Gf-Um-Gf-Am-Cf-Am-Uf- Cm-Cf-Am-Gf-Gm-Af-Cm-Af-Am- Cf-Um-Uf | 1089 | Ams-Afs-Gm-Uf-Um-Gf-Um-Cf-Cm- Uf-Gm-Gf-Am-Uf-Gm-Uf-Cm-Af-Cm- Uf-Cms-Cfs-Am | 1175 |
| B971-AL | Gfs-Ums-Gf-Am-Cf-Am-Uf-Cm-Cf- Am-Gf-Gm-Af-Cm-Af-Am-Cf-Um- Uf-Cm-Uf | 1090 | Ams-Gfs-Am-Af-Gm-Uf-Um-Gf-Um- Cf-Cm-Uf-Gm-Gf-Am-Uf-Gm-Uf-Cm- Af-Cms-Ufs-Cm | 1176 |
| B982-AL | Gfs-Ams-Cf-Am-Af-Cm-Uf-Um-Cf- Um-Cf-Gm-Gf-Um-Gf-Am-Cf-Um- Cf-Am-Af | 1091 | Ums-Ufs-Gm-Af-Gm-Uf-Cm-Af-Cm-Cf- Gm-Af-Gm-Af-Am-Gf-Um-Uf-Gm-Uf- Cms-Cfs-Um | 1177 |
| B984-AL | Cfs-Ams-Af-Cm-Uf-Um-Cf-Um-Cf- Gm-Gf-Um-Gf-Am-Cf-Um-Cf-Am- Af-Gm-Uf | 1092 | Ams-Cfs-Um-Uf-Gm-Af-Gm-Uf-Cm- Af-Cm-Cf-Gm-Af-Gm-Af-Am-Gf-Um- Uf-Gms-Ufs-Cm | 1178 |
| B992-AL | Cfs-Gms-Gf-Um-Gf-Am-Cf-Um-Cf- Am-Af-Gm-Uf-Gm-Cf-Cm-Cf-Um- Uf-Cm-Af | 1093 | Ums-Gfs-Am-Af-Gm-Gf-Gm-Cf-Am- Cf-Um-Uf-Gm-Af-Gm-Uf-Cm-Af-Cm- Cf-Gms-Afs-Gm | 1179 |
| B994-AL | Gfs-Ums-Gf-Am-Cf-Um-Cf-Am-Af- Gm-Uf-Gm-Cf-Cm-Cf-Um-Uf-Cm- Af-Cm-Uf | 1094 | Ams-Gfs-Um-Gf-Am-Af-Gm-Gf-Gm- Cf-Am-Cf-Um-Uf-Gm-Af-Gm-Uf-Cm- Af-Cms-Cfs-Gm | 1180 |
| C1835-DV29P | Ums-Ums-Gm-Gm-Gm-Um-Uf-Um- Uf-Am-Af-Am-Am-Um-Um-Am-Af- Am-Gm-Um-Am | 1181 | UmsEVP-Afs-Cm-Uf-Uf-Uf-Am-Am- Um-Um-Um-Um-Am-Af-Am-Af-Cm- Cm-Cm-Am-Ams-Ums-Um | 1216 |
| C1835-DV26P | Ums-Ums-Gm-Gm-Gm-Um-Uf-Um- Uf-Af-Af-Am-Am-Um-Um-Am-Af- Am-Gm-Um-Am | 1182 | UmsEVP-Afs-Cf-Uf-Um-Uf-Am-Am- Um-Um-Um-Um-Am-Af-Am-Af-Cm- Cm-Cm-Am-Ams-Ums-Um | 1217 |
| C1835-DV27P | Ums-Ums-Gm-Gm-Gm-Um-Uf-Um- Uf-Af-Af-Am-Am-Um-Um-Am-Af- Am-Gm-Um-Am | 1182 | UmsEVP-Afs-Cm-Uf-Uf-Uf-Am-Am- Um-Um-Um-Um-Am-Af-Am-Af-Cm- Cm-Cm-Am-Ams-Ums-Um | 1216 |
| C1835-DV28P | Ums-Ums-Gm-Gm-Gm-Um-Uf-Um- Uf-Am-Af-Am-Am-Um-Um-Am-Af- Am-Gm-Um-Am | 1181 | UmsEVP-Afs-Cf-Uf-Um-Uf-Am-Am- Um-Um-Um-Um-Am-Af-Am-Af-Cm- Cm-Cm-Am-Ams-Ums-Um | 1217 |
| C1835-DV32P | Ums-Ums-Gm-Gm-Gm-Um-Uf-Um- Uf-Am-Af-Am-Am-Um-Um-Am-Af- Am-Gm-Um-Am | 1181 | UmsEVP-Afs-Cm-Um-Uf-Uf-Am-Am- Um-Um-Um-Um-Am-Af-Am-Af-Cm- Cm-Cm-Am-Ams-Ums-Um | 1218 |
| C1835-DV34P | Ums-Ums-Gm-Gm-Gm-Um-Uf-Um- Uf-Am-Af-Am-Am-Um-Um-Am-Af- Am-Gm-Um-Am | 1181 | UmsEVP-Afs-Cf-Um-Um-Uf-Am-Am- Um-Um-Um-Um-Am-Af-Am-Af-Cm- Cm-Cm-Am-Ams-Ums-Um | 1219 |
| C1835-DV25P | Ums-Ums-Gm-Gm-Gm-Um-Uf-Um- Uf-Af-Af-Am-Am-Um-Um-Am-Af- Am-Gm-Um-Am | 1182 | UmsEVP-Afs-Cm-Um-Um-Uf-Am-Am- Um-Um-Um-Um-Am-Af-Am-Af-Cm- Cm-Cm-Am-Ams-Ums-Um | 1220 |
| C1835-DV33P | Ums-Ums-Gm-Gm-Gm-Um-Uf-Um- Uf-Am-Af-Am-Am-Um-Um-Am-Af- Am-Gm-Um-Am | 1181 | UmsEVP-Afs-Cf-Um-Uf-Uf-Am-Am- Um-Um-Um-Um-Am-Af-Am-Af-Cm- Cm-Cm-Am-Ams-Ums-Um | 1221 |
| C1835-DV22 | Ums-Ums-Gm-Gm-Gm-Um-Uf-Um- Uf-Af-Af-Am-Am-Um-Um-Am-Am- Am-Gm-Um-Am | 1183 | Ums-Afs-Cm-Um-Um-Uf-Am-Am-Um- Um-Um-Um-Am-Af-Am-Af-Cm-Cm- Cm-Am-Ams-Ums-Um | 1222 |
| C1816-DV29P | Ums-Ums-Cm-Am-Am-Gm-Uf-Um- Gf-Am-Gf-Am-Am-Cm-Am-Am-Af- Am-Am-Um-Um | 1184 | AmsEVP-Afs-Um-Uf-Uf-Uf-Um-Gm- Um-Um-Cm-Um-Cm-Af-Am-Cf-Um- Um-Gm-Am-Ams-Ams-Am | 1223 |
| C1816-DV26P | Ums-Ums-Cm-Am-Am-Gm-Uf-Um- Gf-Af-Gf-Am-Am-Cm-Am-Am-Af- Am-Am-Um-Um | 1185 | AmsEVP-Afs-Uf-Uf-Um-Uf-Um-Gm- Um-Um-Cm-Um-Cm-Af-Am-Cf-Um- Um-Gm-Am-Ams-Ams-Am | 1224 |
| C1816-DV27P | Ums-Ums-Cm-Am-Am-Gm-Uf-Um- Gf-Af-Gf-Am-Am-Cm-Am-Am-Af- Am-Am-Um-Um | 1185 | AmsEVP-Afs-Um-Uf-Uf-Uf-Um-Gm- Um-Um-Cm-Um-Cm-Af-Am-Cf-Um- Um-Gm-Am-Ams-Ams-Am | 1223 |
| C1816-DV28P | Ums-Ums-Cm-Am-Am-Gm-Uf-Um- Gf-Am-Gf-Am-Am-Cm-Am-Am-Af- Am-Am-Um-Um | 1184 | AmsEVP-Afs-Uf-Uf-Um-Uf-Um-Gm- Um-Um-Cm-Um-Cm-Af-Am-Cf-Um- Um-Gm-Am-Ams-Ams-Am | 1224 |
| C1816-DV32P | Ums-Ums-Cm-Am-Am-Gm-Uf-Um- Gf-Am-Gf-Am-Am-Cm-Am-Am-Af- Am-Am-Um-Um | 1184 | AmsEVP-Afs-Um-Um-Uf-Uf-Um-Gm- Um-Um-Cm-Um-Cm-Af-Am-Cf-Um- Um-Gm-Am-Ams-Ams-Am | 1225 |
| C1816-DV34P | Ums-Ums-Cm-Am-Am-Gm-Uf-Um- Gf-Am-Gf-Am-Am-Cm-Am-Am-Af- Am-Am-Um-Um | 1184 | AmsEVP-Afs-Uf-Um-Um-Uf-Um-Gm- Um-Um-Cm-Um-Cm-Af-Am-Cf-Um- Um-Gm-Am-Ams-Ams-Am | 1226 |
| C1816-DV25P | Ums-Ums-Cm-Am-Am-Gm-Uf-Um- Gf-Af-Gf-Am-Am-Cm-Am-Am-Af- Am-Am-Um-Um | 1185 | AmsEVP-Afs-Um-Um-Um-Uf-Um-Gm- Um-Um-Cm-Um-Cm-Af-Am-Cf-Um- Um-Gm-Am-Ams-Ams-Am | 1227 |
| C1816-DV33P | Ums-Ums-Cm-Am-Am-Gm-Uf-Um- Gf-Am-Gf-Am-Am-Cm-Am-Am-Af- Am-Am-Um-Um | 1184 | AmsEVP-Afs-Uf-Um-Uf-Uf-Um-Gm- Um-Um-Cm-Um-Cm-Af-Am-Cf-Um- Um-Gm-Am-Ams-Ams-Am | 1228 |
| C1816-DV22 | Ums-Ums-Cm-Am-Am-Gm-Uf-Um- Gf-Af-Gf-Am-Am-Cm-Am-Am-Am- Am-Am-Um-Um | 1186 | Ams-Afs-Um-Um-Um-Uf-Um-Gm-Um- Um-Cm-Um-Cm-Af-Am-Cf-Um-Um- Gm-Am-Ams-Ams-Am | 1229 |
| C1812-DV29P | Cms-Cms-Um-Um-Um-Um-Cf-Am- Af-Gm-Uf-Um-Gm-Am-Gm-Am-Af- Cm-Am-Am-Am | 1187 | UmsEVP-Ufs-Um-Gf-Uf-Uf-Cm-Um- Cm-Am-Am-Cm-Um-Uf-Gm-Af-Am- Am-Am-Gm-Gms-Gms-Am | 1230 |
| C1812-DV26P | Cms-Cms-Um-Um-Um-Um-Cf-Am- Af-Gf-Uf-Um-Gm-Am-Gm-Am-Af- Cm-Am-Am-Am | 1188 | UmsEVP-Ufs-Uf-Gf-Um-Uf-Cm-Um- Cm-Am-Am-Cm-Um-Uf-Gm-Af-Am- Am-Am-Gm-Gms-Gms-Am | 1231 |
| C1812-DV27P | Cms-Cms-Um-Um-Um-Um-Cf-Am- Af-Gf-Uf-Um-Gm-Am-Gm-Am-Af- Cm-Am-Am-Am | 1188 | UmsEVP-Ufs-Um-Gf-Uf-Uf-Cm-Um- Cm-Am-Am-Cm-Um-Uf-Gm-Af-Am- Am-Am-Gm-Gms-Gms-Am | 1230 |
| C1812-DV28P | Cms-Cms-Um-Um-Um-Um-Cf-Am- Af-Gm-Uf-Um-Gm-Am-Gm-Am-Af- Cm-Am-Am-Am | 1187 | UmsEVP-Ufs-Uf-Gf-Um-Uf-Cm-Um- Cm-Am-Am-Cm-Um-Uf-Gm-Af-Am- Am-Am-Gm-Gms-Gms-Am | 1231 |
| C1812-DV32P | Cms-Cms-Um-Um-Um-Um-Cf-Am- Af-Gm-Uf-Um-Gm-Am-Gm-Am-Af- Cm-Am-Am-Am | 1187 | UmsEVP-Ufs-Um-Gm-Uf-Uf-Cm-Um- Cm-Am-Am-Cm-Um-Uf-Gm-Af-Am- Am-Am-Gm-Gms-Gms-Am | 1232 |
| C1812-DV34P | Cms-Cms-Um-Um-Um-Um-Cf-Am- Af-Gm-Uf-Um-Gm-Am-Gm-Am-Af- Cm-Am-Am-Am | 1187 | UmsEVP-Ufs-Uf-Gm-Um-Uf-Cm-Um- Cm-Am-Am-Cm-Um-Uf-Gm-Af-Am- Am-Am-Gm-Gms-Gms-Am | 1233 |
| C1812-DV25P | Cms-Cms-Um-Um-Um-Um-Cf-Am- Af-Gf-Uf-Um-Gm-Am-Gm-Am-Af- Cm-Am-Am-Am | 1188 | UmsEVP-Ufs-Um-Gm-Um-Uf-Cm-Um- Cm-Am-Am-Cm-Um-Uf-Gm-Af-Am- Am-Am-Gm-Gms-Gms-Am | 1234 |
| C1812-DV33P | Cms-Cms-Um-Um-Um-Um-Cf-Am- Af-Gm-Uf-Um-Gm-Am-Gm-Am-Af- Cm-Am-Am-Am | 1187 | UmsEVP-Ufs-Uf-Gm-Uf-Uf-Cm-Um- Cm-Am-Am-Cm-Um-Uf-Gm-Af-Am- Am-Am-Gm-Gms-Gms-Am | 1235 |
| C1812-DV22 | Cms-Cms-Um-Um-Um-Um-Cf-Am- Af-Gf-Uf-Um-Gm-Am-Gm-Am-Am- Cm-Am-Am-Am | 1189 | Ums-Ufs-Um-Gm-Um-Uf-Cm-Um-Cm- Am-Am-Cm-Um-Uf-Gm-Af-Am-Am- Am-Gm-Gms-Gms-Am | 1236 |
| C1579-DV29P | Cms-Cms-Um-Um-Um-Um-Cf-Um- Uf-Cm-Uf-Am-Am-Um-Gm-Am-Gf- Um-Cm-Gm-Am | 1190 | UmsEVP-Cfs-Gm-Af-Cf-Uf-Cm-Am- Um-Um-Am-Gm-Am-Af-Gm-Af-Am- Am-Am-Gm-Gms-Ums-Gm | 1237 |
| C1579-DV26P | Cms-Cms-Um-Um-Um-Um-Cf-Um- Uf-Cf-Uf-Am-Am-Um-Gm-Am-Gf- Um-Cm-Gm-Am | 1191 | UmsEVP-Cfs-Gf-Af-Cm-Uf-Cm-Am- Um-Um-Am-Gm-Am-Af-Gm-Af-Am- Am-Am-Gm-Gms-Ums-Gm | 1238 |
| C1579-DV27P | Cms-Cms-Um-Um-Um-Um-Cf-Um- Uf-Cf-Uf-Am-Am-Um-Gm-Am-Gf- Um-Cm-Gm-Am | 1191 | UmsEVP-Cfs-Gm-Af-Cf-Uf-Cm-Am- Um-Um-Am-Gm-Am-Af-Gm-Af-Am- Am-Am-Gm-Gms-Ums-Gm | 1237 |
| C1579-DV28P | Cms-Cms-Um-Um-Um-Um-Cf-Um- Uf-Cm-Uf-Am-Am-Um-Gm-Am-Gf- Um-Cm-Gm-Am | 1190 | UmsEVP-Cfs-Gf-Af-Cm-Uf-Cm-Am- Um-Um-Am-Gm-Am-Af-Gm-Af-Am- Am-Am-Gm-Gms-Ums-Gm | 1238 |
| C1579-DV32P | Cms-Cms-Um-Um-Um-Um-Cf-Um- Uf-Cm-Uf-Am-Am-Um-Gm-Am-Gf- Um-Cm-Gm-Am | 1190 | UmsEVP-Cfs-Gm-Am-Cf-Uf-Cm-Am- Um-Um-Am-Gm-Am-Af-Gm-Af-Am- Am-Am-Gm-Gms-Ums-Gm | 1239 |
| C1579-DV34P | Cms-Cms-Um-Um-Um-Um-Cf-Um- Uf-Cm-Uf-Am-Am-Um-Gm-Am-Gf- Um-Cm-Gm-Am | 1190 | UmsEVP-Cfs-Gf-Am-Cm-Uf-Cm-Am- Um-Um-Am-Gm-Am-Af-Gm-Af-Am- Am-Am-Gm-Gms-Ums-Gm | 1240 |
| C1579-DV25P | Cms-Cms-Um-Um-Um-Um-Cf-Um- Uf-Cf-Uf-Am-Am-Um-Gm-Am-Gf- Um-Cm-Gm-Am | 1191 | UmsEVP-Cfs-Gm-Am-Cm-Uf-Cm-Am- Um-Um-Am-Gm-Am-Af-Gm-Af-Am- Am-Am-Gm-Gms-Ums-Gm | 1241 |
| C1579-DV33P | Cms-Cms-Um-Um-Um-Um-Cf-Um- Uf-Cm-Uf-Am-Am-Um-Gm-Am-Gf- Um-Cm-Gm-Am | 1190 | UmsEVP-Cfs-Gf-Am-Cf-Uf-Cm-Am- Um-Um-Am-Gm-Am-Af-Gm-Af-Am- Am-Am-Gm-Gms-Ums-Gm | 1242 |
| C1579-DV22 | Cms-Cms-Um-Um-Um-Um-Cf-Um- Uf-Cf-Uf-Am-Am-Um-Gm-Am-Gm- Um-Cm-Gm-Am | 1192 | Ums-Cfs-Gm-Am-Cm-Uf-Cm-Am-Um- Um-Am-Gm-Am-Af-Gm-Af-Am-Am- Am-Gm-Gms-Ums-Gm | 1243 |
| C1836-DV29P | Ums-Gms-Gm-Gm-Um-Um-Uf-Um- Af-Am-Af-Am-Um-Um-Am-Am-Af- Gm-Um-Am-Um | 1193 | AmsEVP-Ufs-Am-Cf-Uf-Uf-Um-Am- Am-Um-Um-Um-Um-Af-Am-Af-Am- Cm-Cm-Cm-Ams-Ams-Um | 1244 |
| C1836-DV26P | Ums-Gms-Gm-Gm-Um-Um-Uf-Um- Af-Af-Af-Am-Um-Um-Am-Am-Af- Gm-Um-Am-Um | 1194 | AmsEVP-Ufs-Af-Cf-Um-Uf-Um-Am- Am-Um-Um-Um-Um-Af-Am-Af-Am- Cm-Cm-Cm-Ams-Ams-Um | 1245 |
| C1836-DV27P | Ums-Gms-Gm-Gm-Um-Um-Uf-Um- Af-Af-Af-Am-Um-Um-Am-Am-Af- Gm-Um-Am-Um | 1194 | AmsEVP-Ufs-Am-Cf-Uf-Uf-Um-Am- Am-Um-Um-Um-Um-Af-Am-Af-Am- Cm-Cm-Cm-Ams-Ams-Um | 1244 |
| C1836-DV28P | Ums-Gms-Gm-Gm-Um-Um-Uf-Um- Af-Am-Af-Am-Um-Um-Am-Am-Af- Gm-Um-Am-Um | 1193 | AmsEVP-Ufs-Af-Cf-Um-Uf-Um-Am- Am-Um-Um-Um-Um-Af-Am-Af-Am- Cm-Cm-Cm-Ams-Ams-Um | 1245 |
| C1836-DV32P | Ums-Gms-Gm-Gm-Um-Um-Uf-Um- Af-Am-Af-Am-Um-Um-Am-Am-Af- Gm-Um-Am-Um | 1193 | AmsEVP-Ufs-Am-Cm-Uf-Uf-Um-Am- Am-Um-Um-Um-Um-Af-Am-Af-Am- Cm-Cm-Cm-Ams-Ams-Um | 1246 |
| C1836-DV34P | Ums-Gms-Gm-Gm-Um-Um-Uf-Um- Af-Am-Af-Am-Um-Um-Am-Am-Af- Gm-Um-Am-Um | 1193 | AmsEVP-Ufs-Af-Cm-Um-Uf-Um-Am- Am-Um-Um-Um-Um-Af-Am-Af-Am- Cm-Cm-Cm-Ams-Ams-Um | 1247 |
| C1789-DV29P | Gms-Ums-Um-Um-Gm-Um-Gf-Am- Af-Am-Cf-Am-Am-Am-Am-Am-Af- Gm-Um-Gm-Um | 1195 | AmsEVP-Cfs-Am-Cf-Uf-Uf-Um-Um- Um-Um-Gm-Um-Um-Uf-Cm-Af-Cm- Am-Am-Am-Cms-Ams-Am | 1248 |
| C1789-DV26P | Gms-Ums-Um-Um-Gm-Um-Gf-Am- Af-Af-Cf-Am-Am-Am-Am-Am-Af- Gm-Um-Gm-Um | 1196 | AmsEVP-Cfs-Af-Cf-Um-Uf-Um-Um- Um-Um-Gm-Um-Um-Uf-Cm-Af-Cm- Am-Am-Am-Cms-Ams-Am | 1249 |
| C1789-DV27P | Gms-Ums-Um-Um-Gm-Um-Gf-Am- Af-Af-Cf-Am-Am-Am-Am-Am-Af- Gm-Um-Gm-Um | 1196 | AmsEVP-Cfs-Am-Cf-Uf-Uf-Um-Um- Um-Um-Gm-Um-Um-Uf-Cm-Af-Cm- Am-Am-Am-Cms-Ams-Am | 1248 |
| C1789-DV28P | Gms-Ums-Um-Um-Gm-Um-Gf-Am- Af-Am-Cf-Am-Am-Am-Am-Am-Af- Gm-Um-Gm-Um | 1195 | AmsEVP-Cfs-Af-Cf-Um-Uf-Um-Um- Um-Um-Gm-Um-Um-Uf-Cm-Af-Cm- Am-Am-Am-Cms-Ams-Am | 1249 |
| C1789-DV32P | Gms-Ums-Um-Um-Gm-Um-Gf-Am- Af-Am-Cf-Am-Am-Am-Am-Am-Af- Gm-Um-Gm-Um | 1195 | AmsEVP-Cfs-Am-Cm-Uf-Uf-Um-Um- Um-Um-Gm-Um-Um-Uf-Cm-Af-Cm- Am-Am-Am-Cms-Ams-Am | 1250 |
| C1789-DV34P | Gms-Ums-Um-Um-Gm-Um-Gf-Am- Af-Am-Cf-Am-Am-Am-Am-Am-Af- Gm-Um-Gm-Um | 1195 | AmsEVP-Cfs-Af-Cm-Um-Uf-Um-Um- Um-Um-Gm-Um-Um-Uf-Cm-Af-Cm- Am-Am-Am-Cms-Ams-Am | 1251 |
| C1789-DV25P | Gms-Ums-Um-Um-Gm-Um-Gf-Am- Af-Af-Cf-Am-Am-Am-Am-Am-Af- Gm-Um-Gm-Um | 1196 | AmsEVP-Cfs-Am-Cm-Um-Uf-Um-Um- Um-Um-Gm-Um-Um-Uf-Cm-Af-Cm- Am-Am-Am-Cms-Ams-Am | 1252 |
| C1789-DV33P | Gms-Ums-Um-Um-Gm-Um-Gf-Am- Af-Am-Cf-Am-Am-Am-Am-Am-Af- Gm-Um-Gm-Um | 1195 | AmsEVP-Cfs-Af-Cm-Uf-Uf-Um-Um- Um-Um-Gm-Um-Um-Uf-Cm-Af-Cm- Am-Am-Am-Cms-Ams-Am | 1253 |
| C1789-DV22 | Gms-Ums-Um-Um-Gm-Um-Gf-Am- Af-Af-Cf-Am-Am-Am-Am-Am-Am- Gm-Um-Gm-Um | 1197 | Ams-Cfs-Am-Cm-Um-Uf-Um-Um-Um- Um-Gm-Um-Um-Uf-Cm-Af-Cm-Am- Am-Am-Cms-Ams-Am | 1254 |
| C1839-DV29P | Gms-Ums-Um-Um-Um-Am-Af-Am- Af-Um-Uf-Am-Am-Am-Gm-Um-Af- Um-Am-Cm-Am | 1198 | UmsEVP-Gfs-Um-Af-Uf-Af-Cm-Um- Um-Um-Am-Am-Um-Uf-Um-Uf-Am- Am-Am-Am-Cms-Cms-Cm | 1255 |
| C1839-DV26P | Gms-Ums-Um-Um-Um-Am-Af-Am- Af-Uf-Uf-Am-Am-Am-Gm-Um-Af- Um-Am-Cm-Am | 1199 | UmsEVP-Gfs-Uf-Af-Um-Af-Cm-Um- Um-Um-Am-Am-Um-Uf-Um-Uf-Am- Am-Am-Am-Cms-Cms-Cm | 1256 |
| C1839-DV27P | Gms-Ums-Um-Um-Um-Am-Af-Am- Af-Uf-Uf-Am-Am-Am-Gm-Um-Af- Um-Am-Cm-Am | 1199 | UmsEVP-Gfs-Um-Af-Uf-Af-Cm-Um- Um-Um-Am-Am-Um-Uf-Um-Uf-Am- Am-Am-Am-Cms-Cms-Cm | 1255 |
| C1839-DV28P | Gms-Ums-Um-Um-Um-Am-Af-Am- Af-Um-Uf-Am-Am-Am-Gm-Um-Af- Um-Am-Cm-Am | 1198 | UmsEVP-Gfs-Uf-Af-Um-Af-Cm-Um- Um-Um-Am-Am-Um-Uf-Um-Uf-Am- Am-Am-Am-Cms-Cms-Cm | 1256 |
| C1839-DV32P | Gms-Ums-Um-Um-Um-Am-Af-Am- Af-Um-Uf-Am-Am-Am-Gm-Um-Af- Um-Am-Cm-Am | 1198 | UmsEVP-Gfs-Um-Am-Uf-Af-Cm-Um- Um-Um-Am-Am-Um-Uf-Um-Uf-Am- Am-Am-Am-Cms-Cms-Cm | 1257 |
| C1839-DV34P | Gms-Ums-Um-Um-Um-Am-Af-Am- Af-Um-Uf-Am-Am-Am-Gm-Um-Af- Um-Am-Cm-Am | 1198 | UmsEVP-Gfs-Uf-Am-Um-Af-Cm-Um- Um-Um-Am-Am-Um-Uf-Um-Uf-Am- Am-Am-Am-Cms-Cms-Cm | 1258 |
| C1791-DV29P | Ums-Ums-Gm-Um-Gm-Am-Af-Am- Cf-Am-Af-Am-Am-Am-Am-Gm-Uf- Gm-Um-Um-Am | 1200 | UmsEVP-Afs-Am-Cf-Af-Cf-Um-Um- Um-Um-Um-Um-Gm-Uf-Um-Uf-Cm- Am-Cm-Am-Ams-Ams-Cm | 1259 |
| C1791-DV26P | Ums-Ums-Gm-Um-Gm-Am-Af-Am- Cf-Af-Af-Am-Am-Am-Am-Gm-Uf- Gm-Um-Um-Am | 1201 | UmsEVP-Afs-Af-Cf-Am-Cf-Um-Um- Um-Um-Um-Um-Gm-Uf-Um-Uf-Cm- Am-Cm-Am-Ams-Ams-Cm | 1260 |
| C1791-DV27P | Ums-Ums-Gm-Um-Gm-Am-Af-Am- Cf-Af-Af-Am-Am-Am-Am-Gm-Uf- Gm-Um-Um-Am | 1201 | UmsEVP-Afs-Am-Cf-Af-Cf-Um-Um- Um-Um-Um-Um-Gm-Uf-Um-Uf-Cm- Am-Cm-Am-Ams-Ams-Cm | 1259 |
| C1791-DV28P | Ums-Ums-Gm-Um-Gm-Am-Af-Am- Cf-Am-Af-Am-Am-Am-Am-Gm-Uf- Gm-Um-Um-Am | 1200 | UmsEVP-Afs-Af-Cf-Am-Cf-Um-Um- Um-Um-Um-Um-Gm-Uf-Um-Uf-Cm- Am-Cm-Am-Ams-Ams-Cm | 1260 |
| C1791-DV32P | Ums-Ums-Gm-Um-Gm-Am-Af-Am- Cf-Am-Af-Am-Am-Am-Am-Gm-Uf- Gm-Um-Um-Am | 1200 | UmsEVP-Afs-Am-Cm-Af-Cf-Um-Um- Um-Um-Um-Um-Gm-Uf-Um-Uf-Cm- Am-Cm-Am-Ams-Ams-Cm | 1261 |
| C1791-DV34P | Ums-Ums-Gm-Um-Gm-Am-Af-Am- Cf-Am-Af-Am-Am-Am-Am-Gm-Uf- Gm-Um-Um-Am | 1200 | UmsEVP-Afs-Af-Cm-Am-Cf-Um-Um- Um-Um-Um-Um-Gm-Uf-Um-Uf-Cm- Am-Cm-Am-Ams-Ams-Cm | 1262 |
| C1795-DV29P | Gms-Ams-Am-Am-Cm-Am-Af-Am- Af-Am-Af-Gm-Um-Gm-Um-Um-Cf- Cm-Cm-Um-Um | 1202 | AmsEVP-Afs-Gm-Gf-Gf-Af-Am-Cm- Am-Cm-Um-Um-Um-Uf-Um-Uf-Gm- Um-Um-Um-Cms-Ams-Cm | 1263 |
| C1795-DV26P | Gms-Ams-Am-Am-Cm-Am-Af-Am- Af-Af-Af-Gm-Um-Gm-Um-Um-Cf- Cm-Cm-Um-Um | 1203 | AmsEVP-Afs-Gf-Gf-Gm-Af-Am-Cm- Am-Cm-Um-Um-Um-Uf-Um-Uf-Gm- Um-Um-Um-Cms-Ams-Cm | 1264 |
| C1795-DV27P | Gms-Ams-Am-Am-Cm-Am-Af-Am- Af-Af-Af-Gm-Um-Gm-Um-Um-Cf- Cm-Cm-Um-Um | 1203 | AmsEVP-Afs-Gm-Gf-Gf-Af-Am-Cm- Am-Cm-Um-Um-Um-Uf-Um-Uf-Gm- Um-Um-Um-Cms-Ams-Cm | 1263 |
| C1795-DV28P | Gms-Ams-Am-Am-Cm-Am-Af-Am- Af-Am-Af-Gm-Um-Gm-Um-Um-Cf- Cm-Cm-Um-Um | 1202 | AmsEVP-Afs-Gf-Gf-Gm-Af-Am-Cm- Am-Cm-Um-Um-Um-Uf-Um-Uf-Gm- Um-Um-Um-Cms-Ams-Cm | 1264 |
| C1795-DV32P | Gms-Ams-Am-Am-Cm-Am-Af-Am- Af-Am-Af-Gm-Um-Gm-Um-Um-Cf- Cm-Cm-Um-Um | 1202 | AmsEVP-Afs-Gm-Gm-Gf-Af-Am-Cm- Am-Cm-Um-Um-Um-Uf-Um-Uf-Gm- Um-Um-Um-Cms-Ams-Cm | 1265 |
| C1795-DV34P | Gms-Ams-Am-Am-Cm-Am-Af-Am- Af-Am-Af-Gm-Um-Gm-Um-Um-Cf- Cm-Cm-Um-Um | 1202 | AmsEVP-Afs-Gf-Gm-Gm-Af-Am-Cm- Am-Cm-Um-Um-Um-Uf-Um-Uf-Gm- Um-Um-Um-Cms-Ams-Cm | 1266 |
| C1585s-DV29SP | Cms-Ums-Um-Cm-Um-Af-Am-Uf- Gm-Af-Gm-Um-Cm-Gm-Am-Cf- Um-Um-Um-Am | 1204 | UmsEVP-Afs-Am-Af-Gf-Uf-Cm-Gm- Am-Cm-Um-Cm-Am-Uf-Um-Af-Gm- Am-Am-Gms-Ams-Am | 1267 |
| C1585s-DV26SP | Cms-Ums-Um-Cm-Um-Af-Am-Uf- Gf-Af-Gm-Um-Cm-Gm-Am-Cf-Um- Um-Um-Am | 1205 | UmsEVP-Afs-Af-Af-Gm-Uf-Cm-Gm- Am-Cm-Um-Cm-Am-Uf-Um-Af-Gm- Am-Am-Gms-Ams-Am | 1268 |
| C1585s-DV27SP | Cms-Ums-Um-Cm-Um-Af-Am-Uf- Gf-Af-Gm-Um-Cm-Gm-Am-Cf-Um- Um-Um-Am | 1205 | UmsEVP-Afs-Am-Af-Gf-Uf-Cm-Gm- Am-Cm-Um-Cm-Am-Uf-Um-Af-Gm- Am-Am-Gms-Ams-Am | 1267 |
| C1585s-DV28SP | Cms-Ums-Um-Cm-Um-Af-Am-Uf- Gm-Af-Gm-Um-Cm-Gm-Am-Cf- Um-Um-Um-Am | 1204 | UmsEVP-Afs-Af-Af-Gm-Uf-Cm-Gm- Am-Cm-Um-Cm-Am-Uf-Um-Af-Gm- Am-Am-Gms-Ams-Am | 1268 |
| C1585s-DV32SP | Cms-Ums-Um-Cm-Um-Af-Am-Uf- Gm-Af-Gm-Um-Cm-Gm-Am-Cf- Um-Um-Um-Am | 1204 | UmsEVP-Afs-Am-Am-Gf-Uf-Cm-Gm- Am-Cm-Um-Cm-Am-Uf-Um-Af-Gm- Am-Am-Gms-Ams-Am | 1269 |
| C1585s-DV34SP | Cms-Ums-Um-Cm-Um-Af-Am-Uf- Gm-Af-Gm-Um-Cm-Gm-Am-Cf- Um-Um-Um-Am | 1204 | UmsEVP-Afs-Af-Am-Gm-Uf-Cm-Gm- Am-Cm-Um-Cm-Am-Uf-Um-Af-Gm- Am-Am-Gms-Ams-Am | 1270 |
| C1585s-DV22S | Cms-Ums-Um-Cm-Um-Af-Am-Uf- Gf-Af-Gm-Um-Cm-Gm-Am-Cm- Um-Um-Um-Am | 1206 | Ums-Afs-Am-Am-Gm-Uf-Cm-Gm-Am- Cm-Um-Cm-Am-Uf-Um-Af-Gm-Am- Am-Gms-Ams-Am | 1271 |
| C1576s-DV29SP | Cms-Cms-Am-Cm-Cm-Uf-Um-Uf- Um-Cf-Um-Um-Cm-Um-Am-Af- Um-Gm-Am-Am | 1207 | UmsEVP-Ufs-Cm-Af-Uf-Uf-Am-Gm- Am-Am-Gm-Am-Am-Af-Am-Gf-Gm- Um-Gm-Gms-Gms-Am | 1272 |
| C1576s-DV28SP | Cms-Cms-Am-Cm-Cm-Uf-Um-Uf- Um-Cf-Um-Um-Cm-Um-Am-Af- Um-Gm-Am-Am | 1207 | UmsEVP-Ufs-Cf-Af-Um-Uf-Am-Gm- Am-Am-Gm-Am-Am-Af-Am-Gf-Gm- Um-Gm-Gms-Gms-Am | 1273 |
| C1576s-DV26SP | Cms-Cms-Am-Cm-Cm-Uf-Um-Uf- Uf-Cf-Um-Um-Cm-Um-Am-Af-Um- Gm-Am-Am | 1208 | UmsEVP-Ufs-Cf-Af-Um-Uf-Am-Gm- Am-Am-Gm-Am-Am-Af-Am-Gf-Gm- Um-Gm-Gms-Gms-Am | 1273 |
| C1576s-DV25SP | Cms-Cms-Am-Cm-Cm-Uf-Um-Uf- Uf-Cf-Um-Um-Cm-Um-Am-Af-Um- Gm-Am-Am | 1208 | UmsEVP-Ufs-Cm-Am-Um-Uf-Am-Gm- Am-Am-Gm-Am-Am-Af-Am-Gf-Gm- Um-Gm-Gms-Gms-Am | 1274 |
| C1576s-DV27SP | Cms-Cms-Am-Cm-Cm-Uf-Um-Uf- Uf-Cf-Um-Um-Cm-Um-Am-Af-Um- Gm-Am-Am | 1208 | UmsEVP-Ufs-Cm-Af-Uf-Uf-Am-Gm- Am-Am-Gm-Am-Am-Af-Am-Gf-Gm- Um-Gm-Gms-Gms-Am | 1272 |
| C1576s-DV22S | Cms-Cms-Am-Cm-Cm-Uf-Um-Uf- Uf-Cf-Um-Um-Cm-Um-Am-Am- Um-Gm-Am-Am | 1209 | Ums-Ufs-Cm-Am-Um-Uf-Am-Gm-Am- Am-Gm-Am-Am-Af-Am-Gf-Gm-Um- Gm-Gms-Gms-Am | 1275 |
| C1578s-DV29SP | Ams-Cms-Cm-Um-Um-Uf-Um-Cf- Um-Uf-Cm-Um-Am-Am-Um-Gf- Am-Gm-Um-Am | 1210 | UmsEVP-Afs-Cm-Uf-Cf-Af-Um-Um- Am-Gm-Am-Am-Gm-Af-Am-Af-Am- Gm-Gm-Ums-Gms-Gm | 1276 |
| C1578s-DV28SP | Ams-Cms-Cm-Um-Um-Uf-Um-Cf- Um-Uf-Cm-Um-Am-Am-Um-Gf- Am-Gm-Um-Am | 1210 | UmsEVP-Afs-Cf-Uf-Cm-Af-Um-Um- Am-Gm-Am-Am-Gm-Af-Am-Af-Am- Gm-Gm-Ums-Gms-Gm | 1277 |
| C1578s-DV26SP | Ams-Cms-Cm-Um-Um-Uf-Um-Cf- Uf-Uf-Cm-Um-Am-Am-Um-Gf-Am- Gm-Um-Am | 1211 | UmsEVP-Afs-Cf-Uf-Cm-Af-Um-Um- Am-Gm-Am-Am-Gm-Af-Am-Af-Am- Gm-Gm-Ums-Gms-Gm | 1277 |
| C1578s-DV25SP | Ams-Cms-Cm-Um-Um-Uf-Um-Cf- Uf-Uf-Cm-Um-Am-Am-Um-Gf-Am- Gm-Um-Am | 1211 | UmsEVP-Afs-Cm-Um-Cm-Af-Um-Um- Am-Gm-Am-Am-Gm-Af-Am-Af-Am- Gm-Gm-Ums-Gms-Gm | 1278 |
| C1578s-DV27SP | Ams-Cms-Cm-Um-Um-Uf-Um-Cf- Uf-Uf-Cm-Um-Am-Am-Um-Gf-Am- Gm-Um-Am | 1211 | UmsEVP-Afs-Cm-Uf-Cf-Af-Um-Um- Am-Gm-Am-Am-Gm-Af-Am-Af-Am- Gm-Gm-Ums-Gms-Gm | 1276 |
| C1578s-DV22S | Ams-Cms-Cm-Um-Um-Uf-Um-Cf- Uf-Uf-Cm-Um-Am-Am-Um-Gm- Am-Gm-Um-Am | 1212 | Ums-Afs-Cm-Um-Cm-Af-Um-Um-Am- Gm-Am-Am-Gm-Af-Am-Af-Am-Gm- Gm-Ums-Gms-Gm | 1279 |
| C1838-DV29P | Gms-Gms-Um-Um-Um-Um-Af-Am- Af-Am-Uf-Um-Am-Am-Am-Gm-Uf- Am-Um-Am-Am | 1213 | UmsEVP-Ufs-Am-Uf-Af-Cf-Um-Um- Um-Am-Am-Um-Um-Uf-Um-Af-Am- Am-Am-Cm-Cms-Cms-Am | 1280 |
| C1838-DV28P | Gms-Gms-Um-Um-Um-Um-Af-Am- Af-Am-Uf-Um-Am-Am-Am-Gm-Uf- Am-Um-Am-Am | 1213 | UmsEVP-Ufs-Af-Uf-Am-Cf-Um-Um- Um-Am-Am-Um-Um-Uf-Um-Af-Am- Am-Am-Cm-Cms-Cms-Am | 1281 |
| C1838-DV26P | Gms-Gms-Um-Um-Um-Um-Af-Am- Af-Af-Uf-Um-Am-Am-Am-Gm-Uf- Am-Um-Am-Am | 1214 | UmsEVP-Ufs-Af-Uf-Am-Cf-Um-Um- Um-Am-Am-Um-Um-Uf-Um-Af-Am- Am-Am-Cm-Cms-Cms-Am | 1281 |
| C1838-DV25P | Gms-Gms-Um-Um-Um-Um-Af-Am- Af-Af-Uf-Um-Am-Am-Am-Gm-Uf- Am-Um-Am-Am | 1214 | UmsEVP-Ufs-Am-Um-Am-Cf-Um-Um- Um-Am-Am-Um-Um-Uf-Um-Af-Am- Am-Am-Cm-Cms-Cms-Am | 1282 |
| C1838-DV27P | Gms-Gms-Um-Um-Um-Um-Af-Am- Af-Af-Uf-Um-Am-Am-Am-Gm-Uf- Am-Um-Am-Am | 1214 | UmsEVP-Ufs-Am-Uf-Af-Cf-Um-Um- Um-Am-Am-Um-Um-Uf-Um-Af-Am- Am-Am-Cm-Cms-Cms-Am | 1280 |
| C1838-DV22 | Gms-Gms-Um-Um-Um-Um-Af-Am- Af-Af-Uf-Um-Am-Am-Am-Gm-Um- Am-Um-Am-Am | 1215 | Ums-Ufs-Am-Um-Am-Cf-Um-Um-Um- Am-Am-Um-Um-Uf-Um-Af-Am-Am- Am-Cm-Cms-Cms-Am | 1283 |
| 579P | ACCUUUUCUUCUAAUGAGUCU | 1284 | AGACUCAUUAGAAGAAAAGGUGG | 1297 |
| 789P | UUUGUGAAACAAAAAAGUGA | 1285 | UCACUUUUUUGUUUCACAAACA | 1298 |
| 812P | UUGUUCUCAACUUGAAAAGGG A | 1286 | CCUUUUCAAGUUGAGAACAA | 1299 |
| 576P | CCACCUUUUCUUCUAAUGAGU | 1287 | ACUCAUUAGAAGAAAAGGUGGGA | 1300 |
| 578P | CACCUUUUCUUCUAAUGAGUA | 1288 | UACUCAUUAGAAGAAAAGGUGGG | 1301 |
| 585P | UUCUAAUGAGUCGACUUUA | 1289 | UAAAGUCGACUCAUUAGAA | 1302 |
| AD-60771 | AGAACAAAAAUUGGGUUUUAA | 1290 | UUAAAACCCAAUUUUUGUUCUCA | 1303 |
| AD-85481 | GUCAUCCACAAUGAGAGUACA | 1291 | UGUACUCUCAUUGUGGAUGACGA | 1304 |
| 816P | UCAAGUUGAGAACAAAAAUA | 1292 | UAUUUUUGUUCUCAACUUGAAA | 1305 |
| 835P | GGGUUUUAAAAUUAAAGUAUA | 1293 | UACUUUAAUUUUAAAACCCAA | 1306 |
| 836P | GGUUUUAAAAUUAAAGUAUAC | 1294 | AUACUUUAAUUUUAAAACCCA | 1307 |
| 839P | GUUUUAAAAUUAAAGUAUACA | 1295 | UAUACUUUAAUUUUAAAACCC | 1308 |
| 791P | UUGUGAAACAAAAAAGUGUUU | 1296 | ACACUUUUUUGUUUCACAAUU | 1309 |
| D835-DV26P | Ums-Ums-Gm-Gm-Gm-Um-Uf-Um- Uf-Af-Af-Am-Am-Um-Um-Am-Af- Am-Gm-Um-Am | 1182 | UmsEVP-Afs-Cf-Uf-Um-Uf-Am-Am- Um-Um-Um-Um-Am-Af-Am-Af-Cm- Cm-Cm-Am-Ams-Ums-Um | 1217 |
| D835-DV26P+ | Ums-Ums-Gm-Gm-Gm-Um-Uf-Um- Uf-Af-Af-Am-Am-Um-Um-Am-Af- Am-Gm-Um-Am | 1182 | UmsEVP-Afs-Cf-Uf-Um-Tgna-Am-Am- Um-Um-Um-Um-Am-Af-Am-Af-Cm- Cm-Cm-Am-Ams-Ums-Um | 1348 |
| D835-DV26Pd7 B | Ums-Ums-Gm-Gm-Gm-Um-Uf-Um- Uf-Af-Af-Am-Am-Um-Td-Am-Af- Am-Gm-Um-Am | 1310 | UmsEVP-Afs-Cf-Uf-Um-Uf-Ad-Am- Um-Um-Um-Um-Am-Af-Am-Af-Cm- Cm-Cm-Am-Ams-Ums-Um | 1349 |
| D835-DV26Pd6 7B | Ums-Ums-Gm-Gm-Gm-Um-Uf-Um- Uf-Af-Af-Am-Am-Um-Td-Ad-Af- Am-Gm-Um-Am | 1311 | UmsEVP-Afs-Cf-Uf-Um-Td-Ad-Am- Um-Um-Um-Um-Am-Af-Am-Af-Cm- Cm-Cm-Am-Ams-Ums-Um | 1350 |
| D578s-DV29SP+ | Ams-Cms-Cm-Um-Um-Uf-Um-Cf- Um-Uf-Cm-Um-Am-Am-Um-Gf- Am-Gm-Um-Am | 1210 | UmsEVP-Afs-Cm-Uf-Cf-Agna-Um-Um- Am-Gm-Am-Am-Gm-Af-Am-Af-Am- Gm-Gm-Ums-Gms-Gm | 1351 |
| D578s-DV29SPd 67B | Ams-Cms-Cm-Um-Um-Uf-Um-Cf- Um-Uf-Cm-Um-Am-Ad-Td-Gf-Am- Gm-Um-Am | 1312 | UmsEVP-Afs-Cm-Uf-Cf-Ad-Td-Um- Am-Gm-Am-Am-Gm-Af-Am-Af-Am- Gm-Gm-Ums-Gms-Gm | 1352 |
| D578s-DV29SPd 7B | Ams-Cms-Cm-Um-Um-Uf-Um-Cf- Um-Uf-Cm-Um-Am-Ad-Um-Gf-Am- Gm-Um-Am | 1313 | UmsEVP-Afs-Cm-Uf-Cf-Af-Td-Um- Am-Gm-Am-Am-Gm-Af-Am-Af-Am- Gm-Gm-Ums-Gms-Gm | 1353 |
| D579-DV25PG5 | Cms-Cms-Um-Um-Um-Um-Cf-Um- Uf-Cf-Uf-Am-Am-Um-Gm-Am-Gf- Um-Cm-Gm-Am-G5 | 1314 | UmsEVP-Cfs-Gm-Am-Cm-Uf-Cm-Am- Um-Um-Am-Gm-Am-Af-Gm-Af-Am- Am-Am-Gm-Gms-Ums-Gm | 1241 |
| D579-DV25Pd7 BG5 | Cms-Cms-Um-Um-Um-Um-Cf-Um- Uf-Cf-Uf-Am-Am-Um-Gd-Am-Gf- Um-Cm-Gm-Am-G5 | 1315 | UmsEVP-Cfs-Gm-Am-Cm-Uf-Cd-Am- Um-Um-Am-Gm-Am-Af-Gm-Af-Am- Am-Am-Gm-Gms-Ums-Gm | 1354 |
| D579-DV25Pd6 7BG5 | Cms-Cms-Um-Um-Um-Um-Cf-Um- Uf-Cf-Uf-Am-Am-Um-Gd-Ad-Gf- Um-Cm-Gm-Am-G5 | 1316 | UmsEVP-Cfs-Gm-Am-Cm-Td-Cd-Am- Um-Um-Am-Gm-Am-Af-Gm-Af-Am- Am-Am-Gm-Gms-Ums-Gm | 1355 |
| D789-DV26PG5 | Gms-Ums-Um-Um-Gm-Um-Gf-Am- Af-Af-Cf-Am-Am-Am-Am-Am-Af- Gm-Um-Gm-Um-G5 | 1317 | AmsEVP-Cfs-Af-Cf-Um-Uf-Um-Um- Um-Um-Gm-Um-Um-Uf-Cm-Af-Cm- Am-Am-Am-Cms-Ams-Am | 1249 |
| D789-DV25PG5 | Gms-Ums-Um-Um-Gm-Um-Gf-Am- Af-Af-Cf-Am-Am-Am-Am-Am-Af- Gm-Um-Gm-Um-G5 | 1317 | AmsEVP-Cfs-Am-Cm-Um-Uf-Um-Um- Um-Um-Gm-Um-Um-Uf-Cm-Af-Cm- Am-Am-Am-Cms-Ams-Am | 1252 |
| D789-DV26Pd7 BG5 | Gms-Ums-Um-Um-Gm-Um-Gf-Am- Af-Af-Cf-Am-Am-Am-Ad-Am-Af- Gm-Um-Gm-Um-G5 | 1318 | AmsEVP-Cfs-Af-Cf-Um-Uf-Td-Um- Um-Um-Gm-Um-Um-Uf-Cm-Af-Cm- Am-Am-Am-Cms-Ams-Am | 1356 |
| D789-DV25Pd7 BG5 | Gms-Ums-Um-Um-Gm-Um-Gf-Am- Af-Af-Cf-Am-Am-Am-Ad-Am-Af- Gm-Um-Gm-Um-G5 | 1318 | AmsEVP-Cfs-Am-Cm-Um-Uf-Td-Um- Um-Um-Gm-Um-Um-Uf-Cm-Af-Cm- Am-Am-Am-Cms-Ams-Am | 1357 |
| D789-DV26Pd6 7BG5 | Gms-Ums-Um-Um-Gm-Um-Gf-Am- Af-Af-Cf-Am-Am-Am-Ad-Ad-Af- Gm-Um-Gm-Um-G5 | 1319 | AmsEVP-Cfs-Af-Cf-Um-Td-Td-Um- Um-Um-Gm-Um-Um-Uf-Cm-Af-Cm- Am-Am-Am-Cms-Ams-Am | 1358 |
| D789-DV25Pd6 7BG5 | Gms-Ums-Um-Um-Gm-Um-Gf-Am- Af-Af-Cf-Am-Am-Am-Ad-Ad-Af- Gm-Um-Gm-Um-G5 | 1319 | AmsEVP-Cfs-Am-Cm-Um-Td-Td-Um- Um-Um-Gm-Um-Um-Uf-Cm-Af-Cm- Am-Am-Am-Cms-Ams-Am | 1359 |
| D791-DV26PG5 | Ums-Ums-Gm-Um-Gm-Am-Af-Am- Cf-Af-Af-Am-Am-Am-Am-Gm-Uf- Gm-Um-Um-Am-G5 | 1320 | UmsEVP-Afs-Af-Cf-Am-Cf-Um-Um- Um-Um-Um-Um-Gm-Uf-Um-Uf-Cm- Am-Cm-Am-Ams-Ams-Cm | 1260 |
| D791-DV26Pd7 BG5 | Ums-Ums-Gm-Um-Gm-Am-Af-Am- Cf-Af-Af-Am-Am-Am-Ad-Gm-Uf- Gm-Um-Um-Am-G5 | 1321 | UmsEVP-Afs-Af-Cf-Am-Cf-Td-Um- Um-Um-Um-Um-Gm-Uf-Um-Uf-Cm- Am-Cm-Am-Ams-Ams-Cm | 1360 |
| D791-DV26Pd6 7BG5 | Ums-Ums-Gm-Um-Gm-Am-Af-Am- Cf-Af-Af-Am-Am-Am-Ad-Gd-Uf- Gm-Um-Um-Am-G5 | 1322 | UmsEVP-Afs-Af-Cf-Am-Cd-Td-Um- Um-Um-Um-Um-Gm-Uf-Um-Uf-Cm- Am-Cm-Am-Ams-Ams-Cm | 1361 |
| D795-DV27PG5 | Gms-Ams-Am-Am-Cm-Am-Af-Am- Af-Af-Af-Gm-Um-Gm-Um-Um-Cf- Cm-Cm-Um-Um-G5 | 1323 | AmsEVP-Afs-Gm-Gf-Gf-Af-Am-Cm- Am-Cm-Um-Um-Um-Uf-Um-Uf-Gm- Um-Um-Um-Cms-Ams-Cm | 1263 |
| D795-DV27Pd6 7BG5 | Gms-Ams-Am-Am-Cm-Am-Af-Am- Af-Af-Af-Gm-Um-Gm-Td-Td-Cf- Cm-Cm-Um-Um-G5 | 1324 | AmsEVP-Afs-Gm-Gf-Gf-Ad-Ad-Cm- Am-Cm-Um-Um-Um-Uf-Um-Uf-Gm- Um-Um-Um-Cms-Ams-Cm | 1362 |
| D812-DV25PG5 | Cms-Cms-Um-Um-Um-Um-Cf-Am- Af-Gf-Uf-Um-Gm-Am-Gm-Am-Af- Cm-Am-Am-Am-G5 | 1325 | UmsEVP-Ufs-Um-Gm-Um-Uf-Cm-Um- Cm-Am-Am-Cm-Um-Uf-Gm-Af-Am- Am-Am-Gm-Gms-Gms-Am | 1234 |
| D812-DV25Pd6 7BG5 | Cms-Cms-Um-Um-Um-Um-Cf-Am- Af-Gf-Uf-Um-Gm-Am-Gd-Ad-Af- Cm-Am-Am-Am-G5 | 1326 | UmsEVP-Ufs-Um-Gm-Um-Td-Cd-Um- Cm-Am-Am-Cm-Um-Uf-Gm-Af-Am- Am-Am-Gm-Gms-Gms-Am | 1363 |
| D816-DV25PG5 | Jms-Ums-Cm-Am-Am-Gm-Uf-Um- Gf-Af-Gf-Am-Am-Cm-Am-Am-Af- Am-Am-Um-Um-G5 | 1327 | AmsEVP-Afs-Um-Um-Um-Uf-Um-Gm- Um-Um-Cm-Um-Cm-Af-Am-Cf-Um- Um-Gm-Am-Ams-Ams-Am | 1227 |
| D816-DV25Pd6 7BG5 | Jms-Ums-Cm-Am-Am-Gm-Uf-Um- Gf-Af-Gf-Am-Am-Cm-Ad-Ad-Af- Am-Am-Um-Um-G5 | 1328 | AmsEVP-Afs-Um-Um-Um-Td-Td-Gm- Um-Um-Cm-Um-Cm-Af-Am-Cf-Um- Um-Gm-Am-Ams-Ams-Am | 1364 |
| D835-DV26PG5 | Jms-Ums-Gm-Gm-Gm-Um-Uf-Um- Uf-Af-Af-Am-Am-Um-Um-Am-Af- Am-Gm-Um-Am-G5 | 1329 | UmsEVP-Afs-Cf-Uf-Um-Uf-Am-Am- Um-Um-Um-Um-Am-Af-Am-Af-Cm- Cm-Cm-Am-Ams-Ums-Um | 1217 |
| D835-DV26Pd7 BG5 | Jms-Ums-Gm-Gm-Gm-Um-Uf-Um- Uf-Af-Af-Am-Am-Um-Td-Am-Af- Am-Gm-Um-Am-G5 | 1330 | UmsEVP-Afs-Cf-Uf-Um-Uf-Ad-Am- Um-Um-Um-Um-Am-Af-Am-Af-Cm- Cm-Cm-Am-Ams-Ums-Um | 1349 |
| D835-DV26Pd6 7BG5 | Jms-Ums-Gm-Gm-Gm-Um-Uf-Um- Uf-Af-Af-Am-Am-Um-Td-Ad-Af- Am-Gm-Um-Am-G5 | 1331 | UmsEVP-Afs-Cf-Uf-Um-Td-Ad-Am- Um-Um-Um-Um-Am-Af-Am-Af-Cm- Cm-Cm-Am-Ams-Ums-Um | 1350 |
| D838-DV29PG5 | Gms-Gms-Um-Um-Um-Um-Af-Am- Af-Am-Uf-Um-Am-Am-Am-Gm-Uf- Am-Um-Am-Am-G5 | 1332 | UmsEVP-Ufs-Am-Uf-Af-Cf-Um-Um- Um-Am-Am-Um-Um-Uf-Um-Af-Am- Am-Am-Cm-Cms-Cms-Am | 1280 |
| D838-DV29Pd7 BG5 | Gms-Gms-Um-Um-Um-Um-Af-Am- Af-Am-Uf-Um-Am-Am-Ad-Gm-Uf- Am-Um-Am-Am-G5 | 1333 | UmsEVP-Ufs-Am-Uf-Af-Cf-Td-Um- Um-Am-Am-Um-Um-Uf-Um-Af-Am- Am-Am-Cm-Cms-Cms-Am | 1365 |
| D838-DV29Pd6 7BG5 | Gms-Gms-Um-Um-Um-Um-Af-Am- Af-Am-Uf-Um-Am-Am-Ad-Gd-Uf- Am-Um-Am-Am-G5 | 1334 | UmsEVP-Ufs-Am-Uf-Af-Cd-Td-Um- Um-Am-Am-Um-Um-Uf-Um-Af-Am- Am-Am-Cm-Cms-Cms-Am | 1366 |
| D839-DV29Pd7 BG5 | Gms-Ums-Um-Um-Um-Am-Af-Am- Af-Um-Uf-Am-Am-Am-Gd-Um-Af- Um-Am-Cm-Am-G5 | 1336 | UmsEVP-Gfs-Um-Af-Uf-Af-Cd-Um- Um-Um-Am-Am-Um-Uf-Um-Uf-Am- Am-Am-Am-Cms-Cms-Cm | 1367 |
| D839-DV29Pd6 7BG5 | Gms-Ums-Um-Um-Um-Am-Af-Am- Af-Um-Uf-Am-Am-Am-Gd-Td-Af- Um-Am-Cm-Am-G5 | 1337 | UmsEVP-Gfs-Um-Af-Uf-Ad-Cd-Um- Um-Um-Am-Am-Um-Uf-Um-Uf-Am- Am-Am-Am-Cms-Cms-Cm | 1368 |
| D576s-DV29SPG 5 | Cms-Cms-Am-Cm-Cm-Uf-Um-Uf- Um-Cf-Um-Um-Cm-Um-Am-Af- Um-Gm-Am-Am-G5 | 1338 | UmsEVP-Ufs-Cm-Af-Uf-Uf-Am-Gm- Am-Am-Gm-Am-Am-Af-Am-Gf-Gm- Um-Gm-Gms-Gms-Am | 1272 |
| D576s-DV29SPd 7BG5 | Cms-Cms-Am-Cm-Cm-Uf-Um-Uf- Jm-Cf-Um-Um-Cm-Td-Am-Af-Um- Gm-Am-Am-G5 | 1339 | UmsEVP-Ufs-Cm-Af-Uf-Uf-Ad-Gm- Am-Am-Gm-Am-Am-Af-Am-Gf-Gm- Um-Gm-Gms-Gms-Am | 1369 |
| D576s-DV29SPd 67BG5 | Cms-Cms-Am-Cm-Cm-Uf-Um-Uf- Um-Cf-Um-Um-Cm-Td-Ad-Af-Um- Gm-Am-Am-G5 | 1340 | UmsEVP-Ufs-Cm-Af-Uf-Td-Ad-Gm- Am-Am-Gm-Am-Am-Af-Am-Gf-Gm- Um-Gm-Gms-Gms-Am | 1370 |
| D578s-DV29SPG5 | Ams-Cms-Cm-Um-Um-Uf-Um-Cf- Um-Uf-Cm-Um-Am-Am-Um-Gf- Am-Gm-Um-Am-G5 | 1341 | UmsEVP-Afs-Cm-Uf-Cf-Af-Um-Um- Am-Gm-Am-Am-Gm-Af-Am-Af-Am- Gm-Gm-Ums-Gms-Gm | 1276 |
| D578s-DV29SPd 7BG5 | Ams-Cms-Cm-Um-Um-Uf-Um-Cf- Um-Uf-Cm-Um-Am-Ad-Um-Gf-Am- Gm-Um-Am-G5 | 1342 | UmsEVP-Afs-Cm-Uf-Cf-Af-Td-Um- Am-Gm-Am-Am-Gm-Af-Am-Af-Am- Gm-Gm-Ums-Gms-Gm | 1353 |
| D578s-DV29SPd 67BG5 | Ams-Cms-Cm-Um-Um-Uf-Um-Cf- Um-Uf-Cm-Um-Am-Ad-Td-Gf-Am- Gm-Um-Am-G5 | 1343 | UmsEVP-Afs-Cm-Uf-Cf-Ad-Td-Um- Am-Gm-Am-Am-Gm-Af-Am-Af-Am- Gm-Gm-Ums-Gms-Gm | 1352 |
| D585s-DV27SPG 5 | Cms-Ums-Um-Cm-Um-Af-Am-Uf- Gf-Af-Gm-Um-Cm-Gm-Am-Cf-Um- Um-Um-Am-G5 | 1344 | UmsEVP-Afs-Am-Af-Gf-Uf-Cm-Gm- Am-Cm-Um-Cm-Am-Uf-Um-Af-Gm- Am-Am-Gms-Ams-Am | 1267 |
| Zilebesiran | Gms-Ums-Cm-Am-Um-Cm-Cf-Am- Cf-Af-Af-Um-Gm-Am-Gm-Am-Gm- Um-Am-Cm-Am-GL | 1345 | Ums-Gfs-Um-Am-Cm-Tgna-Cm-Um- Cm-Am-Um-Um-Gm-Uf-Gm-Gf-Am- Um-Gm-Am-Cms-Gms-Am | 1371 |
| DNC-DV29PG5 | Gms-Ums-Gm-Am-Am-Am-Cf-Am- Af-Cm-Uf-Am-Gm-Um-Gm-Cm-Af- Cm-Cm-Um-Cm-G5 | 1346 | UmsEVP-Afs-Gm-Af-Gf-Gf-Um-Gm- Cm-Am-Cm-Um-Am-Gf-Um-Uf-Gm- Um-Um-Um-Cms-Ams-Cm | 1372 |
| APC-ZL | Gms-Ums-Cm-Am-Um-Cm-Cf-Am- Cf-Af-Af-Um-Gm-Am-Gm-Am-Gm- Um-Am-Cm-Am | 1347 | Ums-Gfs-Um-Am-Cm-Tgna-Cm-Um- Cm-Am-Um-Um-Gm-Uf-Gm-Gf-Am- Um-Gm-Am-Cms-Gms-Am | 1371 |

Although specific embodiments of the present disclosure have been described above, those skilled in the art should understand that these are merely illustrative examples, and various changes or modifications may be made to these embodiments without departing from the principles and essence of the present disclosure. Accordingly, the scope of protection of the present disclosure is defined by the appended claims.

## Claims

1. A modification template for enhancing the inhibitory effect of a double-stranded oligonucleotide on a target gene, wherein the double-stranded oligonucleotide is an siRNA comprising a sense strand and an antisense strand; each nucleotide of the sense strand and the antisense strand is a modified nucleotide; wherein the sense strand comprises nucleotide sequence 1, and the antisense strand comprises nucleotide sequence 2; the nucleotide sequence 1 and the nucleotide sequence 2 are at least partially reverse complementary to form a duplex region; the reverse complementary duplex region is 17 to 21 bp in length;
from the 5' end to the 3' end, in the nucleotide sequence 2, at least two nucleotides among positions 2 to 6 are 2'-fluoro-modified nucleotides; the nucleotides at position 14 and/or 16 are 2'-fluoro-modified nucleotides; the nucleotides at the remaining positions are 2'-O-methyl-modified nucleotides; and positions 1 and 2 as well as positions of the second and third from last comprise phosphorothioate backbones.

2. The modification template according to claim 1, wherein at least two nucleotides among positions 2 to 6 of the nucleotide sequence 2 are selected from the following combinations:
(1) nucleotides at positions 2 and 6;
(2) nucleotides at positions 2, 3, 4, and 6;
(3) nucleotides at positions 2, 4, 5, and 6;
(4) nucleotides at positions 2, 5, and 6;
(5) nucleotides at positions 2, 3, 5, and 6;
(6) nucleotides at positions 2, 3, and 6;
and/or, from the 5' end to the 3' end, the nucleotide sequence 1 is modified as follows:
(1) the nucleotides at positions 6 and 16 are 2'-fluoro-modified nucleotides; at least two nucleotides among positions 8 to 10, preferably positions 8 to 10 or positions 8 and 10, are 2'-fluoro-modified nucleotides; the nucleotides at the remaining positions are 2'-O-methyl-modified nucleotides; and positions 1 and 2 comprise phosphorothioate backbones; or
(2) the nucleotides at positions 7 and 17 are 2'-fluoro-modified nucleotides; at least two nucleotides among positions 9 to 11, preferably positions 9 to 11 or positions 9 and 11, are 2'-fluoro-modified nucleotides; the nucleotides at the remaining positions are 2'-O-methyl-modified nucleotides; and positions 1 and 2 comprise phosphorothioate backbones.

3. The modification template according to claim 1 or 2, wherein the nucleotide sequence 1 is 21 or 20 nucleotides in length, and the nucleotide sequence 2 is 23 or 22 nucleotides in length;
preferably, the nucleotide sequence 1 is 20 nucleotides in length, and the nucleotide sequence 2 is 22 nucleotides in length; or, the nucleotide sequence 1 is 21 nucleotides in length, and the nucleotide sequence 2 is 23 nucleotides in length.

4. The modification template according to any one of claims 1 to 3, wherein, from the 5' end to the 3' end, the nucleotides in the antisense strand are modified according to any one of the following patterns A, B, C, D, E, F, and G:
A: indicates that, from the 5' end of the antisense strand, the nucleotides at positions 1, 3, 4, 5, 7, 8, 9, 10, 11, 12, 13, 15, 17, 18, 19, 20, 21, 22, and 23 are 2'-O-methyl-modified nucleotides; the nucleotides at positions 2, 6, 14, and 16 are 2'-fluoro-modified nucleotides; and positions 1, 2, 21, and 22 comprise phosphorothioate backbones;
B: indicates that, from the 5' end of the antisense strand, the nucleotides at positions 1, 5, 7, 8, 9, 10, 11, 12, 13, 15, 17, 18, 19, 20, 21, 22, and 23 are 2'-O-methyl-modified nucleotides; the nucleotides at positions 2, 3, 4, 6, 14, and 16 are 2'-fluoro-modified nucleotides; and positions 1, 2, 21, and 22 comprise phosphorothioate backbones;
C: indicates that, from the 5' end of the antisense strand, the nucleotides at positions 1, 3, 7, 8, 9, 10, 11, 12, 13, 15, 17, 18, 19, 20, 21, 22, and 23 are 2'-O-methyl-modified nucleotides; the nucleotides at positions 2, 4, 5, 6, 14, and 16 are 2'-fluoro-modified nucleotides; and positions 1, 2, 21, and 22 comprise phosphorothioate backbones;
D: indicates that, from the 5' end of the antisense strand, the nucleotides at positions 1, 3, 4, 7, 8, 9, 10, 11, 12, 13, 15, 17, 18, 19, 20, 21, 22, and 23 are 2'-O-methyl-modified nucleotides; the nucleotides at positions 2, 5, 6, 14, and 16 are 2'-fluoro-modified nucleotides; and positions 1, 2, 21, and 22 comprise phosphorothioate backbones;
E: indicates that, from the 5' end of the antisense strand, the nucleotides at positions 1, 4, 7, 8, 9, 10, 11, 12, 13, 15, 17, 18, 19, 20, 21, 22, and 23 are 2'-O-methyl-modified nucleotides; the nucleotides at positions 2, 3, 5, 6, 14, and 16 are 2'-fluoro-modified nucleotides; and positions 1, 2, 21, and 22 comprise phosphorothioate backbones;
F: indicates that, from the 5' end of the antisense strand, the nucleotides at positions 1, 4, 5, 7, 8, 9, 10, 11, 12, 13, 15, 17, 18, 19, 20, 21, 22, and 23 are 2'-O-methyl-modified nucleotides; the nucleotides at positions 2, 3, 6, 14, and 16 are 2'-fluoro-modified nucleotides; and positions 1, 2, 21, and 22 comprise phosphorothioate backbones; G: indicates that, from the 5' end of the antisense strand, the nucleotides at positions 1, 3, 4, 5, 7, 8, 9, 10, 11, 12, 13, 15, 17, 18, 19, 20, 21, 22, and 23 are 2'-O-methyl-modified nucleotides; the nucleotides at positions 2, 6, 14, and 16 are 2'-fluoro-modified nucleotides; and positions 1, 2, 21, and 22 comprise phosphorothioate backbones;
G: indicates that, from the 5' end of the antisense strand, the nucleotides at positions 1, 3, 4, 5, 7, 8, 9, 10, 11, 12, 13, 15, 17, 18, 19, 20, 21, 22, and 23 are 2'-O-methyl-modified nucleotides; the nucleotides at positions 2, 6, 14, and 16 are 2'-fluoro-modified nucleotides; and positions 1, 2, 21, and 22 comprise phosphorothioate backbones;
and/or, from the 5' end to the 3' end, the nucleotides in the sense strand are modified according to any one of the following patterns a to d:
a: indicates that, from the 5' end of the sense strand, the nucleotides at positions 1, 2, 3, 4, 5, 6, 8, 12, 13, 14, 15, 16, 18, 19, 20, and 21 are 2'-O-methyl-modified nucleotides; the nucleotides at positions 7, 9, 10, 11, and 17 are 2'-fluoro-modified nucleotides; and positions 1 and 2 comprise phosphorothioate backbones;
b: indicates that, from the 5' end of the sense strand, the nucleotides at positions 1, 2, 3, 4, 5, 6, 8, 10, 12, 13, 14, 15, 16, 18, 19, 20, and 21 are 2'-O-methyl-modified nucleotides; the nucleotides at positions 7, 9, 11, and 17 are 2'-fluoro-modified nucleotides; and positions 1 and 2 comprise phosphorothioate backbones;
c: indicates that, from the 5' end of the sense strand, the nucleotides at positions 1, 2, 3, 4, 5, 6, 8, 12, 13, 14, 15, 16, 17, 18, 19, 20, and 21 are 2'-O-methyl-modified nucleotides; the nucleotides at positions 7, 9, 10, and 11 are 2'-fluoro-modified nucleotides; and positions 1 and 2 comprise phosphorothioate backbones;
d: indicates that, from the 5' end of the sense strand, the nucleotides at positions 1, 2, 3, 4, 5, 6, 8, 11, 12, 13, 14, 15, 16, 17, 18, 19, 20, and 21 are 2'-O-methyl-modified nucleotides; the nucleotides at positions 7, 9, and 10 are 2'-fluoro-modified nucleotides; and positions 1 and 2 comprise phosphorothioate backbones.

5. The modification template according to any one of claims 1 to 4, wherein, from the 5' end to the 3' end, the nucleotides in the antisense strand are modified according to any one of the following patterns A', B', C', D', E', F', and G':
A': indicates that, from the 5' end of the antisense strand, the nucleotides at positions 1, 3, 4, 5, 7, 8, 9, 10, 11, 12, 13, 15, 17, 18, 19, 20, 21, and 22 are 2'-O-methyl-modified nucleotides; the nucleotides at positions 2, 6, 14, and 16 are 2'-fluoro-modified nucleotides; and positions 1, 2, 20, and 21 comprise phosphorothioate backbones;
B': indicates that, from the 5' end of the antisense strand, the nucleotides at positions 1, 5, 7, 8, 9, 10, 11, 12, 13, 15, 17, 18, 19, 20, 21, and 22 are 2'-O-methyl-modified nucleotides; the nucleotides at positions 2, 3, 4, 6, 14, and 16 are 2'-fluoro-modified nucleotides; and positions 1, 2, 20, and 21 comprise phosphorothioate backbones;
C': indicates that, from the 5' end of the antisense strand, the nucleotides at positions 1, 3, 7, 8, 9, 10, 11, 12, 13, 15, 17, 18, 19, 20, 21, and 22 are 2'-O-methyl-modified nucleotides; the nucleotides at positions 2, 4, 5, 6, 14, and 16 are 2'-fluoro-modified nucleotides; and positions 1, 2, 20, and 21 comprise phosphorothioate backbones;
D': indicates that, from the 5' end of the antisense strand, the nucleotides at positions 1, 3, 4, 7, 8, 9, 10, 11, 12, 13, 15, 17, 18, 19, 20, 21, and 22 are 2'-O-methyl-modified nucleotides; the nucleotides at positions 2, 5, 6, 14, and 16 are 2'-fluoro-modified nucleotides; and positions 1, 2, 20, and 21 comprise phosphorothioate backbones;
E': indicates that, from the 5' end of the antisense strand, the nucleotides at positions 1, 4, 7, 8, 9, 10, 11, 12, 13, 15, 17, 18, 19, 20, 21, and 22 are 2'-O-methyl-modified nucleotides; the nucleotides at positions 2, 3, 5, 6, 14, and 16 are 2'-fluoro-modified nucleotides; and positions 1, 2, 20, and 21 comprise phosphorothioate backbones;
F': indicates that, from the 5' end of the antisense strand, the nucleotides at positions 1, 4, 5, 7, 8, 9, 10, 11, 12, 13, 15, 17, 18, 19, 20, 21, and 22 are 2'-O-methyl-modified nucleotides; the nucleotides at positions 2, 3, 6, 14, and 16 are 2'-fluoro-modified nucleotides; and positions 1, 2, 20, and 21 comprise phosphorothioate backbones;
G': indicates that, from the 5' end of the antisense strand, the nucleotides at positions 1, 3, 4, 5, 7, 8, 9, 10, 11, 12, 13, 15, 16, 17, 18, 19, 20, 21, and 22 are 2'-O-methyl-modified nucleotides; the nucleotides at positions 2, 6, and 14 are 2'-fluoro-modified nucleotides; and positions 1, 2, 20, and 21 comprise phosphorothioate backbones;
and/or, from the 5' end to the 3' end, the nucleotides in the sense strand are modified according to any one of the following patterns a', b', c', and d':
a': indicates that, from the 5' end of the sense strand, the nucleotides at positions 1, 2, 3, 4, 5, 7, 11, 12, 13, 14, 15, 17, 18, 19, and 20 are 2'-O-methyl-modified nucleotides; the nucleotides at positions 6, 8, 9, 10, and 16 are 2'-fluoro-modified nucleotides; and positions 1 and 2 comprise phosphorothioate backbones;
b': indicates that, from the 5' end of the sense strand, the nucleotides at positions 1, 2, 3, 4, 5, 7, 9, 11, 12, 13, 14, 15, 17, 18, 19, and 20 are 2'-O-methyl-modified nucleotides; the nucleotides at positions 6, 8, 10, and 16 are 2'-fluoro-modified nucleotides; and positions 1 and 2 comprise phosphorothioate backbones;
c': indicates that, from the 5' end of the sense strand, the nucleotides at positions 1, 2, 3, 4, 5, 7, 11, 12, 13, 14, 15, 16, 17, 18, 19, and 20 are 2'-O-methyl-modified nucleotides; the nucleotides at positions 6, 8, 9, and 10 are 2'-fluoro-modified nucleotides; and positions 1 and 2 comprise phosphorothioate backbones;
d': indicates that, from the 5' end of the sense strand, the nucleotides at positions 1, 2, 3, 4, 5, 7, 10, 11, 12, 13, 14, 15, 16, 17, 18, 19, and 20 are 2'-O-methyl-modified nucleotides; the nucleotides at positions 6, 8, and 9 are 2'-fluoro-modified nucleotides; and positions 1 and 2 comprise phosphorothioate backbones;
preferably, from the 5' end to the 3' end, the double-stranded oligonucleotide is modified according to any one of the following combinations (i) to (x):
(i) the antisense strand is modified according to pattern A, and the sense strand is modified according to pattern a;
(ii) the antisense strand is modified according to pattern B, and the sense strand is modified according to pattern a;
(iii) the antisense strand is modified according to pattern C, and the sense strand is modified according to pattern a;
(iv) the antisense strand is modified according to pattern B, and the sense strand is modified according to pattern b;
(v) the antisense strand is modified according to pattern C, and the sense strand is modified according to pattern b;
(vi) the antisense strand is modified according to pattern G, and the sense strand is modified according to pattern c;
(vii) the antisense strand is modified according to pattern A, and the sense strand is modified according to pattern d;
(viii) the antisense strand is modified according to pattern D, and the sense strand is modified according to pattern b;
(ix) the antisense strand is modified according to pattern E, and the sense strand is modified according to pattern b;
(x) the antisense strand is modified according to pattern F, and the sense strand is modified according to pattern b;
or, from the 5' end to the 3' end, the double-stranded oligonucleotide is modified according to any one of the following combinations (1) to (8):
(1) the antisense strand is modified according to pattern A', and the sense strand is modified according to pattern a';
(2) the antisense strand is modified according to pattern B', and the sense strand is modified according to pattern a';
(3) the antisense strand is modified according to pattern C', and the sense strand is modified according to pattern a';
(4) the antisense strand is modified according to pattern B', and the sense strand is modified according to pattern b';
(5) the antisense strand is modified according to pattern C', and the sense strand is modified according to pattern b';
(6) the antisense strand is modified according to pattern D', and the sense strand is modified according to pattern b';
(7) the antisense strand is modified according to pattern E', and the sense strand is modified according to pattern b';
(8) the antisense strand is modified according to pattern F', and the sense strand is modified according to pattern b'.

6. The modification template according to any one of claims 1 to 5, wherein the nucleotide at position 1 from the 5' end of the antisense strand is a 5'-phosphorylated nucleotide or a nucleotide modified with a 5'-phosphate analog, including, but not limited to, the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are as follows: 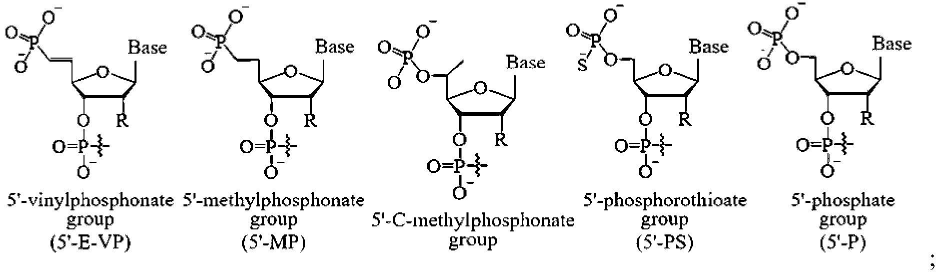
wherein R is hydrogen, hydroxyl, amino, C₁₋₄ alkyl, aryl, C₁₋₄ alkoxy, C₁₋₄ alkylcarbonylamino, or halogen;
the base is selected from adenine, guanine, cytosine, thymine, and uracil;
preferably, the nucleotide at position 1 from the 5' end of the antisense strand is a 5'-vinyl-(E)-phosphonate-2'-O-methyl-modified nucleotide.

7. The modification template according to any one of claims 1 to 6, wherein one or more nucleotides at positions 2 to 8 from the 5' end of the antisense strand, preferably at position 6 and/or 7, comprises a modification group, wherein the modification group is selected from UNA, GNA, and DNA, wherein the structures of UNA and GNA are as follows: 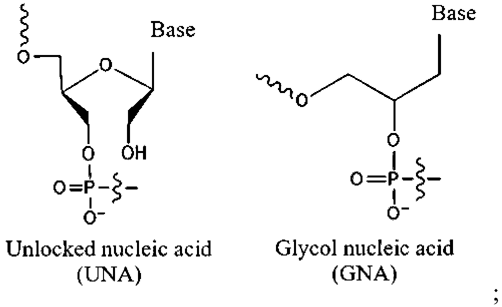 the base in the double-stranded oligonucleotide is selected from one or more adenine, guanine, cytosine, thymine, and uracil.

8. A double-stranded oligonucleotide, wherein the double-stranded oligonucleotide is modified using the modification template according to any one of claims 1 to 7;
preferably, the double-stranded oligonucleotide targets the mRNA of one of the following genes: PCSK9 gene, HBV gene, and AGT gene.

9. A conjugate for reducing gene expression, wherein the conjugate comprises the double-stranded oligonucleotide according to claim 8 and a ligand conjugated thereto.

10. The conjugate according to claim 9, wherein the ligand is conjugated to the 3' end or 5' end of the sense strand of the double-stranded oligonucleotide;
preferably, the ligand is one or more GalNAc derivatives attached via a bivalent or trivalent branched linker;
more preferably, the double-stranded oligonucleotide is conjugated to ligand L96, G4, G5, G6, or G7.

11. A pharmaceutical composition, wherein the pharmaceutical composition comprises the double-stranded oligonucleotide according to claim 8 or the conjugate according to claim 9 or 10, and a pharmaceutically acceptable carrier.

12. Use of the double-stranded oligonucleotide according to claim 8, the conjugate according to claim 9 or 10, or the pharmaceutical composition according to claim 11 in the manufacture of a medicament for modulating or treating a disease or disorder; wherein the medicament achieves the effect of modulating or treating the disease or disorder by downregulating gene expression via the double-stranded oligonucleotide, the conjugate, or the pharmaceutical composition;
preferably, the gene is selected from the following genes: PCSK9 gene, HBV gene, and AGT gene;
more preferably, the disease is selected from one or more chronic hepatitis B, liver fibrosis, cirrhosis, liver cancer, acute hepatitis B, and diseases associated with co-infection of HBV and hepatitis D virus;
the disease is selected from hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction; or
the disease is selected from hypercholesterolemia, atherosclerosis, dyslipidemia, and cardiovascular and cerebrovascular diseases.

13. The double-stranded oligonucleotide according to claim 8, the conjugate according to claim 9 or 10, or the pharmaceutical composition according to claim 11 for use in modulating or treating a disease or disorder; wherein the double-stranded oligonucleotide, the conjugate, or the pharmaceutical composition achieves the effect of modulating or treating the disease or disorder by downregulating gene expression;
preferably, the gene is selected from the following genes: PCSK9 gene, HBV gene, and AGT gene;
more preferably, the disease is selected from one or more chronic hepatitis B, liver fibrosis, cirrhosis, liver cancer, acute hepatitis B, and diseases associated with co-infection of HBV and hepatitis D virus;
the disease is selected from hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction; or
the disease is selected from hypercholesterolemia, atherosclerosis, dyslipidemia, and cardiovascular and cerebrovascular diseases.

14. A method of treating or preventing a disease or disorder that can be modulated or treated by downregulating gene expression, wherein the method comprises administering to a subject in need thereof an effective amount of the double-stranded oligonucleotide according to claim 8, the conjugate according to claim 9 or 10, or the pharmaceutical composition according to claim 11;
preferably, the gene is selected from the following genes: PCSK9 gene, HBV gene, and AGT gene;
more preferably, the disease is selected from one or more chronic hepatitis B, liver fibrosis, cirrhosis, liver cancer, acute hepatitis B, and diseases associated with co-infection of HBV and hepatitis D virus;
the disease is selected from hypertension, borderline hypertension, primary hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid diseases, heart failure, myocardial infarction, angina, stroke, diabetes, nephropathy, renal failure, systemic sclerosis, intrauterine growth retardation (IUGR), and fetal growth restriction; or
the disease is selected from hypercholesterolemia, atherosclerosis, dyslipidemia, and cardiovascular and cerebrovascular diseases.
